# EUROPEAN PATENT SPECIFICATION

(11) **EP 2 776 830 B1**
(45) Date of publication and mention of the grant of the patent: **09.05.2018**
(21) Application number: 12847964.9
(22) Date of filing: 02.11.2012
(51) Int. Cl.: G01N 33/50, C12Q 1/68

(54) **METHOD OF PREDICTING BREAST CANCER PROGNOSIS**
VERFAHREN ZUR VORHERSAGE DER BRUSTKREBSPROGNOSE
MÉTHODE DE PRÉDICTION DU PRONOSTIC DU CANCER DU SEIN

(30) Priority: 08.11.2011 US 201161557238 P; 10.02.2012 US 201261597426 P
(43) Date of publication of application: 17.09.2014
(73) Proprietor: Genomic Health, Inc., Redwood City, CA 94063 (US)
(72) Inventor: BAKER, Joffre B., Redwood City, CA 94063 (US); SINICROPI, Dominick V., Woodside, CA 94062 (US); PELHAM, Robert J., Belmont, CA 94002 (US); CRAGER, Michael, Redwood City, CA 94063 (US); COLLIN, Francois, Berkeley, CA 94702 (US); STEPHANS, James C., Redwood City, CA 94063 (US); LIU, Mei-Lan, San Mateo, CA 94403 (US); MORLAN, John, Redwood City, CA 94063 (US); QU, Kunbin, Palo Alto, CA 94303 (US)
(74) Representative: Gowshall, Jonathan Vallance
(86) International application number: PCT/US2012/063313
(87) International publication number: WO 2013/070521

(56) References cited:
- WO-A1-2008/046182
- WO-A1-2011/044513
- WO-A1-2011/076895
- WO-A1-2011/107819
- WO-A2-2008/079269
- CN-A- 101 851 611
- US-A1- 2008 299 550
- US-A1- 2011 020 370
- US-A1- 2011 123 990
- TU SHIH-HSIN ET AL: "Increased expression of enolase alpha in human breast cancer confers tamoxifen resistance in human breast cancer cells.", BREAST CANCER RESEARCH AND TREATMENT JUN 2010, vol. 121, no. 3, June 2010 (2010-06), pages 539-553, XP002740585, ISSN: 1573-7217
- PEI-YI CHU ET AL: "Overexpression of [alpha]-enolase correlates with poor survival in canine mammary carci", BMC VETERINARY RESEARCH, BIOMED CENTRAL, LONDON, GB, vol. 7, no. 1, 21 October 2011 (2011-10-21), page 62, XP021114153, ISSN: 1746-6148, DOI: 10.1186/1746-6148-7-62
- YU WOO SIK ET AL: "The genome-wide expression profile of 1,2,3,4,6-penta-O-galloyl-beta-D-glucose-t reated MDA-MB-231 breast cancer cells: Molecular target on cancer metabolism", MOLECULES AND CELLS, vol. 32, no. 2, August 2011 (2011-08), pages 123-132, XP002740586,
- TU ET AL.: 'Increased expression of enolase a in human breast cancer confers tamoxifen resistance in human breast cancer cells.' BREAST CANCER RES TREAT. vol. 121, 2010, pages 539 - 553, XP019814348

## Description

### FIELD OF THE INVENTION

The present invention relates to biomarkers associated with breast cancer prognosis. These biomarkers include coding transcripts and their expression products, as well as non-coding transcripts, and are useful for predicting the likelihood of breast cancer recurrence in a breast cancer patient.

### INTRODUCTION

For over a decade, technologies such as DNA microarray and reverse transcription polymerase chain reaction (RT-PCR) have demonstrated that levels of certain RNA transcripts ("gene expression profiles") relate to patient stratification and disease outcomes, especially in a variety of cancers. Several validated and now widely used clinical tests make use of gene expression profiling, such as the Oncotype DX® RT-PCR test, which measures the levels of 21 biomarker RNAs in archival formalin-fixed paraffin-embedded (FFPE) tissue. The *Oncotype* DX® RT-PCR test predicts the risk of recurrence of early estrogen receptor (ER)-positive breast cancer, as well as the likelihood of response to chemotherapy, and is now used to guide treatment decisions for about half of ER+ breast cancer patients in the U.S.

However, RT-PCR is constrained by the number of transcripts and sequence complexity that can be interrogated, especially given the limited amount of patient FFPE RNA available from many tumor specimens. Recent major advances in DNA sequencing ("next generation sequencing") provide massively parallel throughput and data volumes that eclipse the nucleic acid information content possible with other technologies, such as RT-PCR. Next generation sequencing makes feasible unprecedented extensive genome analysis of groups of individuals, including analyses of sequence differences, polymorphisms, mutations, copy number variations, epigenetic variations and transcript abundance (RNA-Seq).

### SUMMARY

A multiplexed, whole genome sequencing methodology was developed to enable whole transcriptome-wide breast cancer biomarker discovery using low amounts of FFPE tissue. The present invention provides biomarkers that associate, positively or negatively, with a particular clinical outcome in breast cancer. These biomarkers are listed in Tables 1-5 and 15. For example, the clinical outcome could be no cancer recurrence or cancer recurrence. The clinical outcome may be defined by clinical endpoints, such as disease or recurrence free survival, metastasis free survival, overall survival, etc.

The present invention accommodates the use of archived paraffin-embedded biopsy material for assay of all markers in the set, and therefore is compatible with the most widely available type of biopsy material. It is also compatible with other different methods of tumor tissue harvest, for example, via core biopsy or fine needle aspiration.

In one aspect, the present invention provides a method of predicting a likelihood of long-term survival without recurrence of breast cancer in a breast cancer patient. The method comprises determining a level of one or more RNA transcripts, or its expression product, in a breast cancer tumor sample obtained from the patient. The RNA transcript or its expression product may be selected from Tables 1 and 15. The likelihood of long-term survival without breast cancer recurrence is then predicted based on the negative or positive correlation of the RNA transcript or its expression product with increased likelihood of long-term survival without breast cancer recurrence. An RNA transcript is negatively correlated with increased long-term survival without recurrence of breast cancer if its direction of association is marked 1 in Tables 1 and 15, and is positively correlated with increased long-term survival without recurrence of breast cancer if its direction of association is marked -lin Tables 1 and 15.

In another aspect, the present invention provides a method of predicting a likelihood of long-term survival without recurrence of breast cancer in an estrogen receptor (ER)-positive breast cancer patient. The method comprises determining a level of one or more RNA transcripts, or its expression product, in a breast cancer tumor sample obtained from the patient. The RNA transcript or its expression product may be selected from Table 2. The likelihood of long-term survival without breast cancer recurrence is then predicted based on the negative or positive correlation of the RNA transcript or its expression product with increased likelihood of long-term survival without breast cancer recurrence. An RNA transcript is negatively correlated with increased long-term survival without recurrence of breast cancer if its direction of association is marked 1 in Table 2, and is positively correlated with increased long-term survival without recurrence of breast cancer if its direction of association is marked -1 in Table 2.

The RNA transcripts, or the expression products, may be grouped into gene networks based on the current understanding of their cellular function. For example, the gene networks include a cell cycle network, ESR1 network, Chr9q22network, Chr17q23-24 network, Chr8q21-24 network, olfactory receptor network, and metabolic-like networks. The present invention therefore also provides a method of predicting a likelihood of long-term survival without breast cancer recurrence in a breast cancer patient by determining a quantitative value, such as a likelihood score, for one or more gene networks based on the level of at least one RNA transcript, or expression product thereof, within the gene network, in a breast cancer tumor sample obtained from the patient. The quantitative value for the gene network may be determined by weighting the contribution of one or more RNA transcripts, or their expression products, to clinical outcome, such as risk of recurrence.

In yet another aspect, the present invention provides a method of predicting a likelihood of long-term survival without recurrence of breast cancer in a breast cancer patient by determining a level of one or more non-coding sequences in a breast cancer tissue sample obtained from the patient. In one embodiment, the non-coding sequence is one or more intronic RNAs selected from Table 3. In another embodiment, the non-coding sequence is one or more long intergenic non-coding regions (lincRNAs) selected from Table 4. In a further embodiment, the non-coding sequence is one or more intergenic sequences selected from Table 5. In yet another embodiment, the non-coding sequence is one or more intergenic regions 1-69 selected from Table 5. The intergenic region may be comprised of one or more intergenic sequences according to Table 5. The likelihood of long-term survival without breast cancer recurrence is predicted based on the negative or positive correlation of the non-coding sequence with increased likelihood of long-term survival without breast cancer recurrence. A non-coding sequence is negatively correlated with increased long-term survival without recurrence of breast cancer if its direction of association is marked 1 in Tables 3-5, and is positively correlated with increased long-term survival without recurrence of breast cancer if its direction of association is marked -1 in Tables 3-5.

In a further aspect, the present invention provides a method of predicting a likelihood of long-term survival without recurrence of breast cancer in a breast cancer patient by determining a level of an RNA transcript, or an expression product thereof, from a metabolic-like network in a breast cancer tumor sample obtained from the patient. The metabolic-like networks include a five-gene set comprising ENO1, IDH2, TMSB10, PGK1, and G6PD, and a fourteen-gene set comprising PGD, TKT, TALDO1, G6PD, GP1, SLC1A5, SLC7A5, OGDH, SUCLG1, ENO1, PGK1, IDH2, ACO2, and FBP1. In one aspect, levels of at least three RNA transcripts, or expression products thereof, selected from ENO1, IDH2, TMSB10, PGK1, and G6PD, are determined. In a specific embodiment, the levels of at least IDH2, PGK1, and G6PD are determined. In yet another embodiment, the levels of ENO1, IDH2, TMSB10, PGK1, and G6PD are determined. In another aspect, levels of at least five RNA transcripts, or expression products thereof, selected from PGD, TKT, TALDO1, G6PD, GP1, SLC1A5, SLC7A5, OGDH, SUCLG1, ENO1, PGK1, IDH2, ACO2, and FBP1, are determined. In a specific embodiment, the levels of the RNA transcripts, or expression products thereof, of PGD, TKT, TALDO1, G6PD, GP1, SLC1A5, SLC7A5, OGDH, SUCLG1, ENO1, PGK1, IDH2, ACO2, and FBP1, are determined. The likelihood of long-term survival without breast cancer recurrence is predicted based on the negative or positive correlation of the RNA transcripts, or expression products thereof, with increased likelihood of long-term survival without breast cancer recurrence. Levels of ENO1, IDH2, TMSB10, PGK1, G6PD, PGD, TKT, TALDO1, GP1, SLC1A5, SLC7A5, OGDH, SUCLG1, and ACO2 are all negatively correlated with an increased likelihood of long-term survival without recurrence of breast cancer, while the level of FBP1 is positively correlated with an increased likelihood of long-term survival without recurrence of breast cancer.

Any of the above methods may utilize a combination of coding and non-coding RNA transcripts for predicting breast cancer prognosis. Moreover, any of the above methods may be performed by whole transcriptome sequencing, reverse transcription polymerase chain reaction (RT-PCR), or by array. Other methods known in the art may be used. In an embodiment of the invention, the breast cancer tumor sample is a fixed, wax-embedded tissue sample or a fine needle biopsy sample. In another embodiment, the level of the RNA transcript, or its expression product, or the level of the non-coding sequence may be normalized.

In an embodiment of the invention, a likelihood score (e.g., a score predicting a likelihood of long-term survival without breast cancer recurrence) can be calculated based on the level or normalized level of the coding RNA transcript, or an expression product thereof, and/or non-coding RNA transcript. A score may be calculated using weighted values based on the level or normalized level of the coding RNA transcript (or expression product thereof) and/or the non-coding RNA transcript, and its contribution to clinical outcome, such as long-term survival without breast cancer recurrence.

### BRIEF DESCRIPTION OF THE DRAWINGS

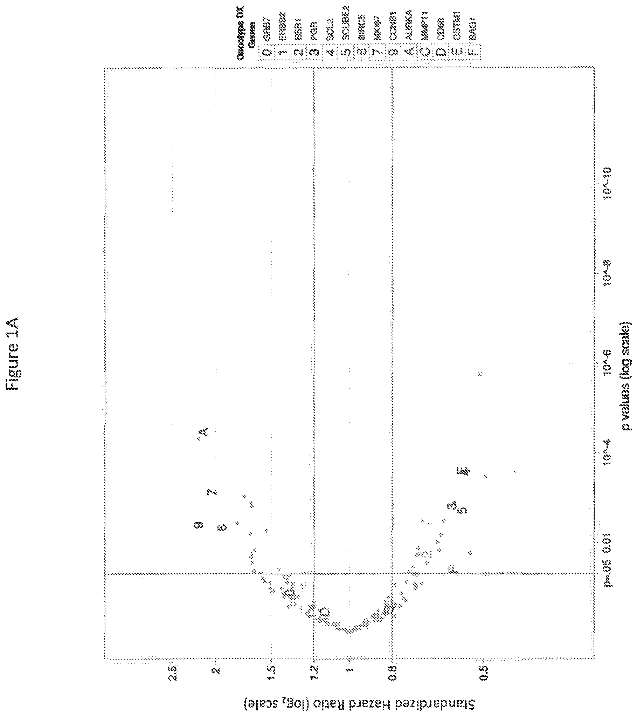

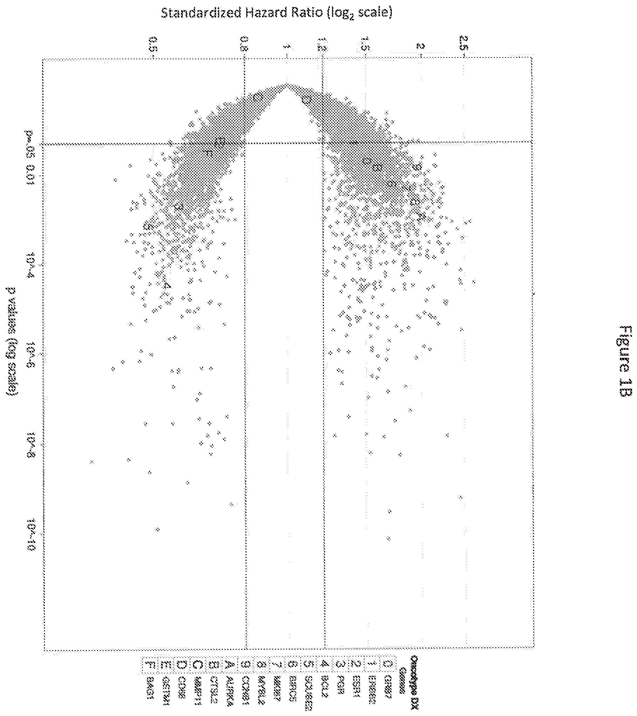

FIG. 1 shows the relationship of increased RNA expression to risk of breast cancer recurrence in 136 breast cancer patients. Each point represents a distinct RNA sequence. The magnitude of the effect size is given by the hazard ratio from Cox proportional hazard analysis and statistical significance by P-Value. Fig. 1A shows an analysis of 192 genes measured by RT-PCR. Tested Onco*type* Dx® genes are indicated. Fig. 1B shows an analysis of assembled RefSeq transcripts as measured by whole transcriptome sequencing.

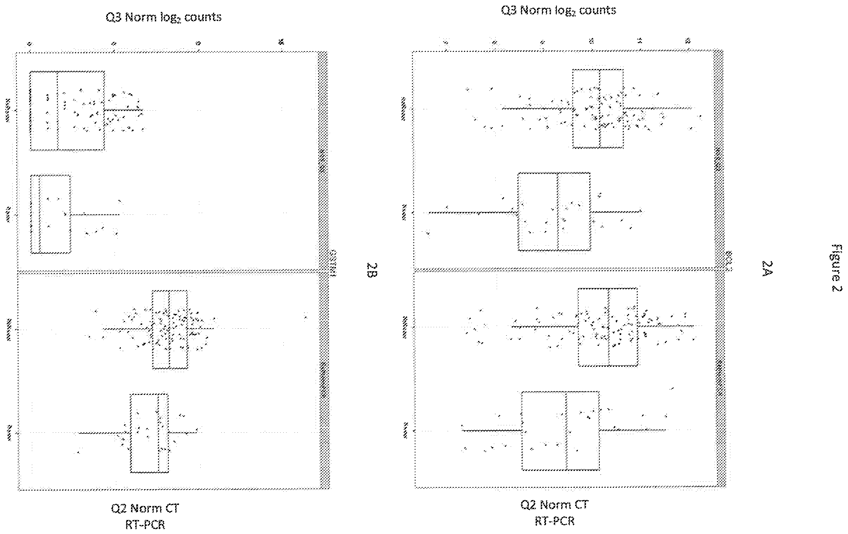

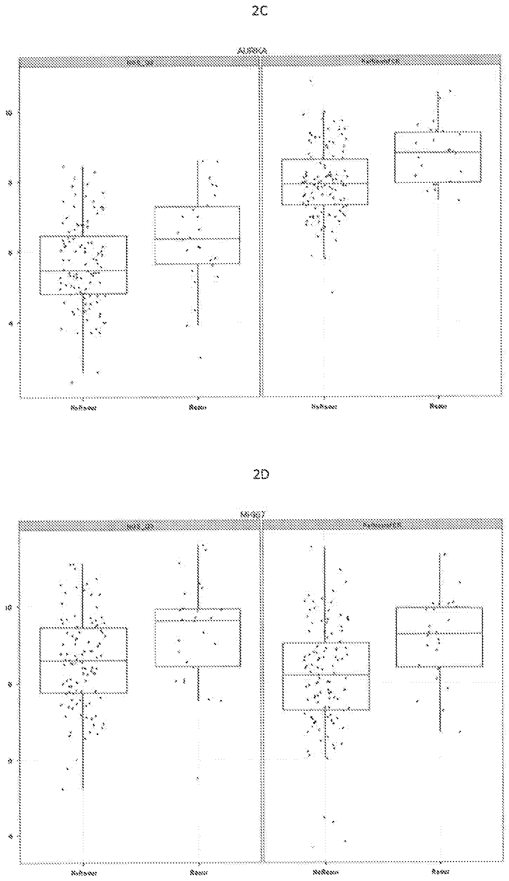

FIG. 2 are boxplots of normalized expression values of RNAs in breast cancer patients, stratified by recurrence status. Each point represents a patient tumor. The bottom and top of the box are the 25^{th} and 75^{th} percentiles and the band within the box is the 50^{th} percentile (the median) of the points in the group. The ends of the whiskers represent the lowest datum still within 1.5 interquartile range (IQR) of the lower quartile, and the highest datum still within 1.5 IQR of the upper quartile. Values from RNA-Seq (left panel) and RT-PCR (right panel) are shown: Fig. 2A: BCL2; Fig. 2B: GSTM1; Fig. 2C: AURKA; Fig. 2D: MKI67.

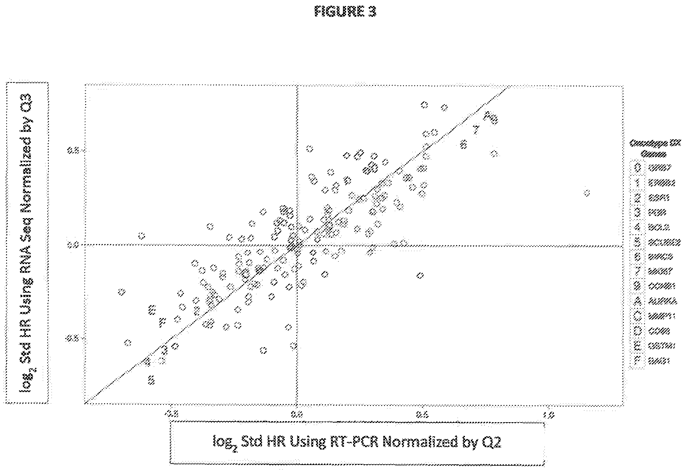

FIG. 3 is a scatter plot of the breast cancer recurrence risk hazard ratios of 192 RNA sequences comparing the RT-PCR results (x-axis) versus RNA-Seq (assembled RefSeq) results (y axis). Each point represents a distinct RNA.

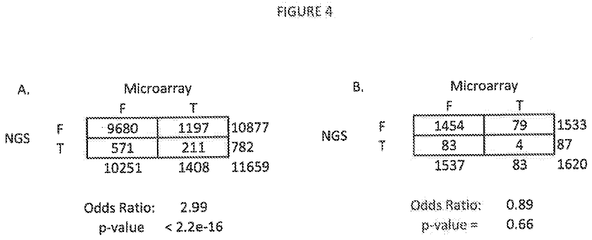

FIG. 4 is a comparison of the genes identified using publicly available microarray data and the NGS ("next generation sequencing") data of the present invention. Fig. 4A shows that there is substantial agreement in the genes identified as prognostic between the two platforms (11,659 genes in common, odds ratio = 2.99). Fig. 4B shows that at the low end of RNA-Seq expression (RNAs with mean counts <10.25), the level of agreement among the two platforms is not statistically significant (1620 genes in common, odds ratio = 0.89).

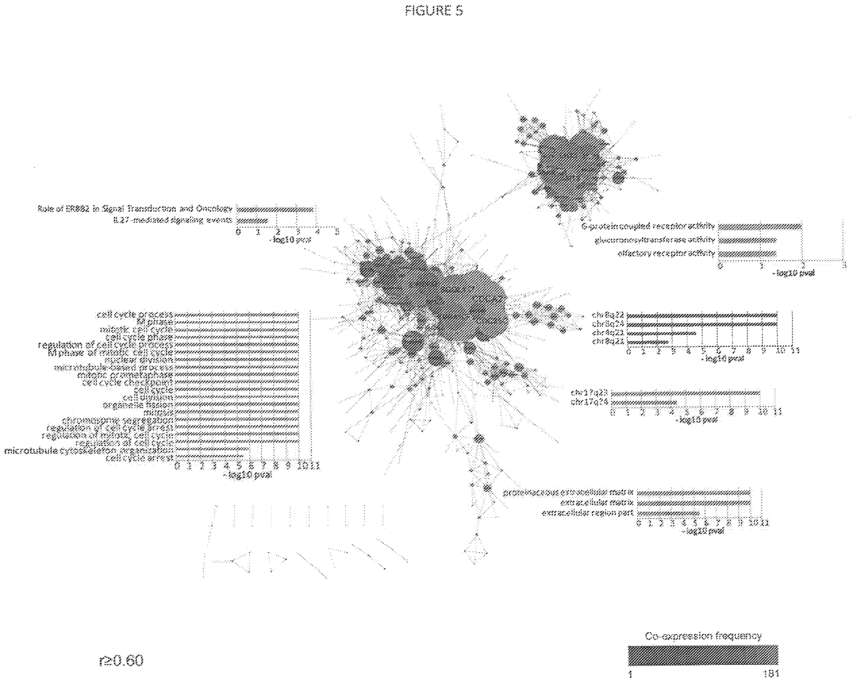

FIG. 5 is a 2D visualization of the network of gene co-expression (with a Pearson correlation coefficient cutoff of ≥0.6) amongst the 1307 identified prognostic RefSeqs generated using Cytoscape 2.8.

### DETAILED DESCRIPTION

Before the present invention and specific exemplary embodiments of the invention are described, it is to be understood that this invention is not limited to particular embodiments described, as such may, of course, vary. It is also to be understood that the terminology used herein is for the purpose of describing particular embodiments only, and is not intended to be limiting, since the scope of the present invention will be limited only by the appended claims.

Where a range of values is provided, it is understood that each intervening value, to the tenth of the unit of the lower limit unless the context clearly dictates otherwise, between the upper and lower limit of that range and any other stated or intervening value in that stated range is encompassed within the invention. The upper and lower limits of these smaller ranges may independently be included in the smaller ranges is also encompassed within the invention, subject to any specifically excluded limit in the stated range. Where the stated range includes one or both of the limits, ranges excluding either both of those included limits are also included in the invention.

As used herein and in the appended claims, the singular forms "a," "an," and "the" include plural referents unless the context clearly dictates otherwise. Thus, for example, reference to "an RNA transcript" includes a plurality of such RNA transcripts.

Unless defined otherwise, technical and scientific terms used herein have the same meaning as commonly understood by one of ordinary skill in the art to which this invention belongs. For example, Singleton et al., Dictionary of Microbiology and Molecular Biology 2nd ed., J. Wiley & Sons (New York, NY 1994), provide one skilled in the art with a general guide to many of the terms used in the present application.

Additionally, the practice of the present invention will employ, unless otherwise indicated, conventional techniques of molecular biology (including recombinant techniques), microbiology, cell biology, and biochemistry, which are within the skill of the art. Such techniques are explained fully in the literature, such as, "Molecular Cloning: A Laboratory Manual", 2nd edition (Sambrook et al., 1989); "Oligonucleotide Synthesis" (M.J. Gait, ed., 1984); "Animal Cell Culture" (R.I. Freshney, ed., 1987); "Methods in Enzymology" (Academic Press, Inc.); "Handbook of Experimental Immunology", 4th edition (D.M. Weir & C.C. Blackwell, eds., Blackwell Science Inc., 1987); "Gene Transfer Vectors for Mammalian Cells" (J.M. Miller & M.P. Calos, eds., 1987); "Current Protocols in Molecular Biology" (F.M. Ausubel et al., eds., 1987); and "PCR: The Polymerase Chain Reaction", (Mullis et al., eds., 1994).

The terms "cancer" and "cancerous" refer to or describe the physiological condition in mammals that is typically characterized by unregulated cell growth. An example of a cancer is breast cancer.

The term "co-expressed" as used herein refers to a statistical correlation between the expression level of one sequence and the expression level of another sequence. Pairwise co-expression may be calculated by various methods known in the art, e.g., by calculating a Pearson correlation coefficient or Spearman correlation coefficient. Co-expressed gene cliques or gene networks may also be identified using a graph theory. An analysis of co-expression may be calculated using normalized data.

The term "correlates" or "correlating" as used herein refers to a statistical association between instances of two events, where events may include numbers, data sets, and the like. For example, when the events involve numbers, a positive correlation (also referred to herein as a "direct correlation") means that as one increases, the other increases as well. A negative correlation (also referred to herein as an "inverse correlation") means that as one increases, the other decreases. The present invention provides coding and non-coding RNA transcripts, or expression products thereof, the levels of which are correlated with a particular outcome measure, such as between the level of an RNA transcript and the likelihood of long-term survival without breast cancer recurrence. For example, the increased level of an RNA transcript may be positively correlated with a likelihood of a good clinical outcome for the patient, such as an increased likelihood of long-term survival without recurrence and/or a positive response to a chemotherapy, and the like. Such a positive correlation may be demonstrated statistically in various ways, e.g. by a low hazard ratio. In another example, the increased level of an RNA transcript may be negatively correlated with a likelihood of good clinical outcome for the patient. In this case, for example, the patient may have a decreased likelihood of long-term survival without recurrence of the cancer and/or a positive response to a chemotherapy, and the like. Such a negative correlation indicates that the patient likely has a poor prognosis or will respond poorly to a chemotherapy, and this may be demonstrated statistically in various ways, e.g., by a high hazard ratio.

As used herein, the term "exon" refers to any segment of an interrupted gene that is represented in the mature RNA product (B. Lewin. Genes IV Cell Press, Cambridge Mass. 1990). As used herein, the terms "intron" and "intronic sequence" refer to any non-coding region found within genes.

The term "expression product" as used herein refers to an expression product of a coding RNA transcript. Thus, the term refers to a polypeptide or protein.

As used herein, the term "intergenic region" refers to a stretch of DNA or RNA sequences located between clusters of genes that contain few or no genes. Intergenic regions are different from intragenic regions (or "introns"), which are non-coding regions that are found between exons within genes. An intergenic region may be comprised of one or more "intergenic sequences." As shown in the Examples below, 69 intergenic regions were found to correlate to long-term survival without breast cancer recurrence, and each intergenic region comprises one or more intergenic sequences. The intergenic sequences are readily available from publicly available information. For example, the UCSC Genome Browser available at http://genome.ucsc.edu/cgi-bin/hgGateway allows inputting of the coordinates, such as the chromosome number and the start/stop positions on the chromosome shown in Tables 4 and 5, to produce an output comprising that sequence.

As used herein, the terms "long intergenic non-coding RNAs" and "lincRNAs" are used interchangeably and refer to non-coding transcripts that are typically longer than 200 nucleotides. As shown in the Examples below, 22 lincRNAs were found to correlate to long-term survival without breast cancer recurrence. The coordinates of these lincRNAs are publicly available and are also listed in Table 4. The sequences of the lincRNAs may also be obtained from publicly available information, such as the UCSC Genome Browser discussed above.

As used herein, the term "level" as used herein refers to qualitative or quantitative determination of the number of copies of a coding or non-coding RNA transcript or a polypeptide/protein. An RNA transcript or a polypeptide/protein exhibits an "increased level" when the level of the RNA transcript or polypeptide/protein is higher in a first sample, such as in a clinically relevant subpopulation of patients (e.g., patients who have experienced cancer recurrence), than in a second sample, such as in a related subpopulation (e.g., patients who did not experience cancer recurrence). In the context of an analysis of a level of an RNA transcript or a polypeptide/protein in a tumor sample obtained from an individual patient, an RNA transcript or polypeptide/protein exhibits "increased level" when the level of the RNA transcript or polypeptide/protein in the subject trends toward, or more closely approximates, the level characteristic of a clinically relevant subpopulation of patients.

Thus, for example, when the RNA transcript analyzed is an RNA transcript that shows an increased level in subjects that experienced long-term survival without cancer recurrence as compared to subjects that did not experience long-term survival without cancer recurrence, then an "increased" level of a given RNA transcript can be described as being positively correlated with a likelihood of long-term survival without cancer recurrence. If the level of the RNA transcript in an individual patient being assessed trends toward a level characteristic of a subject who experienced long-term survival without cancer recurrence, the level of the RNA transcript supports a determination that the individual patient is more likely to experience long-term survival without cancer recurrence. If the level of the RNA transcript in the individual patient trends toward a level characteristic of a subject who experienced cancer recurrence, then the level of the RNA transcript supports a determination that the individual patient is more likely to experience cancer recurrence.

The term "likelihood score" is an arithmetically or mathematically calculated numerical value for aiding in simplifying or disclosing or informing the analysis of more complex quantitative information, such as the correlation of certain levels of the disclosed RNA transcripts, their expression products, or gene networks to a likelihood of a certain clinical outcome in a breast cancer patient, such as likelihood of long-term survival without breast cancer recurrence. A likelihood score may be determined by the application of a specific algorithm. The algorithm used to calculate the likelihood score may group the RNA transcripts, or their expression products, into gene networks. A likelihood score may be determined for a gene network by determining the level of one or more RNA transcripts, or an expression product thereof, and weighting their contributions to a certain clinical outcome such as recurrence. A likelihood score may also be determined for a patient. In an embodiment, a likelihood score is a recurrence score, wherein an increase in the recurrence score negatively correlates with an increased likelihood of long-term survival without breast cancer recurrence. In other words, an increase in the recurrence score correlates with bad prognosis. Examples of methods for determining the likelihood score or recurrence score are disclosed in U.S. Patent No. 7,526,387.

The term "long-term" survival as used herein refers to survival for at least 3 years. In other embodiments, it may refer to survival for at least 5 years, or for at least 10 years following surgery or other treatment.

As used herein, the term "normalized" with regard to a coding or non-coding RNA transcript, or an expression product of the coding RNA transcript, refers to the level of the RNA transcript, or its expression product, relative to the mean levels of transcript/product of a set of reference RNA transcripts, or their expression products. The reference RNA transcripts, or their expression products, are based on their minimal variation across patients, tissues, or treatments. Alternatively, the coding or non-coding RNA transcript, or its expression product, may be normalized to the totality of tested RNA transcripts, or a subset of such tested RNA transcripts.

As used herein, the term "pathology" of cancer includes all phenomena that comprise the well-being of the patient. This includes, without limitation, abnormal or uncontrollable cell growth, metastasis, interference with the normal functioning of neighboring cells, release of cytokines or other secretory products at abnormal levels, suppression or aggravation of inflammatory or immunological response, neoplasia, premalignancy, malignancy, invasion of surrounding or distant tissues or organs, such as lymph nodes.

A "patient response" may be assessed using any endpoint indicating a benefit to the patient, including, without limitation, (1) inhibition, to some extent, of tumor growth, including slowing down and complete growth arrest; (2) reduction in the number of tumor cells; (3) reduction in tumor size; (4) inhibition (i.e., reduction, slowing down or complete stopping) of tumor cell infiltration into adjacent peripheral organs and/or tissues; (5) inhibition (i.e. reduction, slowing down or complete stopping) of metastasis; (6) enhancement of anti-tumor immune response, which may, but does not have to, result in the regression or rejection of the tumor; (7) relief, to some extent, of one or more symptoms associated with the cancer; (8) increase in the length of survival following treatment; and/or (9) decreased mortality at a given point of time following treatment.

The term "prognosis" as used herein, refers to the prediction of the likelihood of cancer-attributable death or progression, including recurrence, metastatic spread, and drug resistance, of neoplastic disease, such as breast cancer. The term "prediction" is used herein to refer to the likelihood that a patient will respond either favorably or unfavorably to a drug or set of drugs, and also the extent of those responses, or that a patient will survive, following surgical removal of the primary tumor and/or chemotherapy for a certain period of time without cancer recurrence. The methods of the present invention can be used clinically to make treatment decisions by choosing the most appropriate treatment modalities for any particular patient. The methods of the present invention are tools in predicting if a patient is likely to respond favorably to a treatment regimen, such as surgical intervention, chemotherapy with a given drug or drug combination, and/or radiation therapy, or whether long-term survival of the patient without cancer recurrence is likely, following surgery and/or termination of chemotherapy or other treatment modalities.

The term "breast cancer prognostic biomarker" refers to an RNA transcript, or an expression product thereof, intronic RNA, lincRNA, intergenic sequence, and/or intergenic region found to be associated with long term survival without breast cancer recurrence as disclosed herein.

The term "reference" RNA transcript or an expression product thereof, as used herein, refers to an RNA transcript or an expression product thereof, whose level can be used to compare the level of an RNA transcript or its expression product in a test sample. In an embodiment of the invention, reference RNA transcripts include housekeeping genes, such as beta-globin, alcohol dehydrogenase, or any other RNA transcript, the level or expression of which does not vary depending on the disease status of the cell containing the RNA transcript or its expression product. In another embodiment, all of the assayed RNA transcripts, or their expression products, or a subset thereof, may serve as reference RNA transcripts or reference RNA expression products.

As used herein, the term "RefSeq RNA" refers to an RNA that can be found in the Reference Sequence (RefSeq) database, a collection of publicly available nucleotide sequences and their protein products built by the National Center for Biotechnology Information (NCBI). The RefSeq database provides an annotated, non-redundant record for each natural biological molecule (i.e. DNA, RNA or protein) included in the database. Thus, a sequence of a RefSeq RNA is well-known and can be found in the RefSeq database at http://www.ncbi.nlm.nih.gov/RefSeq/. *See also* Pruitt et al., Nucl. Acids Res. 33(Supp 1):D501-D504 (2005). Accession numbers for each RefSeq, which include accession numbers for any alternative splice forms, are provided in Tables 1 and 2 and in Table B. The intronic sequences for a RefSeq are also publicly available. Nonetheless, the coordinates for each intronic sequence listed in Table 3 are provided in Table A. Therefore, the sequence of each RNA sequence in Tables 1-3 and 15 are readily available from publicly available sources.

As used herein, the term "RNA transcript" refers to the RNA transcription product of DNA and includes coding and non-coding RNA transcripts. RNA transcripts include, for example, mRNA, an unspliced RNA, a splice variant mRNA, a microRNA, fragmented RNA, long intergenic non-coding RNAs (lincRNAs), intergenic RNA sequences or regions, and intronic RNAs.

The terms "subject," "individual," and "patient" are used interchangeably herein to refer to a mammal being assessed for treatment and/or being treated. In an embodiment, the mammal is a human. The terms "subject," "individual," and "patient" thus encompass individuals having cancer (e.g., breast cancer), including those who have undergone or are candidates for resection (surgery) to remove cancerous tissue.

As used herein, the term "surgery" applies to surgical methods undertaken for removal of cancerous tissue, including mastectomy, lumpectomy, lymph node removal, sentinel lymph node dissection, prophylactic mastectomy, prophylactic ovary removal, cryotherapy, and tumor biopsy. The tumor samples used for the methods of the present invention may have been obtained from any of these methods.

The term "tumor" as used herein, refers to all neoplastic cell growth and proliferation, whether malignant or benign, and all pre-cancerous and cancerous cells and tissues.:

The term "tumor sample" as used herein refers to a sample comprising tumor material obtained from a cancer patient. The term encompasses tumor tissue samples, for example, tissue obtained by surgical resection and tissue obtained by biopsy, such as for example, a core biopsy or a fine needle biopsy. In a particular embodiment, the tumor sample is a fixed, wax-embedded tissue sample, such as a formalin-fixed, paraffin-embedded tissue sample. Additionally, the term "tumor sample" encompasses a sample comprising tumor cells obtained from sites other than the primary tumor, e.g., circulating tumor cells. The term also encompasses cells that are the progeny of the patient's tumor cells, e.g. cell culture samples derived from primary tumor cells or circulating tumor cells. The term further encompasses samples that may comprise protein or nucleic acid material shed from tumor cells *in vivo*, e.g., bone marrow, blood, plasma, serum, and the like. The term also encompasses samples that have been enriched for tumor cells or otherwise manipulated after their procurement and samples comprising polynucleotides and/or polypeptides that are obtained from a patient's tumor material.

As used herein, "whole transcriptome sequencing" refers to the use of high throughput sequencing technologies to sequence the entire transcriptome in order to get information about a sample's RNA content. Whole transcriptome sequencing can be done with a variety of platforms for example, the Genome Analyzer (Illumina, Inc., San Diego, CA) and the SOLiD™ Sequencing System (Life Tehcnologies, Carlsbad, CA). However, any platform useful for whole transcriptome sequencing may be used.

The term "RNA-Seq" or "transcriptome sequencing" refers to sequencing performed on RNA (or cDNA) instead of DNA, where typically, the primary goal is to measure expression levels, detect fusion transcripts, alternative splicing, and other genomic alterations that can be better assessed from RNA. RNA-Seq includes whole transcriptome sequencing as well as target specific sequencing.

The term "computer-based system," as used herein, refers to the hardware means, software means, and data storage means used to analyze information. The minimum hardware of a patient computer-based system comprises a central processing unit (CPU), input means, output means, and data storage means. A skilled artisan can readily appreciate that many of the currently available computer-based system are suitable for use in the present invention and may be programmed to perform the specific measurement and/or calculation functions of the present invention.

To "record" data, programming or other information on a computer readable medium refers to a process for storing information, using any such methods as known in the art. Any convenient data storage structure may be chosen, based on the means used to access the stored information. A variety of data processor programs and formats can be used for storage, e.g. word processing text file, database format, etc.

A "processor" or "computing means" references any hardware and/or software combination that will perform the functions required of it. For example, any processor herein may be a programmable digital microprocessor such as available in the form of an electronic controller, mainframe, server or personal computer (desktop or portable). Where the processor is programmable, suitable programming can be communicated from a remote location to the processor, or previously saved in a computer program product (such as a portable or fixed computer readable storage medium, whether magnetic, optical or solid state device based). For example, a magnetic medium or optical disk may carry the programming, and can be read by a suitable reader communicating with each processor at its corresponding station.

The present invention provides RNA transcripts that are prognostic for breast cancer. These RNA transcripts are listed in Tables 1-5 and 15 and include coding and non-coding RNA transcripts. A subset of the RNA transcripts of Table 1 may be further grouped into gene networks, depending on their known function. For example, the gene networks may include a cell cycle network, ESR1 network, Chr9q22 network, Chr17q23-24 network, Chr8q21-24 network, olfactory receptor network, and metabolic-like networks. The cell cycle network comprises the genes listed in Table 6. The ESR1 network comprises BCL2, SCUBE2, CPEB2, IL6ST, DNALI1, PGR, SLC7A8, C6orf97, RSPH1, EVL, BCL2, NXNL2, GATA3, GFRA1, GFRA1, ZNF740, MKL2, AFF3, ERBB4, RABEP1, KDM4B, ESR1, C4orf32, and CPLX1 as shown in Table 7. The Chr9q22 network comprises ASPN, CENPP, ECM2, OGN, and OMD as shown in Table 8. The Chr17q23-24 network comprises CCDC45, POLG2, SMURF2, CCDC47, CLTC, DCAF7, DDX42, FTSJ3, PSMC5, RPS6KB1, SMARCD2, and TEX2 as shown in Table 9. The Chr8q21-24 network comprises CYC1, DGAT1, GPAA1, GRINA, PUF60, PYCRL, RPL8, SQLE, TSTA3, ESRP1, GRHL2, INTS8, MTDH, and UQCRB as shown in Table 10. The olfactory receptor network comprises 134 genes listed in Table 11. OR10H3, OR14J1, OR2J2, OR2W5, OR5T2, OR7E24, OR7G3, OR8S1, and OR9K2 code for olfactory receptors, and MIR1208, MIR1266, MIR1297, MIR133A1, MIR195, MIR196A1, MIR3170, MIR3183, MIR4267, MIR4275, MIR4318, MIR501, MIR501, MIR539, and MIR542 are microRNA precursors. The metabolic-like network comprises a five gene set of ENO1, IDH2, TMSB10, PGK1, and G6PD, or a fourteen gene set of PGD, TKT, TALDO1, G6PD, GP1, SLC1A5, SLC7A5, OGDH, SUCLG1, ENO1, PGK1, IDH2, ACO2, and FBP1. An RNA transcript, or an expression product thereof, is negatively correlated with an increased likelihood of long-term survival without recurrence of breast cancer if the direction of association of the RNA transcript is marked 1 in Tables 1-5 and 15, and is positively correlated with an increased likelihood of long-term survival without recurrence of breast cancer if the direction of association of the RNA transcript is marked -1 in Tables 1-5 and 15. Co-expressed RNA transcripts within a gene network may be substituted for other the RNA transcripts within the same gene network.

The present invention provides methods that utilize the RNA transcripts and associated information. For example, the present invention provides a method of predicting a likelihood that a breast cancer patient will exhibit long-term survival without breast cancer recurrence. The methods of the invention comprise determining the level of at least one RNA transcript, or an expression product thereof, in a tumor sample, and determining the likelihood of long-term survival without breast cancer recurrence based on the correlation between the level of the RNA transcript, or its expression product, and long-term survival without breast cancer recurrence.

For all aspects of the present invention, the methods may further include determining the level of at least two RNA transcripts, or their expression products. It is further contemplated that the methods of the present invention may further include determining the level of at least three, four, five, six, seven, eight, nine, ten, eleven, twelve, thirteen, fourteen, or at least fifteen of the RNA transcripts, or their expression products. For example, the levels of at least three RNA transcripts, or their expression products, selected from ENO1, IDH2, TMSB10, PGK1, and G6PD may be determined. In another aspect, the levels of all five of ENO1, IDH2, TMSB10, PGK1, and G6PD RNA transcripts, or their expression products, may be determined. In another example, at least five RNA transcripts, or expression products thereof, selected from PGD, TKT, TALDO1, G6PD, GP1, SLC1A5, SLC7A5, OGDH, SUCLG1, ENO1, PGK1, IDH2, ACO2, and FBP1 may be determined. In yet another example, the levels of all fourteen of PGD, TKT, TALDO1, G6PD, GP1, SLC1A5, SLC7A5, OGDH, SUCLG1, ENO1, PGK1, IDH2, ACO2, and FBP1 may be determined. Coding and non-coding RNA transcripts may be combined in any of the methods described herein.

The RNA transcripts and associated information provided by the present invention also have utility in the development of therapies to treat cancers and screening patients for inclusion in clinical trials. The RNA transcripts and associated information may further be used to design or produce a reagent that modulates the level or activity of the RNA transcript or its expression product. Such reagents may include, but are not limited to, a drug, an antisense RNA, a small inhibitory RNA (siRNA), a ribozyme, a small molecule, a monoclonal antibody, and a polyclonal antibody.

In various embodiments of the methods of the present invention, various technological approaches are available for determining the levels of the RNA transcripts, including, without limitation, whole transcriptome sequencing, RT-PCR, microarrays, and serial analysis of gene expression (SAGE), which are described in more detail below.

### CORRELATING LEVEL OF AN RNA TRANSCRIPT OR AN EXPRESSION PRODUCT TO A CLINICAL OUTCOME

One skilled in the art will recognize that there are many statistical methods that may be used to determine whether there is a correlation between an outcome of interest (e.g., likelihood of survival) and levels of RNA transcripts or their expression products as described here. This relationship can be presented as a continuous recurrence score (RS), or patients may be stratified into risk groups (e.g., low, intermediate, high). For example, a Cox proportional hazards regression model may fit to a particular clinical endpoint (e.g., RFI, DFS, OS). One assumption of the Cox proportional hazards regression model is the proportional hazards assumption, i.e. the assumption that effect parameters multiply the underlying hazard. Assessments of model adequacy may be performed including, but not limited to, examination of the cumulative sum of martingale residuals. One skilled in the art would recognize that there are numerous statistical methods that may be used (e.g., Royston and Parmer (2002), smoothing spline, etc.) to fit a flexible parametric model using the hazard scale and the Weibull distribution with natural spline smoothing of the log cumulative hazards function, with effects for treatment (chemotherapy or observation) and RS allowed to be time-dependent. (*See, e.g.,* P. Royston, M. Parmer, Statistics in Medicine 21(15:2175-2197 (2002).)

In an exemplary embodiment, power calculations are carried out for the Cox proportional hazards model with a single non-binary covariate using the method proposed by F. Hsieh and P. Lavori, Control Clin Trials 21:552-560 (2000) as implemented in PASS 2008.

Any of the methods described may group the levels of RNA transcripts or their expression products. The grouping of the RNA transcripts or expression products may be performed at least in part based on knowledge of the contribution of the RNA transcripts or their expression products according to physiologic functions or component cellular characteristics, such as in the gene networks described herein. The formation of groups, in addition, can facilitate the mathematical weighting of the contribution of various expression levels to the recurrence/likelihood score. The weighting of a gene network representing a physiological process or component cellular characteristic can reflect the contribution of that process or characteristic to the pathology of the cancer and clinical outcome. Accordingly, the present invention provides gene networks of the RNA transcripts, or their expression products, identified herein for use in the methods disclosed herein.

The coding and non-coding RNA transcripts, and any expression products thereof, of the present invention are listed in Tables 1-5 and 15. In an embodiment of the invention, a level of one or more RNA transcripts, or an expression product thereof, listed in Tables 1 and 15, is negatively correlated with an increased likelihood of long-term survival without recurrence of breast cancer if the direction of association of the RNA transcript is marked 1 in Tables 1 and 15, and is positively correlated with an increased likelihood of long-term survival without recurrence of breast cancer if the direction of association of the RNA transcript is marked -1 in Tables 1 and 15.

In another embodiment of the invention, a level of one or more RNA transcript, or an expression product thereof, listed in Table 2, is negatively correlated with an increased likelihood of long-term survival without recurrence of breast cancer in an ER-positive breast cancer patient if the direction of association of the RNA transcript is marked 1 in Table 2, and is positively correlated with an increased likelihood of long-term survival without recurrence of breast cancer in an ER-positive breast cancer patient if the direction of association of the RNA transcript is marked -1 in Table 2.

In a further embodiment of the invention, a level of an intronic RNA selected from Table 3 is negatively correlated with an increased likelihood of long-term survival without recurrence of breast cancer if the direction of association of the intronic RNA is marked 1 in Table 3, and is positively correlated with an increased likelihood of long-term survival without recurrence of breast cancer if the direction of association of the intronic RNA is marked -1 in Table 3.

In a specific embodiment, a level of one or more long intergenic non-coding region (lincRNA) selected from Table 4 is negatively correlated with an increased likelihood of long-term survival without recurrence of breast cancer if the direction of association of the lincRNA is marked 1 in Table 4, and is positively correlated with an increased likelihood of long-term survival without recurrence of breast cancer if the direction of association of the lincRNA is marked -1 in Table 4.

In another embodiment, a level of one or more intergenic sequence or intergenic region selected from intergenic regions 1-69 listed in Table 5 is negatively correlated with an increased likelihood of long-term survival without recurrence of breast cancer if the direction of association of the intergenic sequence or intergenic region is marked 1 in Table 5, and is positively correlated with an increased likelihood of long-term survival without recurrence of breast cancer if the direction of association of the intergenic sequence or intergenic region is marked -1 in Table 5.

In yet another embodiment, a likelihood score is determined for assessing the likelihood of a certain clinical outcome in a breast cancer patient, such as likelihood of long-term survival without breast cancer recurrence. A likelihood score may be calculated by determining the level of one or more RNA transcripts, or its expression product, selected from Tables 1-5 and 15, and mathematically weighting its contribution to the clinical outcome. In a specific embodiment, a likelihood score is determined for a gene network selected from a cell cycle network, ESR1 network, Chr9q22 network, Chr17q23-24 network, Chr8q21-24 network, olfactory receptor network, and metabolic-like networks by determining the level of one or more RNA transcripts, or an expression product thereof, within a gene network. The level of the one or more RNA transcripts, or its expression product, may be weighted by its contribution to a certain clinical outcome, such as recurrence. A likelihood score may also be determined for a gene network based on the likelihood score of one or more RNA transcripts, or an expression product thereof, within the gene network. In another embodiment, a likelihood score may be determined for a patient, based on the likelihood score of one or more RNA transcripts, or an expression product thereof, and/or the likelihood score of one or more gene networks.

### METHODS TO PREDICT LIKELIHOOD OF LONG-TERM SURVIVAL WITHOUT BREAST CANCER RECURRENCE

As described above, a number of coding and non-coding RNA transcripts that correlate with breast cancer prognosis were identified. The levels of these RNA transcripts, or their expression products, can be determined in a tumor sample obtained from an individual patient who has breast cancer and for whom treatment is being contemplated. Depending on the outcome of the assessment, treatment with chemotherapy may be indicated, or an alternative treatment regimen may be indicated.

In carrying out the method of the present invention, a tumor sample is assayed or measured for a level of an RNA transcript, or its expression product. The tumor sample can be obtained from a solid tumor, e.g., via biopsy, or from a surgical procedure carried out to remove a tumor; or from a tissue or bodily fluid that contains cancer cells. In an embodiment of the invention, the tumor sample is obtained from a patient with breast cancer, such as ER-positive breast cancer. In another embodiment, the level of an RNA transcript, or its expression product, is normalized relative to the level of one or more reference RNA transcripts, or its expression product.

In an embodiment of the invention, the likelihood of long-term survival without breast cancer recurrence in an individual patient is predicted by comparing, directly or indirectly, the level or normalized level of the RNA transcript, or its expression product, in the tumor sample from the individual patient to the level or normalized level of the RNA transcript, or its expression product, in a clinically relevant subpopulation of patients. Thus, as explained above, when the RNA transcript, or its expression product, analyzed is an RNA transcript, or an expression product, that shows increased level in subjects that experienced long-term survival without breast cancer recurrence as compared to subjects that experienced breast cancer recurrence, then if the level of the RNA transcript, or its expression product in an individual patient being assessed trends toward a level characteristic of a subject with long-term survival without breast cancer recurrence, then the RNA transcript or its expression product level supports a determination that the individual patient is more likely to experience long-term survival without breast cancer recurrence. Similarly, where the RNA transcript or its expression product analyzed is an RNA transcript or expression product that is increased in subjects who have experienced breast cancer recurrence as compared subjects who have experienced long-term survival without breast cancer recurrence, then if the level of the RNA transcript, or its expression product, in an individual patient being assessed trends toward a level characteristic of a subject with breast cancer recurrence, then RNA transcript or expression product level supports a determination that the individual patient will more likely experience breast cancer recurrence. Thus, the level of a given RNA transcript, or its expression product, can be described as being positively correlated with a likelihood of long-term survival without breast cancer recurrence, or as being negatively correlated with a likelihood of long-term survival without breast cancer recurrence.

It is understood that the level or normalized level of an RNA transcript, or its expression product, from an individual patient can be compared, directly or indirectly, to the level or normalized level of the RNA transcript, or its expression product, in a clinically relevant subpopulation of patients. For example, when compared indirectly, the level or normalized level of the RNA transcript, or its expression product, from the individual patient may be used to calculate a likelihood of long-term survival without breast cancer recurrence, such as a likelihood/recurrence score (RS) as described above, and compared to a calculated score in the clinically relevant subpopulation of patients.

### METHODS OF ASSAYING LEVELS OF RNA TRANSCRIPTS OR THEIR EXPRESSION PRODUCTS

Methods of expression profiling include methods based on sequencing of polynucleotides, methods based on hybridization analysis of polynucleotides, and proteomics-based methods. Representative methods for sequencing-based analysis include Massively Parallel Sequencing (*see e.g*., Tucker et al., The American J. Human Genetics 85:142-154, 2009) and Serial Analysis of Gene Expression (SAGE). Exemplary methods known in the art for the quantification of mRNA expression in a sample include northern blotting and *in situ* hybridization (Parker & Barnes, Methods in Molecular Biology 106:247-283 (1999)); RNAse protection assays (Hod, Biotechniques 13:852-854 (1992)); and PCR-based methods, such as reverse transcription polymerase chain reaction (RT-PCR) (Weis et al., Trends in Genetics 8:263-264 (1992)). Antibodies may be employed that can recognize sequence-specific duplexes, including DNA duplexes, RNA duplexes, and DNA-RNA hybrid duplexes or DNA-protein duplexes.

### Nucleic Acid Sequencing-Based Methods

Nucleic acid sequencing technologies are suitable methods for expression analysis. The principle underlying these methods is that the number of times a cDNA sequence is detected in a sample is directly related to the relative RNA levels corresponding to that sequence. These methods are sometimes referred to by the term Digital Gene Expression (DGE) to reflect the discrete numeric property of the resulting data. Early methods applying this principle were Serial Analysis of Gene Expression (SAGE) and Massively Parallel Signature Sequencing (MPSS). *See, e.g.,* S. Brenner, et al., Nature Biotechnology 18(6):630-634 (2000).

More recently, the advent of "next-generation" sequencing technologies has made DGE simpler, higher throughput, and more affordable. As a result, more laboratories are able to utilize DGE to screen the expression of more nucleic acids in more individual patient samples than previously possible. *See, e.g.,* J. Marioni, Genome Research 18(9):1509-1517 (2008); R. Morin, Genome Research 18(4):610-621 (2008); A. Mortazavi, Nature Methods 5(7):621-628 (2008); N. Cloonan, Nature Methods 5(7):613-619 (2008). Massively parallel sequencing methods have also enabled whole genome or transcriptome sequencing, allowing the analysis of not only coding but also non-coding sequencees. As reviewed in Tucker et al., The American J. Human Genetics 85:142-154 (2009), there are several commercially available massively parallel sequencing platforms, such as the Illumina Genome Analyzer (Illumina, Inc., San Diego, CA), Applied Biosystems SOLiD™ Sequencer (Life Technologies, Carlsbad, CA), Roche GS-FLX 454 Genome Sequencer (Roche Applied Science, Germany), and the Helicos® Genetic Analysis Platform (Helicos Biosciences Corp., Cambridge, MA). Other developing technologies may be used.

### Reverse Transcription PCR (RT-PCR)

The starting material is typically total RNA isolated from a human tumor, usually from a primary tumor. Optionally, normal tissues from the same patient can be used as an internal control. RNA can be extracted from a tissue sample, e.g., from a sample that is fresh, frozen (e.g. fresh frozen), or paraffin-embedded and fixed (e.g. formalin-fixed).

General methods for RNA extraction are well known in the art and are disclosed in standard textbooks of molecular biology, including Ausubel et al., Current Protocols of Molecular Biology, John Wiley and Sons (1997). Methods for RNA extraction from paraffin embedded tissues are disclosed, for example, in Rupp and Locker, Lab Invest. 56:A67 (1987), and De Andrés et al., BioTechniques 18:42044 (1995). In particular, RNA isolation can be performed using a purification kit, buffer set and protease from commercial manufacturers, such as Qiagen, according to the manufacturer's instructions. For example, total RNA from cells in culture can be isolated using Qiagen RNeasy mini-columns. Other commercially available RNA isolation kits include MasterPure™ Complete DNA and RNA Purification Kit (EPICENTRE®, Madison, WI), and Paraffin Block RNA Isolation Kit (Ambion, Inc.). Total RNA from tissue samples can be isolated using RNA Stat-60 (Tel-Test). RNA prepared from a tumor sample can be isolated, for example, by cesium chloride density gradient centrifugation. The isolated RNA may then be depleted of ribosomal RNA as described in U.S. Pub. No. 2011/0111409.

The sample containing the RNA is then subjected to reverse transcription to produce cDNA from the RNA template, followed by exponential amplification in a PCR reaction. The two most commonly used reverse transcriptases are avian myeloblastosis virus reverse transcriptase (AMV-RT) and Moloney murine leukemia virus reverse transcriptase (MMLV-RT). The reverse transcription step is typically primed using specific primers, random hexamers, or oligo-dT primers, depending on the circumstances and the goal of expression profiling. For example, extracted RNA can be reverse-transcribed using a GeneAmp RNA PCR kit (Perkin Elmer, CA, USA), following the manufacturer's instructions. The derived cDNA can then be used as a template in the subsequent PCR reaction.

PCR-based methods use a thermostable DNA-dependent DNA polymerase, such as a Taq DNA polymerase. For example, TaqMan® PCR typically utilizes the 5'-nuclease activity of Taq or Tth polymerase to hydrolyze a hybridization probe bound to its target amplicon, but any enzyme with equivalent 5' nuclease activity can be used. Two oligonucleotide primers are used to generate an amplicon typical of a PCR reaction product. A third oligonucleotide, or probe, can be designed to facilitate detection of a nucleotide sequence of the amplicon located between the hybridization sites of the two PCR primers. The probe can be detectably labeled, e.g., with a reporter dye, and can further be provided with both a fluorescent dye, and a quencher fluorescent dye, as in a Taqman® probe configuration. Where a Taqman® probe is used, during the amplification reaction, the Taq DNA polymerase enzyme cleaves the probe in a template-dependent manner. The resultant probe fragments disassociate in solution, and signal from the released reporter dye is free from the quenching effect of the second fluorophore. One molecule of reporter dye is liberated for each new molecule synthesized, and detection of the unquenched reporter dye provides the basis for quantitative interpretation of the data.

TaqMan® RT-PCR can be performed using commercially available equipment, such as, for example, ABI PRISM 7700™ Sequence Detection System™ (Perkin-Elmer-Applied Biosystems, Foster City, CA, USA), or Lightcycler (Roche Molecular Biochemicals, Mannheim, Germany). In a preferred embodiment, the 5' nuclease procedure is run on a real-time quantitative PCR device such as the ABI PRISM 7700™ Sequence Detection System™. The system consists of a thermocycler, laser, charge-coupled device (CCD), camera and computer. The system amplifies samples in a 384-well format on a thermocycler. The RT-PCR may be performed in triplicate wells with an equivalent of 2ng RNA input per 10 µL-reaction volume. During amplification, laser-induced fluorescent signal is collected in real-time through fiber optics cables for all wells, and detected at the CCD. The system includes software for running the instrument and for analyzing the data.

5'-Nuclease assay data are generally initially expressed as a threshold cycle ("Cₜ"). Fluorescence values are recorded during every cycle and represent the amount of product amplified to that point in the amplification reaction. The threshold cycle (Cₜ) is generally described as the point when the fluorescent signal is first recorded as statistically significant.

To minimize errors and the effect of sample-to-sample variation, RT-PCR is usually performed using an internal standard. The ideal internal standard gene (also referred to as a reference gene) is expressed at a constant level among cancerous and non-cancerous tissue of the same origin (i.e., a level that is not significantly different among normal and cancerous tissues), and is not significantly affected by the experimental treatment (i.e., does not exhibit a significant difference in expression level in the relevant tissue as a result of exposure to chemotherapy). RNAs most frequently used to normalize patterns of gene expression are mRNAs for the housekeeping genes glyceraldehyde-3-phosphate-dehydrogenase (GAPDH) and β-actin. Gene expression measurements can be normalized relative to the mean of one or more (e.g., 2, 3, 4, 5, or more) reference genes. Reference-normalized expression measurements can range from 0 to 15, where a one unit increase generally reflects a 2-fold increase in RNA quantity.

Real time PCR is compatible both with quantitative competitive PCR, where an internal competitor for each target sequence is used for normalization, and with quantitative comparative PCR using a normalization gene contained within the sample, or a housekeeping gene for RT-PCR. For further details see, e.g. Held et al., Genome Research 6:986-994 (1996).

### Design of PCR Primers and Probes

PCR primers and probes can be designed based upon exon, intron, or intergenic sequences present in the RNA transcript of interest. Primer/probe design can be performed using publicly available software, such as the DNA BLAT software developed by Kent, W.J., Genome Res. 12(4):656-64 (2002), or by the BLAST software including its variations.

Where necessary or desired, repetitive sequences of the target sequence can be masked to mitigate non-specific signals. Exemplary tools to accomplish this include the Repeat Masker program available on-line through the Baylor College of Medicine, which screens DNA sequences against a library of repetitive elements and returns a query sequence in which the repetitive elements are masked. The masked sequences can then be used to design primer and probe sequences using any commercially or otherwise publicly available primer/probe design packages, such as Primer Express (Applied Biosystems); MGB assay-by-design (Applied Biosystems); Primer3 (Steve Rozen and Helen J. Skaletsky (2000) Primer3 on the WWW for general users and for biologist programmers. In: Rrawetz S, Misener S (eds) Bioinformatics Methods and Protocols: Methods in Molecular Biology. Humana Press, Totowa, NJ, pp 365-386).

Other factors that can influence PCR primer design include primer length, melting temperature (Tm), and G/C content, specificity, complementary primer sequences, and 3 '-end sequence. In general, optimal PCR primers are generally 17-30 bases in length, and contain about 20-80%, such as, for example, about 50-60% G+C bases, and exhibit Tm's between 50 and 80 °C, e.g. about 50 to 70 °C.

For further guidelines for PCR primer and probe design *see, e.g.* Dieffenbach, CW. et al, "General Concepts for PCR Primer Design" in: PCR Primer, A Laboratory Manual, Cold Spring Harbor Laboratory Press, New York, 1995, pp. 133-155; Innis and Gelfand, "Optimization of PCRs" in: PCR Protocols, A Guide to Methods and Applications, CRC Press, London, 1994, pp. 5-11; and Plasterer, T.N. Primerselect: Primer and probe design. Methods Mol. Biol. 70:520-527 (1997).

### MassARRAY® System

In MassARRAY-based methods, such as the exemplary method developed by Sequenom, Inc. (San Diego, CA) following the isolation of RNA and reverse transcription, the obtained cDNA is spiked with a synthetic DNA molecule (competitor), which matches the targeted cDNA region in all positions, except a single base, and serves as an internal standard. The cDNA/competitor mixture is PCR amplified and is subjected to a post-PCR shrimp alkaline phosphatase (SAP) enzyme treatment, which results in the dephosphorylation of the remaining nucleotides. After inactivation of the alkaline phosphatase, the PCR products from the competitor and cDNA are subjected to primer extension, which generates distinct mass signals for the competitor- and cDNA-derived PCR products. After purification, these products are dispensed on a chip array, which is pre-loaded with components needed for analysis with matrix- assisted laser desorption ionization time-of-flight mass spectrometry (MALDI-TOF MS) analysis. The cDNA present in the reaction is then quantified by analyzing the ratios of the peak areas in the mass spectrum generated. For further details see, e.g. Ding and Cantor, Proc. Natl. Acad Sci. USA 100:3059-3064 (2003).

### Other PCR-based Methods

Further PCR-based techniques that can find use in the methods disclosed herein include, for example, BeadArray® technology (Illumina, San Diego, CA; Oliphant et al., Discovery of Markers for Disease (Supplement to Biotechniques), June 2002; Ferguson et al., Analytical Chemistry 72:5618 (2000)); BeadsArray for Detection of Gene Expression® (BADGE), using the commercially available LuminexlOO LabMAP® system and multiple color-coded microspheres (Luminex Corp., Austin, TX) in a rapid assay for gene expression (Yang et al., Genome Res. 11:1888-1898 (2001)); and high coverage expression profiling (HiCEP) analysis (Fukumura et al., Nucl. Acids. Res. 31(16) e94 (2003).

### Microarrays

In this method, polynucleotide sequences of interest (including cDNAs and oligonucleotides) are arrayed on a substrate. The arrayed sequences are then contacted under conditions suitable for specific hybridization with detectably labeled cDNA generated from RNA of a sample. The source of RNA typically is total RNA isolated from a tumor sample, and optionally from normal tissue of the same patient as an internal control or cell lines. RNA can be extracted, for example, from frozen or archived paraffin-embedded and fixed (e.g. formalin-fixed) tissue samples.

For example, PCR amplified inserts of cDNA clones of a gene to be assayed are applied to a substrate in a dense array. Usually at least 10,000 nucleotide sequences are applied to the substrate. For example, the microarrayed genes, immobilized on the microchip at 10,000 elements each, are suitable for hybridization under stringent conditions. Fluorescently labeled cDNA probes may be generated through incorporation of fluorescent nucleotides by reverse transcription of RNA extracted from tissues of interest. Labeled cDNA probes applied to the chip hybridize with specificity to each spot of DNA on the array. After washing under stringent conditions to remove non-specifically bound probes, the chip is scanned by confocal laser microscopy or by another detection method, such as a CCD camera. Quantitation of hybridization of each arrayed element allows for assessment of corresponding mRNA abundance.

With dual color fluorescence, separately labeled cDNA probes generated from two sources of RNA are hybridized pair wise to the array. The relative abundance of the transcripts from the two sources corresponding to each specified gene is thus determined simultaneously. The miniaturized scale of the hybridization affords a convenient and rapid evaluation of the expression pattern for large numbers of genes. Such methods have been shown to have the sensitivity required to detect rare transcripts, which are expressed at a few copies per cell, and to reproducibly detect at least approximately two-fold differences in the expression levels (Schena et at, Proc. Natl. Acad. Sci. USA 93(2): 106-149 (1996)). Microarray analysis can be performed on commercially available equipment, following the manufacturer's protocols, such as by using the Affymetrix GenChip® technology, or Incyte's microarray technology.

### Isolating RNA from Body Fluids

Methods of isolating RNA for expression analysis from blood, plasma and serum (*see* for example, Tsui NB et al. (2002) Clin. Chem. 48,1647-53 and references cited therein) and from urine (*see* for example, Boom R et al. (1990) J Clin Microbiol. 28, 495-503 and reference cited therein) have been described.

### Immunohistochemistry

Immunohistochemistry methods are also suitable for detecting the expression levels of genes and applied to the method disclosed herein. Antibodies (e.g., monoclonal antibodies) that specifically bind a gene product of a gene of interest can be used in such methods. The antibodies can be detected by direct labeling of the antibodies themselves, for example, with radioactive labels, fluorescent labels, hapten labels such as biotin, or an enzyme such as horse radish peroxidase or alkaline phosphatase. Alternatively, unlabeled primary antibody can be used in conjunction with a labeled secondary antibody specific for the primary antibody. Immunohistochemistry protocols and kits are well known in the art and are commercially available.

### Proteomics

The term "proteome" is defined as the totality of the proteins present in a sample (e.g. tissue, organism, or cell culture) at a certain point of time. Proteomics includes, among other things, study of the global changes of protein expression in a sample (also referred to as "expression proteomics"). Proteomics typically includes the following steps: (1) separation of individual proteins in a sample by 2-D gel electrophoresis (2-D PAGE); (2) identification of the individual proteins recovered from the gel, e.g. my mass spectrometry or N- terminal sequencing, and (3) analysis of the data using bioinformatics.

### GENERAL DESCRIPTION OF THE RNA ISOLATION AND PREPARATION FROM FIXED, PARAFFIN-EMBEDDED SAMPLES FOR WHOLE TRANSCRIPTOME SEQUENCING

The steps of a representative protocol for profiling gene expression levels using fixed, paraffin-embedded tissues as the RNA source are provided in various published journal articles. (*See, e.g.,* T.E. Godfrey et al,. J. Molec. Diagnostics 2: 84-91 (2000); K. Specht et al., Am. J. Pathol. 158: 419-29 (2001), M. Cronin, et al., Am J Pathol 164:35-42 (2004)). Modified methods can used for whole transcriptome sequencing as described in the Examples section. Briefly, a representative process starts with cutting a tissue sample section (e.g. about 10 µm thick sections of a paraffin-embedded tumor tissue sample). The RNA is then extracted, and ribosomal RNA may be deleted as described in U.S. Pub. No. 2011/0111409. cDNA sequencing libraries may be prepared that are directional and single or paired-end using commercially available kits such as the ScriptSeq™ mRNA-Seq Library Preparation Kit (Epicenter Biotechnologies, Madison, WI). The libraries may also be barcoded for multiplex sequencing using commercially available barcode primers such as the RNA-Seq Barcode Primers from Epicenter Biotechnologies (Madison, WI). PCR is then carried out to generate the second strand of cDNA to incorporate the barcodes and to amplify the libraries. After the libraries are quantified, the sequencing libraries may be sequenced as described herein.

### COEXPRESSION ANALYSIS

To perform particular biological processes, genes often work together in a concerted way, i.e. they are co-expressed. Co-expressed gene networks identified for a disease process like cancer can also serve as prognostic biomarkers. Such co-expressed genes can be assayed in lieu of, or in addition to, assaying the biomarker with which they co-express.

One skilled in the art will recognize that many co-expression analysis methods now known or later developed will fall within the scope and spirit of the present invention. These methods may incorporate, for example, correlation coefficients, co-expression network analysis, clique analysis, etc., and may be based on expression data from RT-PCR, microarrays, sequencing, and other similar technologies. For example, gene expression clusters can be identified using pairwise analysis of correlation based on Pearson or Spearman correlation coefficients. (*See e.g,* Pearson K. and Lee A., Biometrika 2:357 (1902); C. Spearman, Amer. J. Psychol. 15:72-101 (1904); J. Myers, A. Well, Research Design and Statistical Analysis, p. 508 (2nd Ed., 2003).) In general, a correlation coefficient of equal to or greater than 0.3 is considered to be statistically significant in a sample size of at least 20. (*See e.g.,* G. Norman, D. Streiner, Biostatistics: The Bare Essentials, 137-138 (3rd Ed. 2007).)

### REFERENCE NORMALIZATION

In order to minimize expression measurement variations due to non-biological variations in samples, e.g., the amount and quality of product to be measured, the level of an RNA transcript or its expression product may be normalized relative to the mean levels obtained for one or more reference RNA transcripts or their expression products. Examples of reference RNA transcripts or expression products include housekeeping genes, such as GAPDH. Alternatively, all of the assayed RNA transcripts or expression products, or a subset thereof, may also serve as reference. On a transcript (or protein)-by-transcript (or protein) basis, measured normalized amount of a patient tumor RNA or protein may be compared to the amount found in a cancer tissue reference set. *See e.g.,* Cronin, M. et al., Am. Soc. Investigative Pathology 164:35-42 (2004). The normalization may be carried out such that a one unit increase in normalized level of an RNA transcript or expression product generally reflects a 2-fold increase in quantity present in the sample.

### KITS OF THE INVENTION

The materials for use in the methods of the present invention are suited for preparation of kits produced in accordance with well known procedures. The present invention thus provides kits comprising agents, which may include primers and/or probes, for quantitating the level of the disclosed RNA transcripts or their expression products via methods such as whole transcriptome sequencing or RT-PCR for predicting prognostic outcome. Such kits may optionally contain reagents for the extraction of RNA from tumor samples, in particular, fixed paraffin-embedded tissue samples and/or reagents for whole transcriptome sequencing. In addition, the kits may optionally comprise the reagent(s) with an identifying description or label or instructions relating to their use in the methods of the present invention. The kits may comprise containers (including microliter plates suitable for use in an automated implementation of the method), each with one or more of the various reagents (typically in concentrated form) utilized in the methods, including, for example, pre-fabricated microarrays, buffers, the appropriate nucleotide triphosphates (e.g., dATP, dCTP, dGTP and dTTP; or rATP, rCTP, rGTP and UTP), reverse transcriptase, DNA polymerase, RNA polymerase, and one or more probes and primers of the present invention (e.g., appropriate length poly(T) or random primers linked to a promoter reactive with the RNA polymerase). Mathematical algorithms used to estimate or quantify prognostic information are also potential components of kits.

### REPORTS

The methods of this invention are suited for the preparation of reports summarizing the predictions resulting from the methods of the present invention. A "report" as described herein, is an electronic or tangible document that includes elements that provide information of interest relating to a likelihood assessment and its results. A subject report includes at least a likelihood assessment, e.g., an indication as to the likelihood that a cancer patient will exhibit long-term survival without breast cancer recurrence. A subject report can be completely or partially electronically generated, e.g., presented on an electronic display (e.g., computer monitor). A report can further include one or more of: 1) information regarding the testing facility; 2) service provider information; 3) patient data; 4) sample data; 5) an interpretive report, which can include various information including: a) indication; b) test data, where test data can include a normalized level of one or more RNA transcripts of interest, and 6) other features.

The present invention therefore provides methods of creating reports and the reports resulting therefrom. The report may include a summary of the levels of the RNA transcripts, or the expression products of such RNA transcripts, in the cells obtained from the patient's tumor sample. The report may include a prediction that the patient has an increased likelihood of long-term survival without breast cancer recurrence or the report may include a prediction that the subject has a decreased likelihood of long-term survival without breast cancer recurrence. The report may include a recommendation for a treatment modality such as surgery alone or surgery in combination with chemotherapy. The report may be presented in electronic format or on paper.

Thus, in some embodiments, the methods of the present invention further include generating a report that includes information regarding the patient's likelihood of long-term survival without breast cancer recurrence. For example, the methods of the present invention can further include a step of generating or outputting a report providing the results of a patient response likelihood assessment, which can be provided in the form of an electronic medium (e.g., an electronic display on a computer monitor), or in the form of a tangible medium (e.g., a report printed on paper or other tangible medium).

A report that includes information regarding the likelihood that a patient will exhibit long-term survival without breast cancer recurrence, is provided to a user. An assessment as to the likelihood that a cancer patient will exhibit long-term survival without breast cancer reucrrence, is referred to as a "likelihood assessment." A person or entity who prepares a report ("report generator") may also perform the likelihood assessment. The report generator may also perform one or more of sample gathering, sample processing, and data generation, e.g., the report generator may also perform one or more of: a) sample gathering; b) sample processing; c) measuring a level of an RNA transcript or its expression product; d) measuring a level of a reference RNA transcript or its expression product; and e) determining a normalized level of an RNA transcript or its expression product. Alternatively, an entity other than the report generator can perform one or more sample gathering, sample processing, and data generation.

The term "user" or "client" refers to a person or entity to whom a report is transmitted, and may be the same person or entity who does one or more of the following: a) collects a sample; b) processes a sample; c) provides a sample or a processed sample; and d) generates data for use in the likelihood assessment. In some cases, the person or entity who provides sample collection and/or sample processing and/or data generation, and the person who receives the results and/or report may be different persons, but are both referred to as "users" or "clients." In certain embodiments, e.g., where the methods are completely executed on a single computer, the user or client provides for data input and review of data output. A "user" can be a health professional (e.g., a clinician, a laboratory technician, a physician (e.g., an oncologist, surgeon, pathologist), etc.).

In embodiments where the user only executes a portion of the method, the individual who, after computerized data processing according to the methods of the invention, reviews data output (e.g., results prior to release to provide a complete report, a complete, or reviews an "incomplete" report and provides for manual intervention and completion of an interpretive report) is referred to herein as a "reviewer." The reviewer may be located at a location remote to the user (e.g., at a service provided separate from a healthcare facility where a user may be located).

Where government regulations or other restrictions apply (e.g., requirements by health, malpractice, or liability insurance), all results, whether generated wholly or partially electronically, are subjected to a quality control routine prior to release to the user.

### COMPUTER -BASED SYSTEMS AND METHODS

The methods and systems described herein can be implemented in numerous ways. In one embodiment of the invention, the methods involve use of a communications infrastructure, for example, the internet. Several embodiments of the invention are discussed below. The present invention may also be implemented in various forms of hardware, software, firmware, processors, or a combination thereof. The methods and systems described herein can be implemented as a combination of hardware and software. The software can be implemented as an application program tangibly embodied on a program storage device, or different portions of the software implemented in the user's computing environment (e.g., as an applet) and on the reviewer's computing environment, where the reviewer may be located at a remote site (e.g., at a service provider's facility).

In an embodiment of the invention, during or after data input by the user, portions of the data processing can be performed in the user-side computing environment. For example, the user-side computing environment can be programmed to provide for defined test codes to denote a likelihood "score," where the score is transmitted as processed or partially processed responses to the reviewer's computing environment in the form of test code for subsequent execution of one or more algorithms to provide a result and/or generate a report in the reviewer's computing environment. The score can be a numerical score (representative of a numerical value) or a non-numerical score representative of a numerical value or range of numerical values (e.g., "A": representative of a 90-95% likelihood of a positive response; "High": representative of a greater than 50% chance of a positive response (or some other selected threshold of likelihood); "Low": representative of a less than 50% chance of a positive response (or some other selected threshold of likelihood), and the like.

As a computer system, the system generally includes a processor unit. The processor unit operates to receive information, which can include test data (e.g., level of an RNA transcript or its expression product; level of a reference RNA transcript or its expression product; normalized level of an RNA transcript or its expression product) and may also include other data such as patient data. This information received can be stored at least temporarily in a database, and data analyzed to generate a report as described above.

Part or all of the input and output data can also be sent electronically. Certain output data (e.g., reports) can be sent electronically or telephonically (e.g., by facsimile, using devices such as fax back). Exemplary output receiving devices can include a display element, a printer, a facsimile device and the like. Electronic forms of transmission and/or display can include email, interactive television, and the like. In an embodiment of the invention, all or a portion of the input data and/or output data (e.g., usually at least the final report) are maintained on a web server for access, preferably confidential access, with typical browsers. The data may be accessed or sent to health professionals as desired. The input and output data, including all or a portion of the final report, can be used to populate a patient's medical record that may exist in a confidential database as the healthcare facility.

The present invention also contemplates a computer-readable storage medium (e.g., CD-ROM, memory key, flash memory card, diskette, etc.) having stored thereon a program which, when executed in a computing environment, provides for implementation of algorithms to carry out all or a portion of the results of a likelihood assessment as described herein. Where the computer-readable medium contains a complete program for carrying out the methods described herein, the program includes program instructions for collecting, analyzing and generating output, and generally includes computer readable code devices for interacting with a user as described herein, processing that data in conjunction with analytical information, and generating unique printed or electronic media for that user.

Where the storage medium includes a program that provides for implementation of a portion of the methods described herein (e.g., the user-side aspect of the methods (e.g., data input, report receipt capabilities, etc.)), the program provides for transmission of data input by the user (e.g., via the internet, via an intranet, etc.) to a computing environment at a remote site. Processing or completion of processing of the data is carried out at the remote site to generate a report. After review of the report, and completion of any needed manual intervention, to provide a complete report, the complete report is then transmitted back to the user as an electronic document or printed document (e.g., fax or mailed paper report). The storage medium containing a program according to the invention can be packaged with instructions (e.g., for program installation, use, etc.) recorded on a suitable substrate or a web address where such instructions may be obtained. The computer-readable storage medium can also be provided in combination with one or more reagents for carrying out a likelihood assessment (e.g., primers, probes, arrays, or such other kit components).

### EXAMPLE 1

### Materials and Methods

### Patients

One hundred and thirty-six primary breast cancer FFPE tumor specimens with clinical outcomes were provided by Providence St. Joseph Medical Center (Burbank, CA), with institutional review board approval. The time to first recurrence of breast cancer or death due to breast cancer (including death due to unknown cause) was determined from these records. Patients who were still alive without breast cancer recurrence or who died due to known other causes were considered censored at the time of last follow-up or death. These tumor specimens were used for biomarker discovery in the development of the Onco*type* DX® assay. *See e.g.,* U.S. Patent No. 7,081,340; S. Paik et al., The New England Journal of Medicine 351, 2817 (2004). For the present study, 136 specimens had adequate RNA remaining. Among the 136 patients, 26 experienced breast cancer recurrence or death due to breast cancer.

### RNA-Seq Sample Preparation and Sequencing

Total RNA was prepared from three 10-µm-thick sections of FFPE tumor tissue as previously described using the MasterPure™ Purification Kit (Epicentre® Biotechnologies, Madison, WI). M. Cronin et al., The American Journal of Pathology 164, 35 (Jan, 2004). One hundred nanograms of the isolated RNA were depleted of ribosomal RNA as described. *See* U.S. Pub. No. 2011/0111409. Sequencing libraries for whole transcriptome analysis were prepared using ScriptSeq™ mRNA-Seq Library Preparation Kits (Epicentre® Biotechnologies, Madison, WI). During the cDNA synthesis step, additional incubation for 90 minutes at 37°C was implemented in the reverse transcription step to increase library yield. After 3'-terminal tagging, the di-tagged cDNA was purified using MinElute® PCR Purification Kits (Qiagen, Valencia, CA). Two 6 base index sequences were used to prepare barcoded libraries for duplex sequencing (RNA-Seq Barcode Primers; Epicentre® Biotechnologies, Madison, WI). PCR was carried out through 16 cycles to generate the second strand of cDNA, incorporate barcodes, and amplify libraries. The amplified libraries were size-selected by a solid phase reversible immobilization, paramagnetic bead-based process (Agencourt® AMPure® XP System; Beckman Coulter Genomics, Danvers, MA). Libraries were quantified by PicoGreen® assay (Life Technologies, Carlsbad, CA) and visualized with an Agilent Bioanalyzer using a DNA 1000 kit (Agilent Technologies, Waldbronn, Germany).

TruSeq™ SR Cluster Kits v2 (Illumina Inc.; San Diego, CA) were used for cluster generation in an Illumina cBOT™ instrument following the manufacturer's protocol. Two indexed libraries were loaded into each lane of flow cells. Sequencing was performed on an Illumina HiSeq®2000 instrument (Illumina, Inc.) by the manufacturer's protocol. Multiplexed single-read runs were carried out with a total of 57 cycles per run (including 7 cycles for the index sequences).

### Data Quality Assessment

Each sequencing lane was duplexed with two patient sample libraries using a 6 base barcode to differentiate between them. The mean read ratio +/-SD between the two samples in each lane was 1.05±0.38 and the mean +/-SD percentage of un-discerned barcodes was 2.08%±1.63%. Using principal components analysis and other exploratory data analysis methods, no systematic differences were found among samples associated with flow cell or barcode.

In a run-in phase of the study, duplicate libraries were prepared for 8 samples selected at random from the study set of 136. RefSeq RNA coverage for these libraries ranged between 3.1M and 6.7M uniquely mapped reads. Log count Pearson correlations among duplicate libraries ranged between 0.947 and 0.985. Single libraries were prepared for the remaining 128 samples and distributed in duplex mode among the lanes of 8 flow-cells. Sequencing in 3 lanes failed. Two libraries had low yield, resulting in low coverage. Three lanes were flagged by various Illumina process monitoring indices: low Q30 (coverage = 2.8M and 4.2M), high cluster density (coverage = 1.6M and 1.8M), or inadequate imaging (coverage = 3.3M and 3.1M). For the remaining lanes, sample coverage ranged between 2.5M and 7.3M reads. New libraries for the samples that had low yield were prepared and sequenced. Libraries in the failed and flagged lanes, as well as some of the low coverage samples, were re-sequenced. Replicate correlations among all sequenced samples were very high, 0.985 for the samples with the high cluster density in the original run, and over 0.990 for all others. For the analysis data set, data for one of each of the duplicate libraries from the run-in experiment were kept. For the samples for which new libraries were prepared and for the samples in the failed and flagged lanes, the reads from the subsequent run were used. For the samples with low coverage for which the library was reprocessed, reads from the two runs were pooled. For the rest of the samples, the reads from the single lane were used. Results differed little when other data analysis procedures were used, for example, using only the second run when libraries were reprocessed.

### Statistics and Bioinformatics

With the exception noted below, all primary analysis of sequence data was performed in CASAVA 1.7, the standard data processing package from Illumina. De-multiplexing of sample indices was set with 1 mismatch tolerance to separate the two samples within each lane. Raw FASTQ sequences were trimmed from both ends before mapping to the human genome (UCSC release, version 19), to address 3' end adapter contamination and random RT primer artifacts, and 5' end terminal-tagging oligonucleotide artifacts. The libraries as prepared contain strand-of-origin (directional) sequence information. Annotated RNA counts (defined by refFlat.txt from UCSC) were calculated by CASAVA 1.7 both with and without consideration of strand-of-origin information. Although retained in the mapping process, CASAVA does not provide directional counts by default. These counts were obtained by splitting the mapped (export.txt) file into two parts, one with sense strand counts, the other with antisense strand counts, and processing them independently. Raw FASTQ sequence was mapped with Bowtie (B. Langmead et al., Genome Biology 10, R25 (2009)) in parallel with CASAVA to count ribosomal RNA transcripts.

Data were analyzed in 3 categories: first, RefSeq RNAs, about 80% of which are exon sequences, consolidated for each gene; second, intronic RNA sequences, consolidated for each gene; third, intergenic sequences. RNAs with maximum counts less than 5 among the 136 patients were excluded from analysis. Of 21,283 total RefSeq transcripts counted by CASAVA, 821 had a maximum count less than 5, leaving 20,462 RefSeq transcripts for analysis. Similar to a recently published procedure described by Bullard et al. (BMC Bioinformatics 11, 94 (2010)), log₂ raw RNA counts (setting the log2 for a 0 count to 0) were normalized by subtracting the 3rd quartile of the log₂ RefSeq RNA counts and adding the cohort mean 3rd quartile ("Q3 normalization"). For analysis of RefSeq and intergenic RNAs normalization, RefSeq RNA data were used. For analysis of intronic RNAs normalization, intronic RNA data were used.

Standardized hazard ratios for breast cancer recurrence for each RNA, that is, the proportional change in the hazard with a 1-standard deviation increase in the normalized level of the RNA, were calculated using univariate Cox proportional hazard regression analyses (Cox, Journal of the Royal Statistical Society: Series B (Methodological) 34, 187 (1972)). The robust standard error estimate of Lin and Wei (Journal of the American Statistical Society, 84, 1074 (1989)) was used to accommodate possible departures from the assumptions of Cox regression, including nonlinearity of the relationship of gene expression with log hazard and nonproportional hazards. False discovery rates (FDR, q-values) were assessed using the method of Storey *(*Journal of the Royal Statistical Society, Series B 64, 479 (2002)) with a "tuning parameter" of λ=0.5. Analyses were conducted to identify true discovery degree of association (TDRDA) sets of RNAs with absolute standardized hazard ratio greater than a specified lower bound while controlling the FDR at 10% (Crager, Statistics in Medicine 29, 33 (2010). Taking individual RNAs identified at this FDR, the analysis finds the maximum lower bound for which the RNA is included in a TDRDA set. Also computed was an estimate of each RNA's actual standardized hazard ratio corrected for regression to the mean. *Id.*

Expression of 192 transcripts in the same tumor RNAs was measured using previously described RT-PCR methods (Cronin et al., The American Journal of Pathology 164, 35 (Jan, 2004); Cronin et al., Clinical Chemistry 50, 1464 (Aug, 2004)). Standardized hazard ratios associating the expression of each gene (normalized by subtracting each gene's crossing threshold (C_{T}) from the cohort median C_{T}) with cancer recurrence were computed using the same methods used for evaluation of the RNA-Seq data.

### Identifying Intergenic Sequences

Intergenic regions were identified by a novel program that evaluates genomic regions that vary widely in length and on a population basis. This program was developed to evaluate intergenic regions having wide variations in length, and to use data from a population of subjects rather than an individual subject. The uniquely mapped reads from all 136 patients were analyzed to identify clusters of reads that might arise from intergenic transcripts. Genomic regions containing less than 2 mapped reads of genomic sequence were not counted to eliminate potential noise from mis-mapping orgenomic DNA contamination. The remaining reads were clustered into individual read "islands" based on the overlap of their mapped coordinates to the hg 19 reference human genome, which resulted in 12,750,071 islands in all 136 patient samples. Any islands within 30 base pairs (bp) of each other were grouped together as regions of interest (ROI) producing a total of 6,633,258 ROIs. The number of ROIs were further reduced by the following criteria: 1) The average number of reads mapped to the ROI was ≥ 5 across all 136 patients, 2) the length of the ROI was at least 100 bp, and 3) the read depth (average read number divided by the length of the ROI) was ≥ 0.075. Applying these criteria reduced the number of ROIs to 23,024. ROIs were classified as intergenic regions if they did not overlap with the transcripts (including non-coding ones) annotated in the refFlat.txt file obtained from UCSC, thereby eliminating overlap with known exons and introns of protein-coding genes and well annotated non-protein coding transcripts. A total of 2,101 intergenic regions were identified by this computational procedure.

### EXAMPLE 2

### Evaluation of whole transcriptome RNA-Seq as a platform for biomarker discovery

Patient clinical characteristics are shown in Table 12. One-hundred and ten patients (81 %) had no involved nodes. There was a mixture of chemotherapy and hormonal therapy usage. Estrogen receptor (ER) status was not included in patient records. Therefore, normalized ESR1 mRNA levels obtained in the present RNA-Seq study were used to identify 111 tumors as estrogen-receptor positive and 25 as estrogen-receptor negative. Use of RT-PCR rather than RNA-Seq for this purpose yielded similar but not identical results, identifying as ER+ two more patients, for a total of 113. Archive ages of FFPE tumor blocks ranged from 5 to 12.4 years (median 8.5 years).

RNA-Seq results were successfully generated for all 136 patients, with an average of 43 million median reads per patient (86 million median reads per Illumina Hiseq 2000 flow cell lane). Sixty-nine percent of these uniquely mapped to the human genome: 19.2% to exons, 64.9 % to introns, and 15.9% to intergenic regions. Ribosomal RNA accounted for less than 0.3% of the total reads. On average, 17,248 Refseq transcripts were detected per patient, 66% with greater than 10 counts, and 47% with greater than 100 counts.

Use of third quartile normalization effectively mitigated trends in overall coverage related to sample age and produced stable estimates of expression with relative log expression (RLE, individual gene log₂ count minus within-patient median log₂ count) values that were centered on zero and relatively tightly distributed around 0, an indicator of effective normalization.

Figure 1A displays results from the historical RT-PCR 192 candidate gene screen of the Providence 136 patient cohort, relating increasing mRNA expression to recurrence risk hazard ratios and statistical significance. As shown, fourteen of the sixteen cancer-related genes in the *Oncotype* DX® panel were assayed, and most were identified with Hazard Ratios greater than 1.2 or less than 0.8 and P values <0.05.

The effect sizes and statistical significance of *Oncotype* DX® genes were similar when screening was carried out by whole transcriptome RNA-Seq rather than RT-PCR (compare Figures 1A and 1B). This is shown in detail on a gene by gene basis in box plots (Figure 2). A scatter plot of log hazard ratios demonstrates overall concordance between the 192 gene RT-PCR results with the RNA-Seq analyses (Lin et al., Journal of the Royal Statistical Society, Series B 84, 1074 (1989)) (Lin concordance correlation: 0.810; Pearson correlation coefficient: 0.813; Figure 3). Significantly, RNA-Seq further associates many RefSeq RNAs with disease recurrence: a total of 1307 at FDR<10% (Table 1), hereafter referred to as "identified RefSeq RNAs." In contrast, the 192 gene RT-PCR study identified 32 RNAs at FDR<10%, and consumed five-fold more input RNA. Together, these results indicate that RNA-Seq can provide a practical, sensitive and precise platform for genome-wide biomarker discovery in FFPE tissue.

### EXAMPLE 3

### RefSeq Transcripts and Gene Networks that Associate with Risk of Breast Recurrence

There were 1307 RefSeqs associated with disease recurrence outcome at FDR <10% (Table 1). Because the reproducibility of within-sample transcript counts inevitably decreases as transcript abundance decreases, the impact of transcript abundance on initial biomarker discovery was evaluated. These 1307 RNAs were binned with respect to count abundance. Accounting for the 821 transcripts with maximum counts less than 5, which were deliberately excluded from analysis, rare transcripts (with less than 10 median counts) represent 28% of all RefSeq transcripts. The percent of RNAs identified decreases but is not dramatically different as median counts decrease from greater than 1,000 to 10-99. Even at median counts less than 10, the percent of RNAs identified fell by less than half compared to sequences present at higher abundance.

Among the 1307 identified RefSeq RNAs, many relate to recurrence with very high statistical significance (Table 1). TDRDA analysis identified 144 with standardized hazard ratio greater than 1.1, controlling FDR at 10%. Estimated standardized hazard ratios corrected for regression to the mean are as high as 1.66. Uncorrected hazard ratios range from approximately 0.4 to 2.5. The ratio of RNAs for which high expression associates with increased risk of cancer recurrence, versus decreased risk is approximately 1.

The library chemistry used in this study provides DNA strand-of-origin information for transcripts. The analysis that identifies 1307 prognostic RefSeq RNAs is not filtered for directionality. When this is done, 1023 of these RefSeq transcripts are still associated with disease recurrence at FDR<10% when only sense strand counts are analyzed. Less than 10% of the total RefSeq counts locate in the anti-sense direction. Nevertheless, 798 anti-sense transcripts associate with recurrence risk.

### Validation of the Association of RefSeq transcripts with Breast Cancer Prognosis in an Independent Cohort

The performance of these identified RNAs was further evaluated using public gene expression data from an independent cohort of breast cancer patient tumors that had been assayed by DNA microarray technology. The microarray data set was assembled by merging patient sets published in two articles (M. J. van de Vijver et al., New England Journal of Medicine 347, 1999 (2002); L. J. Van't Veer et al., Nature 415, 530 (2002)), providing data on 337 patients ("NKI dataset"). Metastasis-free survival information was available for 319 patients. Standardized hazard ratios for cancer recurrence were estimated for each gene targeted by the microarray using univariate Cox proportional hazard regression analysis. Genes were identified as prognostic using a 10% FDR threshold as was done with the RNA-Seq data. Among the 11,659 genes common to both platforms, there is highly significant agreement in the classification of genes as prognostic (Figure 4) but concordance falls off as transcript abundance decreases. For RNA-Seq RNAs present at >100 counts, 44% were identified as prognostic in the NKI dataset.

### Gene Networks

Hierarchical clustering (Eisen et al., Proceedings of the National Academy of Sciences 95, 14863 (1998)) of the 1307 identified RefSeq RNAs (Table 1) suggests the presence of co-expressed gene networks. Cytoscape (P. Shannon et al., Genome Research 13, 2498 (2003); M. E. Smoot et al., Bioinformatics 27, 431 (2011)) was used to evaluate the subset of these RNAs for which each member correlates in its expression with at least one other RNA at R ≥0.6. Figure 5 graphically represents the resulting correlation matrix of 597 genes and 4011 interactions. One prominent (51 member) RefSeq RNA network represented in Figure 5 is enriched in RNAs with Reactome database annotations (G. Joshi-Tope et al., Nucleic Acids Res 33, D428 (2005)) that are functionally related to regulation of the cell cycle and mitosis, and associates with poor prognosis ("cell cycle network") (Table 6). This network includes three of the five proliferation-associated Onco*type* DX® genes (BIRC5, MYBL2, MKI67). A second network is enriched in RNAs that co-express with the estrogen receptor gene (ESR1) ("ESR1 network") and associate with reduced recurrence risk, including the *Oncotype* DX® genes, BCL2 and SCUBE2. The other ESR1 network genes include CPEB2, IL6ST, DNALI1, PGR, SLC7A8, C6orf97, RSPH1, EVL, BCL2, NXNL2, GATA3, GFRA1, GFRA1, ZNF740, MKL2, AFF3, ERBB4, RABEP1, KDM4B, ESR1, C4orf32, and CPLX1 (Table 7). ESR1 itself is not statistically associated with disease outcome in our RNA-Seq results, nor was it previously found to be significant in this cohort by RT-PCR analysis.

This analysis also reveals several novel RNA networks, three of which map to discrete cytogenetic bands (Figure 5): 1) a network of five poor prognosis RNAs mapping to a 289 kilobase region located at Chr9q22 ("Chr9q22 network"), which includes ASPN, CENPP, ECM2, OGN, and OMD (Table 8); 2) a network of twelve RNAs mapping to a 6.6 megabase region of Chr17q23-24 ("Chr17q23-24 network"), which includes CCDC45, POLG2, SMURF2, CCDC47, CLTC, DCAF7, DDX42, FTSJ3, PSMC5, RPS6KB1, SMARCD2, and TEX2 (Table 9); and 3) a fourteen RNA network mapping to a 47 megabase span on Chr8q21-24 ("Chr8q21-24 network"), which includes CYC1, DGAT1, GPAA1, GRINA, PUF60, PYCRL, RPL8, SQLE, TSTA3, ESRP1, GRHL2, INTS8, MTDH, and UQCRB (Table 10). Finally, Figure 5 represents a large (134 member) RNA network that has strong Gene Ontology and Biocarta annotations to olfactory signaling, glucose metabolism, and glucuronidation ("olfactory receptor network") (Table 11). Nine of the transcripts in this novel network encode olfactory receptors. (OR10H3, OR14J1, OR2J2, OR2W5, OR5T2, OR7E24, OR7G3, OR8S1, and OR9K2). Fifteen are microRNA precursors (MIR1208, MIR1266, MIR1297, MIR133A1, MIR195, MIR196A1, MIR3170, MIR3183, MIR4267, MIR4275, MIR4318, MIR501, MIR501, MIR539, and MIR542). Most of the RNAs in this network are rare (raw median counts less than 10). All but 2 of them associate with poor prognosis as shown in Table 1.

**TABLE 6: Cell cycle network**

| | | | | |
|---|---|---|---|---|
| ANLN | CDC25C | EPR1 | KIF18B | NUSAP1 |
| ASPM | CDCA2 | EXO1 | KIF20A | PGK1 |
| BIRC5 | CENPE | FAM83D | KIF23 | PRC1 |
| BLM | CENPF | GTSE1 | KIF2C | PRR11 |
| BUB1 | CENPN | HIST1H2AH | KPNA2 | RRM2 |
| BUB1B | CENPO | HJURP | MELK | SGOL1 |
| C15orf23 | CEP55 | HMMR | MKI67 | TRIP13 |
| CASC5 | DLGAP5 | INCENP | MYBL2 | TROAP |
| CCNA2 | E2F1 | KIF11 | NEK2 | TUBA1B |
| CCNB2 | ECT2 | KIF14 | NUP93 | UBE2C |
| | | | | ZNF695 |

**TABLE 7: ESR1 network**

| | | | | | |
|---|---|---|---|---|---|
| BCL2 | SCUBE2 | CPEB2 | IL6ST | DNALI1 | PGR |
| SLC7A8 | C6orf97 | RSPH1 | EVL | BCL2 | NXNL2 |
| GATA3 | GFRA1 | GFRA1 | ZNF740 | MKL2 | AFF3 |
| ERBB4 | RABEP1 | KDM4B | ESR1 | C4orf32 | CPLX1 |

**TABLE 8: Chr9q22 network**

| | | | | |
|---|---|---|---|---|
| ASPN | CENPP | ECM2 | OGN | OMD |

**TABLE 9: Chr17q23-24 network**

| | | | | | |
|---|---|---|---|---|---|
| CCDC45 | POLG2 | SMURF2 | CCDC47 | CLTC | DCAF7 |
| DDX42 | FTSJ3 | PSMC5 | RPS6KB1 | SMARCD2 | TEX2 |

**TABLE 10: Chr8q21-24 network**

| | | | | | | |
|---|---|---|---|---|---|---|
| CYC1 | DGAT1 | GPAA1 | GRINA | PUF60 | PYCRL | RPL8 |
| SQLE | TSTA3 | ESRP1 | GRHL2 | INTS8 | MTDH | UQCRB |

**TABLE 11: Olfactory receptor network**

| | | | | |
|---|---|---|---|---|
| AFM | F11 | LOC285577 | NR1H4 | SLC30A10 |
| APCS | F9 | LOC401177 | OCM | SLC30A3 |
| APQBEC1 | FAM131C | LOC401242 | OPALIN | SNAR-G1 |
| ATOH7 | FAM169B | LOC642006 | OR10H3 | SNCB |
| ATXN3L | FAM9C | LOC646960 | OR14J1 | SNORD116 |
| BARHL2 | FEZF1 | LOC729966 | OR2J2 | SNORD188 |
| C17orf64 | FOXD4L5 | LRIT2 | QR2W5 | SP8 |
| C18orf26 | FTMT | LYPD2 | OR5T2 | SPRR3 |
| C19orf75 | FZD9 | MAGEB2 | 0R7E24 | SPRYD5 |
| C20orf185 | GBX2 | MIP1208 | OR7G3 | STATH |
| C7orf72 | GPR33 | MIR1266 | OR8S1 | TAAR6 |
| C9orf27 | GPX5 | MIR1297 | OR9K2 | TFAP2D |
| CA5A | GSTA2 | MIR133A1 | PABPC1L2B | TRIM10 |
| CAMKV | GSTTP1 | MIR195 | PACRGL | TRYX3 |
| CHAT | HBZ | MIR196A1 | PCDH11Y | TSFAN19 |
| CLCN1 | HCRTR2 | MIR3170 | PGA3 | TXNDC8 |
| CLEC18B | HMX1 | MIR3183 | PNLIP | UGT2A1 |
| COL20A1 | HMX3 | MIR4267 | POM121L10 | UGT2B10 |
| COL9A1 | KCNJ4 | MIR4275 | POTEG | UGJ2B7 |
| COX8C | KCNV1 | MIR4318 | POU3F4 | VWC2 |
| CRYZ | KRT20 | MIR501 | PRSS41 | ZFP42 |
| DEFB133 | KRT72 | MIRS39 | RAB28 | ZNF705D |
| DEFB135 | KRT78 | MIR542 | RAB9BP1 | |
| DNAJC5G | KRT83 | MOXD2P | RP1-177G6 | |
| DOC2GP | LHX5 | NCRNA0020 | RXFP2 | |
| DSCR10 | LOC100129 | NCRNA0022 | SCRT2 | |
| EVX1 | LOC100133 | NPFFR1 | SERPINB10 | |
| EVX2 | LOC144742 | NPPB | SI | |

**Table 12. Case characteristics and outcomes**

| **Characteristic** | **No. patients/no. analyzed (%)** |
|---|---|
| Tumor size (cm) | |
| 0-2 | 81/136 (60%) |
| 2-5 | 49/136 (36%) |
| >5 | 6/136 (4%) |
| | |
| No. lymph nodes at primary diagnosis | |
| 0 | 110/136 (81%) |
| 1-9 | 11/136 (8%) |
| 10-15 | 9/136 (7%) |
| 16-20 | 6/136 (4%) |
| | |
| Adjuvant tamoxifen | |
| Yes | 54/136 (40%) |
| No | 77/136 (57%) |
| Unknown | 5/136 (4%) |
| | |
| Adjuvant chemotherapy | |
| Yes | 51/136 (38%) |
| No | 79/136 (58%) |
| Unknown | 6/136 (4%) |
| | |
| ER Status* | |
| ER positive | 111/136 (82%) |
| ER negative | 25/136 (18%) |
| | |
| Vital Status | |
| Distant recurrence, death due to breast cancer, or death due to unknown cause | |
| Total | 26/136 (19%) |
| ER positive | 16/136 (12%) |
| ER negative | 10/136 (7%) |
| Alive without distant recurrence or death due to other cause | |
| Total | 110/136 (81%) |
| ER positive | 95/136 (70%) |
| ER negative | 15/136 (11%) |

| | |
|---|---|
| *ER status determined by RNA-Seq analysis as described | |

### EXAMPLE 4

### Risk of recurrence in ER+ patients

ER status, which is often described in clinical practice in binary terms as ER+ and ER- via immunohistochemistry evaluation of breast tumors, dichotomizes breast cancer with respect to clinical outcome and gene expression profiles. While ER status information was not part of patient records for this study cohort, RNA-Seq ESR1 counts were used to separate patients. This analysis is presented in Table 2. This is a novel method of defining ER status but note the small population size (10 recurrence events) and the absence of hormonal therapy in a significant fraction of those patients that were defined as ER+. Administration of hormonal therapy (e.g., tamoxifen or an aromatase inhibitor) is current standard clinical practice, and both significantly decreases recurrence risk and influences the nature of biomarkers that predict recurrence. Nevertheless, this analysis does identify the expected cell cycle gene signature as a marker of high recurrence risk (exemplified by the genes CCNA2; CENPN, KIF20, ARPP19 and BUB3). In all, expression of 363 RefSeq transcripts relate to recurrence risk at FDR<10% (Table 2). Within this set of transcripts, the most prominent RefSeq RNA network found using Cytoscape as described above is similar to the rare "olfactory receptor network" that was identified in the analysis of the entire 136 patient cohort. In the ESR1+ patients, this olfactory receptor network consists of 86 RefSeq RNAs (see Table 13), 6 of which are olfactory receptors (OR14J1, OR2B3, OR2J2, OR2W5, OR5T2, OR8S1) and 8 pre-microRNAs (MIR1208, MIR1251, MIR1266, MIR195, MIR4275, MIR4318, MIR542, MIR548I2). All RNAs in this network associate with increased risk of disease recurrence as shown in Table 2.

**TABLE 13: Olfactory receptor network in ER-positive patients**

| | | | | |
|---|---|---|---|---|
| APCS | APOBEC1 | ATXN3L | BAGE | C17orf88 |
| C18orf26 | C19orf75 | C9orf27 | CA5A | CCDC105 |
| CHAT | COL20A1 | COX7B2 | COX8C | DEFB133 |
| DKFZp779M | DSCR10 | DUSP13 | EVX1 | EVX2 |
| FAM169B | FEZF1 | GAB4 | GDF7 | GPR50 |
| GPX5 | GSTA2 | GUCY2F | HCRTR2 | HMX1 |
| HMX3 | KRT78 | KRT83 | LOC100129 | LOC144742 |
| LOC285577 | LOC286186 | LOC401177 | LOC642345 | LOC645971 |
| LOC647107 | LOC729966 | LPO | LRIT2 | LYPD2 |
| MAGEA10 | MAGEB10 | MIR1208 | MIR1251 | MIR1266 |
| MIR195 | MIR4275 | MIR4318 | MIR542 | MIR548I2 |
| NCRNA0020 | NCRNA0022 | NKX1-2 | NPPB | OR14J1 |
| OR2B3 | OR2J2 | OR2W5 | OR5T2 | OR8S1 |
| PCDH11Y | PGA3 | POTEG | PRDM9 | PRSS41 |
| RAB9BP1 | RAET1L | RNASE9 | RP1-177G6 | SCRT2 |
| SERPINB10 | SLC17A6 | SNAR-G1 | SNCB | SNORD115 |
| SNORD116 | SNORD18C | SOST | SPRR3 | TPTE |
| WFDC9 | | | | |

### EXAMPLE 5

### Association of Intronic Sequences with Breast Cancer Prognosis

Reads mapping to intronic regions of the genome account for -65% of all of the sequence data. Introns tend to co-express with exons of the same genes (median R = 0.67), although these correlations vary over a wide range, from roughly zero to over 0.9. The percent of intronic RNAs that map in the antisense direction is slightly higher than in the case of RefSeq RNAs (median: ∼7.5% versus ∼5%, respectively). A large number (1698) of intronic RNAs associate with breast cancer recurrence (at FDR < 10%; non-directional analysis; Table 3), with ranges of hazard ratios and p-values are similar to those of the above-identified 1307 RefSeq RNAs.

Over two thirds (1154) of the identified intronic transcripts do not lie within the prognostic RefSeq RNAs listed in Table 1 above. That is, their cognate assembled exons are not also discovered. (Among the 100 most highly statistically significant intronic RNAs this fraction is 0.44.) This subset of the identified intronic RNAs is particularly likely to contain prognostic information that is not captured by the RefSeq RNAs. The basis for these might be statistical: average counts for all intronic RNAs are more than threefold higher than for all exons, so signal to noise ratio is more favorable for discovery of intronic RNAs. Nevertheless, in the population of exons that are not discovered along with discovered cognate introns, average exon abundance is just a little lower than in the entire population of discovered exons (mean counts (244, versus 312 average counts, respectively). This result is consistent with these intronic RNAs carrying prognostic information that is not carried in their corresponding exons.

### EXAMPLE 6

### Association of Intergenic Sequences with Breast Cancer Prognosis

Two approaches were used to search for biomarkers within the population of intergenic RNAs, first by interrogating reads that map to 2,500 well-documented long intergenic non-coding RNAs (lincRNAs) (A. M. Khalil et al., Proceedings of the National Academy of Sciences of the United States of America 106, 11667 (Jul 14, 2009)). Twenty-two of these (Table 4), associate with breast cancer recurrence risk at FDR<10%. Second, intergenic transcripts were screened more broadly by using a novel computational algorithm described in Example 1 to identify clusters of reads that map to intergenic regions of the genome in one or more of the tumor specimens. The number of reads mapped to these clusters was used as a measure of the relative expression of putative intergenic transcripts. Altogether, 2101 putative intergenic transcripts were identified, 775 of which are contained in or overlap with lincRNAs that have been identified previously in one or more previous studies of non-coding transcripts and their expression was tested for association with recurrence of breast cancer in the cohort of 136 patients. Expression of 194 (9%) of these transcripts correlates with breast cancer recurrence at FDR < 10%. This list of 194 transcripts was further condensed by merging clusters of reads separated by < 1000 bp to produce a set of 69 intergenic transcripts associated with recurrence of breast cancer (Table 5). Thirty-two of these 69 associate with decreased recurrence risk. The criterion for merging of clusters (< 1000 bp) is supported by the observation that the median correlation coefficient for co-expression of the merged clusters is extraordinarily high (median R = 0.94). Non-merged transcripts exhibited weak co-expression (median R = 0.27).

### EXAMPLE 7

In a second study, 78 patient samples as described in Cobleigh et al., Clin. Cancer Res. 11:8623-8631 (2005) and in U.S. Patent No. 7,569,345 were obtained from women with invasive breast cancer and ≥ 10 positive nodes with no evidence of metastatic disease who had surgery at Rush University Medical Center from 1979 to 1999. Clinical outcome data were available for all patients. Patients who were still alive without breast cancer recurrence or who died due to known other causes were considered censored at the time of last follow-up or death. For the present study, 76 specimens had adequate RNA remaining for RNA-Seq.

Clinical characteristics of the 78 patients are shown in Table 14. RNA preparation, sequencing, and data analyses were performed as described in Example 1. Table 15 shows 125 RefSeq genes identified by RNA-Seq that were associated with breast cancer recurrence at FDR <10%. RefSeqs marked with "1" were associated with an increased likelihood of breast cancer recurrence and those marked with "-1" were associated with a decreased likelihood of breast cancer recurrence. Table 15 shows the maximum lower bound, greater or equal to 0 for identified genes at 10% FDR; StdHR, which is the estimated standardized Hazard Ratio from the proportional hazards model; StdHR.qvalue, which is the q-value computed from the set of StdHR p-values derived from the robust estimate of standard errors and using Storey's procedure as implemented in R qvalue package with lambda=0.5; and StdHR.pv, which is the p-value of the estimated standardized coefficient for null hypothesis coeff=0 or HR (hazard ratio)=1. The accession numbers of each of the 125 RefSeqs are shown in Table B. Twenty of these genes were also associated with recurrence in the first study described in Example 1. This overlap is unlikely to occur by chance (p<2.5 X 10⁻⁵).

**TABLE 14. Case characteristics and outcomes**

| **Characteristic** | **No. patients/no. analyzed (%)** |
|---|---|
| Mean age ± SD (range), years | 57±13 (33-86) |
| | |
| Tumor size (cm) | |
| 0-2 | 26/78 (33%) |
| 2-5 | 28/78 (36%) |
| >5 | 24/78 (31%) |
| No. lymph nodes at primary diagnosis | |
| 0-9 | 0/78 (0%) |
| 10-15 | 40/78 (51%) |
| 15-20 | 18/78 (23%) |
| 20-30 | 12/78 (15%) |
| >30 | 8/78 (10%) |
| Adjuvant tamoxifen | |
| Yes | 42/78 (54%) |
| No | 36/78 (46%) |
| Adjuvant chemotherapy | |
| Yes | 62/78 (80%) |
| No | 16/78 (20%) |
| Tumor grade | |
| 1 | 11/78 (14%) |
| 2 | 37/78 (47%) |
| 3 | 30/78 (38%) |
| Vital Status | |
| Distant recurrence, death due to breast cancer, or death due to unknown cause | 55/78 (71%) |
| | |
| Alive without distant recurrence or death due to other cause | 23/78(29%) |

| **Table 15. List of Assembled RefSeq RNAs Associated with Risk of Breast Cancer Recurrence in 76 Breast Cancer Patients** | | | | | |
|---|---|---|---|---|---|
| **Gene** | **Direction of Association (1=Higher Expression means Higher Risk)** | **Maximum lower bound ≥0 @ 10% FDR** | **StdHR** | **StdHR.qvalue** | **StdHR.pv** |
| TRPS1 | -1 | 0.05 | 0.600271454 | 0.013404626 | 3.19E-06 |
| SHROOM3 | -1 | 0.05 | 0.592316052 | 0.013404626 | 4.52E-06 |
| GREB1L | -1 | 0.05 | 0.589787342 | 0.013404626 | 4.78E-06 |
| MICA | -1 | 0.05 | 0.53495849 | 0.013404626 | 6.17E-06 |
| PIP4K2B | 1 | 0.05 | 1.766657976 | 0.013404626 | 1.26E-06 |
| CWC25 | **1** | 0.05 | 1.710144609 | 0.013404626 | 3.99E-06 |
| SLITRK2 | 1 | 0.05 | 1.742894004 | 0.013404626 | 4.77E-06 |
| C3orf18 | -1 | 0.049 | 0.603537151 | 0.013404626 | 6.91E-06 |
| C3orf15 | -1 | 0.049 | 0.56549207 | 0.014911144 | 8.54E-06 |
| DIS3L | -1 | 0.049 | 0.467081559 | 0.017557656 | 1.31E-05 |
| PSMD8 | **1** | 0.048 | 1.584581143 | 0.013404626 | 6.47E-06 |
| B4GALT5 | 1 | 0.047 | 1.722034237 | 0.015499558 | 1.07E-05 |
| ARPC1B | 1 | 0.046 | 1.621399637 | 0.015499558 | 9.90E-06 |
| SLC26A5 | -1 | 0.04 | 0.616339501 | 0.018957169 | 1.52E-05 |
| ZNF837 | -1 | 0.04 | 0.548382663 | 0.023868625 | 2.13E-05 |
| BEND5 | -1 | 0.038 | 0.589144544 | 0.023868625 | 2.38E-05 |
| LOC100128675 | -1 | 0.038 | 0.565506214 | 0.023868625 | 2.55E-05 |
| GRTP1 | -1 | 0.038 | 0.545883691 | 0.023868625 | 2.60E-05 |
| AP1M1 | 1 | 0.035 | 1.600383771 | 0.023868625 | 2.43E-05 |
| RTN4RL2 | -1 | 0.03 | 0.610998068 | 0.029939316 | 3.43E-05 |
| MAGI1 | -1 | 0.03 | 0.616876931 | 0.030777252 | 3.93E-05 |
| ENPP5 | -1 | 0.03 | 0.595416002 | 0.031338122 | 4.31E-05 |
| BZRAP1 | -1 | 0.03 | 0.584798633 | 0.030777252 | 4.06E-05 |
| DDB1 | **1** | 0.03 | 1.870965036 | 0.033898745 | 5.06E-05 |
| NEDD4L | -1 | 0.029 | 0.606690251 | 0.031907709 | 4.57E-05 |
| TMEM56 | -1 | 0.028 | 0.68559784 | 0.030777252 | 3.91E-05 |
| LRRC49 | -1 | 0.028 | 0.525377682 | 0.036410064 | 6.25E-05 |
| CYBASC3 | **1** | 0.028 | 1.661030039 | 0.033898745 | 5.44E-05 |
| CLINT1 | **1** | 0.028 | 1.671589497 | 0.033898745 | 5.38E-05 |
| CLCA2 | 1 | 0.027 | 1.703214484 | 0.036410064 | 6.44E-05 |
| RPRD1A | -1 | 0.026 | 0.588692979 | 0.038013045 | 7.05E-05 |
| TSPAN10 | -1 | 0.026 | 0.571930206 | 0.038013045 | 7.19E-05 |
| BCL2 | **-1** | 0.023 | 0.607244603 | 0.038114312 | 7.57E-05 |
| BBS5 | -1 | 0.022 | 0.706595678 | 0.036410064 | 6.47E-05 |
| LOC100128640 | -1 | 0.022 | 0.611210083 | 0.039413064 | 8.14E-05 |
| CAMK2G | 1 | 0.022 | 1.577924166 | 0.039413064 | 8.36E-05 |
| TSPAN14 | 1 | 0.022 | 1.677559326 | 0.042870801 | 9.33E-05 |
| RWDD3 | 1 | 0.021 | 0.712509834 | 0.038114312 | 7.64E-05 |
| TRAK1 | -1 | 0.021 | 0.584711667 | 0.047369248 | 0.000117029 |
| LOC730668 | -1 | 0.021 | 0.545574613 | 0.047369248 | 0.000122501 |
| ZNF621 | -1 | 0.021 | 0.53393884 | 0.047369248 | 0.000120123 |
| PELI3 | -1 | 0.021 | 0.536673514 | 0.047369248 | 0.00012757 |
| NCOA3 | 1 | 0.021 | 1.602212601 | 0.047369248 | 0.000107001 |
| PSMB3 | 1 | 0.021 | 1.835670697 | 0.047369248 | 0.00012513 |
| ZNF763 | -1 | 0.018 | 0.646608385 | 0.047369248 | 0.000114793 |
| CHDH | -1 | 0.018 | 0.625931486 | 0.047369248 | 0.000123182 |
| SUSD4 | -1 | 0.017 | 0.681812692 | 0.047369248 | 0.000117816 |
| DNAH14 | -1 | 0.017 | 0.568526034 | 0.051201952 | 0.000149628 |
| KRT4 | 1 | 0.016 | 1.544128591 | 0.048843319 | 0.000137137 |
| STK3 | 1 | 0.015 | 1.577969259 | 0.051201952 | 0.000148028 |
| CCDC24 | -1 | 0.014 | 0.711374716 | 0.048843319 | 0.000135924 |
| IQCH | -1 | 0.013 | 0.690451374 | 0.05619298 | 0.000167433 |
| NCRNA00173 | -1 | 0.013 | 0.636905593 | 0.05928902 | 0.000183576 |
| GTPBP10 | -1 | 0.013 | 0.607771723 | 0.05928902 | 0.000200438 |
| EML2 | -1 | 0.013 | 0.594614968 | 0.05928902 | 0.000197289 |
| LAPTM4B | 1 | 0.013 | 1.559498582 | 0.057278344 | 0.000173949 |
| SLC38A2 | 1 | 0.013 | 1.663522257 | 0.05928902 | 0.000197664 |
| SCARNA15 | 1 | 0.013 | 1.873906539 | 0.05928902 | 0.000194249 |
| MID2 | -1 | 0.011 | 0.675617922 | 0.05928902 | 0.000196751 |
| SLC26A8 | -1 | 0.01 | 0.662530005 | 0.063180343 | 0.000220834 |
| NKAPP1 | -1 | 0.01 | 0.610606787 | 0.063891002 | 0.000226979 |
| CLN5 | -1 | 0.009 | 0.728165906 | 0.062238826 | 0.000213977 |
| TLR5 | -1 | 0.009 | 0.622951299 | 0.067031927 | 0.000241979 |
| NPY5R | -1 | 0.009 | 0.574139163 | 0.070777897 | 0.000259557 |
| LOC402778 | -1 | 0.008 | 0.678984812 | 0.070973513 | 0.000272475 |
| RPL14 | -1 | 0.008 | 0.656553359 | 0.070836186 | 0.00026788 |
| C6orf155 | -1 | 0.008 | 0.643543245 | 0.071686076 | 0.000283425 |
| ABCA3 | -1 | 0.008 | 0.635422012 | 0.070836186 | 0.000267888 |
| LOC723809 | 1 | 0.008 | 1.659680175 | 0.071686076 | 0.000282431 |
| EPB49 | -1 | 0.006 | 0.682134101 | 0.076840825 | 0.000308209 |
| PLEKHA7 | -1 | 0.006 | 0.631608043 | 0.077339749 | 0.000314641 |
| RNU6ATAC | 1 | 0.006 | 1.879268984 | 0.081043767 | 0.000334354 |
| LAMC2 | -1 | 0.004 | 0.692500587 | 0.082276981 | 0.000357633 |
| MST1 | -1 | 0.004 | 0.680494962 | 0.082276981 | 0.000353331 |
| GSTM2 | -1 | 0.004 | 0.631940594 | 0.082276981 | 0.000348608 |
| ZNF337 | -1 | 0.004 | 0.605531534 | 0.082276981 | 0.0003583 |
| MTR | -1 | 0.004 | 0.601352242 | 0.083092569 | 0.000372137 |
| WBSCR27 | -1 | 0.004 | 0.574418341 | 0.083092569 | 0.000372492 |
| LOC100130093 | -1 | 0.003 | 0.727169905 | 0.083092569 | 0.000376135 |
| FGFRL1 | -1 | 0.003 | 0.684954343 | 0.086078557 | 0.000409381 |
| TSNAXIP1 | -1 | 0.003 | 0.667743065 | 0.086078557 | 0.000397407 |
| BAIAP3 | -1 | 0.003 | 0.657836003 | 0.086078557 | 0.000401136 |
| TSC1 | -1 | 0.003 | 0.635691021 | 0.086078557 | 0.000406333 |
| LDLRAD3 | -1 | 0.003 | 0.609908608 | 0.087926789 | 0.000430472 |
| NPY1R | -1 | 0.003 | 0.536099497 | 0.087926789 | 0.000433424 |
| IKBIP | 1 | 0.003 | 1.630733535 | 0.087926789 | 0.000438324 |
| WDR67 | 1 | 0.003 | 1.660073877 | 0.087926789 | 0.000434133 |
| HMGCR | 1 | 0.003 | 1.692917307 | 0.089657364 | 0.000452088 |
| ZNF415 | -1 | 0.002 | 0.702493074 | 0.092515604 | 0.000493007 |
| LMX1B | -1 | 0.002 | 0.644058753 | 0.090095896 | 0.000459462 |
| RBBP8 | -1 | 0.002 | 0.611872583 | 0.093229516 | 0.000511211 |
| SORBS2 | -1 | 0.002 | 0.57041462 | 0.091505704 | 0.000474625 |
| LASP1 | 1 | 0.002 | 1.577783177 | 0.092781758 | 0.000499741 |
| PLCB3 | 1 | 0.002 | 1.591205516 | 0.092515604 | 0.000491513 |
| CKAP4 | 1 | 0.002 | 1.608591213 | 0.093229516 | 0.000520375 |
| RPPH1 | 1 | 0.002 | 1.722187729 | 0.093229516 | 0.000523521 |
| SCARNA16 | 1 | 0.002 | 1.796667248 | 0.091505704 | 0.000477138 |
| MFSD2A | 1 | 0.002 | 1.81578939 | 0.093229516 | 0.000517013 |
| ICA1 | -1 | 0.001 | 0.713463664 | 0.095431649 | 0.000611726 |
| OPLAH | -1 | 0.001 | 0.689897885 | 0.095431649 | 0.000620497 |
| C2orf55 | -1 | 0.001 | 0.674335475 | 0.094641728 | 0.000573056 |
| ZNF48 | -1 | 0.001 | 0.669187687 | 0.095431649 | 0.000618057 |
| APC2 | -1 | 0.001 | 0.66649698 | 0.095431649 | 0.000633369 |
| PSD | -1 | 0.001 | 0.641610877 | 0.094641728 | 0.000555159 |
| FAM201A | -1 | 0.001 | 0.635911763 | 0.095431649 | 0.000634315 |
| C9orf156 | -1 | 0.001 | 0.607820806 | 0.094641728 | 0.000567374 |
| LOC644759 | -1 | 0.001 | 0.593692626 | 0.094641728 | 0.000578394 |
| PPP1R9A | -1 | 0.001 | 0.572844709 | 0.094641728 | 0.000585681 |
| LRRN2 | -1 | 0.001 | 0.569366964 | 0.094641728 | 0.000582567 |
| OGFRL1 | -1 | 0.001 | 0.542834434 | 0.095431649 | 0.000597706 |
| OGDHL | 1 | 0.001 | 1.506439856 | 0.094641728 | 0.000542532 |
| G6PD | 1 | 0.001 | 1.527692381 | 0.094641728 | 0.000539162 |
| TMSB10 | 1 | 0.001 | 1.517112962 | 0.094641728 | 0.000579912 |
| VASP | 1 | 0.001 | 1.553404873 | 0.095431649 | 0.000632006 |
| SEC23A | 1 | 0.001 | 1.563911218 | 0.095431649 | 0.000608307 |
| TMEM86A | 1 | 0.001 | 1.655435111 | 0.094641728 | 0.000568909 |
| ZNF774 | -1 | 0 | 0.714351245 | 0.09802593 | 0.000674027 |
| CLIC6 | -1 | 0 | 0.691206304 | 0.098757456 | 0.000691447 |
| ATL1 | -1 | 0 | 0.686405034 | 0.098757456 | 0.000695989 |
| C3orf23 | -1 | 0 | 0.657672146 | 0.097719921 | 0.000663413 |
| CHKB | -1 | 0 | 0.627745021 | 0.097719921 | 0.000666323 |
| ARMCX6 | -1 | 0 | 0.614708597 | 0.097719921 | 0.000661617 |
| PTPN18 | -1 | 0 | 0.600263531 | 0.098757456 | 0.000701692 |
| ENTPD8 | -1 | 0 | 0.589002346 | 0.098757456 | 0.000696835 |
| MTFR1 | 1 | 0 | 1.470708659 | 0.099147778 | 0.000710146 |

### EXAMPLE 8

### Identification of Metabolic-Like Gene Networks Associated with Breast Cancer Prognosis

IDH2 was identified as a gene that associated with recurrence risk in the "Providence" patient cohort described in Example 1 (see Table 1) but did not belong to either the proliferation or estrogen receptor gene groups of the *Oncotype* DX® Breast Cancer Assay. In fact, IDH2 encodes a key central metabolism gene, isocitrate dehydrogenase 2. It was discovered that IDH2 co-expresses with four other genes (ENO1, TMSB10, PGK1, and G6PD) that also co-express with each other, as show in Table 16. All but TMSB10 have known associations with metabolic pathways.

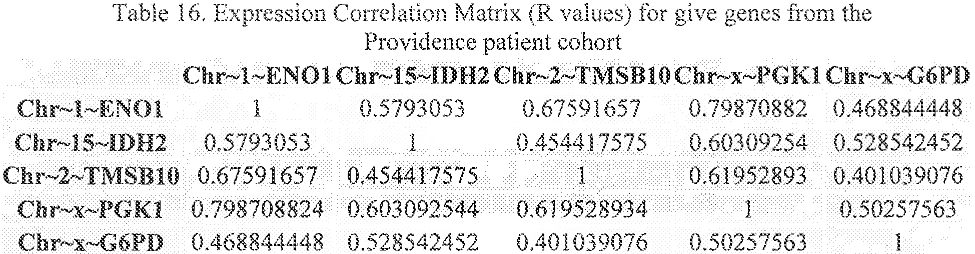

### IDH2

IDH1 encodes isocitrate dehydrogenase 2, which is an NADP(+)-dependent isocitrate dehydrogenase found in the mitochondria. It plays a role in intermediary metabolism and energy production. The protein may tightly associate or interact with the pyruvate dehydrogenase complex.

### ENO1

ENO1 encodes alpha-enolase, one of three enolase isoenzymes found in mammals. Each isoenzyme is a homodimer composed of 2 alpha, 2 gamma, or 2 beta subunits, and functions as a glycolytic enzyme. Alternative splicing of this gene results in a shorter isoform that has been shown to bind to the c-myc promoter and function as a tumor suppressor. Several pseudogenes have been identified, including one on the long arm of chromosome 1. Alpha-enolase has also been identified as an autoantigen in Hashimoto encephalopathy.

### PGK1

The PGK1 gene encodes phosphoglycerate kinase, another glycolytic enzyme, which converts 1,3-diphosphoglycerate to 3-phosphoglycerate. This reaction generates one molecule of adenosine triphosphate (ATP), which is the main energy source in cells.

### G6PD

G6PD encodes glucose-6-phosphate dehydrogenase, a cytosolic enzyme whose main function is to produce NADPH in the pentose phosphate pathway. This pathway generates both energy and molecular building blocks for nucleic acids and aromatic amino acids.

### TMSB10

TMSB10 encodes thymosin beta-10, which plays an important role in the organization of the cytoskeleton. It binds to and sequesters actin monomers (G actin) and therefore inhibits actin polymerization.

The association of these five genes with recurrence rate was explored in the Providence patient cohort (see Example 1), "Rush" patient cohort (see Example 7), and the Netherlands Cancer Institute ("NKI") patient cohort (van de Vijver et al., N. Engl. J. Med. 347:1999-2009, 2002). As shown in Table 17, all five genes independently significantly associated with increased risk of recurrence in all three patient cohorts.

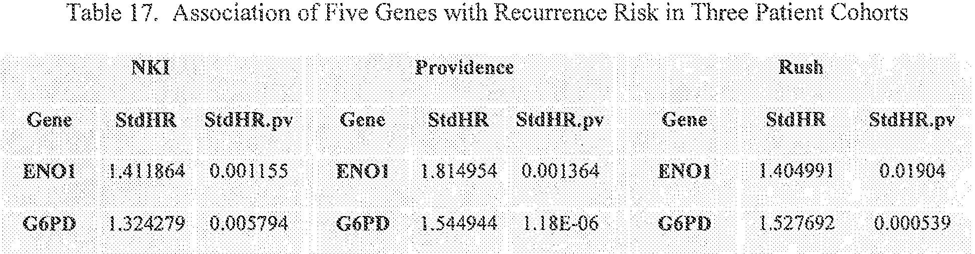

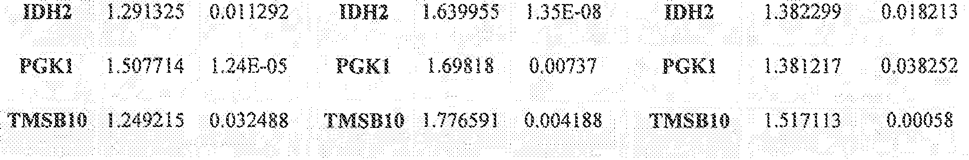

The expression cohesion of the five genes was compared with the cohesion of expression of the proliferation gene group (comprising Ki-67, STK15, SURV, CCNB1, MYBL2) and estrogen receptor gene group (comprising ER, PR, BCL2, SCUBE2) of the *Oncotype* DX® Breast Cancer Assay. , The Pearson R value averages and ranges from the Providence cohort were as follows: five genes: R=0.56 (range 0.48-0.63); proliferation gene group: R=0.67 (range 0.62-0.70); estrogen receptor gene group: R=0.58 (range 0.55-0.62). Moreover, the five genes also co-expressed with the five proliferation genes with a Pearson correlation of R=0.44 (range 0.31-0.60). However, the cohesion of expression of each of the five genes with the five-gene cluster is higher than with the proliferation gene group. This analysis indicates that the five genes do belong to a co-expressed gene module that is approximately as cohesive as the previously defined proliferation and estrogen receptor co-expressed gene modules and that can justifiably be considered a distinct co-expressed gene module. This suggests that inclusion of one or more of the five genes (ENO1, G6PD, IDH2, PGK1, TMSB10) may provide additional prognostic information to the Oncotype DX® Recurrence Score® result.

Because the five gene set described above included at least three genes involved in central metabolism, the results of Table 1 were analyzed to identify additional genes that belong to the glycolysis, the citric acid (TCA) cycle, and the pentose phosphate pathways and that associate with risk of breast cancer recurrence. Fourteen (14) genes were found to have ap<0.005: PGD; TKT; TALD01; G6PD; GPI; SLC1A5; SLC7A5; OGDH; SUCLG1; ENO1; PGK1; IDH2; ACO2; and FBP1. As shown in Table 1, all of these genes except for FBP1, was associated with increased likelihood of cancer recurrence. This latter result is consistent with the fact that the FBP1gene product is anabolic (catalyzes gluconeogenesis), whereas the others are catabolic (generate energy).

The 14 gene set and 5 gene set were subjected to a gene set analysis ("GSA") by the method of Efron and Tibshirani (The Annals of Applied Statistics 1:107-129, 2007), which assesses the significance of pre-defined gene sets, rather than individual genes. The GSA scores for the 14 gene set and 5 gene set were evaluated in the Providence, Rush, and NKI cohorts, and compared to GSA scores of >800 canonical pathway ("CP") gene sets from the larger C2 ("curated gene sets") collection developed at the Broad Institute (*see* Molecular Signatures Database (MgSigDB) v3.0 on the Gene Set Enrichment Analysis website of the Broad (*see also* Subramanian et al. PNAS 102:15545-15550, 2005). The GSA scores, p values, and rank among all the gene sets are shown in Table 18.

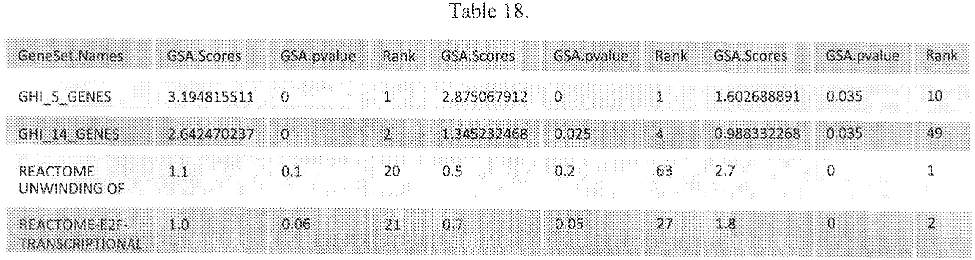

As can be seen from Table 18, the 5 gene set and the 14 gene set both exhibited high GSA scores in all three patient cohorts, as indicated by their ranks among GSA scores for all >800 canonical gene sets. Also, the p values of the 5 gene and 14 gene metabolic gene modules were statistically significant across all three patient cohorts ((p<0.05).

While the present invention has been described with reference to what is considered to be specific embodiments, it is to be understood that the invention is not so limited. To the contrary, the invention is intended to cover various modifications and equivalents included within the spirit and scope of the appended claims.

| Table 1. List of Assembled RefSeq RNAs Associated with Risk of Breast Cancer Recurrence in 136 Breast Cancer Patients | | | | | | | |
|---|---|---|---|---|---|---|---|
| Gene | Accession No. | Direction of Association (1=Higher Expression Means Higher Risk) | p-value | Standardized Hazard Ratio Estimate (95% Confidence Interval) | q-value (FDR) | Maximum Lower Bound Std HR @ 10% FDR | Regression-to-the-Mean-Corrected Std HR |
| KIF1B | NM_183416 | 1 | 5.88E-10 | 2.43 (1.84, 3.23) | <0.1% | 1.29 | 1.66 |
| KIF1B | NM_015074 | 1 | 5.88E-10 | 2.43 (1.84, 3.23) | <0.1% | 1.29 | 1.66 |
| ZFP3 | NM_153018 | -1 | 3.99E-09 | 0.36 (0.26, 0.51) | <0.1% | 1.29 | 1.62 |
| FAM21C | NM_015262 | -1 | 1.20E-10 | 0.51 (0.41, 0.62) | <0.1% | 1.25 | 1.62 |
| FAM21C | NM_001169107 | -1 | 1.20E-10 | 0.51 (0.41, 0.62) | <0.1% | 1.25 | 1.62 |
| FAM21C | NM_001169106 | -1 | 1.20E-10 | 0.51 (0.41, 0.62) | <0.1% | 1.25 | 1.62 |
| FU14186 | NR_037596 | -1 | 4.41E-09 | 0.44 (0.33, 0.58) | <0.1% | 1.25 | 1.61 |
| DAP | NM_004394 | 1 | 1.43E-08 | 2.27 (1.71, 3.02) | <0.1% | 1.24 | 1.59 |
| P2RX4 | NM_002560 | -1 | 2.29E-09 | 0.49 (0.39, 0.62) | <0.1% | 1.24 | 1.6 |
| GGCT | NR_037669 | 1 | 7.24E-11 | 1.68 (1.43, 1.96) | <0.1% | 1.21 | 1.52 |
| GGCT | NM_001199815 | 1 | 7.24E-11 | 1.68 (1.43, 1.96) | <0.1% | 1.21 | 1.52 |
| GGCT | NM_001199817 | 1 | 7.24E-11 | 1.68 (1.43, 1.96) | <0.1% | 1.21 | 1.52 |
| GGCT | NM_001199816 | 1 | 7.24E-11 | 1.68 (1.43, 1.96) | <0.1% | 1.21 | 1.52 |
| GGCT | NM_024051 | 1 | 7.24E-11 | 1.68 (1.43, 1.96) | <0.1% | 1.21 | 1.52 |
| TMEM91 | NM_001098823 | -1 | 2.68E-08 | 0.48 (0.37, 0.62) | <0.1% | 1.21 | 1.56 |
| TMEM91 | NM_001042595 | -1 | 2.68E-08 | 0.48 (0.37, 0.62) | <0.1% | 1.21 | 1.56 |
| TMEM91 | NM_001098821 | -1 | 2.68E-08 | 0.48 (0.37, 0.62) | <0.1% | 1.21 | 1.56 |
| TMEM91 | NM_001098822 | -1 | 2.68E-08 | 0.48 (0.37, 0.62) | <0.1% | 1.21 | 1.56 |
| TMEM91 | NM_001098825 | -1 | 2.68E-08 | 0.48 (0.37, 0.62) | <0.1% | 1.21 | 1.56 |
| TMEM91 | NM_001098824 | -1 | 2.68E-08 | 0.48 (0.37, 0.62) | <0.1% | 1.21 | 1.56 |
| ANKRD27 | NM_032139 | 1 | 2.87E-10 | 1.69 (1.43, 1.99) | <0.1% | 1.2 | 1.52 |
| CDK20 | NM_001170639 | -1 | 1.33E-09 | 0.60 (0.50, 0.70) | <0.1% | 1.19 | 1.5 |
| CDK20 | NM_178432 | -1 | 1.33E-09 | 0.60 (0.50, 0.70) | <0.1% | 1.19 | 1.5 |
| CDK20 | NM_001170640 | -1 | 1.33E-09 | 0.60 (0.50, 0.70) | <0.1% | 1.19 | 1.5 |
| CDK20 | NM_001039803 | -1 | 1.33E-09 | 0.60 (0.50, 0.70) | <0.1% | 1.19 | 1.5 |
| CDK20 | NM_012119 | -1 | 1.33E-09 | 0.60 (0.50, 0.70) | <0.1% | 1.19 | 1.5 |
| GKAP1 | NM_001135953 | -1 | 2.68E-08 | 0.55 (0.45, 0.68) | <0.1% | 1.19 | 1.52 |
| GKAP1 | NM_025211 | -1 | 2.68E-08 | 0.55 (0.45, 0.68) | <0.1% | 1.19 | 1.52 |
| NAA35 | NM_024635 | -1 | 6.53E-07 | 0.43 (0.30, 0.60) | <0.1% | 1.19 | 1.5 |
| OMD | NM_005014 | -1 | 4.64E-07 | 0.41 (0.29, 0.58) | <0.1% | 1.19 | 1.51 |
| OR10H3 | NM_013938 | 1 | 5.26E-09 | 1.78 (1.47, 2.16) | <0.1% | 1.19 | 1.53 |
| TSPAN17 | NM_012171 | 1 | 5.21E-08 | 1.88 (1.50, 2.37) | <0.1% | 1.19 | 1.53 |
| TSPAN17 | NM_130465 | 1 | 5.21E-08 | 1.88 (1.50, 2.37) | <0.1% | 1.19 | 1.53 |
| TSPAN17 | NM_001006616 | 1 | 5.21E-08 | 1.88 (1.50, 2.37) | <0.1% | 1.19 | 1.53 |
| IDH2 | NM_002168 | 1 | 1.34E-08 | 1.64 (1.38, 1.95) | <0.1% | 1.17 | 1.47 |
| KCNV1 | NM_014379 | 1 | 3.04E-08 | 1.74 (1.43, 2.12) | <0.1% | 1.17 | 1.5 |
| SPTAN1 | NM_003127 | -1 | 6.87E-07 | 0.47 (0.34, 0.63) | <0.1% | 1.17 | 1.5 |
| SPTAN1 | NM_001130438 | -1 | 6.87E-07 | 0.47 (0.34, 0.63) | <0.1% | 1.17 | 1.5 |
| SPTAN1 | NM_001195532 | -1 | 6.87E-07 | 0.47 (0.34, 0.63) | <0.1% | 1.17 | 1.5 |
| CANX | NM_001746 | 1 | 1.14E-06 | 2.08 (1.55, 2.80) | <0.1% | 1.16 | 1.49 |
| CANX | NM_001024649 | 1 | 1.14E-06 | 2.08 (1.55, 2.80) | <0.1% | 1.16 | 1.49 |
| CENPP | NM_001012267 | -1 | 1.82E-07 | 0.55 (0.44, 0.69) | <0.1% | 1.16 | 1.49 |
| CYB5D2 | NR_023346 | -1 | 4.13E-07 | 0.55 (0.44, 0.69) | <0.1% | 1.16 | 1.48 |
| CYB5D2 | NR_023347 | -1 | 4.13E-07 | 0.55 (0.44, 0.69) | <0.1% | 1.16 | 1.48 |
| CYB5D2 | NM_144611 | -1 | 4.13E-07 | 0.55 (0.44, 0.69) | <0.1% | 1.16 | 1.48 |
| MMP15 | NM_002428 | 1 | 3.19E-06 | 2.46 (1.68, 3.59) | 0.10% | 1.16 | 1.46 |
| RANBP10 | NM_020850 | 1 | 1.79E-07 | 1.84 (1.46, 2.31) | <0.1% | 1.16 | 1.5 |
| RTN1 | NM_021136 | 1 | 1.15E-06 | 0.47 (0.35, 0.64) | <0.1% | 1.16 | 1.49 |
| RTN1 | NM_206852 | 1 | 1.15E-06 | 0.47 (0.35, 0.64) | <0.1% | 1.16 | 1.49 |
| RTN1 | NM_206857 | -1 | 1.15E-06 | 0.47 (0.35, 0.64) | <0.1% | 1.16 | 1.49 |
| STK32B | NM_018401 | -1 | 9.39E-07 | 0.49 (0.37, 0.65) | <0.1% | 1.16 | 1.49 |
| TKT | NM_01135055 | 1 | 7.52E-07 | 1.94 (1.49, 2.53) | <0.1% | 1.16 | 1.49 |
| TKT | NM_001064 | 1 | 7.52E-07 | 1.94 (1.49, 2.53) | <0.1% | 1.16 | 1.49 |
| TRABD | NM_025204 | 1 | 5.97E-09 | 1.53 (1.32, 1.76) | <0.1% | 1.16 | 1.43 |
| UBXN11 | NM_183008 | 4 | 9.88E-09 | 0.64 (0.55, 0.74) | <0.1% | 1.16 | 1.44 |
| UBXN11 | NM_145345 | -1 | 9.88E-09 | 0.64 (0.55, 0.74) | <0.1% | 1.16 | 1.44 |
| UBXN11 | NM_01077262 | -1 | 9.88E-09 | 0.64 (0.55, 0.74) | <0.1% | 1.16 | 1.44 |
| ULBP3 | NM_024518 | 1 | 1.34E-06 | 2.02 (1.52, 2.69) | <0.1% | 1.16 | 1.49 |
| ADAMTS16 | NM_139056 | -1 | 2.10E-06 | 0.54 (0.42, 0.70) | 0.10% | 1.15 | 1.46 |
| ARMCX1 | NM_016608 | -1 | 8.63E-09 | 0.67 (0.59, 0.77) | <0.1% | 1.15 | 1.4 |
| CCDC68 | NM_001143829 | -1 | 4.63E-06 | 0.44 (0.31, 0.63) | 0.10% | 1.15 | 1.46 |
| CCDC68 | NM_025214 | -1 | 4.63E-06 | 0.44 (0.31, 0.63) | 0.10% | 1.15 | 1.46 |
| CDC25B | NM_004358 | 1 | 6.27E-06 | 2.10 (1.52, 2.91) | 0.10% | 1.15 | 1.45 |
| CDC25B | NM_021873 | 1 | 6.27E-06 | 2.10 (1.52, 2.91) | 0.10% | 1.15 | 1.45 |
| CDC25B | NM_021872 | 1 | 6.27E-06 | 2.10 (1.52, 2.91) | 0.10% | 1.15 | 1.45 |
| GAN | NM_022041 | 1 | 4.35E-06 | 2.18 (1.56, 3.04) | 0.10% | 1.15 | 1.46 |
| GLIS2 | NM_032575 | -1 | 3.86E-07 | 0.57 (0.46, 0.71) | <0.1% | 1.15 | 1.47 |
| GPR126 | NM_198569 | 1 | 5.93E-06 | 2.14 (1.54, 2.98) | 0.10% | 1.15 | 1.45 |
| GPR126 | NM_020455 | 1 | 5.93E-06 | 2.14 (1.54, 2.98) | 0.10% | 1.15 | 1.45 |
| GPR126 | NM_01032395 | 1 | 5.93E-06 | 2.14 (1.54, 2.98) | 0.10% | 1.15 | 1.45 |
| GPR126 | NM_001032394 | 1 | 5.93E-06 | 2.14 (1.54, 2.98) | 0.10% | 1.15 | 1.45 |
| INTU | NM_015693 | -1 | 4.31E-07 | 0.57 (0.46, 0.71) | <0.1% | 1.15 | 1.47 |
| LIPT1 | NM_145197 | -1 | 1.50E-08 | 0.66 (0.57, 0.76) | <0.1% | 1.15 | 1.42 |
| LIPT1 | NM_145196 | -1 | 1.50E-08 | 0.66 (0.57, 0.76) | <0.1% | 1.15 | 1.42 |
| LIPT1 | NM_015929 | -1 | 1.50E-08 | 0.66 (0.57, 0.76) | <0.1% | 1.15 | 1.42 |
| LIPT1 | NM_145198 | -1 | 1.50E-08 | 0.66 (0.57, 0.76) | <0.1% | 1.15 | 1.42 |
| LIPT1 | NM_145199 | -1 | 1.50E-08 | 0.66 (0.57, 0.76) | <0.1% | 1.15 | 1.42 |
| MEGF9 | NM_001080497 | -1 | 5.50E-06 | 0.48 (0.35, 0.66) | 0.10% | 1.15 | 1.45 |
| OR7E24 | NM_001079935 | 1 | 1.56E-08 | 1.50 (1.30, 1.72) | <0.1% | 1.15 | 1.41 |
| PHOSPHO2 | NM_001008489 | -1 | 1.27E-07 | 0.63 (0.54, 0.75) | <0.1% | 1.15 | 1.43 |
| PHOSPHO2 | NM_001199287 | -1 | 1.27E-07 | 0.63 (0.54, 0.75) | <0.1% | 1.15 | 1.43 |
| PHOSPHO2 | NM_001199288 | -1 | 1.27E-07 | 0.63 (0.54, 0.75) | <0.1% | 1.15 | 1.43 |
| PHOSPHO2 | NM_001199285 | -1 | 1.27E-07 | 0.63 (0.54, 0.75) | <0.1% | 1.15 | 1.43 |
| PHOSPHO2 | NM_001199286 | -1 | 1.27E-07 | 0.63 (0.54, 0.75) | <0.1% | 1.15 | 1.43 |
| TEX2 | NM_018469 | 1 | 1.34E-07 | 1.68 (1.38, 2.03) | <0.1% | 1.15 | 1.47 |
| TXNDC16 | NM_020784 | -1 | 2.75E-08 | 0.66 (0.57, 0.77) | <0.1% | 1.15 | 1.41 |
| TXNDC16 | NM_001160047 | -1 | 2.75E-08 | 0.66 (0.57, 0.77) | <0.1% | 1.15 | 1.41 |
| ZNF141 | NM_003441 | -1 | 3.45E-08 | 0.63 (0.54, 0.74) | <0.1% | 1.15 | 1.44 |
| ZNF239 | NM_001099282 | -1 | 9.30E-08 | 0.63 (0.53, 0.74) | <0.1% | 1.15 | 1.44 |
| ZNF239 | NM_005674 | -1 | 9.30E-08 | 0.63 (0.53, 0.74) | <0.1% | 1.15 | 1.44 |
| ZNF239 | NM_001099284 | -1 | 9.30E-08 | 0.63 (0.53, 0.74) | <0.1% | 1.15 | 1.44 |
| ZNF239 | NM_001099283 | -1 | 9.30E-08 | 0.63 (0.53, 0.74) | <0.1% | 1.15 | 1.44 |
| ZNF429 | NM_001001415 | -1 | 5.67E-09 | 0.67 (0.58, 0.77) | <0.1% | 1.15 | 1.41 |
| ACP1 | NM_004300 | 1 | 3.01E-06 | 1.80 (1.41, 2.31) | 0.10% | 1.14 | 1.45 |
| ACP1 | NM_007099 | 1 | 3.01E-06 | 1.80 (1.41, 2.31) | 0.10% | 1.14 | 1.45 |
| ACP1 | NR_024080 | 1 | 3.01E-06 | 1.80 (1.41, 2.31) | 0.10% | 1.14 | 1.45 |
| ACP1 | NM_00140649 | 1 | 3.01E-06 | 1.80 (1.41, 2.31) | 0.10% | 1.14 | 1.45 |
| C17orf96 | NM_001130677 | 1 | 1.84E-06 | 1.69 (1.36, 2.10) | <0.1% | 1.14 | 1.44 |
| C7orf30 | NM_138446 | 1 | 6.82E-06 | 2.08 (1.51, 2.86) | 0.10% | 1.14 | 1.45 |
| CCDC40 | NM_017950 | -1 | 8.80E-06 | 0.50 (0.37, 0.68) | 0.10% | 1.14 | 1.44 |
| CENPN | NM_00110625 | 1 | 1.37E-05 | 2.27 (1.57, 3.29) | 0.20% | 1.14 | 1.43 |
| CENPN | NM_018455 | 1 | 1.37E-05 | 2.27 (1.57, 3.29) | 0.20% | 1.14 | 1.43 |
| CENPN | NM_001100624 | 1 | 1.37E-05 | 2.27 (1.57, 3.29) | 0.20% | 1.14 | 1.43 |
| CLINT1 | NM_014666 | 1 | 6.00E-06 | 1.98 (1.47, 2.66) | 0.10% | 1.14 | 1.45 |
| CLINT1 | NM_001195556 | 1 | 6.00E-06 | 1.98 (1.47, 2.66) | 0.10% | 1.14 | 1.45 |
| CLINT1 | NM_001195555 | 1 | 6.00E-06 | 1.98 (1.47, 2.66) | 0.10% | 1.14 | 1.45 |
| FAM131C | NM_182623 | 1 | 3.59E-06 | 1.87 (1.43, 2.43) | 0.10% | 1.14 | 1.46 |
| FZD9 | NM_00303508 | 1 | 4.24E-07 | 1.60 (1.33, 1.92) | <0.1% | 1.14 | 1.43 |
| GPRIN1 | NM_052899 | 1 | 1.21E-05 | 2.27 (1.57, 3.27) | 0.20% | 1.14 | 1.43 |
| HMGCS1 | NM_002130 | 1 | 2.74E-07 | 1.59 (1.33, 1.89) | <0.1% | 1.14 | 1.43 |
| HMGCS1 | NM_001098272 | 1 | 2.74E-07 | 1.59 (1.33, 1.89) | <0.1% | 1.14 | 1.43 |
| KIF20A | NM_005733 | 1 | 3.69E-05 | 2.62 (1.66, 4.14) | 0.40% | 1.14 | 1.38 |
| KRT83 | NM_002282 | 1 | 6.89E-07 | 1.66 (1.36, 2.04) | <0.1% | 1.14 | 1.45 |
| MEX3B | NM_032246 | 1 | 1.37E-05 | 2.25 (1.56, 3.25) | 0.20% | 1.14 | 1.43 |
| NUDT19 | NM_001105570 | 1 | 7.72E-06 | 2.03 (1.49, 2.77) | 0.10% | 1.14 | 1.45 |
| RNF145 | NM_001199383 | 1 | 6.56E-06 | 1.96 (1.46, 2.62) | 0.10% | 1.14 | 1.45 |
| RNF145 | NM_001199382 | 1 | 6.56E-06 | 1.96 (1.46, 2.62) | 0.10% | 1.14 | 1.45 |
| RNF145 | NM_144726 | 1 | 6.56E-06 | 1.96 (1.46, 2.62) | 0.10% | 1.14 | 1.45 |
| RNF145 | NM_001199381 | 1 | 6.56E-06 | 1.96 (146, 2.62) | 0.10% | 1.14 | 1.45 |
| RNF145 | NM_001199380 | 1 | 6.56E-06 | 1.96 (1.46, 2.62) | 0.10% | 1.14 | 1.45 |
| RPS23 | NM_001025 | -1 | 2.24E-06 | 0.56 (0.44, 0.71) | 0.10% | 1.14 | 1.45 |
| SDAD1 | NM_018115 | 1 | 2.98E-07 | 1.54 (1.31, 1.82) | <0.1% | 1.14 | 1.41 |
| SNAR-G1 | NR_004383 | 1 | 8.18E-07 | 1.59 (1.32, 1.92) | <0.1% | 1.14 | 1.42 |
| TBX4 | NM_018488 | 1 | 1.12E-06 | 1.68 (1.36, 2.06) | <0.1% | 1.14 | 1.44 |
| ULK4 | NM_017886 | -1 | 1.18E-05 | 0.48 (0.34, 0.66) | 0.20% | 1.14 | 1.43 |
| USO1 | NM_003715 | 1 | 1.01E-07 | 1.51 (1.30, 1.76) | <0.1% | 1.14 | 1.4 |
| ZNF214 | NM_013249 | -1 | 9.49E-06 | 0.51 (0.38, 0.68) | 0.10% | 1.14 | 1.44 |
| ZNF540 | NM_152606 | -1 | 4.66E-07 | 0.64 (0.54, 0.76) | <0.1% | 1.14 | 1.41 |
| ZNF540 | NM_001172225 | -1 | 4.66E-07 | 0.64 (0.54, 0.76) | <0.1% | 1.14 | 1.41 |
| ZNF540 | NM_001172226 | -1 | 4.66E-07 | 0.64 (0.54, 0.76) | <0.1% | 1.14 | 1.41 |
| ZNF823 | NM_001080493 | -1 | 1.72E-08 | 0.70 (0.62, 0.79) | <0.1% | 1.14 | 1.37 |
| ADC | NM_052998 | -1 | 4.67E-07 | 0.67 (0.57, 0.78) | <0.1% | 1.13 | 1.38 |
| ADHFE1 | NM_144650 | -1 | 1.40E-05 | 0.53 (0.40, 0.71) | 0.20% | 1.13 | 1.42 |
| C3orf15 | NM_033364 | -1 | 1.71E-05 | 0.47 (0.34, 0.67) | 0.20% | 1.13 | 1.42 |
| CYB5B | NM_030579 | 1 | 4.23E-05 | 2.44 (1.59, 3.73) | 0.40% | 1.13 | 1.39 |
| DCDC5 | NM_020869 | -1 | 3.07E-05 | 0.45 (0.31, 0.65) | 0.30% | 1.13 | 1.4 |
| DNAH6 | NM_001370 | -1 | 6.69E-06 | 0.58 (0.46, 0.73) | 0.10% | 1.13 | 1.42 |
| ECM2 | NM 001393 | -1 | 2.26E-05 | 0.45 (0.31, 0.65) | 0.30% | 1.13 | 1.41 |
| ECM2 | NM_001197295 | -1 | 2.26E-05 | 0.45 (0.31, 0.65) | 0.30% | 1.13 | 1.41 |
| ECM2 | NM_01197296 | -1 | 2.26E-05 | 0.45 (0.31, 0.65) | 0.30% | 1.13 | 1.41 |
| G6PD | NM_000402 | 1 | 1.18E-06 | 1.54 (1.30, 1.84) | <0.1% | 1.13 | 1.4 |
| G6PD | NM_001042351 | 1 | 1.18E-06 | 1.54 (1.30, 1.84) | <0.1% | 1.13 | 1.4 |
| GRHL2 | NM_024915 | 1 | 2.62E-05 | 2.07 (1.47, 2.91) | 0.30% | 1.13 | 1.42 |
| GTSE1 | NM_016426 | 1 | 3.48E-05 | 2.45 (1.60, 3.75) | 0.40% | 1.13 | 1.39 |
| HTR7P1 | NR_002774 | -1 | 1.10E-05 | 0.52 (0.39, 0.69) | 0.20% | 1.13 | 1.43 |
| LOC100133308 | NR_024472 | 1 | 1.27E-06 | 1.54 (1.30, 1.84) | <0.1% | 1.13 | 1.4 |
| LOC400657 | NR_024484 | -1 | 1.20E-08 | 0.72 (0.64, 0.80) | <0.1% | 1.13 | 1.34 |
| MAGEA10 | NM_021048 | 1 | 1.09E-06 | 1.54 (1.30, 1.83) | <0.1% | 1.13 | 1.4 |
| MAGEA10 | NM_001011543 | 1 | 1.09E-06 | 1.54 (1.30, 1.83) | <0.1% | 1.13 | 1.4 |
| MPP6 | NM_016447 | 1 | 1.45E-05 | 2.05 (1.48, 2.83) | 0.20% | 1.13 | 1.43 |
| RAB28 | NM_001017979 | -1 | 4.43E-10 | 0.74 (0.68, 0.82) | <0.1% | 1.13 | 1.31 |
| RAB28 | NM_001159601 | -1 | 4.43E-10 | 0.74 (0.68, 0.82) | <0.1% | 1.13 | 1.31 |
| RAB28 | NM_004249 | -1 | 4.43E-10 | 0.74 (0.68, 0.82) | <0.1% | 1.13 | 1.31 |
| SMURF2 | NM_022739 | 1 | 3.92E-06 | 1.76 (1.38, 2.23) | 0.10% | 1.13 | 1.44 |
| TXNRD1 | NM_182742 | 1 | 2.99E-05 | 2.13 (1.49, 3.03) | 0.30% | 1.13 | 1.41 |
| TXNRD1 | NM_003330 | 1 | 2.99E-05 | 2.13 (1.49, 3.03) | 0.30% | 1.13 | 1.41 |
| TXNRD1 | NM_001093771 | 1 | 2.99E-05 | 2.13 (1.49, 3.03) | 0.30% | 1.13 | 1.41 |
| TXNRD1 | NM_182743 | 1 | 2.99E-05 | 2.13 (1.49, 3.03) | 0.30% | 1.13 | 1.41 |
| TXNRD1 | NM_182729 | 1 | 2.99E-05 | 2.13 (1.49, 3.03) | 0.30% | 1.13 | 1.41 |
| ZNF44 | NM_016264 | -1 | 2.33E-05 | 0.48 (0.34, 0.67) | 0.30% | 1.13 | 1.41 |
| ZNF44 | NM_001164276 | -1 | 2.33E-05 | 0.48 (0.34, 0.67) | 0.30% | 1.13 | 1.41 |
| BBS5 | NM_152384 | -1 | 8.81E-06 | 0.60 (0.47, 0.75) | 0.10% | 1.12 | 1.41 |
| C14orf45 | NM_025057 | -1 | 3.59E-05 | 0.49 (0.35, 0.69) | 0.40% | 1.12 | 1.4 |
| CAMTA2 | NM_001171166 | -1 | 2.25E-05 | 0.53 (0.39, 0.71) | 0.30% | 1.12 | 1.41 |
| CAMTA2 | NM_001171168 | -1 | 2.25E-05 | 0.53 (0.39, 0.71) | 0.30% | 1.12 | 1.41 |
| CAMTA2 | NM_01171167 | -1 | 2.25E-05 | 0.53 (0.39, 0.71) | 0.30% | 1.12 | 1.41 |
| CAMTA2 | NM_015099 | -1 | 2.25E-05 | 0.53 (0.39, 0.71) | 0.30% | 1.12 | 1.41 |
| CRYZ | NM_001130042 | -1 | 3.86E-08 | 0.73 (0.65, 0.81) | <0.1% | 1.12 | 1.33 |
| CRYZ | NM_001130043 | -1 | 3.86E-08 | 0.73 (0.65, 0.81) | <0.1% | 1.12 | 1.33 |
| CRYZ | NM_001889 | -1 | 3.86E-08 | 0.73 (0.65, 0.81) | <0.1% | 1.12 | 1.33 |
| CRYZ | NM_001134759 | -1 | 3.86E-08 | 0.73 (0.65, 0.81) | <0.1% | 1.12 | 1.33 |
| FDX1 | NM_004109 | -1 | 3.82E-06 | 0.63 (0.52, 0.77) | 0.10% | 1.12 | 1.4 |
| G3BP2 | NM_203504 | 1 | 2.29E-06 | 1.54 (1.29, 1.83) | 0.10% | 1.12 | 1.39 |
| G3BP2 | NM_203505 | 1 | 2.29E-06 | 1.54 (1.29, 1.83) | 0.10% | 1.12 | 1.39 |
| G3BP2 | NM_012297 | 1 | 2.29E-06 | 1.54 (1.29, 1.83) | 0.10% | 1.12 | 1.39 |
| GARS | NM_002047 | 1 | 6.42E-06 | 1.67 (1.34, 2.09) | 0.10% | 1.12 | 1.42 |
| HYDIN | NM_032821 | -1 | 1.37E-05 | 0.54 (0.41, 0.71) | 0.20% | 1.12 | 1.42 |
| HYDIN | NM_017558 | -1 | 1.37E-05 | 0.54 (0.41, 0.71) | 0.20% | 1.12 | 1.42 |
| HYDIN | NM_001198542 | -1 | 1.37E-05 | 0.54 (0.41, 0.71) | 0.20% | 1.12 | 1.42 |
| HYDIN | NM_001198543 | -1 | 1.37E-05 | 0.54 (0.41, 0.71) | 0.20% | 1.12 | 1.42 |
| IFT74 | NM_001099222 | -1 | 1.72E-05 | 0.55 (0.42, 0.72) | 0.20% | 1.12 | 1.42 |
| IFT74 | NM_025103 | -1 | 1.72E-05 | 0.55 (0.42, 0.72) | 0.20% | 1.12 | 1.42 |
| IFT74 | NM_01099224 | -1 | 1.72E-05 | 0.55 (0.42, 0.72) | 0.20% | 1.12 | 1.42 |
| IFT74 | NM_001099223 | -1 | 1.72E-05 | 0.55 (0.42, 0.72) | 0.20% | 1.12 | 1.42 |
| KCNJ4 | NM_152868 | 1 | 3.50E-06 | 1.59 (1.31, 1.93) | 0.10% | 1.12 | 1.4 |
| KCNJ4 | NM_004981 | 1 | 3.50E-06 | 1.59 (1.31, 1.93) | 0.10% | 1.12 | 1.4 |
| MAPK6 | NM_002748 | 1 | 3.70E-08 | 1.37 (1.22, 1.53) | <0.1% | 1.12 | 1.32 |
| MYOF | NM_133337 | -1 | 8.78E-06 | 0.60 (0.48, 0.75) | 0.10% | 1.12 | 1.41 |
| MYOF | NM_013451 | -1 | 8.78E-06 | 0.60 (0.48, 0.75) | 0.10% | 1.12 | 1.41 |
| SH3GLB2 | NM_020145 | -1 | 1.89E-05 | 0.52 (0.39, 0.70) | 0.20% | 1.12 | 1.42 |
| TRIM45 | NM_001145635 | -1 | 2.13E-05 | 0.54 (0.40, 0.71) | 0.30% | 1.12 | 1.41 |
| TRIM45 | NM_025188 | -1 | 2.13E-05 | 0.54 (0.40, 0.71) | 0.30% | 1.12 | 1.41 |
| VDAC1 | NM_003374 | 1 | 7.01E-05 | 2.45 (1.57, 3.80) | 0.60% | 1.12 | 1.37 |
| VDAC1 | NR_036625 | 1 | 7.01E-05 | 2.45 (1.57, 3.80) | 0.60% | 1.12 | 1.37 |
| VDAC1 | NR_036624 | 1 | 7.01E-05 | 2.45 (1.57, 3.80) | 0.60% | 1.12 | 1.37 |
| BCL2 | NM_000657 | -1 | 3.28E-05 | 0.54 (0.40, 0.72) | 0.30% | 1.11 | 1.4 |
| BCL2 | NM_000633 | -1 | 3.28E-05 | 0.54 (0.40, 0.72) | 0.30% | 1.11 | 1.4 |
| C9orf3 | NM_001193329 | -1 | 1.59E-05 | 0.60 (0.48, 0.76) | 0.20% | 1.11 | 1.4 |
| C9orf3 | NM_001193331 | -1 | 1.59E-05 | 0.60 (0.48, 0.76) | 0.20% | 1.11 | 1.4 |
| C9orf3 | NM_032823 | -1 | 1.59E-05 | 0.60 (0.48, 0.76) | 0.20% | 1.11 | 1.4 |
| CEP72 | NM_018140 | 1 | 9.55E-05 | 2.27 (1.50, 3.43) | 0.70% | 1.11 | 1.37 |
| DNALI1 | NM_003462 | -1 | 9.88E-06 | 0.63 (0.51, 0.77) | 0.10% | 1.11 | 1.39 |
| ECEL1 | NM_004826 | 1 | 7.48E-06 | 1.54 (1.28, 1.86) | 0.10% | 1.11 | 1.38 |
| FAM179B | NM_015091 | -1 | 6.45E-05 | 0.45 (0.31, 0.67) | 0.50% | 1.11 | 1.38 |
| HEATR2 | NM_017802 | 1 | 2.70E-05 | 1.83 (1.38, 2.43) | 0.30% | 1.11 | 1.41 |
| IRAK4 | NM_001145256 | -1 | 0.000036189 | 0.51 (0.37, 0.70) | 0.40% | 1.11 | 1.4 |
| IRAK4 | NM_001114182 | -1 | 0.000036189 | 0.51 (0.37, 0.70) | 0.40% | 1.11 | 1.4 |
| IRAK4 | NM_001145258 | -1 | 0.000036189 | 0.51 (0.37, 0.70) | 0.40% | 1.11 | 1.4 |
| IRAK4 | NM_ 001145257 | -1 | 0.000036189 | 0.51 (0.37, 0.70) | 0.40% | 1.11 | 1.4 |
| IRAK4 | NM_016123 | -1 | 0.000036189 | 0.51 (0.37, 0.70) | 0.40% | 1.11 | 1.4 |
| LOC147804 | NR_003148 | -1 | 5.76E-05 | 0.51 (0.36, 0.71) | 0.50% | 1.11 | 1.39 |
| LOC645323 | NR_015436 | 1 | 0.000032725 | 1.83 (1.38, 2.43) | 0.30% | 1.11 | 1.41 |
| LOC645323 | NR_024384 | 1 | 0.000032725 | 1.83 (1.38, 2.43) | 0.30% | 1.11 | 1.41 |
| LOC645323 | NR_024383 | 1 | 0.000032725 | 1.83 (1.38, 2.43) | 0.30% | 1.11 | 1.41 |
| LRGUK | NM_144648 | -1 | 5.37E-05 | 0.51 (0.37, 0.71) | 0.50% | 1.11 | 1.39 |
| LRRC46 | NM_0333413 | -1 | 3.76E-05 | 0.51 (0.37, 0.70) | 0.40% | 1.11 | 1.4 |
| NOL8 | NM_ 017948 | -1 | 4.65E-05 | 0.53 (0.39, 0.72) | 0.40% | 1.11 | 1.4 |
| NOL8 | NR_024020 | -1 | 4.65E-05 | 0.53 (0.39, 0.72) | 0.40% | 1.11 | 1.4 |
| OR2J2 | NM_030905 | 1 | 4.20E-06 | 1.53 (1.28, 1.84) | 0.10% | 1.11 | 1.38 |
| PERP | NM_0222121 | 1 | 2.52E-05 | 1.73 (1.34, 2.23) | 0.30% | 1.11 | 1.4 |
| PHB | NM_002634 | 1 | 1.15E-05 | 1.63 (1.31, 2.03) | 0.20% | 1.11 | 1.4 |
| SCRN3 | NM_024583 | -1 | 8.57E-06 | 0.62 (0.50, 0.76) | 0.10% | 1.11 | 1.4 |
| SCRN3 | NM_001193528 | -1 | 8.57E-06 | 0.62 (0.50, 0.76) | 0.10% | 1.11 | 1.4 |
| SLC35D2 | NM_007001 | -1 | 4.26E-05 | 0.53 (0.39, 0.72) | 0.40% | 1.11 | 1.4 |
| SMARCD2 | NM_001098426 | 1 | 6.36E-06 | 1.60 (1.30, 1.96) | 0.10% | 1.11 | 1.4 |
| SPIN1 | NM_006717 | -1 | 0.000050879 | 0.47 (0.32, 0.67) | 0.50% | 1.11 | 1.39 |
| STARD3NL | NM_032016 | 1 | 3.16E-05 | 1.91 (1.41, 2.59) | 0.30% | 1.11 | 1.41 |
| TUBB1 | NM_030773 | -1 | 1.63E-05 | 0.60 (0.48, 0.76) | 0.20% | 1.11 | 1.4 |
| ZNF211 | NM_198855 | -1 | 2.09E-05 | 0.60 (0.47, 0.76) | 0.20% | 1.11 | 1.4 |
| ZNF211 | NM_006385 | -1 | 2.09E-05 | 0.60 (0.47, 0.76) | 0.20% | 1.11 | 1.4 |
| ZNF227 | NM_182490 | -1 | 0.000077689 | 0.47 (0.32, 0.68) | 0.60% | 1.11 | 1.38 |
| ZNF626 | NM_145297 | -1 | 1.76E-05 | 0.58 (0.46, 0.75) | 0.20% | 1.11 | 1.41 |
| ZNF626 | NM_001076675 | -1 | 1.76E-05 | 0.58 (0.46, 0.75) | 0.20% | 1.11 | 1.41 |
| ACAP2 | NM_012287 | 1 | 5.36E-05 | 1.81 (1.36, 2.41) | 0.50% | 1.1 | 1.39 |
| COL9A1 | NM_078485 | 1 | 2.06E-07 | 1.32 (1.19, 1.47) | <0.1% | 1.1 | 1.29 |
| COL9A1 | NM_001851 | 1 | 2.06E-07 | 1.32 (1.19, 1.47) | <0.1% | 1.1 | 1.29 |
| IQCD | NM_138451 | -1 | 6.49E-05 | 0.51 (0.37, 0.71) | 0.50% | 1.1 | 1.39 |
| LOC100131726 | NR_024479 | 1 | 6.29E-05 | 1.98 (1.42, 2.76) | 0.50% | 1.1 | 1.39 |
| LOC401242 | NR_033379 | 1 | 5.07E-06 | 1.44 (1.23, 1.68) | 0.10% | 1.1 | 1.34 |
| MTHFSD | NR_027490 | 1 | 7.01E-05 | 1.87 (1.37, 2.54) | 0.60% | 1.1 | 1.39 |
| MTHFSD | NR_027489 | 1 | 7.01E-05 | 1.87 (1.37, 2.54) | 0.60% | 1.1 | 1.39 |
| MTHFSD | NM_022764 | 1 | 7.01E-05 | 1.87 (1.37, 2.54) | 0.60% | 1.1 | 1.39 |
| MTHFSD | NM_001159379 | 1 | 7.01E-05 | 1.87 (1.37, 2.54) | 0.60% | 1.1 | 1.39 |
| MTHFSD | NM_001159378 | 1 | 7.01E-05 | 1.87 (1.37, 2.54) | 0.60% | 1.1 | 1.39 |
| MTHFSD | NM_001159377 | 1 | 7.01E-05 | 1.87 (1.37, 2.54) | 0.60% | 1.1 | 1.39 |
| MTHFSD | NM_001159380 | 1 | 7.01E-05 | 1.87 (1.37, 2.54) | 0.60% | 1.1 | 1.39 |
| NCRNA00200 | NR_015376 | 1 | 1.02E-06 | 1.37 (1.21, 1.55) | <0.1% | 1.1 | 1.31 |
| NMNAT3 | NM_178177 | -1 | 6.81E-05 | 0.52 (0.38, 0.72) | 0.60% | 1.1 | 1.39 |
| RAI2 | NM_021785 | -1 | 6.18E-05 | 0.52 (0.37, 0.71) | 0.50% | 1.1 | 1.39 |
| RAI2 | NR_033348 | -1 | 6.18E-05 | 0.52 (0.37, 0.71) | 0.50% | 1.1 | 1.39 |
| RAI2 | NR_033349 | -1 | 6.18E-05 | 0.52 (0.37, 0.71) | 0.50% | 1.1 | 1.39 |
| RAI2 | NM_001172732 | -1 | 6.18E-05 | 0.52 (0.37, 0.71) | 0.50% | 1.1 | 1.39 |
| RAI2 | NM_001172739 | -1 | 6.18E-05 | 0.52 (0.37, 0.71) | 0.50% | 1.1 | 1.39 |
| RAI2 | NM_001172743 | -1 | 6.18E-05 | 0.52 (0.37, 0.71) | 0.50% | 1.1 | 1.39 |
| RCHY1 | NM_001009922 | 1 | 5.97E-06 | 1.45 (1.23, 1.70) | 0.10% | 1.1 | 1.35 |
| RCHY1 | NM_015436 | 1 | 5.97E-06 | 1.45 (1.23, 1.70) | 0.10% | 1.1 | 1.35 |
| RCHY1 | NM_01008925 | 1 | 5.97E-06 | 1.45 (1.23, 1.70) | 0.10% | 1.1 | 1.35 |
| SI | NM_001041 | 1 | 1.46E-08 | 1.27 (1.17, 1.38) | <0.1% | 1.1 | 1.25 |
| SNORD18B | NR_002442 | 1 | 1.73E-05 | 1.55 (1.27, 1.90) | 0.20% | 1.1 | 1.38 |
| TMOD3 | NM_014547 | 1 | 3.74E-07 | 1.32 (1.19, 1.47) | <0.1% | 1.1 | 1.29 |
| UGT2B7 | NM_001074 | 1 | 8.45E-06 | 1.49 (1.25, 1.77) | 0.10% | 1.1 | 1.36 |
| ZNF433 | NM_001080411 | -1 | 5.49E-05 | 0.53 (0.39, 0.72) | 0.50% | 1.1 | 1.39 |
| AACSP1 | NR_024035 | 1 | 1.38E-05 | 1.46 (1.23, 1.73) | 0.20% | 1.09 | 1.35 |
| AKD1 | NM_001145128 | -1 | 1.23E-05 | 0.70 (0.60, 0.82) | 0.20% | 1.09 | 1.33 |
| AKD1 | NM_145025 | -1 | 1.23E-05 | 0.70 (0.60, 0.82) | 0.20% | 1.09 | 1.33 |
| ANKFN1 | NM_153228 | 1 | 0.000106619 | 1.73 (1.31, 2.28) | 0.80% | 1.09 | 1.37 |
| ANKRDS4 | NR_036556 | 1 | 0.000155771 | 1.98 (1.39, 2.82) | 1.00% | 1.09 | 1.37 |
| ANKRDS4 | NM_138797 | 1 | 0.000155771 | 1.98 (1.39, 2.82) | 1.00% | 1.09 | 1.37 |
| ASB13 | NM_024701 | -1 | 4.43E-06 | 0.72 (0.62, 0.83) | 0.10% | 1.09 | 1.32 |
| ASB13 | NR_037164 | -1 | 4.43E-06 | 0.72 (0.62, 0.83) | 0.10% | 1.09 | 1.32 |
| ASB13 | NR_024581 | -1 | 4.43E-06 | 0.72 (0.62, 0.83) | 0.10% | 1.09 | 1.32 |
| C17orf64 | NM_181707 | 1 | 4.20E-06 | 1.38 (1.20, 1.58) | 0.10% | 1.09 | 1.31 |
| C17orf68 | NM_025099 | -1 | 0.000123317 | 0.52 (0.38, 0.73) | 0.90% | 1.09 | 1.37 |
| C5 | NM_001735 | -1 | 0.00013293 | 0.55 (0.40, 0.75) | 0.90% | 1.09 | 1.37 |
| C7orf72 | NM_001161834 | 1 | 4.05E-07 | 1.30 (1.17, 1.43) | <0.1% | 1.09 | 1.27 |
| CASC1 | NM_018272 | -1 | 8.53E-05 | 0.58 (0.44, 0.76) | 0.70% | 1.09 | 1.38 |
| CASC1 | NM_001082972 | -1 | 8.53E-05 | 0.58 (0.44, 0.76) | 0.70% | 1.09 | 1.38 |
| CASC1 | NM_001082973 | -1 | 8.53E-05 | 0.58 (0.44, 0.76) | 0.70% | 1.09 | 1.38 |
| CCDC47 | NM_020198 | 1 | 2.73E-05 | 1.57 (1.27, 1.94) | 0.30% | 1.09 | 1.37 |
| CCDC66 | NM_001141947 | -1 | 9.67E-05 | 0.54 (0.40, 0.74) | 0.70% | 1.09 | 1.38 |
| CCDC66 | NR_024460 | -1 | 9.67E-05 | 0.54 (0.40, 0.74) | 0.70% | 1.09 | 1.38 |
| CCDC66 | NM_001012506 | -1 | 9.67E-05 | 0.54 (0.40, 0.74) | 0.70% | 1.09 | 1.38 |
| CCNA2 | NM_001237 | 1 | 0.000135444 | 2.06 (1.42, 3.00) | 0.90% | 1.09 | 1.37 |
| COL20A1 | NM_020882 | 1 | 1.40E-05 | 1.45 (1.23, 1.71) | 0.20% | 1.09 | 1.34 |
| DDIT4 | NM_019058 | 1 | 0.000150778 | 1.93 (1.37, 2.71) | 1.00% | 1.09 | 1.37 |
| DSCR10 | NR_027695 | 1 | 1.43E-05 | 1.44 (1.22, 1.70) | 0.20% | 1.09 | 1.34 |
| EIF3B | NM_003751 | 1 | 0.000111832 | 1.82 (1.34, 2.48) | 0.80% | 1.09 | 1.38 |
| EIF3B | NM_001037283 | 1 | 0.000111832 | 1.82 (1.34, 2.48) | 0.80% | 1.09 | 1.38 |
| EIF4G2 | NM_001418 | 1 | 5.03E-05 | 1.66 (1.30, 2.12) | 0.50% | 1.09 | 1.38 |
| EIF4G2 | NM_001172705 | 1 | 5.03E-05 | 1.66 (1.30, 2.12) | 0.50% | 1.09 | 1.38 |
| EIF4G2 | NM_001042559 | 1 | 5.03E-05 | 1.66 (1.30, 2.12) | 0.50% | 1.09 | 1.38 |
| EPS15L1 | NM_021235 | -1 | 8.57E-05 | 0.53 (0.38, 0.72) | 0.70% | 1.09 | 1.38 |
| FAM18A | NM_001079512 | -1 | 0.000040247 | 0.61 (0.48, 0.77) | 0.40% | 1.09 | 1.38 |
| FAM9C | NM_174901 | 1 | 1.14E-06 | 1.30 (1.17, 1.44) | <0.1% | 1.09 | 1.27 |
| FU45244 | NR_015415 | -1 | 0.000114635 | 0.55 (0.41, 0.75) | 0.80% | 1.09 | 1.37 |
| FTMT | NM_177478 | 1 | 3.09E-05 | 1.50 (1.24, 1.81) | 0.30% | 1.09 | 1.35 |
| GBX2 | NM_01485 | 1 | 1.64E-05 | 1.44 (1.22, 1.70) | 0.20% | 1.09 | 1.33 |
| GOLT1B | NM_016072 | 1 | 3.70E-05 | 1.57 (1.27, 1.94) | 0.40% | 1.09 | 1.37 |
| GPR56 | NM_001145774 | 1 | 0.000188458 | 2.06 (1.41, 3.02) | 1.20% | 1.09 | 1.36 |
| GPR56 | NM_201524 | 1 | 0.000188458 | 2.06 (1.41, 3.02) | 1.20% | 1.09 | 1.36 |
| GPR56 | NM_005682 | 1 | 0.000188458 | 2.06 (1.41, 3.02) | 1.20% | 1.09 | 1.36 |
| GPR56 | NM_001145771 | 1 | 0.000188458 | 2.06 (1.41, 3.02) | 1.20% | 1.09 | 1.36 |
| GPR56 | NM_001145770 | 1 | 0.000188458 | 2.06 (1.41, 3.02) | 1.20% | 1.09 | 1.36 |
| GPR56 | NM_001145773 | 1 | 0.000188458 | 2.06 (1.41, 3.02) | 1.20% | 1.09 | 1.36 |
| GPR56 | NM_001145772 | 1 | 0.000188458 | 2.06 (1.41, 3.02) | 1.20% | 1.09 | 1.36 |
| GPR56 | NM_201525 | 1 | 0.000188458 | 2.06 (1.41, 3.02) | 1.20% | 1.09 | 1.36 |
| HEBP1 | NM_015987 | -1 | 0.000177204 | 0.50 (0.35, 0.72) | 1.10% | 1.09 | 1.36 |
| HLA-DOA | NM_002119 | -1 | 3.94E-05 | 0.60 (0.47, 0.77) | 0.40% | 1.09 | 1.38 |
| HRASLS5 | NM_054108 | -1 | 0.000093912 | 0.54 (0.40, 0.74) | 0.70% | 1.09 | 1.38 |
| HRASLS5 | NM_001146728 | -1 | 0.000093912 | 0.54 (0.40, 0.74) | 0.70% | 1.09 | 1.38 |
| HRASLS5 | NM_001146729 | -1 | 0.000093912 | 0.54 (0.40, 0.74) | 0.70% | 1.09 | 1.38 |
| JAK1 | NM_002227 | -1 | 0.000193139 | 0.46 (0.31, 0.69) | 1.20% | 1.09 | 1.35 |
| KMO | NM_03679 | 1 | 0.000149756 | 2.05 (1.42, 2.98) | 1.00% | 1.09 | 1.37 |
| KRT78 | NM_173352 | 1 | 2.66E-05 | 1.48 (1.23, 1.78) | 0.30% | 1.09 | 1.35 |
| LDLRAD3 | NM_174902 | -1 | 4.34E-05 | 0.64 (0.51, 0.79) | 0.40% | 1.09 | 1.36 |
| MAP3K14 | NM_003954 | -1 | 9.19E-05 | 0.54 (0.40, 0.74) | 0.70% | 1.09 | 1.38 |
| MIR196A1 | NR_029582 | 1 | 2.40E-06 | 1.36 (1.19, 1.54) | 0.10% | 1.09 | 1.3 |
| MIR4275 | NR_036237 | 1 | 6.24E-06 | 1.43 (1.22, 1.66) | 0.10% | 1.09 | 1.34 |
| NCRNA00221 | NR_027457 | 1 | 5.62E-06 | 1.38 (1.20, 1.58) | 0.10% | 1.09 | 1.31 |
| NGRN | NM_001033088 | 1 | 2.38E-05 | 1.47 (1.23, 1.76) | 0.30% | 1.09 | 1.34 |
| NGRN | NR_028052 | 1 | 2.38E-05 | 1.47 (1.23, 1.76) | 0.30% | 1.09 | 1.34 |
| NME1 | NM_000269 | 1 | 7.11E-05 | 1.83 (1.36, 2.46) | 0.60% | 1.09 | 1.39 |
| NME1 | NM_198175 | 1 | 7.11E-05 | 1.83 (1.36, 2.46) | 0.60% | 1.09 | 1.39 |
| NSUN2 | NM_001193455 | 1 | 3.78E-05 | 1.61 (1.29, 2.03) | 0.40% | 1.09 | 1.38 |
| NSUN2 | NM_017755 | 1 | 3.78E-05 | 1.61 (1.29, 2.03) | 0.40% | 1.09 | 1.38 |
| OR7G3 | NM_001001958 | 1 | 6.32E-06 | 1.36 (1.19, 1.56) | 0.10% | 1.09 | 1.3 |
| PIGA | NM_002641 | 1 | 0.000138341 | 2.13 (1.44, 3.14) | 0.90% | 1.09 | 1.36 |
| PIGA | NM_020473 | 1 | 0.000138341 | 2.13 (1.44, 3.14) | 0.90% | 1.09 | 1.36 |
| PIGA | NR_033835 | 1 | 0.000138341 | 2.13 (1.44, 3.14) | 0.90% | 1.09 | 1.36 |
| PIGA | NR_033836 | 1 | 0.000138341 | 2.13 (1.44, 3.14) | 0.90% | 1.09 | 1.36 |
| PRR11 | NR_018304 | 1 | 0.000134896 | 2.14 (1.45, 3.16) | 0.90% | 1.09 | 1.36 |
| PSMC5 | NM_002805 | 1 | 3.01E-05 | 1.62 (1.29, 2.02) | 0.30% | 1.09 | 1.38 |
| PSMC5 | NM_001199163 | 1 | 3.01E-05 | 1.62 (1.29, 2.02) | 0.30% | 1.09 | 1.38 |
| RDH10 | NM_172037 | 1 | 0.000090299 | 1.81 (1.35, 2.44) | 0.70% | 1.09 | 1.38 |
| RFWD3 | NM_018124 | 1 | 0.000103613 | 1.85 (1.35, 2.51) | 0.80% | 1.09 | 1.38 |
| RILPL2 | NM_145058 | -1 | 4.52E-05 | 0.61 (0.48, 0.77) | 0.40% | 1.09 | 1.38 |
| ROBO1 | NM_002941 | 1 | 6.22E-05 | 1.66 (1.29, 2.12) | 0.50% | 1.09 | 1.38 |
| ROBO1 | NM_001145845 | 1 | 6.22E-05 | 1.66 (1.29, 2.12) | 0.50% | 1.09 | 1.38 |
| ROBO1 | NM_133631 | 1 | 6.22E-05 | 1.66 (1.29, 2.12) | 0.50% | 1.09 | 1.38 |
| SEPT3 | NM_019106 | 1 | 0.000202646 | 2.23 (1.46, 3.41) | 1.20% | 1.09 | 1.35 |
| SEPT3 | NM_145733 | 1 | 0.000202646 | 2.23 (1.46, 3.41) | 1.20% | 1.09 | 1.35 |
| SF3B3 | NM_012426 | 1 | 8.05E-05 | 1.86 (1.37, 2.53) | 0.60% | 1.09 | 1.39 |
| SKINTL | NR_026749 | -1 | 0.000155637 | 0.47 (0.32, 0.70) | 1.00% | 1.09 | 1.36 |
| SLC1A5 | NM_001145145 | 1 | 0.000173422 | 2.23 (1.47, 3.38) | 1.10% | 1.09 | 1.35 |
| SLC1A5 | NM_005628 | 1 | 0.000173422 | 2.23 (1.47, 3.38) | 1.10% | 1.09 | 1.35 |
| SLC1A5 | NM_001145144 | 1 | 0.000173422 | 2.23 (1.47, 3.38) | 1.10% | 1.09 | 1.35 |
| SUN1 | NM_001171945 | 1 | 3.24E-05 | 1.56 (1.27, 1.93) | 0.30% | 1.09 | 1.37 |
| SUN1 | NM_001171946 | 1 | 3.24E-05 | 1.56 (1.27, 1.93) | 0.30% | 1.09 | 1.37 |
| SUN1 | NM_001171944 | 1 | 3.24E-05 | 1.56 (1.27, 1.93) | 0.30% | 1.09 | 1.37 |
| SUN1 | NM_025154 | 1 | 3.24E-05 | 1.56 (1.27, 1.93) | 0.30% | 1.09 | 1.37 |
| SUN1 | NM_001130965 | 1 | 3.24E-05 | 1.56 (1.27, 1.93) | 0.30% | 1.09 | 1.37 |
| TCEA3 | NM_003196 | -1 | 1.33E-05 | 0.67 (0.56, 0.80) | 0.20% | 1.09 | 1.36 |
| TIGD7 | NM_033208 | -1 | 7.22E-05 | 0.60 (0.46, 0.77) | 0.60% | 1.09 | 1.37 |
| TMED3 | NM_007364 | 1 | 0.000118078 | 1.84 (1.35, 2.50) | 0.80% | 1.09 | 1.38 |
| UPK1A | NM_007000 | 1 | 3.33E-05 | 1.52 (1.25, 1.86) | 0.30% | 1.09 | 1.36 |
| ZNF554 | NM_001102651 | -1 | 1.43E-05 | 0.67 (0.56, 0.80) | 0.20% | 1.09 | 1.36 |
| ZZEF1 | NM_015113 | -1 | 0.000101352 | 0.51 (0.36, 0.71) | 0.70% | 1.09 | 1.38 |
| ANKRD17 | NM_198889 | 1 | 4.07E-05 | 1.48 (1.23, 1.79) | 0.40% | 1.08 | 1.34 |
| ANKRD17 | NM_032217 | 1 | 4.07E-05 | 1.48 (1.23, 1.79) | 0.40% | 1.08 | 1.34 |
| C1orf168 | NM_001004303 | -1 | 0.000196331 | 0.52 (0.36, 0.73) | 1.20% | 1.08 | 1.36 |
| CCDC103 | NM_213607 | -1 | 0.000156354 | 0.54 (0.39, 0.74) | 1.00% | 1.08 | 1.36 |
| DBNDD1 | NM_ 001042610 | 1 | 0.000265114 | 2.29 (1.47, 3.59) | 1.40% | 1.08 | 1.33 |
| DBNDD1 | NM_024043 | 1 | 0.000265114 | 2.29 (1.47, 3.59) | 1.40% | 1.08 | 1.33 |
| EBF4 | NM_001110514 | -1 | 0.000177638 | 0.56 (0.42, 0.76) | 1.10% | 1.08 | 1.36 |
| EVX1 | NM_001989 | 1 | 4.40E-05 | 1.46 (1.22, 1.75) | 0.40% | 1.08 | 1.33 |
| FAM120AOS | NM_198841 | -1 | 0.000311715 | 0.48 (0.32, 0.71) | 1.50% | 1.08 | 1.33 |
| FYTTD1 | NM_032288 | 1 | 0.000093964 | 1.62 (1.27, 2.06) | 0.70% | 1.08 | 1.36 |
| FYTTD1 | NR_ 027840 | 1 | 0.000093964 | 1.62 (1.27, 2.06) | 0.70% | 1.08 | 1.36 |
| FYTTD1 | NM_001011537 | 1 | 0.000093964 | 1.62 (1.27, 2.06) | 0.70% | 1.08 | 1.36 |
| GOLGA1 | NM_002077 | -1 | 0.000103896 | 0.62 (0.48, 0.79) | 0.80% | 1.08 | 1.36 |
| GPI | NM_001184722 | 1 | 3.17E-05 | 1.46 (1.22, 1.75) | 0.30% | 1.08 | 1.34 |
| GPI | NM_000175 | 1 | 3.17E-05 | 1.46 (1.22, 1.75) | 0.30% | 1.08 | 1.34 |
| GPR157 | NM_024980 | 1 | 0.000371571 | 2.47 (1.50, 4.07) | 1.70% | 1.08 | 1.31 |
| GSTM3 | NR_.024537 | -1 | 0.000174236 | 0.54 (0.39, 0.74) | 1.10% | 1.08 | 1.36 |
| GSTM3 | NM_000849 | -1 | 0.000174236 | 0.54 (0.39, 0.74) | 1.10% | 1.08 | 1.36 |
| GUK1 | NM_000858 | 1 | 0.000160068 | 1.84 (1.34, 2.53) | 1.00% | 1.08 | 1.37 |
| GUK1 | NM_001159391 | 1 | 0.000160068 | 1.84 (1.34, 2.53) | 1.00% | 1.08 | 1.37 |
| GUK1 | NM_001159390 | 1 | 0.000160068 | 1.84 (1.34, 2.53) | 1.00% | 1.08 | 1.37 |
| IGF2BP2 | NM_001007225 | 1 | 0.000223003 | 1.98 (1.38, 2.85) | 1.30% | 1.08 | 1.36 |
| IGF2BP2 | NM_006548 | 1 | 0.000223003 | 1.98 (1.38, 2.85) | 1.30% | 1.08 | 1.36 |
| KDM4B | NM_015015 | -1 | 0.000289488 | 0.49 (0.33, 0.72) | 1.50% | 1.08 | 1.34 |
| KIAA0226 | NM_014687 | 1 | 0.00010158 | 1.66 (1.29, 2.15) | 0.70% | 1.08 | 1.37 |
| KIAA0226 | NM_001145642 | 1 | 0.00010158 | 1.66 (1.29, 2.15) | 0.70% | 1.08 | 1.37 |
| KRT81 | NM_002281 | 1 | 0.000191877 | 1.79 (1.32, 2.43) | 1.20% | 1.08 | 1.36 |
| LYPD6B | NM_177964 | -1 | 0.000135793 | 0.60 (0.46,0.78) | 0.90% | 1.08 | 1.36 |
| MAPT | NM_001123066 | -1 | 0.000216473 | 0.56 (0.41, 0.76) | 1.20% | 1.08 | 1.35 |
| MAPT | NM_001123067 | -1 | 0.000216473 | 0.56 (0.41, 0.76) | 1.20% | 1.08 | 1.35 |
| MAPT | NM_016835 | -1 | 0.000216473 | 0.56 (0.41, 0.76) | 1.20% | 1.08 | 1.35 |
| MAPT | NM_005910 | -1 | 0.000216473 | 0.56 (0.41, 0.76) | 1.20% | 1.08 | 1.35 |
| MAPT | NM_016841 | -1 | 0.000216473 | 0.56 (0.41, 0.76) | 1.20% | 1.08 | 1.35 |
| MAPT | NM_016834 | -1 | 0.000216473 | 0.56 (0.41, 0.76) | 1.20% | 1.08 | 1.35 |
| MIR3170 | NR_036129 | 1 | 1.03E-05 | 1.38 (1.19, 1.59) | 0.20% | 1.08 | 1.31 |
| MIR4267 | NR_036225 | 1 | 7.93E-06 | 1.33 (1.17, 1.51) | 0.10% | 1.08 | 1.28 |
| NDUFS6 | NM_004553 | 1 | 0.000254346 | 1.91 (1.35, 2.70) | 1.40% | 1.08 | 1.35 |
| NPY1R | NM_000909 | -1 | 0.00017825 | 0.55 (0.41, 0.75) | 1.10% | 1.08 | 1.36 |
| NRIP3 | NM_020645 | -1 | 0.000247739 | 0.50 (0.34, 0.72) | 1.30% | 1.08 | 1.35 |
| NUDT8 | NM_181843 | 1 | 7.84E-05 | 1.53 (1.24, 1.90) | 0.60% | 1.08 | 1.35 |
| OPALIN | NM_033207 | 1 | 3.68E-05 | 1.43 (1.21, 1.69) | 0.40% | 1.08 | 1.32 |
| OPALIN | NM_001040102 | 1 | 3.68E-05 | 1.43 (1.21, 1.69) | 0.40% | 1.08 | 1.32 |
| OPALIN | NM_001040103 | 1 | 3.68E-05 | 1.43 (1.21, 1.69) | 0.40% | 1.08 | 1.32 |
| PABPC1L2B | NM_001042506 | 1 | 9.64E-07 | 1.24 (1.14, 1.36) | <0.1% | 1.08 | 1.23 |
| PDE6B | NM_001145291 | -1 | 0.000100399 | 0.59 (0.46, 0.77) | 0.70% | 1.08 | 1.37 |
| PDE6B | NM_001145292 | -1 | 0.000100399 | 0.59 (0.46, 0.77) | 0.70% | 1.08 | 1.37 |
| PDE6B | NM_000283 | -1 | 0.000100399 | 0.59 (0.46, 0.77) | 0.70% | 1.08 | 1.37 |
| PRR7 | NM_030567 | 1 | 0.000274525 | 2.20 (1.44, 3.37) | 1.40% | 1.08 | 1.34 |
| PRR7 | NM_001174101 | 1 | 0.000274525 | 2.20 (1.44, 3.37) | 1.40% | 1.08 | 1.34 |
| PRR7 | NM_001174102 | 1 | 0.000274525 | 2.20 (1.44, 3.37) | 1.40% | 1.08 | 1.34 |
| PTP4A1 | NM_003463 | 1 | 2.66E-05 | 1.45 (1.22, 1.73) | 0.30% | 1.08 | 1.34 |
| RGS22 | NM_015668 | -1 | 0.000336901 | 0.44 (0.28, 0.69) | 1.60% | 1.08 | 1.32 |
| SECISBP2L | NM_014701 | 1 | 0.000213295 | 1.98 (1.38, 2.84) | 1.20% | 1.08 | 1.36 |
| SECISBP2L | NM_001193489 | 1 | 0.000213295 | 1.98 (1.38, 2.84) | 1.20% | 1.08 | 1.36 |
| SFXN1 | NM_022754 | 1 | 0.000256108 | 1.89 (1.34, 2.66) | 1.40% | 1.08 | 1.35 |
| SLC27A1 | NM_198580 | -1 | 0.00014439 | 0.59 (0.45, 0.78) | 1.00% | 1.08 | 1.36 |
| SLC35C1 | NM_018389 | 1 | 0.000285265 | 1.97 (1.37, 2.84) | 1.50% | 1.08 | 1.35 |
| SLC35C1 | NM_001145265 | 1 | 0.000285265 | 1.97 (1.37, 2.84) | 1.50% | 1.08 | 1.35 |
| SLC35C1 | NM_001145266 | 1 | 0.000285265 | 1.97 (1.37, 2.84) | 1.50% | 1.08 | 1.35 |
| SP8 | NM_182700 | 1 | 6.71E-06 | 1.31 (1.17, 1.48) | 0.10% | 1.08 | 1.27 |
| SP8 | NM_198956 | 1 | 6.71E-06 | 1.31 (1.17, 1.48) | 0.10% | 1.08 | 1.27 |
| TBRG4 | NM_004749 | 1 | 8.91E-05 | 1.54 (1.24, 1.90) | 0.70% | 1.08 | 1.35 |
| TBRG4 | NM_199122 | 1 | 8.91E-05 | 1.54 (1.24, 1.90) | 0.70% | 1.08 | 1.35 |
| TBRG4 | NM_030900 | 1 | 8.91E-05 | 1.54 (1.24, 1.90) | 0.70% | 1.08 | 1.35 |
| TFRC | NM_003234 | 1 | 0.000294668 | 2.08 (1.40, 3.09) | 1.50% | 1.08 | 1.34 |
| TFRC | NM_001128148 | 1 | 0.000294668 | 2.08 (1.40, 3.09) | 1.50% | 1.08 | 1.34 |
| USP39 | NM_006590 | 1 | 0.000298617 | 2.18 (1.43, 3.33) | 1.50% | 1.08 | 1.34 |
| WDR19 | NM_025132 | -1 | 0.000213805 | 0.53 (0.38, 0.74) | 1.20% | 1.08 | 1.35 |
| YWHAZ | NM_001135699 | 1 | 0.000160718 | 1.78 (1.32, 2.41) | 1.00% | 1.08 | 1.37 |
| YWHAZ | NM_001135701 | 1 | 0.000160718 | 1.78 (1.32, 2.41) | 1.00% | 1.08 | 1.37 |
| YWHAZ | NM_001135702 | 1 | 0.000160718 | 1.78 (1.32, 2.41) | 1.00% | 1.08 | 1.37 |
| YWHAZ | NM_001135700 | 1 | 0.000160718 | 1.78 (1.32, 2.41) | 1.00% | 1.08 | 1.37 |
| YWHAZ | NM_145690 | 1 | 0.000160718 | 1.78 (1.32, 2.41) | 1.00% | 1.08 | 1.37 |
| YWHAZ | NM_003406 | 1 | 0.000160718 | 1.78 (1.32, 2.41) | 1.00% | 1.08 | 1.37 |
| ZBED4 | NM_ 014838 | 1 | 0.000018032 | 1.37 (1.19, 1.59) | 0.20% | 1.08 | 1.3 |
| ZBTB26 | NM_020924 | -1 | 0.000088049 | 0.65 (0.52, 0.80) | 0.70% | 1.08 | 1.35 |
| ZNF174 | NM_001032292 | -1 | 0.00021639 | 0.48 (0.32, 0.71) | 1.20% | 1.08 | 1.35 |
| ZNF174 | NM_003450 | -1 | 0.00021639 | 0.48 (0.32, 0.71) | 1.20% | 1.08 | 1.35 |
| ZNF440 | NM_152357 | -1 | 0.000254996 | 0.53 (0.37, 0.74) | 1.40% | 1.08 | 1.35 |
| ZNF441 | NM_152355 | -1 | 0.000204868 | 0.55 (0.40, 0.75) | 1.20% | 1.08 | 1.36 |
| ZNF563 | NM_145276 | -1 | 7.39E-05 | 0.62 (0.49, 0.78) | 0.60% | 1.08 | 1.36 |
| ZNF763 | NM_001012753 | -1 | 0.00014465 | 0.57 (0.42, 0.76) | 1.00% | 1.08 | 1.36 |
| ZNF843 | NM_001136509 | -1 | 0.00031279 | 0.45 (0.29, 0.70) | 1.50% | 1.08 | 1.33 |
| ZNF844 | NM_001136501 | -1 | 0.000119899 | 0.58 (0.44, 0.76) | 0.80% | 1.08 | 1.37 |
| ANKRD30A | NM_052997 | -1 | 0.00042874 | 0.52 (0.36, 0.75) | 1.90% | 1.07 | 1.33 |
| AP1G1 | NM_001128 | 1 | 0.000237846 | 1.75 (1.30, 2.37) | 1.30% | 1.07 | 1.35 |
| AP1G1 | NM_001030007 | 1 | 0.000237846 | 1.75 (1.30, 2.37) | 1.30% | 1.07 | 1.35 |
| BRD1 | NM_014577 | 1 | 4.25E-05 | 1.40 (1.19, 1.65) | 0.40% | 1.07 | 1.31 |
| BUD13 | NM_032725 | -1 | 0.00033707 | 0.53 (0.38, 0.75) | 1.60% | 1.07 | 1.34 |
| BUD13 | NM_001159736 | -1 | 0.00033707 | 0.53 (0.38, 0.75) | 1.60% | 1.07 | 1.34 |
| BZRAP1 | NM_004758 | -1 | 0.000315545 | 0.54 (0.39, 0.76) | 1.50% | 1.07 | 1.34 |
| BZRAP1 | NM_024418 | -1 | 0.000315545 | 0.54 (0.39, 0.76) | 1.50% | 1.07 | 1.34 |
| C17orf91 | NR_028505 | -1 | 0.000246829 | 0.60 (0.46, 0.79) | 1.30% | 1.07 | 1.34 |
| C17orf91 | NR_028504 | -1 | 0.000246829 | 0.60 (0.46, 0.79) | 1.30% | 1.07 | 1.34 |
| C17orf91 | NR_028502 | -1 | 0.000246829 | 0.60 (0.46, 0.79) | 1.30% | 1.07 | 1.34 |
| C17orf91 | NR_028503 | -1 | 0.000246829 | 0.60 (0.46, 0.79) | 1.30% | 1.07 | 1.34 |
| C1orf220 | NR_033186 | -1 | 0.000206926 | 0.63 (0.50, 0.81) | 1.20% | 1.07 | 1.34 |
| C1orf51 | NM_144697 | -1 | 0.000164444 | 0.60 (0.46, 0.78) | 1.00% | 1.07 | 1.35 |
| C2orf65 | NM_138804 | -1 | 0.000242505 | 0.57 (0.42, 0.77) | 1.30% | 1.07 | 1.35 |
| C9orf27 | NR_024032 | 1 | 3.24E-06 | 1.26 (1.15, 1.40) | 0.10% | 1.07 | 1.24 |
| C9orf64 | NM_032307 | -1 | 0.000258832 | 0.57 (0.42, 0.77) | 1.40% | 1.07 | 1.35 |
| CACNA1F | NM_005183 | -1 | 0.000196901 | 0.59 (0.45, 0.78) | 1.20% | 1.07 | 1.35 |
| CBX3 | NM_007276 | 1 | 0.00012881 | 1.53 (1.23, 1.90) | 0.90% | 1.07 | 1.34 |
| CBX3 | NM_016587 | 1 | 0.00012881 | 1.53 (1.23, 1.90) | 0.90% | 1.07 | 1.34 |
| CLIC6 | NM_53277 | -1 | 0.000469076 | 0.47 (0.30, 0.72) | 2.00% | 1.07 | 1.32 |
| COX6B1 | NM_001863 | 1 | 7.59E-05 | 1.45 (1.21, 1.74) | 0.60% | 1.07 | 1.32 |
| CWF19L2 | NM_152434 | -1 | 0.000485073 | 0.49 (0.33, 0.73) | 2.00% | 1.07 | 1.32 |
| CX3CR1 | NM_01171171 | -1 | 0.000471876 | 0.48 (0.32, 0.72) | 2.00% | 1.07 | 1.32 |
| CX3CR1 | NM_001171172 | -1 | 0.000471876 | 0.48 (0.32, 0.72) | 2.00% | 1.07 | 1.32 |
| CX3CR1 | NM_01171174 | -1 | 0.000471876 | 0.48 (0.32, 0.72) | 2.00% | 1.07 | 1.32 |
| CX3CR1 | NM_001337 | -1 | 0.000471876 | 0.48 (0.32, 0.72) | 2.00% | 1.07 | 1.32 |
| DCDC1 | NM_181807 | -1 | 0.000241354 | 0.55 (0.40, 0.76) | 1.30% | 1.07 | 1.35 |
| DET1 | NM_07996 | -1 | 5.20E-05 | 0.73 (0.63, 0.85) | 0.50% | 1.07 | 1.29 |
| DET1 | NM_01144074 | -1 | 5.20E-05 | 0.73 (0.63, 0.85) | 0.50% | 1.07 | 1.29 |
| DET1 | NR_026645 | -1 | 5.20E-05 | 0.73 (0.63, 0.85) | 0.50% | 1.07 | 1.29 |
| EGLN1 | NM_022051 | 1 | 0.000408781 | 1.87 (1.32, 2.64) | 1.80% | 1.07 | 1.34 |
| EXO1 | NM_03686 | 1 | 0.000583818 | 2.40 (1.46, 3.96) | 2.30% | 1.07 | 1.3 |
| EXO1 | NM_006027 | 1 | 0.000583818 | 2.40 (1.46, 3.96) | 2.30% | 1.07 | 1.3 |
| EXO1 | NM_130398 | 1 | 0.000583818 | 2.40 (1.46, 3.96) | 2.30% | 1.07 | 1.3 |
| FAM92A1 | NM_145269 | 1 | 0.000422905 | 1.96 (1.35, 2.84) | 1.90% | 1.07 | 1.34 |
| FARP2 | NM_014808 | -1 | 0.000339695 | 0.56 (0.40, 0.77) | 1.60% | 1.07 | 1.34 |
| FCGBP | NM_003890 | -1 | 0.000347245 | 0.57 (0.42, 0.77) | 1.60% | 1.07 | 1.34 |
| FTSJ3 | NM_017647 | 1 | 0.000174103 | 1.54 (1.23, 1.93) | 1.10% | 1.07 | 1.34 |
| GFPT1 | NM_002056 | 1 | 0.000294139 | 1.66 (1.26, 2.19) | 1.50% | 1.07 | 1.34 |
| GMCL1L | NR_003281 | 1 | 0.000277335 | 1.72 (1.28, 2.31) | 1.40% | 1.07 | 1.35 |
| GPX5 | NM_003996 | 1 | 0.00018895 | 1.58 (1.24, 2.02) | 1.20% | 1.07 | 1.34 |
| GPX5 | NM_001509 | 1 | 0.00018895 | 1.58 (1.24, 2.02) | 1.20% | 1.07 | 1.34 |
| HIST1H2AJ | NM_021066 | 1 | 0.000389048 | 2.18 (1.42, 3.35) | 1.80% | 1.07 | 1.33 |
| INTS1 | NM_001080453 | 1 | 0.000236705 | 1.60 (1.25, 2.06) | 1.30% | 1.07 | 1.34 |
| LOC284023 | NR_024349 | -1 | 6.57E-05 | 0.69 (0.57, 0.83) | 0.50% | 1.07 | 1.33 |
| LOC729966 | NR_036575 | 1 | 3.17E-05 | 1.34 (1.17, 1.54) | 0.30% | 1.07 | 1.28 |
| MIR1208 | NR_031613 | 1 | 0.000232308 | 1.72 (1.29, 2.30) | 1.30% | 1.07 | 1.35 |
| MR1 | NM_001194999 | -1 | 0.000294622 | 0.54 (0.39, 0.75) | 1.50% | 1.07 | 1.35 |
| MR1 | NM_001531 | -1 | 0.000294622 | 0.54 (0.39, 0.75) | 1.50% | 1.07 | 1.35 |
| MR1 | NM_001195035 | -1 | 0.000294622 | 0.54 (0.39, 0.75) | 1.50% | 1.07 | 1.35 |
| MR1 | NM_001195000 | -1 | 0.000294622 | 0.54 (0.39, 0.75) | 1.50% | 1.07 | 1.35 |
| NEGR1 | NM_173808 | -1 | 0.000194753 | 0.58 (0.44, 0.77) | 1.20% | 1.07 | 1.35 |
| NT5C2 | NM_012229 | 1 | 0.000251004 | 1.78 (1.31, 2.43) | 1.40% | 1.07 | 1.35 |
| NT5C2 | NM_001134373 | 1 | 0.000251004 | 1.78 (1.31, 2.43) | 1.40% | 1.07 | 1.35 |
| ONECUT2 | NM_004852 | 1 | 0.000300598 | 1.90 (1.34, 2.69) | 1.50% | 1.07 | 1.35 |
| OR52E8 | NM_001005168 | 1 | 0.000183129 | 1.64 (1.27, 2.12) | 1.10% | 1.07 | 1.35 |
| OR9K2 | NM_001005243 | 1 | 0.000023276 | 1.32 (1.16, 1.49) | 0.30% | 1.07 | 1.27 |
| PADI3 | NM_016233 | 1 | 0.000184726 | 1.59 (1.25, 2.02) | 1.10% | 1.07 | 1.35 |
| PION | NM_017439 | -1 | 0.000214273 | 0.58 (0.44, 0.77) | 1.20% | 1.07 | 1.35 |
| PLXNB2 | NM_012401 | 1 | 0.000187459 | 1.55 (1.23, 1.94) | 1.20% | 1.07 | 1.34 |
| SCARB2 | NM_005506 | 1 | 9.94E-05 | 1.44 (1.20, 1.73) | 0.70% | 1.07 | 1.32 |
| SERPINF2 | NM_001165920 | -1 | 0.000337351 | 0.56 (0.41, 0.77) | 1.60% | 1.07 | 1.34 |
| SERPINF2 | NM_000934 | -1 | 0.000337351 | 0.56 (0.41, 0.77) | 1.60% | 1.07 | 1.34 |
| SERPINF2 | NM_001165921 | -1 | 0.000337351 | 0.56 (0.41, 0.77) | 1.60% | 1.07 | 1.34 |
| SHCBP1 | NM_024745 | 1 | 0.000474183 | 2.26 (1.43, 3.58) | 2.00% | 1.07 | 1.31 |
| SLC3A2 | NM_002394 | 1 | 0.000205239 | 1.75 (1.30, 2.35) | 1.20% | 1.07 | 1.36 |
| SLC3A2 | NM_001012662 | 1 | 0.000205239 | 1.75 (1.30, 2.35) | 1.20% | 1.07 | 1.36 |
| SLC3A2 | NM_001012664 | 1 | 0.000205239 | 1.75 (1.30, 2.35) | 1.20% | 1.07 | 1.36 |
| SLC3A2 | NM_001012663 | 1 | 0.000205239 | 1.75 (1.30, 2.35) | 1.20% | 1.07 | 1.36 |
| SLC3A2 | NM_001013251 | 1 | 0.000205239 | 1.75 (1.30, 2.35) | 1.20% | 1.07 | 1.36 |
| SLC3A2 | NN_001012661 | 1 | 0.000205239 | 1.75 (1.30, 2.35) | 1.20% | 1.07 | 1.36 |
| SLC3A2 | NR_037193 | 1 | 0.000205239 | 1.75 (1.30, 2.35) | 1.20% | 1.07 | 1.36 |
| SMARCC1 | NM_003074 | 1 | 0.000285871 | 1.83 (1.32, 2.53) | 1.50% | 1.07 | 1.35 |
| STK11IP | NM_052902 | -1 | 0.00030755 | 0.58 (0.43, 0.78) | 1.50% | 1.07 | 1.34 |
| SUSD3 | NM_145006 | -1 | 0.000203286 | 0.58 (0.43, 0.77) | 1.20% | 1.07 | 1.35 |
| TBCEL | NM_152715 | -1 | 0.000110632 | 0.64 (0.51, 0.80) | 0.80% | 1.07 | 1.35 |
| TBCEL | NM_001130047 | -1 | 0.000110632 | 0.64 (0.51, 0.80) | 0.80% | 1.07 | 1.35 |
| TMEM151A | NM_153266 | 1 | 0.000109154 | 1.53 (1.23, 1.90) | 0.80% | 1.07 | 1.34 |
| TMEM155 | NM_152399 | 1 | 0.000285542 | 1.81 (1.31, 2.49) | 1.50% | 1.07 | 1.35 |
| TMEM40 | NM_018306 | 1 | 0.000431802 | 2.05 (1.37, 3.05) | 1.90% | 1.07 | 1.33 |
| TMOD2 | NM_014548 | 1 | 0.000120829 | 1.50 (1.22, 1.84) | 0.80% | 1.07 | 1.33 |
| TMOD2 | NM_001142885 | 1 | 0.000120829 | 1.50 (1.22, 1.84) | 0.80% | 1.07 | 1.33 |
| TTC12 | NM_017868 | -1 | 0.000472605 | 0.45 (0.29, 0.70) | 2.00% | 1.07 | 1.31 |
| ZDBF2 | NM_020923 | -1 | 0.00032201 | 0.55 (0.40, 0.76) | 1.60% | 1.07 | 1.34 |
| ZNF326 | NM_182976 | -1 | 0.000367128 | 0.53 (0.37, 0.75) | 1.70% | 1.07 | 1.34 |
| ZNF326 | NM_182975 | -1 | 0.000367128 | 0.53 (0.37, 0.75) | 1.70% | 1.07 | 1.34 |
| ZNF484 | NM_001007101 | -1 | 0.000298688 | 0.52 (0.36, 0.74) | 1.50% | 1.07 | 1.34 |
| ZNF484 | NM_031486 | -1 | 0.000298688 | 0.52 (0.36, 0.74) | 1.50% | 1.07 | 1.34 |
| AARS | NM_001605 | 1 | 0.00039842 | 1.78 (1.29, 2.46) | 1.80% | 1.06 | 1.34 |
| ACCN5 | NM_017419 | 1 | 0.000363129 | 1.64 (1.25, 2.16) | 1.70% | 1.06 | 1.34 |
| ALG3 | NM_005787 | 1 | 0.000546417 | 1.96 (1.34, 2.87) | 2.20% | 1.06 | 1.33 |
| ALG3 | NR_024534 | 1 | 0.000546417 | 1.96 (1.34, 2.87) | 2.20% | 1.06 | 1.33 |
| ALG3 | NR_024533 | 1 | 0.000546417 | 1.96 (1.34, 2.87) | 2.20% | 1.06 | 1.33 |
| ALG3 | NM_001006941 | 1 | 0.000546417 | 1.96 (1.34, 2.87) | 2.20% | 1.06 | 1.33 |
| ANLN | NM_018685 | 1 | 0.000861767 | 2.34 (1.42, 3.86) | 2.90% | 1.06 | 1.29 |
| BARHL2 | NM_020063 | 1 | 9.67E-05 | 1.33 (1.15, 1.53) | 0.70% | 1.06 | 1.27 |
| BBC3 | NM_014417 | -1 | 0.000431133 | 0.58 (0.43, 0.79) | 1.90% | 1.06 | 1.33 |
| BBC3 | NM_001127240 | -1 | 0.000431133 | 0.58 (0.43, 0.79) | 1.90% | 1.06 | 1.33 |
| BBC3 | NM_001127242 | -1 | 0.000431133 | 0.58 (0.43, 0.79) | 1.90% | 1.06 | 1.33 |
| BBC3 | NM_001127241 | -1 | 0.000431133 | 0.58 (0.43, 0.79) | 1.90% | 1.06 | 1.33 |
| C17orf97 | NM_001013672 | -1 | 0.000476721 | 0.56 (0.41, 0.78) | 2.00% | 1.06 | 1.33 |
| C7orf40 | NR_003697 | 1 | 0.000244871 | 1.48 (1.20, 1.83) | 1.30% | 1.06 | 1.32 |
| CCDC45 | NM_138363 | 1 | 0.000315377 | 1.62 (1.25, 2.11) | 1.50% | 1.06 | 1.34 |
| CCT5 | NM_012073 | 1 | 0.000579782 | 1.92 (1.33, 2.79) | 2.20% | 1.06 | 1.33 |
| CFL1 | NM_005507 | 1 | 0.00041237 | 1.62 (1.24, 2.11) | 1.80% | 1.06 | 1.33 |
| CROCC | NM_014675 | -1 | 0.000303212 | 0.61 (0.46, 0.80) | 1.50% | 1.06 | 1.34 |
| DCAF16 | NM_017741 | -1 | 0.000539706 | 0.51 (0.35, 0.75) | 2.10% | 1.06 | 1.32 |
| DUS3L | NM_020175 | -1 | 0.000318893 | 0.59 (0.44, 0.79) | 1.50% | 1.06 | 1.34 |
| DUS3L | NM_001161619 | -1 | 0.000318893 | 0.59 (0.44, 0.79) | 1.50% | 1.06 | 1.34 |
| EFCAB6 | NM_022785 | -1 | 0.000411749 | 0.62 (0.48, 0.81) | 1.80% | 1.06 | 1.33 |
| EFCAB6 | NM_198856 | -1 | 0.000411749 | 0.62 (0.48, 0.81) | 1.80% | 1.06 | 1.33 |
| F9 | NM_000133 | 1 | 4.73E-05 | 1.28 (1.14, 1.44) | 0.40% | 1.06 | 1.24 |
| FAM83D | NM_030919 | 1 | 0.000660782 | 2.17 (1.39, 3.40) | 2.50% | 1.06 | 1.31 |
| FANK1 | NM_145235 | -1 | 0.000363661 | 0.60 (0.45, 0.79) | 1.70% | 1.06 | 1.34 |
| FSD2 | NM_001007122 | -1 | 0.000370322 | 0.61 (0.47, 0.80) | 1.70% | 1.06 | 1.33 |
| GOLPH3 | NM_022130 | 1 | 0.000490776 | 1.86 (1.31, 2.64) | 2.00% | 1.06 | 1.33 |
| GPAA1 | NM_003801 | 1 | 0.000555873 | 1.76 (1.28, 2.42) | 2.20% | 1.06 | 1.33 |
| HBZ | NM_005332 | 1 | 0.000192085 | 1.49 (1.21, 1.83) | 1.20% | 1.06 | 1.32 |
| HOXA13 | NM_000522 | 1 | 0.000245127 | 1.43 (1.18, 1.74) | 1.30% | 1.06 | 1.31 |
| HSD3B7 | NM_01142778 | -1 | 0.00023229 | 0.66 (0.53, 0.82) | 1.30% | 1.06 | 1.33 |
| HSD3B7 | NM_001142777 | -1 | 0.00023229 | 0.66 (0.53, 0.82) | 1.30% | 1.06 | 1.33 |
| HSD3B7 | NM_025193 | -1 | 0.00023229 | 0.66 (0.53, 0.82) | 1.30% | 1.06 | 1.33 |
| IFI27L1 | NM_206949 | -1 | 0.00033531 | 0.61 (0.47, 0.80) | 1.60% | 1.06 | 1.34 |
| IFI27L1 | NM_145249 | -1 | 0.00033531 | 0.61 (0.47, 0.80) | 1.60% | 1.06 | 1.34 |
| IGF2BP1 | NM_006546 | 1 | 0.000397318 | 1.75 (1.28, 2.38) | 1.80% | 1.06 | 1.34 |
| IGF2BP1 | NM_01160423 | 1 | 0.000397318 | 1.75 (1.28, 2.38) | 1.80% | 1.06 | 1.34 |
| KIAA0430 | NM_001184998 | -1 | 0.000703574 | 0.50 (0.34, 0.75) | 2.50% | 1.06 | 1.31 |
| KIAA0430 | NM_14647 | -1 | 0.000703574 | 0.50 (0.34, 0.75) | 2.50% | 1.06 | 1.31 |
| KIAA0430 | NM_001184999 | -1 | 0.000703574 | 0.50 (0.34, 0.75) | 2.50% | 1.06 | 1.31 |
| KIAA1274 | NM_014431 | -1 | 0.00041306 | 0.61 (0.47, 0.80) | 1.80% | 1.06 | 1.33 |
| KIAA1407 | NM_020817 | -1 | 0.0004264 | 0.61 (0.47, 0.81) | 1.90% | 1.06 | 1.33 |
| KPNA2 | NM_002266 | 1 | 0.000608282 | 1.88 (1.31, 2.70) | 2.30% | 1.06 | 1.33 |
| LOC100130148 | NR_024560 | -1 | 0.000334314 | 0.59 (0.44, 0.79) | 1.60% | 1.06 | 1.34 |
| LOC144742 | NR_024246 | 1 | 0.00015428 | 1.41 (1.18, 1.69) | 1.00% | 1.06 | 1.3 |
| LOC642006 | NR_030766 | 1 | 0.000338823 | 1.61 (1.24, 2.08) | 1.60% | 1.06 | 1.34 |
| LRIG3 | NM_001136051 | 1 | 0.000531484 | 1.70 (1.26, 2.30) | 2.10% | 1.06 | 1.33 |
| LRIG3 | NM_153377 | 1 | 0.000531484 | 1.70 (1.26, 2.30) | 2.10% | 1.06 | 1.33 |
| LRIT2 | NM_001017924 | 1 | 8.60E-05 | 1.34 (1.16, 1.54) | 0.70% | 1.06 | 1.27 |
| LYRM5 | NM_001001660 | -1 | 0.00030197 | 0.67 (0.54, 0.83) | 1.50% | 1.06 | 1.32 |
| MEIS3P1 | NR_002211 | -1 | 0.000277359 | 0.66 (0.53, 0.83) | 1.40% | 1.06 | 1.32 |
| METTL7B | NM_152637 | 1 | 0.000534187 | 1.78 (1.28, 2.47) | 2.10% | 1.06 | 1.33 |
| MIR133A1 | NR_029675 | 1 | 0.000236845 | 1.54 (1.22, 1.93) | 1.30% | 1.06 | 1.33 |
| MIR4256 | NR_036210 | 1 | 0.000511214 | 1.72 (1.27, 2.33) | 2.10% | 1.06 | 1.33 |
| MIR4258 | NR_036212 | 1 | 0.000755224 | 2.19 (1.39, 3.46) | 2.70% | 1.06 | 1.3 |
| NEK5 | NM_199289 | -1 | 0.000362458 | 0.65 (0.51, 0.82) | 1.70% | 1.06 | 1.32 |
| NHLRC4 | NM_176677 | -1 | 0.000605306 | 0.55 (0.39, 0.77) | 2.30% | 1.06 | 1.32 |
| NPM3 | NM_006993 | 1 | 0.000857222 | 2.57 (1.47, 4.46) | 2.90% | 1.06 | 1.27 |
| P4HB | NM_000918 | 1 | 0.000519801 | 1.78 (1.29, 2.47) | 2.10% | 1.06 | 1.33 |
| PACRGL | NM_001130727 | -1 | 0.000137922 | 0.70 (0.58, 0.84) | 0.90% | 1.06 | 1.31 |
| PACRGL | NM_145048 | -1 | 0.000137922 | 0.70 (0.58, 0.84) | 0.90% | 1.06 | 1.31 |
| PAPD7 | NM_006999 | 1 | 0.000569528 | 1.84 (1.30, 2.60) | 2.20% | 1.06 | 1.33 |
| PAPD7 | NM_001171806 | 1 | 0.000569528 | 1.84 (1.30, 2.60) | 2.20% | 1.06 | 1.33 |
| PAPD7 | NM_001171805 | 1 | 0.000569528 | 1.84 (1.30, 2.60) | 2.20% | 1.06 | 1.33 |
| PCGF3 | NM_006315 | -1 | 0.000375263 | 0.57 (0.42, 0.78) | 1.70% | 1.06 | 1.34 |
| PIM3 | NM_001001852 | 1 | 0.000223066 | 1.45 (1.19, 1.77) | 1.30% | 1.06 | 1.31 |
| POLK | NM_016218 | -1 | 0.000694544 | 0.51 (0.34, 0.75) | 2.50% | 1.06 | 1.31 |
| PPPDE1 | NM_016076 | 1 | 0.000485656 | 1.95 (1.34, 2.83) | 2.00% | 1.06 | 1.33 |
| PYGO1 | NM_015617 | -1 | 0.000513314 | 0.56 (0.40, 0.77) | 2.10% | 1.06 | 1.33 |
| RASGRP4 | NM_001146205 | -1 | 0.000328822 | 0.60 (0.45, 0.79) | 1.60% | 1.06 | 1.34 |
| RASGRP4 | NM_170604 | -1 | 0.000328822 | 0.60 (0.45, 0.79) | 1.60% | 1.06 | 1.34 |
| RASGRP4 | NM_001146203 | -1 | 0.000328822 | 0.60 (0.45, 0.79) | 1.60% | 1.06 | 1.34 |
| RASGRP4 | NM_001146206 | -1 | 0.000328822 | 0.60 (0.45, 0.79) | 1.60% | 1.06 | 1.34 |
| RASGRP4 | NM_001146204 | -1 | 0.000328822 | 0.60 (0.45, 0.79) | 1.60% | 1.06 | 1.34 |
| RASGRP4 | NM_001146207 | -1 | 0.000328822 | 0.60 (0.45, 0.79) | 1.60% | 1.06 | 1.34 |
| RASGRP4 | NM_001146202 | -1 | 0.000328822 | 0.60 (0.45, 0.79) | 1.60% | 1.06 | 1.34 |
| RHCG | NM_016321 | 1 | 0.000306566 | 1.62 (1.25, 2.10) | 1.50% | 1.06 | 1.34 |
| RP1-177G6-2 | NR_028345 | 1 | 0.000135136 | 1.36 (1.16, 1.58) | 0.90% | 1.06 | 1.28 |
| RP1-177G6-2 | NR_028344 | 1 | 0.000135136 | 1.36 (1.16, 1.58) | 0.90% | 1.06 | 1.28 |
| RPP21 | NM_001199120 | 1 | 0.000396352 | 1.66 (1.26, 2.21) | 1.80% | 1.06 | 1.34 |
| RPP21 | NM_001199121 | 1 | 0.000396352 | 1.66 (1.26, 2.21) | 1.80% | 1.06 | 1.34 |
| RPP21 | NM_024839 | 1 | 0.000396352 | 1.66 (1.26, 2.21) | 1.80% | 1.06 | 1.34 |
| SCUBE2 | NM_001170690 | -1 | 0.000697526 | 0.49 (0.32, 0.74) | 2.50% | 1.06 | 1.31 |
| SCUBE2 | NM_020974 | -1 | 0.000697526 | 0.49 (0.32, 0.74) | 2.50% | 1.06 | 1.31 |
| SEC61A1 | NM_013336 | 1 | 0.000689339 | 1.92 (1.32, 2.79) | 2.50% | 1.06 | 1.32 |
| SENP5 | NM_152699 | 1 | 0.000451373 | 1.64 (1.24, 2.15) | 1.90% | 1.06 | 1.33 |
| SGOL1 | NM_001012411 | 1 | 0.000679983 | 2.16 (1.38, 3.36) | 2.50% | 1.06 | 1.31 |
| SGOL1 | NM_001199253 | 1 | 0.000679983 | 2.16 (1.38, 3.36) | 2.50% | 1.06 | 1.31 |
| SGOL1 | NM_001199252 | 1 | 0.000679983 | 2.16 (1.38, 3.36) | 2.50% | 1.06 | 1.31 |
| SGOL1 | NM_001199251 | 1 | 0.000679983 | 2.16 (1.38, 3.36) | 2.50% | 1.06 | 1.31 |
| SGOL1 | NM_001012412 | 1 | 0.000679983 | 2.16 (1.38, 3.36) | 2.50% | 1.06 | 1.31 |
| SGOL1 | NM_001012409 | 1 | 0.000679983 | 2.16 (1.38, 3.36) | 2.50% | 1.06 | 1.31 |
| SGOL1 | NM_001199254 | 1 | 0.000679983 | 2.16 (1.38, 3.36) | 2.50% | 1.06 | 1.31 |
| SGOL1 | NM_001012410 | 1 | 0.000679983 | 2.16 (1.38, 3.36) | 2.50% | 1.06 | 1.31 |
| SGOL1 | NM_138484 | 1 | 0.000679983 | 2.16 (1.38, 3.36) | 2.50% | 1.06 | 1.31 |
| SGOL1 | NM_001199256 | 1 | 0.000679983 | 2.16 (1.38, 3.36) | 2.50% | 1.06 | 1.31 |
| SGOL1 | NM_001199257 | 1 | 0.000679983 | 2.16 (1.38, 3.36) | 2.50% | 1.06 | 1.31 |
| SGOL1 | NM_001012413 | 1 | 0.000679983 | 2.16 (1.38, 3.36) | 2.50% | 1.06 | 1.31 |
| SGOL1 | NM_001199255 | 1 | 0.000679983 | 2.16 (1.38, 3.36) | 2.50% | 1.06 | 1.31 |
| SIN3B | NM_015260 | -1 | 0.00042699 | 0.56 (0.41, 0.77) | 1.90% | 1.06 | 1.34 |
| SMG6 | NM_017575 | -1 | 0.000476372 | 0.54 (0.38, 0.76) | 2.00% | 1.06 | 1.33 |
| SMG6 | NM_001170957 | -1 | 0.000476372 | 0.54 (0.38, 0.76) | 2.00% | 1.06 | 1.33 |
| STAT5A | NM_003152 | -1 | 0.000296856 | 0.61 (0.46, 0.80) | 1.50% | 1.06 | 1.34 |
| STX6 | NM_005819 | 1 | 0.000513875 | 1.78 (1.29, 2.46) | 2.10% | 1.06 | 1.33 |
| STX8 | NR_033656 | -1 | 0.000363379 | 0.60 (0.45, 0.80) | 1.70% | 1.06 | 1.34 |
| STX8 | NM_004853 | -1 | 0.000363379 | 0.60 (0.45, 0.80) | 1.70% | 1.06 | 1.34 |
| SYNPO2L | NM_024875 | -1 | 0.000517753 | 0.50 (0.34, 0.74) | 2.10% | 1.06 | 1.32 |
| SYNPO2L | NM_001114133 | -1 | 0.000517753 | 0.50 (0.34, 0.74) | 2.10% | 1.06 | 1.32 |
| TMEM101 | NM_032376 | -1 | 0.000487894 | 0.51 (0.35, 0.75) | 2.00% | 1.06 | 1.33 |
| TNFRSF14 | NM_003820 | -1 | 0.000653957 | 0.49 (0.32, 0.74) | 2.50% | 1.06 | 1.31 |
| UBE2C | NM_181799 | 1 | 0.000783989 | 2.51 (1.47, 4.30) | 2.70% | 1.06 | 1.28 |
| UBE2C | NM_181800 | 1 | 0.000783989 | 2.51 (1.47, 4.30) | 2.70% | 1.06 | 1.28 |
| UBE2C | NM_181803 | 1 | 0.000783989 | 2.51 (1.47, 4.30) | 2.70% | 1.06 | 1.28 |
| UBE2C | NM_007019 | 1 | 0.000783989 | 2.51 (1.47, 4.30) | 2.70% | 1.06 | 1.28 |
| UBE2C | NM_181802 | 1 | 0.000783989 | 2.51 (1.47, 4.30) | 2.70% | 1.06 | 1.28 |
| UBE2C | NM_181801 | 1 | 0.000783989 | 2.51 (1.47, 4.30) | 2.70% | 1.06 | 1.28 |
| ZNF287 | NM_020653 | -1 | 0.000326457 | 0.64 (0.51, 0.82) | 1.60% | 1.06 | 1.33 |
| ZNF576 | NM_024327 | -1 | 0.000481181 | 0.57 (0.42, 0.78) | 2.00% | 1.06 | 1.33 |
| ZNF576 | NM_001145347 | -1 | 0.000481181 | 0.57 (0.42, 0.78) | 2.00% | 1.06 | 1.33 |
| ZNF738 | NR_027130 | -1 | 0.000598215 | 0.55 (0.39, 0.77) | 2.30% | 1.06 | 1.32 |
| ABCC10 | NM_033450 | 1 | 0.000517309 | 1.57 (1.22, 2.03) | 2.10% | 1.05 | 1.32 |
| ABCC10 | NM_001198934 | 1 | 0.000517309 | 1.57 (1.22, 2.03) | 2.10% | 1.05 | 1.32 |
| ACTR3C | NM_01164458 | -1 | 0.000635228 | 0.64 (0.50, 0.83) | 2.40% | 1.05 | 1.31 |
| ACTR3C | NM_001164459 | -1 | 0.000635228 | 0.64 (0.50, 0.83) | 2.40% | 1.05 | 1.31 |
| AFM | NM_001133 | 1 | 0.000066704 | 1.23 (1.11, 1.36) | 0.60% | 1.05 | 1.21 |
| AGBL2 | NM_024783 | -1 | 0.000684777 | 0.63 (0.48, 0.82) | 2.50% | 1.05 | 1.32 |
| APOBEC1 | NM_001644 | 1 | 5.53E-05 | 1.22 (1.11, 1.34) | 0.50% | 1.05 | 1.2 |
| ARHGEF6 | NM_004840 | -1 | 0.000770839 | 0.54 (0.38, 0.78) | 2.70% | 1.05 | 1.32 |
| ATAD3A | NM_018188 | 1 | 0.001027877 | 2.28 (1.39, 3.72) | 3.20% | 1.05 | 1.28 |
| ATAD3A | NM_001170536 | 1 | 0.001027877 | 2.28 (1.39, 3.72) | 3.20% | 1.05 | 1.28 |
| ATAD3A | NM_01170535 | 1 | 0.001027877 | 2.28 (1.39, 3.72) | 3.20% | 1.05 | 1.28 |
| ATP8A2 | NM_016529 | -1 | 0.000828234 | 0.56 (0.40, 0.79) | 2.80% | 1.05 | 1.31 |
| AURKA | NM_198437 | 1 | 0.001108089 | 2.00 (1.32, 3.02) | 3.30% | 1.05 | 1.3 |
| AURKA | NM_198436 | 1 | 0.001108089 | 2.00 (1.32, 3.02) | 3.30% | 1.05 | 1.3 |
| AURKA | NM_198435 | 1 | 0.001108089 | 2.00 (1.32, 3.02) | 3.30% | 1.05 | 1.3 |
| AURKA | NM_198434 | 1 | 0.001108089 | 2.00 (1.32, 3.02) | 3.30% | 1.05 | 1.3 |
| AURKA | NM_003600 | 1 | 0.001108089 | 2.00 (1.32, 3.02) | 3.30% | 1.05 | 1.3 |
| AURKA | NM_198433 | 1 | 0.001108089 | 2.00 (1.32, 3.02) | 3.30% | 1.05 | 1.32 |
| BAI2 | NM_001703 | -1 | 0.000813324 | 0.59 (0.43, 0.80) | 2.80% | 1.05 | 1.3 |
| C10orf68 | NM_024688 | -1 | 0.000766871 | 0.54 (0.38, 0.77) | 2.70% | 1.05 | 1.32 |
| C14orf33 | NR_027123 | -1 | 0.000774142 | 0.62 (0.47,0.82) | 2.70% | 1.05 | 1.31 |
| C9orf117 | NM_001012502 | -1 | 0.000748452 | 0.58 (0.43, 0.80) | 2.60% | 1.05 | 1.32 |
| CDC25C | NM_022809 | 1 | 0.00105986 | 2.22 (1.38, 3.57) | 3.30% | 1.05 | 1.29 |
| CDC25C | NM_001790 | 1 | 0.00105986 | 2.22 (1.38, 3.57) | 3.30% | 1.05 | 1.29 |
| CENPO | NM_24322 | 1 | 0.000898127 | 2.08 (1.35, 3.19) | 2.90% | 1.05 | 1.3 |
| CENPO | NM_001199803 | 1 | 0.000898127 | 2.08 (1.35, 3.19) | 2.90% | 1.05 | 1.3 |
| CLCN5 | NM_000084 | 1 | 0.000713564 | 1.83 (1.29, 2.60) | 2.60% | 1.05 | 1.32 |
| CLCN5 | NM_001127899 | 1 | 0.000713564 | 1.83 (1.29, 2.60) | 2.60% | 1.05 | 1.32 |
| CLCN5 | NM_001127898 | 1 | 0.000713564 | 1.83 (1.29, 2.60) | 2.60% | 1.05 | 1.32 |
| CLK1 | NR_027955 | -1 | 0.000894665 | 0.56 (0.39, 0.79) | 2.90% | 1.05 | 1.31 |
| CLK1 | NR_027856 | -1 | 0.000894665 | 0.56 (0.39, 0.79) | 2.90% | 1.05 | 1.31 |
| CLK1 | NM_004071 | -1 | 0.000894665 | 0.56 (0.39, 0.79) | 2.90% | 1.05 | 1.31 |
| CLK1 | NM_001162407 | -1 | 0.000894665 | 0.56 (0.39, 0.79) | 2.90% | 1.05 | 1.31 |
| CLTC | NM_004859 | 1 | 0.000563537 | 1.55 (1.21, 1.98) | 2.20% | 1.05 | 1.32 |
| COPG | NM_016128 | 1 | 0.000702768 | 1.78 (1.28, 2.49) | 2.50% | 1.05 | 1.32 |
| DEFB133 | NM_001166478 | 1 | 0.000294095 | 1.38 (1.16, 1.64) | 1.50% | 1.05 | 1.28 |
| DGCR10 | NR_026651 | 1 | 0.000654253 | 1.69 (1.25, 2.29) | 2.50% | 1.05 | 1.33 |
| DHCR7 | NM_001360 | 1 | 0.000806605 | 1.76 (1.26, 2.44) | 2.80% | 1.05 | 1.32 |
| DHCR7 | NM_001163817 | 1 | 0.000806605 | 1.76 (1.26, 2.44) | 2.80% | 1.05 | 1.32 |
| DLX6-AS1 | NR_015448 | 1 | 0.000705367 | 1.57 (1.21, 2.03) | 2.50% | 1.05 | 1.32 |
| DMRTC2 | NM_001040283 | 1 | 0.000492509 | 1.49 (1.19, 1.86) | 2.00% | 1.05 | 1.31 |
| ESRP1 | NM_001122827 | 1 | 0.000764406 | 1.86 (1.30, 2.67) | 2.70% | 1.05 | 1.32 |
| ESRP1 | NM_017697 | 1 | 0.000764406 | 1.86 (1.30, 2.67) | 2.70% | 1.05 | 1.32 |
| ESRP1 | NM_001122825 | 1 | 0.000764406 | 1.86 (1.30, 2.67) | 2.70% | 1.05 | 1.32 |
| ESRP1 | NM_001122826 | 1 | 0.000764406 | 1.86 (1.30, 2.67) | 2.70% | 1.05 | 1.32 |
| ESRP1 | NM_001034915 | 1 | 0.000764406 | 1.86 (1.30, 2.67) | 2.70% | 1.05 | 1.32 |
| FAM184B | NM_015688 | -1 | 0.000919699 | 0.50 (0.33, 0.75) | 3.00% | 1.05 | 1.3 |
| FGD3 | NM_001083536 | -1 | 0.000878971 | 0.48 (0.31, 0.74) | 2.90% | 1.05 | 1.3 |
| FGD3 | NM_033086 | -1 | 0.000878971 | 0.48 (0.31, 0.74) | 2.90% | 1.05 | 1.3 |
| FRMPD2 | NM_001018071 | -1 | 0.000916891 | 0.50 (0.33, 0.75) | 3.00% | 1.05 | 1.3 |
| FRMPD2 | NR_033178 | -1 | 0.000916891 | 0.50 (0.33, 0.75) | 3.00% | 1.05 | 1.3 |
| GALNS | NM_000512 | 1 | 0.000779802 | 1.73 (1.26, 2.39) | 2.70% | 1.05 | 1.32 |
| GPA33 | NM_005814 | 1 | 0.000455791 | 1.51 (1.20, 1.91) | 1.90% | 1.05 | 1.32 |
| GRIK4 | NM_014619 | -1 | 0.000983512 | 0.49 (0.32, 0.75) | 3.10% | 1.05 | 1.3 |
| GRSF1 | NM_002092 | 1 | 0.000455403 | 1.42 (1.17, 1.73) | 1.90% | 1.05 | 1.29 |
| GRSF1 | NM_001098477 | 1 | 0.000455403 | 1.42 (1.17, 1.73) | 1.90% | 1.05 | 1.29 |
| GSG1 | NM_031289 | -1 | 0.000746247 | 0.57 (0.41, 0.79) | 2.60% | 1.05 | 1.32 |
| GSG1 | NM_153823 | -1 | 0.000746247 | 0.57 (0.41, 0.79) | 2.60% | 1.05 | 1.32 |
| GSG1 | NM_001080555 | -1 | 0.000746247 | 0.57 (0.41, 0.79) | 2.60% | 1.05 | 1.32 |
| GSG1 | NM_001080554 | -1 | 0.000746247 | 0.57 (0.41, 0.79) | 2.60% | 1.05 | 1.32 |
| GSTTP1 | NR_003081 | 1 | 0.000281335 | 1.36 (1.15, 1.61) | 1.50% | 1.05 | 1.28 |
| GZMB | NM_004131 | 1 | 0.000774473 | 1.64 (1.23, 2.20) | 2.70% | 1.05 | 1.32 |
| HIVEP3 | NM_024503 | -1 | 0.000637807 | 0.56 (0.40, 0.78) | 2.40% | 1.05 | 1.32 |
| HIVEP3 | NM_001127714 | -1 | 0.000637807 | 0.56 (0.40, 0.78) | 2.40% | 1.05 | 1.32 |
| HRK | NM_003806 | 1 | 0.000573791 | 1.56 (1.21, 2.02) | 2.20% | 1.05 | 1.32 |
| ICK | NM_016513 | 1 | 0.000708599 | 1.76 (1.27, 2.45) | 2.50% | 1.05 | 1.32 |
| ICK | NM_014920 | 1 | 0.000708599 | 1.76 (1.27, 2.45) | 2.50% | 1.05 | 1.32 |
| IDI1 | NM_004508 | 1 | 0.000576826 | 1.62 (1.23, 2.12) | 2.20% | 1.05 | 1.32 |
| INPP5K | NM_001135642 | -1 | 0.000606796 | 0.61 (0.45, 0.81) | 2.30% | 1.05 | 1.32 |
| INPP5K | NM_130766 | -1 | 0.000606796 | 0.61 (0.45, 0.81) | 2.30% | 1.05 | 1.32 |
| INPP5K | NM_016532 | -1 | 0.000606796 | 0.61 (0.45, 0.81) | 2.30% | 1.05 | 1.32 |
| KDELR2 | NM_001100603 | 1 | 0.000640872 | 1.78 (1.28, 2.48) | 2.40% | 1.05 | 1.33 |
| KDELR2 | NM_006854 | 1 | 0.000640872 | 1.78 (1.28, 2.48) | 2.40% | 1.05 | 1.33 |
| KIAA0528 | NM_014802 | -1 | 0.00067798 | 0.60 (0.45, 0.81) | 2.50% | 1.05 | 1.32 |
| KIAA0664L3 | NR_024034 | -1 | 0.000538791 | 0.68 (0.54, 0.84) | 2.10% | 1.05 | 1.3 |
| KIAA0753 | NM_014804 | -1 | 0.000792844 | 0.58 (0.43, 0.80) | 2.70% | 1.05 | 1.32 |
| KIF14 | NM_014875 | 1 | 0.000991316 | 2.07 (1.34, 3.20) | 3.10% | 1.05 | 1.3 |
| KLHL5 | NM_015990 | -1 | 0.001075498 | 0.51 (0.34, 0.76) | 3.30% | 1.05 | 1.3 |
| KLHL5 | NM_199039 | -1 | 0.001075498 | 0.51 (0.34, 0.76) | 3.30% | 1.05 | 1.3 |
| KLHL5 | NM_001007075 | -1 | 0.001075498 | 0.51 (0.34, 0.76) | 3.30% | 1.05 | 1.3 |
| KLHL5 | NM_001171654 | -1 | 0.001075498 | 0.51 (0.34, 0.76) | 3.30% | 1.05 | 1.3 |
| LARP1 | NM_015315 | 1 | 0.000776893 | 1.98 (1.33, 2.95) | 2.70% | 1.05 | 1.31 |
| LMAN1L | NM_021819 | 1 | 0.000694431 | 1.58 (1.21, 2.05) | 2.50% | 1.05 | 1.32 |
| LOC284440 | NR_026956 | -1 | 0.00064239 | 0.56 (0.40, 0.78) | 2.40% | 1.05 | 1.32 |
| LOC644961 | NR_033926 | -1 | 0.000821483 | 0.51 (0.35, 0.76) | 2.80% | 1.05 | 1.31 |
| MAGEA3 | NM_005362 | 1 | 0.000259711 | 1.40 (1.17, 1.67) | 1.40% | 1.05 | 1.29 |
| MAGEB2 | NM_002364 | 1 | 0.000109866 | 1.28 (1.13, 1.45) | 0.80% | 1.05 | 1.24 |
| MIR1297 | NR_031628 | 1 | 0.000213996 | 1.30 (1.13, 1.50) | 1.20% | 1.05 | 1.25 |
| NXNL2 | NM_001161625 | -1 | 0.000679325 | 0.53 (0.37, 0.77) | 2.50% | 1.05 | 1.32 |
| NXNL2 | NM_145283 | -1 | 0.000679325 | 0.53 (0.37, 0.77) | 2.50% | 1.05 | 1.32 |
| PCDH11Y | NM_032972 | 1 | 0.000256294 | 1.35 (1.15, 1.58) | 1.40% | 1.05 | 1.27 |
| PCDH11Y | NM_032973 | 1 | 0.000256294 | 1.35 (1.15, 1.58) | 1.40% | 1.05 | 1.27 |
| PCDH11Y | NM_032971 | 1 | 0.000256294 | 1.35 (1.15, 1.58) | 1.40% | 1.05 | 1.27 |
| PGD | NM_002631 | 1 | 0.00082458 | 1.77 (1.27, 2.46) | 2.80% | 1.05 | 1.32 |
| PHF2 | NM_005392 | -1 | 0.00054667 | 0.62 (0.47, 0.81) | 2.20% | 1.05 | 1.32 |
| POU3F4 | NM_00307 | 1 | 0.000444079 | 1.56 (1.22, 2.00) | 1.90% | 1.05 | 1.32 |
| POU4F1 | NM_006237 | 1 | 0.000488435 | 1.52 (1.20, 1.92) | 2.00% | 1.05 | 1.32 |
| PTGER3 | NM_001126044 | -1 | 0.000851253 | 0.52 (0.36,0.77) | 2.80% | 1.05 | 1.31 |
| PTGER3 | NM_198716 | -1 | 0.000851253 | 0.52 (0.36, 0.77) | 2.80% | 1.05 | 1.31 |
| PTGER3 | NM_198718 | -1 | 0.000851253 | 0.52 (0.36, 0.77) | 2.80% | 1.05 | 1.31 |
| PTGER3 | NM_198715 | -1 | 0.000851253 | 0.52 (0.36, 0.77) | 2.80% | 1.05 | 1.31 |
| PTGER3 | NM_198719 | -1 | 0.000851253 | 0.52 (0.36, 0.77) | 2.80% | 1.05 | 1.31 |
| PTGER3 | NM_198714 | -1 | 0.000851253 | 0.52 (0.36, 0.77) | 2.80% | 1.05 | 1.31 |
| PTGER3 | NR 028292 | -1 | 0.000851253 | 0.52 (0.36, 0.77) | 2.80% | 1.05 | 1.31 |
| PTGER3 | NR_028293 | -1 | 0.000851253 | 0.52 (0.36, 0.77) | 2.80% | 1.05 | 1.31 |
| PTGER3 | NM_198717 | -1 | 0.000851253 | 0.52 (0.36, 0.77) | 2.80% | 1.05 | 1.31 |
| PTGER3 | NR_028294 | -1 | 0.000851253 | 0.52 (0.36, 0.77) | 2.80% | 1.05 | 1.31 |
| RGP1 | NM_001080496 | -1 | 0.000674124 | 0.64 (0.49, 0.83) | 2.50% | 1.05 | 1.31 |
| RHOV | NM_133639 | 1 | 0.000910194 | 1.91 (1.30, 2.81) | 3.00% | 1.05 | 1.31 |
| RPF1 | NM_025065 | -1 | 0.000516752 | 0.60 (0.45, 0.80) | 2.10% | 1.05 | 1.33 |
| SEMA4B | NM_198925 | 1 | 0.000425026 | 1.49 (1.19, 1.86) | 1.90% | 1.05 | 1.31 |
| SEMA4B | NM_020210 | 1 | 0.000425026 | 1.49 (1.19, 1.86) | 1.90% | 1.05 | 1.31 |
| SLC30A3 | NM_003459 | 1 | 0.000459198 | 1.58 (1.22, 2.03) | 2.00% | 1.05 | 1.33 |
| SLC35A2 | NM_005660 | 1 | 0.000949606 | 2.03 (1.33, 3.08) | 3.00% | 1.05 | 1.3 |
| SLC35A2 | NM_001042498 | 1 | 0.000949606 | 2.03 (1.33, 3.08) | 3.00% | 1.05 | 1.3 |
| SLC35A2 | NM_001032289 | 1 | 0.000949606 | 2.03 (1.33, 3.08) | 3.00% | 1.05 | 1.3 |
| SLC40A1 | NM_014585 | -1 | 0.000901525 | 0.50 (0.33, 0.75) | 2.90% | 1.05 | 1.3 |
| SLC45A4 | NM_001080431 | 1 | 0.000795498 | 1.90 (1.31, 2.76) | 2.70% | 1.05 | 1.32 |
| SLC4A10 | NM_022058 | -1 | 0.000922314 | 0.50 (0.33, 0.75) | 3.00% | 1.05 | 1.3 |
| SLC4A10 | NM_001178016 | -1 | 0.000922314 | 0.50 (0.33, 0.75) | 3.00% | 1.05 | 1.3 |
| SLC4A10 | NM_001178015 | -1 | 0.000922314 | 0.50 (0.33, 0.75) | 3.00% | 1.05 | 1.3 |
| SMPD2 | NM_003080 | -1 | 0.000636112 | 0.62 (0.47, 0.81) | 2.40% | 1.05 | 1.32 |
| SMU1 | NM_018225 | -1 | 0.000694202 | 0.54 (0.37, 0.77) | 2.50% | 1.05 | 1.32 |
| STON2 | NM_033104 | -1 | 0.000687523 | 0.54 (0.38, 0.77) | 2.50% | 1.05 | 1.32 |
| TECTA | NM_005422 | -1 | 0.000626178 | 0.58 (0.43, 0.79) | 2.40% | 1.05 | 1.32 |
| TMEM8B | NM_001042590 | -1 | 0.000970606 | 0.55 (0.39,0.78) | 3.10% | 1.05 | 1.31 |
| TMEM8B | NM_001042589 | -1 | 0.000970606 | 0.55 (0.39, 0.78) | 3.10% | 1.05 | 1.31 |
| TMEM8B | NM_016446 | -1 | 0.000970606 | 0.55 (0.39, 0.78) | 3.10% | 1.05 | 1.31 |
| TRAF3IP1 | NM_015650 | -1 | 0.000567431 | 0.59 (0.44, 0.80) | 2.20% | 1.05 | 1.33 |
| TRAF3IP1 | NM_001139490 | -1 | 0.000567431 | 0.59 (0.44, 0.80) | 2.20% | 1.05 | 1.33 |
| TROAP | NM_005480 | 1 | 0.000902469 | 1.97 (1.32, 2.94) | 2.90% | 1.05 | 1.31 |
| TROAP | NM_001100620 | 1 | 0.000902469 | 1.97 (1.32, 2.94) | 2.90% | 1.05 | 1.31 |
| TSTA3 | NM_003313 | 1 | 0.000809687 | 1.67 (1.24, 2.25) | 2.80% | 1.05 | 1.32 |
| TUBG2 | NM_016437 | -1 | 0.000282337 | 0.72 (0.60, 0.86) | 1.50% | 1.05 | 1.29 |
| TXNDC8 | NM_001003936 | 1 | 0.000444509 | 1.48 (1.19, 1.85) | 1.90% | 1.05 | 1.31 |
| UGT2B10 | NM_001075 | 1 | 0.000213893 | 1.39 (1.17, 1.65) | 1.20% | 1.05 | 1.29 |
| UGT2B10 | NM_001075 | 1 | 0.000213893 | 1.39 (1.17, 1.65) | 1.20% | 1.05 | 1.29 |
| UGT2B10 | NM_001144767 | 1 | 0.000213893 | 1.39 (1.17, 1.65) | 1.20% | 1.05 | 1.29 |
| UGT2B10 | NM_001144767 | 1 | 0.000213893 | 1.39 (1.17, 1.65) | 1.20% | 1.05 | 1.29 |
| WAPAL | NM_015045 | -1 | 0.000856095 | 0.55 (0.39, 0.78) | 2.90% | 1.05 | 1.31 |
| ZNF20 | NM_021143 | -1 | 0.000772771 | 0.58 (0.43, 0.80) | 2.70% | 1.05 | 1.32 |
| ZNF317 | NM_020933 | -1 | 0.000545984 | 0.59 (0.44, 0.80) | 2.20% | 1.05 | 1.33 |
| ZNF317 | NM_001190791 | -1 | 0.000545984 | 0.59 (0.44, 0.80) | 2.20% | 1.05 | 1.33 |
| ZNF516 | NM_014643 | -1 | 0.000706877 | 0.57 (0.41, 0.79) | 2.50% | 1.05 | 1.32 |
| ZNF594 | NM_032530 | -1 | 0.000728948 | 0.56 (0.40, 0.78) | 2.60% | 1.05 | 1.32 |
| ZNF625 | NM_145233 | -1 | 0.00079499 | 0.56 (0.40, 0.79) | 2.70% | 1.05 | 1.32 |
| ZNF695 | NM_020394 | 1 | 0.000959311 | 2.11 (1.35, 3.28) | 3.10% | 1.05 | 1.3 |
| ABR | NM_001092 | -1 | 0.00124577 | 0.57 (0.41, 0.80) | 3.60% | 1.04 | 1.3 |
| ABR | NM_001159746 | -1 | 0.00124577 | 0.57 (0.41, 0.80) | 3.60% | 1.04 | 1.3 |
| ABR | NN_021962 | -1 | 0.00124577 | 0.57 (0.41, 0.80) | 3.60% | 1.04 | 1.3 |
| ACBD3 | NM_022735 | 1 | 0.001654232 | 1.92 (1.28, 2.87) | 4.10% | 1.04 | 1.29 |
| ACY3 | NM_080658 | 1 | 0.000456993 | 1.33 (1.13, 1.55) | 1.90% | 1.04 | 1.26 |
| AMY2B | NM_020978 | -1 | 0.001380606 | 0.59 (0.42, 0.81) | 3.80% | 1.04 | 1.3 |
| AP4E1 | NM_007347 | 1 | 0.000480692 | 1.26 (1.11, 1.44) | 2.00% | 1.04 | 1.22 |
| ASTN2 | NM_001184735 | -1 | 0.001083598 | 0.56 (0.40, 0.79) | 3.30% | 1.04 | 1.31 |
| ASTN2 | NM_198188 | -1 | 0.001083598 | 0.56 (0.40, 0.79) | 3.30% | 1.04 | 1.31 |
| ASTN2 | NM_001184734 | -1 | 0.001083598 | 0.56 (0.40, 0.79) | 3.30% | 1.04 | 1.31 |
| ASTN2 | NM_014010 | -1 | 0.001083598 | 0.56 (0.40, 0.79) | 3.30% | 1.04 | 1.31 |
| ASTN2 | NM_198187 | -1 | 0.001083598 | 0.56 (0.40, 0.79) | 3.30% | 1.04 | 1.31 |
| ASTN2 | NM_198186 | -1 | 0.001083598 | 0.56 (0.40, 0.79) | 3.30% | 1.04 | 1.31 |
| ATP5S | NM_001003805 | -1 | 0.001099833 | 0.60 (0.44, 0.81) | 3.30% | 1.04 | 1.31 |
| ATP5S | NR_033761 | -1 | 0.001099833 | 0.60 (0.44, 0.81) | 3.30% | 1.04 | 1.31 |
| ATP5S | NM_015684 | -1 | 0.001099833 | 0.60 (0.44, 0.81) | 3.30% | 1.04 | 1.31 |
| ATP5S | NM_001003803 | -1 | 0.001099833 | 0.60 (0.44, 0.81) | 3.30% | 1.04 | 1.31 |
| AZU1 | NM_001700 | 1 | 0.001184365 | 1.57 (1.20, 2.06) | 3.50% | 1.04 | 1.3 |
| BOD1L | NM_148894 | -1 | 0.001481913 | 0.53 (0.36, 0.78) | 3.90% | 1.04 | 1.29 |
| C14orf102 | NM_199043 | -1 | 0.001090373 | 0.57 (0.41, 0.80) | 3.30% | 1.04 | 1.31 |
| C14orf102 | NM_017970 | -1 | 0.001090373 | 0.57 (0.41, 0.80) | 3.30% | 1.04 | 1.31 |
| C15orf23 | NM_001142762 | 1 | 0.001423858 | 1.90 (1.28, 2.82) | 3.80% | 1.04 | 1.3 |
| C15orf23 | NM_033286 | 1 | 0.001423858 | 1.90 (1.28, 2.82) | 3.80% | 1.04 | 1.3 |
| C15orf23 | NM_001142761 | 1 | 0.001423858 | 1.90 (1.28, 2.82) | 3.80% | 1.04 | 1.3 |
| C19orf18 | NM_152474 | -1 | 0.001597281 | 0.52 (0.35,0.78) | 4.10% | 1.04 | 1.29 |
| C20orf132 | NM_213631 | -1 | 0.000972217 | 0.58 (0.42, 0.80) | 3.10% | 1.04 | 1.31 |
| C20orf132 | NM_152503 | -1 | 0.000972217 | 0.58 (0.42, 0.80) | 3.10% | 1.04 | 1.31 |
| C20orf132 | NM_213632 | -1 | 0.000972217 | 0.58 (0.42, 0.80) | 3.10% | 1.04 | 1.31 |
| C21orf90 | NR_026547 | 1 | 0.001031689 | 1.54 (1.19, 1.99) | 3.20% | 1.04 | 1.3 |
| C21orf90 | NR_026548 | 1 | 0.001031689 | 1.54 (1.19, 1.99) | 3.20% | 1.04 | 1.3 |
| C3orf65 | NR_027317 | 1 | 0.001227976 | 1.72 (1.24, 2.38) | 3.50% | 1.04 | 1.31 |
| C6orf225 | NM_001033564 | -1 | 0.000824925 | 0.64 (0.49, 0.83) | 2.80% | 1.04 | 1.31 |
| CDCA2 | NM_152562 | 1 | 0.001763324 | 2.19 (1.34, 3.58) | 4.30% | 1.04 | 1.27 |
| CLDN4 | NM_001305 | 1 | 0.001451467 | 1.97 (1.30, 2.98) | 3.90% | 1.04 | 1.29 |
| COL16A1 | NM_001856 | -1 | 0.00135906 | 0.60 (0.44, 0.82) | 3.70% | 1.04 | 1.3 |
| COQ9 | NM_020312 | 1 | 0.001364105 | 1.78 (1.25, 2.53) | 3.70% | 1.04 | 1.3 |
| COX18 | NM_173827 | 1 | 0.000877732 | 1.43 (1.16, 1.76) | 2.90% | 1.04 | 1.29 |
| COX4NB | NM_006067 | 1 | 0.001769748 | 2.07 (1.31, 3.28) | 4.30% | 1.04 | 1.28 |
| COX4NB | NM_001142288 | 1 | 0.001769748 | 2.07 (1.31, 3.28) | 4.30% | 1.04 | 1.28 |
| CYCS | NM_018947 | 1 | 0.001320528 | 1.65 (1.22, 2.25) | 3.70% | 1.04 | 1.3 |
| DACT2 | NM_214462 | 1 | 0.001302736 | 1.62 (1.21, 2.17) | 3.60% | 1.04 | 1.3 |
| DEGS1 | NM_003676 | 1 | 0.001061247 | 1.48 (1.17, 1.87) | 3.30% | 1.04 | 1.29 |
| DUSP9 | NM_001395 | 1 | 0.001256936 | 1.61 (1.20, 2.14) | 3.60% | 1.04 | 1.3 |
| DYNC2H1 | NM_001080463 | -1 | 0.001370915 | 0.59 (0.43, 0.82) | 3.70% | 1.04 | 1.3 |
| DYNC2H1 | NM_001377 | -1 | 0.001370915 | 0.59 (0.43, 0.82) | 3.70% | 1.04 | 1.3 |
| EIF4G1 | NM_198241 | 1 | 0.001353974 | 1.68 (1.22, 2.31) | 3.70% | 1.04 | 1.3 |
| EIF4G1 | NM_001194947 | 1 | 0.001353974 | 1.68 (1.22, 2.31) | 3.70% | 1.04 | 1.3 |
| EIF4G1 | NM_198244 | 1 | 0.001353974 | 1.68 (1.22, 2.31) | 3.70% | 1.04 | 1.3 |
| EIF4G1 | NM_198242 | 1 | 0.001353974 | 1.68 (1.22, 2.31) | 3.70% | 1.04 | 1.3 |
| EIF4G1 | NM_001194946 | 1 | 0.001353974 | 1.68 (1.22, 2.31) | 3.70% | 1.04 | 1.3 |
| EIF4G1 | NM_182917 | 1 | 0.001353974 | 1.68 (1.22,2.31) | 3.70% | 1.04 | 1.3 |
| EIF4G1 | NM_004953 | 1 | 0.001353974 | 1.68 (1.22, 2.31) | 3.70% | 1.04 | 1.3 |
| ENO1 | NM_001428 | 1 | 0.001364087 | 1.81 (1.26, 2.61) | 3.70% | 1.04 | 1.31 |
| FAF2 | NM_014613 | 1 | 0.00108376 | 1.88 (1.29, 2.74) | 3.30% | 1.04 | 1.3 |
| FAM149A | NM_015398 | -1 | 0.001194251 | 0.64 (0.49, 0.84) | 3.50% | 1.04 | 1.3 |
| FAM149A | NM_001006655 | -1 | 0.001194251 | 0.64 (0.49, 0.84) | 3.50% | 1.04 | 1.31 |
| FAM154B | NM_001008226 | -1 | 0.001023443 | 0.54 (0.37, 0.78) | 3.20% | 1.04 | 1.3 |
| FGG | NM_021870 | 1 | 0.000888207 | 1.49 (1.18, 1.88) | 2.90% | 1.04 | 1.3 |
| FGG | NM_000509 | 1 | 0.000888207 | 1.49 (1.18, 1.88) | 2.90% | 1.04 | 1.3 |
| GET4 | NM_015949 | 1 | 0.001268692 | 1.58 (1.20, 2.10) | 3.60% | 1.04 | 1.31 |
| GGT1 | NM_005265 | 1 | 0.00125253 | 1.75 (1.25, 2.46) | 3.60% | 1.04 | 1.31 |
| GGT1 | NM_013430 | 1 | 0.00125253 | 1.75 (1.25, 2.46) | 3.60% | 1.04 | 1.31 |
| GGT1 | NM_001032365 | 1 | 0.00125253 | 1.75 (1.25, 2.46) | 3.60% | 1.04 | 1.31 |
| GGT1 | NM_001032364 | 1 | 0.00125253 | 1.75 (1.25, 2.46) | 3.60% | 1.04 | 1.31 |
| GPC4 | NM_001448 | -1 | 0.000911024 | 0.69 (0.56, 0.86) | 3.00% | 1.04 | 1.29 |
| HNMT | NM_001024074 | -1 | 0.001007463 | 0.61 (0.45, 0.82) | 3.20% | 1.04 | 1.31 |
| HNMT | NM_006895 | -1 | 0.001007463 | 0.61 (0.45, 0.82) | 3.20% | 1.04 | 1.31 |
| HNMT | NM_001024075 | -1 | 0.001007463 | 0.61 (0.45, 0.82) | 3.20% | 1.04 | 1.31 |
| HSBP1 | NM_001537 | 1 | 0.001388414 | 1.93 (1.29, 2.89) | 3.80% | 1.04 | 1.3 |
| ILF2 | NM_004515 | 1 | 0.001444137 | 1.77 (1.25, 2.51) | 3.80% | 1.04 | 1.3 |
| INCENP | NM_001040694 | 1 | 0.001350727 | 1.77 (1.25, 2.52) | 3.70% | 1.04 | 1.3 |
| INCENP | NM_020238 | 1 | 0.001350727 | 1.77 (1.25, 2.52) | 3.70% | 1.04 | 1.3 |
| KIAA0802 | NM_015210 | -1 | 0.000733238 | 0.66 (0.52, 0.84) | 2.60% | 1.04 | 1.3 |
| LOC100129636 | NM_001195202 | 1 | 0.000568239 | 1.41 (1.16, 1.71) | 2.20% | 1.04 | 1.29 |
| LRRC50 | NM_178452 | -1 | 0.001142207 | 0.62 (0.46, 0.83) | 3.40% | 1.04 | 1.3 |
| LSS | NM_001145436 | 1 | 0.00102682 | 1.85 (1.28, 2.68) | 3.20% | 1.04 | 1.31 |
| LSS | NM_001145437 | 1 | 0.00102682 | 1.85 (1.28, 2.68) | 3.20% | 1.04 | 1.31 |
| LSS | NM_001001438 | 1 | 0.00102682 | 1.85 (1.28, 2.68) | 3.20% | 1.04 | 1.31 |
| LSS | NM_002340 | 1 | 0.00102682 | 1.85 (1.28, 2.68) | 3.20% | 1.04 | 1.31 |
| LYPD2 | NM_205545 | 1 | 0.000432072 | 1.29 (1.12, 1.48) | 1.90% | 1.04 | 1.24 |
| MARCH6 | NM_005885 | 1 | 0.001493132 | 1.94 (1.29, 2.93) | 3.90% | 1.04 | 1.29 |
| MDH1B | NM_001039845 | -1 | 0.001226529 | 0.59 (0.43, 0.82) | 3.50% | 1.04 | 1.3 |
| MELK | NM_014791 | 1 | 0.001334491 | 1.89 (1.28, 2.78) | 3.70% | 1.04 | 1.3 |
| MEPE | NM_001184695 | 1 | 0.000345187 | 1.35 (1.14, 1.59) | 1.60% | 1.04 | 1.27 |
| MEPE | NM_001184696 | 1 | 0.000345187 | 1.35 (1.14, 1.59) | 1.60% | 1.04 | 1.27 |
| MEPE | NM_001184694 | 1 | 0.000345187 | 1.35 (1.14, 1.59) | 1.60% | 1.04 | 1.27 |
| MEPE | NM_001184697 | 1 | 0.000345187 | 1.35 (1.14, 1.59) | 1.60% | 1.04 | 1.27 |
| MEPE | NM_020203 | 1 | 0.000345187 | 1.35 (1.14, 1.59) | 1.60% | 1.04 | 1.27 |
| MGAT4B | NM_014275 | 1 | 0.001660199 | 1.94 (1.28, 2.93) | 4.10% | 1.04 | 1.29 |
| MGAT48 | NM_054013 | 1 | 0.001660199 | 1.94 (1.28, 2.93) | 4.10% | 1.04 | 1.29 |
| MIR1266 | NR_031670 | 1 | 0.000694086 | 1.45 (1.17, 1.79) | 2.50% | 1.04 | 1.29 |
| MKL2 | NM_014048 | -1 | 0.001149841 | 0.55 (0.38, 0.79) | 3.40% | 1.04 | 1.3 |
| MND1 | NM_032117 | 1 | 0.001547106 | 1.80 (1.25, 2.59) | 4.00% | 1.04 | 1.3 |
| MORC2 | NM_014941 | 1 | 0.001258176 | 1.73 (1.24, 2.42) | 3.60% | 1.04 | 1.31 |
| MUM1L1 | NM_152423 | -1 | 0.001393772 | 0.60 (0.44, 0.82) | 3.80% | 1.04 | 1.3 |
| MUM1L1 | NM_001171020 | -1 | 0.001393772 | 0.60 (0.44, 0.82) | 3.80% | 1.04 | 1.3 |
| NCRNA00094 | NR_015427 | -1 | 0.001103609 | 0.60 (0.44, 0.82) | 3.30% | 1.04 | 1.31 |
| NDUFV1 | NM_007103 | 1 | 0.001409686 | 1.72 (1.23, 2.41) | 3.80% | 1.04 | 1.3 |
| NDUFV1 | NM_001166102 | 1 | 0.001409686 | 1.72 (1.23, 2.41) | 3.80% | 1.04 | 1.3 |
| NEFL | NM_006158 | -1 | 0.001687285 | 0.45 (0.27, 0.74) | 4.20% | 1.04 | 1.26 |
| NOSTRIN | NM_001171631 | -1 | 0.001252393 | 0.54 (0.37, 0.78) | 3.60% | 1.04 | 1.3 |
| NOSTRIN | NM_052946 | -1 | 0.001252393 | 0.54 (0.37, 0.78) | 3.60% | 1.04 | 1.3 |
| NOSTRIN | NM_001039724 | -1 | 0.001252393 | 0.54 (0.37, 0.78) | 3.60% | 1.04 | 1.3 |
| NOSTRIN | NM_001171632 | -1 | 0.001252393 | 0.54 (0.37, 0.78) | 3.60% | 1.04 | 1.3 |
| NPFFR1 | NM_022146 | 1 | 0.000403573 | 1.37 (1.15, 1.63) | 1.80% | 1.04 | 1.28 |
| NR1H4 | NM_005123 | 1 | 0.000190977 | 1.21 (1.09, 1.34) | 1.20% | 1.04 | 1.19 |
| OR52E6 | NM_001005167 | 1 | 0.000848918 | 1.64 (1.23, 2.18) | 2.80% | 1.04 | 1.32 |
| OR8S1 | NM_001005203 | 1 | 0.00023101 | 1.27 (1.12, 1.45) | 1.30% | 1.04 | 1.23 |
| ORM1 | N_000607 | 1 | 0.000975674 | 1.51 (1.18, 1.93) | 3.10% | 1.04 | 1.3 |
| OSCP1 | NM_145047 | -1 | 0.001246562 | 0.64 (0.49, 0.84) | 3.60% | 1.04 | 1.3 |
| OSCP1 | NM_206837 | -1 | 0.001246562 | 0.64 (0.49, 0.84) | 3.60% | 1.04 | 1.3 |
| PAR-SN | NR_022011 | -1 | 0.001065009 | 0.68 (0.54, 0.86) | 3.30% | 1.04 | 1.29 |
| PDE4A | NM_001111308 | -1 | 0.001540235 | 0.56 (0.39, 0.80) | 4.00% | 1.04 | 1.29 |
| PDE4A | NM_001111307 | -1 | 0.001540235 | 0.56 (0.39, 0.80) | 4.00% | 1.04 | 1.29 |
| PDE4A | NM_006202 | -1 | 0.001540235 | 0.56 (0.39, 0.80) | 4.00% | 1.04 | 1.29 |
| PDE4A | NM_001111309 | -1 | 0.001540235 | 0.56 (0.39, 0.80) | 4.00% | 1.04 | 1.29 |
| PGA3 | NM_001079807 | 1 | 0.000794713 | 1.37 (1.14, 1.65) | 2.70% | 1.04 | 1.27 |
| PHF23 | NM_024297 | -1 | 0.00118066 | 0.57 (0.41, 0.80) | 3.50% | 1.04 | 1.3 |
| PLEKHA8 | NM_001197026 | 1 | 0.001145142 | 1.67 (1.22, 2.26) | 3.40% | 1.04 | 1.31 |
| PLEKHA8 | NM_032639 | 1 | 0.001145142 | 1.67 (1.22, 2.26) | 3.40% | 1.04 | 1.31 |
| PLEKHA8 | NM_001197027 | 1 | 0.001145142 | 1.67 (1.22, 2.26) | 3.40% | 1.04 | 1.31 |
| POLG2 | NM_007215 | 1 | 0.001113968 | 1.50 (1.18, 1.92) | 3.30% | 1.04 | 1.3 |
| POTEG | NR_027480 | 1 | 0.000753813 | 1.52 (1.19, 1.93) | 2.70% | 1.04 | 1.31 |
| POTEG | NM_001005356 | 1 | 0.000753813 | 1.52 (1.19, 1.93) | 2.70% | 1.04 | 1.31 |
| PRDX1 | NM_002574 | 1 | 0.001365139 | 1.96 (1.30, 2.97) | 3.70% | 1.04 | 1.29 |
| PRDX1 | NM_181697 | 1 | 0.001365139 | 1.96 (1.30, 2.97) | 3.70% | 1.04 | 1.29 |
| PRDX1 | NM_181696 | 1 | 0.001365139 | 1.96 (1.30, 2.97) | 3.70% | 1.04 | 1.29 |
| PRKCG | NM_002739 | 1 | 0.000915815 | 1.43 (1.16, 1.77) | 3.00% | 1.04 | 1.29 |
| PRRC1 | NM_130809 | 1 | 0.001282446 | 2.02 (1.32, 3.10) | 3.60% | 1.04 | 1.29 |
| PSMD7 | NM_002811 | 1 | 0.00121422 | 1.76 (1.25, 2.49) | 3.50% | 1.04 | 1.31 |
| PTPN13 | NM_080683 | -1 | 0.001238326 | 0.57 (0.40, 0.80) | 3.60% | 1.04 | 1.3 |
| PTPN13 | NM_080684 | -1 | 0.001238326 | 0.57 (0.40, 0.80) | 3.60% | 1.04 | 1.3 |
| PTPN13 | NM_080685 | -1 | 0.001238326 | 0.57 (0.40, 0.80) | 3.60% | 1.04 | 1.3 |
| PTPN13 | NM_006264 | -1 | 0.001238326 | 0.57 (0.40, 0.80) | 3.60% | 1.04 | 1.3 |
| PTPRF | NM_130440 | 1 | 0.001244621 | 1.71 (1.23, 2.36) | 3.60% | 1.04 | 1.31 |
| PTPRF | NM_002840 | 1 | 0.001244621 | 1.71 (1.23, 2.36) | 3.60% | 1.04 | 1.31 |
| RCBTB1 | NM_018191 | -1 | 0.001286867 | 0.57 (0.41, 0.80) | 3.60% | 1.04 | 1.3 |
| RPH3A | NM_014954 | 1 | 0.00083144 | 1.42 (1.16, 1.75) | 2.80% | 1.04 | 1.29 |
| RPH3A | NM_001143854 | -1 | 0.00083144 | 1.42 (1.16, 1.75) | 2.80% | 1.04 | 1.29 |
| RPS19BP1 | NM_194326 | 1 | 0.00131552 | 2.03 (1.32, 3.12) | 3.70% | 1.04 | 1.29 |
| RRM2 | NM_001034 | 1 | 0.001359597 | 2.12 (1.34, 3.37) | 3.70% | 1.04 | 1.28 |
| RRM2 | NM_001165931 | 1 | 0.001359597 | 2.12 (1.34, 3.37) | 3.70% | 1.04 | 1.28 |
| RTKN2 | NM_145307 | 1 | 0.001215644 | 1.79 (1.26, 2.55) | 3.50% | 1.04 | 1.31 |
| SHMT2 | NM_001166359 | 1 | 0.001409397 | 1.76 (1.24, 2.50) | 3.80% | 1.04 | 1.3 |
| SHMT2 | NM_901166358 | 1 | 0.001409397 | 1.76 (1.24, 2.50) | 3.80% | 1.04 | 1.3 |
| SHMT2 | NM_001166357 | 1 | 0.001409397 | 1.76 (1.24, 2.50) | 3.80% | 1.04 | 1.3 |
| SHMT2 | NM_001166356 | 1 | 0.001409397 | 1.76 (1.24, 2.50) | 3.80% | 1.04 | 1.3 |
| SHMT2 | NM_005412 | 1 | 0.001409397 | 1.76 (1.24, 2.50) | 3.80% | 1.04 | 1.3 |
| SHMT2 | NR_029416 | 1 | 0.001409397 | 1.76 (1.24, 2.50) | 3.80% | 1.04 | 1.3 |
| SHMT2 | NR_029417 | 1 | 0.001409397 | 1.76 (1.24, 2.50) | 3.80% | 1.04 | 1.3 |
| SHMT2 | NR_029415 | 1 | 0.001409397 | 1.76 (1.24, 2.50) | 3.80% | 1.04 | 1.3 |
| SHPK | NM_013276 | -1 | 0.001138133 | 0.50 (0.33, 0.76) | 3.40% | 1.04 | 1.29 |
| SLC43A1 | NM_001198810 | -1 | 0.001564414 | 0.54 (0.37, 0.79) | 4.10% | 1.04 | 1.29 |
| SLC43A1 | NM_003627 | -1 | 0.001564414 | 0.54 (0.37, 0.79) | 4.10% | 1.04 | 1.29 |
| SMAD7 | NM_001190821 | -1 | 0.001127632 | 0.63 (0.48, 0.83) | 3.40% | 1.04 | 1.3 |
| SMAD7 | NM_005904 | -1 | 0.001127632 | 0.63 (0.48, 0.83) | 3.40% | 1.04 | 1.3 |
| SMAD7 | NM_001190822 | -1 | 0.001127632 | 0.63 (0.48, 0.83) | 3.40% | 1.04 | 1.3 |
| SMAD7 | NM_001190823 | -1 | 0.001127632 | 0.63 (0.48, 0.83) | 3.40% | 1.04 | 1.3 |
| SMG5 | NM_015327 | 1 | 0.001368035 | 1.81 (1.26, 2.61) | 3.70% | 1.04 | 1.3 |
| SPAG17 | NM_206996 | -1 | 0.001277464 | 0.54 (0.37, 0.79) | 3.60% | 1.04 | 1.3 |
| SPRR3 | NM_001097589 | 1 | 0.000735393 | 1.43 (1.16, 1.77) | 2.60% | 1.04 | 1.29 |
| SPRR3 | NM_005416 | 1 | 0.000735393 | 1.43 (1.16, 1.77) | 2.60% | 1.04 | 1.29 |
| STARD13 | NM_178007 | -1 | 0.001355506 | 0.51 (0.33, 0.77) | 3.70% | 1.04 | 1.29 |
| STARD13 | NM_178006 | -1 | 0.001355506 | 0.51 (0.33, 0.77) | 3.70% | 1.04 | 1.29 |
| STARD13 | NM_052851 | -1 | 0.001355506 | 0.51 (0.33, 0.77) | 3.70% | 1.04 | 1.29 |
| STH | NM_001007532 | -1 | 0.001002598 | 0.61 (0.45, 0.82) | 3.20% | 1.04 | 1.31 |
| TALDO1 | NM_006755 | 1 | 0.001614891 | 2.06 (1.31, 3.23) | 4.10% | 1.04 | 1.28 |
| TMC6 | NM_007267 | 1 | 0.001392884 | 1.78 (1.25, 2.53) | 3.80% | 1.04 | 1.3 |
| TMC6 | NM_001127198 | 1 | 0.001392884 | 1.78 (1.25, 2.53) | 3.80% | 1.04 | 1.3 |
| TMEM194A | NM_015257 | 1 | 0.001255127 | 1.78 (1.25, 2.52) | 3.60% | 1.04 | 1.31 |
| TMEM194A | NM_001130963 | 1 | 0.001255127 | 1.78 (1.25, 2.52) | 3.60% | 1.04 | 1.31 |
| TMEM229B | NM_182526 | -1 | 0.001174425 | 0.60 (0.44, 0.82) | 3.50% | 1.04 | 1.3 |
| TMPRSS11E | NM_014058 | 1 | 0.001024924 | 1.57 (1.20, 2.06) | 3.20% | 1.04 | 1.31 |
| TOR1AIP2 | NM_022347 | 1 | 0.001376663 | 1.89 (1.28, 2.80) | 3.80% | 1.04 | 1.3 |
| TOR1AIP2 | NM_145034 | 1 | 0.001376663 | 1.89 (1.28, 2.80) | 3.80% | 1.04 | 1.3 |
| TOR1AIP2 | NM_001199260 | 1 | 0.001376663 | 1.89 (1.28, 2.80) | 3.80% | 1.04 | 1.3 |
| TPD52L2 | NM_199359 | 1 | 0.001081496 | 1.60 (1.21, 2.12) | 3.30% | 1.04 | 1.31 |
| TPD52L2 | NM_199360 | 1 | 0.001081496 | 1.60 (1.21, 2.12) | 3.30% | 1.04 | 1.31 |
| TPD52L2 | NM_199361 | 1 | 0.001081496 | 1.60 (1.21, 2.12) | 3.30% | 1.04 | 1.31 |
| TPD52L2 | NM_199362 | 1 | 0.001081496 | 1.60 (1.21, 2.12) | 3.30% | 1.04 | 1.31 |
| TPD52L2 | NM_199363 | 1 | 0.001081496 | 1.60 (1.21, 2.12) | 3.30% | 1.04 | 1.31 |
| TPD52L2 | NM_003288 | 1 | 0.001081496 | 1.60 (1.21, 2.12) | 3.30% | 1.04 | 1.31 |
| TPH2 | NM_173353 | 1 | 0.001086792 | 1.61 (1.21, 2.14) | 3.30% | 1.04 | 1.31 |
| TRAK1 | NM_001042646 | -1 | 0.001111813 | 0.57 (0.41, 0.80) | 3.30% | 1.04 | 1.31 |
| TRAK1 | NM_014965 | -1 | 0.001111813 | 0.57 (0.41, 0.80) | 3.30% | 1.04 | 1.31 |
| TTC23L | NM_144725 | -1 | 0.00182818 | 0.46 (0.28, 0.75) | 4.40% | 1.04 | 1.26 |
| UNC119B | NM_001080533 | -1 | 0.001111663 | 0.56 (0.40, 0.80) | 3.30% | 1.04 | 1.31 |
| ZNF442 | NM_030824 | -1 | 0.001384421 | 0.60 (0.44, 0.82) | 3.80% | 1.04 | 1.3 |
| ZNF512 | NM_032434 | -1 | 0.001588962 | 0.53 (0.35, 0.78) | 4.10% | 1.04 | 1.29 |
| ZNF528 | NM_032423 | -1 | 0.001148516 | 0.54 (0.38, 0.78) | 3.40% | 1.04 | 1.3 |
| ACOX3 | NM_003501 | -1 | 0.001635988 | 0.59 (0.43, 0.82) | 4.10% | 1.03 | 1.29 |
| ACOX3 | NM_001101667 | -1 | 0.001635988 | 0.59 (0.43, 0.82) | 4.10% | 1.03 | 1.29 |
| ANUBL1 | NM_001128324 | -1 | 0.001585772 | 0.57 (0.40, 0.81) | 4.10% | 1.03 | 1.29 |
| ANUBL1 | NM_174890 | -1 | 0.001585772 | 0.57 (0.40, 0.81) | 4.10% | 1.03 | 1.29 |
| AVL9 | NM_015060 | 1 | 0.001403234 | 1.42 (1.14, 1.75) | 3.80% | 1.03 | 1.28 |
| BICD2 | NM_001003800 | -1 | 0.002587505 | 0.52 (0.34, 0.79) | 5.40% | 1.03 | 1.27 |
| BICD2 | NM_015250 | -1 | 0.002587505 | 0.52 (0.34, 0.79) | 5.40% | 1.03 | 1.27 |
| BUB1B | NM_001211 | 1 | 0.002718314 | 1.97 (1.27, 3.08) | 5.50% | 1.03 | 1.27 |
| C10orf82 | NM_144661 | -1 | 0.002795215 | 0.48 (0.29, 0.77) | 5.60% | 1.03 | 1.25 |
| C11orf61 | NM_024631 | -1 | 0.001104328 | 0.72 (0.59, 0.87) | 3.30% | 1.03 | 1.27 |
| C12orf65 | NM_001194995 | -1 | 0.001612306 | 0.75 (0.62, 0.90) | 4.10% | 1.03 | 1.25 |
| C12orf65 | NM_001143905 | -1 | 0.001612306 | 0.75 (0.62, 0.90) | 4.10% | 1.03 | 1.25 |
| C12orf65 | NM_152269 | -1 | 0.001612306 | 0.75 (0.62, 0.90) | 4.10% | 1.03 | 1.25 |
| C14orf147 | NM_138288 | 1 | 0.001459289 | 1.61 (1.20, 2.15) | 3.90% | 1.03 | 1.3 |
| C17orf85 | NM_001114118 | -1 | 0.002505604 | 0.55 (0.37, 0.81) | 5.30% | 1.03 | 1.28 |
| C17orf85 | NM_018553 | -1 | 0.002505604 | 0.55 (0.37, 0.81) | 5.30% | 1.03 | 1.28 |
| C19orf75 | NM_173635 | 1 | 0.00067445 | 1.25 (1.10, 1.41) | 2.50% | 1.03 | 1.21 |
| C1orf198 | NM_032800 | 1 | 0.002616809 | 1.80 (1.23, 2.63) | 5.50% | 1.03 | 1.28 |
| C1orf198 | NM_ 001136495 | 1 | 0.002616809 | 1.80 (1.23, 2.63) | 5.50% | 1.03 | 1.28 |
| C1orf198 | NM_001136494 | 1 | 0.002616809 | 1.80 (1.23, 2.63) | 5.50% | 1.03 | 1.28 |
| C1RL | NM_016546 | -1 | 0.001589374 | 0.61 (0.45, 0.83) | 4.10% | 1.03 | 1.3 |
| C20orf185 | NM_182658 | 1 | 0.001202794 | 1.41 (1.15, 1.74) | 3.50% | 1.03 | 1.28 |
| C4orf29 | NM_001039717 | -1 | 0.002120732 | 0.60 (0.43, 0.83) | 4.90% | 1.03 | 1.29 |
| C5orf54 | NM_022090 | -1 | 0.000954196 | 0.73 (0.61, 0.88) | 3.10% | 1.03 | 1.26 |
| C6orf155 | NR_26807 | -1 | 0.002572118 | 0.55 (0.37, 0.81) | 5.40% | 1.03 | 1.27 |
| C7orf63 | NM_001160138 | -1 | 0.001543596 | 0.59 (0.42, 0.82) | 4.00% | 1.03 | 1.3 |
| C7orf63 | NM_001039706 | -1 | 0.001543596 | 0.59 (0.42, 0.82) | 4.00% | 1.03 | 1.3 |
| C9orf57 | NM _001128618 | 1 | 0.001866683 | 1.51 (1.17, 1.96) | 4.50% | 1.03 | 1.29 |
| C9orf78 | NM_016520 | -1 | 0.002244374 | 0.54 (0.36, 0.80) | 5.00% | 1.03 | 1.28 |
| CASZ1 | NM_017766 | -1 | 0.002238185 | 0.59 (0.43, 0.83) | 5.00% | 1.03 | 1.28 |
| CASZ1 | NM_001079843 | -1 | 0.002238185 | 0.59 (0.43, 0.83) | 5.00% | 1.03 | 1.29 |
| CBLN4 | NM_080617 | -1 | 0.001922952 | 0.58 (0.41, 0.82) | 4.60% | 1.03 | 1.28 |
| CCDC94 | NM_018074 | -1 | 0.002570559 | 0.57 (0.40, 0.82) | 5.40% | 1.03 | 1.28 |
| CDK5RAP2 | NM_001011649 | -1 | 0.001639429 | 0.57 (0.40, 0.81) | 4.10% | 1.03 | 1.29 |
| CDK5RAP2 | NM_018249 | -1 | 0.001639429 | 0.57 (0.40, 0.81) | 4.10% | 1.03 | 1.29 |
| CEP55 | NM_018131 | 1 | 0.002707603 | 2.06 (1.28, 3.30) | 5.50% | 1.03 | 1.26 |
| CEP55 | NM_001127182 | 1 | 0.002707603 | 2.06 (1.28, 3.30) | 5.50% | 1.03 | 1.26 |
| CHAT | NM_020986 | 1 | 0.001696264 | 1.39 (1.13, 1.70) | 4.20% | 1.03 | 1.26 |
| CHAT | NM_001142934 | 1 | 0.001696264 | 1.39 (1.13, 1.70) | 4.20% | 1.03 | 1.26 |
| CHAT | NM_020985 | 1 | 0.001696264 | 1.39 (1.13, 1.70) | 4.20% | 1.03 | 1.26 |
| CHAT | NM_001142933 | 1 | 0.001696264 | 1.39 (1.13, 1.70) | 4.20% | 1.03 | 1.26 |
| CHAT | NM_020549 | 1 | 0.001696264 | 1.39 (1.13, 1.70) | 4.20% | 1.03 | 1.26 |
| CHAT | NM_020984 | 1 | 0.001696264 | 1.39 (1.13,1.70) | 4.20% | 1.03 | 1.26 |
| CHAT | NM_001142929 | 1 | 0.001696264 | 1.39 (1.13, 1.70) | 4.20% | 1.03 | 1.26 |
| COL4A5 | NM_033380 | -1 | 0.002200738 | 0.63 (0.47, 0.85) | 5.00% | 1.03 | 1.28 |
| COL4A5 | NM_000495 | -1 | 0.002200738 | 0.63 (0.47, 0.85) | 5.00% | 1.03 | 1.28 |
| CRELD2 | NM_024324 | 1 | 0.001485273 | 1.40 (1.14, 1.72) | 3.90% | 1.03 | 1.27 |
| CRELD2 | NM_001135101 | 1 | 0.001485273 | 1.40 (1.14, 1.72) | 3.90% | 1.03 | 1.27 |
| CYB5D1 | NM_144607 | -1 | 0.001747826 | 0.58 (0.41, 0.81) | 4.30% | 1.03 | 1.29 |
| CYP19A1 | NM_000103 | 1 | 0.002444512 | 1.73 (1.21, 2.46) | 5.20% | 1.03 | 1.28 |
| CYP19A1 | NM_031226 | 1 | 0.002444512 | 1.73 (1.21, 2.46) | 5.20% | 1.03 | 1.28 |
| DDX20 | NM_007204 | -1 | 0.002289514 | 0.48 (0.30, 0.77) | 5.10% | 1.03 | 1.26 |
| DDX42 | NM_007372 | 1 | 0.001720318 | 1.44 (1.15, 1.81) | 4.20% | 1.03 | 1.28 |
| DDX42 | NM_203499 | 1 | 0.001720318 | 1.44 (1.15, 1.81) | 4.20% | 1.03 | 1.28 |
| DEF8 | NM_207514 | 1 | 0.002624521 | 2.11 (1.30, 3.43) | 5.50% | 1.03 | 1.26 |
| DEF8 | NM_017702 | 1 | 0.002624521 | 2.11 (1.30, 3.43) | 5.50% | 1.03 | 1.26 |
| DGAT1 | NM_012079 | 1 | 0.001720682 | 1.63 (1.20, 2.22) | 4.20% | 1.03 | 1.3 |
| DNAJC28 | NM_017833 | -1 | 0.001409688 | 0.66 (0.51, 0.85) | 3.80% | 1.03 | 1.29 |
| DNAJC28 | NM_001040192 | -1 | 0.001409688 | 0.66 (0.51, 0.85) | 3.80% | 1.03 | 1.29 |
| DYNC1LI2 | NM_006141 | 1 | 0.002089376 | 1.61 (1.19, 2.17) | 4.80% | 1.03 | 1.29 |
| E2F5 | NM_001951 | 1 | 0.002136056 | 2.12 (1.31, 3.43) | 4.90% | 1.03 | 1.27 |
| E2F5 | NM_001083588 | 1 | 0.002136056 | 2.12 (1.31, 3.43) | 4.90% | 1.03 | 1.27 |
| E2F5 | NM_001083589 | 1 | 0.002136056 | 2.12 (1.31, 3.43) | 4.90% | 1.03 | 1.27 |
| EGOT | NR_004428 | -1 | 0.001636301 | 0.65 (0.50, 0.85) | 4.10% | 1.03 | 1.29 |
| ELOVL2 | NM_017770 | -1 | 0.002382215 | 0.62 (0.45, 0.84) | 5.20% | 1.03 | 1.28 |
| ELP3 | NM_018091 | -1 | 0.002213346 | 0.61 (0.44, 0.84) | 5.00% | 1.03 | 1.29 |
| EVX2 | NM_001080458 | 1 | 0.001725038 | 1.37 (1.12, 1.66) | 4.20% | 1.03 | 1.26 |
| FANCD2 | NM_001018115 | 1 | 0.001545769 | 1.74 (1.23, 2.45) | 4.00% | 1.03 | 1.3 |
| FANCD2 | NM_033084 | 1 | 0.001545769 | 1.74 (1.23, 2.45) | 4.00% | 1.03 | 1.3 |
| FBN3 | NM_032447 | 1 | 0.001991297 | 1.57 (1.18, 2.10) | 4.70% | 1.03 | 1.29 |
| FEZF1 | NM_001160264 | 1 | 0.001027358 | 1.39 (1.14, 1.68) | 3.20% | 1.03 | 1.27 |
| FEZF1 | NM_001024613 | 1 | 0.001027358 | 1.39 (1.14, 1.68) | 3.20% | 1.03 | 1.27 |
| FHL3 | NM_004468 | -1 | 0.001474563 | 0.70 (0.56, 0.87) | 3.90% | 1.03 | 1.27 |
| FST | NM_013409 | -1 | 0.001727143 | 0.66 (0.51, 0.85) | 4.20% | 1.03 | 1.29 |
| FST | NM_006350 | -1 | 0.001727143 | 0.66 (0.51, 0.85) | 4.20% | 1.03 | 1.29 |
| FURIN | NM_002569 | 1 | 0.002269916 | 1.64 (1.19, 2.25) | 5.10% | 1.03 | 1.29 |
| FUT2 | NM_001097638 | -1 | 0.001525692 | 0.72 (0.59, 0.88) | 4.00% | 1.03 | 1.27 |
| FUT2 | NM_000511 | -1 | 0.001525692 | 0.72 (0.59, 0.88) | 4.00% | 1.03 | 1.27 |
| GGCX | NM_000821 | 1 | 0.002396435 | 1.80 (1.23, 2.62) | 5.20% | 1.03 | 1.28 |
| GGCX | NM_001142269 | 1 | 0.002396435 | 1.80 (1.23, 2.62) | 5.20% | 1.03 | 1.28 |
| GGH | NM_003878 | 1 | 0.002274114 | 2.01 (1.28, 3.16) | 5.10% | 1.03 | 1.27 |
| GLIPR1L2 | NM_152436 | -1 | 0.001862715 | 0.53 (0.36, 0.79) | 4.50% | 1.03 | 1.28 |
| GNG3 | NM_012202 | -1 | 0.002062355 | 0.64 (0.48, 0.85) | 4.80% | 1.03 | 1.29 |
| GRINA | NM_000837 | 1 | 0.001753597 | 1.62 (1.20, 2.20) | 4.30% | 1.03 | 1.3 |
| GRINA | NM_001009184 | 1 | 0.001753597 | 1.62 (1.20, 2.20) | 4.30% | 1.03 | 1.3 |
| GSTM4 | NR_024538 | -1 | 0.001336578 | 0.65 (0.50, 0.85) | 3.70% | 1.03 | 1.29 |
| GSTM4 | NM_147148 | -1 | 0.001336578 | 0.65 (0.50, 0.85) | 3.70% | 1.03 | 1.29 |
| GSTM4 | NM_000850 | -1 | 0.001336578 | 0.65 (0.50, 0.85) | 3.70% | 1.03 | 1.29 |
| HMMR | NM_001142557 | 1 | 0.002208484 | 2.21 (1.33, 3.66) | 5.00% | 1.03 | 1.26 |
| HMMR | NM_012485 | 1 | 0.002208484 | 2.21 (1.33, 3.66) | 5.00% | 1.03 | 1.26 |
| HMMR | NM_012484 | 1 | 0.002208484 | 2.21 (1.33, 3.66) | 5.00% | 1.03 | 1.26 |
| HMMR | NM_001142556 | 1 | 0.002208484 | 2.21 (1.33, 3.66) | 5.00% | 1.03 | 1.26 |
| HMX3 | NM_01105574 | 1 | 0.001618681 | 1.50 (1.17, 1.93) | 4.10% | 1.03 | 1.29 |
| HTATIP2 | NM_001098523 | 1 | 0.002516349 | 1.85 (1.24, 2.75) | 5.30% | 1.03 | 1.28 |
| HTATIP2 | NM_001098520 | 1 | 0.002516349 | 1.85 (1.24, 2.75) | 5.30% | 1.03 | 1.28 |
| HTATIP2 | NM_001098522 | 1 | 0.002516349 | 1.85 (1.24, 2.75) | 5.30% | 1.03 | 1.28 |
| HTATIP2 | NM_006410 | 1 | 0.002516349 | 1.85 (1.24, 2.75) | 5.30% | 1.03 | 1.28 |
| HTATIP2 | NM_001098521 | 1 | 0.002516349 | 1.85 (1.24, 2.75) | 5.30% | 1.03 | 1.28 |
| IFT57 | NM_018010 | -1 | 0.001435878 | 0.63 (0.47, 0.84) | 3.80% | 1.03 | 1.3 |
| IKBKB | NR_033819 | -1 | 0.002214905 | 0.58 (0.41, 0.82) | 5.00% | 1.03 | 1.28 |
| IKBKB | NM_001190721 | -1 | 0.002214905 | 0.58 (0.41, 0.82) | 5.00% | 1.03 | 1.28 |
| IKBKB | NM_001556 | -1 | 0.002214905 | 0.58 (0.41, 0.82) | 5.00% | 1.03 | 1.28 |
| IKBKB | NM_001190722 | -1 | 0.002214905 | 0.58 (0.41, 0.82) | 5.00% | 1.03 | 1.28 |
| IKBKB | NR_033818 | -1 | 0.002214905 | 0.58 (0.41, 0.82) | 5.00% | 1.03 | 1.28 |
| IKBKB | NM_001190720 | -1 | 0.002214905 | 0.58 (0.41, 0.82) | 5.00% | 1.03 | 1.28 |
| IKBKB | NR_033820 | -1 | 0.002214905 | 0.58 (0.41, 0.82) | 5.00% | 1.03 | 1.28 |
| INTS8 | NM_017864 | 1 | 0.002325883 | 1.59 (1.18, 2.13) | 5.10% | 1.03 | 1.29 |
| IYD | NM_001164694 | 1 | 0.002010449 | 1.71 (1.22, 2.40) | 4.70% | 1.03 | 1.29 |
| IYD | NM_001164695 | 1 | 0.002010449 | 1.71 (1.22, 2.40) | 4.70% | 1.03 | 1.29 |
| IYD | NM_203395 | 1 | 0.002010449 | 1.71 (1.22, 2.40) | 4.70% | 1.03 | 1.29 |
| KBTBD10 | NM_006063 | -1 | 0.001722602 | 0.60 (0.44, 0.83) | 4.20% | 1.03 | 1.29 |
| KIAA1310 | NM_017991 | -1 | 0.002113999 | 0.54 (0.37, 0.80) | 4.90% | 1.03 | 1.28 |
| KIAA1310 | NM_001115016 | -1 | 0.002113999 | 0.54 (0.37, 0.80) | 4.90% | 1.03 | 1.28 |
| KIF25 | NM_030615 | 1 | 0.002238069 | 1.56 (1.17, 2.08) | 5.00% | 1.03 | 1.29 |
| KIF25 | NM_005355 | 1 | 0.002238069 | 1.56 (1.17, 2.08) | 5.00% | 1.03 | 1.29 |
| KIF5C | NM_004522 | -1 | 0.002437989 | 0.52 (0.34, 0.79) | 5.20% | 1.03 | 1.27 |
| KLRC1 | NM_213657 | 1 | 0.002384431 | 1.66 (1.20, 2.31) | 5.20% | 1.03 | 1.29 |
| KLRC1 | NM_213658 | 1 | 0.002384431 | 1.66 (1.20, 2.31) | 5.20% | 1.03 | 1.29 |
| KLRC1 | NM_002259 | 1 | 0.002384431 | 1.66 (1.20, 2.31) | 5.20% | 1.03 | 1.29 |
| KLRC1 | NM_007328 | 1 | 0.002384431 | 1.66 (1.20, 2.31) | 5.20% | 1.03 | 1.29 |
| KLRD1 | NM_002262 | 1 | 0.002060071 | 1.75 (1.23, 2.50) | 4.80% | 1.03 | 1.29 |
| KLRD1 | NM_01114396 | 1 | 0.002060071 | 1.75 (1.23, 2.50) | 4.80% | 1.03 | 1.29 |
| KLRD1 | NM_007334 | 1 | 0.002060071 | 1.75 (1.23, 2.50) | 4.80% | 1.03 | 1.29 |
| LAD1 | NM_005558 | 1 | 0.001800788 | 1.98 (1.29, 3.04) | 4.30% | 1.03 | 1.28 |
| LHX5 | NM_022363 | 1 | 0.00111698 | 1.42 (1.15, 1.75) | 3.30% | 1.03 | 1.28 |
| LHX9 | NM_020204 | 1 | 0.00171801 | 1.41 (1.14, 1.74) | 4.20% | 1.03 | 1.27 |
| LHX9 | NM_001014434 | 1 | 0.00171801 | 1.41 (1.14, 1.74) | 4.20% | 1.03 | 1.27 |
| LOC100289187 | NM_001195543 | -1 | 0.002038702 | 0.55 (0.37, 0.80) | 4.70% | 1.03 | 1.28 |
| LOC100289187 | NM_001195542 | -1 | 0.002038702 | 0.55 (0.37, 0.80) | 4.70% | 1.03 | 1.28 |
| LOC100289187 | NM_001195541 | -1 | 0.002038702 | 0.55 (0.37, 0.80) | 4.70% | 1.03 | 1.28 |
| LOC145474 | NR_027046 | -1 | 0.001569524 | 0.70 (0.56, 0.87) | 4.10% | 1.03 | 1.27 |
| LOC284276 | NR_915417 | -1 | 0.002303624 | 0.46 (0.28, 0.76) | 5.10% | 1.03 | 1.26 |
| LOC648740 | NR_024438 | -1 | 0.002018901 | 0.63 (0.47, 0.84) | 4.70% | 1.03 | 1.29 |
| LRP2 | NM_004525 | -1 | 0.002286764 | 0.61 (0.44, 0.84) | 5.10% | 1.03 | 1.28 |
| LRRC39 | NM_144620 | -1 | 0.001617813 | 0.61 (0.45, 0.83) | 4.10% | 1.03 | 1.29 |
| LRRC48 | NM_031294 | -1 | 0.002131791 | 0.59 (0.42, 0.83) | 4.90% | 1.03 | 1.29 |
| LRRC48 | NM_001130092 | -1 | 0.002131791 | 0.59 (0.42, 0.83) | 4.90% | 1.03 | 1.29 |
| LRRC48 | NM_001130091 | -1 | 0.002131791 | 0.59 (0.42, 0.83) | 4.90% | 1.03 | 1.29 |
| LRRC48 | NM_001130090 | -1 | 0.002131791 | 0.59 (0.42,0.83) | 4.90% | 1.03 | 1.29 |
| METT10D | NM_024086 | -1 | 0.002064663 | 0.55 (0.37, 0.80) | 4.80% | 1.03 | 1.28 |
| MIR3143 | NR_036096 | 1 | 0.001719228 | 1.51 (1.17, 1.95) | 4.20% | 1.03 | 1.29 |
| MIR539 | NR_030256 | 1 | 0.001267367 | 1.53 (1.18, 1.98) | 3.60% | 1.03 | 1.3 |
| MRPL46 | NM_022163 | 1 | 0.002420243 | 1.72 (1.21, 2.44) | 5.20% | 1.03 | 1.29 |
| MRPS6 | NM_032476 | 1 | 0.00225102 | 1.79 (1.23, 2.61) | 5.00% | 1.03 | 1.29 |
| MUC21 | NM_001010909 | 1 | 0.002145168 | 1.56 (1.18, 2.08) | 4.90% | 1.03 | 1.29 |
| MYBL2 | NM_002466 | 1 | 0.002287866 | 1.94 (1.27, 2.96) | 5.10% | 1.03 | 1.28 |
| MYO15B | NR_003587 | -1 | 0.001947645 | 0.61 (0.44, 0.83) | 4.60% | 1.03 | 1.29 |
| NCAM2 | NM_004540 | -1 | 0.002198648 | 0.57 (0.39, 0.81) | 5.00% | 1.03 | 1.28 |
| NEIL3 | NM_018248 | 1 | 0.002666588 | 1.89 (1.25, 2.86) | 5.50% | 1.03 | 1.27 |
| NEK10 | NM_199347 | -1 | 0.002329341 | 0.54 (0.37, 0.81) | 5.10% | 1.03 | 1.28 |
| NEK2 | NM_002497 | 1 | 0.002473048 | 2.01 (1.28, 3.15) | 5.30% | 1.03 | 1.27 |
| NFXL1 | NM_152995 | 1 | 0.001183142 | 1.45 (1.16, 1.82) | 3.50% | 1.03 | 1.29 |
| NPPB | NM_002521 | 1 | 0.000858578 | 1.35 (1.13, 1.61) | 2.90% | 1.03 | 1.26 |
| OGN | NM_014057 | -1 | 0.002456696 | 0.45 (0.27, 0.75) | 5.30% | 1.03 | 1.25 |
| OGN | NM_033014 | -1 | 0.002456696 | 0.45 (0.27, 0.75) | 5.30% | 1.03 | 1.25 |
| PARD3B | NM_205863 | -1 | 0.001655273 | 0.57 (0.41, 0.81) | 4.10% | 1.03 | 1.29 |
| PARD3B | NM_057177 | -1 | 0.001655273 | 0.57 (0.41, 0.81) | 4.10% | 1.03 | 1.29 |
| PARD3B | NM_152526 | -1 | 0.001655273 | 0.57 (0.41, 0.81) | 4.10% | 1.03 | 1.29 |
| PCM1 | NM_006197 | -1 | 0.002032488 | 0.50 (0.32, 0.78) | 4.70% | 1.03 | 1.27 |
| PDE11A | NM_001077197 | -1 | 0.001651988 | 0.64 (0.48, 0.84) | 4.10% | 1.03 | 1.29 |
| PDE11A | NM_016953 | -1 | 0.001651988 | 0.64 (0.48, 0.84) | 4.10% | 1.03 | 1.29 |
| PDE11A | NM_001077196 | -1 | 0.001651988 | 0.64 (0.48, 0.84) | 4.10% | 1.03 | 1.29 |
| PDE11A | NM_001077358 | -1 | 0.001651988 | 0.64 (0.48, 0.84) | 4.10% | 1.03 | 1.29 |
| PDIA6 | NM_005742 | 1 | 0.001634746 | 1.67 (1.21, 2.29) | 4.10% | 1.03 | 1.3 |
| PEX1 | NM_000466 | -1 | 0.002362939 | 0.61 (0.44, 0.84) | 5.20% | 1.03 | 1.28 |
| PEX13 | NM_002618 | 1 | 0.001593701 | 1.69 (1.22, 2.34) | 4.10% | 1.03 | 1.3 |
| PGCP | NM_016134 | -1 | 0.001725315 | 0.60 (0.44, 0.83) | 4.20% | 1.03 | 1.29 |
| PGR | NM_000926 | -1 | 0.001909419 | 0.57 (0.40, 0.81) | 4.50% | 1.03 | 1.29 |
| PHF2P1 | NR_002801 | 1 | 0.001167977 | 1.35 (1.13, 1.62) | 3.40% | 1.03 | 1.26 |
| PIK3C2A | NM_002645 | -1 | 0.001585299 | 0.59 (0.43, 0.82) | 4.10% | 1.03 | 1.3 |
| PMAIP1 | NM_021127 | -1 | 0.001954098 | 0.64 (0.49, 0.85) | 4.60% | 1.03 | 1.29 |
| POMC | NM_001035256 | 1 | 0.001777068 | 1.50 (1.16, 1.93) | 4.30% | 1.03 | 1.29 |
| POMC | NM_000939 | 1 | 0.001777068 | 1.50 (1.16, 1.93) | 4.30% | 1.03 | 1.29 |
| PPP1R2P1 | NR_027771 | 1 | 0.00211075 | 1.58 (1.18, 2.12) | 4.90% | 1.03 | 1.29 |
| PRC1 | NM_0003981 | 1 | 0.0023281 | 1.83 (1.24, 2.70) | 5.10% | 1.03 | 1.28 |
| PRC1 | NM_199414 | 1 | 0.0023281 | 1.83 (1.24, 2.70) | 5.10% | 1.03 | 1.28 |
| PRC1 | NM_199413 | 1 | 0.0023281 | 1.83 (1.24, 2.70) | 5.10% | 1.03 | 1.28 |
| PRSS41 | NM_01135086 | 1 | 0.000819741 | 1.29 (1.11, 1.50) | 2.80% | 1.03 | 1.24 |
| PVRL4 | NM_030916 | 1 | 0.002436856 | 1.77 (1.22, 2.55) | 5.20% | 1.03 | 1.28 |
| PVT1 | NR_003367 | -1 | 0.000985501 | 0.76 (0.65, 0.89) | 3.10% | 1.03 | 1.24 |
| PYCRL | NM_023078 | 1 | 0.002594279 | 2.02 (1.28, 3.20) | 5.40% | 1.03 | 1.27 |
| RAB1A | NM_004161 | 1 | 0.001769368 | 1.72 (1.22, 2.42) | 4.30% | 1.03 | 1.3 |
| RAB1A | NM_015543 | 1 | 0.001769368 | 1.72 (1.22, 2.42) | 4.30% | 1.03 | 1.3 |
| RAB9BP1 | NR_000039 | 1 | 0.0016054 | 1.48 (1.16, 1.89) | 4.10% | 1.03 | 1.29 |
| RAET1K | NR_024045 | 1 | 0.001800197 | 1.51 (1.16, 1.95) | 4.30% | 1.03 | 1.29 |
| RAPGEF5 | NM_012294 | 1 | 0.002154043 | 1.56 (1.17, 2.08) | 4.90% | 1.03 | 1.29 |
| RBM24 | NM_001143941 | -1 | 0.002564648 | 0.51 (0.33, 0.79) | 5.40% | 1.03 | 1.27 |
| RBM24 | NM_153020 | -1 | 0.002564648 | 0.51 (0.33, 0.79) | 5.40% | 1.03 | 1.27 |
| RBM24 | NM_001143942 | -1 | 0.002564648 | 0.51 (0.33, 0.79) | 5.40% | 1.03 | 1.27 |
| RPL6 | NM_000970 | -1 | 0.001139844 | 0.71 (0.57, 0.87) | 3.40% | 1.03 | 1.28 |
| RPL6 | NM_ 001024662 | -1 | 0.001139844 | 0.71 (0.57, 0.87) | 3.40% | 1.03 | 1.28 |
| SALL2 | NM_005407 | -1 | 0.001427511 | 0.66 (0.52, 0.85) | 3.80% | 1.03 | 1.29 |
| SETBP1 | NM_015559 | -1 | 0.002219007 | 0.52 (0.34, 0.79) | 5.00% | 1.03 | 1.27 |
| SETBP1 | NM_ 001130110 | -1 | 0.002219007 | 0.52 (0.34, 0.79) | 5.00% | 1.03 | 1.27 |
| SFI1 | NM_001007467 | -1 | 0.002372759 | 0.62 (0.45, 0.84) | 5.20% | 1.03 | 1.28 |
| SFI1 | NM_014775 | -1 | 0.002372759 | 0.62 (0.45, 0.84) | 5.20% | 1.03 | 1.28 |
| SHC2 | NM_012435 | -1 | 0.001912735 | 0.57 (0.41, 0.82) | 4.50% | 1.03 | 1.29 |
| SKA3 | NM_145061 | 1 | 0.002169941 | 2.22 (1.33, 3.69) | 4.90% | 1.03 | 1.26 |
| SKA3 | NM_001166017 | 1 | 0.002169941 | 2.22 (1.33, 3.69) | 4.90% | 1.03 | 1.26 |
| SLC25A12 | NM_003705 | -1 | 0.002428642 | 0.58 (0.40, 0.82) | 5.20% | 1.03 | 1.28 |
| SNCB | NM_001001502 | 1 | 0.00093158 | 1.37 (1.14, 1.66) | 3.00% | 1.03 | 1.27 |
| SNCB | NM_003085 | 1 | 0.00093158 | 1.37 (1.14, 1.66) | 3.00% | 1.03 | 1.27 |
| SNORA4 | NR_02588 | 1 | 0.002448167 | 1.75 (1.22, 2.50) | 5.20% | 1.03 | 1.28 |
| SNX29 | NML001080530 | -1 | 0.002032127 | 0.66 (0.51, 0.86) | 4.70% | 1.03 | 1.28 |
| SPRYD5 | NM_0032681 | 1 | 0.001186552 | 1.40 (1.14, 1.72) | 3.50% | 1.03 | 1.27 |
| SRD5A1 | NM_001047 | 1 | 0.001898168 | 1.80 (1.24, 2.62) | 4.50% | 1.03 | 1.29 |
| SRM | NM_003132 | 1 | 0.00241018 | 2.18 (1.32, 3.59) | 5.20% | 1.03 | 1.26 |
| STK36 | NM_015690 | -1 | 0.001844542 | 0.61 (0.44, 0.83) | 4.40% | 1.03 | 1.29 |
| SUCLG1 | NM_003849 | 1 | 0.00282029 | 2.20 (1.31, 3.68) | 5.60% | 1.03 | 1.25 |
| SYNC | NM_030786 | -1 | 0.001703858 | 0.62 (0.46, 0.84) | 4.20% | 1.03 | 1.29 |
| SYNC | NM_001161708 | -1 | 0.001703858 | 0.62 (0.46, 0.84) | 4.20% | 1.03 | 1.29 |
| TBX3 | NM_005996 | -1 | 0.001955061 | 0.60 (0.44, 0.83) | 4.60% | 1.03 | 1.29 |
| TBX3 | NM_016569 | -1 | 0.001955061 | 0.60 (0.44, 0.83) | 4.60% | 1.03 | 1.29 |
| TMEM165 | NM_018475 | 1 | 0.00206432 | 1.59 (1.18, 2.13) | 4.80% | 1.03 | 1.29 |
| TMEM214 | NM_017727 | 1 | 0.001586499 | 1.76 (1.24, 2.50) | 4.10% | 1.03 | 1.3 |
| TMEM214 | NM_001083590 | 1 | 0.001586499 | 1.76 (1.24, 2.50) | 4.10% | 1.03 | 1.3 |
| TPRG1 | NM_198485 | 1 | 0.001640284 | 0.56 (0.39, 0.81) | 4.10% | 1.03 | 1.29 |
| TRIM10 | NM_052828 | 1 | 0.000954844 | 1.38 (1.14, 1.68) | 3.10% | 1.03 | 1.27 |
| TRIM10 | NM_006778 | 1 | 0.000954844 | 1.38 (1.14, 1.68) | 3.10% | 1.03 | 1.27 |
| TRIM8 | NM_030912 | -1 | 0.001839592 | 0.60 (0.44, 0.83) | 4.40% | 1.03 | 1.29 |
| UBA6 | NM_018227 | 1 | 0.001297805 | 1.52 (1.18, 1.96) | 3.60% | 1.03 | 1.3 |
| UGT2A1 | NM_006798 | 1 | 0.001411139 | 1.31 (1.11, 1.55) | 3.80% | 1.03 | 1.24 |
| USP16 | NM_006447 | -1 | 0.001528441 | 0.58 (0.41, 0.81) | 4.00% | 1.03 | 1.3 |
| USP16 | NM_001032410 | -1 | 0.001528441 | 0.58 (0.41, 0.81) | 4.00% | 1.03 | 1.3 |
| USP16 | NM_001001992 | -1 | 0.001528441 | 0.58 (0.41, 0.81) | 4.00% | 1.03 | 1.3 |
| WDR26 | NM_025160 | 1 | 0.002023353 | 1.90 (1.26, 2.85) | 4.70% | 1.03 | 1.28 |
| WDR26 | NM_01115113 | 1 | 0.002023353 | 1.90 (1.26, 2.85) | 4.70% | 1.03 | 1.28 |
| WDR81 | NM_001163673 | -1 | 0.001691753 | 0.60 (0.43, 0.82) | 4.20% | 1.03 | 1.29 |
| WDR81 | NM_152348 | -1 | 0.001691753 | 0.60 (0.43, 0.82) | 4.20% | 1.03 | 1.29 |
| WDR81 | NM_001163811 | -1 | 0.001691753 | 0.60 (0.43, 0.82) | 4.20% | 1.03 | 1.29 |
| WDR81 | NM_LP01163809 | -1 | 0.001691753 | 0.60 (0.43, 0.82) | 4.20% | 1.03 | 1.29 |
| YPEL3 | NM_001145524 | -1 | 0.001617231 | 0.58 (0.41, 0.81) | 4.10% | 1.03 | 1.29 |
| YPEL3 | NM_031477 | -1 | 0.001617231 | 0.58 (0.41, 0.81) | 4.10% | 1.03 | 1.29 |
| ZBTB1 | NM_001123329 | -1 | 0.00145875 | 0.67 (0.53, 0.86) | 3.90% | 1.03 | 1.29 |
| ZBTB1 | NM_014950 | -1 | 0.00145875 | 0.67 (0.53, 0.86) | 3.90% | 1.03 | 1.29 |
| ZFHX2 | NM_033400 | -1 | 0.002397291 | 0.58 (0.41, 0.82) | 5.20% | 1.03 | 1.28 |
| ZMAT1 | NR_036431 | -1 | 0.002074568 | 0.55 (0.38, 0.81) | 4.80% | 1.03 | 1.28 |
| ZMAT1 | NM_001011657 | -1 | 0.002074568 | 0.55 (0.38, 0.81) | 4.80% | 1.03 | 1.28 |
| ZNF219 | NM_016423 | -1 | 0.001654385 | 0.59 (0.43, 0.82) | 4.10% | 1.03 | 1.29 |
| ZNF219 | NM_001101672 | -1 | 0.001654385 | 0.59 (0.43, 0.82) | 4.10% | 1.03 | 1.29 |
| ZNF219 | NM_001102454 | -1 | 0.001654385 | 0.59 (0.43, 0.82) | 4.10% | 1.03 | 1.29 |
| ZNF682 | NM_001077349 | -1 | 0.001906028 | 0.61 (0.44, 0.83) | 4.50% | 1.03 | 1.29 |
| ZNF682 | NM_033196 | -1 | 0.001906028 | 0.61 (0.44, 0.83) | 4.50% | 1.03 | 1.29 |
| ZNF705D | NM_001039615 | 1 | 0.001227404 | 1.35 (1.13, 1.62) | 3.50% | 1.03 | 1.26 |
| ZNF747 | NM_023931 | -1 | 0.002131843 | 0.64 (0.48, 0.85) | 4.90% | 1.03 | 1.28 |
| ZNF808 | NM_001039886 | -1 | 0.002223951 | 0.52 (0.34, 0.79) | 5.00% | 1.03 | 1.27 |
| ABCA11P | NR_002451 | -1 | 0.002873693 | 0.67 (0.51, 0.87) | 5.70% | 1.02 | 1.27 |
| ABCA12 | NM_015657 | 1 | 0.00300345 | 1.71 (1.20, 2.44) | 5.80% | 1.02 | 1.28 |
| ABCA12 | NM_173076 | 1 | 0.00300345 | 1.71 (1.20, 2.44) | 5.80% | 1.02 | 1.28 |
| AMT | NM_000481 | -1 | 0.002804366 | 0.65 (0.50, 0.86) | 5.60% | 1.02 | 1.27 |
| AMT | NM_001164711 | -1 | 0.002804366 | 0.65 (0.50, 0.86) | 5.60% | 1.02 | 1.27 |
| AMT | NM_001164710 | -1 | 0.002804366 | 0.65 (0.50, 0.86) | 5.60% | 1.02 | 1.27 |
| AMT | NM_001164712 | -1 | 0.002804366 | 0.65 (0.50, 0.86) | 5.60% | 1.02 | 1.27 |
| AMT | NR_028435 | -1 | 0.002804366 | 0.65 (0.50, 0.86) | 5.60% | 1.02 | 1.27 |
| ANAPC2 | NM_013366 | -1 | 0.00312125 | 0.66 (0.50, 0.87) | 6.00% | 1.02 | 1.27 |
| ANKRA2 | NM_023039 | -1 | 0.002819729 | 0.55 (0.37, 0.81) | 5.60% | 1.02 | 1.27 |
| AP2M1 | NM_001025205 | 1 | 0.002571858 | 1.57 (1.17, 2.11) | 5.40% | 1.02 | 1.28 |
| AP2M1 | NM_004068 | 1 | 0.002571858 | 1.57 (1.17, 2.11) | 5.40% | 1.02 | 1.28 |
| ARF1 | NM_001024227 | 1 | 0.003644362 | 1.90 (1.23, 2.92) | 6.50% | 1.02 | 1.26 |
| ARF1 | NM_001658 | 1 | 0.003644362 | 1.90 (1.23, 2.92) | 6.50% | 1.02 | 1.26 |
| ARF1 | NM_001024228 | 1 | 0.003644362 | 1.90 (1.23, 2.92) | 6.50% | 1.02 | 1.26 |
| ARF1 | NM_001024226 | 1 | 0.003644362 | 1.90 (1.23, 2.92) | 6.50% | 1.02 | 1.26 |
| ATAD1 | NM_032810 | -1 | 0.004007343 | 0.63 (0.45, 0.86) | 6.80% | 1.02 | 1.27 |
| ATOH7 | NM_145178 | 1 | 0.00372128 | 1.46 (1.13, 1.90) | 6.60% | 1.02 | 1.26 |
| ATP9B | NM_198531 | -1 | 0.002646669 | 0.58 (0.41, 0.83) | 5.50% | 1.02 | 1.28 |
| ATXN3L | NM_001135995 | 1 | 0.002748019 | 1.28 (1.09, 1.50) | 5.60% | 1.02 | 1.22 |
| BCL2L2 | NM_004050 | -1 | 0.003979713 | 0.49 (0.31, 0.80) | 6.80% | 1.02 | 1.24 |
| BCL2L2 | NM_001199839 | -1 | 0.003979713 | 0.49 (0.31, 0.80) | 6.80% | 1.02 | 1.24 |
| BENDS | NM_024603 | -1 | 0.00262047 | 0.59 (0.42, 0.83) | 5.50% | 1.02 | 1.28 |
| BLM | NM_000057 | 1 | 0.003237886 | 1.81 (1.22, 2.69) | 6.10% | 1.02 | 1.27 |
| C10orf79 | NM_025145 | -1 | 0.003108477 | 0.64 (0.48, 0.86) | 6.00% | 1.02 | 1.27 |
| C10orf91 | NM_173541 | 1 | 0.002971656 | 1.41 (1.13, 1.78) | 5.80% | 1.02 | 1.26 |
| C14orf34 | NR_026796 | 1 | 0.0029697 | 1.65 (1.19, 2.29) | 5.80% | 1.02 | 1.28 |
| C14orf34 | NR_026797 | 1 | 0.0029697 | 1.65 (1.19, 2.29) | 5.80% | 1.02 | 1.28 |
| C15orf39 | NM_015492 | 1 | 0.002808036 | 1.64 (1.19, 2.28) | 5.60% | 1.02 | 1.28 |
| C15orf42 | NM_152259 | 1 | 0.004003005 | 1.73 (1.19, 2.51) | 6.80% | 1.02 | 1.27 |
| C17orf48 | NM_020233 | -1 | 0.003577923 | 0.69 (0.54, 0.89) | 6.40% | 1.02 | 1.26 |
| C17orf49 | NM_001142798 | -1 | 0.003771563 | 0.57 (0.39, 0.83) | 6.60% | 1.02 | 1.26 |
| C17orf49 | NM_174893 | -1 | 0.003771563 | 0.57 (0.39, 0.83) | 6.60% | 1.02 | 1.26 |
| C17orf49 | NM_001142799 | -1 | 0.003771563 | 0.57 (0.39, 0.83) | 6.60% | 1.02 | 1.26 |
| C18orf26 | NM_173629 | 1 | 0.002705318 | 1.52 (1.16, 1.99) | 5.50% | 1.02 | 1.28 |
| C1orf131 | NM_152379 | 1 | 0.003424964 | 1.93 (1.24, 2.99) | 6.30% | 1.02 | 1.26 |
| C1orf173 | NM_001002912 | -1 | 0.003149398 | 0.57 (0.39, 0.83) | 6.00% | 1.02 | 1.27 |
| Clorf63 | NM_020317 | -1 | 0.004174801 | 0.53 (0.34, 0.82) | 7.00% | 1.02 | 1.25 |
| C20orf26 | NM_001167816 | -1 | 0.004008624 | 0.56 (0.38, 0.83) | 6.80% | 1.02 | 1.26 |
| C20orf26 | NM_015585 | -1 | 0.004008624 | 0.56 (0.38, 0.83) | 6.80% | 1.02 | 1.26 |
| C3orf25 | NM_207307 | -1 | 0.004109239 | 0.58 (0.39, 0.84) | 6.90% | 1.02 | 1.26 |
| C4orf32 | NM_152400 | -1 | 0.00283046 | 0.60 (0.43, 0.84) | 5.60% | 1.02 | 1.28 |
| C6orf97 | NM_025059 | -1 | 0.003169135 | 0.63 (0.46, 0.86) | 6.00% | 1.02 | 1.27 |
| C7orf54 | NR_027330 | 1 | 0.002867207 | 1.97 (1.26, 3.07) | 5.70% | 1.02 | 1.27 |
| C9orf53 | NR_024274 | 1 | 0.002759518 | 1.53 (1.16, 2.03) | 5.60% | 1.02 | 1.28 |
| CAP2 | NM_006366 | -1 | 0.002320274 | 0.65 (0.49, 0.86) | 5.10% | 1.02 | 1.28 |
| CASP14 | NM_012114 | 1 | 0.003818253 | 1.57 (1.16, 2.13) | 6.70% | 1.02 | 1.27 |
| CBX2 | NM_032647 | 1 | 0.003622138 | 1.74 (1.20, 2.53) | 6.50% | 1.02 | 1.27 |
| CBX2 | NM_005189 | 1 | 0.003622138 | 1.74 (1.20, 2.53) | 6.50% | 1.02 | 1.27 |
| CCDC104 | NM_080667 | -1 | 0.003352077 | 0.57 (0.39, 0.83) | 6.20% | 1.02 | 1.27 |
| CCDC3 | NM_031455 | -1 | 0.002624067 | 0.58 (0.41, 0.83) | 5.50% | 1.02 | 1.28 |
| CCDC76 | NM_019083 | -1 | 0.003763724 | 0.52 (0.34, 0.81) | 6.60% | 1.02 | 1.25 |
| CCNH | NM_001239 | -1 | 0.002673892 | 0.64 (0.48, 0.86) | 5.50% | 1.02 | 1.28 |
| CCNH | NM_00199189 | -1 | 0.002673892 | 0.64 (0.48, 0.86) | 5.50% | 1.02 | 1.28 |
| CD1E | NM_001042583 | -1 | 0.002526018 | 0.60 (0.44, 0.84) | 5.40% | 1.02 | 1.28 |
| CD1E | NM_001185112 | -1 | 0.002526018 | 0.60 (0.44, 0.84) | 5.40% | 1.02 | 1.28 |
| CD1E | NM_001185113 | -1 | 0.002526018 | 0.60 (0.44, 0.84) | 5.40% | 1.02 | 1.28 |
| CD1E | NM_001185114 | -1 | 0.002526018 | 0.60 (0.44, 0.84) | 5.40% | 1.02 | 1.28 |
| CD1E | NM_001185110 | -1 | 0.002526018 | 0.60 (0.44, 0.84) | 5.40% | 1.02 | 1.28 |
| CD1E | NM_001042584 | -1 | 0.002526018 | 0.60 (0.44, 0.84) | 5.40% | 1.02 | 1.28 |
| CD1E | NM_001185108 | -1 | 0.002526018 | 0.60 (0.44, 0.84) | 5.40% | 1.02 | 1.28 |
| CD1E | NM_001042585 | -1 | 0.002526018 | 0.60 (0.44, 0.84) | 5.40% | 1.02 | 1.28 |
| CD1E | NM_001042586 | -1 | 0.002526018 | 0.60 (0.44, 0.84) | 5.40% | 1.02 | 1.28 |
| CD1E | NM_001042587 | -1 | 0.002526018 | 0.60 (0.44, 0.84) | 5.40% | 1.02 | 1.28 |
| CD1E | NM_001185107 | -1 | 0.002526018 | 0.60 (0.44, 0.84) | 5.40% | 1.02 | 1.28 |
| CD1E | NM_030893 | -1 | 0.002526018 | 0.60 (0.44, 0.84) | 5.40% | 1.02 | 1.28 |
| CD1E | NM_001185115 | -1 | 0.002526018 | 0.60 (0.44, 0.84) | 5.40% | 1.02 | 1.28 |
| CDC6 | NM_001254 | 1 | 0.002718983 | 1.76 (1.22, 2.55) | 5.50% | 1.02 | 1.28 |
| CDRT1 | NM_006382 | 1 | 0.003880336 | 1.65 (1.17, 2.32) | 6.70% | 1.02 | 1.27 |
| CENPE | NM_001813 | 1 | 0.002827598 | 1.93 (1.25, 2.97) | 5.60% | 1.02 | 1.27 |
| CHEK1 | NM_001274 | 1 | 0.002862306 | 1.81 (1.23, 2.67) | 5.70% | 1.02 | 1.28 |
| CHEK1 | NM_001114122 | 1 | 0.002862306 | 1.81 (1.23, 2.67) | 5.70% | 1.02 | 1.28 |
| CHEK1 | NM_001114121 | 1 | 0.002862306 | 1.81 (1.23, 2.67) | 5.70% | 1.02 | 1.28 |
| CHML | NM_001821 | 1 | 0.003315346 | 1.64 (1.18, 2.29) | 6.20% | 1.02 | 1.28 |
| CIRBP | NM_001280 | -1 | 0.004062264 | 0.61 (0.43, 0.85) | 6.90% | 1.02 | 1.27 |
| CIRBP | NR_023313 | -1 | 0.004062264 | 0.61 (0.43, 0.85) | 6.90% | 1.02 | 1.27 |
| CIRBP | NR_023312 | -1 | 0.004062264 | 0.61 (0.43, 0.85) | 6.90% | 1.02 | 1.27 |
| CLCN1 | NM_000083 | 1 | 0.003227573 | 1.40 (1.12, 1.75) | 6.10% | 1.02 | 1.26 |
| CMAS | NM_018686 | 1 | 0.002866433 | 1.48 (1.14, 1.91) | 5.70% | 1.02 | 1.27 |
| CNGB3 | NM_019098 | 1 | 0.00409123 | 1.81 (1.21, 2.71) | 6.90% | 1.02 | 1.26 |
| COL6A4P1 | NR_027927 | 1 | 0.003761834 | 1.50 (1.14, 1.97) | 6.60% | 1.02 | 1.27 |
| COX8C | NM_182971 | 1 | 0.003378935 | 1.36 (1.11, 1.68) | 6.20% | 1.02 | 1.25 |
| CPLX1 | NM_006651 | -1 | 0.002909325 | 0.64 (0.47, 0.86) | 5.70% | 1.02 | 1.28 |
| CRABP1 | NM_004378 | 1 | 0.002927588 | 1.69 (1.20, 2.38) | 5.70% | 1.02 | 1.28 |
| CREBBP | NM_004380 | -1 | 0.003871914 | 0.54 (0.36, 0.82) | 6.70% | 1.02 | 1.26 |
| CREBBP | NM_001079846 | -1 | 0.003871914 | 0.54 (0.36, 0.82) | 6.70% | 1.02 | 1.26 |
| CSDC2 | NM_014460 | -1 | 0.004125038 | 0.59 (0.41, 0.84) | 6.90% | 1.02 | 1.26 |
| CYC1 | NM_001916 | 1 | 0.003973641 | 1.69 (1.18, 2.42) | 6.80% | 1.02 | 1.27 |
| DEFB135 | NM_001033017 | 1 | 0.002321015 | 1.51 (1.16, 1.96) | 5.10% | 1.02 | 1.28 |
| DIAPH1 | NM_005219 | 1 | 0.004027699 | 1.70 (1.18, 2.44) | 6.90% | 1.02 | 1.27 |
| DIAPH1 | NM_001079812 | 1 | 0.004027699 | 1.70 (1.18, 2.44) | 6.90% | 1.02 | 1.27 |
| DIXDC1 | NM_01037954 | -1 | 0.003336293 | 0.60 (0.43, 0.84) | 6.20% | 1.02 | 1.27 |
| DIXDC1 | NM_033425 | -1 | 0.003336293 | 0.60 (0.43, 0.84) | 6.20% | 1.02 | 1.27 |
| DKK3 | NM_013253 | -1 | 0.00325914 | 0.70 (0.55, 0.89) | 6.10% | 1.02 | 1.26 |
| DKK3 | NM_015881 | -1 | 0.00325914 | 0.70 (0.55, 0.89) | 6.10% | 1.02 | 1.26 |
| DKK3 | NM_001018057 | -1 | 0.00325914 | 0.70 (0.55, 0.89) | 6.10% | 1.02 | 1.26 |
| DNAJC12 | NM_021800 | -1 | 0.003141124 | 0.61 (0.43, 0.84) | 6.00% | 1.02 | 1.27 |
| DNAJC12 | NM_201262 | -1 | 0.003141124 | 0.61 (0.43, 0.84) | 6.00% | 1.02 | 1.27 |
| DNMT3B | NM_175848 | 1 | 0.002642423 | 1.68 (1.20, 2.36) | 5.50% | 1.02 | 1.28 |
| DNMT3B | NM_006892 | 1 | 0.002642423 | 1.68 (1.20, 2.36) | 5.50% | 1.02 | 1.28 |
| DNMT3B | NM_175849 | 1 | 0.002642423 | 1.68 (1.20, 2.36) | 5.50% | 1.02 | 1.28 |
| DNMT3B | NM_175850 | 1 | 0.002642423 | 1.68 (1.20, 2.36) | 5.50% | 1.02 | 1.28 |
| DOC2GP | NR_033791 | 1 | 0.001636762 | 1.29 (1.10, 1.52) | 4.10% | 1.02 | 1.23 |
| DOK1 | NM_001381 | -1 | 0.002983362 | 0.54 (0.35, 0.81) | 5.80% | 1.02 | 1.27 |
| DOK1 | NM_001197260 | -1 | 0.002983362 | 0.54 (0.35, 0.81) | 5.80% | 1.02 | 1.27 |
| DUSP13 | NM_001007273 | 1 | 0.002852767 | 1.66 (1.19, 2.30) | 5.70% | 1.02 | 1.28 |
| DUSP13 | NM_001007271 | 1 | 0.002852767 | 1.66 (1.19, 2.30) | 5.70% | 1.02 | 1.28 |
| DUSP13 | NM_001007272 | 1 | 0.002852767 | 1.66 (1.19, 2.30) | 5.70% | 1.02 | 1.28 |
| DUSP13 | NM_016364 | 1 | 0.002852767 | 1.66 (1.19, 2.30) | 5.70% | 1.02 | 1.28 |
| E2F1 | NM_005225 | 1 | 0.002966928 | 1.81 (1.22, 2.68) | 5.80% | 1.02 | 1.27 |
| ESRP2 | NM_024939 | 1 | 0.004340825 | 1.90 (1.22, 2.96) | 7.10% | 1.02 | 1.25 |
| EVL | NM_016337 | -1 | 0.003294207 | 0.50 (0.31, 0.79) | 6.20% | 1.02 | 1.25 |
| FADS1 | NM_013402 | 1 | 0.003861317 | 1.69 (1.18, 2.41) | 6.70% | 1.02 | 1.27 |
| FAM110B | NM_147189 | -1 | 0.002415136 | 0.69 (0.54, 0.88) | 5.20% | 1.02 | 1.27 |
| FAM161A | NM_032180 | -1 | 0.003214575 | 0.68 (0.52, 0.88) | 6.10% | 1.02 | 1.27 |
| FAM26E | NM_153711 | -1 | 0.003603321 | 0.63 (0.46, 0.86) | 6.40% | 1.02 | 1.27 |
| FBP1 | NM_000507 | -1 | 0.004003056 | 0.59 (0.41, 0.85) | 6.80% | 1.02 | 1.26 |
| FBP1 | NM_001127628 | -1 | 0.004003056 | 0.59 (0.41, 0.85) | 6.80% | 1.02 | 1.26 |
| FLI37201 | NR_026835 | -1 | 0.003681387 | 0.62 (0.45, 0.86) | 6.50% | 1.02 | 1.27 |
| FRY | NM_023037 | -1 | 0.002884774 | 0.53 (0.35,0.80) | 5.70% | 1.02 | 1.27 |
| GSTA2 | NM_000846 | 1 | 0.003594941 | 1.43 (1.12, 1.82) | 6.40% | 1.02 | 1.26 |
| GUCA1A | NM_000409 | 1 | 0.003909224 | 1.58 (1.16, 2.15) | 6.70% | 1.02 | 1.27 |
| HIST1H1C | NM_005319 | 1 | 0.003225985 | 1.68 (1.19, 2.38) | 6.10% | 1.02 | 1.28 |
| HIST1H2AG | NM_021064 | 1 | 0.003513384 | 2.14 (1.28, 3.56) | 6.40% | 1.02 | 1.25 |
| HIST1H2AH | NM_080596 | 1 | 0.003239424 | 1.96 (1.25, 3.06) | 6.10% | 1.02 | 1.26 |
| HIST1H2AL | NM_003511 | 1 | 0.003196371 | 2.03 (1.27, 3.26) | 6.10% | 1.02 | 1.26 |
| HJURP | NM_018410 | 1 | 0.003065128 | 1.91 (1.24, 2.93) | 5.90% | 1.02 | 1.27 |
| HMGB3 | NM_005342 | 1 | 0.003821392 | 1.67 (1.18, 2.35) | 6.70% | 1.02 | 1.27 |
| HPGDS | NM_014485 | -1 | 0.003509052 | 0.67 (0.51, 0.88) | 6.40% | 1.02 | 1.27 |
| ICA1L | NM_138468 | -1 | 0.003324089 | 0.60 (0.42, 0.84) | 6.20% | 1.02 | 1.27 |
| ICA1L | NM_178231 | -1 | 0.003324089 | 0.60 (0.42, 0.84) | 6.20% | 1.02 | 1.27 |
| INCA1 | NM_001167987 | -1 | 0.003836717 | 0.62 (0.45, 0.86) | 6.70% | 1.02 | 1.27 |
| INCA1 | NM_001167986 | -1 | 0.003836717 | 0.62 (0.45, 0.86) | 6.70% | 1.02 | 1.27 |
| INCA1 | NM_001167985 | -1 | 0.003836717 | 0.62 (0.45, 0.86) | 6.70% | 1.02 | 1.27 |
| INCA1 | NM_213726 | -1 | 0.003836717 | 0.62 (0.45, 0.86) | 6.70% | 1.02 | 1.27 |
| KIAA0556 | NM_015202 | -1 | 0.00378804 | 0.58 (0.40, 0.84) | 6.70% | 1.02 | 1.26 |
| KIAA0649 | NM_014911 | -1 | 0.003489552 | 0.60 (0.43, 0.85) | 6.30% | 1.02 | 1.27 |
| KIAA1632 | NM_020964 | -1 | 0.004110188 | 0.57 (0.39, 0.84) | 6.90% | 1.02 | 1.26 |
| KIF11 | NM_004523 | 1 | 0.003849177 | 1.77 (1.20, 2.61) | 6.70% | 1.02 | 1.27 |
| KIF13B | NM_015254 | -1 | 0.003183516 | 0.56 (0.38, 0.82) | 6.00% | 1.02 | 1.27 |
| KIF18B | NM_001080443 | 1 | 0.004363327 | 1.89 (1.22, 2.92) | 7.10% | 1.02 | 1.25 |
| KIF2C | NM_006845 | 1 | 0.004041299 | 2.10 (1.27, 3.49) | 6.90% | 1.02 | 1.24 |
| KLHDC2 | NM_014315 | -1 | 0.002795444 | 0.59 (0.42, 0.84) | 5.60% | 1.02 | 1.28 |
| KLHDC4 | NM_001184856 | 1 | 0.004284709 | 2.00 (1.24, 3.21) | 7.10% | 1.02 | 1.25 |
| KLHDC4 | NM_001184854 | 1 | 0.004284709 | 2.00 (1.24, 3.21) | 7.10% | 1.02 | 1.25 |
| KLHDC4 | NM_017566 | 1 | 0.004284709 | 2.00 (1.24, 3.21) | 7.10% | 1.02 | 1.25 |
| LEMD1 | NM_001001552 | 1 | 0.003532642 | 1.43 (1.12, 1.82) | 6.40% | 1.02 | 1.26 |
| LEMD1 | NM_001199051 | 1 | 0.003532642 | 1.43 (1.12, 1.82) | 6.40% | 1.02 | 1.26 |
| LEMD1 | NM_001199052 | 1 | 0.003532642 | 1.43 (1.12, 1.82) | 6.40% | 1.02 | 1.26 |
| LEMD1 | NR_037583 | 1 | 0.003532642 | 1.43 (1.12, 1.82) | 6.40% | 1.02 | 1.26 |
| LEMD1 | NM_001199050 | 1 | 0.003532642 | 1.43 (1.12, 1.82) | 6.40% | 1.02 | 1.26 |
| LETMD1 | NM_015416 | -1 | 0.003381908 | 0.55 (0.37, 0.82) | 6.20% | 1.02 | 1.26 |
| LETMD1 | NM_001024668 | -1 | 0.003381908 | 0.55 (0.37, 0.82) | 6.20% | 1.02 | 1.26 |
| LOC100128338 | NR_033849 | 1 | 0.003117505 | 1.65 (1.18, 2.29) | 6.00% | 1.02 | 1.28 |
| LOC100190938 | NR_024462 | -1 | 0.003341277 | 0.64 (0.47, 0.86) | 6.20% | 1.02 | 1.27 |
| LOC100190938 | NR_024461 | -1 | 0.003341277 | 0.64 (0.47, 0.86) | 6.20% | 1.02 | 1.27 |
| LOC254559 | NR_015411 | 1 | 0.003591948 | 1.78 (1.21, 2.63) | 6.40% | 1.02 | 1.27 |
| LOC285577 | NR_033898 | 1 | 0.002606176 | 1.24 (1.08, 1.43) | 5.50% | 1.02 | 1.2 |
| LOC401177 | NR_033975 | 1 | 0.001598219 | 1.23 (1.08, 1.40) | 4.10% | 1.02 | 1.2 |
| LOC646960 | NM_001195129 | 1 | 0.002275041 | 1.35 (1.11, 1.64) | 5.10% | 1.02 | 1.25 |
| LOC728606 | NR_024259 | -1 | 0.003080953 | 0.56 (0.38, 0.82) | 5.90% | 1.02 | 1.27 |
| LOC90834 | NR_026993 | 1 | 0.002787462 | 1.49 (1.15, 1.93) | 5.60% | 1.02 | 1.27 |
| LRP12 | NM_001135703 | 1 | 0.003559777 | 1.69 (1.19, 2.41) | 6.40% | 1.02 | 1.27 |
| LRP12 | NM_013437 | 1 | 0.003559777 | 1.69 (1.19, 2.41) | 6.40% | 1.02 | 1.27 |
| LRP8 | NM_004631 | 1 | 0.003269601 | 1.87 (1.23, 2.84) | 6.10% | 1.02 | 1.27 |
| LRP8 | NM_017522 | 1 | 0.003269601 | 1.87 (1.23, 2.84) | 6.10% | 1.02 | 1.27 |
| LRP8 | NM_033300 | 1 | 0.003269601 | 1.87 (1.23, 2.84) | 6.10% | 1.02 | 1.27 |
| LRP8 | NM_001018054 | 1 | 0.003269601 | 1.87 (1.23, 2.84) | 6.10% | 1.02 | 1.27 |
| LRRC4C | NM_020929 | -1 | 0.003685417 | 0.56 (0.38, 0.83) | 6.50% | 1.02 | 1.26 |
| LSM4 | NM_012321 | 1 | 0.002719555 | 1.53 (1.16, 2.01) | 5.50% | 1.02 | 1.28 |
| MACF1 | NM_033044 | -1 | 0.003907446 | 0.59 (0.42, 0.85) | 6.70% | 1.02 | 1.27 |
| MACF1 | NM_012090 | -1 | 0.003907446 | 0.59 (0.42, 0.85) | 6.70% | 1.02 | 1.27 |
| MBIP | NM_016586 | 1 | 0.00342382 | 1.87 (1.23, 2.86) | 6.30% | 1.02 | 1.26 |
| MBIP | NM_001144891 | 1 | 0.00342382 | 1.87 (1.23, 2.86) | 6.30% | 1.02 | 1.26 |
| MGC87042 | NM_207342 | 1 | 0.002700608 | 1.46 (1.14, 1.87) | 5.50% | 1.02 | 1.27 |
| MGC87042 | NM_001164460 | 1 | 0.002700608 | 1.46 (1.14, 1.87) | 5.50% | 1.02 | 1.27 |
| MIR542 | NR_030399 | 1 | 0.002819598 | 1.53 (1.16, 2.01) | 5.60% | 1.02 | 1.28 |
| MTHFD2 | NM_006636 | 1 | 0.00387651 | 1.72 (1.19, 2.47) | 6.70% | 1.02 | 1.27 |
| MTHFD2 | NR_027405 | 1 | 0.00387651 | 1.72 (1.19, 2.47) | 6.70% | 1.02 | 1.27 |
| NCRNA00168 | NR_033387 | 1 | 0.002775618 | 1.39 (1.12, 1.72) | 5.60% | 1.02 | 1.26 |
| NCRNA00242 | NR_026781 | -1 | 0.003823018 | 0.54 (0.36, 0.82) | 6.70% | 1.02 | 1.26 |
| NCRNA00287 | NR_026677 | -1 | 0.002783862 | 0.60 (0.43, 0.84) | 5.60% | 1.02 | 1.28 |
| NHSL1 | NM_020464 | 1 | 0.003334053 | 1.72 (1.20, 2.48) | 6.20% | 1.02 | 1.27 |
| NHSL1 | NM_001144060 | 1 | 0.003334053 | 1.72 (1.20, 2.48) | 6.20% | 1.02 | 1.27 |
| NTRK3 | NM_001012338 | -1 | 0.003070056 | 0.57 (0.40, 0.83) | 5.90% | 1.02 | 1.27 |
| NTRK3 | NM_002530 | -1 | 0.003070056 | 0.57 (0.40, 0.83) | 5.90% | 1.02 | 1.27 |
| NTRK3 | NM_001007156 | -1 | 0.003070056 | 0.57 (0.40, 0.83) | 5.90% | 1.02 | 1.27 |
| NUDT15 | NM_018283 | 1 | 0.003029893 | 1.74 (1.21, 2.52) | 5.90% | 1.02 | 1.28 |
| NUDT16P1 | NR_027766 | -1 | 0.00283035 | 0.66 (0.51, 0.87) | 5.60% | 1.02 | 1.27 |
| NUDT16P1 | NR_002949 | -1 | 0.00283035 | 0.66 (0.51, 0.87) | 5.60% | 1.02 | 1.27 |
| NUDT21 | NM_007006 | 1 | 0.002332854 | 1.57 (1.17, 2.10) | 5.10% | 1.02 | 1.29 |
| NUP155 | NM_004298 | 1 | 0.002877998 | 1.74 (1.21, 2.51) | 5.70% | 1.02 | 1.28 |
| NUP155 | NM_153485 | 1 | 0.002877998 | 1.74 (1.21, 2.51) | 5.70% | 1.02 | 1.28 |
| NXPH4 | NM_007224 | 1 | 0.003577666 | 1.74 (1.20, 2.53) | 6.40% | 1.02 | 1.27 |
| OR2A4 | NM_030908 | 1 | 0.002718898 | 1.46 (1.14, 1.86) | 5.50% | 1.02 | 1.27 |
| OR2W5 | NM_001004698 | 1 | 0.001067476 | 1.21 (1.08, 1.36) | 3.30% | 1.02 | 1.19 |
| P2RY13 | NM_176894 | -1 | 0.002437174 | 0.65 (0.50, 0.86) | 5.20% | 1.02 | 1.28 |
| PALM | NM_002579 | -1 | 0.003697526 | 0.67 (0.51, 0.88) | 6.50% | 1.02 | 1.26 |
| PALM | NM_001040134 | -1 | 0.003697526 | 0.67 (0.51, 0.88) | 6.50% | 1.02 | 1.26 |
| PCDH11X | NM_032969 | 1 | 0.002679604 | 1.52 (1.16, 2.01) | 5.50% | 1.02 | 1.28 |
| PCDH11X | NM_001168360 | 1 | 0.002679604 | 1.52 (1.16, 2.01) | 5.50% | 1.02 | 1.28 |
| PCDH11X | NM_032969 | 1 | 0.002679604 | 1.52 (1.16, 2.01) | 5.50% | 1.02 | 1.28 |
| PCDH11X | NM_032967 | 1 | 0.002679604 | 1.52 (1.16, 2.01) | 5.50% | 1.02 | 1.28 |
| PCDH11X | NM_001168363 | 1 | 0.002679604 | 1.52 (1.16, 2.01) | 5.50% | 1.02 | 1.28 |
| PCDH11X | NM_001168362 | 1 | 0.002679604 | 1.52 (1.16, 2.01) | 5.50% | 1.02 | 1.28 |
| PCDH11X | NM_001168361 | 1 | 0.002679604 | 1.52 (1.16, 2.01) | 5.50% | 1.02 | 1.28 |
| PCDH11X | NM_014522 | 1 | 0.002679604 | 1.52 (1.16, 2.01) | 5.50% | 1.02 | 1.28 |
| PCSK4 | NM_017573 | -1 | 0.002585422 | 0.60 (0.43, 0.84) | 5.40% | 1.02 | 1.28 |
| PCSK5 | NM_006200 | -1 | 0.002479594 | 0.71 (0.56, 0.88) | 5.30% | 1.02 | 1.26 |
| PCSK5 | NM_001190482 | -1 | 0.002479594 | 0.71 (0.56, 0.88) | 5.30% | 1.02 | 1.26 |
| PCTP | NM_001102402 | 1 | 0.00297204 | 1.62 (1.18, 2.22) | 5.80% | 1.02 | 1.28 |
| PCTP | NM_021213 | 1 | 0.00297204 | 1.62 (1.18, 2.22) | 5.80% | 1.02 | 1.28 |
| PLTP | NM_006227 | 1 | 0.00399448 | 1.58 (1.16, 2.16) | 6.80% | 1.02 | 1.27 |
| PLTP | NM_182676 | 1 | 0.00399448 | 1.58 (1.16, 2.16) | 6.80% | 1.02 | 1.27 |
| PNLIP | NM_000936 | 1 | 0.00197645 | 1.31 (1.11, 1.56) | 4.70% | 1.02 | 1.24 |
| PODXL2 | NM_015720 | 1 | 0.003706982 | 1.91 (1.23, 2.96) | 6.60% | 1.02 | 1.26 |
| PPP1R14B | NM_138689 | 1 | 0.002581467 | 1.64 (1.19, 2.26) | 5.40% | 1.02 | 1.28 |
| PPP1R3C | NM_005398 | -1 | 0.003328551 | 0.61 (0.44, 0.85) | 6.20% | 1.02 | 1.27 |
| PRRC2B | NM_013318 | -1 | 0.004341057 | 0.51 (0.32, 0.81) | 7.10% | 1.02 | 1.24 |
| PSMD2 | NM_002808 | 1 | 0.003173532 | 1.76 (1.21, 2.56) | 6.00% | 1.02 | 1.27 |
| PUF60 | NM_001136033 | 1 | 0.003952504 | 1.67 (1.18, 2.36) | 6.80% | 1.02 | 1.27 |
| PUF60 | NM_014281 | 1 | 0.003952504 | 1.67 (1.18, 2.36) | 6.80% | 1.02 | 1.27 |
| PUF60 | NM_078480 | 1 | 0.003952504 | 1.67 (1.18, 2.36) | 6.80% | 1.02 | 1.27 |
| RABEP1 | NM_001083585 | -1 | 0.003501962 | 0.54 (0.35, 0.82) | 6.40% | 1.02 | 1.26 |
| RABEP1 | NM_004703 | -1 | 0.003501962 | 0.54 (0.35, 0.82) | 6.40% | 1.02 | 1.26 |
| RABGAP1 | NM_012197 | -1 | 0.003415188 | 0.54 (0.36, 0.82) | 6.30% | 1.02 | 1.26 |
| RIPK4 | NM_020639 | 1 | 0.003549519 | 2.23 (1.30, 3.83) | 6.40% | 1.02 | 1.24 |
| RNF214 | NM_207343 | -1 | 0.002544607 | 0.63 (0.47, 0.85) | 5.40% | 1.02 | 1.28 |
| RNF214 | NM_001077239 | -1 | 0.002544607 | 0.63 (0.47, 0.85) | 5.40% | 1.02 | 1.28 |
| RPPH1 | NR_002312 | 1 | 0.00400948 | 1.72 (1.19, 2.48) | 6.80% | 1.02 | 1.27 |
| SCNN1D | NM_002978 | -1 | 0.002973296 | 0.60 (0.42, 0.84) | 5.80% | 1.02 | 1.28 |
| SCNN1D | NR_037668 | -1 | 0.002973296 | 0.60 (0.42, 0.84) | 5.80% | 1.02 | 1.28 |
| SCNN1D | NM_001130413 | -1 | 0.002973296 | 0.60 (0.42, 0.84) | 5.80% | 1.02 | 1.28 |
| SCRT2 | NM_033129 | 1 | 0.001721059 | 1.23 (1.08, 1.41) | 4.20% | 1.02 | 1.2 |
| SDCCAG3 | NM_001039707 | 1 | 0.003470913 | 1.88 (1.23, 2.88) | 6.30% | 1.02 | 1.26 |
| SDCCAG3 | NM_001039708 | 1 | 0.003470913 | 1.88 (1.23, 2.88) | 6.30% | 1.02 | 1.26 |
| SDCCAG3 | NM_006643 | 1 | 0.003470913 | 1.88 (1.23, 2.88) | 6.30% | 1.02 | 1.26 |
| SERINC5 | NM_178276 | 1 | 0.003590438 | 1.51 (1.14, 2.00) | 6.40% | 1.02 | 1.27 |
| SERINC5 | NM_001174071 | 1 | 0.003590438 | 1.51 (1.14, 2.00) | 6.40% | 1.02 | 1.27 |
| SERINC5 | NM_001174072 | 1 | 0.003590438 | 1.51 (1.14, 2.00) | 6.40% | 1.02 | 1.27 |
| SEZ6L | NM_001184776 | -1 | 0.004348114 | 0.49 (0.30, 0.80) | 7.10% | 1.02 | 1.24 |
| SEZ6L | NM_021115 | -1 | 0.004348114 | 0.49 (0.30, 0.80) | 7.10% | 1.02 | 1.24 |
| SEZ6L | NM_001184777 | -1 | 0.004348114 | 0.49 (0.30, 0.80) | 7.10% | 1.02 | 1.24 |
| SEZ6L | NM_001184775 | -1 | 0.004348114 | 0.49 (0.30, 0.80) | 7.10% | 1.02 | 1.24 |
| SEZ6L | NM_001184774 | -1 | 0.004348114 | 0.49 (0.30, 0.80) | 7.10% | 1.02 | 1.24 |
| SEZ6L | NM_001184773 | -1 | 0.004348114 | 0.49 (0.30, 0.80) | 7.10% | 1.02 | 1.24 |
| SGSM2 | NM_014853 | -1 | 0.002777129 | 0.55 (0.38, 0.82) | 5.60% | 1.02 | 1.27 |
| SGSM2 | NM_001098509 | -1 | 0.002777129 | 0.55 (0.38, 0.82) | 5.60% | 1.02 | 1.27 |
| SLC15A1 | NM_005073 | 1 | 0.003057481 | 1.64 (1.18, 2.28) | 5.90% | 1.02 | 1.28 |
| SLC26A6 | NM_022911 | 1 | 0.002410675 | 1.55 (1.17, 2.05) | 5.20% | 1.02 | 1.28 |
| SLC26A6 | NM_134426 | 1 | 0.002410675 | 1.55 (1.17, 2.05) | 5.20% | 1.02 | 1.28 |
| SLC26A6 | NM_134263 | 1 | 0.002410675 | 1.55 (1.17, 2.05) | 5.20% | 1.02 | 1.28 |
| SLC26A6 | NM_001040454 | 1 | 0.002410675 | 1.55 (1.17, 2.05) | 5.20% | 1.02 | 1.28 |
| SLC7A5 | NM_003486 | 1 | 0.003613709 | 1.88 (1.23, 2.89) | 6.50% | 1.02 | 1.26 |
| SLC7A8 | NM_182728 | -1 | 0.00381186 | 0.63 (0.46, 0.86) | 6.70% | 1.02 | 1.27 |
| SLC7A8 | NM_012244 | -1 | 0.00381186 | 0.63 (0.46, 0.86) | 6.70% | 1.02 | 1.27 |
| SMG1 | NM_015092 | -1 | 0.003487141 | 0.57 (0.39, 0.83) | 6.30% | 1.02 | 1.27 |
| SNRPD3 | NM_004175 | 1 | 0.003488489 | 1.54 (1.15, 2.07) | 6.30% | 1.02 | 1.27 |
| SPPL2B | NM_152988 | -1 | 0.003064537 | 0.64 (0.48, 0.86) | 5.90% | 1.02 | 1.27 |
| SPPL2B | NM_001077238 | -1 | 0.003064537 | 0.64 (0.48, 0.86) | 5.90% | 1.02 | 1.27 |
| ST8SIA6 | NM_001004470 | -1 | 0.002791716 | 0.61 (0.44, 0.84) | 5.60% | 1.02 | 1.28 |
| STAU2 | NM_001164380 | 1 | 0.002783168 | 1.62 (1.18, 2.22) | 5.60% | 1.02 | 1.28 |
| STAU2 | NM_014393 | 1 | 0.002783168 | 1.62 (1.18, 2.22) | 5.60% | 1.02 | 1.28 |
| STAU2 | NM_001164384 | 1 | 0.002783168 | 1.62 (1.18, 2.22) | 5.60% | 1.02 | 1.28 |
| STAU2 | NM_001164382 | 1 | 0.002783168 | 1.62 (1.18, 2.22) | 5.60% | 1.02 | 1.28 |
| STAU2 | NM_001164381 | 1 | 0.002783168 | 1.62 (1.18, 2.22) | 5.60% | 1.02 | 1.28 |
| STAU2 | NM_001164385 | 1 | 0.002783168 | 1.62 (1.18, 2.22) | 5.60% | 1.02 | 1.28 |
| STAU2 | NM_001164383 | 1 | 0.002783168 | 1.62 (1.18, 2.22) | 5.60% | 1.02 | 1.28 |
| STC2 | NM_003714 | -1 | 0.002916534 | 0.54 (0.35, 0.81) | 5.70% | 1.02 | 1.27 |
| SYT5 | NM_003180 | 1 | 0.002277553 | 1.32 (1.10, 1.58) | 5.10% | 1.02 | 1.24 |
| SYT9 | NM_175733 | -1 | 0.003474054 | 0.65 (0.48, 0.87) | 6.30% | 1.02 | 1.27 |
| TAAR6 | NM_175067 | 1 | 0.002711741 | 1.45 (1.14, 1.84) | 5.50% | 1.02 | 1.27 |
| TAF13 | NM_005645 | 1 | 0.003926491 | 1.57 (1.16, 2.13) | 6.80% | 1.02 | 1.27 |
| TFAP2D | NM_172238 | 1 | 0.001850588 | 1.38 (1.13, 1.70) | 4.40% | 1.02 | 1.26 |
| THOC3 | NM_032361 | 1 | 0.004239158 | 2.03 (1.25, 3.29) | 7.00% | 1.02 | 1.25 |
| TMED9 | NM_017510 | 1 | 0.002890032 | 1.91 (1.25, 2.92) | 5.70% | 1.02 | 1.27 |
| TMEM30C | NR_028358 | 1 | 0.002560059 | 1.51 (1.15, 1.96) | 5.40% | 1.02 | 1.28 |
| TMEM30C | NR_028357 | 1 | 0.002560059 | 1.51 (1.15, 1.96) | 5.40% | 1.02 | 1.28 |
| TMOD4 | NM_013353 | -1 | 0.003403959 | 0.58 (0.40, 0.84) | 6.30% | 1.02 | 1.27 |
| TMPRSS13 | NM_001077263 | 1 | 0.003417183 | 1.79 (1.21, 2.66) | 6.30% | 1.02 | 1.27 |
| TMSB10 | NM_021103 | 1 | 0.004188438 | 1.78 (1.20, 2.63) | 7.00% | 1.02 | 1.26 |
| TNRC6A | NM_014494 | -1 | 0.003074972 | 0.51 (0.32, 0.79) | 5.90% | 1.02 | 1.26 |
| TRAF3 | NM_145726 | -1 | 0.00293145 | 0.53 (0.35, 0.81) | 5.70% | 1.02 | 1.27 |
| TRAF3 | NM_001199427 | -1 | 0.00293145 | 0.53 (0.35, 0.81) | 5.70% | 1.02 | 1.27 |
| TRAF3 | NM_ 003300 | -1 | 0.00293145 | 0.53 (0.35, 0.81) | 5.70% | 1.02 | 1.27 |
| TRAF3 | NM_145725 | -1 | 0.00293145 | 0.53 (0.35, 0.81) | 5.70% | 1.02 | 1.27 |
| TRAM1 | NM_014294 | 1 | 0.002697324 | 1.64 (1.19, 2.26) | 5.50% | 1.02 | 1.28 |
| TRAM1L1 | NM_152402 | -1 | 0.003024509 | 0.64 (0.48, 0.86) | 5.80% | 1.02 | 1.27 |
| TRPC7 | NM_001167577 | 1 | 0.003198969 | 1.42 (1.12, 1.79) | 6.10% | 1.02 | 1.26 |
| TRPC7 | NM_020389 | 1 | 0.003198969 | 1.42 (1.12, 1.79) | 6.10% | 1.02 | 1.26 |
| TRPC7 | NM_001167576 | 1 | 0.003198969 | 1.42 (1.12, 1.79) | 6.10% | 1.02 | 1.26 |
| TSC1 | NM_001162427 | -1 | 0.004320082 | 0.53 (0.34, 0.82) | 7.10% | 1.02 | 1.25 |
| TSC1 | NM_00368 | -1 | 0.004320082 | 0.53 (0.34, 0.82) | 7.10% | 1.02 | 1.25 |
| TSC1 | NM_001162426 | -1 | 0.004320082 | 0.53 (0.34, 0.82) | 7.10% | 1.02 | 1.25 |
| TSNAX-DISC1 | NR_028399 | 1 | 0.003821941 | 1.51 (1.14, 1.99) | 6.70% | 1.02 | 1.27 |
| TSNAX-DISC1 | NR_028400 | 1 | 0.003821941 | 1.51 (1.14, 1.99) | 6.70% | 1.02 | 1.27 |
| TSNAX-DISC1 | NR_028398 | 1 | 0.003821941 | 1.51 (1.14, 1.99) | 6.70% | 1.02 | 1.27 |
| TSNAX-DISC1 | NR_028397 | 1 | 0.003821941 | 1.51 (1.14, 1.99) | 6.70% | 1.02 | 1.27 |
| TSNAX-DISC1 | NR_028393 | 1 | 0.003821941 | 1.51 (1.14, 1.99) | 6.70% | 1.02 | 1.27 |
| TSNAX-DISC1 | NR_028396 | 1 | 0.003821941 | 1.51 (1.14, 1.99) | 6.70% | 1.02 | 1.27 |
| TSNAX-DISC1 | NR_028395 | 1 | 0.003821941 | 1.51 (1.14, 1.99) | 6.70% | 1.02 | 1.27 |
| TSNAX-DISC1 | NR_028394 | 1 | 0.003821941 | 1.51 (1.14, 1.99) | 6.70% | 1.02 | 1.27 |
| TTC30A | NM_152275 | -1 | 0.002251039 | 0.66 (0.51, 0.86) | 5.00% | 1.02 | 1.28 |
| TTF1 | NM_007344 | -1 | 0.002675227 | 0.61 (0.44, 0.84) | 5.50% | 1.02 | 1.28 |
| TUBA1C | NM_032704 | 1 | 0.003245041 | 2.12 (1.29, 3.50) | 6.10% | 1.02 | 1.25 |
| TUSC1 | NM_001004125 | -1 | 0.003519464 | 0.66 (0.50, 0.87) | 640% | 1.02 | 1.27 |
| UBXN10 | NM_152376 | -1 | 0.002709535 | 0.63 (0.46, 0.85) | 5.50% | 1.02 | 1.28 |
| UQCRB | NM_006294 | 1 | 0.003314393 | 1.76 (1.21, 2.56) | 6.20% | 1.02 | 1.27 |
| UQCRB | NM_001199975 | 1 | 0.003314393 | 1.76 (1.21, 2.56) | 6.20% | 1.02 | 1.27 |
| VILL | NM_015873 | -1 | 0.003667617 | 0.65 (0.49, 0.87) | 6.50% | 1.02 | 1.27 |
| VWC2 | NM_198570 | 1 | 0.002039885 | 1.31 (1.10, 1.56) | 4.70% | 1.02 | 1.24 |
| WDR47 | NM_901142551 | -1 | 0.002875073 | 0.74 (0.61, 0.90) | 5.70% | 1.02 | 1.24 |
| WDR47 | NM_001142550 | -1 | 0.002875073 | 0.74 (0.61, 0.90) | 5.70% | 1.02 | 1.24 |
| WDR47 | NM_014969 | -1 | 0.002875073 | 0.74 (0.61, 0.90) | 5.70% | 1.02 | 1.24 |
| WDR75 | NM_032168 | -1 | 0.003637692 | 0.53 (0.35, 0.82) | 6.50% | 1.02 | 1.26 |
| XPOT | NM_007235 | 1 | 0.003529958 | 1.66 (1.18, 2.33) | 6.40% | 1.02 | 1.27 |
| ZER1 | NM_006336 | -1 | 0.003281615 | 0.61 (0.44, 0.85) | 6.10% | 1.02 | 1.27 |
| ZFP42 | NM_174900 | 1 | 0.002027244 | 1.34 (1.11, 1.61) | 4.70% | 1.02 | 1.25 |
| ZFYVE26 | NM_015346 | -1 | 0.003488824 | 0.47 (0.28, 0.78) | 6.30% | 1.02 | 1.24 |
| ZNF487P | NR_026693 | -1 | 0.002718434 | 0.62 (0.45, 0.85) | 5.50% | 1.02 | 1.28 |
| ZNF610 | NM_001161427 | -1 | 0.00379397 | 0.66 (0.50, 0.88) | 6.70% | 1.02 | 1.26 |
| ZNF610 | NM_001161425 | -1 | 0.00379397 | 0.66 (0.50, 0.88) | 6.70% | 1.02 | 1.26 |
| ZNF610 | NM_001161426 | -1 | 0.00379397 | 0.66 (0.50, 0.88) | 6.70% | 1.02 | 1.26 |
| ZNF610 | NM_173530 | -1 | 0.00379397 | 0.66 (0.50, 0.88) | 6.70% | 1.02 | 1.26 |
| ZNF653 | NM_138783 | -1 | 0.00333192 | 0.58 (0.40, 0.83) | 6.20% | 1.02 | 1.27 |
| ZNF655 | NM_024061 | -1 | 0.002999752 | 0.58 (0.40, 0.83) | 5.80% | 1.02 | 1.27 |
| ZNF655 | NM_001083956 | -1 | 0.002999752 | 0.58 (0.40, 0.83) | 5.80% | 1.02 | 1.27 |
| ZNF655 | NM_001009958 | -1 | 0.002999752 | 0.58 (0.40, 0.83) | 5.80% | 1.02 | 1.27 |
| ZNF655 | NM_001085368 | -1 | 0.002999752 | 0.58 (0.40, 0.83) | 5.80% | 1.02 | 1.27 |
| ZNF655 | NM_001085366 | -1 | 0.002999752 | 0.58 (0.40, 0.83) | 5.80% | 1.02 | 1.27 |
| ZNF655 | NM_138494 | -1 | 0.002999752 | 0.58 (0.40, 0.83) | 5.80% | 1.02 | 1.27 |
| ZNF655 | NM_001009960 | -1 | 0.002999752 | 0.58 (0.40, 0.83) | 5.80% | 1.02 | 1.27 |
| ZNF655 | NM_001085367 | -1 | 0.002999752 | 0.58 (0.40, 0.83) | 5.80% | 1.02 | 1.27 |
| ZNF701 | NM_018260 | -1 | 0.003268807 | 0.59 (0.41, 0.84) | 6.10% | 1.02 | 1.27 |
| ZNF701 | NM_001172655 | -1 | 0.003268807 | 0.59 (0.41, 0.84) | 6.10% | 1.02 | 1.27 |
| ZNF740 | NM_001004304 | -1 | 0.004118629 | 0.58 (0.40, 0.84) | 6.90% | 1.02 | 1.26 |
| ZNF788 | NR 027049 | -1 | 0.00249849 | 0.60 (0.43, 0.84) | 5.30% | 1.02 | 1.28 |
| ABCD1 | NM_000033 | 1 | 0.005827069 | 1.69 (1.16, 2.46) | 8.30% | 1.01 | 1.25 |
| ABCG8 | NM_022437 | 1 | 0.00511131 | 1.41 (1.11, 1.80) | 7.70% | 1.01 | 1.25 |
| ACAT1 | NM_000019 | -1 | 0.00445572 | 0.66 (0.50, 0.88) | 7.20% | 1.01 | 1.26 |
| ACO2 | NM_001098 | 1 | 0.004314322 | 1.70 (1.18, 2.44) | 7.10% | 1.01 | 1.27 |
| ACTL8 | NM_030812 | 1 | 0.005453351 | 1.59 (1.15, 2.20) | 7.90% | 1.01 | 1.26 |
| ADRA2A | NM_000681 | -1 | 0.004645911 | 0.59 (0.41, 0.85) | 7.30% | 1.01 | 1.26 |
| AFF3 | NM_001025108 | -1 | 0.005402057 | 0.63 (0.45, 0.87) | 7.90% | 1.01 | 1.26 |
| AFF3 | NM_002285 | -1 | 0.005402057 | 0.63 (0.45, 0.87) | 7.90% | 1.01 | 1.26 |
| AGAP4 | NM_133446 | -1 | 0.006470733 | 0.61 (0.43, 0.87) | 8.70% | 1.01 | 1.25 |
| AGAP5 | NM_001144000 | -1 | 0.005031188 | 0.64 (0.47, 0.87) | 7.60% | 1.01 | 1.26 |
| AGTR1 | NM_004835 | -1 | 0.004024812 | 0.63 (0.46, 0.86) | 6.90% | 1.01 | 1.27 |
| AGTR1 | NM_009585 | -1 | 0.004024812 | 0.63 (0.46, 0.86) | 6.90% | 1.01 | 1.27 |
| AGTR1 | NM_000685 | -1 | 0.004024812 | 0.63 (0.46, 0.86) | 6.90% | 1.01 | 1.27 |
| AGTR1 | NM_032049 | -1 | 0.004024812 | 0.63 (0.46, 0.86) | 6.90% | 1.01 | 1.27 |
| AGTR1 | NM_031850 | -1 | 0.004024812 | 0.63 (0.46, 0.86) | 6.90% | 1.01 | 1.27 |
| ALG10B | NM_001013620 | -1 | 0.004720162 | 0.64 (0.47, 0.87) | 7.40% | 1.01 | 1.26 |
| AMDHD1 | NM_152435 | -1 | 0.0061723 | 0.60 (0.41, 0.86) | 8.50% | 1.01 | 1.25 |
| ARMC9 | NM_025139 | -1 | 0.004214321 | 0.63 (0.46, 0.86) | 7.00% | 1.01 | 1.26 |
| ARPC5 | NM_005717 | 1 | 0.005346223 | 1.71 (1.17, 2.50) | 7.90% | 1.01 | 1.26 |
| ASPM | NM_018136 | 1 | 0.004397362 | 1.87 (1.22, 2.89) | 7.10% | 1.01 | 1.26 |
| ASPN | NM_017680 | -1 | 0.005869465 | 0.59 (0.40, 0.86) | 8.30% | 1.01 | 1.25 |
| ASPN | NM_001193335 | -1 | 0.005869465 | 0.59 (0.40, 0.86) | 8.30% | 1.01 | 1.25 |
| ATP2A2 | NM_001681 | 1 | 0.005992169 | 1.56 (1.14, 2.15) | 8.40% | 1.01 | 1.26 |
| ATP2A2 | NM_001135765 | 1 | 0.005992169 | 1.56 (1.14, 2.15) | 8.40% | 1.01 | 1.26 |
| ATP2A2 | NM 170665 | 1 | 0.005992169 | 1.56 (1.14, 2.15) | 8.40% | 1.01 | 1.26 |
| AUP1 | NM_181575 | 1 | 0.00584932 | 2.10 (1.24, 3.57) | 8.30% | 1.01 | 1.23 |
| BIRC5 | NM_001168 | 1 | 0.005913421 | 1.71 (1.17, 2.51) | 8.30% | 1.01 | 1.25 |
| BIRC5 | NM_001012270 | 1 | 0.005913421 | 1.71 (1.17, 2.51) | 8.30% | 1.01 | 1.25 |
| BIRC5 | NM_001012271 | 1 | 0.005913421 | 1.71 (1.17, 2.51) | 8.30% | 1.01 | 1.25 |
| BTRC | NM_003939 | -1 | 0.00443052 | 0.55 (0.37, 0.83) | 7.10% | 1.01 | 1.25 |
| BTRC | NM_033637 | -1 | 0.00443052 | 0.55 (0.37, 0.83) | 7.10% | 1.01 | 1.25 |
| BUB1 | NM_004336 | 1 | 0.004890814 | 2.22 (1.27, 3.86) | 7.50% | 1.01 | 1.23 |
| C12orf35 | NM 018169 | -1 | 0.00533497 | 0.51 (0.32, 0.82) | 7.90% | 1.01 | 1.24 |
| C12orf48 | NM_017915 | 1 | 0.004706823 | 2.07 (1.25, 3.43) | 7.40% | 1.01 | 1.24 |
| C14orf38 | NM_001164399 | -1 | 0.0065299 | 0.61 (0.43, 0.87) | 8.70% | 1.01 | 1.25 |
| C16orf53 | NM_024516 | -1 | 0.004893843 | 0.58 (0.40, 0.85) | 7.50% | 1.01 | 1.26 |
| C1orf65 | NM_152610 | 1 | 0.00558459 | 1.52 (1.13, 2.05) | 8.10% | 1.01 | 1.26 |
| C1orf88 | NM_181643 | -1 | 0.006237565 | 0.64 (0.46, 0.88) | 8.50% | 1.01 | 1.25 |
| C22orf15 | NM_182520 | 1 | 0.006327763 | 1.56 (1.13, 2.14) | 8.60% | 1.01 | 1.25 |
| C2orf77 | NM_001085447 | -1 | 0.005006318 | 0.63 (0.46, 0.87) | 7.60% | 1.01 | 1.26 |
| C4orf14 | NM_032313 | 1 | 0.005587578 | 1.73 (1.17, 2.56) | 8.10% | 1.01 | 1.25 |
| C5orf36 | NM_173665 | -1 | 0.004656591 | 0.61 (0.43, 0.86) | 7.30% | 1.01 | 1.26 |
| C5orf36 | NM_001145678 | -1 | 0.004656591 | 0.61 (0.43, 0.86) | 7.30% | 1.01 | 1.26 |
| C7orf70 | NN_001037163 | 1 | 0.006375541 | 1.77 (1.17, 2.67) | 8.60% | 1.01 | 1.25 |
| C9orf156 | NM_016481 | -1 | 0.005997679 | 0.68 (0.51, 0.89) | 8.40% | 1.01 | 1.25 |
| CACNA1D | NM_001128840 | -1 | 0.005382035 | 0.63 (0.46, 0.87) | 7.90% | 1.01 | 1.26 |
| CACNA1D | NM_000720 | -1 | 0.005382035 | 0.63 (0.46, 0.87) | 7.90% | 1.01 | 1.26 |
| CACNA1D | NM_001128839 | -1 | 0.005382035 | 0.63 (0.46, 0.87) | 7.90% | 1.01 | 1.26 |
| CALCOCO1 | NM_020898 | -1 | 0.00571028 | 0.63 (0.45, 0.87) | 8.20% | 1.01 | 1.25 |
| CALCOCO1 | NM_001143682 | -1 | 0.00571028 | 0.63 (0.45, 0.87) | 8.20% | 1.01 | 1.25 |
| CALCOCO1 | NR_026554 | -1 | 0.00571028 | 0.63 (0.45, 0.87) | 8.20% | 1.01 | 1.25 |
| CAMKV | NM_024046 | 1 | 0.004566931 | 1.44 (1.12, 1.85) | 7.20% | 1.01 | 1.26 |
| CASC5 | NM_170589 | 1 | 0.005135757 | 2.23 (1.27, 3.92) | 7.70% | 1.01 | 1.22 |
| CASC5 | NM_144508 | 1 | 0.005135757 | 2.23 (1.27, 3.92) | 7.70% | 1.01 | 1.22 |
| CCDC146 | NM_020879 | -1 | 0.006404542 | 0.62 (0.44, 0.87) | 8.60% | 1.01 | 1.25 |
| CCDC85A | NM_001080433 | -1 | 0.004932883 | 0.64 (0.47, 0.88) | 7.50% | 1.01 | 1.26 |
| CDK5R2 | NM_003936 | 1 | 0.004167684 | 1.31 (1.09, 1.58) | 7.00% | 1.01 | 1.23 |
| CENPF | NM_016343 | 1 | 0.006440669 | 1.86 (1.19, 2.91) | 8.70% | 1.01 | 1.24 |
| CHMP1A | NM_002768 | 1 | 0.005284675 | 1.93 (1.22, 3.07) | 7.80% | 1.01 | 1.24 |
| CHMP1A | NM_001083314 | 1 | 0.005284675 | 1.93 (1.22, 3.07) | 7.80% | 1.01 | 1.24 |
| CHRNA5 | NM_000745 | 1 | 0.004550155 | 1.82 (1.20, 2.75) | 7.20% | 1.01 | 1.26 |
| CIB1 | NM_006384 | 1 | 0.005380782 | 1.41 (1.11, 1.80) | 7.90% | 1.01 | 1.25 |
| CLEC18B | NM_001011880 | 1 | 0.005051242 | 1.39 (1.10, 1.74) | 7.60% | 1.01 | 1.24 |
| CLPTM1L | NM_030782 | 1 | 0.005097651 | 1.61 (1.15, 2.25) | 7.70% | 1.01 | 1.26 |
| CLRN1 | NM_052995 | -1 | 0.006214457 | 0.54 (0.34, 0.84) | 8.50% | 1.01 | 1.24 |
| CLRN1 | NM_174878 | -1 | 0.006214457 | 0.54 (0.34, 0.84) | 8.50% | 1.01 | 1.24 |
| CLRN1 | NM_001195794 | -1 | 0.006214457 | 0.54 (0.34, 0.84) | 8.50% | 1.01 | 1.24 |
| CLUAP1 | NM_015041 | -1 | 0.005214813 | 0.55 (0.36, 0.84) | 7.70% | 1.01 | 1.25 |
| CLUAP1 | NM_024793 | -1 | 0.005214813 | 0.55 (0.36, 0.84) | 7.70% | 1.01 | 1.25 |
| CNGB1 | NM_001135639 | 1 | 0.004913341 | 1.44 (1.12, 1.85) | 7.50% | 1.01 | 1.25 |
| CNGB1 | NM_001297 | 1 | 0.004913341 | 1.44 (1.12, 1.85) | 7.50% | 1.01 | 1.25 |
| COL6A4P2 | NR_027898 | -1 | 0.004926414 | 0.62 (0.45, 0.87) | 7.50% | 1.01 | 1.26 |
| COPE | NM_199442 | 1 | 0.004505534 | 1.56 (1.15, 2.12) | 7.20% | 1.01 | 1.27 |
| COPE | NM_007263 | 1 | 0.004505534 | 1.56 (1.15, 2.12) | 7.20% | 1.01 | 1.27 |
| COPE | NM_199444 | 1 | 0.004505534 | 1.56 (1.15, 2.12) | 7.20% | 1.01 | 1.27 |
| CPEB2 | NM_001177381 | -1 | 0.005399996 | 0.60 (0.42, 0.86) | 7.90% | 1.01 | 1.25 |
| CPEB2 | NM_182646 | -1 | 0.005399996 | 0.60 (0.42, 0.86) | 7.90% | 1.01 | 1.25 |
| CPEB2 | NM_001177384 | -1 | 0.005399996 | 0.60 (0.42, 0.86) | 7.90% | 1.01 | 1.25 |
| CPEB2 | NM_182485 | -1 | 0.005399996 | 0.60 (0.42, 0.86) | 7.90% | 1.01 | 1.25 |
| CPEB2 | NM_001177382 | -1 | 0.005399996 | 0.60 (0.42, 0.86) | 7.90% | 1.01 | 1.25 |
| CPEB2 | NM_001177383 | -1 | 0.005399996 | 0.60 (0.42, 0.86) | 7.90% | 1.01 | 1.25 |
| CPLX3 | NM_001030005 | 1 | 0.004089055 | 1.54 (1.15, 2.08) | 6.90% | 1.01 | 1.27 |
| CSPG5 | NM_006574 | 1 | 0.004918683 | 1.64 (1.16, 2.31) | 7.50% | 1.01 | 1.26 |
| CT62 | NM_001102658 | -1 | 0.005814335 | 0.61 (0.42, 0.87) | 8.30% | 1.01 | 1.25 |
| CTCF | NM_001191022 | 1 | 0.006404212 | 1.78 (1.18, 2.70) | 8.60% | 1.01 | 1.25 |
| CTCF | NM_006565 | 1 | 0.006404212 | 1.78 (1.18, 2.70) | 8.60% | 1.01 | 1.25 |
| CTSA | NM_000308 | 1 | 0.006003343 | 1.58 (1.14, 2.20) | 8.40% | 1.01 | 1.26 |
| CTSA | NM_001127695 | 1 | 0.006003343 | 1.58 (1.14, 2.20) | 8.40% | 1.01 | 1.26 |
| CTSA | NM_001167594 | 1 | 0.006003343 | 1.58 (1.14, 2.20) | 8.40% | 1.01 | 1.26 |
| CYBRD1 | NM_001127383 | -1 | 0.004229211 | 0.64 (0.47, 0.87) | 7.00% | 1.01 | 1.26 |
| CYBRD1 | NM_024843 | -1 | 0.004229211 | 0.64 (0.47, 0.87) | 7.00% | 1.01 | 1.26 |
| DBF4 | NM_006716 | 1 | 0.004613835 | 1.87 (1.21, 2.88) | 7.30% | 1.01 | 1.25 |
| DBI | NM_001178042 | 1 | 0.006179033 | 1.45 (1.11, 1.90) | 8.50% | 1.01 | 1.25 |
| DBI | NM_001079863 | 1 | 0.006179033 | 1.45 (1.11, 1.90) | 8.50% | 1.01 | 1.25 |
| DBI | NM_001178043 | 1 | 0.006179033 | 1.45 (1.11, 1.90) | 8.50% | 1.01 | 1.25 |
| DBI | NM_001178041 | 1 | 0.006179033 | 1.45 (1.11, 1.90) | 8.50% | 1.01 | 1.25 |
| DBI | NM_001178017 | 1 | 0.006179033 | 1.45 (1.11, 1.90) | 8.50% | 1.01 | 1.25 |
| DBI | NM_001079862 | 1 | 0.006179033 | 1.45 (1.11, 1.90) | 8.50% | 1.01 | 1.25 |
| DBI | NM_020548 | 1 | 0.006179033 | 1.45 (1.11, 1.90) | 8.50% | 1.01 | 1.25 |
| DCAF7 | NM_005828 | 1 | 0.005185203 | 1.38 (1.10, 1.73) | 7.70% | 1.01 | 1.24 |
| DDB2 | NM_000107 | -1 | 0.005118224 | 0.63 (0.46, 0.87) | 7.70% | 1.01 | 1.26 |
| DGCR2 | NM_005137 | 1 | 0.006382852 | 1.63 (1.15, 2.31) | 8.60% | 1.01 | 1.25 |
| DGCR2 | NM_001173534 | 1 | 0.006382852 | 1.63 (1.15, 2.31) | 8.60% | 1.01 | 1.25 |
| DGCR2 | NM_001173533 | 1 | 0.006382852 | 1.63 (1.15, 2.31) | 8.60% | 1.01 | 1.25 |
| DGCR2 | NM_01184781 | 1 | 0.006382852 | 1.63 (1.15, 2.31) | 8.60% | 1.01 | 1.25 |
| DGCR2 | NR_033674 | 1 | 0.006382852 | 1.63 (1.15, 2.31) | 8.60% | 1.01 | 1.25 |
| DGCR9 | NR_024159 | 1 | 0.005993007 | 1.59 (1.14, 2.22) | 8.40% | 1.01 | 1.26 |
| DHRS3 | NM_004753 | 1 | 0.006567842 | 1.68 (1.16, 2.45) | 8.80% | 1.01 | 1.25 |
| DHX32 | NM_018180 | -1 | 0.006433684 | 0.58 (0.40, 0.86) | 8.70% | 1.01 | 1.25 |
| DKK4 | NM_014420 | -1 | 0.006648293 | 0.56 (0.37, 0.85) | 8.80% | 1.01 | 1.24 |
| DLGAP5 | NM_001146015 | 1 | 0.006185847 | 1.81 (1.18, 2.78) | 8.50% | 1.01 | 1.25 |
| DLGAP5 | NM_014750 | 1 | 0.006185847 | 1.81 (1.18, 2.78) | 8.50% | 1.01 | 1.25 |
| DNA2 | NM_001080449 | 1 | 0.005009309 | 1.65 (1.16, 2.35) | 7.60% | 1.01 | 1.26 |
| DNAH7 | NM_018897 | -1 | 0.004588402 | 0.68 (0.52, 0.89) | 7.30% | 1.01 | 1.26 |
| DNAJB11 | NM_016306 | 1 | 0.005873219 | 1.60 (1.14, 2.23) | 8.30% | 1.01 | 1.26 |
| DNAJC3 | NM_006260 | 1 | 0.006415627 | 1.54 (1.13, 2.10) | 8.60% | 1.01 | 1.25 |
| DNAJC5G | NM_173650 | 1 | 0.00515377 | 1.31 (1.08, 1.59) | 7.70% | 1.01 | 1.22 |
| DPY30 | NM_032574 | 1 | 0.006797135 | 2.07 (1.22, 3.50) | 8.90% | 1.01 | 1.22 |
| EBP | NM_006579 | 1 | 0.006200067 | 1.72 (1.17, 2.53) | 8.50% | 1.01 | 1.25 |
| EFHC1 | NR_033327 | -1 | 0.004990192 | 0.58 (0.40, 0.85) | 7.60% | 1.01 | 1.25 |
| EFHC1 | NM_018100 | -1 | 0.004990192 | 0.58 (0.40, 0.85) | 7.60% | 1.01 | 1.25 |
| EFHC1 | NM_001172420 | -1 | 0.004990192 | 0.58 (0.40, 0.85) | 7.60% | 1.01 | 1.25 |
| EFNA3 | NM_004952 | 1 | 0.006168226 | 1.55 (1.13, 2.12) | 8.50% | 1.01 | 1.26 |
| EFNA5 | NM_001962 | 1 | 0.005036941 | 1.70 (1.17, 2.46) | 7.60% | 1.01 | 1.26 |
| EID2B | NM_152361 | -1 | 0.00574852 | 0.66 (0.49, 0.89) | 8.20% | 1.01 | 1.25 |
| ELOVL6 | NM_024090 | 1 | 0.004827708 | 1.77 (1.19, 2.63) | 7.40% | 1.01 | 1.26 |
| ELOVL6 | NM_001130721 | 1 | 0.004827708 | 1.77 (1.19, 2.63) | 7.40% | 1.01 | 1.26 |
| EMP3 | NM_001425 | -1 | 0.004017208 | 0.64 (0.47, 0.87) | 6.90% | 1.01 | 1.27 |
| EPCAM | NM_002354 | 1 | 0.006251902 | 1.69 (1.16, 2.46) | 8.50% | 1.01 | 1.25 |
| EPR1 | NR_002219 | 1 | 0.00602072 | 1.80 (1.18, 2.73) | 8.40% | 1.01 | 1.25 |
| ERBB4 | NM_005235 | -1 | 0.004724805 | 0.62 (0.45, 0.86) | 7.40% | 1.01 | 1.26 |
| ERBB4 | NM_001042599 | -1 | 0.004724805 | 0.62 (0.45, 0.86) | 7.40% | 1.01 | 1.26 |
| ERN2 | NM_033266 | 1 | 0.00383262 | 1.46 (1.13, 1.88) | 6.70% | 1.01 | 1.26 |
| F11 | NM_000128 | 1 | 0.004222479 | 1.48 (1.13, 1.93) | 7.00% | 1.01 | 1.26 |
| FADS2 | NM_004265 | 1 | 0.00574684 | 1.85 (1.20, 2.86) | 8.20% | 1.01 | 1.25 |
| FAM102A | NM_203305 | -1 | 0.005644483 | 0.62 (0.44, 0.87) | 8.10% | 1.01 | 1.25 |
| FAM102A | NM_001035254 | -1 | 0.005644483 | 0.62 (0.44, 0.87) | 8.10% | 1.01 | 1.25 |
| FAM155B | NM_015686 | 1 | 0.005996344 | 1.62 (1.15, 2.29) | 8.40% | 1.01 | 1.26 |
| FAM19A1 | NM_213609 | 1 | 0.006014024 | 1.54 (1.13, 2.09) | 8.40% | 1.01 | 1.26 |
| FAM200A | NM_145111 | -1 | 0.004137595 | 0.67 (0.51, 0.88) | 6.90% | 1.01 | 1.26 |
| FBXL2 | NM_001171713 | -1 | 0.005598664 | 0.67 (0.51, 0.89) | 8.10% | 1.01 | 1.25 |
| FBXL2 | NM_012157 | -1 | 0.005598664 | 0.67 (0.51, 0.89) | 8.10% | 1.01 | 1.25 |
| FBXO45 | NM_001105573 | 1 | 0.00563526 | 1.69 (1.17, 2.46) | 8.10% | 1.01 | 1.26 |
| FDXACB1 | NM_138378 | -1 | 0.004699684 | 0.71 (0.56, 0.90) | 7.40% | 1.01 | 1.25 |
| FGF2 | NM_002006 | -1 | 0.004723067 | 0.57 (0.39, 0.84) | 7.40% | 1.01 | 1.26 |
| FU30403 | NR_034159 | -1 | 0.004967441 | 0.63 (0.46, 0.87) | 7.50% | 1.01 | 1.26 |
| FU41200 | NR_033863 | 1 | 0.004925314 | 1.75 (1.18, 2.58) | 7.50% | 1.01 | 1.26 |
| FU44635 | NM_207422 | -1 | 0.006189338 | 0.63 (0.45, 0.88) | 8.50% | 1.01 | 1.25 |
| FOXM1 | NM_202002 | 1 | 0.004332916 | 1.63 (1.17, 2.28) | 7.10% | 1.01 | 1.27 |
| FOXM1 | NM_202003 | 1 | 0.004332916 | 1.63 (1.17, 2.28) | 7.10% | 1.01 | 1.27 |
| FOXM1 | NM_021953 | 1 | 0.004332916 | 1.63 (1.17, 2.28) | 7.10% | 1.01 | 1.27 |
| FSTL3 | NM_005860 | -1 | 0.004470174 | 0.61 (0.44, 0.86) | 7.20% | 1.01 | 1.26 |
| GALNT14 | NM_024572 | 1 | 0.006578564 | 1.80 (1.18, 2.74) | 8.80% | 1.01 | 1.24 |
| GALNTL1 | NM_001168368 | -1 | 0.004149532 | 0.63 (0.46, 0.86) | 6.90% | 1.01 | 1.26 |
| GALNTL1 | NM_ 020692 | -1 | 0.004149532 | 0.63 (0.46, 0.86) | 6.90% | 1.01 | 1.26 |
| GFRA1 | NM_145793 | -1 | 0.005973491 | 0.61 (0.43, 0.87) | 8.40% | 1.01 | 1.25 |
| GFRA1 | NM_001145453 | -1 | 0.005973491 | 0.61 (0.43, 0.87) | 8.40% | 1.01 | 1.25 |
| GFRA1 | NM_005264 | -1 | 0.005973491 | 0.61 (0.43, 0.87) | 8.40% | 1.01 | 1.25 |
| GLRX2 | NM_016066 | 1 | 0.006629201 | 2.04 (1.22, 3.43) | 8.80% | 1.01 | 1.23 |
| GLRX2 | NM_197962 | 1 | 0.006629201 | 2.04 (1.22, 3.43) | 8.80% | 1.01 | 1.23 |
| GPR17 | NM_001161415 | -1 | 0.004359195 | 0.66 (0.50, 0.88) | 7.10% | 1.01 | 1.26 |
| GPR17 | NM_001161417 | -1 | 0.004359195 | 0.66 (0.50, 0.88) | 7.10% | 1.01 | 1.26 |
| GPR17 | NM_001161416 | -1 | 0.004359195 | 0.66 (0.50, 0.88) | 7.10% | 1.01 | 1.26 |
| GPR17 | NM_005291 | -1 | 0.004359195 | 0.66 (0.50, 0.88) | 7.10% | 1.01 | 1.26 |
| GPR33 | NM_001197184 | 1 | 0.004512989 | 1.30 (1.08, 1.56) | 7.20% | 1.01 | 1.22 |
| GPR33 | NR_036675 | 1 | 0.004512989 | 1.30 (1.08, 1.56) | 7.20% | 1.01 | 1.22 |
| GPR63 | NM_001143957 | 1 | 0.006597235 | 1.68 (1.15, 2.44) | 8.80% | 1.01 | 1.25 |
| GPR63 | NM_030784 | 1 | 0.006597235 | 1.68 (1.15, 2.44) | 8.80% | 1.01 | 1.25 |
| GREB1L | NM_001142966 | -1 | 0.004120083 | 0.63 (0.46, 0.86) | 6.90% | 1.01 | 1.27 |
| GRPEL2 | NM_152407 | 1 | 0.005432863 | 1.99 (1.23, 3.24) | 7.90% | 1.01 | 1.24 |
| HIF1A | NM_001530 | 1 | 0.006328987 | 1.64 (1.15, 2.33) | 8.60% | 1.01 | 1.25 |
| HIF1A | NM_181054 | 1 | 0.006328987 | 1.64 (1.15, 2.33) | 8.60% | 1.01 | 1.25 |
| HIST2H2AC | NM_003517 | 1 | 0.00431244 | 1.73 (1.19, 2.52) | 7.10% | 1.01 | 1.26 |
| HMX1 | NM_018942 | 1 | 0.003648411 | 1.34 (1.10, 1.63) | 6.50% | 1.01 | 1.24 |
| HOMER1 | NM_004272 | 1 | 0.004348297 | 1.84 (1.21, 2.79) | 7.10% | 1.01 | 1.26 |
| HSPA12A | NM_025015 | -1 | 0.00605439 | 0.62 (0.44, 0.87) | 8.40% | 1.01 | 1.25 |
| HSPBAP1 | NM_024610 | -1 | 0.005349517 | 0.60 (0.42, 0.86) | 7.90% | 1.01 | 1.25 |
| IDH1 | NM_005896 | 1 | 0.004957583 | 1.57 (1.15, 2.15) | 7.50% | 1.01 | 1.26 |
| IL20RB | NM_144717 | 1 | 0.004776866 | 1.77 (1.19, 2.64) | 7.40% | 1.01 | 1.26 |
| IL6ST | NM_001190981 | -1 | 0.006042753 | 0.55 (0.36, 0.84) | 8.40% | 1.01 | 1.24 |
| IL6ST | NM_175767 | -1 | 0.006042753 | 0.55 (0.36, 0.84) | 8.40% | 1.01 | 1.24 |
| IL6ST | NM_002184 | -1 | 0.006042753 | 0.55 (0.36, 0.84) | 8.40% | 1.01 | 1.24 |
| IPW | NR_023915 | -1 | 0.004512207 | 0.64 (0.47, 0.87) | 7.20% | 1.01 | 1.26 |
| IRGM | NM_001145805 | 1 | 0.004422765 | 1.53 (1.14, 2.06) | 7.10% | 1.01 | 1.26 |
| ITGB4 | NM_001005731 | 1 | 0.004372283 | 1.60 (1.16, 2.21) | 7.10% | 1.01 | 1.27 |
| ITGB4 | NM_000213 | 1 | 0.004372283 | 1.60 (1.16, 2.21) | 7.10% | 1.01 | 1.27 |
| ITGB4 | NN_001005619 | 1 | 0.004372283 | 1.60 (1.16, 2.21) | 7.10% | 1.01 | 1.27 |
| KDELR1 | NM_006801 | 1 | 0.005637 | 1.63 (1.15, 2.31) | 8.10% | 1.01 | 1.26 |
| KDM5A | NM_001042603 | -1 | 0.006234911 | 0.58 (0.39, 0.86) | 8.50% | 1.01 | 1.25 |
| KIAA1109 | NM_015312 | -1 | 0.004341596 | 0.63 (0.46, 0.87) | 7.10% | 1.01 | 1.26 |
| KIAA1217 | NM_001098500 | -1 | 0.004307668 | 0.60 (0.43, 0.85) | 7.10% | 1.01 | 1.26 |
| KIAA1217 | NM_019590 | -1 | 0.004307668 | 0.60 (0.43, 0.85) | 7.10% | 1.01 | 1.26 |
| KIAA1217 | NM_001098501 | -1 | 0.004307668 | 0.60 (0.43, 0.85) | 7.10% | 1.01 | 1.26 |
| KIAA1324 | NM_020775 | -1 | 0.004339938 | 0.68 (0.52, 0.88) | 7.10% | 1.01 | 1.26 |
| KIF23 | NM_138555 | 1 | 0.005200001 | 1.91 (1.21, 3.01) | 7.70% | 1.01 | 1.25 |
| KIF23 | NM_004856 | 1 | 0.005200001 | 1.91 (1.21, 3.01) | 7.70% | 1.01 | 1.25 |
| KIF27 | NM_017576 | -1 | 0.005549882 | 0.66 (0.50, 0.89) | 8.00% | 1.01 | 1.25 |
| KIRREL | NM_018240 | -1 | 0.004133502 | 0.67 (0.51, 0.88) | 6.90% | 1.01 | 1.26 |
| KLHL7 | NR_033328 | 1 | 0.005009326 | 1.46 (1.12, 1.91) | 7.60% | 1.01 | 1.26 |
| KLHL7 | NM_018846 | 1 | 0.005009326 | 1.46 (1.12, 1.91) | 7.60% | 1.01 | 1.26 |
| KLHL7 | NR_033329 | 1 | 0.005009326 | 1.46 (1.12, 1.91) | 7.60% | 1.01 | 1.26 |
| KLHL7 | NM_001172428 | 1 | 0.005009326 | 1.46 (1.12, 1.91) | 7.60% | 1.01 | 1.26 |
| KLHL7 | NM_001031710 | 1 | 0.005009326 | 1.46 (1.12, 1.91) | 7.60% | 1.01 | 1.26 |
| KRT20 | NM 019010 | 1 | 0.004928084 | 1.36 (1.10, 1.68) | 7.50% | 1.01 | 1.24 |
| KRT72 | NM_001146225 | 1 | 0.006068111 | 1.44 (1.11, 1.86) | 8.40% | 1.01 | 1.25 |
| KRT72 | NM_080747 | 1 | 0.006068111 | 1.44 (1.11, 1.86) | 8.40% | 1.01 | 1.25 |
| KRT72 | NM_001146226 | 1 | 0.006068111 | 1.44 (1.11, 1.86) | 8.40% | 1.01 | 1.25 |
| LIN54 | NM_001115007 | 1 | 0.004747521 | 1.88 (1.21, 2.93) | 7.40% | 1.01 | 1.25 |
| LIN54 | NM_194282 | 1 | 0.004747521 | 1.88 (1.21, 2.93) | 7.40% | 1.01 | 1.25 |
| LIN54 | NM_001115008 | 1 | 0.004747521 | 1.88 (1.21, 2.93) | 7.40% | 1.01 | 1.25 |
| LINGO1 | NM_032808 | 1 | 0.004519614 | 1.93 (1.23, 3.03) | 7.20% | 1.01 | 1.25 |
| LOC100129046 | NR_034091 | -1 | 0.005971508 | 0.58 (0.40, 0.86) | 8.40% | 1.01 | 1.25 |
| LOC100499484 | NR_036526 | -1 | 0.004306021 | 0.60 (0.42, 0.85) | 7.10% | 1.01 | 1.26 |
| LOC344595 | NR_028301 | -1 | 0.004316669 | 0.61 (0.43, 0.86) | 7.10% | 1.01 | 1.26 |
| LOC344595 | NR_028302 | -1 | 0.004316669 | 0.61 (0.43, 0.86) | 7.10% | 1.01 | 1.26 |
| LOC728323 | NR_024437 | -1 | 0.004035146 | 0.69 (0.53, 0.89) | 6.90% | 1.01 | 1.26 |
| LRRIQ1 | NM_001079910 | -1 | 0.006050079 | 0.62 (0.44, 0.87) | 8.40% | 1.01 | 1.25 |
| LRRIQ1 | NM 032165 | -1 | 0.006050079 | 0.62 (0.44, 0.87) | 8.40% | 1.01 | 1.25 |
| MAD2L1 | NM_002358 | 1 | 0.006190566 | 1.86 (1.19, 2.90) | 8.50% | 1.01 | 1.24 |
| MAGT1 | NM_032121 | 1 | 0.006485501 | 1.59 (1.14, 2.22) | 8.70% | 1.01 | 1.25 |
| MAPK81P3 | NM_001040439 | -1 | 0.0062091 | 0.62 (0.44, 0.87) | 8.50% | 1.01 | 1.25 |
| MAPK8IP3 | NM_015133 | -1 | 0.0062091 | 0.62 (0.44, 0.87) | 8.50% | 1.01 | 1.25 |
| MATN3 | NM 002381 | -1 | 0.004632692 | 0.61 (0.43, 0.86) | 7.30% | 1.01 | 1.26 |
| METTL3 | NM_019852 | -1 | 0.005935671 | 0.59 (0.41, 0.86) | 8.40% | 1.01 | 1.25 |
| MFSD7 | NM_032219 | -1 | 0.005041383 | 0.68 (0.52, 0.89) | 7.60% | 1.01 | 1.25 |
| MIR501 | NR 030225 | 1 | 0.004596181 | 1.39 (1.11, 1.74) | 7.30% | 1.01 | 1.25 |
| MKI67 | NM_002417 | 1 | 0.004838869 | 1.85 (1.21, 2.83) | 7.40% | 1.01 | 1.25 |
| MKI67 | NM_001145966 | 1 | 0.004838869 | 1.85 (1.21, 2.83) | 7.40% | 1.01 | 1.25 |
| MOXD2P | NR_024346 | 1 | 0.00477089 | 1.52 (1.14, 2.02) | 7.40% | 1.01 | 1.26 |
| MPHOSPH6 | NM_005792 | 1 | 0.00484696 | 1.48 (1.13, 1.94) | 7.40% | 1.01 | 1.26 |
| MTDH | NM_178812 | 1 | 0.004800288 | 1.55 (1.14, 2.10) | 7.40% | 1.01 | 1.26 |
| MTMR9LP | NR_026850 | -1 | 0.00483402 | 0.67 (0.51, 0.89) | 7.40% | 1.01 | 1.26 |
| MUC12 | NM_001164462 | -1 | 0.006098226 | 0.59 (0.40, 0.86) | 8.40% | 1.01 | 1.25 |
| MVD | NM_002461 | 1 | 0.005857959 | 2.04 (1.23, 3.39) | 8.30% | 1.01 | 1.23 |
| NBN | NM_002485 | 1 | 0.005163332 | 1.63 (1.16, 2.30) | 7.70% | 1.01 | 1.26 |
| NBPF1 | NM_017940 | -1 | 0.005126436 | 0.63 (0.46, 0.87) | 7.70% | 1.01 | 1.26 |
| NOTCH 1 | NM_017617 | 1 | 0.004096101 | 1.50 (1.14, 1.99) | 6.90% | 1.01 | 1.27 |
| NPC1L1 | NM_001101648 | 1 | 0.005266683 | 1.44 (1.11, 1.85) | 7.80% | 1.01 | 1.25 |
| NPC1L1 | NM_013389 | 1 | 0.005266683 | 1.44 (1.11, 1.85) | 7.80% | 1.01 | 1.25 |
| NUAK1 | NM_014840 | -1 | 0.00483339 | 0.66 (0.50, 0.88) | 7.40% | 1.01 | 1.26 |
| NUF2 | NM_145697 | 1 | 0.004332713 | 1.64 (1.17, 2.30) | 7.10% | 1.01 | 1.27 |
| NUF2 | NM_031423 | 1 | 0.004332713 | 1.64 (1.17, 2.30) | 7.10% | 1.01 | 1.27 |
| NUP93 | NM_014669 | 1 | 0.005806313 | 1.70 (1.17, 2.48) | 8.30% | 1.01 | 1.25 |
| NUSAP1 | NM_001129897 | 1 | 0.005759989 | 1.86 (1.20, 2.88) | 8.20% | 1.01 | 1.25 |
| NUSAP1 | NM_018454 | 1 | 0.005759989 | 1.86 (1.20, 2.88) | 8.20% | 1.01 | 1.25 |
| NUSAP1 | NM_016359 | 1 | 0.005759989 | 1.86 (1.20, 2.88) | 8.20% | 1.01 | 1.25 |
| OCM | NM_001097622 | 1 | 0.004906929 | 1.30 (1.08, 1.56) | 7.50% | 1.01 | 1.22 |
| ODC1 | NM_002539 | 1 | 0.006073792 | 1.66 (1.16, 2.38) | 8.40% | 1.01 | 1.25 |
| OGDHL | NM_001143997 | 1 | 0.005912877 | 1.58 (1.14, 2.18) | 8.30% | 1.01 | 1.26 |
| OGDHL | NM_001143996 | 1 | 0.005912877 | 1.58 (1.14, 2.18) | 8.30% | 1.01 | 1.26 |
| OGDHL | NM_018245 | 1 | 0.005912877 | 1.58 (1.14, 2.18) | 8.30% | 1.01 | 1.26 |
| OR14J1 | NM_030946 | 1 | 0.004503814 | 1.46 (1.12, 1.89) | 7.20% | 1.01 | 1.26 |
| OR5T2 | NM_001004746 | 1 | 0.005223593 | 1.71 (1.17, 2.50) | 7.70% | 1.01 | 1.26 |
| P4HA1 | NM_001017962 | 1 | 0.004247315 | 1.44 (1.12, 1.84) | 7.00% | 1.01 | 1.26 |
| P4HA1 | NM_001142595 | 1 | 0.004247315 | 1.44 (1.12, 1.84) | 7.00% | 1.01 | 1.26 |
| P4HA1 | NM_001142596 | 1 | 0.004247315 | 1.44 (1.12, 1.84) | 7.00% | 1.01 | 1.26 |
| P4HA1 | NM_000917 | 1 | 0.004247315 | 1.44 (1.12, 1.84) | 7.00% | 1.01 | 1.26 |
| PACSIN2 | NM_001184970 | 1 | 0.004070682 | 1.60 (1.16, 2.21) | 6.90% | 1.01 | 1.27 |
| PACSIN2 | NM_007229 | 1 | 0.004070682 | 1.60 (1.16, 2.21) | 6.90% | 1.01 | 1.27 |
| PACSIN2 | NM_001184971 | 1 | 0.004070682 | 1.60 (1.16, 2.21) | 6.90% | 1.01 | 1.27 |
| PAPL | NM_001004318 | 1 | 0.00430893 | 1.48 (1.13, 1.93) | 7.10% | 1.01 | 1.26 |
| PAR1 | NR_022009 | -1 | 0.005038754 | 0.64 (0.47, 0.87) | 7.60% | 1.01 | 1.26 |
| PCDP1 | NM_001029996 | -1 | 0.00550873 | 0.56 (0.37, 0.84) | 8.00% | 1.01 | 1.25 |
| PCP4L1 | NM_001102566 | 1 | 0.005705339 | 1.77 (1.18, 2.64) | 8.20% | 1.01 | 1.25 |
| PCYOX1L | NM_024028 | -1 | 0.006326101 | 0.63 (0.45, 0.88) | 8.60% | 1.01 | 1.25 |
| PDZRN3 | NM_015009 | -1 | 0.004230075 | 0.63 (0.46, 0.87) | 7.00% | 1.01 | 1.26 |
| PGRMC1 | NM_006667 | 1 | 0.005470607 | 1.48 (1.12, 1.95) | 7.90% | 1.01 | 1.26 |
| PIAS2 | NM_004671 | -1 | 0.006115119 | 0.69 (0.53, 0.90) | 8.50% | 1.01 | 1.25 |
| PIAS2 | NM_173206 | -1 | 0.006115119 | 0.69 (0.53, 0.90) | 8.50% | 1.01 | 1.25 |
| PIH1D2 | NM_001082619 | -1 | 0.00647425 | 0.59 (0.40, 0.86) | 8.70% | 1.01 | 1.25 |
| PIH1D2 | NM_138789 | -1 | 0.00647425 | 0.59 (0.40, 0.86) | 8.70% | 1.01 | 1.25 |
| PINK1 | NM_032409 | -1 | 0.006364293 | 0.70 (0.54, 0.90) | 8.60% | 1.01 | 1.24 |
| PLA2G2F | NM_022819 | 1 | 0.004792716 | 1.53 (1.14, 2.05) | 7.40% | 1.01 | 1.26 |
| PLCD3 | NM_133373 | -1 | 0.004835269 | 0.68 (0.52, 0.89) | 7.40% | 1.01 | 1.25 |
| PRKAR2A | NM_004157 | 1 | 0.004456628 | 1.74 (1.19, 2.56) | 7.20% | 1.01 | 1.26 |
| PSMA4 | NM_001102667 | 1 | 0.006063723 | 1.93 (1.21, 3.07) | 8.40% | 1.01 | 1.24 |
| PSMA4 | NM_001102668 | 1 | 0.006063723 | 1.93 (1.21, 3.07) | 8.40% | 1.01 | 1.24 |
| PSMA4 | NM_002789 | 1 | 0.006063723 | 1.93 (1.21, 3.07) | 8.40% | 1.01 | 1.24 |
| PSMC4 | NM_006503 | 1 | 0.004218907 | 1.38 (1.11, 1.72) | 7.00% | 1.01 | 1.25 |
| PSMC4 | NM_153001 | 1 | 0.004218907 | 1.38 (1.11, 1.72) | 7.00% | 1.01 | 1.25 |
| PSMG3 | NM_001134340 | 1 | 0.004706287 | 1.68 (1.17, 2.40) | 7.40% | 1.01 | 1.26 |
| PSMG3 | NM_032302 | 1 | 0.004706287 | 1.68 (1.17, 2.40) | 7.40% | 1.01 | 1.26 |
| RALA | NM_005402 | 1 | 0.005804625 | 1.66 (1.16, 2.39) | 8.30% | 1.01 | 1.26 |
| RECQL4 | NM_004260 | 1 | 0.005197018 | 1.75 (1.18, 2.58) | 7.70% | 1.01 | 1.26 |
| RHEB | NM_005614 | 1 | 0.005237779 | 1.90 (1.21, 2.98) | 7.80% | 1.01 | 1.25 |
| RLIM | NM_183353 | 1 | 0.005064841 | 1.66 (1.16, 2.36) | 7.60% | 1.01 | 1.26 |
| RLIM | NM_016120 | 1 | 0.005064841 | 1.66 (1.16, 2.36) | 7.60% | 1.01 | 1.26 |
| RPL23AP53 | NR_003572 | -1 | 0.006006153 | 0.60 (0.42, 0.86) | 8.40% | 1.01 | 1.25 |
| RPL8 | NM_000973 | 1 | 0.005660274 | 1.52 (1.13, 2.05) | 8.10% | 1.01 | 1.26 |
| RPL8 | NM_033301 | 1 | 0.005660274 | 1.52 (1.13, 2.05) | 8.10% | 1.01 | 1.26 |
| RSPH1 | NM_080860 | -1 | 0.004552589 | 0.64 (0.46, 0.87) | 7.20% | 1.01 | 1.26 |
| RUNX1 | NM_001001890 | -1 | 0.005414839 | 0.53 (0.34, 0.83) | 7.90% | 1.01 | 1.24 |
| RUNX1 | NM_001754 | -1 | 0.005414839 | 0.53 (0.34, 0.83) | 7.90% | 1.01 | 1.24 |
| RUNX1 | NM_001122607 | -1 | 0.005414839 | 0.53 (0.34, 0.83) | 7.90% | 1.01 | 1.24 |
| RXFP2 | NM_130806 | 1 | 0.004747273 | 1.24 (1.07, 1.44) | 7.40% | 1.01 | 1.19 |
| RXFP2 | NM_001166058 | 1 | 0.004747273 | 1.24 (1.07, 1.44) | 7.40% | 1.01 | 1.19 |
| SC4MOL | NM_006745 | 1 | 0.00570173 | 1.51 (1.13, 2.02) | 8.20% | 1.01 | 1.26 |
| SC4MOL | NM_001017369 | 1 | 0.00570173 | 1.51 (1.13, 2.02) | 8.20% | 1.01 | 1.26 |
| SCD | NM_005063 | 1 | 0.00542315 | 1.91 (1.21, 3.02) | 7.90% | 1.01 | 1.24 |
| SEC13 | NR_024273 | 1 | 0.006369561 | 1.64 (1.15, 2.35) | 8.60% | 1.01 | 1.25 |
| SEC13 | NM_001136232 | 1 | 0.006369561 | 1.64 (1.15, 2.35) | 8.60% | 1.01 | 1.25 |
| SEC13 | NR_024272 | 1 | 0.006369561 | 1.64 (1.15, 2.35) | 8.60% | 1.01 | 1.25 |
| SEC13 | NM_183352 | 1 | 0.006369561 | 1.64 (1.15, 2.35) | 8.60% | 1.01 | 1.25 |
| SEC24A | NM_021982 | 1 | 0.006121721 | 1.65 (1.15, 2.37) | 8.50% | 1.01 | 1.25 |
| SERPIND1 | NM_000185 | -1 | 0.004249699 | 0.57 (0.39, 0.84) | 7.00% | 1.01 | 1.26 |
| SFXN5 | NM_144579 | -1 | 0.005790443 | 0.64 (0.47, 0.88) | 8.20% | 1.01 | 1.25 |
| SKA2 | NN_182620 | 1 | 0.004751619 | 1.58 (1.15, 2.18) | 7.40% | 1.01 | 1.26 |
| SKA2 | NM_001100595 | 1 | 0.004751619 | 1.58 (1.15, 2.18) | 7.40% | 1.01 | 1.26 |
| SLC30A10 | NM_018713 | 1 | 0.005198017 | 1.37 (1.10, 1.71) | 7.70% | 1.01 | 1.24 |
| SLC41A2 | NM_032148 | 1 | 0.005498771 | 1.44 (1.11, 1.87) | 8.00% | 1.01 | 1.25 |
| SLC44A1 | NM_080546 | -1 | 0.004388528 | 0.62 (0.45, 0.86) | 7.10% | 1.01 | 1.26 |
| SLC9A5 | NM_004594 | 1 | 0.006146031 | 1.70 (1.16, 2.47) | 8.50% | 1.01 | 1.25 |
| SMG7 | NM_173156 | 1 | 0.005723927 | 1.47 (1.12, 1.93) | 8.20% | 1.01 | 1.25 |
| SMG7 | NM_201569 | 1 | 0.005723927 | 1.47 (1.12, 1.93) | 8.20% | 1.01 | 1.25 |
| SMG7 | NM_201568 | 1 | 0.005723927 | 1.47 (1.12, 1.93) | 8.20% | 1.01 | 1.25 |
| SMG7 | NM_001174061 | 1 | 0.005723927 | 1.47 (1.12, 1.93) | 8.20% | 1.01 | 1.25 |
| SND1 | NM_014390 | 1 | 0.004484231 | 1.70 (1.18, 2.45) | 7.20% | 1.01 | 1.26 |
| SNORD116-12 | NR_003327 | 1 | 0.006075002 | 1.32 (1.08, 1.60) | 8.40% | 1.01 | 1.22 |
| SNRNP70 | NM_003089 | -1 | 0.004606328 | 0.54 (0.36, 0.83) | 7.30% | 1.01 | 1.25 |
| SQSTM1 | NM_003900 | 1 | 0.004571164 | 1.83 (1.20, 2.78) | 7.20% | 1.01 | 1.26 |
| SQSTM1 | NM_001142299 | 1 | 0.004571164 | 1.83 (1.20, 2.78) | 7.20% | 1.01 | 1.26 |
| SQSTM1 | NM_001142298 | 1 | 0.004571164 | 1.83 (1.20, 2.78) | 7.20% | 1.01 | 1.26 |
| SREBF2 | NM_004599 | 1 | 0.004241128 | 1.61 (1.16, 2.22) | 7.00% | 1.01 | 1.27 |
| SRSF5 | NM_006925 | -1 | 0.005863942 | 0.54 (0.35, 0.84) | 8.30% | 1.01 | 1.24 |
| SRSF5 | NM_001039465 | -1 | 0.005863942 | 0.54 (0.35, 0.84) | 8.30% | 1.01 | 1.24 |
| SSR1 | NM_003144 | 1 | 0.005569398 | 1.63 (1.15, 2.31) | 8.10% | 1.01 | 1.26 |
| STATH | NM_001009181 | 1 | 0.003857366 | 1.36 (1.11, 1.68) | 6.70% | 1.01 | 1.24 |
| STATH | NM_003154 | 1 | 0.003857366 | 1.36 (1.11, 1.68) | 6.70% | 1.01 | 1.24 |
| STIP1 | NM_006819 | 1 | 0.006434469 | 1.58 (1.14, 2.19) | 8.70% | 1.01 | 1.25 |
| STX1A | NM_004603 | 1 | 0.006602765 | 1.71 (1.16, 2.52) | 8.80% | 1.01 | 1.25 |
| STX1A | NM_001165903 | 1 | 0.006602765 | 1.71 (1.16, 2.52) | 8.80% | 1.01 | 1.25 |
| STX1B | NM_052874 | -1 | 0.004730101 | 0.61 (0.44, 0.86) | 7.40% | 1.01 | 1.26 |
| SUMO3 | NM_006936 | 1 | 0.005665759 | 1.83 (1.19, 2.80) | 8.10% | 1.01 | 1.25 |
| TADA1 | NM_053053 | 1 | 0.00455637 | 1.54 (1.14, 2.08) | 7.20% | 1.01 | 1.26 |
| TARS | NM_152295 | 1 | 0.005460832 | 1.63 (1.15, 2.30) | 7.90% | 1.01 | 1.26 |
| TBC1D19 | NM_018317 | -1 | 0.004309516 | 0.66 (0.50, 0.88) | 7.10% | 1.01 | 1.26 |
| TCTN2 | NM_024809 | -1 | 0.00482289 | 0.58 (0.40, 0.85) | 7.40% | 1.01 | 1.26 |
| TCTN2 | NM_001143850 | -1 | 0.00482289 | 0.58 (0.40, 0.85) | 7.40% | 1.01 | 1.26 |
| TMEM132A | NM_178031 | 1 | 0.005666749 | 1.66 (1.16, 2.38) | 8.10% | 1.01 | 1.26 |
| TMEM132A | NM_017870 | 1 | 0.005666749 | 1.66 (1.16, 2.38) | 8.10% | 1.01 | 1.26 |
| TMEM150A | NR_033179 | 1 | 0.004738486 | 1.78 (1.19, 2.67) | 7.40% | 1.01 | 1.26 |
| TMEM150A | NM_001031738 | 1 | 0.004738486 | 1.78 (1.19, 2.67) | 7.40% | 1.01 | 1.26 |
| TMEM200C | NM_001080209 | -1 | 0.006303527 | 0.59 (0.41, 0.86) | 8.60% | 1.01 | 1.25 |
| TOMM70A | NM_014820 | 1 | 0.005119582 | 1.65 (1.16, 2.34) | 7.70% | 1.01 | 1.26 |
| TPD52L1 | NM_003287 | 1 | 0.00467689 | 1.74 (1.18, 2.55) | 7.30% | 1.01 | 1.26 |
| TPD52L1 | NM_001003397 | 1 | 0.00467689 | 1.74 (1.18, 2.55) | 7.30% | 1.01 | 1.26 |
| TPD52L1 | NM_001003396 | 1 | 0.00467689 | 1.74 (1.18, 2.55) | 7.30% | 1.01 | 1.26 |
| TPD52L1 | NM_001003395 | 1 | 0.00467689 | 1.74 (1.18, 2.55) | 7.30% | 1.01 | 1.26 |
| TPTE | NM_199259 | 1 | 0.003424575 | 1.34 (1.10, 1.63) | 6.30% | 1.01 | 1.24 |
| TPTE | NM_199261 | 1 | 0.003424575 | 1.34 (1.10, 1.63) | 6.30% | 1.01 | 1.24 |
| TPTE | NM_199260 | 1 | 0.003424575 | 1.34 (1.10, 1.63) | 6.30% | 1.01 | 1.24 |
| TRIM66 | NM_014818 | -1 | 0.006607717 | 0.60 (0.41, 0.87) | 8.80% | 1.01 | 1.25 |
| TRIP13 | NM_004237 | 1 | 0.006392425 | 2.19 (1.25, 3.86) | 8.60% | 1.01 | 1.22 |
| TRIP13 | NM_001166260 | 1 | 0.006392425 | 2.19 (1.25, 3.86) | 8.60% | 1.01 | 1.22 |
| TRPC6 | NM_004621 | -1 | 0.004611756 | 0.63 (0.46, 0.87) | 7.30% | 1.01 | 1.26 |
| TRPM1 | NM_002420 | 1 | 0.003408407 | 1.31 (1.09, 1.56) | 6.30% | 1.01 | 1.23 |
| TRYX3 | NM_001001317 | 1 | 0.005404072 | 1.62 (1.15, 2.28) | 7.90% | 1.01 | 1.26 |
| TSPAN19 | NM_001100917 | 1 | 0.005213327 | 1.31 (1.08, 1.59) | 7.70% | 1.01 | 1.22 |
| TTC4 | NM_004623 | -1 | 0.005824634 | 0.53 (0.34, 0.83) | 8.30% | 1.01 | 1.24 |
| UNC5B | NM_170744 | -1 | 0.005298662 | 0.63 (0.46, 0.87) | 7.80% | 1.01 | 1.26 |
| VTA1 | NM_016485 | 1 | 0.006405376 | 1.46 (1.11, 1.92) | 8.60% | 1.01 | 1.25 |
| WDR60 | NM_018051 | -1 | 0.005425427 | 0.64 (0.47, 0.88) | 7.90% | 1.01 | 1.26 |
| WDR65 | NM_001195831 | -1 | 0.005313098 | 0.62 (0.45, 0.87) | 7.80% | 1.01 | 1.26 |
| WDR65 | NM_001167965 | -1 | 0.005313098 | 0.62 (0.45, 0.87) | 7.80% | 1.01 | 1.26 |
| WDR65 | NM_152498 | -1 | 0.005313098 | 0.62 (0.45, 0.87) | 7.80% | 1.01 | 1.26 |
| WDR65 | NM_001167966 | -1 | 0.005313098 | 0.62 (0.45, 0.87) | 7.80% | 1.01 | 1.26 |
| WDR65 | NR_030778 | -1 | 0.005313098 | 0.62 (0.45, 0.87) | 7.80% | 1.01 | 1.26 |
| WDTC1 | NM_015023 | -1 | 0.006462542 | 0.69 (0.53, 0.90) | 8.70% | 1.01 | 1.24 |
| YIF1A | NM_020470 | 1 | 0.004460223 | 1.70 (1.18, 2.46) | 7.20% | 1.01 | 1.26 |
| ZBTB4 | NM_001128833 | -1 | 0.004317842 | 0.63 (0.46, 0.86) | 7.10% | 1.01 | 1.26 |
| ZBTB4 | NM_020899 | -1 | 0.004317842 | 0.63 (0.46, 0.86) | 7.10% | 1.01 | 1.26 |
| ZCCHC7 | NM_032226 | -1 | 0.00624057 | 0.51 (0.31, 0.83) | 8.50% | 1.01 | 1.23 |
| ZNF43 | NM_003423 | -1 | 0.005996525 | 0.64 (0.47, 0.88) | 8.40% | 1.01 | 1.25 |
| ZNF438 | NM_001143771 | -1 | 0.005439166 | 0.62 (0.44, 0.87) | 7.90% | 1.01 | 1.26 |
| ZNF438 | NM_001143767 | -1 | 0.005439166 | 0.62 (0.44, 0.87) | 7.90% | 1.01 | 1.26 |
| ZNF438 | NM_001143766 | -1 | 0.005439166 | 0.62 (0.44, 0.87) | 7.90% | 1.01 | 1.26 |
| ZNF438 | NM_001143770 | -1 | 0.005439166 | 0.62 (0.44, 0.87) | 7.90% | 1.01 | 1.26 |
| ZNF438 | NM_182755 | -1 | 0.005439166 | 0.62 (0.44, 0.87) | 7.90% | 1.01 | 1.26 |
| ZNF438 | NR_026560 | -1 | 0.005439166 | 0.62 (0.44, 0.87) | 7.90% | 1.01 | 1.26 |
| ZNF438 | NM_001143768 | -1 | 0.005439166 | 0.62 (0.44, 0.87) | 7.90% | 1.01 | 1.26 |
| ZNF438 | NM_001143769 | -1 | 0.005439166 | 0.62 (0.44, 0.87) | 7.90% | 1.01 | 1.26 |
| ZNF564 | NM_144976 | -1 | 0.004988278 | 0.59 (0.41, 0.85) | 7.60% | 1.01 | 1.26 |
| ZNF668 | NM_024706 | -1 | 0.004456171 | 0.66 (0.50, 0.88) | 7.20% | 1.01 | 1.26 |
| ZNF668 | NM_001172670 | -1 | 0.004456171 | 0.66 (0.50, 0.88) | 7.20% | 1.01 | 1.26 |
| ZNF668 | NM_001172669 | -1 | 0.004456171 | 0.66 (0.50, 0.88) | 7.20% | 1.01 | 1.26 |
| ZNF668 | NM_001172668 | -1 | 0.004456171 | 0.66 (0.50, 0.88) | 7.20% | 1.01 | 1.26 |
| ZNF839 | NM_018335 | -1 | 0.005746311 | 0.56 (0.37, 0.85) | 8.20% | 1.01 | 1.25 |
| ADAM22 | NM_004194 | -1 | 0.008205802 | 0.65 (0.47, 0.89) | 9.80% | 1 | 1.24 |
| ADAM22 | NM_021722 | -1 | 0.008205802 | 0.65 (0.47, 0.89) | 9.80% | 1 | 1.24 |
| ADAM22 | NM_021721 | -1 | 0.008205802 | 0.65 (0.47, 0.89) | 9.80% | 1 | 1.24 |
| ADAM22 | NM_021723 | -1 | 0.008205802 | 0.65 (0.47, 0.89) | 9.80% | 1 | 1.24 |
| ADAM22 | NM_016351 | -1 | 0.008205802 | 0.65 (0.47, 0.89) | 9.80% | 1 | 1.24 |
| ADM | NM_001124 | 1 | 0.007902715 | 1.60 (1.13, 2.27) | 9.70% | 1 | 1.25 |
| ALG9 | NM_001077691 | -1 | 0.006987624 | 0.69 (0.52, 0.90) | 9.10% | 1 | 1.24 |
| ALG9 | NM_024740 | -1 | 0.006987624 | 0.69 (0.52, 0.90) | 9.10% | 1 | 1.24 |
| ALG9 | NM_001077690 | -1 | 0.006987624 | 0.69 (0.52, 0.90) | 9.10% | 1 | 1.24 |
| ALG9 | NM_001077692 | -1 | 0.006987624 | 0.69 (0.52, 0.90) | 9.10% | 1 | 1.24 |
| APCS | NM_001639 | 1 | 0.00751427 | 1.21 (1.05, 1.40) | 9.40% | 1 | 1.18 |
| ARMCX4 | NR_028407 | -1 | 0.007606047 | 0.59 (0.40, 0.87) | 9.50% | 1 | 1.24 |
| ATL2 | NM_022374 | 1 | 0.006753427 | 1.47 (1.11, 1.94) | 8.90% | 1 | 1.25 |
| ATL2 | NM_001135673 | 1 | 0.006753427 | 1.47 (1.11, 1.94) | 8.90% | 1 | 1.25 |
| ATL2 | NR_024191 | 1 | 0.006753427 | 1.47 (1.11, 1.94) | 8.90% | 1 | 1.25 |
| BAZ2B | NM_013450 | -1 | 0.007896861 | 0.63 (0.44, 0.88) | 9.70% | 1 | 1.24 |
| BNIP3 | NM_004052 | 1 | 0.008214484 | 1.55 (1.12, 2.13) | 9.90% | 1 | 1.25 |
| BRDT | NM_001726 | -1 | 0.00773667 | 0.53 (0.33, 0.85) | 9.60% | 1 | 1.23 |
| BRDT | NM_207189 | -1 | 0.00773667 | 0.53 (0.33, 0.85) | 9.60% | 1 | 1.23 |
| BUB3 | NM_004725 | 1 | 0.007105129 | 1.48 (1.11, 1.98) | 9.10% | 1 | 1.25 |
| BUB3 | NM_001007793 | 1 | 0.007105129 | 1.48 (1.11, 1.98) | 9.10% | 1 | 1.25 |
| C15orf2 | NM_018958 | 1 | 0.007451532 | 1.51 (1.12, 2.04) | 9.30% | 1 | 1.25 |
| C6orf123 | NR_026773 | 1 | 0.007331897 | 1.64 (1.14, 2.34) | 9.30% | 1 | 1.25 |
| C9orf116 | NM_144654 | -1 | 0.007781927 | 0.62 (0.44, 0.88) | 9.60% | 1 | 1.24 |
| C9orf116 | NM_001048265 | -1 | 0.007781927 | 0.62 (0.44, 0.88) | 9.60% | 1 | 1.24 |
| CA5A | NM_001739 | 1 | 0.007743248 | 1.74 (1.16, 2.62) | 9.60% | 1 | 1.24 |
| CASK | NM_01126055 | 1 | 0.008238136 | 1.60 (1.13, 2.26) | 9.90% | 1 | 1.24 |
| CASK | NM_001126054 | 1 | 0.008238136 | 1.60 (1.13, 2.26) | 9.90% | 1 | 1.24 |
| CASK | NM_003688 | 1 | 0.008238136 | 1.60 (1.13, 2.26) | 9.90% | 1 | 1.24 |
| CAV3 | NM_033337 | 1 | 0.007209523 | 1.56 (1.13, 2.15) | 9.20% | 1 | 1.25 |
| CAV3 | NM_001234 | 1 | 0.007209523 | 1.56 (1.13, 2.15) | 9.20% | 1 | 1.25 |
| CCDC41 | NM_016122 | -1 | 0.008020691 | 0.60 (0.41, 0.88) | 9.70% | 1 | 1.24 |
| CCDC41 | NM_001042399 | -1 | 0.008020691 | 0.60 (0.41, 0.88) | 9.70% | 1 | 1.24 |
| CCNB2 | NM_004701 | 1 | 0.007331535 | 2.05 (1.21, 3.47) | 9.30% | 1 | 1.22 |
| CCR10 | NM_016602 | -1 | 0.007757267 | 0.60 (0.41, 0.87) | 9.60% | 1 | 1.24 |
| CDHR3 | NM_152750 | -1 | 0.008293294 | 0.63 (0.44, 0.89) | 9.90% | 1 | 1.24 |
| CHCHD2 | NM_016139 | 1 | 0.006949779 | 1.76 (1.17, 2.65) | 9.10% | 1 | 1.24 |
| CHP | NM_007236 | 1 | 0.007990476 | 1.61 (1.13, 2.30) | 9.70% | 1 | 1.25 |
| CHRNA6 | NM_004198 | -1 | 0.00809738 | 0.64 (0.47, 0.89) | 9.80% | 1 | 1.24 |
| CHRNA6 | NM_01199279 | -1 | 0.00809738 | 0.64 (0.47, 0.89) | 9.80% | 1 | 1.24 |
| CHRNE | NM_000080 | -1 | 0.007041906 | 0.68 (0.51, 0.90) | 9.10% | 1 | 1.24 |
| CMYA5 | NM_153610 | -1 | 0.006763751 | 0.61 (0.42, 0.87) | 8.90% | 1 | 1.25 |
| COL8A2 | NM_005202 | -1 | 0.007593958 | 0.64 (0.46, 0.89) | 9.50% | 1 | 1.24 |
| COLEC12 | NM_130386 | -1 | 0.007113712 | 0.67 (0.50, 0.90) | 9.20% | 1 | 1.25 |
| COQ4 | NM_016035 | -1 | 0.008371696 | 0.66 (0.48, 0.90) | 10.00% | 1 | 1.24 |
| COX5A | NM_004255 | 1 | 0.008188332 | 1.79 (1.16, 2.76) | 9.80% | 1 | 1.23 |
| CREB3L2 | NM_194071 | 1 | 0.007316954 | 1.72 (1.16, 2.55) | 9.30% | 1 | 1.24 |
| CSNK2A2 | NM_001896 | 1 | 0.007911319 | 1.76 (1.16, 2.67) | 9.70% | 1 | 1.24 |
| CST5 | NM_001900 | -1 | 0.007660357 | 0.41 (0.21, 0.79) | 9.50% | 1 | 1.19 |
| CTDSP1 | NM_021198 | -1 | 0.006755543 | 0.63 (0.45, 0.88) | 8.90% | 1 | 1.25 |
| CTDSP1 | NM_182642 | -1 | 0.006755543 | 0.63 (0.45, 0.88) | 8.90% | 1 | 1.25 |
| DCDC2 | NM_001195610 | -1 | 0.007215502 | 0.61 (0.43, 0.88) | 9.20% | 1 | 1.24 |
| DCDC2 | NM_016356 | -1 | 0.007215502 | 0.61 (0.43, 0.88) | 9.20% | 1 | 1.24 |
| DSCR4 | NM_005867 | 1 | 0.006963957 | 1.35 (1.09, 1.68) | 9.10% | 1 | 1.23 |
| ECT2 | NM_018098 | 1 | 0.007045075 | 1.62 (1.14, 2.30) | 9.10% | 1 | 1.25 |
| EPRS | NM_004446 | 1 | 0.007248665 | 1.70 (1.15, 2.50) | 9.20% | 1 | 1.25 |
| ESRRA | NM_004451 | 1 | 0.008289243 | 1.77 (1.16, 2.71) | 9.90% | 1 | 1.24 |
| F5 | NM_000130 | 1 | 0.007141726 | 1.45 (1.11, 1.90) | 9.20% | 1 | 1.25 |
| FAM169B | NM_182562 | 1 | 0.006944878 | 1.33 (1.08, 1.63) | 9.10% | 1 | 1.22 |
| FAM38B | NM_022068 | -1 | 0.007143819 | 0.50 (0.30, 0.83) | 9.20% | 1 | 1.22 |
| FBXO15 | NM_152676 | -1 | 0.008149201 | 0.63 (0.44, 0.89) | 9.80% | 1 | 1.24 |
| FBXO15 | NM_001142958 | -1 | 0.008149201 | 0.63 (0.44, 0.89) | 9.80% | 1 | 1.24 |
| FCAR | NM_133279 | -1 | 0.00758047 | 0.60 (0.42, 0.87) | 9.50% | 1 | 1.24 |
| FCAR | NM_133274 | -1 | 0.00758047 | 0.60 (0.42, 0.87) | 9.50% | 1 | 1.24 |
| FCAR | NM_133273 | -1 | 0.00758047 | 0.60 (0.42, 0.87) | 9.50% | 1 | 1.24 |
| FCAR | NM_002000 | -1 | 0.00758047 | 0.60 (0.42, 0.87) | 9.50% | 1 | 1.24 |
| FCAR | NM_133277 | -1 | 0.00758047 | 0.60 (0.42, 0.87) | 9.50% | 1 | 1.24 |
| FCAR | NM_133278 | -1 | 0.00758047 | 0.60 (0.42, 0.87) | 9.50% | 1 | 1.24 |
| FCAR | NM_133271 | -1 | 0.00758047 | 0.60 (0.42, 0.87) | 9.50% | 1 | 1.24 |
| FCAR | NM_133269 | -1 | 0.00758047 | 0.60 (0.42, 0.87) | 9.50% | 1 | 1.24 |
| FCAR | NM_133272 | -1 | 0.00758047 | 0.60 (0.42, 0.87) | 9.50% | 1 | 1.24 |
| FCGR2C | NM_201563 | -1 | 0.007000324 | 0.62 (044, 0.88) | 9.10% | 1 | 1.25 |
| FMNL3 | NM_175736 | -1 | 0.007177176 | 0.64 (0.47, 0.89) | 9.20% | 1 | 1.25 |
| FMNL3 | NM_198900 | -1 | 0.007177176 | 0.64 (0.47, 0.89) | 9.20% | 1 | 1.25 |
| FOXD4L5 | NM_01126334 | 1 | 0.00836323 | 1.43 (1.10, 1.87) | 10.00% | 1 | 1.24 |
| FOXJ2 | NM_018416 | -1 | 0.006621301 | 0.65 (0.48, 0.89) | 8.80% | 1 | 1.25 |
| GALNT5 | NM_014568 | -1 | 0.007050223 | 0.61 (0.43, 0.87) | 9.10% | 1 | 1.25 |
| GATA3 | NM_001002295 | -1 | 0.007666942 | 0.66 (0.48, 0.89) | 9.50% | 1 | 1.24 |
| GATA3 | NM_002051 | -1 | 0.007666942 | 0.66 (048, 0.89) | 9.50% | 1 | 1.24 |
| GATAD2A | NM_017660 | 1 | 0.006855234 | 1.35 (1.09, 1.68) | 9.00% | 1 | 1.23 |
| GGT3P | NR_003267 | 1 | 0.00774438 | 1.41 (1.10, 1.83) | 9.60% | 1 | 1.24 |
| GOT1 | NM_002079 | 1 | 0.007928124 | 1.68 (1.14, 2.45) | 9.70% | 1 | 1.24 |
| GPR12 | NM_005288 | 1 | 0.006922215 | 1.38 (1.09, 1.75) | 9.10% | 1 | 1.24 |
| GPR156 | NM_153002 | 1 | 0.007917594 | 1.57 (1.13, 2.20) | 9.70% | 1 | 1.25 |
| GPR156 | NM_001168271 | 1 | 0.007917594 | 1.57 (1.13, 2.20) | 9.70% | 1 | 1.25 |
| HCRTR2 | NM_001526 | 1 | 0.007044644 | 1.27 (1.07, 1.52) | 9.10% | 1 | 1.21 |
| HDC | NM_002112 | -1 | 0.006715858 | 0.61 (0.43, 0.87) | 8.90% | 1 | 1.25 |
| HNRNPA2B1 | NM_002137 | 1 | 0.006950753 | 1.38 (1.09, 1.75) | 9.10% | 1 | 1.24 |
| HNRNPA2B1 | NM_031243 | 1 | 0.006950753 | 1.38 (1.09, 1.75) | 9.10% | 1 | 1.24 |
| HOXB13 | NM_006361 | 1 | 0.00679441 | 1.59 (1.14, 2.21) | 8.90% | 1 | 1.25 |
| INSIG1 | NM_005542 | 1 | 0.007908076 | 1.75 (1.16, 2.64) | 9.70% | 1 | 1.24 |
| INSIG1 | NM_198337 | 1 | 0.007908076 | 1.75 (1.16, 2.64) | 9.70% | 1 | 1.24 |
| INSIG1 | NM_198336 | 1 | 0.007908076 | 1.75 (1.16, 2.64) | 9.70% | 1 | 1.24 |
| ITIH3 | NM_002217 | -1 | 0.008089965 | 0.58 (0.39, 0.87) | 9.80% | 1 | 1.24 |
| KCNK5 | NM_003740 | 1 | 0.006644671 | 1.52 (1.12, 2.05) | 8.80% | 1 | 1.25 |
| KIAA1609 | NM_020947 | 1 | 0.006816877 | 1.56 (1.13, 2.15) | 9.00% | 1 | 1.25 |
| KLF6 | NR_027653 | 1 | 0.007216712 | 1.54 (1.12, 2.10) | 9.20% | 1 | 1.25 |
| KLF6 | NM_001160124 | 1 | 0.007216712 | 1.54 (1.12, 2.10) | 9.20% | 1 | 1.25 |
| KLF6 | NM_001300 | 1 | 0.007216712 | 1.54 (1.12, 2.10) | 9.20% | 1 | 1.25 |
| KLF6 | NM_001160125 | 1 | 0.007216712 | 1.54 (1.12, 2.10) | 9.20% | 1 | 1.25 |
| LIX1 | NM_153234 | 1 | 0.007284268 | 1.70 (1.15, 2.50) | 9.30% | 1 | 1.25 |
| LOC145845 | NR_024264 | 1 | 0.007192082 | 1.42 (1.10, 1.83) | 9.20% | 1 | 1.24 |
| LPCAT1 | NM_024830 | 1 | 0.006993251 | 1.67 (1.15, 2.42) | 9.10% | 1 | 1.25 |
| MAGI2 | NM_012301 | -1 | 0.008130755 | 0.57 (0.38, 0.86) | 9.80% | 1 | 1.23 |
| MAP3K12 | NM_006301 | -1 | 0.007048049 | 0.62 (0.44, 0.88) | 9.10% | 1 | 1.25 |
| MAP3K12 | NM_001193511 | -1 | 0.007048049 | 0.62 (0.44, 0.88) | 9.10% | 1 | 1.25 |
| MFGE8 | NM_001114614 | 1 | 0.006919473 | 1.56 (1.13, 2.16) | 9.10% | 1 | 1.25 |
| MFGE8 | NM_005928 | 1 | 0.006919473 | 1.56 (1.13, 2.16) | 9.10% | 1 | 1.23 |
| MINK1 | NM_015716 | -1 | 0.007173123 | 0.54 (0.35, 0.85) | 9.20% | 1 | 1.23 |
| MINK1 | NM_153827 | -1 | 0.007173123 | 0.54 (0.35, 0.85) | 9.20% | 1 | 1.23 |
| MINK1 | NM_170663 | -1 | 0.007173123 | 0.54 (0.35, 0.85) | 9.20% | 1 | 1.23 |
| MINK1 | NM_001024937 | -1 | 0.007173123 | 0.54 (0.35, 0.85) | 9.20% | 1 | 1.23 |
| MIR195 | NR_029712 | 1 | 0.00787247 | 1.37 (1.09, 1.74) | 9.70% | 1 | 1.25 |
| MIR3183 | NR_036148 | 1 | 0.006747778 | 1.68 (1.15, 2.44) | 8.90% | 1 | 1.25 |
| MIR4318 | NR_036202 | 1 | 0.007957654 | 1.61 (1.13, 2.29) | 9.70% | 1 | 1.24 |
| MTA1 | NM_004689 | -1 | 0.007359437 | 0.59 (0.40, 0.87) | 9.30% | 1 | 1.25 |
| NOL11 | NM_015462 | 1 | 0.006772141 | 1.56 (1.13, 2.14) | 8.90% | 1 | 1.23 |
| NR1I2 | NM_022002 | -1 | 0.007225406 | 0.55 (0.36, 0.85) | 9.20% | 1 | 1.23 |
| NR1I2 | NM_003889 | -1 | 0.007225406 | 0.55 (0.36, 0.85) | 9.20% | 1 | 1.23 |
| NR1I2 | NM_0333013 | -1 | 0.007225406 | 0.55 (0.36, 0.85) | 9.20% | 1 | 1.24 |
| NXF1 | NM_001081491 | -1 | 0.00708175 | 0.60 (0.41, 0.87) | 9.10% | 1 | 1.24 |
| NXF1 | NM_006362 | -1 | 0.00708175 | 0.60 (0.41, 0.87) | 9.10% | 1 | 1.25 |
| OGDH | NM_002541 | 1 | 0.007291529 | 1.46 (1.11, 1.93) | 9.30% | 1 | 1.25 |
| OGDH | NM_001003941 | 1 | 0.007291529 | 1.46 (1.11, 1.93) | 9.30% | 1 | 1.25 |
| OGDH | NM_001165036 | 1 | 0.007291529 | 1.46 (1.11, 1.93) | 9.30% | 1 | 1.24 |
| OPLAH | NM_017570 | 1 | 0.007363069 | 1.73 (1.16, 2.59) | 9.30% | 1 | 1.25 |
| OR52E4 | NM_001005165 | 1 | 0.007563025 | 1.58 (1.13, 2.22) | 9.40% | 1 | 1.24 |
| PA2G4P4 | NR_003284 | -1 | 0.007765185 | 0.66 (0.48, 0.90) | 9.60% | 1 | 1.24 |
| PALLD | NM_001166108 | -1 | 0.007412269 | 0.62 (0.44, 0.88) | 9.30% | 1 | 1.24 |
| PALLD | NM_001166109 | -1 | 0.007412269 | 0.62 (0.44, 0.88) | 9.30% | 1 | 1.24 |
| PALLD | NM_001166110 | -1 | 0.007412269 | 0.62 (0.44, 0.88) | 9.30% | 1 | 1.24 |
| PALLD | NM_016081 | -1 | 0.007412269 | 0.62 (0.44, 0.88) | 9.30% | 1 | 1.25 |
| PARP11 | NM 020367 | -1 | 0.006969466 | 0.62 (0.44, 0.88) | 9.10% | 1 | 1.24 |
| PFN4 | NM_199346 | -1 | 0.007867198 | 0.63 (0.44, 0.88) | 9.70% | 1 | 1.24 |
| PGK1 | NM_000291 | 1 | 0.007371341 | 1.70 (1.15, 2.50) | 9.30% | 1 | 1.24 |
| PIK3C3 | NM_002647 | -1 | 0.007928522 | 0.63 (0.45, 0.89) | 9.70% | 1 | 1.24 |
| PLA2G16 | NM_001128203 | -1 | 0.007338932 | 0.59 (0.40, 0.87) | 9.30% | 1 | 1.24 |
| PLA2G16 | NM_007069 | -1 | 0.007338932 | 0.59 (0.40, 0.87) | 9.30% | 1 | 1.24 |
| POM121L10P | NR_024593 | 1 | 0.008015909 | 1.47 (1.11, 1.96) | 9.70% | 1 | 1.24 |
| PPIB | NM_000942 | 1 | 0.008136977 | 1.60 (1.13, 2.27) | 9.80% | 1 | 1.25 |
| PPIG | NM_004792 | -1 | 0.007743418 | 0.67 (0.50, 0.90) | 9.60% | 1 | 1.24 |
| PRAME | NM_206954 | 1 | 0.00807234 | 1.60 (1.13, 2.28) | 9.80% | 1 | 1.25 |
| PRAME | NM_206953 | 1 | 0.00807234 | 1.60 (1.13, 2.28) | 9.80% | 1 | 1.25 |
| PRAME | NM_006115 | 1 | 0.00807234 | 1.60 (1.13, 2.28) | 9.80% | 1 | 1.25 |
| PRAME | NM_206956 | 1 | 0.00807234 | 1.60 (1.13, 2.28) | 9.80% | 1 | 1.25 |
| PRAME | NM_206955 | 1 | 0.00807234 | 1.60 (1.13, 2.28) | 9.80% | 1 | 1.25 |
| PTPRT | NM_007050 | -1 | 0.007844377 | 0.64 (0.46, 0.89) | 9.70% | 1 | 1.24 |
| PTPRT | NM_133170 | -1 | 0.007844377 | 0.64 (0.46, 0.89) | 9.70% | 1 | 1.24 |
| PVR | NM_006505 | 1 | 0.007178986 | 1.73 (1.16, 2.57) | 9.20% | 1 | 1.24 |
| PVR | NM_001135768 | 1 | 0.007178986 | 1.73 (1.16, 2.57) | 9.20% | 1 | 1.24 |
| PVR | NM_001135769 | 1 | 0.007178986 | 1.73 (1.16, 2.57) | 9.20% | 1 | 1.24 |
| PVR | NM_001135770 | 1 | 0.007178986 | 1.73 (1.16, 2.57) | 9.20% | 1 | 1.24 |
| RBBP6 | NM_006910 | -1 | 0.008045393 | 0.59 (0.40, 0.87) | 9.80% | 1 | 1.24 |
| RBBP6 | NM_032626 | -1 | 0.008045393 | 0.59 (0.40, 0.87) | 9.80% | 1 | 1.24 |
| RBBP6 | NM_018703 | -1 | 0.008045393 | 0.59 (0.40, 0.87) | 9.80% | 1 | 1.24 |
| RBM38 | NM_017495 | 1 | 0.007553277 | 1.79 (1.17, 2.73) | 9.40% | 1 | 1.24 |
| RBM38 | NM_183425 | 1 | 0.007553277 | 1.79 (1.17, 2.73) | 9.40% | 1 | 1.24 |
| RILPL1 | NM_178314 | -1 | 0.007013837 | 0.61 (0.43, 0.88) | 9.10% | 1 | 1.25 |
| RNFT1 | NM_016125 | 1 | 0.007629214 | 1.78 (1.17, 2.73) | 9.50% | 1 | 1.24 |
| RPAP2 | NM_024813 | -1 | 0.007531085 | 0.57 (0.38, 0.86) | 9.40% | 1 | 1.24 |
| RPS6KB1 | NM_003161 | 1 | 0.008065183 | 1.43 (1.10, 1.87) | 9.80% | 1 | 1.24 |
| SAMD15 | NM_001010860 | -1 | 0.007388814 | 0.67 (0.50, 0.90) | 9.30% | 1 | 1.24 |
| SCARNA23 | NR_003007 | 1 | 0.007993669 | 1.51 (1.11, 2.04) | 9.70% | 1 | 1.25 |
| SERPINB10 | NM_005024 | 1 | 0.006313415 | 1.26 (1.07, 1.49) | 8.60% | 1 | 1.2 |
| SERPINB8 | NM_002640 | -1 | 0.007336561 | 0.64 (0.46, 0.89) | 9.30% | 1 | 1.25 |
| SERPINB8 | NM_001031848 | -1 | 0.007336561 | 0.64 (0.46, 0.89) | 9.30% | 1 | 1.25 |
| SERPINB8 | NM_198833 | -1 | 0.007336561 | 0.64 (0.46, 0.89) | 9.30% | 1 | 1.25 |
| SHISA3 | NM_001080505 | 1 | 0.007387467 | 1.38 (1.09, 1.75) | 9.30% | 1 | 1.24 |
| SLC25A13 | NR_027662 | 1 | 0.007684969 | 1.69 (1.15, 2.49) | 9.50% | 1 | 1.24 |
| SLC25A13 | NM_014251 | 1 | 0.007684969 | 1.69 (1.15, 2.49) | 9.50% | 1 | 1.24 |
| SLC25A13 | NM_001160210 | 1 | 0.007684969 | 1.69 (1.15, 2.49) | 9.50% | 1 | 1.24 |
| SLC35C2 | NM_173073 | 1 | 0.006914089 | 1.48 (1.11, 1.97) | 9.10% | 1 | 1.25 |
| SLC35C2 | NM_015945 | 1 | 0.006914089 | 1.48 (1.11, 1.97) | 9.10% | 1 | 1.25 |
| SLC35C2 | NM_173179 | 1 | 0.006914089 | 1.48 (1.11, 1.97) | 9.10% | 1 | 1.25 |
| SLC37A2 | NM_001145290 | -1 | 0.007397611 | 0.61 (0.42, 0.88) | 9.30% | 1 | 1.24 |
| SLC37A2 | NM_198277 | -1 | 0.007397611 | 0.61 (0.42, 0.88) | 9.30% | 1 | 1.24 |
| SPAG7 | NM_004890 | -1 | 0.007433804 | 0.66 (0.49, 0.89) | 9.30% | 1 | 1.24 |
| SQLE | NM_003129 | 1 | 0.008169599 | 1.61 (1.13, 2.29) | 9.80% | 1 | 1.24 |
| SRR | NM_021947 | -1 | 0.007802797 | 0.68 (0.51, 0.90) | 9.60% | 1 | 1.24 |
| STAU1 | NM_004602 | 1 | 0.007966457 | 1.46 (1.10, 1.93) | 9.70% | 1 | 1.24 |
| STAU1 | NM_017454 | 1 | 0.007966457 | 1.46 (1.10, 1.93) | 9.70% | 1 | 1.24 |
| STAU1 | NM_001037328 | 1 | 0.007966457 | 1.46 (1.10, 1.93) | 9.70% | 1 | 1.24 |
| STAU1 | NM_017452 | 1 | 0.007966457 | 1.46 (1.10, 1.93) | 9.70% | 1 | 1.24 |
| STAU1 | NM_017453 | 1 | 0.007966457 | 1.46 (1.10, 1.93) | 9.70% | 1 | 1.24 |
| SURF4 | NM_033161 | 1 | 0.00710138 | 1.61 (1.14, 2.28) | 9.10% | 1 | 1.25 |
| TBC1D16 | NM_019020 | -1 | 0.007903348 | 0.57 (0.38, 0.86) | 9.70% | 1 | 1.23 |
| TCP11L2 | NM_152772 | -1 | 0.006709472 | 0.63 (0.46, 0.88) | 8.90% | 1 | 1.25 |
| THEG | NM_016585 | 1 | 0.007652171 | 1.50 (1.11, 2.03) | 9.50% | 1 | 1.25 |
| THEG | NM_199202 | 1 | 0.007652171 | 1.50 (1.11, 2.03) | 9.50% | 1 | 1.25 |
| TMED11P | NR_033768 | -1 | 0.008112229 | 0.60 (0.41, 0.88) | 9.80% | 1 | 1.24 |
| TMEM189 | NM_001162505 | 1 | 0.007119372 | 1.79 (1.17, 2.74) | 9.20% | 1 | 1.24 |
| TMEM189 | NR_027889 | 1 | 0.007119372 | 1.79 (1.17, 2.74) | 9.20% | 1 | 1.24 |
| TMEM189 | NM_199129 | 1 | 0.007119372 | 1.79 (1.17, 2.74) | 9.20% | 1 | 1.24 |
| TNFSF12 | NR_037146 | -1 | 0.006806283 | 0.65 (0.48, 0.89) | 8.90% | 1 | 1.25 |
| TNFSF12 | NM_003809 | -1 | 0.006806283 | 0.65 (0.48, 0.89) | 8.90% | 1 | 1.25 |
| TNK1 | NM_003985 | -1 | 0.007866596 | 0.63 (0.45, 0.89) | 9.70% | 1 | 1.24 |
| TNS1 | NM_022648 | -1 | 0.008198294 | 0.60 (0.41, 0.88) | 9.80% | 1 | 1.24 |
| TRIM39 | NM_021253 | -1 | 0.006654211 | 0.67 (0.50, 0.89) | 8.80% | 1 | 1.25 |
| TRIM39 | NM_172016 | -1 | 0.006654211 | 0.67 (0.50, 0.89) | 8.80% | 1 | 1.25 |
| TUBA1B | NM_006082 | 1 | 0.006986711 | 1.56 (1.13, 2.15) | 9.10% | 1 | 1.25 |
| USP51 | NM_201286 | -1 | 0.007929716 | 0.66 (0.49, 0.90) | 9.70% | 1 | 1.24 |
| WDR52 | NM_001164496 | -1 | 0.007394432 | 0.61 (0.42, 0.87) | 9.30% | 1 | 1.24 |
| WDR52 | NM_018338 | -1 | 0.007394432 | 0.61 (0.42, 0.87) | 9.30% | 1 | 1.24 |
| WWP2 | NM_199424 | 1 | 0.006541054 | 1.34 (1.09,1.66) | 8.70% | 1 | 1.23 |
| WWP2 | NM_007014 | 1 | 0.006541054 | 1.34 (1.09, 1.66) | 8.70% | 1 | 1.23 |
| WWP2 | NM_199423 | 1 | 0.006541054 | 1.34 (1.09, 1.66) | 8.70% | 1 | 1.23 |
| XPO1 | NM_003400 | 1 | 0.006983767 | 1.63 (1.14, 2.34) | 9.10% | 1 | 1.25 |
| YES1 | NM_005433 | 1 | 0.007385459 | 1.78 (1.17, 2.70) | 9.30% | 1 | 1.24 |
| ZFAND1 | NM_001170797 | 1 | 0.007371855 | 1.59 (1.13, 2.23) | 9.30% | 1 | 1.25 |
| ZFAND1 | NM_001170796 | 1 | 0.007371855 | 1.59 (1.13, 2.23) | 9.30% | 1 | 1.25 |
| ZFAND1 | NR_033193 | 1 | 0.007371855 | 1.59 (1.13, 2.23) | 9.30% | 1 | 1.25 |
| ZFAND1 | NR_033194 | 1 | 0.007371855 | 1.59 (1.13, 2.23) | 9.30% | 1 | 1.25 |
| ZFAND1 | NM_024699 | 1 | 0.007371855 | 1.59 (1.13, 2.23) | 9.30% | 1 | 1.25 |
| ZFAND1 | NR_033195 | 1 | 0.007371855 | 1.59 (1.13, 2.23) | 9.30% | 1 | 1.25 |
| ZFAND1 | NR_033196 | 1 | 0.007371855 | 1.59 (1.13, 2.23) | 9.30% | 1 | 1.25 |
| ZMYND10 | NM_015896 | -1 | 0.008232538 | 0.61 (0.42, 0.88) | 9.90% | 1 | 1.24 |
| ZNF160 | NM_001102603 | -1 | 0.008143054 | 0.58 (0.39, 0.87) | 9.80% | 1 | 1.24 |
| ZNF160 | NM_198893 | -1 | 0.008143054 | 0.58 (0.39, 0.87) | 9.80% | 1 | 1.24 |
| ZNF160 | NM_033288 | -1 | 0.008143054 | 0.58 (0.39, 0.87) | 9.80% | 1 | 1.24 |
| ZNF175 | NM_007147 | -1 | 0.007576003 | 0.63 (0.44, 0.88) | 9.50% | 1 | 1.24 |
| ZNF187 | NM_001023560 | -1 | 0.006949723 | 0.67 (0.50, 0.90) | 9.10% | 1 | 1.25 |
| ZNF187 | NM_001111039 | -1 | 0.006949723 | 0.67 (0.50, 0.90) | 9.10% | 1 | 1.25 |
| ZNF187 | NM_152736 | -1 | 0.006949723 | 0.67 (0.50, 0.90) | 9.10% | 1 | 1.25 |
| ZNF486 | NM_052852 | -1 | 0.008185949 | 0.50 (0.30, 0.84) | 9.80% | 1 | 1.21 |
| ZNF782 | NM_001001662 | -1 | 0.007410404 | 0.50 (0.30, 0.83) | 9.30% | 1 | 1.22 |

| Table 2. List of Assembled RefSeq RNAs Associated with Risk of Breast Cancer Recurrence In 111 Patients Designated as ESR1 Positive | | | | | | | |
|---|---|---|---|---|---|---|---|
| Gene | Accession no. | Direction of Association (1=Higher Expression Means Higher Risk) | p-value | Standardized Hazard Ratio Estimate (95% Confidence Interval) | q-value (FDR) | Maximum Lower Bound Std HR @ 10% FDR | Regression-to-the-Mean-Corrected Std HR |
| C17orf97 | NM_001013672 | -1 | 1.28E-08 | 0.38 (6.27, 0.53) | <0.1% | 1.24 | 1.58 |
| ZFP3 | NM_153018 | -1 | 6.18E-08 | 0.31 (0.21, 0.48) | <0.1% | 1.24 | 1.52 |
| ULBP3 | NM_024518 | 1 | 1.95E-08 | 2.51 (1.82, 3.46) | <0.1% | 1.23 | 1.6 |
| GUCY2F | NM_001522 | 1 | 3.37E-11 | 1.68 (1.44, 1.96) | <0.1% | 1.2 | 1.53 |
| IFT74 | NM_001099222 | -1 | 2.40E-08 | 0.49 (0.38, 0.63) | <0.1% | 1.2 | 1.55 |
| IFT74 | NM_025103 | -1 | 2.40E-08 | 0.49 (0.38, 0.63) | <0.1% | 1.2 | 1.55 |
| IFT74 | NM_001099224 | -1 | 2.40E-08 | 0.49 (0.38, 0.63) | <0.1% | 1.2 | 1.55 |
| IFT74 | NM_001099223 | -1 | 2.40E-08 | 0.49 (0.38, 0.63) | <0.1% | 1.2 | 1.55 |
| MIR1208 | NR_031613 | 1 | 1.43E-08 | 1.95 (1.55, 2.46) | <0.1% | 1.2 | 1.56 |
| PADI3 | NM_016233 | 1 | 9.99E-09 | 1.93 (1.54, 2.42) | <0.1% | 1.2 | 1.56 |
| RPP21 | NM_001199120 | 1 | 3.14E-08 | 2.02 (1.58, 2.59) | <0.1% | 1.2 | 1.56 |
| RPP21 | NM_001199121 | 1 | 3.14E-08 | 2.02 (1.58, 2.59) | <0.1% | 1.2 | 1.56 |
| RPP21 | NM_024839 | 1 | 3.14E-08 | 2.02 (1.58, 2.59) | <0.1% | 1.2 | 1.56 |
| CWF19L2 | NM_152434 | -1 | 1.30E-06 | 0.37 (0.24, 0.55) | 0.10% | 1.17 | 1.46 |
| ZNF141 | NM_003441 | -1 | 6.43E-07 | 0.41 (0.29, 0.58) | <0.1% | 1.17 | 1.49 |
| RP1-177G6-2 | NR_028345 | 1 | 1.05E-09 | 1.55 (1.35, 1.79) | <0.1% | 1.16 | 1.45 |
| RP1-177G6-2 | NR_028344 | 1 | 1.05E-09 | 1.55 (1.35, 1.79) | <0.1% | 1.16 | 1.45 |
| APOL3 | NR_027834 | -1 | 5.44E-07 | 0.50 (0.38, 0.66) | <0.1% | 1.15 | 1.49 |
| APOL3 | NR_027833 | -1 | 5.44E-07 | 0.50 (0.38, 0.66) | <0.1% | 1.15 | 1.49 |
| APOL3 | NR_027835 | -1 | 5.44E-07 | 0.50 (0.38, 0.66) | <0.1% | 1.15 | 1.49 |
| APOL3 | NM_145642 | -1 | 5.44E-07 | 0.50 (0.38, 0.66) | <0.1% | 1.15 | 1.49 |
| APOL3 | NM_145641 | -1 | 5.44E-07 | 0.50 (0.38, 0.66) | <0.1% | 1.15 | 1.49 |
| APOL3 | NM_145640 | -1 | 5.44E-07 | 0.50 (0.38, 0.66) | <0.1% | 1.15 | 1.49 |
| CYB5B | NM_030579 | 1 | 6.41E-06 | 3.17 (1.92, 5.22) | 0.20% | 1.15 | 1.42 |
| DEGS1 | NM_003676 | 1 | 2.21E-07 | 1.76 (1.42, 2.18) | <0.1% | 1.15 | 1.49 |
| FLI14186 | NR_037596 | -1 | 1.79E-06 | 0.46 (0.34, 0.63) | 0.10% | 1.15 | 1.47 |
| HMX1 | NM_018942 | 1 | 2.17E-09 | 1.53 (1.33, 1.75) | <0.1% | 1.15 | 1.43 |
| KIAA0664L3 | NR_024034 | -1 | 2.18E-07 | 0.56 (0.45, 0.70) | <0.1% | 1.15 | 1.48 |
| KRT83 | NM_002282 | 1 | 2.64E-08 | 1.60 (1.35, 1.88) | <0.1% | 1.15 | 1.45 |
| ZNF326 | NM_182976 | -1 | 4.30E-06 | 0.41 (0.28, 0.60) | 0.20% | 1.15 | 1.44 |
| ZNF326 | NM_182975 | -1 | 4.30E-06 | 0.41 (0.28, 0.60) | 0.20% | 1.15 | 1.44 |
| ZNF486 | NM_052852 | -1 | 3.31E-06 | 0.35 (0.23, 0.55) | 0.10% | 1.15 | 1.43 |
| NAA35 | NM_024635 | -1 | 5.42E-06 | 0.43 (0.30, 0.62) | 0.20% | 1.14 | 1.44 |
| RBMXL1 | NM_001162536 | -1 | 3.46E-06 | 0.45 (0.32, 0.63) | 0.10% | 1.14 | 1.45 |
| RBMXL1 | NM_019610 | -1 | 3.46E-06 | 0.45 (0.32, 0.63) | 0.10% | 1.14 | 1.45 |
| TMEM81 | NM_203376 | 1 | 1.08E-05 | 3.15 (1.89, 5.26) | 0.30% | 1.14 | 1.4 |
| CENPN | NM_001100625 | 1 | 1.82E-05 | 3.08 (1.84, 5.15) | 0.40% | 1.13 | 1.39 |
| CENPN | NM_018455 | 1 | 1.82E-05 | 3.08 (1.84, 5.15) | 0.40% | 1.13 | 1.39 |
| CENPN | NM_001100624 | 1 | 1.82E-05 | 3.08 (1.84, 5.15) | 0.40% | 1.13 | 1.39 |
| FUBP1 | NM_003902 | -1 | 1.31E-05 | 0.36 (0.22, 0.57) | 0.30% | 1.13 | 1.38 |
| LOC642345 | NR_033829 | 1 | 2.97E-07 | 1.62 (1.35, 1.95) | <0.1% | 1.13 | 1.44 |
| ZZZ3 | NM_015534 | -1 | 1.63E-05 | 0.34 (0.21, 0.56) | 0.40% | 1.13 | 1.37 |
| CDK20 | NM_001170639 | -1 | 1.0423E-05 | 0.46 (0.33, 0.65) | 0.30% | 1.12 | 1.42 |
| CDK20 | NM_179432 | -1 | 1.0423E-05 | 0.46 (0.33, 0.65) | 0.30% | 1.12 | 1.42 |
| CDK20 | NM_001170640 | -1 | 1.0423E-05 | 0.46 (0.33, 0.65) | 0.30% | 1.12 | 1.42 |
| CDK20 | NM_001039803 | -1 | 1.0423E-05 | 0.46 (0.33, 0.65) | 0.30% | 1.12 | 1.42 |
| CDK20 | NM_012119 | -1 | 1.0423E-05 | 0.46 (0.33, 0.65) | 0.30% | 1.12 | 1.42 |
| DEFB133 | NM_001166478 | 1 | 1.74E-07 | 1.50 (1.29, 1.75) | <0.1% | 1.12 | 1.4 |
| GLMN | NM_053274 | -1 | 2.57E-05 | 0.36 (0.22, 0.58) | 0.60% | 1.12 | 1.36 |
| LOC100129636 | NM_0001195202 | 1 | 2.82E-06 | 1.83 (1.42, 2.36) | 0.10% | 1.12 | 1.46 |
| LPO | NM_001160102 | 1 | 6.17E-07 | 1.60 (1.33, 1.92) | <0.1% | 1.12 | 1.43 |
| LPO | NM_006151 | 1 | 6.17E-07 | 1.60 (1.33, 1.92) | <0.1% | 1.12 | 1.43 |
| LPO | NR_027647 | 1 | 6.17E-07 | 1.60 (1.33, 1.92) | <0.1% | 1.12 | 1.43 |
| OMA1 | NM_145243 | -1 | 4.89E-06 | 0.53 (0.40, 0.70) | 0.20% | 1.12 | 1.44 |
| PCDH11Y | NM_032972 | 1 | 1.89E-07 | 1.48 (1.28, 1.72) | <0.1% | 1.12 | 1.39 |
| PCDH11Y | NM_032973 | 1 | 1.89E-07 | 1.48 (1.28, 1.72) | <0.1% | 1.12 | 1.39 |
| PCDH11Y | NM_032971 | 1 | 1.89E-07 | 1.48 (1.28, 1.72) | <0.1% | 1.12 | 1.39 |
| PPP3CB | NM_021132 | 1 | 1.02E-06 | 1.70 (1.37, 2.10) | 0.10% | 1.12 | 1.45 |
| PPP3CB | NM_01142354 | 1 | 1.02E-06 | 1.70 (1.37, 2.10) | 0.10% | 1.12 | 1.45 |
| PPP3CB | NM_001142353 | 1 | 1.02E-06 | 1.70 (1.37, 2.10) | 0.10% | 1.12 | 1.45 |
| ATXN3L | NM_001135995 | 1 | 2.23E-07 | 1.40 (1.23, 1.60) | <0.1% | 1.11 | 1.34 |
| C3orf15 | NM_033364 | -1 | 2.52E-05 | 0.42 (0.28, 0.63) | 0.60% | 1.11 | 1.39 |
| C9orf27 | NR_024032 | 1 | 6.75E-09 | 1.34 (1.21, 1.47) | <0.1% | 1.11 | 1.31 |
| CCDC105 | NM_173482 | 1 | 1.01E-06 | 1.55 (1.30, 1.84) | 0.10% | 1.11 | 1.41 |
| FANK1 | NM_145235 | -1 | 1.13E-05 | 0.49 (0.35, 0.67) | 0.30% | 1.11 | 1.42 |
| GGCT | NR_037669 | 1 | 1.21E-05 | 1.86 (1.41, 2.45) | 0.30% | 1.11 | 1.44 |
| GGCT | NM_01199815 | 1 | 1.21E-05 | 1.86 (1.41, 2.45) | 0.30% | 1.11 | 1.44 |
| GGCT | NM_001199817 | 1 | 1.21E-05 | 1.86 (1.41, 2.45) | 0.30% | 1.11 | 1.44 |
| GGCT | NM_001199816 | 1 | 1.21E-05 | 1.86 (1.41, 2.45) | 0.30% | 1.11 | 1.44 |
| GGCT | NM_024051 | 1 | 1.21E-05 | 1.86 (1.41, 2.45) | 0.30% | 1.11 | 1.44 |
| GPAA1 | NM_003801 | 1 | 2.95E-05 | 2.28 (1.55, 3.35) | 0.60% | 1.11 | 1.41 |
| HCRTR2 | NM_001526 | 1 | 2.02E-07 | 1.41 (1.24, 1.60) | <0.1% | 1.11 | 1.35 |
| KLHDC8A | NM_018203 | -1 | 4.87E-05 | 0.36 (0.22, 0.59) | 0.80% | 1.11 | 1.35 |
| LOC339290 | NR_015389 | -1 | 4.68E-05 | 0.35 (0.21, 0.58) | 0.80% | 1.11 | 1.34 |
| LOC729966 | NR_036575 | 1 | 8.19E-08 | 1.40 (1.24, 1.59) | <0.1% | 1.11 | 1.35 |
| MARCH6 | NM_005885 | 1 | 2.93E-05 | 2.22 (1.53, 3.23) | 0.60% | 1.11 | 1.42 |
| MEX3B | NM_032246 | 1 | 2.55E-05 | 2.66 (1.69, 4.20) | 0.60% | 1.11 | 1.4 |
| NQO1 | NM_01025434 | 1 | 3.17E-05 | 2.58 (1.65, 4.04) | 0.60% | 1.11 | 1.4 |
| NQO1 | NM_000903 | 1 | 3.17E-05 | 2.58 (1.65, 4.04) | 0.60% | 1.11 | 1.4 |
| NQO1 | NM_001025433 | 1 | 3.17E-05 | 2.58 (1.65, 4.04) | 0.60% | 1.11 | 1.4 |
| ORM1 | NM_000607 | 1 | 1.22E-06 | 1.55 (1.30, 1.85) | 0.10% | 1.11 | 1.4 |
| RAET1L | NM_130900 | 1 | 2.80E-06 | 1.66 (1.34, 2.05) | 0.10% | 1.11 | 1.43 |
| SERPINB10 | NN_005024 | 1 | 1.11E-07 | 1.43 (1.25, 1.63) | <0.1% | 1.11 | 1.36 |
| TMOD3 | NM_014547 | 1 | 2.08E-08 | 1.39 (1.24, 1.55) | <0.1% | 1.11 | 1.34 |
| TSKU | NM_015516 | 1 | 6.77E-06 | 1.84 (1.41, 2.41) | 0.20% | 1.11 | 1.45 |
| CG030 | NR_026928 | -1 | 2.93E-05 | 0.49 (0.35, 0.68) | 0.60% | 1.1 | 1.4 |
| GAN | NM_022041 | 1 | 7.87E-05 | 3.09 (1.76, 5.40) | 1.10% | 1.1 | 1.34 |
| LRGUK | NM_144648 | -1 | 3.71E-05 | 0.45 (0.31, 0.66) | 0.70% | 1.1 | 1.39 |
| MAGEA10 | NM_021048 | 1 | 7.05E-07 | 1.45 (1.25, 1.67) | <0.1% | 1.1 | 1.36 |
| MAGEA10 | NM_01011543 | 1 | 7.05E-07 | 1.45 (1.25, 1.67) | <0.1% | 1.1 | 1.36 |
| NCRNA00200 | NR_015376 | 1 | 9.14E-07 | 1.44 (1.24, 1.66) | 0.10% | 1.1 | 1.36 |
| NCRNA00221 | NR_027457 | 1 | 1.13E-06 | 1.45 (1.25, 1.68) | 0.10% | 1.1 | 1.36 |
| PGA3 | NM_001079807 | 1 | 2.24E-06 | 1.47 (1.25, 1.73) | 0.10% | 1.1 | 1.37 |
| PYCRL | NM_023078 | 1 | 7.01E-05 | 2.88 (1.71, 4.86) | 1.10% | 1.1 | 1.35 |
| AZU1 | NM_001700 | 1 | 4.61E-05 | 1.90 (1.40, 2.60) | 0.80% | 1.09 | 1.41 |
| C21orf63 | NM_058187 | -1 | 4.43E-05 | 0.48 (0.34, 0.68) | 0.80% | 1.09 | 1.39 |
| C7orf30 | NM_138446 | 1 | 3.48E-05 | 1.94 (1.42, 2.64) | 0.70% | 1.09 | 1.41 |
| CHAT | NM_020986 | 1 | 5.09E-06 | 1.50 (1.26, 1.78) | 0.20% | 1.09 | 1.37 |
| CHAT | NM_001142934 | 1 | 5.09E-06 | 1.50 (1.26, 1.78) | 0.20% | 1.09 | 1.37 |
| CHAT | NM 020985 | 1 | 5.09E-06 | 1.50 (1.26,1.78) | 0.20% | 1.09 | 1.37 |
| CHAT | NM_001142933 | 1 | 5.09E-06 | 1.50 (1.26, 1.78) | 0.20% | 1.09 | 1.37 |
| CHAT | NM_020549 | 1 | 5.09E-06 | 1.50 (1.26, 1.78) | 0.20% | 1.09 | 1.37 |
| CHAT | NM_020984 | 1 | 5.09E-06 | 1.50 (1.26,1.78) | 0.20% | 1.09 | 1.37 |
| CHAT | NM_001142929 | 1 | 5.09E-06 | 1.50 (1.26, 1.78) | 0.20% | 1.09 | 1.37 |
| COX8C | NM_182971 | 1 | 8.22E-06 | 1.55 (1.28, 1.88) | 0.30% | 1.09 | 1.39 |
| GOLPH3 | NM_022130 | 1 | 9.17E-05 | 2.38 (1.54, 3.67) | 1.30% | 1.09 | 1.37 |
| GSTA2 | NM_000846 | 1 | 5.09E-06 | 1.50 (1.26, 1.78) | 0.20% | 1.09 | 1.37 |
| J AK1 | NM_002227 | -1 | 9.2776E-05 | 0.38 (0.23, 0.62) | 1.30% | 1.09 | 1.33 |
| LOC647107 | NR_033945 | 1 | 1.27E-05 | 1.64 (1.31, 2.04) | 0.30% | 1.09 | 1.4 |
| LRIT2 | NM_001017924 | 1 | 6.98E-07 | 1.38 (1.21, 1.56) | <0.1% | 1.09 | 1.32 |
| LRP12 | NM_001135703 | 1 | 6.92E-05 | 2.17 (1.48, 3.18) | 1.10% | 1.09 | 1.39 |
| LRP12 | NM_013437 | 1 | 6.92E-05 | 2.17 (1.48, 3.18) | 1.10% | 1.09 | 1.39 |
| MAPK6 | NM_002748 | 1 | 4.24E-07 | 1.37 (1.21, 1.55) | <0.1% | 1.09 | 1.32 |
| MIR1266 | NR_031670 | 1 | 1.70E-05 | 1.68 (1.33, 2.12) | 0.40% | 1.09 | 1.41 |
| MIR3117 | NR_036060 | 1 | 4.01E-05 | 2.00 (1.44, 2.78) | 0.70% | 1.09 | 1.41 |
| PAR-SN | NR_022011 | -1 | 1.63E-05 | 0.61 (0.48, 0.76) | 0.40% | 1.09 | 1.39 |
| SF3B3 | NM_012426 | 1 | 4.66E-05 | 2.11 (1.47, 3.02) | 0.80% | 1.09 | 1.41 |
| SLC35D2 | NM_007001 | -1 | 4.11E-05 | 0.54 (0.41, 0.73) | 0.70% | 1.09 | 1.39 |
| TFF2 | NM_005423 | 1 | 3.48E-06 | 1.42 (1.22, 1.65) | 0.10% | 1.09 | 1.34 |
| USO1 | NM_003715 | 1 | 1.60E-06 | 1.40 (1.22, 1.60) | 0.10% | 1.09 | 1.33 |
| WFDC9 | NM_147198 | 1 | 7.58E-05 | 2.21 (1.49, 3.28) | 1.10% | 1.09 | 1.39 |
| ACAP2 | NM_012287 | 1 | 6.50E-05 | 1.92 (1.39, 2.64) | 1.00% | 1.08 | 1.4 |
| ACSL5 | NM_016234 | -1 | 0.00011296 | 0.45 (0.30, 0.67) | 1.50% | 1.08 | 1.35 |
| ACSL5 | NM_203380 | -1 | 0.00011296 | 0.45 (0.30, 0.67) | 1.50% | 1.08 | 1.35 |
| ACSL5 | NM_203379 | -1 | 0.00011296 | 0.45 (0.30, 0.67) | 1.50% | 1.08 | 1.35 |
| APOBEC1 | NM_001644 | 1 | 1.27E-07 | 1.27 (1.16, 1.39) | <0.1% | 1.08 | 1.25 |
| CCNA2 | NM_001237 | 1 | 0.00013361 | 2.46 (1.55, 3.91) | 1.70% | 1.08 | 1.36 |
| DBNDD1 | NM_001042610 | 1 | 0.00014445 | 2.73 (1.63, 4.58) | 1.80% | 1.08 | 1.34 |
| DBNDD1 | NM_024043 | 1 | 0.00014445 | 2.73 (1.63, 4.58) | 1.80% | 1.08 | 1.34 |
| EVX2 | NM_001080458 | 1 | 1.16E-05 | 1.51 (1.25, 1.81) | 0.30% | 1.08 | 1.37 |
| FAM18A | NM_001079512 | -1 | 3.73E-05 | 0.59 (0.46, 0.76) | 0.70% | 1.08 | 1.38 |
| GRSF1 | NM_002092 | 1 | 1.87E-05 | 1.51 (1.25, 1.83) | 0.50% | 1.08 | 1.36 |
| GRSF1 | NM_001098477 | 1 | 1.87E-05 | 1.51 (1.25, 1.83) | 0.50% | 1.08 | 1.36 |
| LOC145845 | NR_024264 | 1 | 5.07E-05 | 1.79 (1.35, 2.37) | 0.80% | 1.08 | 1.4 |
| LOC645971 | NM_001195256 | 1 | 1.00E-04 | 2.15 (1.46, 3.16) | 1.40% | 1.08 | 1.38 |
| LRRC37A4 | NR_002940 | -1 | 2.18E-05 | 0.66 (0.54, 0.80) | 0.50% | 1.08 | 1.35 |
| MIR542 | NR_030399 | 1 | 3.03E-05 | 1.65 (1.31, 2.10) | 0.60% | 1.08 | 1.39 |
| OR14J1 | NM_030946 | 1 | 2.85E-05 | 1.65 (1.30, 2.08) | 0.60% | 1.08 | 1.39 |
| PKN2 | NM_06256 | -1 | 0.00014097 | 0.33 (0.18, 0.58) | 1.80% | 1.08 | 1.29 |
| PRPF38B | NR_037185 | -1 | 0.00016322 | 0.36 (0.22, 0.62) | 2.00% | 1.08 | 1.3 |
| PRPF38B | NM_18061 | -1 | 0.00016322 | 0.36 (0.22, 0.62) | 2.00% | 1.08 | 1.3 |
| RAB9BP1 | NR_00039 | 1 | 2.04E-05 | 1.59 (1.29, 1.97) | 0.50% | 1.08 | 1.39 |
| SERINC5 | NM_178276 | 1 | 3.54E-05 | 1.72 (1.33, 2.23) | 0.70% | 1.08 | 1.4 |
| SERINC5 | NM_001174071 | 1 | 3.54E-05 | 1.72 (1.33, 2.23) | 0.70% | 1.08 | 1.4 |
| SERINC5 | NM_001174072 | 1 | 3.54E-05 | 1.72 (1.33, 2.23) | 0.70% | 1.08 | 1.4 |
| TRPM8 | NM_024080 | 1 | 2.78E-05 | 1.56 (1.27, 1.92) | 0.60% | 1.08 | 1.37 |
| USPL1 | NM_005800 | -1 | 0.00016305 | 0.38 (0.23, 0.63) | 2.00% | 1.08 | 1.31 |
| ZNF227 | NM_182490 | -1 | 0.00013956 | 0.41 (0.26, 0.65) | 1.80% | 1.08 | 1.33 |
| ZNF512 | NM_032434 | -1 | 7.91E-05 | 0.46 (0.32, 0.68) | 1.10% | 1.08 | 1.36 |
| ZNF595 | NM_182524 | -1 | 3.08E-05 | 0.64 (0.51, 0.79) | 0.60% | 1.08 | 1.36 |
| AP1G1 | NM_001128 | 1 | 0.00015448 | 2.04 (1.41, 2.96) | 1.90% | 1.07 | 1.37 |
| AP1G1 | NM_001030007 | 1 | 0.00015448 | 2.04 (1.41, 2.96) | 1.90% | 1.07 | 1.37 |
| AP4E1 | NM_007347 | 1 | 1.22E-05 | 1.35 (1.18, 1.55) | 0.30% | 1.07 | 1.3 |
| C15orf34 | NR_027262 | -1 | 7.82E-05 | 0.59 (0.45, 0.76) | 1.10% | 1.07 | 1.37 |
| COL20A1 | NM_020882 | 1 | 2.83E-05 | 1.44 (1.21, 1.71) | 0.60% | 1.07 | 1.33 |
| COX7B2 | NM_130902 | 1 | 1.35E-06 | 1.25 (1.14, 1.38) | 0.10% | 1.07 | 1.23 |
| EVX1 | NM_001989 | 1 | 3.66E-05 | 1.58 (1.27, 1.95) | 0.70% | 1.07 | 1.37 |
| HLA-DOA | NM_002119 | -1 | 7.86E-05 | 0.55 (0.41, 0.74) | 1.10% | 1.07 | 1.37 |
| MMP15 | NM_002428 | 1 | 0.0001989 | 2.48 (1.54, 4.00) | 2.30% | 1.07 | 1.34 |
| PIBF1 | NM_006346 | -1 | 0.00012808 | 0.50 (0.35, 0.71) | 1.70% | 1.07 | 1.36 |
| SNCB | NM_001001502 | 1 | 2.13E-05 | 1.43 (1.21, 1.68) | 0.50% | 1.07 | 1.33 |
| SNCB | NM_003085 | 1 | 2.13E-05 | 1.43 (1.21, 1.68) | 0.50% | 1.07 | 1.33 |
| STX8 | NR_033656 | -1 | 0.00012845 | 0.52 (0.37, 0.73) | 1.70% | 1.07 | 1.36 |
| STX8 | NM_004853 | -1 | 0.00012845 | 0.52 (0.37, 0.73) | 1.70% | 1.07 | 1.36 |
| TMEM91 | NM_001098823 | -1 | 0.00013958 | 0.51 (0.36, 0.72) | 1.80% | 1.07 | 1.36 |
| TMEM91 | NM_001042595 | -1 | 0.00013958 | 0.51 (0.36, 0.72) | 1.80% | 1.07 | 1.36 |
| TMEM91 | NM_001098821 | -1 | 0.00013958 | 0.51 (0.36, 0.72) | 1.80% | 1.07 | 1.36 |
| TMEM91 | NM_001098822 | -1 | 0.00013958 | 0.51 (0.36, 0.72) | 1.80% | 1.07 | 1.36 |
| TMEM91 | NM_001098825 | -1 | 0.00013958 | 0.51 (0.36, 0.72) | 1.80% | 1.07 | 1.36 |
| TMEM91 | NM_001098824 | -1 | 0.00013958 | 0.51 (0.36, 0.72) | 1.80% | 1.07 | 1.36 |
| ZNF738 | NR_027130 | -1 | 0.00018472 | 0.44 (0.28, 0.67) | 2.20% | 1.07 | 1.33 |
| ANKZF1 | NM_018089 | -1 | 0.00013353 | 0.54 (0.39,0.74) | 1.70% | 1.06 | 1.36 |
| ANKZF1 | NM_001042410 | -1 | 0.00013353 | 0.54 (0.39, 0.74) | 1.70% | 1.06 | 1.36 |
| APCS | NM_001639 | 1 | 6.64E-06 | 1.27 (1.15, 1.41) | 0.20% | 1.06 | 1.24 |
| BAGE | NM_001187 | 1 | 4.69E-05 | 1.44 (1.21,1.72) | 0.80% | 1.06 | 1.33 |
| FAM21C | NM_015262 | -1 | 0.00021151 | 0.49 (0.33, 0.71) | 2.30% | 1.06 | 1.34 |
| FAM21C | NM_001169107 | -1 | 0.00021151 | 0.49 (0.33, 0.71) | 2.30% | 1.06 | 1.34 |
| FAM21C | NM_001169106 | -1 | 0.00021151 | 0.49 (0.33, 0.71) | 2.30% | 1.06 | 1.34 |
| GFOD2 | NM_030819 | 1 | 0.0002683 | 2.24 (1.45, 3.45) | 2.70% | 1.06 | 1.34 |
| GFOD2 | NR_027398 | 1 | 0.0002683 | 2.24 (1.45, 3.45) | 2.70% | 1.06 | 1.34 |
| GFOD2 | NR_027399 | 1 | 0.0002683 | 2.24 (1.45, 3.45) | 2.70% | 1.06 | 1.34 |
| GPX5 | NM_003996 | 1 | 7.14E-05 | 1.58 (1.26, 1.98) | 1.10% | 1.06 | 1.36 |
| GPX5 | NM_001509 | 1 | 7.14E-05 | 1.58 (1.26, 1.98) | 1.10% | 1.06 | 1.36 |
| IRAK4 | NM_001145256 | -1 | 0.00018709 | 0.47 (0.32, 0.70) | 2.20% | 1.06 | 1.34 |
| IRAK4 | NM_01114182 | -1 | 0.00018709 | 0.47 (0.32, 0.70) | 2.20% | 1.06 | 1.34 |
| IRAK4 | NM_001145258 | -1 | 0.00018709 | 0.47 (0.32, 0.70) | 2.20% | 1.06 | 1.34 |
| IRAK4 | NM_001145257 | -1 | 0.00018709 | 0.47 (0.32, 0.70) | 2.20% | 1.06 | 1.34 |
| IRAK4 | NM_016123 | -1 | 0.00018709 | 0.47 (0.32, 0.70) | 2.20% | 1.06 | 1.34 |
| KIAA1324 | NM_020775 | -1 | 0.00020766 | 0.46 (0.31, 0.69) | 2.30% | 1.06 | 1.33 |
| LOC286186 | NR_033893 | 1 | 6.18E-05 | 1.50 (1.23, 1.83) | 1.00% | 1.06 | 1.35 |
| LOC401177 | NR_033975 | 1 | 9.63E-06 | 1.29 (1.15, 1.45) | 0.30% | 1.06 | 1.26 |
| LRRC46 | NM_033413 | -1 | 0.00020068 | 0.48 (0.33, 0.71) | 2.30% | 1.06 | 1.34 |
| MIR1251 | NR_031653 | 1 | 0.00019134 | 1.89 (1.35, 2.65) | 2.20% | 1.06 | 1.37 |
| MIR195 | NR_029712 | 1 | 9.96E-05 | 1.56 (1.25, 1.96) | 1.40% | 1.06 | 1.36 |
| MIR4275 | NR_036237 | 1 | 6.85E-05 | 1.53 (1.24, 1.89) | 1.10% | 1.06 | 1.35 |
| NKX1-2 | NM_001146340 | 1 | 9.19E-05 | 1.58 (1.26, 1.99) | 1.30% | 1.06 | 1.36 |
| OR2W5 | NM_001004698 | 1 | 3.95E-06 | 1.25 (1.14, 1.38) | 0.10% | 1.06 | 1.23 |
| SCARB2 | NM_005506 | 1 | 0.00010943 | 1.71 (1.30, 2.24) | 1.50% | 1.06 | 1.38 |
| SNAR-G1 | NR_004383 | 1 | 7.37E-05 | 1.47 (1.21, 1.77) | 1.10% | 1.06 | 1.33 |
| TKT | NM_001135055 | 1 | 0.00011508 | 1.77 (1.32, 2.37) | 1.60% | 1.06 | 1.38 |
| TKT | NM_001064 | 1 | 0.00011508 | 1.77 (1.32, 2.37) | 1.60% | 1.06 | 1.38 |
| TMOD2 | NM_014548 | 1 | 6.75E-05 | 1.50 (1.23, 1.82) | 1.10% | 1.06 | 1.34 |
| TMOD2 | NM_001142885 | 1 | 6.75E-05 | 1.50 (1.23, 1.82) | 1.10% | 1.06 | 1.34 |
| USP33 | NM_201626 | -1 | 0.00030132 | 0.36 (0.21, 0.63) | 3.00% | 1.06 | 1.28 |
| USP33 | NM_015017 | -1 | 0.00030132 | 0.36 (0.21, 0.63) | 3.00% | 1.06 | 1.28 |
| USP33 | NM_201624 | -1 | 0.00030132 | 0.36 (0.21, 0.63) | 3.00% | 1.06 | 1.28 |
| VDAC1 | NM_003374 | 1 | 0.00026933 | 2.74 (1.59, 4.72) | 2.70% | 1.06 | 1.31 |
| VDAC1 | NR_036625 | 1 | 0.00026933 | 2.74 (1.59, 4.72) | 2.70% | 1.06 | 1.31 |
| VDAC1 | NR_036624 | 1 | 0.00026933 | 2.74 (1.59, 4.72) | 2.70% | 1.06 | 1.31 |
| C12orf35 | NM_018169 | -1 | 0.00036212 | 0.34 (0.19, 0.62) | 3.40% | 1.05 | 1.27 |
| GALNS | NM_000512 | 1 | 0.00030441 | 2.31 (1.47, 3.65) | 3.00% | 1.05 | 1.34 |
| KRT78 | NM_173352 | 1 | 5.86E-05 | 1.41 (1.19, 1.66) | 1.00% | 1.05 | 1.31 |
| LSM4 | NM_012321 | 1 | 0.00018189 | 1.79 (1.32, 2.43) | 2.20% | 1.05 | 1.37 |
| MIR4318 | NR_036202 | 1 | 0.00020629 | 1.76 (1.30, 2.37) | 2.30% | 1.05 | 1.36 |
| OR5T2 | NM_001004746 | 1 | 0.00020529 | 1.85 (1.34, 2.57) | 2.30% | 1.05 | 1.37 |
| PNPLA4 | NM_004650 | -1 | 0.00020112 | 0.57 (0.43, 0.77) | 2.30% | 1.05 | 1.35 |
| PNPLA4 | NM_001142389 | -1 | 0.00020112 | 0.57 (0.43, 0.77) | 2.30% | 1.05 | 1.35 |
| PNPLA4 | NM_001172672 | -1 | 0.00020112 | 0.57 (0.43, 0.77) | 2.30% | 1.05 | 1.35 |
| SNORD116-12 | NR_003327 | 1 | 0.00013681 | 1.49 (1.21, 1.82) | 1.80% | 1.05 | 1.33 |
| TFAP2A | NM_003220 | -1 | 0.00019691 | 0.60 (0.45, 0.78) | 2.30% | 1.05 | 1.34 |
| TFAP2A | NM_001032280 | -1 | 0.00019691 | 0.60 (0.45, 0.78) | 2.30% | 1.05 | 1.34 |
| TFAP2A | NM_001042425 | -1 | 0.00019691 | 0.60 (0.45, 0.78) | 2.30% | 1.05 | 1.34 |
| TTC9 | NM_015351 | 1 | 0.00025891 | 1.89 (1.34, 2.67) | 2.60% | 1.05 | 1.36 |
| C17orf88 | NR_026770 | 1 | 0.00022622 | 1.66 (1.27, 2.17) | 2.40% | 1.04 | 1.36 |
| C19orf75 | NM_173635 | 1 | 3.05E-05 | 1.26 (1.13, 1.40) | 0.60% | 1.04 | 1.23 |
| DUSP13 | NM_001007273 | 1 | 0.00031933 | 1.95 (1.35, 2.80) | 3.10% | 1.04 | 1.35 |
| DUSP13 | NM_001007271 | 1 | 0.00031933 | 1.95 (1.35, 2.80) | 3.10% | 1.04 | 1.35 |
| DUSP13 | NM_001007272 | 1 | 0.00031933 | 1.95 (1.35, 2.80) | 3.10% | 1.04 | 1.35 |
| DUSP13 | NM_016364 | 1 | 0.00031933 | 1.95 (1.35, 2.80) | 3.10% | 1.04 | 1.35 |
| G3BP2 | NM_203504 | 1 | 0.00018881 | 1.58 (1.24, 2.02) | 2.20% | 1.04 | 1.35 |
| G3BP2 | NM_203505 | 1 | 0.00018881 | 1.58 (1.24, 2.02) | 2.20% | 1.04 | 1.35 |
| G3BP2 | NM_012297 | 1 | 0.00018881 | 1.58 (1.24, 2.02) | 2.20% | 1.04 | 1.35 |
| HSPBAP1 | NM_024610 | -1 | 0.00032784 | 0.48 (0.32, 0.72) | 3.10% | 1.04 | 1.32 |
| LRRIQ1 | NM_001079910 | -1 | 0.00037432 | 0.50 (0.34, 0.73) | 3.50% | 1.04 | 1.32 |
| LRRIQ1 | NM_032165 | -1 | 0.00037432 | 0.50 (0.34, 0.73) | 3.50% | 1.04 | 1.32 |
| LYPD2 | NM_205545 | 1 | 4.58E-05 | 1.27 (1.13, 1.43) | 0.80% | 1.04 | 1.24 |
| MAGEB10 | NM_182506 | 1 | 0.00022296 | 1.50 (1.21, 1.86) | 2.40% | 1.04 | 1.33 |
| MGC87042 | NM_207342 | 1 | 0.00026627 | 1.56 (1.23, 1.99) | 2.70% | 1.04 | 1.34 |
| MGC87042 | NM_001164460 | 1 | 0.00026627 | 1.56 (1.23, 1.99) | 2.70% | 1.04 | 1.34 |
| MOBKL1A | NM_173468 | 1 | 0.00023167 | 1.52 (1.22, 1.91) | 2.40% | 1.04 | 1.33 |
| PILRA | NM_013439 | -1 | 0.00022195 | 0.63 (0.50, 0.81) | 2.40% | 1.04 | 1.33 |
| PILRA | NM_178273 | -1 | 0.00022195 | 0.63 (0.50, 0.81) | 2.40% | 1.04 | 1.33 |
| PILRA | NM_178272 | -1 | 0.00022195 | 0.63 (0.50, 0.81) | 2.40% | 1.04 | 1.33 |
| POGK | NM_017542 | 1 | 0.00029434 | 1.82 (1.32, 2.51) | 2.90% | 1.04 | 1.36 |
| PRSS41 | NM_001135086 | 1 | 3.53E-05 | 1.25 (1.12, 1.39) | 0.70% | 1.04 | 1.23 |
| SOST | NM_025237 | 1 | 0.00021545 | 1.59 (1.24, 2.03) | 2.30% | 1.04 | 1.35 |
| ZNF140 | NM_003440 | -1 | 0.00023704 | 0.64 (0.51, 0.81) | 2.50% | 1.04 | 1.33 |
| ZNF682 | NM_001077349 | -1 | 0.00029535 | 0.53 (0.37, 0.75) | 2.90% | 1.04 | 1.33 |
| ZNF682 | NM_033196 | -1 | 0.00029535 | 0.53 (0.37, 0.75) | 2.90% | 1.04 | 1.33 |
| AAGAB | NM_024666 | 1 | 0.0003973 | 1.50 (1.20, 1.89) | 3.70% | 1.03 | 1.32 |
| AARS | NM_001605 | 1 | 0.00051635 | 2.05 (1.37, 3.08) | 4.40% | 1.03 | 1.33 |
| ADHFE1 | NM_144650 | -1 | 0.00059544 | 0.53 (0.37, 0.76) | 4.80% | 1.03 | 1.31 |
| ARPP19 | NM_006628 | 1 | 0.00025921 | 1.32 (1.14, 1.53) | 2.60% | 1.03 | 1.26 |
| BZRAP1 | NM_004758 | -1 | 0.00068405 | 0.50 (0.34, 0.75) | 5.30% | 1.03 | 1.3 |
| BZRAP1 | NM_024418 | -1 | 0.00068405 | 0.50 (0.34, 0.75) | 5.30% | 1.03 | 1.3 |
| C14orf102 | NM_199043 | -1 | 0.00056259 | 0.50 (0.34, 0.74) | 4.70% | 1.03 | 1.31 |
| C14orf102 | NM_017970 | -1 | 0.00056259 | 0.50 (0.34, 0.74) | 4.70% | 1.03 | 1.31 |
| C17orf68 | NM_025099 | -1 | 0.00057208 | 0.38 (0.22, 0.66) | 4.70% | 1.03 | 1.27 |
| CLUL1 | NM_014410 | -1 | 0.00044779 | 0.57 (0.42, 0.78) | 4.00% | 1.03 | 1.32 |
| CLUL1 | NM_199167 | -1 | 0.00044779 | 0.57 (0.42, 0.78) | 4.00% | 1.03 | 1.32 |
| DEF8 | NM_207514 | 1 | 0.00060461 | 3.04 (1.61, 5.73) | 4.90% | 1.03 | 1.27 |
| DEF8 | NM_017702 | 1 | 0.00060461 | 3.04 (1.61, 5.73) | 4.90% | 1.03 | 1.27 |
| DKFZp779M06 52 | NR_027134 | 1 | 0.00043937 | 1.53 (1.21, 1.95) | 4.00% | 1.03 | 1.32 |
| DKK4 | NM_014420 | -1 | 0.00068191 | 0.43 (0.27, 0.70) | 5.30% | 1.03 | 1.28 |
| DSCR10 | NR_027695 | 1 | 0.00037115 | 1.40 (1.16, 1.68) | 3.50% | 1.03 | 1.29 |
| FAM169B | NM_182562 | 1 | 0.00023936 | 1.46 (1.19, 1.78) | 2.50% | 1.03 | 1.32 |
| GARS | NM_002047 | 1 | 0.0002165 | 1.39 (1.17, 1.65) | 2.30% | 1.03 | 1.29 |
| HIST2H2AC | NM_003517 | 1 | 0.00055643 | 2.72 (1.54, 4.79) | 4.70% | 1.03 | 1.29 |
| HMX3 | NM_001105574 | 1 | 0.00032223 | 1.63 (1.25, 2.13) | 3.10% | 1.03 | 1.34 |
| HOXA13 | NM_000522 | 1 | 0.00047312 | 1.47 (1.18, 1.82) | 4.20% | 1.03 | 1.31 |
| IDI1 | NM_004508 | 1 | 0.00059295 | 2.54 (1.49, 4.33) | 4.80% | 1.03 | 1.3 |
| JUND | NM_005354 | 1 | 0.00044453 | 1.44 (1.18, 1.77) | 4.00% | 1.03 | 1.3 |
| LOC285577 | NR_033898 | 1 | 0.00019503 | 1.30 (1.13, 1.50) | 2.20% | 1.03 | 1.25 |
| LOC648740 | NR_024438 | -1 | 0.00063182 | 0.54 (0.38, 0.77) | 5.00% | 1.03 | 1.31 |
| MCMBP | NM_024834 | 1 | 0.00056948 | 1.77 (1.28, 2.46) | 4.70% | 1.03 | 1.34 |
| MIR133A1 | NR_029675 | 1 | 0.00035238 | 1.67 (1.26, 2.22) | 3.30% | 1.03 | 1.35 |
| MMP25 | NM_022468 | -1 | 0.00050839 | 0.45 (0.28, 0.70) | 4.40% | 1.03 | 1.3 |
| NPPB | NM_002521 | 1 | 0.00015182 | 1.29 (1.13, 1.47) | 1.90% | 1.03 | 1.25 |
| NTRK3 | NM_001012338 | -1 | 0.00048234 | 0.50 (0.34, 0.74) | 4.20% | 1.03 | 1.31 |
| NTRK3 | NM_002530 | -1 | 0.00048234 | 0.50 (0.34, 0.74) | 4.20% | 1.03 | 1.31 |
| NTRK3 | NM_001007156 | -1 | 0.00048234 | 0.50 (0.34, 0.74) | 4.20% | 1.03 | 1.31 |
| ODF2L | NM_001007022 | -1 | 0.00054085 | 0.50 (0.34, 0.74) | 4.60% | 1.03 | 1.31 |
| ODF2L | NM_020729 | -1 | 0.00054085 | 0.50 (0.34, 0.74) | 4.60% | 1.03 | 1.31 |
| ODF2L | NM_001184765 | -1 | 0.00054085 | 0.50 (0.34, 0.74) | 4.60% | 1.03 | 1.31 |
| ODF2L | NM_001184766 | -1 | 0.00054085 | 0.50 (0.34, 0.74) | 4.60% | 1.03 | 1.31 |
| OR2B3 | NM_001005226 | 1 | 0.00055355 | 1.60 (1.23, 2.09) | 4.70% | 1.03 | 1.33 |
| OR8S1 | NM_001005203 | 1 | 0.00014925 | 1.33 (1.15, 1.54) | 1.90% | 1.03 | 1.27 |
| PGM5P2 | NR_002836 | -1 | 0.00047907 | 0.48 (0.32, 0.72) | 4.20% | 1.03 | 1.31 |
| RAD51L3 | NM_133629 | 1 | 0.00053965 | 1.68 (1.25, 2.25) | 4.60% | 1.03 | 1.34 |
| RAD51L3 | NM_001142571 | 1 | 0.00053965 | 1.68 (1.25, 2.25) | 4.60% | 1.03 | 1.34 |
| RAD51L3 | NM_002878 | 1 | 0.00053965 | 1.68 (1.25, 2.25) | 4.60% | 1.03 | 1.34 |
| RBM27 | NM_018989 | 1 | 0.00056605 | 1.93 (1.33, 2.80) | 4.70% | 1.03 | 1.33 |
| RILPL2 | NM_145058 | -1 | 0.00047818 | 0.61 (0.46, 0.80) | 4.20% | 1.03 | 1.32 |
| RNASE9 | NM_001001673 | 1 | 0.00047969 | 2.10 (1.39, 3.20) | 4.20% | 1.03 | 1.33 |
| RNASE9 | NM_001110358 | 1 | 0.00047969 | 2.10 (1.39, 3.20) | 4.20% | 1.03 | 1.33 |
| RNASE9 | NM_001110359 | 1 | 0.00047969 | 2.10 (1.39, 3.20) | 4.20% | 1.03 | 1.33 |
| RNASE9 | NM_001110360 | 1 | 0.00047969 | 2.10 (1.39, 3.20) | 4.20% | 1.03 | 1.33 |
| RNASE9 | NM_001110361 | 1 | 0.00047969 | 2.10 (1.39, 3.20) | 4.20% | 1.03 | 1.33 |
| RNASE9 | NM_001110356 | 1 | 0.00047969 | 2.10 (1.39, 3.20) | 4.20% | 1.03 | 1.33 |
| RNASE9 | NM_001110357 | 1 | 0.00047969 | 2.10 (1.39, 3.20) | 4.20% | 1.03 | 1.33 |
| SCRT2 | NM_033129 | 1 | 0.00019487 | 1.27 (1.12, 1.44) | 2.20% | 1.03 | 1.23 |
| SENP5 | NM_152699 | 1 | 0.00049234 | 1.68 (1.26, 2.26) | 4.30% | 1.03 | 1.34 |
| SPRR3 | NM_001097589 | 1 | 0.00034206 | 1.40 (1.16, 1.68) | 3.30% | 1.03 | 1.29 |
| SPRR3 | NM_005416 | 1 | 0.00034206 | 1.40 (1.16, 1.68) | 3.30% | 1.03 | 1.29 |
| TPTE | NM_199259 | 1 | 0.00025602 | 1.34 (1.15, 1.58) | 2.60% | 1.03 | 1.27 |
| TPTE | NM_199261 | 1 | 0.00025602 | 1.34 (1.15, 1.58) | 2.60% | 1.03 | 1.27 |
| TPTE | NM_199260 | 1 | 0.00025602 | 1.34 (1.15, 1.58) | 2.60% | 1.03 | 1.27 |
| TSC22D3 | NM_198057 | 1 | 0.00043616 | 1.96 (1.35, 2.85) | 4.00% | 1.03 | 1.34 |
| TSC22D3 | NM_004089 | 1 | 0.00043616 | 1.96 (1.35, 2.85) | 4.00% | 1.03 | 1.34 |
| TSC22D3 | NM_001015881 | 1 | 0.00043616 | 1.96 (1.35, 2.85) | 4.00% | 1.03 | 1.34 |
| USP40 | NM_018218 | -1 | 0.00058533 | 0.52 (0.36, 0.75) | 4.80% | 1.03 | 1.31 |
| ZCWPW1 | NM_017984 | -1 | 0.00057031 | 0.59 (0.43, 0.80) | 4.70% | 1.03 | 1.32 |
| AMY2B | NM_020978 | -1 | 0.00080037 | 0.46 (0.29, 0.72) | 5.90% | 1.02 | 1.28 |
| ATG5 | NM_004849 | 1 | 0.00080307 | 1.39 (1.15, 1.68) | 5.90% | 1.02 | 1.28 |
| C18orf26 | NM_173629 | 1 | 0.00074956 | 1.66 (1.24, 2.23) | 5.70% | 1.02 | 1.33 |
| C2orf70 | NM_001105519 | 1 | 0.00075733 | 1.87 (1.30, 2.69) | 5.70% | 1.02 | 1.33 |
| CCDC66 | NM_001141947 | -1 | 0.0008218 | 0.54 (0.37, 0.77) | 6.00% | 1.02 | 1.3 |
| CCDC66 | NR_024460 | -1 | 0.0008218 | 0.54 (0.37, 0.77) | 6.00% | 1.02 | 1.3 |
| CCDC66 | NM_001012506 | -1 | 0.0008218 | 0.54 (0.37, 0.77) | 6.00% | 1.02 | 1.3 |
| CCDC93 | NM_019044 | -1 | 0.00078652 | 0.49 (0.32, 0.74) | 5.90% | 1.02 | 1.29 |
| COQ6 | NM_182480 | -1 | 0.00096649 | 0.60 (0.45, 0.81) | 6.50% | 1.02 | 1.3 |
| COQ6 | NM_182476 | -1 | 0.00096649 | 0.60 (0.45, 0.81) | 6.50% | 1.02 | 1.3 |
| CRNDE | NR_034106 | 1 | 0.00080709 | 2.12 (1.37, 3.29) | 5.90% | 1.02 | 1.31 |
| CRNDE | NR_034105 | 1 | 0.00080709 | 2.12 (1.37, 3.29) | 5.90% | 1.02 | 1.31 |
| CRY1 | NM_004075 | -1 | 0.00115448 | 0.45 (0.28, 0.73) | 7.10% | 1.02 | 1.27 |
| CSF2RB | NM_000395 | -1 | 0.00105596 | 0.47 (0.30, 0.74) | 6.80% | 1.02 | 1.28 |
| CYP20A1 | NM_177538 | -1 | 0.00090837 | 0.40 (0.23, 0.69) | 6.30% | 1.02 | 1.26 |
| DAG1 | NM_001177644 | 1 | 0.00099617 | 2.35 (1.41, 3.92) | 6.60% | 1.02 | 1.29 |
| DAG1 | NM_001177643 | 1 | 0.00099617 | 2.35 (1.41, 3.92) | 6.60% | 1.02 | 1.29 |
| DAG1 | NM_001177642 | 1 | 0.00099617 | 2.35 (1.41, 3.92) | 6.60% | 1.02 | 1.29 |
| DAG1 | NM_001177641 | 1 | 0.00099617 | 2.35 (1.41, 3.92) | 6.60% | 1.02 | 1.29 |
| DAG1 | NM_001177640 | 1 | 0.00099617 | 2.35 (1.41, 3.92) | 6.60% | 1.02 | 1.29 |
| DAG1 | NM_001177634 | 1 | 0.00099617 | 2.35 (1.41, 3.92) | 6.60% | 1.02 | 1.29 |
| DAG1 | NM_004393 | 1 | 0.00099617 | 2.35 (1.41, 3.92) | 6.60% | 1.02 | 1.29 |
| DAG1 | NM_01165928 | 1 | 0.00099617 | 2.35 (1.41, 3.92) | 6.60% | 1.02 | 1.29 |
| DAG1 | NM_001177637 | 1 | 0.00099617 | 2.35 (1.41, 3.92) | 6.60% | 1.02 | 1.29 |
| DAG1 | NM_001177639 | 1 | 0.00099617 | 2.35 (1.41, 3.92) | 6.60% | 1.02 | 1.29 |
| DAG1 | NM_001177638 | 1 | 0.00099617 | 2.35 (1.41, 3.92) | 6.60% | 1.02 | 1.29 |
| DAG1 | NM_001177635 | 1 | 0.00099617 | 2.35 (1.41, 3.92) | 6.60% | 1.02 | 1.29 |
| DAG1 | NM_001177636 | 1 | 0.00099617 | 2.35 (1.41, 3.92) | 6.60% | 1.02 | 1.29 |
| DAP | NM_004394 | 1 | 0.00098142 | 2.05 (1.34, 3.15) | 6.60% | 1.02 | 1.31 |
| DDX56 | NM_019082 | 1 | 0.00044191 | 1.38 (1.15, 1.64) | 4.00% | 1.02 | 1.28 |
| DNAH6 | NM_001370 | -1 | 0.00084767 | 0.53 (0.36, 0.77) | 6.10% | 1.02 | 1.3 |
| DUSP9 | NM_ 001395 | 1 | 0.00099293 | 1.67 (1.23, 2.27) | 6.60% | 1.02 | 1.32 |
| EBP | NM_006579 | 1 | 0.0008591 | 2.33 (1.42, 3.82) | 6.10% | 1.02 | 1.3 |
| FEZF1 | NM_001160264 | 1 | 0.00066372 | 1.53 (1.20, 1.95) | 5.20% | 1.02 | 1.32 |
| FEZF1 | NM_001024613 | 1 | 0.00066372 | 1.53 (1.20, 1.95) | 5.20% | 1.02 | 1.32 |
| FRMPD2 | NM_001018071 | -1 | 0.00102321 | 0.51 (0.34, 0.76) | 6.70% | 1.02 | 1.29 |
| FRMPD2 | NR_033178 | -1 | 0.00102321 | 0.51 (0.34, 0.76) | 6.70% | 1.02 | 1.29 |
| G6PD | NM_000402 | 1 | 0.00095547 | 2.06 (1.34, 3.16) | 6.50% | 1.02 | 1.31 |
| G6PD | NM_001042351 | 1 | 0.00095547 | 2.06 (1.34, 3.16) | 6.50% | 1.02 | 1.31 |
| GABPB1 | NM_016654 | 1 | 0.0007582 | 1.44 (1.16, 1.78) | 5.70% | 1.02 | 1.29 |
| GABPB1 | NM_181427 | 1 | 0.0007582 | 1.44 (1.16, 1.78) | 5.70% | 1.02 | 1.29 |
| GABPB1 | NM_005254 | 1 | 0.0007582 | 1.44 (1.16, 1.78) | 5.70% | 1.02 | 1.29 |
| GABPB1 | NM_ 002041 | 1 | 0.0007582 | 1.44 (1.16, 1.78) | 5.70% | 1.02 | 1.29 |
| GABPB1 | NM_016655 | 1 | 0.0007582 | 1.44 (1.16, 1.78) | 5.70% | 1.02 | 1.29 |
| GALNT1 | NM_020474 | 1 | 0.00088364 | 1.87 (1.29, 2.71) | 6.20% | 1.02 | 1.32 |
| GPR50 | NM_004224 | 1 | 0.00059725 | 1.26 (1.11, 1.45) | 4.80% | 1.02 | 1.22 |
| HKR1 | NM_181786 | -1 | 0.00088702 | 0.48 (0.32, 0.74) | 6.20% | 1.02 | 1.29 |
| HSPA14 | NM_016299 | 1 | 0.00115563 | 2.11 (1.34, 3.30) | 7.10% | 1.02 | 1.3 |
| IGF2BP2 | NM_001007225 | 1 | 0.00095786 | 2.69 (1.49, 4.83) | 6.50% | 1.02 | 1.27 |
| IGF2BP2 | NM_006548 | 1 | 0.00095786 | 2.69 (1.49, 4.83) | 6.50% | 1.02 | 1.27 |
| IPW | NR_023915 | -1 | 0.00102737 | 0.62 (0.46, 0.82) | 6.70% | 1.02 | 1.3 |
| KIAA0467 | NM_015284 | -1 | 0.00107902 | 0.39 (0.22, 0.69) | 6.80% | 1.02 | 1.25 |
| KIF20A | NM_005733 | 1 | 0.00106069 | 2.64 (1.48, 4.72) | 6.80% | 1.02 | 1.27 |
| LOC144742 | NR_024246 | 1 | 0.00076829 | 1.38 (1.14, 1.66) | 5.80% | 1.02 | 1.28 |
| LRRC39 | NM_144620 | -1 | 0.00099003 | 0.54 (0.38, 0.78) | 6.60% | 1.02 | 1.3 |
| MIR24-2 | NR_029497 | 1 | 0.00095954 | 1.64 (1.22, 2.19) | 6.50% | 1.02 | 1.32 |
| MTHFR | NM_005957 | -1 | 0.00117686 | 0.46 (0.29, 0.74) | 7.20% | 1.02 | 1.27 |
| MTHFSD | NR_027490 | 1 | 0.00078921 | 1.89 (1.30, 2.73) | 5.90% | 1.02 | 1.32 |
| MTHFSD | NR_027489 | 1 | 0.00078921 | 1.89 (1.30, 2.73) | 5.90% | 1.02 | 1.32 |
| MTHFSD | NM_022764 | 1 | 0.00078921 | 1.89 (1.30, 2.73) | 5.90% | 1.02 | 1.32 |
| MTHFSD | NM_ 001159379 | 1 | 0.00078921 | 1.89 (1.30, 2.73) | 5.90% | 1.02 | 1.32 |
| MTHFSD | NM_001159378 | 1 | 0.00078921 | 1.89 (1.30, 2.73) | 5.90% | 1.02 | 1.32 |
| MTHFSD | NM_001159377 | 1 | 0.00078921 | 1.89 (1.30, 2.73) | 5.90% | 1.02 | 1.32 |
| MTHFSD | NM_001159380 | 1 | 0.00078921 | 1.89 (1.30, 2.73) | 5.90% | 1.02 | 1.32 |
| MYSM1 | NM_001085487 | -1 | 0.00086284 | 0.38 (0.21, 0.67) | 6.10% | 1.02 | 1.25 |
| N4BP2L2 | NM_033111 | -1 | 0.00082672 | 0.54 (0.38, 0.78) | 6.00% | 1.02 | 1.3 |
| N4BP2L2 | NM_014887 | -1 | 0.00082672 | 0.54 (0.38, 0.78) | 6.00% | 1.02 | 1.3 |
| NENF | NM_013349 | 1 | 0.00111716 | 2.06 (1.33, 3.19) | 7.00% | 1.02 | 1.3 |
| NENF | NR_26598 | 1 | 0.00111716 | 2.06 (1.33, 3.19) | 7.00% | 1.02 | 1.3 |
| NPY1R | NM_00909 | -1 | 0.00069841 | 0.53 (0.37, 0.77) | 5.40% | 1.02 | 1.31 |
| OR2J2 | NM_030905 | 1 | 0.0006695 | 1.55 (1.20, 1.99) | 5.20% | 1.02 | 1.32 |
| PLA2G2F | NM_022819 | 1 | 0.00106176 | 1.72 (1.24, 2.38) | 6.80% | 1.02 | 1.32 |
| POTEG | NR_027480 | 1 | 0.0008735 | 1.50 (1.18, 1.90) | 6.20% | 1.02 | 1.31 |
| POTEG | NM_001005356 | 1 | 0.0008735 | 1.50 (1.18, 1.90) | 6.20% | 1.02 | 1.31 |
| PRDM9 | NM_020227 | 1 | 0.00109686 | 1.80 (1.26, 2.56) | 6.90% | 1.02 | 1.32 |
| PRPF38A | NM_032864 | -1 | 0.00092473 | 0.45 (0.28, 0.72) | 6.40% | 1.02 | 1.28 |
| RPAP2 | NM_024813 | -1 | 0.0010711 | 0.35 (0.19, 0.66) | 6.80% | 1.02 | 1.23 |
| SERPINF2 | NM_001165920 | -1 | 0.00083318 | 0.51 (0.34, 0.76) | 6.00% | 1.02 | 1.3 |
| SERPINF2 | NM_000934 | -1 | 0.00083318 | 0.51 (0.34, 0.76) | 6.00% | 1.02 | 1.3 |
| SERPINF2 | NM_001165921 | -1 | 0.00083318 | 0.51 (0.34, 0.76) | 6.00% | 1.02 | 1.3 |
| SLC17A6 | NM_020346 | 1 | 0.00061603 | 1.59 (1.22, 2.07) | 4.90% | 1.02 | 1.33 |
| SMC5 | NM_015110 | -1 | 0.00090526 | 0.52 (0.35, 0.76) | 6.30% | 1.02 | 1.3 |
| SNORD115-40 | NR_003355 | 1 | 0.00100408 | 1.58 (1.20, 2.08) | 6.60% | 1.02 | 1.31 |
| SRSF11 | NM_01190987 | -1 | 0.00107233 | 0.52 (0.35, 0.77) | 6.80% | 1.02 | 1.29 |
| SRSF11 | NM_004768 | -1 | 0.00107233 | 0.52 (0.35, 0.77) | 6.80% | 1.02 | 1.29 |
| THEG | NM_016585 | 1 | 0.00063156 | 1.70 (1.25, 2.30) | 5.00% | 1.02 | 1.33 |
| THEG | NM_199202 | 1 | 0.00063156 | 1.70 (1.25, 2.30) | 5.00% | 1.02 | 1.33 |
| TMEM30C | NR_028358 | 1 | 0.00083733 | 1.66 (1.23, 2.23) | 6.00% | 1.02 | 1.32 |
| TMEM30C | NR_028357 | 1 | 0.00083733 | 1.66 (1.23, 2.23) | 6.00% | 1.02 | 1.32 |
| TRAF3IP1 | NM_015650 | -1 | 0.00080751 | 0.47 (0.30, 0.73) | 5.90% | 1.02 | 1.29 |
| TRAF3IP1 | NM_001139490 | -1 | 0.00080751 | 0.47 (0.30, 0.73) | 5.90% | 1.02 | 1.29 |
| TTC12 | NM_ 017868 | -1 | 0.00111797 | 0.40 (0.23, 0.69) | 7.00% | 1.02 | 1.25 |
| USP24 | NM 015306 | -1 | 0.00099068 | 0.46 (0.29, 0.73) | 6.60% | 1.02 | 1.28 |
| WBP4 | NM_007187 | -1 | 0.00083437 | 0.48 (0.32, 0.74) | 6.00% | 1.02 | 1.29 |
| ZNF440 | NM_152357 | -1 | 0.00113941 | 0.50 (0.33, 0.76) | 7.10% | 1.02 | 1.28 |
| ZNF644 | NM_032186 | -1 | 0.001187 | 0.44 (0.27, 0.72) | 7.20% | 1.02 | 1.26 |
| ZNF644 | NM_016620 | -1 | 0.001187 | 0.44 (0.27, 0.72) | 7.20% | 1.02 | 1.26 |
| ZNF644 | NM_201269 | -1 | 0.001187 | 0.44 (0.27, 0.72) | 7.20% | 1.02 | 1.26 |
| ZNF701 | NM_018260 | -1 | 0.00075297 | 0.47 (0.31, 0.73) | 5.70% | 1.02 | 1.29 |
| ZNF701 | NM_001172655 | -1 | 0.00075297 | 0.47 (0.31, 0.73) | 5.70% | 1.02 | 1.29 |
| AGBL2 | NM_024783 | -1 | 0.00142443 | 0.61 (0.45, 0.82) | 7.80% | 1.01 | 1.29 |
| BRP44 | NM_001143674 | 1 | 0.00172654 | 1.99 (1.30, 3.07) | 8.70% | 1.01 | 1.29 |
| BRP44 | NM_015415 | 1 | 0.00172654 | 1.99 (1.30, 3.07) | 8.70% | 1.01 | 1.29 |
| BRP44 | NR_026550 | 1 | 0.00172654 | 1.99 (1.30, 3.07) | 8.70% | 1.01 | 1.29 |
| BUB3 | NM_004725 | 1 | 0.00114919 | 1.73 (1.24, 2.40) | 7.10% | 1.01 | 1.32 |
| BUB3 | NM_001007793 | 1 | 0.00114919 | 1.73 (1.24, 2.40) | 7.10% | 1.01 | 1.32 |
| C10orf68 | NM_024688 | -1 | 0.00138334 | 0.52 (0.34, 0.77) | 7.70% | 1.01 | 1.28 |
| C1orf168 | NM_001004303 | -1 | 0.00118419 | 0.53 (0.36, 0.78) | 7.20% | 1.01 | 1.29 |
| CA5A | NM_001739 | 1 | 0.00164802 | 1.82 (1.25, 2.64) | 8.50% | 1.01 | 1.3 |
| CCDC39 | NM_181426 | -1 | 0.00165504 | 0.69 (0.55, 0.87) | 8.50% | 1.01 | 1.27 |
| CDC25B | NM_P04358 | 1 | 0.00150067 | 1.93 (1.28, 2.88) | 8.10% | 1.01 | 1.3 |
| CDC25B | NM_021873 | 1 | 0.00150067 | 1.93 (1.28, 2.88) | 8.10% | 1.01 | 1.3 |
| CDC25B | NM_021872 | 1 | 0.00150067 | 1.93 (1.28, 2.88) | 8.10% | 1.01 | 1.3 |
| CDK5RAP2 | NM_001011649 | -1 | 0.00157691 | 0.49 (0.32, 0.76) | 8.30% | 1.01 | 1.27 |
| CDK5RAP2 | NM_018249 | -1 | 0.00157691 | 0.49 (0.32, 0.76) | 8.30% | 1.01 | 1.27 |
| CEP110 | NM_007018 | -1 | 0.00153006 | 0.40 (0.23, 0.71) | 8.20% | 1.01 | 1.24 |
| CEP72 | NM_018140 | 1 | 0.00125053 | 2.10 (1.34, 3.28) | 7.30% | 1.01 | 1.3 |
| CRABP1 | NM_004378 | 1 | 0.00130325 | 1.86 (1.27, 2.71) | 7.50% | 1.01 | 1.31 |
| CROCC | NM_014675 | -1 | 0.00134738 | 0.58 (0.42, 0.81) | 7.50% | 1.01 | 1.29 |
| DBT | NM_001918 | -1 | 0.00183357 | 0.36 (0.19, 0.68) | 9.00% | 1.01 | 1.21 |
| DGCR10 | NR_026651 | 1 | 0.00120541 | 1.77 (1.25, 2.51) | 7.20% | 1.01 | 1.31 |
| DMRTC2 | NM_001040283 | 1 | 0.0016167 | 1.43 (1.14, 1.78) | 8.40% | 1.01 | 1.28 |
| EIF4EBP1 | NM_004095 | 1 | 0.00155936 | 1.58 (1.19, 2.09) | 8.30% | 1.01 | 1.3 |
| FAM120AOS | NM_198841 | -1 | 0.00179665 | 0.45 (0.27, 0.74) | 8.90% | 1.01 | 1.25 |
| FAM184B | NM_015688 | -1 | 0.00132516 | 0.41 (0.24, 0.71) | 7.50% | 1.01 | 1.25 |
| FANCD2 | NM_001018115 | 1 | 0.00173006 | 1.97 (1.29, 3.00) | 8.70% | 1.01 | 1.29 |
| FANCD2 | NM_033084 | 1 | 0.00173006 | 1.97 (1.29, 3.00) | 8.70% | 1.01 | 1.29 |
| FANCL | NM_001114636 | -1 | 0.00127386 | 0.61 (0.45, 0.82) | 7.40% | 1.01 | 1.29 |
| FANCL | NM_018062 | -1 | 0.00127386 | 0.61 (0.45, 0.82) | 7.40% | 1.01 | 1.29 |
| FRY | NM_023037 | -1 | 0.00162735 | 0.48 (0.30, 0.76) | 8.40% | 1.01 | 1.26 |
| GAB4 | NM_001037814 | 1 | 0.0010639 | 1.31 (1.12, 1.55) | 6.80% | 1.01 | 1.25 |
| GATS | NR 028040 | -1 | 0.00180899 | 0.45 (0.27, 0.74) | 9.00% | 1.01 | 1.25 |
| GATS | NR_028038 | -1 | 0.00180899 | 0.45 (0.27, 0.74) | 9.00% | 1.01 | 1.25 |
| GATS | NR_028039 | -1 | 0.00180899 | 0.45 (0.27, 0.74) | 9.00% | 1.01 | 1.25 |
| GATS | NM 178831 | -1 | 0.00180899 | 0.45 (0.27, 0.74) | 9.00% | 1.01 | 1.25 |
| GDF15 | NM_004864 | 1 | 0.00152308 | 1.91 (1.28, 2.86) | 8.20% | 1.01 | 1.3 |
| GDF7 | NM_182828 | 1 | 0.00091412 | 1.28 (1.10, 1.47) | 6.30% | 1.01 | 1.23 |
| GMCL1L | NR_003281 | 1 | 0.00162165 | 1.74 (1.23, 2.45) | 8.40% | 1.01 | 1.3 |
| GPRIN1 | NM_052899 | 1 | 0.00164633 | 2.21 (1.35, 3.63) | 8.50% | 1.01 | 1.28 |
| HAX1 | NM_006118 | 1 | 0.00171232 | 2.26 (1.36, 3.76) | 8.70% | 1.01 | 1.27 |
| HAX1 | NM_001018837 | 1 | 0.00171232 | 2.26 (1.36, 3.76) | 8.70% | 1.01 | 1.27 |
| HYDIN | NM_032821 | -1 | 0.0013742 | 0.55 (0.38, 0.79) | 7.60% | 1.01 | 1.29 |
| HYDIN | NM_017558 | -1 | 0.0013742 | 0.55 (0.38, 0.79) | 7.60% | 1.01 | 1.29 |
| HYDIN | NM_001198542 | -1 | 0.0013742 | 0.55 (0.38, 0.79) | 7.60% | 1.01 | 1.29 |
| HYDIN | NM_001198543 | -1 | 0.0013742 | 0.55 (0.38, 0.79) | 7.60% | 1.01 | 1.29 |
| KDELR1 | NM_006801 | 1 | 0.00120971 | 1.94 (1.30, 2.90) | 7.20% | 1.01 | 1.31 |
| LAMP1 | NM_005561 | 1 | 0.00113874 | 1.89 (1.29, 2.77) | 7.10% | 1.01 | 1.31 |
| LOC646862 | NM_001195135 | 1 | 0.00178119 | 1.81 (1.25, 2.62) | 8.90% | 1.01 | 1.3 |
| MAP3K14 | NM_003954 | -1 | 0.00125057 | 0.51 (0.34, 0.77) | 7.30% | 1.01 | 1.28 |
| MEGF9 | NM_001080497 | -1 | 0.00183087 | 0.37 (0.20, 0.69) | 9.00% | 1.01 | 1.22 |
| MIR54812 | NR_031688 | 1 | 0.00172026 | 1.48 (1.16, 1.90) | 8.70% | 1.01 | 1.29 |
| MITD1 | NM_138798 | -1 | 0.00149032 | 0.58 (0.42, 0.81) | 8.10% | 1.01 | 1.29 |
| MRPL45P2 | NR_033934 | -1 | 0.00136399 | 0.49 (0.31, 0.76) | 7.60% | 1.01 | 1.27 |
| MST4 | NM_001042452 | 1 | 0.00185523 | 2.47 (1.40, 4.35) | 9.00% | 1.01 | 1.26 |
| MST4 | NM_016542 | 1 | 0.00185523 | 2.47 (1.40, 4.35) | 9.00% | 1.01 | 1.26 |
| MST4 | NM_001042453 | 1 | 0.00185523 | 2.47 (1.40, 4.35) | 9.00% | 1.01 | 1.26 |
| MVD | NM_002461 | 1 | 0.001646 | 2.33 (1.38, 3.94) | 8.50% | 1.01 | 1.27 |
| NCRNA00115 | NR_024321 | -1 | 0.00141638 | 0.49 (0.32, 0.76) | 7.80% | 1.01 | 1.27 |
| NCRNA00287 | NR_026677 | -1 | 0.00120549 | 0.50 (0.32, 0.76) | 7.20% | 1.01 | 1.28 |
| NFAM1 | NM_145912 | -1 | 0.00171875 | 0.55 (0.38, 0.80) | 8.70% | 1.01 | 1.28 |
| NME1 | NM_000269 | 1 | 0.00140178 | 1.88 (1.28, 2.77) | 7.70% | 1.01 | 1.31 |
| NME1 | NM_198175 | 1 | 0.00140178 | 1.88 (1.28, 2.77) | 7.70% | 1.01 | 1.31 |
| NUDT21 | NM_007006 | 1 | 0.0017855 | 1.91 (1.27, 2.86) | 8.90% | 1.01 | 1.3 |
| PARD3B | NM_205863 | -1 | 0.00120268 | 0.61 (0.46, 0.82) | 7.20% | 1.01 | 1.29 |
| PARD3B | NM_057177 | -1 | 0.00120268 | 0.61 (0.46, 0.82) | 7.20% | 1.01 | 1.29 |
| PARD3B | NM_152526 | -1 | 0.00120268 | 0.61 (0.46, 0.82) | 7.20% | 1.01 | 1.29 |
| PBX1 | NM_002585 | 1 | 0.00147845 | 1.64 (1.21, 2.23) | 8.00% | 1.01 | 1.31 |
| RFWD3 | NM_018124 | 1 | 0.00121663 | 1.83 (1.27, 2.64) | 7.20% | 1.01 | 1.31 |
| RFX7 | NM_022841 | -1 | 0.00158617 | 0.46 (0.28, 0.74) | 8.30% | 1.01 | 1.26 |
| RGS4 | NM_005613 | 1 | 0.00159894 | 2.17 (1.34, 3.51) | 8.40% | 1.01 | 1.28 |
| RGS4 | NM_001113381 | 1 | 0.00159894 | 2.17 (1.34, 3.51) | 8.40% | 1.01 | 1.28 |
| RGS4 | NM_001102445 | 1 | 0.00159894 | 2.17 (1.34, 3.51) | 8.40% | 1.01 | 1.28 |
| RGS4 | NM_001113380 | 1 | 0.00159894 | 2.17 (1.34, 3.51) | 8.40% | 1.01 | 1.28 |
| RHCG | NM_016321 | 1 | 0.0015871 | 1.72 (1.23, 2.40) | 8.30% | 1.01 | 1.31 |
| RNPC3 | NM_017619 | -1 | 0.0015841 | 0.45 (0.28, 0.74) | 8.30% | 1.01 | 1.26 |
| SEC61A1 | NM_013336 | 1 | 0.00127515 | 1.92 (1.29, 2.85) | 7.40% | 1.01 | 1.31 |
| SFXN1 | NM_022754 | 1 | 0.00126021 | 1.97 (1.30, 2.97) | 7.40% | 1.01 | 1.3 |
| SGIP1 | NM_032291 | 1 | 0.00131363 | 2.33 (1.39, 3.90) | 7.50% | 1.01 | 1.28 |
| SH3GLB2 | NM_020145 | -1 | 0.00133594 | 0.57 (0.40, 0.80) | 7.50% | 1.01 | 1.29 |
| SIN3B | NM_015260 | -1 | 0.001324 | 0.47 (0.30, 0.75) | 7.50% | 1.01 | 1.27 |
| SLC1A5 | NM 001145145 | 1 | 0.00133504 | 2.79 (1.49, 5.23) | 7.50% | 1.01 | 1.25 |
| SLC1A5 | NM_005628 | 1 | 0.00133504 | 2.79 (1.49, 5.23) | 7.50% | 1.01 | 1.25 |
| SLC1A5 | NM_001145144 | 1 | 0.00133504 | 2.79 (1.49, 5.23) | 7.50% | 1.01 | 1.25 |
| SMU1 | NM_018225 | -1 | 0.00176915 | 0.51 (0.34, 0.78) | 8.90% | 1.01 | 1.27 |
| SNORD18C | NR_^{.}002443 | 1 | 0.00106266 | 1.56 (1.20, 2.04) | 6.80% | 1.01 | 1.31 |
| SPCS1 | NM_014041 | 1 | 0.00124058 | 2.15 (1.35, 3.42) | 7.30% | 1.01 | 1.29 |
| TBRG4 | NM_004749 | 1 | 0.0009404 | 1.46 (1.17, 1.83) | 6.40% | 1.01 | 1.3 |
| TBRG4 | NM 199122 | 1 | 0.0009404 | 1.46 (1.17, 1.83) | 6.40% | 1.01 | 1.3 |
| TBRG4 | NM_030900 | 1 | 0.0009404 | 1.46 (1.17, 1.83) | 6.40% | 1.01 | 1.3 |
| TEX2 | NM_018469 | 1 | 0.00126006 | 1.64 (1.22, 2.22) | 7.40% | 1.01 | 1.31 |
| TNFSF4 | NM_003326 | 1 | 0.00129531 | 2.33 (1.39, 3.90) | 7.50% | 1.01 | 1.28 |
| UPK1A | NM_007000 | 1 | 0.00147695 | 1.46 (1.16, 1.84) | 8.00% | 1.01 | 1.29 |
| ZIC2 | NM_007129 | 1 | 0.00133972 | 2.12 (1.34, 3.36) | 7.50% | 1.01 | 1.29 |
| ZNF317 | NM_020933 | -1 | 0.00145044 | 0.58 (0.41, 0.81) | 7.90% | 1.01 | 1.29 |
| ZNF317 | NM_001190791 | -1 | 0.00145044 | 0.58 (0.41, 0.81) | 7.90% | 1.01 | 1.29 |
| ZNF626 | NM_145297 | -1 | 0.0015675 | 0.53 (0.36, 0.79) | 8.30% | 1.01 | 1.28 |
| ZNF626 | NM_001076675 | -1 | 0.0015675 | 0.53 (0.36, 0.79) | 8.30% | 1.01 | 1.28 |
| ZNF829 | NM_001171979 | -1 | 0.00120516 | 0.56 (0.39, 0.79) | 7.20% | 1.01 | 1.29 |
| ZNF829 | NM_001037232 | -1 | 0.00120516 | 0.56 (0.39, 0.79) | 7.20% | 1.01 | 1.29 |
| ZRANB2 | NM_005455 | -1 | 0.00127193 | 0.47 (0.30, 0.75) | 7.40% | 1.01 | 1.27 |
| ZRANB2 | NM_203350 | -1 | 0.00127193 | 0.47 (0.30, 0.75) | 7.40% | 1.01 | 1.27 |
| APOBEC2 | NM_006789 | -1 | 0.00205447 | 0.50 (0.33, 0.78) | 9.60% | 1 | 1.26 |
| BICD2 | NM_001003800 | -1 | 0.00208035 | 0.43 (0.25, 0.73) | 9.60% | 1 | 1.24 |
| BICD2 | NM_015250 | -1 | 0.00208035 | 0.43 (0.25, 0.73) | 9.60% | 1 | 1.24 |
| BUD13 | NM_032725 | -1 | 0.0020833 | 0.47 (0.29, 0.76) | 9.60% | 1 | 1.25 |
| BUD13 | NM_001159736 | -1 | 0.0020833 | 0.47 (0.29, 0.76) | 9.60% | 1 | 1.25 |
| CCDC103 | NM_213607 | -1 | 0.00214716 | 0.55 (0.38, 0.81) | 9.80% | 1 | 1.27 |
| CHCHD1 | NM_203298 | 1 | 0.00187362 | 1.95 (1.28, 2.97) | 9.10% | 1 | 1.29 |
| CLDN4 | NM_001305 | 1 | 0.00214986 | 1.92 (1.27, 2.91) | 9.80% | 1 | 1.29 |
| CLK1 | NR_027855 | -1 | 0.00214838 | 0.54 (0.36, 0.80) | 9.80% | 1 | 1.27 |
| CLK1 | NR_027856 | -1 | 0.00214838 | 0.54 (0.36, 0.80) | 9.80% | 1 | 1.27 |
| CLK1 | NM_004071 | -1 | 0.00214838 | 0.54 (0.36, 0.80) | 9.80% | 1 | 1.27 |
| CLK1 | NM_001162407 | -1 | 0.00214838 | 0.54 (0.36, 0.80) | 9.80% | 1 | 1.27 |
| DCK | NM_000788 | 1 | 0.00205322 | 1.78 (1.23, 2.57) | 9.60% | 1 | 1.3 |
| EGLN1 | NM_022051 | 1 | 0.00196644 | 1.82 (1.25, 2.66) | 9.40% | 1 | 1.3 |
| ESRP2 | NM_024939 | 1 | 0.00213417 | 2.28 (1.35, 3.86) | 9.80% | 1 | 1.26 |
| FNBP4 | NM_015308 | -1 | 0.0020334 | 0.53 (0.35, 0.79) | 9.60% | 1 | 1.27 |
| FUT11 | NM_173540 | 1 | 0.00189847 | 1.98 (1.29, 3.06) | 9.20% | 1 | 1.29 |
| FYTTD1 | NM_032288 | 1 | 0.00192913 | 1.54 (1.17, 2.03) | 9.20% | 1 | 1.29 |
| FYTTD1 | NR_027840 | 1 | 0.00192913 | 1.54 (1.17, 2.03) | 9.20% | 1 | 1.29 |
| FYTTD1 | NM_001011537 | 1 | 0.00192913 | 1.54 (1.17, 2.03) | 9.20% | 1 | 1.29 |
| GALNTL1 | NM_001168368 | -1 | 0.00217436 | 0.48 (0.30, 0.77) | 9.90% | 1 | 1.25 |
| GALNTL1 | NM_020692 | -1 | 0.00217436 | 0.48 (0.30, 0.77) | 9.90% | 1 | 1.25 |
| GSTM3 | NR_024537 | -1 | 0.00185428 | 0.50 (0.32, 0.77) | 9.00% | 1 | 1.26 |
| GSTM3 | NM_000849 | -1 | 0.00185428 | 0.50 (0.32, 0.77) | 9.00% | 1 | 1.26 |
| HECW2 | NM_020760 | -1 | 0.0021151 | 0.55 (0.38, 0.81) | 9.70% | 1 | 1.27 |
| KIF27 | NM_017576 | -1 | 0.00181164 | 0.58 (0.41, 0.81) | 9.00% | 1 | 1.28 |
| KRTCAP2 | NM_173852 | 1 | 0.00192061 | 1.92 (1.27, 2.90) | 9.20% | 1 | 1.29 |
| LAMA1 | NM_005559 | 1 | 0.00202571 | 2.18 (1.33, 3.56) | 9.60% | 1 | 1.27 |
| MIR603 | NR_030334 | 1 | 0.00201767 | 1.78 (1.24, 2.57) | 9.60% | 1 | 1.3 |
| MYO5A | NM_000259 | 1 | 0.00177654 | 1.36 (1.12, 1.65) | 8.90% | 1 | 1.26 |
| MYO5A | NM_001142495 | 1 | 0.00177654 | 1.36 (1.12, 1.65) | 8.90% | 1 | 1.26 |
| PCGF3 | NM_006315 | -1 | 0.00202742 | 0.54 (0.36, 0.80) | 9.60% | 1 | 1.27 |
| RAB11B | NM_004218 | -1 | 0.00191529 | 0.48 (0.30, 0.76) | 9.20% | 1 | 1.26 |
| SCD | NM_005063 | 1 | 0.00208616 | 2.39 (1.37, 4.16) | 9.60% | 1 | 1.26 |
| SMG5 | NM_015327 | 1 | 0.0020908 | 1.80 (1.24, 2.62) | 9.60% | 1 | 1.29 |
| SYNPO | NM_001109974 | -1 | 0.00206694 | 0.51 (0.33, 0.78) | 9.60% | 1 | 1.26 |
| SYNPO | NM_007286 | -1 | 0.00206694 | 0.51 (0.33, 0.78) | 9.60% | 1 | 1.26 |
| SYNPO | NM_001166209 | -1 | 0.00206694 | 0.51 (0.33, 0.78) | 9.60% | 1 | 1.26 |
| SYNPO | NM_001166208 | -1 | 0.00206694 | 0.51 (0.33, 0.78) | 9.60% | 1 | 1.26 |
| TAF5L | NM_014409 | 1 | 0.00200753 | 1.92 (1.27, 2.90) | 9.50% | 1 | 1.29 |
| TAF5L | NM_001025247 | 1 | 0.00200753 | 1.92 (1.27, 2.90) | 9.50% | 1 | 1.29 |
| USP16 | NM_006447 | -1 | 0.00184893 | 0.56 (0.39, 0.81) | 9.00% | 1 | 1.28 |
| USP16 | NM_001032410 | -1 | 0.00184893 | 0.56 (0.39, 0.81) | 9.00% | 1 | 1.28 |
| USP16 | NM_001001992 | -1 | 0.00184893 | 0.56 (0.39, 0.81) | 9.00% | 1 | 1.28 |
| ZNF44 | NM_016264 | -1 | 0.00189847 | 0.36 (0.19, 0.68) | 9.20% | 1 | 1.21 |
| ZNF44 | NM_001164276 | -1 | 0.00189847 | 0.36 (0.19, 0.68) | 9.20% | 1 | 1.21 |

| Table 3. Assembled Intronic RNAs identified as Associated with Risk of Breast Cancer Recurrence in 136 Breast Cancer Patients | | | | | | |
|---|---|---|---|---|---|---|
| Intron | Direction of Association (1=Higher Expression Means Higher Risk) | p-value | Standardized Hazard Ratio Estimate (95% Confidence Interval) | q-value (FDR) | Maximum Lower Bound Std HR @ 10% FDR | Regression-to-the-Mean-Corrected Std HR |
| NUDT19 | 1 | 2.99E-13 | 2.22 (1.79, 2.75) | <0.1% | 1.35 | 1.77 |
| CLINT1 | 1 | 1.74E-11 | 2.17 (1.73, 2.72) | <0.1% | 1.31 | 1.71 |
| CLINT1 | 1 | 1.74E-11 | 2.17 (1.73, 2.72) | <0.1% | 1.31 | 1.71 |
| CLINT1 | 1 | 1.74E-11 | 2.17 (1.73, 2.72) | <0.1% | 1.31 | 1.71 |
| KIF1B | 1 | 8.27E-09 | 2.85 (2.00, 4.07) | <0.1% | 1.3 | 1.65 |
| KIF1B | 1 | 8.27E-09 | 2.85 (2.00, 4.07) | <0.1% | 1.3 | 1.65 |
| ANKRD27 | 1 | 5.11E-16 | 1.66 (1.47, 1.88) | <0.1% | 1.28 | 1.57 |
| NOL8 | -1 | 3.80E-09 | 0.43 (0.32, 0.57) | <0.1% | 1.28 | 1.67 |
| NOL8 | -1 | 3.80E-09 | 0.43 (0.32, 0.57) | <0.1% | 1.28 | 1.67 |
| TTLL3 | -1 | 4.56E-09 | 0.41 (0.30, 0.55) | <0.1% | 1.28 | 1.68 |
| TTLL3 | -1 | 4.56E-09 | 0.41 (0.30, 0.55) | <0.1% | 1.28 | 1.68 |
| SECISBP2L | 1 | 3.74E-10 | 1.93 (1.57, 2.37) | <0.1% | 1.25 | 1.62 |
| SECISBP2L | 1 | 3.74E-10 | 1.93 (1.57, 2.37) | <0.1% | 1.25 | 1.62 |
| FOXJ2 | -1 | 5.35E-08 | 0.48 (0.37, 0.62) | <0.1% | 1.21 | 1.6 |
| SEC24A | 1 | 7.26E-09 | 1.90 (1.53, 2.37) | <0.1% | 1.21 | 1.58 |
| FRS3 | -1 | 2.70E-08 | 0.52 (0.41, 0.65) | <0.1% | 1.2 | 1.58 |
| LOC100129518 | 1 | 1.04E-09 | 1.73 (1.45, 2.07) | <0.1% | 1.2 | 1.54 |
| MEGF9 | -1 | 3.53E-07 | 0.42 (0.30, 0.59) | <0.1% | 1.2 | 1.58 |
| CANX | 1 | 6.36E-08 | 1.94 (1.52, 2.46) | <0.1% | 1.19 | 1.55 |
| CANX | 1 | 6.36E-08 | 1.94 (1.52, 2.46) | <0.1% | 1.19 | 1.55 |
| LRCH3 | 1 | 5.43E-09 | 1.71 (1.43, 2.04) | <0.1% | 1.19 | 1.52 |
| MPP6 | 1 | 6.89E-09 | 1.74 (1.44, 2.10) | <0.1% | 1.19 | 1.53 |
| TOMM20L | -1 | 3.52E-08 | 0.56 (0.45, 0.68) | <0.1% | 1.19 | 1.54 |
| ZNF484 | -1 | 7.30E-07 | 0.43 (0.31, 0.60) | <0.1% | 1.19 | 1.56 |
| ZNF484 | -1 | 7.30E-07 | 0.43 (0.31, 0.60) | <0.1% | 1.19 | 1.56 |
| AACSP1 | 1 | 1.95E-08 | 1.71 (1.42, 2.06) | <0.1% | 1.18 | 1.51 |
| CDHR2 | 1 | 2.94E-07 | 1.83 (1.45, 2.31) | <0.1% | 1.17 | 1.51 |
| CDHR2 | 1 | 2.94E-07 | 1.83 (1.45, 2.31) | <0.1% | 1.17 | 1.51 |
| CEP152 | 1 | 6.13E-07 | 1.87 (1.46, 2.39) | <0.1% | 1.17 | 1.51 |
| CEP152 | 1 | 6.13E-07 | 1.87 (1.46, 2.39) | <0.1% | 1.17 | 1.51 |
| FAM83A | 1 | 3.27E-06 | 2.24 (1.59, 3.14) | 0.10% | 1.17 | 1.5 |
| FAM83A | 1 | 3.27E-06 | 2.24 (1.59, 3.14) | 0.10% | 1.17 | 1.5 |
| GALNT12 | 1 | 7.50E-08 | 1.70 (1.40, 2.06) | <0.1% | 1.17 | 1.49 |
| HIVEP2 | 1 | 1.01E-06 | 2.17 (1.59, 2.96) | <0.1% | 1.17 | 1.52 |
| HOMEZ | -1 | 1.87E-08 | 0.61 (0.52, 0.73) | <0.1% | 1.17 | 1.49 |
| NCCRP1 | 1 | 1.69E-07 | 1.72 (1.41, 2.12) | <0.1% | 1.17 | 1.49 |
| NPRL2 | -1 | 1.39E-10 | 0.69 (0.62, 0.77) | <0.1% | 1.17 | 1.4 |
| POLG2 | 1 | 8.71E-08 | 1.68 (1.39, 2.03) | <0.1% | 1.17 | 1.48 |
| PRAM1 | -1 | 1.56E-07 | 0.54 (0.43, 0.68) | <0.1% | 1.17 | 1.53 |
| SPATA21 | -1 | 1.74E-07 | 0.55 (0.44, 0.69) | <0.1% | 1.17 | 1.53 |
| SUN1 | 1 | 2.45E-08 | 1.60 (1.36, 1.89) | <0.1% | 1.17 | 1.46 |
| SUN1 | 1 | 2.45E-08 | 1.60 (1.36, 1.89) | <0.1% | 1.17 | 1.46 |
| SUN1 | 1 | 2.45E-08 | 1.60 (1.36, 1.89) | <0.1% | 1.17 | 1.46 |
| SUN1 | 1 | 2.45E-08 | 1.60 (1.36, 1.89) | <0.1% | 1.17 | 1.46 |
| SUN1 | 1 | 2.45E-08 | 1.60 (1.36, 1.89) | <0.1% | 1.17 | 1.46 |
| TCTN2 | -1 | 7.74E-07 | 0.52 (0.40, 0.68) | <0.1% | 1.17 | 1.52 |
| TCTN2 | -1 | 7.74E-07 | 0.52 (0.40, 0.68) | <0.1% | 1.17 | 1.52 |
| TMEM101 | -1 | 1.18E-06 | 0.51 (0.39, 0.67) | <0.1% | 1.17 | 1.52 |
| ZMAT1 | -1 | 1.22E-06 | 0.46 (0.34, 0.63) | <0.1% | 1.17 | 1.54 |
| ZMAT1 | -1 | 1.22E-06 | 0.46 (0.34, 0.63) | <0.1% | 1.17 | 1.54 |
| C14orf50 | -1 | 9.54E-08 | 0.61 (0.51, 0.73) | <0.1% | 1.16 | 1.47 |
| C2orf73 | -1 | 2.94E-07 | 0.57 (0.46, 0.71) | <0.1% | 1.16 | 1.5 |
| C9orf64 | -1 | 2.45E-06 | 0.49 (0.37, 0.66) | <0.1% | 1.16 | 1.52 |
| KCMF1 | 1 | 3.74E-07 | 1.73 (1.40, 2.13) | <0.1% | 1.16 | 1.48 |
| PDE6B | -1 | 5.88E-06 | 0.45 (0.32, 0.64) | 0.10% | 1.16 | 1.5 |
| PDE6B | -1 | 5.88E-06 | 0.45 (0.32, 0.64) | 0.10% | 1.16 | 1.5 |
| PDE6B | -1 | 5.88E-06 | 0.45 (0.32, 0.64) | 0.10% | 1.16 | 1.5 |
| SF3B3 | 1 | 4.06E-06 | 2.03 (1.50, 2.75) | 0.10% | 1.16 | 1.49 |
| SIRT6 | -1 | 2.10E-08 | 0.64 (0.55, 0.75) | <0.1% | 1.16 | 1.45 |
| SIRT6 | -1 | 2.10E-08 | 0.64 (0.55, 0.75) | <0.1% | 1.16 | 1.45 |
| SLC35D1 | -1 | 4.54E-06 | 0.45 (0.32, 0.63) | 0.10% | 1.16 | 1.51 |
| SMURF2 | 1 | 3.34E-07 | 1.75 (1.41, 2.16) | <0.1% | 1.16 | 1.49 |
| SPIN1 | -1 | 5.97E-06 | 0.43 (0.30, 0.62) | 0.10% | 1.16 | 1.5 |
| TEX2 | 1 | 1.23E-07 | 1.62 (1.35, 1.94) | <0.1% | 1.16 | 1.46 |
| ZNF44 | -1 | 1.16E-06 | 0.53 (0.40, 0.68) | <0,1% | 1.16 | 1.52 |
| ZNF44 | -1 | 1.16E-06 | 0.53 (0.40, 0.68) | <0.1% | 1.16 | 1.52 |
| ACAP2 | 1 | 2.30E-06 | 1.82 (1.42, 2.33) | <0.1% | 1.15 | 1.48 |
| CCDC47 | 1 | 2.83E-07 | 1.61 (1.34, 1.92) | <0.1% | 1.15 | 1.45 |
| CIC | -1 | 3.57E-06 | 0.52 (0.40, 0.69) | 0.10% | 1.15 | 1.5 |
| GPR126 | 1 | 5.67E-06 | 2.00 (1.48, 2.69) | 0.10% | 1.15 | 1.48 |
| GPR126 | 1 | 5.67E-06 | 2.00 (1.48, 2.69) | 0.10% | 1.15 | 1.48 |
| GPR126 | 1 | 5.67E-06 | 2.00 (1.48, 2.69) | 0.10% | 1.15 | 1.48 |
| GPR126 | 1 | 5.67E-06 | 2.00 (1.48, 2.69) | 0.10% | 1.15 | 1.48 |
| IDH2 | 1 | 1.20E-07 | 1.60 (1.34, 1.90) | <0.1% | 1.15 | 1.45 |
| KIAA0649 | -1 | 2.15E-06 | 0.54 (0.42, 0.70) | <0.1% | 1.15 | 1.5 |
| LOC100128977 | -1 | 7.62E-06 | 0.50 (0.37, 0.68) | 0.10% | 1.15 | 1.49 |
| MAGEA10 | 1 | 7.79E-08 | 1.55 (1.32, 1.82) | <0.1% | 1.15 | 1.43 |
| MAGEA10 | 1 | 7.79E-08 | 1.55 (1.32, 1.82) | <0.1% | 1.15 | 1.43 |
| MARCH6 | 1 | 8.72E-06 | 2.14 (1.53, 2.99) | 0.10% | 1.15 | 1.47 |
| MCART6 | -1 | 1.04E-05 | 0.48 (0.35, 0.67) | 0.10% | 1.15 | 1.48 |
| PAPD7 | 1 | 1.08E-05 | 2.13 (1.52, 2.99) | 0.10% | 1.15 | 1.46 |
| PAPD7 | 1 | 1.08E-05 | 2.13 (1.52, 2.99) | 0.10% | 1.15 | 1.46 |
| PAPD7 | 1 | 1.08E-05 | 2.13 (1.52, 2.99) | 0.10% | 1.15 | 1.46 |
| PLD1 | 1 | 3.54E-08 | 1.53 (1.32, 1.78) | <0.1% | 1.15 | 1.43 |
| PLD1 | 1 | 3.54E-08 | 1.53 (1.32, 1.78) | <0.1% | 1.15 | 1.43 |
| RAD9B | -1 | 1.83E-06 | 0.57 (0.45, 0.72) | <0.1% | 1.15 | 1.48 |
| SMARCD2 | 1 | 7.24E-07 | 1.63 (1.34, 1.98) | <0.1% | 1.15 | 1.45 |
| TRPM8 | 1 | 1.83E-06 | 1.82 (1.42, 2.33) | <0.1% | 1.15 | 1.48 |
| TTF1 | -1 | 1.08E-05 | 0.47 (0.33, 0.66) | 0.10% | 1.15 | 1.48 |
| UGT2B7 | 1 | 4.60E-08 | 1.50 (1.29, 1.73) | <0.1% | 1.15 | 1.41 |
| WARS2 | -1 | 2.69E-05 | 0.40 (0.26, 0.61) | 0.20% | 1.15 | 1.45 |
| WARS2 | -1 | 2.69E-05 | 0.40 (0.26, 0.61) | 0.20% | 1.15 | 1.45 |
| ZDHHC19 | 1 | 1.05E-05 | 2.22 (1.56, 3.17) | 0.10% | 1.15 | 1.46 |
| ZFR | 1 | 1.11E-05 | 2.08 (1.50, 2.88) | 0.10% | 1.15 | 1.46 |
| ANKRD17 | 1 | 2.20E-07 | 1.49 (1.28, 1.74) | <0.1% | 1.14 | 1.4 |
| ANKRD17 | 1 | 2.20E-07 | 1.49 (1.28,1.74) | <0.1% | 1.14 | 1.4 |
| C10orf93 | -1 | 2.49E-05 | 044 (0.30, 0.65) | 0.20% | 1.14 | 1.46 |
| DAP | 1 | 1.93E-05 | 2.05 (1.47, 2.85) | 0.20% | 1.14 | 1.45 |
| G3BP2 | 1 | 1.38E-07 | 1.50 (1.29, 1.75) | <0.1% | 1.14 | 1.4 |
| G3BP2 | 1 | 1.38E-07 | 1.50 (1.29, 1.75) | <0.1% | 1.14 | 1.4 |
| G3BP2 | 1 | 1.38E-07 | 1.50 (1.29, 1.75) | <0.1% | 1.14 | 1.4 |
| GAN | 1 | 2.40E-05 | 2.16 (1.51, 3.09) | 0.20% | 1.14 | 1.44 |
| GSTM2 | -1 | 1.12E-05 | 0.53 (0.40, 0.70) | 0.10% | 1.14 | 1.47 |
| GSTM2 | -1 | 1.12E-05 | 0.53 (0.40, 0.70) | 0.10% | 1.14 | 1.47 |
| IGF2BP2 | 1 | 6.05E-06 | 1.84 (1.41, 2.40) | 0.10% | 1.14 | 1.46 |
| IGF2BP2 | 1 | 6.05E-06 | 1.84 (1.41, 2.40) | 0.10% | 1.14 | 1.46 |
| INPP5K | -1 | 1.47E-05 | 0.48 (0.35, 0.67) | 0.10% | 1.14 | 1.47 |
| INPP5K | -1 | 1.47E-05 | 0.48 (0.35, 0.67) | 0.10% | 1.14 | 1.47 |
| INPP5K | -1 | 1.47E-05 | 0.48 (0.35, 0.67) | 0.10% | 1.14 | 1.47 |
| LANCL1 | -1 | 2.01E-05 | 0.48 (0.34, 0.67) | 0.20% | 1.14 | 1.47 |
| LANCL1 | -1 | 2.01E-05 | 0.48 (0.34, 0.67) | 0.20% | 1.14 | 1.47 |
| LANCL1 | -1 | 2.01E-05 | 0.48 (0.34, 0.67) | 0.20% | 1.14 | 1.47 |
| MT4 | 1 | 8.97E-10 | 1.35 (1.22, 1.48) | <0.1% | 1.14 | 1.32 |
| SCN1B | -1 | 1.11E-05 | 0.52 (0.39, 0.70) | 0.10% | 1.14 | 1.47 |
| SCN1B | -1 | 1.11E-05 | 0.52 (0.39, 0.70) | 0.10% | 1.14 | 1.47 |
| TXNRD1 | 1 | 9.57E-06 | 1.93 (1.44, 2.58) | 0.10% | 1.14 | 1.46 |
| TXNRD1 | 1 | 9.57E-06 | 1.93 (1.44, 2.58) | 0.10% | 1.14 | 1.46 |
| TXNRD1 | 1 | 9.57E-06 | 1.93 (1.44, 2.58) | 0.10% | 1.14 | 1.46 |
| TXNRD1 | 1 | 9.57E-06 | 1.93 (1.44, 2.58) | 0.10% | 1.14 | 1.46 |
| TXNRD1 | 1 | 9.57E-06 | 1.93 (1.44, 2.58) | 0.10% | 1.14 | 1.46 |
| USO1 | 1 | 1.96E-07 | 1.49 (1.28, 1.74) | <0.1% | 1.14 | 1.4 |
| ZBED4 | 1 | 1.01E-07 | 1.47 (1.27, 1.69) | <0.1% | 1.14 | 1.39 |
| ZFP42 | 1 | 1.85E-07 | 1.50 (1.29, 1.75) | <0.1% | 1.14 | 1.4 |
| ZNF415 | -1 | 5.33E-06 | 0.57 (0.44, 0.72) | 0.10% | 1.14 | 1.47 |
| ZNF415 | -1 | 5.33E-06 | 0.57 (0.44, 0.72) | 0.10% | 1.14 | 1.47 |
| ZNF415 | -1 | 5.33E-06 | 0.57 (0.44, 0.72) | 0.10% | 1.14 | 1.47 |
| ZNF415 | -1 | 5.33E-06 | 0.57 (0.44, 0.72) | 0.10% | 1.14 | 1.47 |
| ZNF625 | -1 | 6.20E-06 | 0.53 (0.40, 0.69) | 0.10% | 1.14 | 1.48 |
| ACBD4 | -1 | 4.09E-06 | 0.62 (0.51, 0.76) | 0.10% | 1.13 | 1.43 |
| ACBD4 | -1 | 4.09E-06 | 0.62 (0.51, 0.76) | 0.10% | 1.13 | 1.43 |
| ACBD4 | -1 | 4.09E-06 | 0.62 (0.51, 0.76) | 0.10% | 1.13 | 1.43 |
| ACBD4 | -1 | 4.09E-06 | 0.62 (0.51, 0.76) | 0.10% | 1.13 | 1.43 |
| ACBD4 | -1 | 4.09E-06 | 0.62 (0.51, 0.76) | 0.10% | 1.13 | 1.43 |
| ANKRD19 | -1 | 7.74E-06 | 0.56 (0.44, 0.72) | 0.10% | 1.13 | 1.46 |
| BPTF | 1 | 2.42E-06 | 1.61 (1.32, 1.96) | <0.1% | 1.13 | 1.43 |
| BPTF | 1 | 2.42E-06 | 1.61 (1.32, 1.96) | <0.1% | 1.13 | 1.43 |
| C17orf49 | -1 | 5.27E-07 | 0.69 (0.59, 0.79) | <0.1% | 1.13 | 1.38 |
| C17orf49 | -1 | 5.27E-07 | 0.69 (0.59, 0.79) | <0.1% | 1.13 | 1.38 |
| C17orf49 | -1 | 5.27E-07 | 0.69 (0.59, 0.79) | <0.1% | 1.13 | 1.38 |
| C2orf84 | -1 | 3.24E-06 | 0.62 (0.51, 0.76) | 0.10% | 1.13 | 1.43 |
| C3orf15 | -1 | 5.65E-05 | 0.45 (0.30, 0.66) | 0.40% | 1.13 | 1.43 |
| C6orf225 | -1 | 3.63E-06 | 0.60 (0.49, 0.75) | 0.10% | 1.13 | 1.45 |
| CCDC45 | 1 | 3.36E-06 | 1.62 (1.32, 1.99) | 0.10% | 1.13 | 1.43 |
| CDK20 | -1 | 0.000014394 | 0.52 (0.38, 0.70) | 0.10% | 1.13 | 1.47 |
| CDK20 | -1 | 0.000014394 | 0.52 (0.38, 0.70) | 0.10% | 1.13 | 1.47 |
| CDK20 | -1 | 0.000014394 | 0.52 (0.38, 0.70) | 0.10% | 1.13 | 1.47 |
| CDK20 | -1 | 0.000014394 | 0.52 (0.38, 0.70) | 0.10% | 1.13 | 1.47 |
| CDK20 | -1 | 0.000014394 | 0.52 (0.38, 0.70) | 0.10% | 1.13 | 1.47 |
| COG4 | 1 | 2.32E-05 | 1.94 (1.43, 2.64) | 0.20% | 1.13 | 1.44 |
| COG4 | 1 | 2.32E-05 | 1.94 (1.43, 2.64) | 0.20% | 1.13 | 1.44 |
| CXCL13 | 1 | 1.08E-05 | 1.84 (1.40, 2.41) | 0.10% | 1.13 | 1.45 |
| FBP1 | -1 | 2.23E-05 | 0.53 (0.40, 0.71) | 0.20% | 1.13 | 1.45 |
| FBP1 | -1 | 2.23E-05 | 0.53 (0.40, 0.71) | 0.20% | 1.13 | 1.45 |
| FRMPD2 | -1 | 7.60E-06 | 0.57 (0.45, 0.73) | 0.10% | 1.13 | 1.46 |
| FRMPD2 | -1 | 7.60E-06 | 0.57 (0.45, 0.73) | 0.10% | 1.13 | 1.46 |
| GUCA2B | 1 | 4.56E-06 | 1.63 (1.32, 2.00) | 0.10% | 1.13 | 1.42 |
| LALBA | 1 | 1.41E-05 | 1.76 (1.36, 2.28) | 0.10% | 1.13 | 1.43 |
| LOC100131366 | 1 | 7.36E-06 | 1.70 (1.35, 2.14) | 0.10% | 1.13 | 1.43 |
| LRIG3 | 1 | 7.68E-06 | 1.70 (1.35, 2.15) | 0.10% | 1.13 | 1.43 |
| LRIG3 | 1 | 7.68E-06 | 1.70 (1.35, 2.15) | 0.10% | 1.13 | 1.43 |
| OLIG2 | 1 | 2.36E-05 | 2.09 (1.48, 2.94) | 0.20% | 1.13 | 1.44 |
| PADI3 | 1 | 1.40E-05 | 1.80 (1.38, 2.35) | 0.10% | 1.13 | 1.44 |
| PRICKLE4 | -1 | 1.17E-07 | 0.69 (0.60, 0.79) | <0.1% | 1.13 | 1.38 |
| RNF145 | 1 | 1.10E-05 | 1.80 (1.38, 2.33) | 0.10% | 1.13 | 1.44 |
| RNF145 | 1 | 1.10E-05 | 1.80 (1.38, 2.33) | 0.10% | 1.13 | 1.44 |
| RNF145 | 1 | 1.10E-05 | 1.80 (1.38, 2.33) | 0.10% | 1.13 | 1.44 |
| RNF145 | 1 | 1.10E-05 | 1.80 (1.38, 2.33) | 0.10% | 1.13 | 1.44 |
| RNF145 | 1 | 1.10E-05 | 1.80 (1.38, 2.33) | 0.10% | 1.13 | 1.44 |
| SNRPN | -1 | 7.19E-06 | 0.57 (0.45, 0.73) | 0.10% | 1.13 | 1.46 |
| SNRPN | -1 | 7.19E-06 | 0.57 (0.45, 0.73) | 0.10% | 1.13 | 1.46 |
| SNRPN | -1 | 7.19E-06 | 0.57 (0.45, 0.73) | 0.10% | 1.13 | 1.46 |
| SNRPN | -1 | 7.19E-06 | 0.57 (0.45, 0.73) | 0.10% | 1.13 | 1.46 |
| SNRPN | -1 | 7.19E-06 | 0.57 (0.45, 0.73) | 0.10% | 1.13 | 1.46 |
| SUSD3 | -1 | 1.88E-05 | 0.50 (0.37, 0.69) | 0.20% | 1.13 | 1.46 |
| TBX10 | 1 | 3.65E-06 | 1.61 (1.32, 1.98) | 0.10% | 1.13 | 1.42 |
| TRAIP | 1 | 4.20E-05 | 2.16 (1.49, 3.12) | 0.30% | 1.13 | 1.42 |
| UGT2B10 | 1 | 1.95E-07 | 1.47 (1.27, 1.70) | <0.1% | 1.13 | 1.38 |
| UGT2B10 | 1 | 1.95E-07 | 1.47 (1.27, 1.70) | <0.1% | 1.13 | 1.38 |
| UGT2B10 | 1 | 1.95E-07 | 1.47 (1.27, 1.70) | <0.1% | 1.13 | 1.38 |
| UGT2B10 | 1 | 1.95E-07 | 1.47 (1.27, 1.70) | <0.1% | 1.13 | 1.38 |
| ZNF214 | -1 | 6.93E-06 | 0.57 (0.44, 0.73) | 0.10% | 1.13 | 1.46 |
| ZNF835 | -1 | 4.54E-05 | 0.45 (0.31, 0.66) | 0.30% | 1.13 | 1.44 |
| ATAD2B | 1 | 0.000010614 | 1.61 (1.30, 1.99) | 0.10% | 1.12 | 1.41 |
| C10orf18 | 1 | 1.23E-05 | 1.61 (1.30, 2.00) | 0.10% | 1.12 | 1.41 |
| C14orf79 | -1 | 5.74E-05 | 0.51 (0.37, 0.71) | 0.40% | 1.12 | 1.43 |
| C9orf27 | 1 | 3.70E-06 | 1.51 (1.27, 1.79) | 0.10% | 1.12 | 1.38 |
| CALU | 1 | 1.63E-05 | 1.75 (1.35, 2.25) | 0.20% | 1.12 | 1.43 |
| CALU | 1 | 1.63E-05 | 1.75 (1.35, 2.25) | 0.20% | 1.12 | 1.43 |
| CALU | 1 | 1.63E-05 | 1.75 (1.35, 2.25) | 0.20% | 1.12 | 1.43 |
| CALU | 1 | 1.63E-05 | 1.75 (1.35, 2.25) | 0.20% | 1.12 | 1.43 |
| CALU | 1 | 1.63E-05 | 1.75 (1.35, 2.25) | 0.20% | 1.12 | 1.43 |
| CALU | 1 | 1.63E-05 | 1.75 (1.35, 2.25) | 0.20% | 1.12 | 1.43 |
| CARD18 | 1 | 4.65E-06 | 1.54 (1.28, 1.86) | 0.10% | 1.12 | 1.39 |
| CCL2 | -1 | 3.89E-05 | 0.53 (0.39, 0.72) | 0.30% | 1.12 | 1.44 |
| CELF6 | -1 | 7.61E-05 | 0.50 (0.35, 0.70) | 0.50% | 1.12 | 1.43 |
| CELF6 | -1 | 7.61E-05 | 0.50 (0.35, 0.70) | 0.50% | 1.12 | 1.43 |
| CELF6 | -1 | 7.61E-05 | 0.50 (0.35, 0.70) | 0.50% | 1.12 | 1.43 |
| CREBL2 | -1 | 2.02E-05 | 0.61 (0.48, 0.76) | 0.20% | 1.12 | 1.42 |
| CSHL1 | 1 | 1.03E-06 | 1.42 (1.23, 1.63) | <0.1% | 1.12 | 1.35 |
| CSHL1 | 1 | 1.03E-06 | 1.42 (1.23, 1.63) | <0.1% | 1.12 | 1.35 |
| CSHL1 | 1 | 1.03E-06 | 1.42 (1.23, 1.63) | <0.1% | 1.12 | 1.35 |
| CSHL1 | 1 | 1.03E-06 | 1.42 (1.23, 1.63) | <0.1% | 1.12 | 1.35 |
| CXorf64 | 1 | 1.85E-06 | 1.49 (1.26, 1.75) | <0.1% | 1.12 | 1.38 |
| DCDC5 | -1 | 0.000050342 | 0.49 (0.35, 0.69) | 0.30% | 1.12 | 1.44 |
| DDX42 | 1 | 3.64E-06 | 1.54 (1.28, 1.85) | 0.10% | 1.12 | 1.4 |
| DDX42 | 1 | 3.64E-06 | 1.54 (1.28, 1.85) | 0.10% | 1.12 | 1.4 |
| DLG1 | 1 | 1.70E-05 | 1.74 (1.35, 2.24) | 0.20% | 1.12 | 1.43 |
| DLG1 | 1 | 1.70E-05 | 1.74 (1.35, 2.24) | 0.20% | 1.12 | 1.43 |
| DSCR10 | 1 | 9.45E-07 | 1.48 (1.26, 1.73) | <0.1% | 1.12 | 1.38 |
| HIST1H2BK | 1 | 6.59E-05 | 2.00 (1.42, 2.82) | 0.40% | 1.12 | 1.41 |
| HRASLS5 | -1 | 2.85E-05 | 0.53 (0.39, 0.71) | 0.20% | 1.12 | 1.45 |
| HRASLS5 | -1 | 2.85E-05 | 0.53 (0.39, 0.71) | 0.20% | 1.12 | 1.45 |
| HRASLS5 | -1 | 2.85E-05 | 0.53 (0.39, 0.71) | 0.20% | 1.12 | 1.45 |
| INSIG1 | 1 | 6.08E-05 | 1.98 (1.42, 2.76) | 0.40% | 1.12 | 1.41 |
| INSIG1 | 1 | 6.08E-05 | 1.98 (1.42, 2.76) | 0.40% | 1.12 | 1.41 |
| INSIG1 | 1 | 6.08E-05 | 1.98 (1.42, 2.76) | 0.40% | 1.12 | 1.41 |
| IRF2BP2 | 1 | 4.85E-05 | 2.06 (1.45, 2.92) | 0.30% | 1.12 | 1.42 |
| IRF2BP2 | 1 | 4.85E-05 | 2.06 (1.45, 2.92) | 0.30% | 1.12 | 1.42 |
| ITLN2 | 1 | 5.74E-05 | 1.91 (1.40, 2.63) | 0.40% | 1.12 | 1.41 |
| KLHL23 | -1 | 3.95E-05 | 0.51 (0.37, 0.70) | 0.30% | 1.12 | 1.44 |
| KLHL23 | -1 | 3.95E-05 | 0.51 (0.37, 0.70) | 0.30% | 1.12 | 1.44 |
| LCE1C | 1 | 3.13E-06 | 1.48 (1.26, 1.74) | 0.10% | 1.12 | 1.37 |
| MTCH1 | 1 | 1.38E-06 | 1.45 (1.24, 1.68) | <0.1% | 1.12 | 1.36 |
| MYOF | -1 | 1.35E-05 | 0.62 (0.50, 0.77) | 0.10% | 1.12 | 1.42 |
| MYOF | -1 | 1.35E-05 | 0.62 (0.50, 0.77) | 0.10% | 1.12 | 1.42 |
| NHSL1 | 1 | 3.34E-05 | 1.87 (1.39, 2.51) | 0.30% | 1.12 | 1.43 |
| NHSL1 | 1 | 3.34E-05 | 1.87 (1.39, 2.51) | 0.30% | 1.12 | 1.43 |
| NRM | -1 | 5.27E-05 | 0.52 (0.38, 0.71) | 0.40% | 1.12 | 1.43 |
| NSD1 | 1 | 1.13E-06 | 1.49 (1.27, 1.74) | <0.1% | 1.12 | 1.38 |
| NSD1 | 1 | 1.13E-06 | 1.49 (1.27, 1.74) | <0.1% | 1.12 | 1.38 |
| NUP54 | 1 | 1.51E-06 | 1.47 (1.26, 1.73) | <0.1% | 1.12 | 1.37 |
| PDXDC2P | 1 | 6.41E-05 | 1.94 (1.40, 2.69) | 0.40% | 1.12 | 1.41 |
| PIR | 1 | 4.31E-05 | 1.96 (1.42, 2.70) | 0.30% | 1.12 | 1.42 |
| PIR | 1 | 4.31E-05 | 1.96 (1.42, 2.70) | 0.30% | 1.12 | 1.42 |
| PLEKHM1P | 1 | 7.39E-06 | 1.54 (1.28, 1.86) | 0.10% | 1.12 | 1.39 |
| PRRC1 | 1 | 6.79E-05 | 2.10 (1.46, 3.02) | 0.40% | 1.12 | 1.41 |
| PSMG3 | 1 | 0.000078564 | 2.30 (1.52, 3.48) | 0.50% | 1.12 | 1.4 |
| PSMG3 | 1 | 0.000078564 | 2.30 (1.52, 3.48) | 0.50% | 1.12 | 1.4 |
| PYY | -1 | 5.43E-05 | 0.49 (0.34, 0.69) | 0.40% | 1.12 | 1.44 |
| RAX | 1 | 3.33E-05 | 1.75 (1.35, 2.29) | 0.30% | 1.12 | 1.42 |
| RTN1 | -1 | 5.29E-05 | 0.47 (0.33, 0.68) | 0.40% | 1.12 | 1.44 |
| RTN1 | -1 | 5.29E-05 | 0.47 (0.33, 0.68) | 0.40% | 1.12 | 1.44 |
| RTN1 | -1 | 5.29E-05 | 0.47 (0.33, 0.68) | 0.40% | 1.12 | 1.44 |
| SLC35D2 | -1 | 6.31E-06 | 0.62 (0.51, 0.77) | 0.10% | 1.12 | 1.42 |
| SLC7A5 | 1 | 3.32E-05 | 1.88 (1.40, 2.53) | 0.30% | 1.12 | 1.43 |
| SQSTM1 | 1 | 2.49E-05 | 1.78 (1.36, 2.33) | 0.20% | 1.12 | 1.43 |
| SQSTM1 | 1 | 2.49E-05 | 1.78 (1.36, 2.33) | 0.20% | 1.12 | 1.43 |
| SQSTM1 | 1 | 2.49E-05 | 1.78 (1.36, 2.33) | 0.20% | 1.12 | 1.43 |
| TBX4 | 1 | 3.19E-05 | 1.81 (1.37, 2.39) | 0.30% | 1.12 | 1.42 |
| TKT | 1 | 4.20E-05 | 1.81 (1.36, 2.41) | 0.30% | 1.12 | 1.42 |
| TKT | 1 | 4.20E-05 | 1.81 (1.36, 2.41) | 0.30% | 1.12 | 1.42 |
| TMEM26 | -1 | 2.62E-05 | 0.55 (0.41, 0.73) | 0.20% | 1.12 | 1.44 |
| TMEM8B | -1 | 0.000067009 | 0.50 (0.36, 0.70) | 0.40% | 1.12 | 1.43 |
| TMEM8B | -1 | 0.000067009 | 0.50 (0.36, 0.70) | 0.40% | 1.12 | 1.43 |
| TMEM8B | -1 | 0.000067009 | 0.50 (0.36, 0.70) | 0.40% | 1.12 | 1.43 |
| USP8 | 1 | 3.60E-09 | 1.29 (1.18, 1.40) | <0.1% | 1.12 | 1.27 |
| USP8 | 1 | 3.60E-09 | 1.29 (1.18, 1.40) | <0.1% | 1.12 | 1.27 |
| USP8 | 1 | 3.60E-09 | 1.29 (1.18, 1.40) | <0.1% | 1.12 | 1.27 |
| WDR70 | 1 | 4.01E-05 | 1.98 (1.43, 2.73) | 0.30% | 1.12 | 1.42 |
| WDR78 | -1 | 1.82E-05 | 0.58 (0.45, 0.74) | 0.20% | 1.12 | 1.44 |
| WDR78 | -1 | 1.82E-05 | 0.58 (0.45, 0.74) | 0.20% | 1.12 | 1.44 |
| ZNF433 | -1 | 4.82E-06 | 0.63 (0.52, 0.77) | 0.10% | 1.12 | 1.42 |
| ZNF563 | -1 | 1.21E-05 | 0.59 (0.47, 0.75) | 0.10% | 1.12 | 1.44 |
| ZNF788 | -1 | 2.72E-05 | 0.59 (0.46, 0.75) | 0.20% | 1.12 | 1.43 |
| ZNF836 | -1 | 4.08E-05 | 0.51 (0.37, 0.71) | 0.30% | 1.12 | 1.44 |
| AK8 | -1 | 5.55E-05 | 0.54 (0.40, 0.73) | 0.40% | 1.11 | 1.43 |
| C11orf70 | -1 | 0.000056385 | 0.59 (0.46, 0.76) | 0.40% | 1.11 | 1.41 |
| C11orf70 | -1 | 0.000056385 | 0.59 (0.46, 0.76) | 0.40% | 1.11 | 1.41 |
| C15orf23 | 1 | 0.000143369 | 2.27 (1.49, 3.46) | 0.70% | 1.11 | 1.38 |
| C15orf23 | 1 | 0.000143369 | 2.27 (1.49, 3.46) | 0.70% | 1.11 | 1.38 |
| C15orf23 | 1 | 0.000143369 | 2.27 (1.49, 3.46) | 0.70% | 1.11 | 1.38 |
| C16orf52 | -1 | 8.04E-05 | 0.54 (0.40, 0.73) | 0.50% | 1.11 | 1.42 |
| C1orf168 | -1 | 0.000132664 | 0.50 (0.35, 0.71) | 0.70% | 1.11 | 1.41 |
| C20orf26 | -1 | 0.000125264 | 0.46 (0.31, 0.69) | 0.60% | 1.11 | 1.41 |
| C20orf26 | -1 | 0.000125264 | 0.46 (0.31, 0.69) | 0.60% | 1.11 | 1.41 |
| CX3CR1 | -1 | 6.03E-05 | 0.54 (0.40, 0.73) | 0.40% | 1.11 | 1.43 |
| CX3CR1 | -1 | 6.03E-05 | 0.54 (0.40, 0.73) | 0.40% | 1.11 | 1.43 |
| CX3CR1 | -1 | 6.03E-05 | 0.54 (0.40, 0.73) | 0.40% | 1.11 | 1.43 |
| CX3CR1 | -1 | 6.03E-05 | 0.54 (0.40, 0.73) | 0.40% | 1.11 | 1.43 |
| DAB1 | -1 | 0.000102041 | 0.52 (0.38, 0.73) | 0.60% | 1.11 | 1.42 |
| DMRTC2 | 1 | 5.88E-05 | 1.70 (1.31, 2.20) | 0.40% | 1.11 | 1.4 |
| FAM98A | 1 | 0.000152723 | 2.15 (1.45, 3.20) | 0.70% | 1.11 | 1.38 |
| FAM9C | 1 | 7.08E-07 | 1.39 (1.22, 1.58) | <0.1% | 1.11 | 1.33 |
| FBXO15 | -1 | 0.00014207 | 0.49 (0.34, 0.71) | 0.70% | 1.11 | 1.41 |
| FBXO15 | -1 | 0.00014207 | 0.49 (0.34, 0.71) | 0.70% | 1.11 | 1.41 |
| GABPB1 | 1 | 3.07E-07 | 1.32 (1.19, 1.47) | <0.1% | 1.11 | 1.29 |
| GABPB1 | 1 | 3.07E-07 | 1.32 (1.19, 1.47) | <0.1% | 1.11 | 1.29 |
| GABPB1 | 1 | 3.07E-07 | 1.32 (1.19, 1.47) | <0.1% | 1.11 | 1.29 |
| GABPB1 | 1 | 3.07E-07 | 1.32 (1.19, 1.47) | <0.1% | 1.11 | 1.29 |
| GABPB1 | 1 | 3.07E-07 | 1.32 (1.19, 1.47) | <0.1% | 1.11 | 1.29 |
| GARS | 1 | 0.000025817 | 1.62 (1.29, 2.03) | 0.20% | 1.11 | 1.4 |
| GLRA1 | 1 | 4.64E-05 | 1.66 (1.30, 2.12) | 0.30% | 1.11 | 1.4 |
| GLRA1 | 1 | 4.64E-05 | 1.66 (1.30, 2.12) | 0.30% | 1.11 | 1.4 |
| GOLPH3 | 1 | 0.000116993 | 1.95 (1.39, 2.74) | 0.60% | 1.11 | 1.39 |
| GP9 | 1 | 1.93E-07 | 1.34 (1.20, 1.49) | <0.1% | 1.11 | 1.3 |
| GPRIN1 | 1 | 9.56E-05 | 2.00 (1.41, 2.84) | 0.50% | 1.11 | 1.4 |
| IGFBP3 | 1 | 0.000195883 | 2.33 (1.49, 3.64) | 0.90% | 1.11 | 1.36 |
| IGFBP3 | 1 | 0.000195883 | 2.33 (1.49, 3.64) | 0.90% | 1.11 | 1.36 |
| KCNJ5 | -1 | 9.79E-05 | 0.50 (0.35, 0.71) | 0.50% | 1.11 | 1.42 |
| KLHL5 | -1 | 0.000134571 | 0.49 (0.34, 0.71) | 0.70% | 1.11 | 1.41 |
| KLHL5 | -1 | 0.000134571 | 0.49 (0.34, 0.71) | 0.70% | 1.11 | 1.41 |
| KLHL5 | -1 | 0.000134571 | 0.49 (0.34, 0.71) | 0.70% | 1.11 | 1.41 |
| KLHL5 | -1 | 0.000134571 | 0.49 (0.34, 0.71) | 0.70% | 1.11 | 1.41 |
| KRT27 | 1 | 1.79E-05 | 1.54 (1.27, 1.88) | 0.20% | 1.11 | 1.38 |
| LMAN1L | 1 | 0.000147619 | 2.05 (1.41, 2.96) | 0.70% | 1.11 | 1.38 |
| LOC100271722 | -1 | 2.89E-05 | 0.64 (0.52, 0.79) | 0.20% | 1.11 | 1.39 |
| LYL1 | -1 | 4.58E-05 | 0.58 (0.45, 0.75) | 0.30% | 1.11 | 1.42 |
| MAD1L1 | 1 | 0.000102969 | 1.87 (1.36, 2.56) | 0.60% | 1.11 | 1.4 |
| MAD1L1 | 1 | 0.000102969 | 1.87 (1.36, 2.56) | 0.60% | 1.11 | 1.4 |
| MAD1L1 | 1 | 0.000102969 | 1.87 (1.36, 2.56) | 0.60% | 1.11 | 1.4 |
| MAPK6 | 1 | 1.97E-07 | 1.33 (1.20, 1.48) | <0.1% | 1.11 | 1.3 |
| MRPS26 | -1 | 6.20E-06 | 0.70 (0.60, 0.82) | 0.10% | 1.11 | 1.35 |
| NAGS | -1 | 0.00010598 | 0.50 (0.36, 0.71) | 0.60% | 1.11 | 1.42 |
| OTP | 1 | 3.84E-05 | 1.62 (1.29, 2.05) | 0.30% | 1.11 | 1.39 |
| PINK1 | -1 | 7.63E-05 | 0.53 (0.39, 0.73) | 0.50% | 1.11 | 1.42 |
| RESP18 | 1 | 6.15E-06 | 1.46 (1.24, 1.72) | 0.10% | 1.11 | 1.36 |
| SLC22A6 | 1 | 5.02E-05 | 1.82 (1.36, 2.42) | 0.30% | 1.11 | 1.41 |
| SLC22A6 | 1 | 5.02E-05 | 1.82 (1.36, 2.42) | 0.30% | 1.11 | 1.41 |
| SLC22A6 | 1 | 5.02E-05 | 1.82 (1.36, 2.42) | 0.30% | 1.11 | 1.41 |
| SLC22A6 | 1 | 5.02E-05 | 1.82 (1.36, 2.42) | 0.30% | 1.11 | 1.41 |
| SPATC1 | 1 | 0.000142376 | 2.22 (1.47, 3.36) | 0.70% | 1.11 | 1.38 |
| SPATC1 | 1 | 0.000142376 | 2.22 (1.47, 3.36) | 0.70% | 1.11 | 1.38 |
| TBX19 | 1 | 5.35E-05 | 1.76 (1.34, 2.32) | 0.40% | 1.11 | 1.41 |
| TNFRSF14 | -1 | 0.000166546 | 0.47 (0.31, 0.69) | 0.80% | 1.11 | 1.4 |
| TRPM7 | 1 | 7.91E-09 | 1.27 (1.17, 1.38) | <0.1% | 1.11 | 1.25 |
| TSC22D1 | 1 | 0.000124731 | 2.10 (1.44, 3.07) | 0.60% | 1.11 | 1.39 |
| TSC22D1 | 1 | 0.000124731 | 2.10 (1.44, 3.07) | 0.60% | 1.11 | 1.39 |
| WWP2 | 1 | 2.60E-05 | 1.60 (1.29, 2.00) | 0.20% | 1.11 | 1.39 |
| WWP2 | 1 | 2.60E-05 | 1.60 (1.29, 2.00) | 0.20% | 1.11 | 1.39 |
| WWP2 | 1 | 2.60E-05 | 1.60 (1.29, 2.00) | 0.20% | 1.11 | 1.39 |
| ZNF136 | -1 | 0.000105463 | 0.49 (0.35, 0.71) | 0.60% | 1.11 | 1.42 |
| ZNF175 | -1 | 5.57E-05 | 0.56 (0.42, 0.74) | 0.40% | 1.11 | 1.42 |
| ZNF396 | -1 | 2.46E-05 | 0.61 (0.48, 0.76) | 0.20% | 1.11 | 1.42 |
| ZNF429 | -1 | 0.000110212 | 0.51 (0.36, 0.72) | 0.60% | 1.11 | 1.41 |
| ZNF491 | -1 | 1.25E-05 | 0.66 (0.55, 0.80) | 0.10% | 1.11 | 1.38 |
| ZNF721 | -1 | 6.35E-05 | 0.57 (0.43, 0.75) | 0.40% | 1.11 | 1.42 |
| ZNF79 | -1 | 1.31E-05 | 0.64 (0.53, 0.78) | 0.10% | 1.11 | 1.4 |
| ACSBG2 | -1 | 0.000112734 | 0.58 (0.44, 0.76) | 0.60% | 1.1 | 1.4 |
| ADCK5 | 1 | 0.000108958 | 1.77 (1.33, 2.37) | 0.60% | 1.1 | 1.39 |
| ANKS6 | 1 | 0.000189078 | 1.98 (1.38, 2.83) | 0.90% | 1.1 | 1.38 |
| ARMC9 | -1 | 4.50E-05 | 0.62 (0.49, 0.78) | 0.30% | 1.1 | 1.4 |
| ATP1A3 | 1 | 0.000078271 | 1.65 (1.29, 2.12) | 0.50% | 1.1 | 1.38 |
| ATP7B | -1 | 8.98E-05 | 0.59 (0.45, 0.77) | 0.50% | 1.1 | 1.4 |
| ATP7B | -1 | 8.98E-05 | 0.59 (0.45, 0.77) | 0.50% | 1.1 | 1.4 |
| BGN | -1 | 4.71E-05 | 0.65 (0.53, 0.80) | 0.30% | 1.1 | 1.38 |
| C1orf146 | -1 | 7.80E-05 | 0.59 (0.45, 0.76) | 0.50% | 1.1 | 1.41 |
| CFHR5 | 1 | 0.000144896 | 1.85 (1.35, 2.54) | 0.70% | 1.1 | 1.39 |
| CREB3 | -1 | 4.27E-05 | 0.64 (0.52, 0.79) | 0.30% | 1.1 | 1.38 |
| CROCC | -1 | 0.000170204 | 0.52 (0.37, 0.73) | 0.80% | 1.1 | 1.4 |
| DNAH7 | -1 | 9.52E-05 | 0.56 (0.41, 0.75) | 0.50% | 1.1 | 1.41 |
| DSCR4 | 1 | 5.00E-05 | 1.64 (1.29, 2.09) | 0.30% | 1.1 | 1.39 |
| EGLN1 | 1 | 9.70E-05 | 1.80 (1.34, 2.41) | 0.50% | 1.1 | 1.39 |
| GGCT | 1 | 0.000050126 | 1.56 (1.26, 1.94) | 0.30% | 1.1 | 1.37 |
| GGCT | 1 | 0.000050126 | 1.56 (1.26, 1.94) | 0.30% | 1.1 | 1.37 |
| GGCT | 1 | 0.000050126 | 1.56 (1.26, 1.94) | 0.30% | 1.1 | 1.37 |
| GGCT | 1 | 0.000050126 | 1.56 (1.26, 1.94) | 0.30% | 1.1 | 1.37 |
| GGCT | 1 | 0.000050126 | 1.56 (1.26, 1.94) | 0.30% | 1.1 | 1.37 |
| GRHL2 | 1 | 0.000165793 | 2.03 (1.40, 2.93) | 0.80% | 1.1 | 1.38 |
| GRIK4 | -1 | 0.000250003 | 0.43 (0.27, 0.67) | 1.00% | 1.1 | 1.38 |
| HIP1 | -1 | 4.88E-05 | 0.63 (0.50, 0.79) | 0.30% | 1.1 | 1.39 |
| INCENP | 1 | 0.000223059 | 2.03 (1.39, 2.96) | 0.90% | 1.1 | 1.37 |
| INCENP | 1 | 0.000223059 | 2.03 (1.39, 2.96) | 0.90% | 1.1 | 1.37 |
| KCNH7 | 1 | 1.43E-05 | 1.45 (1.23, 1.72) | 0.10% | 1.1 | 1.35 |
| KCNH7 | 1 | 1.43E-05 | 1.45 (1.23, 1.72) | 0.10% | 1.1 | 1.35 |
| KMO | 1 | 0.000124999 | 1.75 (1.32, 2.33) | 0.60% | 1.1 | 1.39 |
| LCE3E | 1 | 1.98E-06 | 1.35 (1.19, 1.53) | <0.1% | 1.1 | 1.3 |
| LOC100130950 | -1 | 0.000224114 | 0.48 (0.33, 0.71) | 1.00% | 1.1 | 1.39 |
| LYPD6B | -1 | 0.000130737 | 0.56 (0.41, 0.75) | 0.70% | 1.1 | 1.4 |
| MAGEA5 | 1 | 2.64E-05 | 1.50 (1.24, 1.81) | 0.20% | 1.1 | 1.36 |
| MYO1C | -1 | 0.000148018 | 0.52 (0.37, 0.73) | 0.70% | 1.1 | 1.41 |
| MYO1C | -1 | 0.000148018 | 0.52 (0.37, 0.73) | 0.70% | 1.1 | 1.41 |
| MYO1C | -1 | 0.000148018 | 0.52 (0.37, 0.73) | 0.70% | 1.1 | 1.41 |
| NDRG1 | 1 | 0.00006342 | 1.60 (1.27, 2.02) | 0.40% | 1.1 | 1.38 |
| NDRG1 | 1 | 0.00006342 | 1.60 (1.27, 2.02) | 0.40% | 1.1 | 1.38 |
| ONECUT2 | 1 | 0.000116006 | 1.87 (1.36, 2.57) | 0.60% | 1.1 | 1.39 |
| PAIP1 | 1 | 3.95E-05 | 1.61 (1.28, 2.02) | 0.30% | 1.1 | 1.39 |
| PAIP1 | 1 | 3.95E-05 | 1.61 (1.28, 2.02) | 0.30% | 1.1 | 1.39 |
| PAIP1 | 1 | 3.95E-05 | 1.61 (1.28, 2.02) | 0.30% | 1.1 | 1.39 |
| RAPGEF5 | 1 | 7.22E-05 | 1.71 (1.31, 2.22) | 0.40% | 1.1 | 1.39 |
| RNASE2 | 1 | 1.62E-05 | 1.48 (1.24, 1.76) | 0.20% | 1.1 | 1.36 |
| SCARB2 | 1 | 2.26E-05 | 1.47 (1.23, 1.77) | 0.20% | 1.1 | 1.35 |
| SEC14L3 | 1 | 1.41E-05 | 1.47 (1.23, 1.74) | 0.10% | 1.1 | 1.35 |
| SELO | 1 | 3.85E-06 | 1.37 (1.20, 1.57) | 0.10% | 1.1 | 1.31 |
| SLC15A1 | 1 | 0.000192161 | 2.05 (1.41, 2.99) | 0.90% | 1.1 | 1.38 |
| SUTRK5 | 1 | 0.000119475 | 1.86 (1.35, 2.55) | 0.60% | 1.1 | 1.39 |
| SPINK5 | 1 | 0.00014485 | 1.79 (1.33, 2.42) | 0.70% | 1.1 | 1.38 |
| SPINK5 | 1 | 0.00014485 | 1.79 (1.33, 2.42) | 0.70% | 1.1 | 1.38 |
| SPINK5 | 1 | 0.00014485 | 1.79 (1.33, 2.42) | 0.70% | 1.1 | 1.38 |
| TMEM164 | -1 | 0.000184554 | 0.51 (0.36, 0.72) | 0.80% | 1.1 | 1.4 |
| TMEM164 | -1 | 0.000184554 | 0.51 (0.36, 0.72) | 0.80% | 1.1 | 1.4 |
| TMOD3 | 1 | 4.02E-08 | 1.27 (1.17, 1.38) | <0.1% | 1.1 | 1.25 |
| TRIM32 | -1 | 0.000162106 | 0.53 (0.38, 0.74) | 0.80% | 1.1 | 1.4 |
| TRIM32 | -1 | 0.000162106 | 0.53 (0.38, 0.74) | 0.80% | 1.1 | 1.4 |
| TRIP13 | 1 | 0.00024036 | 2.25 (1.46, 3.47) | 1.00% | 1.1 | 1.36 |
| TRIP13 | 1 | 0.00024036 | 2.25 (1.46, 3.47) | 1.00% | 1.1 | 1.36 |
| ZNF347 | -1 | 0.000123774 | 0.55 (0.40, 0.74) | 0.60% | 1.1 | 1.41 |
| ZNF347 | -1 | 0.000123774 | 0.55 (0.40, 0.74) | 0.60% | 1.1 | 1.41 |
| ZNF347 | -1 | 0.000123774 | 0.55 (0.40, 0.74) | 0.60% | 1.1 | 1.41 |
| BCAR3 | -1 | 0.000312727 | 0.49 (0.33, 0.72) | 1.20% | 1.09 | 1.38 |
| BCL2 | -1 | 0.000271683 | 0.53 (0.38, 0.75) | 1.10% | 1.09 | 1.39 |
| BCL2 | -1 | 0.000271683 | 0.53 (0.38, 0.75) | 1.10% | 1.09 | 1.39 |
| BCL7C | -1 | 0.00023086 | 0.53 (0.38, 0.74) | 1.00% | 1.09 | 1.39 |
| BICD2 | -1 | 0.000133159 | 0.57 (0.43, 0.76) | 0.70% | 1.09 | 1.4 |
| BICD2 | -1 | 0.000133159 | 0.57 (0.43, 0.76) | 0.70% | 1.09 | 1.4 |
| C18orf54 | 1 | 0.000202177 | 1.74 (1.30, 2.34) | 0.90% | 1.09 | 1.37 |
| CA10 | 1 | 1.34E-05 | 1.37 (1.19, 1.57) | 0.10% | 1.09 | 1.3 |
| CA10 | 1 | 1.34E-05 | 1.37 (1.19, 1.57) | 0.10% | 1.09 | 1.3 |
| CA10 | 1 | 1.34E-05 | 1.37 (1.19, 1.57) | 0.10% | 1.09 | 1.3 |
| CLIC6 | -1 | 0.000542023 | 0.38 (0.22, 0.66) | 1.70% | 1.09 | 1.33 |
| EFCAB6 | -1 | 0.000168016 | 0.59 (0.44, 0.77) | 0.80% | 1.09 | 1.39 |
| EFCAB6 | -1 | 0.000168016 | 0.59 (0.44, 0.77) | 0.80% | 1.09 | 1.39 |
| EIF2B2 | -1 | 0.000117388 | 0.59 (0.45, 0.77) | 0.60% | 1.09 | 1.4 |
| ENO2 | -1 | 0.000134221 | 0.58 (0.44, 0.77) | 0.70% | 1.09 | 1.4 |
| FAM66C | -1 | 0.000163471 | 0.59 (0.45, 0.78) | 0.80% | 1.09 | 1.39 |
| FANK1 | -1 | 0.000257189 | 0.54 (0.39, 0.75) | 1.00% | 1.09 | 1.39 |
| FGFRL1 | -1 | 0.000111716 | 0.60 (0.46, 0.78) | 0.60% | 1.09 | 1.39 |
| FGFRL1 | -1 | 0.000111716 | 0.60 (0.46, 0.78) | 0.60% | 1.09 | 1.39 |
| FGFRL1 | -1 | 0.000111716 | 0.60 (0.46, 0.78) | 0.60% | 1.09 | 1.39 |
| FOXH1 | 1 | 2.16E-05 | 1.39 (1.20, 1.62) | 0.20% | 1.09 | 1.32 |
| HIF1A | 1 | 0.000181495 | 1.79 (1.32, 2.43) | 0.80% | 1.09 | 1.38 |
| HIF1A | 1 | 0.000181495 | 1.79 (1.32, 2.43) | 0.80% | 1.09 | 1.38 |
| HJURP | 1 | 0.000352551 | 2.12 (1.40, 3.20) | 1.30% | 1.09 | 1.35 |
| LOC642345 | 1 | 0.000195592 | 1.75 (1.30, 2.35) | 0.90% | 1.09 | 1.37 |
| LOX | 1 | 0.000192189 | 1.82 (1.33, 2.50) | 0.90% | 1.09 | 1.38 |
| LOX | 1 | 0.000192189 | 1.82 (1.33, 2.50) | 0.90% | 1.09 | 1.38 |
| LRIT2 | 1 | 2.01E-06 | 1.31 (1.17, 1.46) | <0.1% | 1.09 | 1.27 |
| MYF5 | 1 | 7.41E-06 | 1.34 (1.18, 1.52) | 0.10% | 1.09 | 1.29 |
| MYH2 | -1 | 7.14E-05 | 0.63 (0.51, 0.79) | 0.40% | 1.09 | 1.38 |
| MYH2 | -1 | 7.14E-05 | 0.63 (0.51, 0.79) | 0.40% | 1.09 | 1.38 |
| NAA35 | -1 | 0.000347317 | 0.48 (0.32, 0.72) | 1.30% | 1.09 | 1.38 |
| NCRNA00221 | 1 | 3.23E-05 | 1.42 (1.20, 1.67) | 0.30% | 1.09 | 1.33 |
| NINJ1 | -1 | 0.000222676 | 0.54 (0.38, 0.75) | 0.90% | 1.09 | 1.39 |
| NPY1R | -1 | 0.000241391 | 0.51 (0.35, 0.73) | 1.00% | 1.09 | 1.39 |
| NR1I2 | -1 | 0.000242263 | 0.55 (0.40, 0.75) | 1.00% | 1.09 | 1.39 |
| NR1I2 | -1 | 0.000242263 | 0.55 (0.40, 0.75) | 1.00% | 1.09 | 1.39 |
| NR1I2 | -1 | 0.000242263 | 0.55 (0.40, 0.75) | 1.00% | 1.09 | 1.39 |
| NUP205 | 1 | 0.000108468 | 1.57 (1.25, 1.96) | 0.60% | 1.09 | 1.36 |
| RAI2 | -1 | 0.000402941 | 0.44 (0.28, 0.70) | 1.40% | 1.09 | 1.36 |
| RAI2 | -1 | 0.000402941 | 0.44 (0.28, 0.70) | 1.40% | 1.09 | 1.36 |
| RAI2 | -1 | 0.000402941 | 0.44 (0.28, 0.70) | 1.40% | 1.09 | 1.36 |
| RAI2 | -1 | 0.000402941 | 0.44 (0.28, 0.70) | 1.40% | 1.09 | 1.36 |
| RAI2 | -1 | 0.000402941 | 0.44 (0.28, 0.70) | 1.40% | 1.09 | 1.36 |
| RAI2 | -1 | 0.000402941 | 0.44 (0.28, 0.70) | 1.40% | 1.09 | 1.36 |
| RDH10 | 1 | 0.000149274 | 1.79 (1.33, 2.42) | 0.70% | 1.09 | 1.38 |
| RP1-177G6-2 | 1 | 2.00E-06 | 1.29 (1.16, 1.43) | <0.1% | 1.09 | 1.26 |
| RP1-177G6-2 | 1 | 2.00E-06 | 1.29 (1.16, 1.43) | <0.1% | 1.09 | 1.26 |
| SCD | 1 | 0.000258891 | 2.04 (1.39, 2.99) | 1.00% | 1.09 | 1.37 |
| SREBF2 | 1 | 0.000116715 | 1.66 (1.28, 2.15) | 0.60% | 1.09 | 1.38 |
| TCEA3 | -1 | 0.000146171 | 0.60 (0.46, 0.78) | 0.70% | 1.09 | 1.39 |
| TROAP | 1 | 0.000469135 | 2.65 (1.53, 4.57) | 1.60% | 1.09 | 1.31 |
| TROAP | 1 | 0.000469135 | 2.65 (1.53, 4.57) | 1.60% | 1.09 | 1.31 |
| UIMC1 | 1 | 4.35E-05 | 1.47 (1.22, 1.77) | 0.30% | 1.09 | 1.34 |
| UIMC1 | 1 | 4.35E-05 | 1.47 (1.22, 1.77) | 0.30% | 1.09 | 1.34 |
| UIMC1 | 1 | 4.35E-05 | 1.47 (1.22, 1.77) | 0.30% | 1.09 | 1.34 |
| XPA | -1 | 0.000185424 | 0.57 (0.43, 0.77) | 0.80% | 1.09 | 1.39 |
| XPA | -1 | 0.000185424 | 0.57 (0.43, 0.77) | 0.80% | 1.09 | 1.39 |
| YWHAZ | 1 | 0.000084608 | 1.54 (1.24, 1.91) | 0.50% | 1.09 | 1.36 |
| YWHAZ | 1 | 0.000084608 | 1.54 (1.24, 1.91) | 0.50% | 1.09 | 1.36 |
| YWHAZ | 1 | 0.000084608 | 1.54 (1.24, 1.91) | 0.50% | 1.09 | 1.36 |
| YWHAZ | 1 | 0.000084608 | 1.54 (1.24, 1.91) | 0.50% | 1.09 | 1.36 |
| YWHAZ | 1 | 0.000084608 | 1.54 (1.24, 1.91) | 0.50% | 1.09 | 1.36 |
| YWHAZ | 1 | 0.000084608 | 1.54 (1.24, 1.91) | 0.50% | 1.09 | 1.36 |
| ZNF626 | -1 | 0.000106466 | 0.61 (0.48, 0.78) | 0.60% | 1.09 | 1.39 |
| ZNF626 | -1 | 0.000106466 | 0.61 (0.48, 0.78) | 0.60% | 1.09 | 1.39 |
| ZNF709 | -1 | 0.000178037 | 0.56 (0.41, 0.76) | 0.80% | 1.09 | 1.4 |
| ADHFE1 | -1 | 0.000429195 | 0.51 (0.35, 0.74) | 1.50% | 1.08 | 1.37 |
| AP1G1 | 1 | 0.000239608 | 1.74 (1.29, 2.33) | 1.00% | 1.08 | 1.37 |
| AP1G1 | 1 | 0.000239608 | 1.74 (1.29, 2.33) | 1.00% | 1.08 | 1.37 |
| AP4E1 | 1 | 6.06E-06 | 1.28 (1.15, 1.42) | 0.10% | 1.08 | 1.25 |
| BRD7 | 1 | 0.000271783 | 1.72 (1.29, 2.31) | 1.10% | 1.08 | 1.36 |
| BRD7 | 1 | 0.000271783 | 1.72 (1.29, 2.31) | 1.10% | 1.08 | 1.36 |
| C11orf24 | 1 | 0.000366903 | 1.84 (1.32, 2.57) | 1.30% | 1.08 | 1.36 |
| C11orf40 | 1 | 0.00012651 | 1.53 (1.23, 1.91) | 0.60% | 1.08 | 1.35 |
| C15orf42 | 1 | 0.000366951 | 1.89 (1.33, 2.68) | 1.30% | 1.08 | 1.36 |
| C17orf68 | -1 | 0.000267187 | 0.55 (0.40, 0.76) | 1.10% | 1.08 | 1.39 |
| C17orf85 | -1 | 0.000408898 | 0.49 (0.33, 0.73) | 1.40% | 1.08 | 1.37 |
| C17orf85 | -1 | 0.000408898 | 0.49 (0.33, 0.73) | 1.40% | 1.08 | 1.37 |
| C18orf26 | 1 | 0.000269333 | 1.73 (1.29, 2.33) | 1.10% | 1.08 | 1.36 |
| C2orf34 | 1 | 0.000480591 | 2.01 (1.36, 2.96) | 1.60% | 1.08 | 1.35 |
| CALHM2 | -1 | 0.000105504 | 0.65 (0.53, 0.81) | 0.60% | 1.08 | 1.36 |
| CALHM2 | -1 | 0.000105504 | 0.65 (0.53, 0.81) | 0.60% | 1.08 | 1.36 |
| CNGB3 | 1 | 0.000314415 | 1.85 (1.32, 2.58) | 1.20% | 1.08 | 1.36 |
| FLJ21408 | -1 | 0.00017901 | 0.61 (0.47, 0.79) | 0.80% | 1.08 | 1.38 |
| GALK2 | 1 | 0.000248814 | 1.80 (1.31, 2.47) | 1.00% | 1.08 | 1.37 |
| GALK2 | 1 | 0.000248814 | 1.80 (1.31, 2.47) | 1.00% | 1.08 | 1.37 |
| GNB2L1 | 1 | 0.000244751 | 1.77 (1.30, 2.40) | 1.00% | 1.08 | 1.37 |
| HAUS1 | -1 | 0.000455791 | 0.46 (0.30, 0.71) | 1.50% | 1.08 | 1.36 |
| HAUS1 | -1 | 0.000455791 | 0.46 (0.30, 0.71) | 1.50% | 1.08 | 1.36 |
| IL17F | 1 | 7.09E-05 | 1.42 (1.19, 1.69) | 0.40% | 1.08 | 1.32 |
| KIAA0174 | 1 | 0.000369657 | 1.91 (1.34, 2.73) | 1.30% | 1.08 | 1.36 |
| KLF3 | 1 | 0.000371138 | 1.81 (1.31, 2.52) | 1.30% | 1.08 | 1.36 |
| LDLRAD3 | -1 | 0.00028695 | 0.55 (0.39, 0.76) | 1.10% | 1.08 | 1.38 |
| LOC100302640 | -1 | 0.000386012 | 0.50 (0.35, 0.74) | 1.40% | 1.08 | 1.38 |
| MAP3K14 | -1 | 0.000279615 | 0.55 (0.40, 0.76) | 1.10% | 1.08 | 1.38 |
| MVP | -1 | 0.000337882 | 0.55 (0.40, 0.76) | 1.20% | 1.08 | 1.38 |
| MVP | -1 | 0.000337882 | 0.55 (0.40, 0.76) | 1.20% | 1.08 | 1.38 |
| NEUROD2 | 1 | 8.72E-05 | 1.49 (1.22, 1.82) | 0.50% | 1.08 | 1.34 |
| NFXL1 | 1 | 9.20E-05 | 1.51 (1.23, 1.86) | 0.50% | 1.08 | 1.35 |
| NXF5 | -1 | 0.000312889 | 0.54 (0.39, 0.76) | 1.20% | 1.08 | 1.38 |
| NXF5 | -1 | 0.000312889 | 0.54 (0.39, 0.76) | 1.20% | 1.08 | 1.38 |
| PABPN1L | 1 | 7.99E-05 | 1.46 (1.21, 1.76) | 0.50% | 1.08 | 1.33 |
| PACSIN2 | 1 | 0.000313177 | 1.80 (1.31, 2.48) | 1.20% | 1.08 | 1.36 |
| PACSIN2 | 1 | 0.000313177 | 1.80 (1.31, 2.48) | 1.20% | 1.08 | 1.36 |
| PACSIN2 | 1 | 0.000313177 | 1.80 (1.31, 2.48) | 1.20% | 1.08 | 1.36 |
| PLCD3 | -1 | 0.00020193 | 0.63 (0.49, 0.80) | 0.90% | 1.08 | 1.37 |
| PRSS41 | 1 | 5.10E-05 | 1.45 (1.21, 1.73) | 0.30% | 1.08 | 1.33 |
| RGPD2 | 1 | 0.000329641 | 1.83 (1.32, 2.55) | 1.20% | 1.08 | 1.36 |
| RGPD2 | 1 | 0.000329641 | 1.83 (1.32, 2.55) | 1.20% | 1.08 | 1.36 |
| RIPK4 | 1 | 0.000629791 | 2.40 (1.45, 3.96) | 1.80% | 1.08 | 1.31 |
| RPF1 | -1 | 0.000340725 | 0.52 (0.36, 0.74) | 1.20% | 1.08 | 1.38 |
| SLCO6A1 | 1 | 0.000282574 | 1.72 (1.28, 2.31) | 1.10% | 1.08 | 1.36 |
| SPTAN1 | -1 | 0.000302919 | 0.55 (0.40, 0.76) | 1.20% | 1.08 | 1.38 |
| SPTAN1 | -1 | 0.000302919 | 0.55 (0.40, 0.76) | 1.20% | 1.08 | 1.38 |
| SPTAN1 | -1 | 0.000302919 | 0.55 (0.40, 0.76) | 1.20% | 1.08 | 1.38 |
| ST8SIA3 | 1 | 3.00E-07 | 1.24 (1.14, 1.34) | <0.1% | 1.08 | 1.22 |
| TMEM106A | -1 | 0.000264069 | 0.57 (0.42, 0.77) | 1.10% | 1.08 | 1.38 |
| VCX3B | 1 | 3.69E-05 | 1.41 (1.20, 1.66) | 0.30% | 1.08 | 1.32 |
| ADAMTSL2 | -1 | 0.000193672 | 0.65 (0.52, 0.82) | 0.90% | 1.07 | 1.35 |
| ADAMTSL2 | -1 | 0.000193672 | 0.65 (0.52, 0.82) | 0.90% | 1.07 | 1.35 |
| ALG14 | -1 | 0.000562384 | 0.52 (0.36, 0.75) | 1.70% | 1.07 | 1.36 |
| ANLN | 1 | 0.000800701 | 2.41 (1.44, 4.02) | 2.10% | 1.07 | 1.3 |
| ANUBL1 | -1 | 0.000562446 | 0.50 (0.34, 0.74) | 1.70% | 1.07 | 1.36 |
| ANUBL1 | -1 | 0.000562446 | 0.50 (0.34, 0.74) | 1.70% | 1.07 | 1.36 |
| ARRDC3 | 1 | 0.000609525 | 1.87 (1.31, 2.67) | 1.80% | 1.07 | 1.34 |
| ATG5 | 1 | 6.58E-05 | 1.31 (1.15, 1.49) | 0.40% | 1.07 | 1.26 |
| ATP1A1OS | -1 | 0.00067514 | 0.44 (0.27, 0.70) | 1.90% | 1.07 | 1.34 |
| ATP1A1OS | -1 | 0.00067514 | 0.44 (0.27, 0.70) | 1.90% | 1.07 | 1.34 |
| ATP1A1OS | -1 | 0.00067514 | 0.44 (0.27, 0.70) | 1.90% | 1.07 | 1.34 |
| ATP1A1OS | -1 | 0.00067514 | 0.44 (0.27, 0.70) | 1.90% | 1.07 | 1.34 |
| ATP1A1OS | -1 | 0.00067514 | 0.44 (0.27, 0.70) | 1.90% | 1.07 | 1.34 |
| AURKA | 1 | 0.000564508 | 1.96 (1.34, 2.86) | 1.70% | 1.07 | 1.34 |
| AURKA | 1 | 0.000564508 | 1.96 (1.34, 2.86) | 1.70% | 1.07 | 1.34 |
| AURKA | 1 | 0.000564508 | 1.96 (1.34, 2.86) | 1.70% | 1.07 | 1.34 |
| AURKA | 1 | 0.000564508 | 1.96 (1.34, 2.86) | 1.70% | 1.07 | 1.34 |
| AURKA | 1 | 0.000564508 | 1.96 (1.34, 2.86) | 1.70% | 1.07 | 1.34 |
| AURKA | 1 | 0.000564508 | 1.96 (1.34, 2.86) | 1.70% | 1.07 | 1.34 |
| BRD1 | 1 | 0.000134308 | 1.37 (1.17, 1.62) | 0.70% | 1.07 | 1.29 |
| C20orf191 | 1 | 0.00028668 | 1.50 (1.20, 1.86) | 1.10% | 1.07 | 1.33 |
| C2orf77 | -1 | 0.000330919 | 0.58 (0.43, 0.78) | 1.20% | 1.07 | 1.37 |
| C5 | -1 | 0.000477055 | 0.56 (0.41, 0.78) | 1.60% | 1.07 | 1.37 |
| C6orf103 | -1 | 0.00075103 | 0.49 (0.32, 0.74) | 2.10% | 1.07 | 1.35 |
| CAMTA2 | -1 | 0.000585964 | 0.54 (0.38, 0.77) | 1.80% | 1.07 | 1.36 |
| CAMTA2 | -1 | 0.000585964 | 0.54 (0.38, 0.77) | 1.80% | 1.07 | 1.36 |
| CAMTA2 | -1 | 0.000585964 | 0.54 (0.38, 0.77) | 1.80% | 1.07 | 1.36 |
| CAMTA2 | -1 | 0.000585964 | 0.54 (0.38, 0.77) | 1.80% | 1.07 | 1.36 |
| CCDC40 | -1 | 0.000595988 | 0.55 (0.39, 0.77) | 1.80% | 1.07 | 1.36 |
| CENPP | -1 | 0.00100693 | 0.39 (0.22, 0.68) | 2.50% | 1.07 | 1.31 |
| CHRNA6 | -1 | 0.000417968 | 0.60 (0.45, 0.80) | 1.40% | 1.07 | 1.36 |
| CHRNA6 | -1 | 0.000417968 | 0.60 (0.45, 0.80) | 1.40% | 1.07 | 1.36 |
| DCTN3 | -1 | 0.000423018 | 0.59 (0.44, 0.79) | 1.50% | 1.07 | 1.37 |
| DCTN3 | -1 | 0.000423018 | 0.59 (0.44, 0.79) | 1.50% | 1.07 | 1.37 |
| DDX5 | 1 | 0.000330009 | 1.53 (1.21, 1.92) | 1.20% | 1.07 | 1.33 |
| DNAJC24 | -1 | 0.000858705 | 0.38 (0.21, 0.67) | 2.20% | 1.07 | 1.31 |
| EBF4 | -1 | 0.000723231 | 0.52 (0.36, 0.76) | 2.00% | 1.07 | 1.35 |
| ENO1 | 1 | 0.000286748 | 1.61 (1.25, 2.09) | 1.10% | 1.07 | 1.35 |
| ESRP1 | 1 | 0.000526689 | 1.83 (1.30, 2.58) | 1.70% | 1.07 | 1.35 |
| ESRP1 | 1 | 0.000526689 | 1.83 (1.30, 2.58) | 1.70% | 1.07 | 1.35 |
| ESRP1 | 1 | 0.000526689 | 1.83 (1.30, 2.58) | 1.70% | 1.07 | 1.35 |
| ESRP1 | 1 | 0.000526689 | 1.83 (1.30, 2.58) | 1.70% | 1.07 | 1.35 |
| ESRP1 | 1 | 0.000526689 | 1.83 (1.30, 2.58) | 1.70% | 1.07 | 1.35 |
| EVX1 | 1 | 0.000155183 | 1.47 (1.21, 1.80) | 0.70% | 1.07 | 1.33 |
| EXO1 | 1 | 0.000815942 | 2.21 (1.39, 3.52) | 2.10% | 1.07 | 1.31 |
| EXO1 | 1 | 0.000815942 | 2.21 (1.39, 3.52) | 2.10% | 1.07 | 1.31 |
| EXO1 | 1 | 0.000815942 | 2.21 (1.39, 3.52) | 2.10% | 1.07 | 1.31 |
| FAHD1 | -1 | 0.000285382 | 0.63 (0.49, 0.81) | 1.10% | 1.07 | 1.36 |
| FAHD1 | -1 | 0.000285382 | 0.63 (0.49, 0.81) | 1.10% | 1.07 | 1.36 |
| FTSJ3 | 1 | 0.000250886 | 1.56 (1.23, 1.98) | 1.00% | 1.07 | 1.35 |
| GJB6 | 1 | 0.000361532 | 1.59 (1.23, 2.05) | 1.30% | 1.07 | 1.34 |
| GJB6 | 1 | 0.000361532 | 1.59 (1.23, 2.05) | 1.30% | 1.07 | 1.34 |
| GJB6 | 1 | 0.000361532 | 1.59 (1.23, 2.05) | 1.30% | 1.07 | 1.34 |
| GJB6 | 1 | 0.000361532 | 1.59 (1.23, 2.05) | 1.30% | 1.07 | 1.34 |
| GTF2IRD2B | -1 | 0.000489747 | 0.53 (0.37, 0.76) | 1.60% | 1.07 | 1.37 |
| HIBADH | 1 | 7.51E-05 | 1.41 (1.19, 1.67) | 0.50% | 1.07 | 1.31 |
| HIVEP3 | -1 | 0.000580798 | 0.50 (0.34, 0.74) | 1.80% | 1.07 | 1.36 |
| HIVEP3 | -1 | 0.000580798 | 0.50 (0.34, 0.74) | 1.80% | 1.07 | 1.36 |
| HMGCL | -1 | 0.00045336 | 0.56 (0.41, 0.78) | 1.50% | 1.07 | 1.37 |
| HMGCL | -1 | 0.00045336 | 0.56 (0.41, 0.78) | 1.50% | 1.07 | 1.37 |
| HSBP1 | 1 | 0.000764687 | 2.07 (1.36, 3.17) | 2.10% | 1.07 | 1.32 |
| INTS1 | 1 | 0.000425057 | 1.59 (1.23, 2.06) | 1.50% | 1.07 | 1.34 |
| KIAA1274 | -1 | 0.000322419 | 0.62 (0.48, 0.80) | 1.20% | 1.07 | 1.36 |
| KIF4A | 1 | 0.000565152 | 2.01 (1.35, 2.99) | 1.70% | 1.07 | 1.34 |
| KLHL28 | -1 | 0.000499515 | 0.52 (0.36, 0.75) | 1.60% | 1.07 | 1.37 |
| KPNA2 | 1 | 0.000366643 | 1.80 (1.30, 2.49) | 1.30% | 1.07 | 1.36 |
| KRT81 | 1 | 0.000377924 | 1.69 (1.27, 2.26) | 1.30% | 1.07 | 1.35 |
| LOC100128881 | 1 | 0.000625392 | 2.08 (1.37, 3.16) | 1.80% | 1.07 | 1.33 |
| LOC100129636 | 1 | 0.000323962 | 1.68 (1.27, 2.23) | 1.20% | 1.07 | 1.36 |
| LOC284276 | -1 | 0.000423828 | 0.57 (0.42, 0.78) | 1.50% | 1.07 | 1.37 |
| LOXL1 | -1 | 0.000510308 | 0.59 (0.44, 0.79) | 1.60% | 1.07 | 1.36 |
| LRP8 | 1 | 0.000675065 | 2.00 (1.34, 2.98) | 1.90% | 1.07 | 1.33 |
| LRP8 | 1 | 0.000675065 | 2.00 (1.34, 2.98) | 1.90% | 1.07 | 1.33 |
| LRP8 | 1 | 0.000675065 | 2.00 (1.34, 2.98) | 1.90% | 1.07 | 1.33 |
| LRP8 | 1 | 0.000675065 | 2.00 (1.34, 2.98) | 1.90% | 1.07 | 1.33 |
| LRRC8A | -1 | 0.00053552 | 0.51 (0.35, 0.75) | 1.70% | 1.07 | 1.36 |
| LRRC8A | -1 | 0.00053552 | 0.51 (0.35, 0.75) | 1.70% | 1.07 | 1.36 |
| LRRC8A | -1 | 0.00053552 | 0.51 (0.35, 0.75) | 1.70% | 1.07 | 1.36 |
| MAP7 | 1 | 0.000510806 | 1.69 (1.26, 2.26) | 1.60% | 1.07 | 1.34 |
| MAP7 | 1 | 0.000510806 | 1.69 (1.26, 2.26) | 1.60% | 1.07 | 1.34 |
| MAP7 | 1 | 0.000510806 | 1.69 (1.26, 2.26) | 1.60% | 1.07 | 1.34 |
| MAP7 | 1 | 0.000510806 | 1.69 (1.26, 2.26) | 1.60% | 1.07 | 1.34 |
| MAP7 | 1 | 0.000510806 | 1.69 (1.26, 2.26) | 1.60% | 1.07 | 1.34 |
| MAP7 | 1 | 0.000510806 | 1.69 (1.26, 2.26) | 1.60% | 1.07 | 1.34 |
| MAP7 | 1 | 0.000510806 | 1.69 (1.26, 2.26) | 1.60% | 1.07 | 1.34 |
| MAP7 | 1 | 0.000510806 | 1.69 (1.26, 2.26) | 1.60% | 1.07 | 1.34 |
| MAP7 | 1 | 0.000510806 | 1.69 (1.26, 2.26) | 1.60% | 1.07 | 1.34 |
| MAP7 | 1 | 0.000510806 | 1.69 (1.26, 2.26) | 1.60% | 1.07 | 1.34 |
| MGC3771 | -1 | 0.000568687 | 0.55 (0.39, 0.77) | 1.70% | 1.07 | 1.36 |
| MGC3771 | -1 | 0.000568687 | 0.55 (0.39, 0.77) | 1.70% | 1.07 | 1.36 |
| MT1G | 1 | 0.000312285 | 1.56 (1.22, 1.98) | 1.20% | 1.07 | 1.34 |
| NGRN | 1 | 0.000282469 | 1.57 (1.23, 2.01) | 1.10% | 1.07 | 1.35 |
| NGRN | 1 | 0.000282469 | 1.57 (1.23, 2.01) | 1.10% | 1.07 | 1.35 |
| NLRP8 | -1 | 0.000622004 | 0.50 (0.34, 0.74) | 1.80% | 1.07 | 1.36 |
| NPHP1 | -1 | 0.000473665 | 0.54 (0.38, 0.76) | 1.60% | 1.07 | 1.37 |
| NPHP1 | -1 | 0.000473665 | 0.54 (0.38, 0.76) | 1.60% | 1.07 | 1.37 |
| NPHP1 | -1 | 0.000473665 | 0.54 (0.38, 0.76) | 1.60% | 1.07 | 1.37 |
| NPHP1 | -1 | 0.000473665 | 0.54 (0.38, 0.76) | 1.60% | 1.07 | 1.37 |
| NPY | 1 | 0.00011211 | 1.35 (1.16, 1.57) | 0.60% | 1.07 | 1.28 |
| PGK1 | 1 | 0.000239238 | 1.59 (1.24, 2.04) | 1.00% | 1.07 | 1.35 |
| PGR | -1 | 0.000509769 | 0.53 (0.37, 0.76) | 1.60% | 1.07 | 1.37 |
| PIH1D2 | -1 | 0.000210904 | 0.65 (0.52, 0.82) | 0.90% | 1.07 | 1.35 |
| PIH1D2 | -1 | 0.000210904 | 0.65 (0.52, 0.82) | 0.90% | 1.07 | 1.35 |
| PLA2G2E | 1 | 0.000289748 | 1.57 (1.23, 2.00) | 1.10% | 1.07 | 1.34 |
| PLD2 | -1 | 0.000493619 | 0.57 (0.42, 0.78) | 1.60% | 1.07 | 1.37 |
| PLEKHA8 | 1 | 0.000432361 | 1.63 (1.24, 2.13) | 1.50% | 1.07 | 1.34 |
| PLEKHA8 | 1 | 0.000432361 | 1.63 (1.24, 2.13) | 1.50% | 1.07 | 1.34 |
| PLEKHA8 | 1 | 0.000432361 | 1.63 (1.24, 2.13) | 1.50% | 1.07 | 1.34 |
| PMAIP1 | -1 | 0.000483779 | 0.54 (0.39, 0.77) | 1.60% | 1.07 | 1.37 |
| PRC1 | 1 | 0.000661503 | 1.87 (1.30, 2.67) | 1.90% | 1.07 | 1.34 |
| PRC1 | 1 | 0.000661503 | 1.87 (1.30, 2.67) | 1.90% | 1.07 | 1.34 |
| PRC1 | 1 | 0.000661503 | 1.87 (1.30, 2.67) | 1.90% | 1.07 | 1.34 |
| PRKAG2 | -1 | 0.000805839 | 0.48 (0.31, 0.74) | 2.10% | 1.07 | 1.35 |
| PRKAG2 | -1 | 0.000805839 | 0.48 (0.31, 0.74) | 2.10% | 1.07 | 1.35 |
| PRKAG2 | -1 | 0.000805839 | 0.48 (0.31, 0.74) | 2.10% | 1.07 | 1.35 |
| PRR7 | 1 | 0.000736074 | 2.09 (1.36, 3.21) | 2.00% | 1.07 | 1.33 |
| PRR7 | 1 | 0.000736074 | 2.09 (1.36, 3.21) | 2.00% | 1.07 | 1.33 |
| PRR7 | 1 | 0.000736074 | 2.09 (1.36, 3.21) | 2.00% | 1.07 | 1.33 |
| SARNP | 1 | 0.000200909 | 1.48 (1.20, 1.83) | 0.90% | 1.07 | 1.33 |
| SARNP | 1 | 0.000200909 | 1.48 (1.20, 1.83) | 0.90% | 1.07 | 1.33 |
| SARNP | 1 | 0.000200909 | 1.48 (1.20, 1.83) | 0.90% | 1.07 | 1.33 |
| SCUBE2 | -1 | 0.000658247 | 0.48 (0.32, 0.73) | 1.90% | 1.07 | 1.35 |
| SCUBE2 | -1 | 0.000658247 | 0.48 (0.32, 0.73) | 1.90% | 1.07 | 1.35 |
| SEC31A | 1 | 0.000358109 | 1.68 (1.26, 2.23) | 1.30% | 1.07 | 1.35 |
| SEC31A | 1 | 0.000358109 | 1.68 (1.26, 2.23) | 1.30% | 1.07 | 1.35 |
| SEC31A | 1 | 0.000358109 | 1.68 (1.26, 2.23) | 1.30% | 1.07 | 1.35 |
| SEC31A | 1 | 0.000358109 | 1.68 (1.26, 2.23) | 1.30% | 1.07 | 1.35 |
| SEC31A | 1 | 0.000358109 | 1.68 (1.26, 2.23) | 1.30% | 1.07 | 1.35 |
| SEC31A | 1 | 0.000358109 | 1.68 (1.26, 2.23) | 1.30% | 1.07 | 1.35 |
| SEMA4B | 1 | 0.000291864 | 1.53 (1.21, 1.92) | 1.10% | 1.07 | 1.34 |
| SEMA4B | 1 | 0.000291864 | 1.53 (1.21, 1.92) | 1.10% | 1.07 | 1.34 |
| SERPINB3 | 1 | 4..56E-05 | 1.34 (1.17, 1.55) | 0.30% | 1.07 | 1.28 |
| SEZ6L | -1 | 0.00098828 | 0.42 (0.25, 0.70) | 2.40% | 1.07 | 1.32 |
| SEZ6L | -1 | 0.00098828 | 0.42 (0.25, 0.70) | 2.40% | 1.07 | 1.32 |
| SEZ6L | -1 | 0.00098828 | 0.42 (0.25, 0.70) | 2.40% | 1.07 | 1.32 |
| SEZ6L | -1 | 0.00098828 | 0.42 (0.25, 0.70) | 2.40% | 1.07 | 1.32 |
| SEZ6L | -1 | 0.00098828 | 0.42 (0.25, 0.70) | 2.40% | 1.07 | 1.32 |
| SEZ6L | -1 | 0.00098828 | 0.42 (0.25, 0.70) | 2.40% | 1.07 | 1.32 |
| SH3GLB2 | -1 | 0.000498384 | 0.57 (0.41, 0.78) | 1.60% | 1.07 | 1.37 |
| SLC10A7 | 1 | 0.000332852 | 1.59 (1.23, 2.05) | 1.20% | 1.07 | 1.34 |
| SLC10A7 | 1 | 0.000332852 | 1.59 (1.23, 2.05) | 1.20% | 1.07 | 1.34 |
| SLC12A1 | 1 | 0.000204056 | 1.49 (1.21, 1.83) | 0.90% | 1.07 | 1.33 |
| SLC12A1 | 1 | 0.000204056 | 1.49 (1.21, 1.83) | 0.90% | 1.07 | 1.33 |
| SLC6A17 | 1 | 0.000424003 | 1.66 (1.25, 2.19) | 1.50% | 1.07 | 1.35 |
| SND1 | 1 | 0.000527599 | 1.82 (1.30, 2.55) | 1.70% | 1.07 | 1.35 |
| SPATA4 | -1 | 0.000630371 | 0.51 (0.35, 0.75) | 1.80% | 1.07 | 1.36 |
| SPPL2A | 1 | 5.23E-06 | 1.25 (1.14, 1.38) | 0.10% | 1.07 | 1.23 |
| SURF1 | -1 | 0.000200776 | 0.64 (0.51, 0.81) | 0.90% | 1.07 | 1.36 |
| SYT9 | -1 | 0.000684006 | 0.54 (0.38, 0.77) | 1.90% | 1.07 | 1.36 |
| TBRG4 | 1 | 0.000200243 | 1.49 (1.21, 1.85) | 0.90% | 1.07 | 1.33 |
| TBRG4 | 1 | 0.000200243 | 1.49 (1.21, 1.85) | 0.90% | 1.07 | 1.33 |
| TBRG4 | 1 | 0.000200243 | 1.49 (1.21, 1.85) | 0.90% | 1.07 | 1.33 |
| TFRC | 1 | 0.000315917 | 1.71 (1.28, 2.29) | 1.20% | 1.07 | 1.36 |
| TFRC | 1 | 0.000315917 | 1.71 (1.28, 2.29) | 1.20% | 1.07 | 1.36 |
| TRABD | 1 | 0.000122576 | 1.47 (1.21, 1.79) | 0.60% | 1.07 | 1.33 |
| TSTD2 | -1 | 0.000818708 | 0.48 (0.31, 0.74) | 2.10% | 1.07 | 1.34 |
| TUG1 | 1 | 0.000491525 | 1.91 (1.33, 2.76) | 1.60% | 1.07 | 1.35 |
| UCA1 | -1 | 0.000575028 | 0.54 (0.38, 0.76) | 1.80% | 1.07 | 1.36 |
| UGT2A1 | 1 | 0.000173025 | 1.43 (1.19, 1.73) | 0.80% | 1.07 | 1.32 |
| ULBP3 | 1 | 0.000274042 | 1.57 (1.23, 2.00) | 1.10% | 1.07 | 1.35 |
| USP39 | 1 | 0.000493416 | 1.78 (1.29, 2.47) | 1.60% | 1.07 | 1.35 |
| VPS41 | 1 | 0.000370817 | 1.62 (1.24, 2.12) | 1.30% | 1.07 | 1.35 |
| VPS41 | 1 | 0.000370817 | 1.62 (1.24, 2.12) | 1.30% | 1.07 | 1.35 |
| WDR19 | -1 | 0.000547998 | 0.54 (0.38, 0.77) | 1.70% | 1.07 | 1.36 |
| XAB2 | -1 | 0.000225163 | 0.68 (0.55, 0.83) | 1.00% | 1.07 | 1.34 |
| ZNF516 | -1 | 0.000417543 | 0.53 (0.38, 0.76) | 1.40% | 1.07 | 1.37 |
| ZNF665 | -1 | 0.000397142 | 0.57 (0.42, 0.78) | 1.40% | 1.07 | 1.37 |
| ZNF768 | -1 | 0.00038939 | 0.60 (0.46, 0.80) | 1.40% | 1.07 | 1.36 |
| ZNF782 | -1 | 0.000573696 | 0.57 (0.41, 0.78) | 1.80% | 1.07 | 1.36 |
| ZSCAN30 | -1 | 0.000437804 | 0.55 (0.39, 0.77) | 1.50% | 1.07 | 1.37 |
| ZSCAN30 | -1 | 0.000437804 | 0.55 (0.39, 0.77) | 1.50% | 1.07 | 1.37 |
| ADC | -1 | 0.00054168 | 0.63 (0.48, 0.82) | 1.70% | 1.06 | 1.35 |
| ANKRD30A | -1 | 0.000861337 | 0.55 (0.39, 0.78) | 2.20% | 1.06 | 1.35 |
| ANKRD5 | -1 | 0.000461483 | 0.63 (0.48, 0.81) | 1.50% | 1.06 | 1.35 |
| ANKRD5 | -1 | 0.000461483 | 0.63 (0.48, 0.81) | 1.50% | 1.06 | 1.35 |
| ARMCX4 | -1 | 0.000894017 | 0.53 (0.36, 0.77) | 2.30% | 1.06 | 1.35 |
| ATP5S | -1 | 0.000805348 | 0.52 (0.35, 0.76) | 2.10% | 1.06 | 1.35 |
| ATP5S | -1 | 0.000805348 | 0.52 (0.35, 0.76) | 2.10% | 1.06 | 1.35 |
| ATP5S | -1 | 0.000805348 | 0.52 (0.35, 0.76) | 2.10% | 1.06 | 1.35 |
| ATP5S | -1 | 0.000805348 | 0.52 (0.35, 0.76) | 2.10% | 1.06 | 1.35 |
| AZI2 | 1 | 0.000790058 | 1.85 (1.29, 2.65) | 2.10% | 1.06 | 1.33 |
| AZI2 | 1 | 0.000790058 | 1.85 (1.29, 2.65) | 2.10% | 1.06 | 1.33 |
| AZI2 | 1 | 0.000790058 | 1.85 (1.29, 2.65) | 2.10% | 1.06 | 1.33 |
| BCAS3 | 1 | 0.000602033 | 1.60 (1.22, 2.10) | 1.80% | 1.06 | 1.33 |
| BCAS3 | 1 | 0.000602033 | 1.60 (1.22, 2.10) | 1.80% | 1.06 | 1.33 |
| C16orf71 | -1 | 0.000979549 | 0.51 (0.34, 0.76) | 2.40% | 1.06 | 1.34 |
| C1orf173 | -1 | 0.000725234 | 0.54 (0.37, 0.77) | 2.00% | 1.06 | 1.36 |
| C1orf198 | 1 | 0.000803782 | 1.80 (1.28, 2.54) | 2.10% | 1.06 | 1.33 |
| C1orf198 | 1 | 0.000803782 | 1.80 (1.28, 2.54) | 2.10% | 1.06 | 1.33 |
| C1orf198 | 1 | 0.000803782 | 1.80 (1.28, 2.54) | 2.10% | 1.06 | 1.33 |
| C2orf78 | 1 | 0.000483098 | 1.58 (1.22, 2.04) | 1.60% | 1.06 | 1.33 |
| C4orf45 | 1 | 0.000764755 | 1.79 (1.28, 2.51) | 2.10% | 1.06 | 1.34 |
| CALCOCO2 | 1 | 0.00019697 | 1.33 (1.15, 1.55) | 0.90% | 1.06 | 1.27 |
| CDCA8 | 1 | 0.001355782 | 2.59 (1.45, 4.65) | 2.90% | 1.06 | 1.27 |
| CEP110 | -1 | 0.00077961 | 0.52 (0.36, 0.76) | 2.10% | 1.06 | 1.35 |
| CGRRF1 | -1 | 0.000645901 | 0.60 (0.45, 0.81) | 1.90% | 1.06 | 1.35 |
| CNTNAP1 | -1 | 0.000689153 | 0.60 (0.45, 0.81) | 2.00% | 1.06 | 1.35 |
| COL11A2 | 1 | 0.00025518 | 1.37 (1.16, 1.62) | 1.00% | 1.06 | 1.29 |
| COL11A2 | 1 | 0.00025518 | 1.37 (1.16, 1.62) | 1.00% | 1.06 | 1.29 |
| COL11A2 | 1 | 0.00025518 | 1.37 (1.16, 1.62) | 1.00% | 1.06 | 1.29 |
| COL11A2 | 1 | 0.00025518 | 1.37 (1.16, 1.62) | 1.00% | 1.06 | 1.29 |
| COPB1 | 1 | 0.000543696 | 1.69 (1.26, 2.28) | 1.70% | 1.06 | 1.34 |
| COPB1 | 1 | 0.000543696 | 1.69 (1.26, 2.28) | 1.70% | 1.06 | 1.34 |
| COPB1 | 1 | 0.000543696 | 1.69 (1.26, 2.28) | 1.70% | 1.06 | 1.34 |
| CREB3L2 | 1 | 0.00060177 | 1.72 (1.26, 2.35) | 1.80% | 1.06 | 1.34 |
| DCDC1 | -1 | 0.000539347 | 0.59 (0.44, 0.80) | 1.70% | 1.06 | 1.36 |
| EFNA5 | 1 | 0.000714322 | 1.71 (1.25, 2.34) | 2.00% | 1.06 | 1.34 |
| EIF3B | 1 | 0.000597068 | 1.67 (1.25, 2.24) | 1.80% | 1.06 | 1.34 |
| EIF3B | 1 | 0.000597068 | 1.67 (1.25, 2.24) | 1.80% | 1.06 | 1.34 |
| ELOVL2 | -1 | 0.000664075 | 0.56 (0.40, 0.78) | 1.90% | 1.06 | 1.36 |
| FBXO8 | -1 | 0.000806276 | 0.56 (0.40, 0.79) | 2.10% | 1.06 | 1.35 |
| FGD3 | -1 | 0.000941348 | 0.49 (0.32, 0.75) | 2.40% | 1.06 | 1.34 |
| FGD3 | -1 | 0.000941348 | 0.49 (0.32, 0.75) | 2.40% | 1.06 | 1.34 |
| GALNT5 | -1 | 0.000684822 | 0.55 (0.39, 0.78) | 1.90% | 1.06 | 1.36 |
| GTSE1 | 1 | 0.000929866 | 2.10 (1.35, 3.26) | 2.40% | 1.06 | 1.32 |
| INHBE | 1 | 0.000208267 | 1.37 (1.16, 1.61) | 0.90% | 1.06 | 1.29 |
| IPMK | 1 | 0.00077886 | 1.67 (1.24, 2.24) | 2.10% | 1.06 | 1.33 |
| KRT4 | 1 | 0.00047149 | 1.55 (1.21, 1.98) | 1.60% | 1.06 | 1.33 |
| LDLR | 1 | 0.000708045 | 1.70 (1.25, 2.31) | 2.00% | 1.06 | 1.34 |
| LDLR | 1 | 0.000708045 | 1.70 (1.25, 2.31) | 2.00% | 1.06 | 1.34 |
| LDLR | 1 | 0.000708045 | 1.70 (1.25, 2.31) | 2.00% | 1.06 | 1.34 |
| LDLR | 1 | 0.000708045 | 1.70 (1.25, 2.31) | 2.00% | 1.06 | 1.34 |
| LDLR | 1 | 0.000708045 | 1.70 (1.25, 2.31) | 2.00% | 1.06 | 1.34 |
| LDLR | 1 | 0.000708045 | 1.70 (1.25, 2.31) | 2.00% | 1.06 | 1.34 |
| LIN28A | 1 | 0.000535605 | 1.50 (1.19, 1.88) | 1.70% | 1.06 | 1.32 |
| LOC100128788 | -1 | 0.001181148 | 0.48 (0.31, 0.75) | 2.70% | 1.06 | 1.33 |
| LOC100128788 | -1 | 0.001181148 | 0.48 (0.31, 0.75) | 2.70% | 1.06 | 1.33 |
| LOC283038 | -1 | 0.000583488 | 0.60 (0.45, 0.80) | 1.80% | 1.06 | 1.36 |
| MAPT | -1 | 0.000668769 | 0.59 (0.43, 0.80) | 1.90% | 1.06 | 1.35 |
| MAPT | -1 | 0.000668769 | 0.59 (0.43, 0.80) | 1.90% | 1.06 | 1.35 |
| MAPT | -1 | 0.000668769 | 0.59 (0.43, 0.80) | 1.90% | 1.06 | 1.35 |
| MAPT | -1 | 0.000668769 | 0.59 (0.43, 0.80) | 1.90% | 1.06 | 1.35 |
| MAPT | -1 | 0.000668769 | 0.59 (0.43, 0.80) | 1.90% | 1.06 | 1.35 |
| MAPT | -1 | 0.000668769 | 0.59 (0.43, 0.80) | 1.90% | 1.06 | 1.35 |
| MATN3 | -1 | 0.000797381 | 0.59 (0.43, 0.80) | 2.10% | 1.06 | 1.35 |
| MKI67 | 1 | 0.001246003 | 2.15 (1.35, 3.41) | 2.80% | 1.06 | 1.3 |
| MKI67 | 1 | 0.001246003 | 2.15 (1.35, 3.41) | 2.80% | 1.06 | 1.3 |
| MORC2 | 1 | 0.000833522 | 1.86 (1.29, 2.68) | 2.20% | 1.06 | 1.33 |
| NLGN2 | -1 | 0.000244843 | 0.71 (0.59, 0.85) | 1.00% | 1.06 | 1.31 |
| NT5C2 | 1 | 0.000631267 | 1.73 (1.26, 2.37) | 1.80% | 1.06 | 1.34 |
| NT5C2 | 1 | 0.000631267 | 1.73 (1.26, 2.37) | 1.80% | 1.06 | 1.34 |
| OSBP2 | 1 | 0.000812952 | 1.71 (1.25, 2.33) | 2.10% | 1.06 | 1.33 |
| PDE11A | -1 | 0.000734955 | 0.57 (0.42, 0.79) | 2.00% | 1.06 | 1.35 |
| PDE11A | -1 | 0.000734955 | 0.57 (0.42, 0.79) | 2.00% | 1.06 | 1.35 |
| PDE11A | -1 | 0.000734955 | 0.57 (0.42, 0.79) | 2.00% | 1.06 | 1.35 |
| PDE11A | -1 | 0.000734955 | 0.57 (0.42, 0.79) | 2.00% | 1.06 | 1.35 |
| PI3 | 1 | 0.000476303 | 1.49 (1.19, 1.86) | 1.60% | 1.06 | 1.32 |
| PIGG | -1 | 0.000571553 | 0.60 (0.45, 0.80) | 1.70% | 1.06 | 1.36 |
| PIGG | -1 | 0.000571553 | 0.60 (0.45, 0.80) | 1.70% | 1.06 | 1.36 |
| POLDIP3 | 1 | 0.000528218 | 1.55 (1.21, 1.98) | 1.70% | 1.06 | 1.33 |
| POLDIP3 | 1 | 0.000528218 | 1.55 (1.21, 1.98) | 1.70% | 1.06 | 1.33 |
| POU4F3 | 1 | 0.000229501 | 1.40 (1.17, 1.67) | 1.00% | 1.06 | 1.3 |
| PRDX6 | 1 | 0.000780619 | 1.70 (1.25, 2.32) | 2.10% | 1.06 | 1.33 |
| PRKD1 | 1 | 0.000548992 | 1.50 (1.19, 1.89) | 1.70% | 1.06 | 1.32 |
| PSMD7 | 1 | 0.000563721 | 1.68 (1.25, 2.25) | 1.70% | 1.06 | 1.34 |
| RILPL2 | -1 | 0.000768058 | 0.57 (0.41, 0.79) | 2.10% | 1.06 | 1.35 |
| RORB | -1 | 0.000604719 | 0.59 (0.44, 0.80) | 1.80% | 1.06 | 1.36 |
| RPS3 | 1 | 0.000337452 | 1.44 (1.18, 1.75) | 1.20% | 1.06 | 1.31 |
| RTN4RL1 | -1 | 0.000608443 | 0.63 (0.49, 0.82) | 1.80% | 1.06 | 1.34 |
| SC4MOL | 1 | 0.000437147 | 1.51 (1.20, 1.89) | 1.50% | 1.06 | 1.32 |
| SC4MOL | 1 | 0.000437147 | 1.51 (1.20, 1.89) | 1.50% | 1.06 | 1.32 |
| SLC40A1 | -1 | 0.001006056 | 0.51 (0.34, 0.76) | 2.50% | 1.06 | 1.34 |
| SLC9A6 | 1 | 0.00076266 | 1.81 (1.28, 2.56) | 2.10% | 1.06 | 1.34 |
| SLC9A6 | 1 | 0.00076266 | 1.81 (1.28, 2.56) | 2.10% | 1.06 | 1.34 |
| SLC9A6 | 1 | 0.00076266 | 1.81 (1.28, 2.56) | 2.10% | 1.06 | 1.34 |
| TAX1BP1 | 1 | 0.000335077 | 1.39 (1.16, 1.66) | 1.20% | 1.06 | 1.29 |
| TAX1BP1 | 1 | 0.000335077 | 1.39 (1.16, 1.66) | 1.20% | 1.06 | 1.29 |
| TMEM39A | 1 | 0.000692396 | 1.77 (1.27, 2.47) | 2.00% | 1.06 | 1.34 |
| TMEM5 | -1 | 0.000852491 | 0.56 (0.40, 0.79) | 2.20% | 1.06 | 1.35 |
| TMOD2 | 1 | 0.000221131 | 1.43 (1.18, 1.72) | 0.90% | 1.06 | 1.31 |
| TMOD2 | 1 | 0.000221131 | 1.43 (1.18, 1.72) | 0.90% | 1.06 | 1.31 |
| TMPRSS11E | 1 | 0.000771096 | 1.72 (1.25, 2.36) | 2.10% | 1.06 | 1.33 |
| TNFAIP8L2 | -1 | 0.000648477 | 0.57 (0.41, 0.79) | 1.90% | 1.06 | 1.36 |
| TNFSF12-TNFSF13 | -1 | 0.000593768 | 0.58 (0.43, 0.79) | 1.80% | 1.06 | 1.36 |
| TRAF3IP1 | -1 | 0.000786338 | 0.56 (0.40, 0.79) | 2.10% | 1.06 | 1.35 |
| TRAF3IP1 | -1 | 0.000786338 | 0.56 (0.40, 0.79) | 2.10% | 1.06 | 1.35 |
| TRIM10 | 1 | 0.000210243 | 1.33 (1.14, 1.55) | 0.90% | 1.06 | 1.27 |
| TRIM10 | 1 | 0.000210243 | 1.33 (1.14, 1.55) | 0.90% | 1.06 | 1.27 |
| VILL | -1 | 0.000741792 | 0.60 (0.44, 0.81) | 2.10% | 1.06 | 1.35 |
| ZFHX2 | -1 | 0.000723459 | 0.55 (0.39, 0.78) | 2.00% | 1.06 | 1.36 |
| ZNF652 | 1 | 0.000337669 | 1.51 (1.20, 1.89) | 1.20% | 1.06 | 1.33 |
| ZNF652 | 1 | 0.000337669 | 1.51 (1.20, 1.89) | 1.20% | 1.06 | 1.33 |
| ACCN5 | 1 | 0.001052716 | 1.88 (1.29, 2.74) | 2.50% | 1.05 | 1.32 |
| ACTL8 | 1 | 0.000978531 | 1.78 (1.26, 2.52) | 2.40% | 1.05 | 1.33 |
| AGTR1 | -1 | 0.001529539 | 0.50 (0.32, 0.77) | 3.10% | 1.05 | 1.32 |
| AGTR1 | -1 | 0.001529539 | 0.50 (0.32, 0.77) | 3.10% | 1.05 | 1.32 |
| AGTR1 | -1 | 0.001529539 | 0.50 (0.32, 0.77) | 3.10% | 1.05 | 1.32 |
| AGTR1 | -1 | 0.001529539 | 0.50 (0.32, 0.77) | 3.10% | 1.05 | 1.32 |
| AGTR1 | -1 | 0.001529539 | 0.50 (0.32, 0.77) | 3.10% | 1.05 | 1.32 |
| APOF | 1 | 0.000357244 | 1.36 (1.15, 1.61) | 1.30% | 1.05 | 1.28 |
| ARPP19 | 1 | 0.000140299 | 1.23 (1.11, 1.37) | 0.70% | 1.05 | 1.21 |
| ATN1 | -1 | 0.00113338 | 0.57 (0.41, 0.80) | 2.60% | 1.05 | 1.34 |
| ATN1 | -1 | 0.00113338 | 0.57 (0.41, 0.80) | 2.60% | 1.05 | 1.34 |
| BIRC6 | 1 | 0.001242408 | 1.68 (1.23, 2.30) | 2.80% | 1.05 | 1.32 |
| BTG3 | 1 | 0.00134171 | 1.79 (1.25, 2.55) | 2.90% | 1.05 | 1.32 |
| BTG3 | 1 | 0.00134171 | 1.79 (1.25, 2.55) | 2.90% | 1.05 | 1.32 |
| C10orf79 | -1 | 0.001408252 | 0.59 (0.42, 0.81) | 3.00% | 1.05 | 1.33 |
| C14orf105 | 1 | 0.001588324 | 1.90 (1.27, 2.82) | 3.20% | 1.05 | 1.31 |
| C14orf135 | -1 | 0.001008184 | 0.60 (0.44, 0.81) | 2.50% | 1.05 | 1.34 |
| C16orf61 | 1 | 0.001288809 | 1.78 (1.25, 2.53) | 2.90% | 1.05 | 1.32 |
| C17orf102 | 1 | 0.000181996 | 1.24 (1.11, 1.39) | 0.80% | 1.05 | 1.21 |
| C17orf64 | 1 | 0.00063311 | 1.48 (1.18, 1.86) | 1.80% | 1.05 | 1.31 |
| C20orf132 | -1 | 0.00106845 | 0.53 (0.36, 0.77) | 2.60% | 1.05 | 1.34 |
| C20orf132 | -1 | 0.00106845 | 0.53 (0.36, 0.77) | 2.60% | 1.05 | 1.34 |
| C20orf132 | -1 | 0.00106845 | 0.53 (0.36, 0.77) | 2.60% | 1.05 | 1.34 |
| C21orf29 | 1 | 0.001264027 | 1.74 (1.24, 2.44) | 2.80% | 1.05 | 1.32 |
| C6orf155 | -1 | 0.001092707 | 0.53 (0.36, 0.78) | 2.60% | 1.05 | 1.34 |
| C9orf102 | -1 | 0.001523324 | 0.50 (0.33, 0.77) | 3.10% | 1.05 | 1.32 |
| C9orf156 | -1 | 0.001347547 | 0.52 (0.34, 0.77) | 2.90% | 1.05 | 1.33 |
| C9orf43 | -1 | 0.001198516 | 0.60 (0.44, 0.82) | 2.70% | 1.05 | 1.34 |
| CASC1 | -1 | 0.000936736 | 0.59 (0.43, 0.81) | 2.40% | 1.05 | 1.34 |
| CASC1 | -1 | 0.000936736 | 0.59 (0.43, 0.81) | 2.40% | 1.05 | 1.34 |
| CASC1 | -1 | 0.000936736 | 0.59 (0.43, 0.81) | 2.40% | 1.05 | 1.34 |
| CASK | 1 | 0.001007193 | 1.81 (1.27, 2.57) | 2.50% | 1.05 | 1.33 |
| CASK | 1 | 0.001007193 | 1.81 (1.27, 2.57) | 2.50% | 1.05 | 1.33 |
| CASK | 1 | 0.001007193 | 1.81 (1.27, 2.57) | 2.50% | 1.05 | 1.33 |
| CBFB | 1 | 0.001037169 | 1.65 (1.22, 2.23) | 2.50% | 1.05 | 1.32 |
| CBFB | 1 | 0.001037169 | 1.65 (1.22, 2.23) | 2.50% | 1.05 | 1.32 |
| CCNB2 | 1 | 0.001408285 | 1.72 (1.23, 2.40) | 3.00% | 1.05 | 1.32 |
| CDK13 | 1 | 7.62E-05 | 1.22 (1.11, 1.35) | 0.50% | 1.05 | 1.2 |
| CDK13 | 1 | 7.62E-05 | 1.22 (1.11, 1.35) | 0.50% | 1.05 | 1.2 |
| CEP72 | 1 | 0.001459446 | 1.82 (1.26, 2.63) | 3.10% | 1.05 | 1.31 |
| CKAP2L | 1 | 0.001319145 | 2.12 (1.34, 3.35) | 2.90% | 1.05 | 1.3 |
| CLTC | 1 | 0.000745682 | 1.49 (1.18, 1.88) | 2.10% | 1.05 | 1.31 |
| COX5A | 1 | 0.000669975 | 1.51 (1.19, 1.91) | 1.90% | 1.05 | 1.32 |
| CRNN | 1 | 0.000789381 | 1.45 (1.17, 1.80) | 2.10% | 1.05 | 1.3 |
| CYP19A1 | 1 | 0.001318685 | 1.75 (1.24, 2.47) | 2.90% | 1.05 | 1.32 |
| CYP19A1 | 1 | 0.001318685 | 1.75 (1.24, 2.47) | 2.90% | 1.05 | 1.32 |
| DCHS1 | -1 | 0.001479235 | 0.56 (0.39, 0.80) | 3.10% | 1.05 | 1.33 |
| DET1 | -1 | 0.000618527 | 0.72 (0.59, 0.87) | 1.80% | 1.05 | 1.29 |
| DET1 | -1 | 0.000618527 | 0.72 (0.59, 0.87) | 1.80% | 1.05 | 1.29 |
| DET1 | -1 | 0.000618527 | 0.72 (0.59, 0.87) | 1.80% | 1.05 | 1.29 |
| DNAJA1 | -1 | 0.001074517 | 0.64 (0.49, 0.84) | 2.60% | 1.05 | 1.33 |
| EPGN | 1 | 0.001109057 | 1.71 (1.24, 2.36) | 2.60% | 1.05 | 1.32 |
| ERBB4 | -1 | 0.001313858 | 0.59 (0.42, 0.81) | 2.90% | 1.05 | 1.33 |
| ERBB4 | -1 | 0.001313858 | 0.59 (0.42, 0.81) | 2.90% | 1.05 | 1.33 |
| EXOC3L | 1 | 0.001576091 | 2.03 (1.31, 3.14) | 3.20% | 1.05 | 1.3 |
| FBN3 | 1 | 0.000998771 | 1.55 (1.19, 2.02) | 2.50% | 1.05 | 1.31 |
| FBXO22 | 1 | 0.000970814 | 1.79 (1.27, 2.53) | 2.40% | 1.05 | 1.33 |
| FBXO22 | 1 | 0.000970814 | 1.79 (1.27, 2.53) | 2.40% | 1.05 | 1.33 |
| FBXO22 | 1 | 0.000970814 | 1.79 (1.27, 2.53) | 2.40% | 1.05 | 1.33 |
| FBXO36 | -1 | 0.000983445 | 0.56 (040, 0.79) | 2.40% | 1.05 | 1.35 |
| FU26245 | 1 | 0.000549548 | 1.46 (1.18, 1.81) | 1.70% | 1.05 | 1.31 |
| FU46361 | 1 | 0.000467876 | 1.42 (1.17, 1.73) | 1.60% | 1.05 | 1.3 |
| GALNT13 | 1 | 0.001008619 | 1.66 (1.23, 2.24) | 2.50% | 1.05 | 1.32 |
| GALNTL1 | -1 | 0.001388679 | 0.54 (0.37, 0.79) | 3.00% | 1.05 | 1.33 |
| GALNTL1 | -1 | 0.001388679 | 0.54 (0.37, 0.79) | 3.00% | 1.05 | 1.33 |
| GPR107 | -1 | 0.001371972 | 0.46 (0.29, 0.74) | 2.90% | 1.05 | 1.32 |
| GPR107 | -1 | 0.001371972 | 0.46 (0.29, 0.74) | 2.90% | 1.05 | 1.32 |
| GPR107 | -1 | 0.001371972 | 0.46 (0.29, 0.74) | 2.90% | 1.05 | 1.32 |
| GSTA5 | 1 | 0.000405719 | 1.37 (1.15, 1.62) | 1.40% | 1.05 | 1.28 |
| HHLA3 | -1 | 0.001094742 | 0.58 (0.42, 0.80) | 2.60% | 1.05 | 1.34 |
| HHLA3 | -1 | 0.001094742 | 0.58 (0.42, 0.80) | 2.60% | 1.05 | 1.34 |
| HHLA3 | -1 | 0.001094742 | 0.58 (0.42, 0.80) | 2.60% | 1.05 | 1.34 |
| HHLA3 | -1 | 0.001094742 | 0.58 (0.42, 0.80) | 2.60% | 1.05 | 1.34 |
| HINFP | -1 | 0.000768103 | 0.65 (0.50, 0.84) | 2.10% | 1.05 | 1.33 |
| HINFP | -1 | 0.000768103 | 0.65 (0.50, 0.84) | 2.10% | 1.05 | 1.33 |
| HMGCR | 1 | 0.000609854 | 1.48 (1.18, 1.85) | 1.80% | 1.05 | 1.31 |
| HMGCR | 1 | 0.000609854 | 1.48 (1.18, 1.85) | 1.80% | 1.05 | 1.31 |
| HYDIN | -1 | 0.001297953 | 0.58 (0.42, 0.81) | 2.90% | 1.05 | 1.34 |
| HYDIN | -1 | 0.001297953 | 0.58 (0.42, 0.81) | 2.90% | 1.05 | 1.34 |
| HYDIN | -1 | 0.001297953 | 0.58 (0.42, 0.81) | 2.90% | 1.05 | 1.34 |
| HYDIN | -1 | 0.001297953 | 0.58 (0.42, 0.81) | 2.90% | 1.05 | 1.34 |
| IARS2 | 1 | 0.001187981 | 1.68 (1.23, 2.30) | 2.70% | 1.05 | 1.32 |
| IFT88 | -1 | 0.001402791 | 0.57 (0.40, 0.80) | 3.00% | 1.05 | 1.33 |
| IFT88 | -1 | 0.001402791 | 0.57 (0.40, 0.80) | 3.00% | 1.05 | 1.33 |
| IGF2BP1 | 1 | 0.000806815 | 1.61 (1.22, 2.13) | 2.10% | 1.05 | 1.33 |
| IGF2BP1 | 1 | 0.000806815 | 1.61 (1.22, 2.13) | 2.10% | 1.05 | 1.33 |
| IL1F10 | 1 | 0.000806465 | 1.47 (1.17, 1.84) | 2.10% | 1.05 | 1.3 |
| IL1F10 | 1 | 0.000806465 | 1.47 (1.17, 1.84) | 2.10% | 1.05 | 1.3 |
| INTU | -1 | 0.000791038 | 0.64 (0.49, 0.83) | 2.10% | 1.05 | 1.34 |
| IQGAP3 | 1 | 0.001269109 | 2.03 (1.32, 3.13) | 2.80% | 1.05 | 1.31 |
| KYNU | 1 | 0.000833126 | 1.48 (1.18, 1.87) | 2.20% | 1.05 | 1.31 |
| KYNU | 1 | 0.000833126 | 1.48 (1.18, 1.87) | 2.20% | 1.05 | 1.31 |
| KYNU | 1 | 0.000833126 | 1.48 (1.18, 1.87) | 2.20% | 1.05 | 1.31 |
| LEKR1 | -1 | 0.001095433 | 0.55 (0.39, 0.79) | 2.60% | 1.05 | 1.34 |
| LEKR1 | -1 | 0.001095433 | 0.55 (0.39, 0.79) | 2.60% | 1.05 | 1.34 |
| LOC100129046 | -1 | 0.001505992 | 0.56 (0.40, 0.80) | 3.10% | 1.05 | 1.33 |
| LOC100130015 | 1 | 0.000973928 | 1.60 (1.21, 2.11) | 2.40% | 1.05 | 1.32 |
| LOC100130015 | 1 | 0.000973928 | 1.60 (1.21, 2.11) | 2.40% | 1.05 | 1.32 |
| LOC386758 | -1 | 0.001056927 | 0.60 (0.44, 0.82) | 2.50% | 1.05 | 1.34 |
| LOC386758 | -1 | 0.001056927 | 0.60 (0.44, 0.82) | 2.50% | 1.05 | 1.34 |
| LOC386758 | -1 | 0.001056927 | 0.60 (0.44, 0.82) | 2.50% | 1.05 | 1.34 |
| LOC389765 | -1 | 0.001342509 | 0.56 (0.39, 0.80) | 2.90% | 1.05 | 1.34 |
| LOC401242 | 1 | 0.000156056 | 1.27 (1.12, 1.43) | 0.70% | 1.05 | 1.23 |
| LRRC48 | -1 | 0.001316854 | 0.54 (0.38, 0.79) | 2.90% | 1.05 | 1.34 |
| LRRC48 | -1 | 0.001316854 | 0.54 (0.38, 0.79) | 2.90% | 1.05 | 1.34 |
| LRRC48 | -1 | 0.001316854 | 0.54 (0.38, 0.79) | 2.90% | 1.05 | 1.34 |
| LRRC48 | -1 | 0.001316854 | 0.54 (0.38, 0.79) | 2.90% | 1.05 | 1.34 |
| MAGEA12 | 1 | 0.000281529 | 1.29 (1.12, 1.48) | 1.10% | 1.05 | 1.24 |
| MAGEA12 | 1 | 0.000281529 | 1.29 (1.12, 1.48) | 1.10% | 1.05 | 1.24 |
| MAGEA12 | 1 | 0.000281529 | 1.29 (1.12, 1.48) | 1.10% | 1.05 | 1.24 |
| MLF1IP | 1 | 0.00175043 | 1.95 (1.28, 2.97) | 3.40% | 1.05 | 1.3 |
| MRPL54 | -1 | 0.001508008 | 0.52 (0.35, 0.78) | 3.10% | 1.05 | 1.33 |
| MTNR1A | 1 | 0.001122268 | 1.71 (1.24, 2.36) | 2.60% | 1.05 | 1.32 |
| MYBL2 | 1 | 0.001307241 | 1.82 (1.26, 2.63) | 2.90% | 1.05 | 1.32 |
| NBPF1 | -1 | 0.001260206 | 0.58 (0.41, 0.81) | 2.80% | 1.05 | 1.34 |
| NCBP1 | -1 | 0.001356126 | 0.57 (0.41, 0.80) | 2.90% | 1.05 | 1.33 |
| NCOA5 | -1 | 0.001192077 | 0.55 (0.39, 0.79) | 2.70% | 1.05 | 1.34 |
| NUBP1 | -1 | 0.000937501 | 0.59 (0.43, 0.81) | 2.40% | 1.05 | 1.34 |
| NUP155 | 1 | 0.00100456 | 1.82 (1.27, 2.59) | 2.50% | 1.05 | 1.33 |
| NUP155 | 1 | 0.00100456 | 1.82 (1.27, 2.59) | 2.50% | 1.05 | 1.33 |
| OPN3 | 1 | 0.001078316 | 1.89 (1.29, 2.78) | 2.60% | 1.05 | 1.32 |
| PABPC1 | 1 | 0.001122107 | 1.62 (1.21, 2.17) | 2.60% | 1.05 | 1.32 |
| PACRGL | -1 | 0.000964235 | 0.59 (0.43, 0.81) | 2.40% | 1.05 | 1.34 |
| PACRGL | -1 | 0.000964235 | 0.59 (0.43, 0.81) | 2.40% | 1.05 | 1.34 |
| PALM | -1 | 0.000885604 | 0.59 (0.43, 0.80) | 2.30% | 1.05 | 1.35 |
| PALM | -1 | 0.000885604 | 0.59 (0.43, 0.80) | 2.30% | 1.05 | 1.35 |
| PHF2 | -1 | 0.001491851 | 0.58 (0.41, 0.81) | 3.10% | 1.05 | 1.33 |
| PIF1 | 1 | 0.001559389 | 2.22 (1.35, 3.64) | 3.20% | 1.05 | 1.29 |
| PLA2G16 | -1 | 0.001171353 | 0.52 (0.35, 0.77) | 2.70% | 1.05 | 1.34 |
| PLA2G16 | -1 | 0.001171353 | 0.52 (0.35, 0.77) | 2.70% | 1.05 | 1.34 |
| PNPLA4 | -1 | 0.001295225 | 0.55 (0.38, 0.79) | 2.90% | 1.05 | 1.34 |
| PNPLA4 | -1 | 0.001295225 | 0.55 (0.38, 0.79) | 2.90% | 1.05 | 1.34 |
| PNPLA4 | -1 | 0.001295225 | 0.55 (0.38, 0.79) | 2.90% | 1.05 | 1.34 |
| PPIA | 1 | 0.000543774 | 1.42 (1.16, 1.73) | 1.70% | 1.05 | 1.3 |
| PPM1M | -1 | 0.000929062 | 0.67 (0.52, 0.85) | 2.40% | 1.05 | 1.32 |
| PPM1M | -1 | 0.000929062 | 0.67 (0.52, 0.85) | 2.40% | 1.05 | 1.32 |
| PPP1CB | 1 | 0.001307362 | 1.75 (1.24, 2.45) | 2.90% | 1.05 | 1.32 |
| PPP1CB | 1 | 0.001307362 | 1.75 (1.24, 2.45) | 2.90% | 1.05 | 1.32 |
| PRRG1 | 1 | 0.001335665 | 2.05 (1.32, 3.17) | 2.90% | 1.05 | 1.3 |
| PRRG1 | 1 | 0.001335665 | 2.05 (1.32, 3.17) | 2.90% | 1.05 | 1.3 |
| PRRG1 | 1 | 0.001335665 | 2.05 (1.32, 3.17) | 2.90% | 1.05 | 1.3 |
| PRRG1 | 1 | 0.001335665 | 2.05 (1.32, 3.17) | 2.90% | 1.05 | 1.3 |
| PRRG1 | 1 | 0.001335665 | 2.05 (1.32, 3.17) | 2.90% | 1.05 | 1.3 |
| PYGO1 | -1 | 0.000933796 | 0.59 (0.43, 0.80) | 2.40% | 1.05 | 1.35 |
| RASGEF1C | 1 | 0.000782881 | 1.63 (1.23, 2.17) | 2.10% | 1.05 | 1.33 |
| RBBP6 | -1 | 0.001298068 | 0.53 (0.36, 0.78) | 2.90% | 1.05 | 1.33 |
| RBBP6 | -1 | 0.001298068 | 0.53 (0.36, 0.78) | 2.90% | 1.05 | 1.33 |
| RBBP6 | -1 | 0.001298068 | 0.53 (0.36, 0.78) | 2.90% | 1.05 | 1.33 |
| RBM24 | -1 | 0.00141751 | 0.54 (0.37, 0.79) | 3.00% | 1.05 | 1.33 |
| RBM24 | -1 | 0.00141751 | 0.54 (0.37, 0.79) | 3.00% | 1.05 | 1.33 |
| RBM24 | -1 | 0.00141751 | 0.54 (0.37, 0.79) | 3.00% | 1.05 | 1.33 |
| REM2 | -1 | 0.000882487 | 0.63 (0.48, 0.83) | 2.30% | 1.05 | 1.34 |
| SEPSECS | -1 | 0.001221472 | 0.59 (0.43, 0.81) | 2.80% | 1.05 | 1.34 |
| SHCBP1 | 1 | 0.001555367 | 1.94 (1.29, 2.93) | 3.20% | 1.05 | 1.3 |
| SLC25A12 | -1 | 0.001161342 | 0.53 (0.36, 0.78) | 2.70% | 1.05 | 1.34 |
| SRP9 | 1 | 0.001180657 | 1.64 (1.22, 2.22) | 2.70% | 1.05 | 1.32 |
| SRP9 | 1 | 0.001180657 | 1.64 (1.22, 2.22) | 2.70% | 1.05 | 1.32 |
| SYT6 | -1 | 0.00123582 | 0.51 (0.34, 0.77) | 2.80% | 1.05 | 1.33 |
| TARBP2 | -1 | 0.000916321 | 0.62 (0.47, 0.82) | 2.30% | 1.05 | 1.34 |
| TARBP2 | -1 | 0.000916321 | 0.62 (0.47, 0.82) | 2.30% | 1.05 | 1.34 |
| TARBP2 | -1 | 0.000916321 | 0.62 (0.47, 0.82) | 2.30% | 1.05 | 1.34 |
| TCAM1P | 1 | 0.000940114 | 1.64 (1.22, 2.20) | 2.40% | 1.05 | 1.32 |
| THAP4 | -1 | 0.001221209 | 0.55 (0.38, 0.79) | 2.80% | 1.05 | 1.34 |
| THAP4 | -1 | 0.001221209 | 0.55 (0.38, 0.79) | 2.80% | 1.05 | 1.34 |
| TLR3 | -1 | 0.001127044 | 0.59 (0.43, 0.81) | 2.60% | 1.05 | 1.34 |
| TMEM127 | -1 | 0.000890354 | 0.59 (0.43, 0.81) | 2.30% | 1.05 | 1.35 |
| TMEM127 | -1 | 0.000890354 | 0.59 (0.43, 0.81) | 2.30% | 1.05 | 1.35 |
| TPTE | 1 | 0.000758552 | 1.57 (1.21, 2.04) | 2.10% | 1.05 | 1.32 |
| TPTE | 1 | 0.000758552 | 1.57 (1.21, 2.04) | 2.10% | 1.05 | 1.32 |
| TPTE | 1 | 0.000758552 | 1.57 (1.21, 2.04) | 2.10% | 1.05 | 1.32 |
| TRNP1 | -1 | 0.001093981 | 0.62 (0.46, 0.83) | 2.60% | 1.05 | 1.33 |
| TRPV1 | -1 | 0.001166172 | 0.51 (0.34, 0.77) | 2.70% | 1.05 | 1.34 |
| TRPV1 | -1 | 0.001166172 | 0.51 (0.34, 0.77) | 2.70% | 1.05 | 1.34 |
| TRPV1 | -1 | 0.001166172 | 0.51 (0.34, 0.77) | 2.70% | 1.05 | 1.34 |
| TRPV1 | -1 | 0.001166172 | 0.51 (0.34, 0.77) | 2.70% | 1.05 | 1.34 |
| TTC23L | -1 | 0.001233032 | 0.57 (0.41, 0.80) | 2.80% | 1.05 | 1.34 |
| TTC26 | -1 | 0.000762816 | 0.66 (0.52, 0.84) | 2.10% | 1.05 | 1.33 |
| TTC26 | -1 | 0.000762816 | 0.66 (0.52, 0.84) | 2.10% | 1.05 | 1.33 |
| TTC26 | -1 | 0.000762816 | 0.66 (0.52, 0.84) | 2.10% | 1.05 | 1.33 |
| UNC5B | -1 | 0.001174314 | 0.61 (0.46, 0.82) | 2.70% | 1.05 | 1.33 |
| USP9X | 1 | 0.001402489 | 1.73 (1.24, 2.43) | 3.00% | 1.05 | 1.32 |
| USP9X | 1 | 0.001402489 | 1.73 (1.24, 2.43) | 3.00% | 1.05 | 1.32 |
| WDR16 | -1 | 0.001158488 | 0.53 (0.36, 0.78) | 2.70% | 1.05 | 1.34 |
| WDR16 | -1 | 0.001158488 | 0.53 (0.36, 0.78) | 2.70% | 1.05 | 1.34 |
| YWHAE | -1 | 0.001115707 | 0.52 (0.35, 0.77) | 2.60% | 1.05 | 1.34 |
| YWHAE | -1 | 0.001115707 | 0.52 (0.35, 0.77) | 2.60% | 1.05 | 1.34 |
| ZBTB26 | -1 | 0.001225918 | 0.59 (0.43, 0.81) | 2.80% | 1.05 | 1.34 |
| ZFP112 | -1 | 0.00123204 | 0.54 (0.37, 0.78) | 2.80% | 1.05 | 1.34 |
| ZFP112 | -1 | 0.00123204 | 0.54 (0.37, 0.78) | 2.80% | 1.05 | 1.34 |
| ZFP28 | -1 | 0.000981399 | 0.58 (0.42, 0.80) | 2.40% | 1.05 | 1.34 |
| ZFP3 | -1 | 0.001069592 | 0.56 (0.40, 0.79) | 2.60% | 1.05 | 1.34 |
| ZNF358 | -1 | 0.000693963 | 0.66 (0.52, 0.84) | 2.00% | 1.05 | 1.33 |
| ZNF420 | -1 | 0.001324154 | 0.57 (0.40, 0.80) | 2.90% | 1.05 | 1.34 |
| ZNF597 | -1 | 0.000903345 | 0.59 (0.44, 0.81) | 2.30% | 1.05 | 1.34 |
| ZNF610 | -1 | 0.001280906 | 0.60 (0.44, 0.82) | 2.80% | 1.05 | 1.33 |
| ZNF610 | -1 | 0.001280906 | 0.60 (0.44, 0.82) | 2.80% | 1.05 | 1.33 |
| ZNF610 | -1 | 0.001280906 | 0.60 (0.44, 0.82) | 2.80% | 1.05 | 1.33 |
| ZNF610 | -1 | 0.001280906 | 0.60 (0.44, 0.82) | 2.80% | 1.05 | 1.33 |
| ZNF862 | -1 | 0.00105257 | 0.55 (0.39, 0.79) | 2.50% | 1.05 | 1.34 |
| ABCA12 | 1 | 0.002161248 | 1.74 (1.22, 2.47) | 3.90% | 1.04 | 1.3 |
| ABCA12 | 1 | 0.002161248 | 1.74 (1.22, 2.47) | 3.90% | 1.04 | 1.3 |
| ACSMS | -1 | 0.002054386 | 0.56 (0.39, 0.81) | 3.80% | 1.04 | 1.32 |
| ADM2 | 1 | 0.001274536 | 1.46 (1.16, 1.84) | 2.80% | 1.04 | 1.29 |
| AMDHD1 | -1 | 0.001536666 | 0.61 (0.45, 0.83) | 3.10% | 1.04 | 1.33 |
| AMN1 | -1 | 0.002178846 | 0.52 (0.35, 0.79) | 3.90% | 1.04 | 1.31 |
| AMN1 | -1 | 0.002178846 | 0.52 (0.35, 0.79) | 3.90% | 1.04 | 1.31 |
| ANKAR | -1 | 0.002007581 | 0.62 (0.46, 0.84) | 3.70% | 1.04 | 1.32 |
| B3GNT8 | -1 | 0.001926614 | 0.55 (0.38, 0.80) | 3.60% | 1.04 | 1.32 |
| BBS5 | -1 | 0.001546773 | 0.60 (0.44, 0.82) | 3.20% | 1.04 | 1.33 |
| BDNF-AS1 | -1 | 0.001646972 | 0.64 (0.48, 0.84) | 3.30% | 1.04 | 1.32 |
| BDNF-AS1 | -1 | 0.001646972 | 0.64 (0.48, 0.84) | 3.30% | 1.04 | 1.32 |
| BDNF-AS1 | -1 | 0.001646972 | 0.64 (0.48, 0.84) | 3.30% | 1.04 | 1.32 |
| BDNF-AS1 | -1 | 0.001646972 | 0.64 (0.48, 0.84) | 3.30% | 1.04 | 1.32 |
| BDNF-AS1 | -1 | 0.001646972 | 0.64 (0.48, 0.84) | 3.30% | 1.04 | 1.32 |
| BZRAP1 | -1 | 0.001603336 | 0.58 (0.41, 0.81) | 3.20% | 1.04 | 1.33 |
| BZRAP1 | -1 | 0.001603336 | 0.58 (0.41, 0.81) | 3.20% | 1.04 | 1.33 |
| C22orf9 | -1 | 0.001457169 | 0.59 (0.42, 0.81) | 3.10% | 1.04 | 1.33 |
| C22orf9 | -1 | 0.001457169 | 0.59 (0.42, 0.81) | 3.10% | 1.04 | 1.33 |
| C5orf22 | 1 | 0.00164001 | 1.82 (1.25, 2.64) | 3.30% | 1.04 | 1.31 |
| C7orf30 | 1 | 0.001468584 | 1.43 (1.15, 1.79) | 3.10% | 1.04 | 1.28 |
| C7orf53 | 1 | 0.00256683 | 1.97 (1.27, 3.06) | 4.30% | 1.04 | 1.28 |
| C7orf53 | 1 | 0.00256683 | 1.97 (1.27, 3.06) | 4.30% | 1.04 | 1.28 |
| C8orf75 | 1 | 0.001454158 | 1.62 (1.20, 2.19) | 3.10% | 1.04 | 1.31 |
| C9orf89 | -1 | 0.001984738 | 0.52 (0.34, 0.78) | 3.70% | 1.04 | 1.32 |
| CBX3 | 1 | 0.001431883 | 1.40 (1.14, 1.72) | 3.00% | 1.04 | 1.27 |
| CBX3 | 1 | 0.001431883 | 1.40 (1.14, 1.72) | 3.00% | 1.04 | 1.27 |
| CELA2B | -1 | 0.001884979 | 0.56 (0.39, 0.81) | 3.60% | 1.04 | 1.32 |
| CENPF | 1 | 0.002672209 | 2.01 (1.28, 3.18) | 4.40% | 1.04 | 1.28 |
| CENPI | 1 | 0.00200314 | 1.76 (1.23, 2.52) | 3.70% | 1.04 | 1.3 |
| CLRN1 | -1 | 0.001811077 | 0.59 (0.42, 0.82) | 3.50% | 1.04 | 1.32 |
| CLRN1 | -1 | 0.001811077 | 0.59 (0.42, 0.82) | 3.50% | 1.04 | 1.32 |
| CLRN1 | -1 | 0.001811077 | 0.59 (0.42, 0.82) | 3.50% | 1.04 | 1.32 |
| CNOT1 | 1 | 0.002044545 | 1.67 (1.20, 2.31) | 3.80% | 1.04 | 1.3 |
| CNOT1 | 1 | 0.002044545 | 1.67 (1.20, 2.31) | 3.80% | 1.04 | 1.3 |
| COX4I1 | 1 | 0.001837639 | 1.78 (1.24, 2.56) | 3.50% | 1.04 | 1.31 |
| CREBBP | -1 | 0.002076809 | 0.56 (0.39, 0.81) | 3.80% | 1.04 | 1.32 |
| CREBBP | -1 | 0.002076809 | 0.56 (0.39, 0.81) | 3.80% | 1.04 | 1.32 |
| DCP1B | -1 | 0.002232642 | 0.55 (0.37, 0.81) | 4.00% | 1.04 | 1.32 |
| DEGS2 | -1 | 0.002313022 | 0.58 (0.41, 0.83) | 4.10% | 1.04 | 1.32 |
| DNAH6 | -1 | 0.001467242 | 0.62 (0.46, 0.83) | 3.10% | 1.04 | 1.33 |
| DYNLRB2 | -1 | 0.001832614 | 0.62 (0.46, 0.84) | 3.50% | 1.04 | 1.32 |
| EIF3E | 1 | 0.001989926 | 1.72 (1.22, 2.42) | 3.70% | 1.04 | 1.3 |
| EPRS | 1 | 0.002365345 | 1.81 (1.24, 2.67) | 4.10% | 1.04 | 1.29 |
| ETFDH | -1 | 0.00230326 | 0.58 (0.41, 0.82) | 4.10% | 1.04 | 1.32 |
| FAM131C | 1 | 0.002298543 | 1.77 (1.23, 2.55) | 4.10% | 1.04 | 1.3 |
| GALC | -1 | 0.001803912 | 0.61 (0.44, 0.83) | 3.50% | 1.04 | 1.32 |
| GALC | -1 | 0.001803912 | 0.61 (0.44, 0.83) | 3.50% | 1.04 | 1.32 |
| GALNT14 | 1 | 0.001837704 | 1.76 (1.23, 2.52) | 3.50% | 1.04 | 1.31 |
| HEATR2 | 1 | 0.001823293 | 1.54 (1.18, 2.03) | 3.50% | 1.04 | 1.3 |
| HEATR3 | 1 | 0.001554545 | 1.73 (1.23, 2.42) | 3.20% | 1.04 | 1.31 |
| HEATR7A | 1 | 0.001790133 | 1.61 (1.19, 2.18) | 3.50% | 1.04 | 1.3 |
| HEATR7A | 1 | 0.001790133 | 1.61 (1.19, 2.18) | 3.50% | 1.04 | 1.3 |
| HEATR7A | 1 | 0.001790133 | 1.61 (1.19, 2.18) | 3.50% | 1.04 | 1.3 |
| HMGCS1 | 1 | 0.001069341 | 1.47 (1.17, 1.85) | 2.60% | 1.04 | 1.3 |
| HMGCS1 | 1 | 0.001069341 | 1.47 (1.17, 1.85) | 2.60% | 1.04 | 1.3 |
| HOXB9 | 1 | 0.001509092 | 1.61 (1.20, 2.17) | 3.10% | 1.04 | 1.31 |
| IFT74 | -1 | 0.001765925 | 0.58 (0.42, 0.82) | 3.40% | 1.04 | 1.33 |
| IFT74 | -1 | 0.001765925 | 0.58 (0.42, 0.82) | 3.40% | 1.04 | 1.33 |
| IFT74 | -1 | 0.001765925 | 0.58 (0.42, 0.82) | 3.40% | 1.04 | 1.33 |
| IFT74 | -1 | 0.001765925 | 0.58 (0.42, 0.82) | 3.40% | 1.04 | 1.33 |
| KCNK5 | 1 | 0.001161172 | 1.52 (1.18, 1.97) | 2.70% | 1.04 | 1.31 |
| KDELR2 | 1 | 0.001508021 | 1.70 (1.22, 2.35) | 3.10% | 1.04 | 1.31 |
| KDELR2 | 1 | 0.001508021 | 1.70 (1.22, 2.35) | 3.10% | 1.04 | 1.31 |
| KDM4B | -1 | 0.001823372 | 0.53 (0.35, 0.79) | 3.50% | 1.04 | 1.32 |
| KIAA0226 | 1 | 0.001751246 | 1.55 (1.18, 2.04) | 3.40% | 1.04 | 1.3 |
| KIAA0226 | 1 | 0.001751246 | 1.55 (1.18, 2.04) | 3.40% | 1.04 | 1.3 |
| KIAA1737 | -1 | 0.002117405 | 0.59 (0.42, 0.83) | 3.90% | 1.04 | 1.32 |
| LOC100130581 | -1 | 0.00105557 | 0.72 (0.59, 0.88) | 2.50% | 1.04 | 1.29 |
| LOC100130581 | -1 | 0.00105557 | 0.72 (0.59, 0.88) | 2.50% | 1.04 | 1.29 |
| LOC100288730 | -1 | 0.002074681 | 0.55 (0.37, 0.80) | 3.80% | 1.04 | 1.32 |
| LOC285629 | 1 | 0.001639129 | 1.47 (1.16, 1.87) | 3.30% | 1.04 | 1.29 |
| LOC348021 | 1 | 0.000629483 | 1.26 (1.10, 1.44) | 1.80% | 1.04 | 1.22 |
| LOC389033 | -1 | 0.001863566 | 0.56 (0.38, 0.81) | 3.50% | 1.04 | 1.32 |
| LOC494141 | 1 | 0.001552204 | 1.50 (1.17, 1.92) | 3.20% | 1.04 | 1.3 |
| LOC494141 | 1 | 0.001552204 | 1.50 (1.17,1.92) | 3.20% | 1.04 | 1.3 |
| LOC494141 | 1 | 0.001552204 | 1.50 (1.17, 1.92) | 3.20% | 1.04 | 1.3 |
| LPPR2 | -1 | 0.001724289 | 0.62 (0.45, 0.83) | 3.40% | 1.04 | 1.32 |
| LPPR2 | -1 | 0.001724289 | 0.62 (0.45, 0.83) | 3.40% | 1.04 | 1.32 |
| LRP12 | 1 | 0.001577381 | 1.60 (1.20, 2.15) | 3.20% | 1.04 | 1.31 |
| LRP12 | 1 | 0.001577381 | 1.60 (1.20, 2.15) | 3.20% | 1.04 | 1.31 |
| LRP2 | -1 | 0.001813236 | 0.55 (0.38, 0.80) | 3.50% | 1.04 | 1.32 |
| LYPD2 | 1 | 0.001349535 | 1.39 (1.14, 1.70) | 2.90% | 1.04 | 1.27 |
| MAP7D1 | -1 | 0.001687535 | 0.57 (0.40, 0.81) | 3.30% | 1.04 | 1.33 |
| MED16 | -1 | 0.001899319 | 0.59 (0.42, 0.82) | 3.60% | 1.04 | 1.32 |
| MEMO1 | 1 | 0.002174585 | 2.05 (1.29, 3.24) | 3.90% | 1.04 | 1.28 |
| MEMO1 | 1 | 0.002174585 | 2.05 (1.29, 3.24) | 3.90% | 1.04 | 1.28 |
| MICAL3 | 1 | 0.001535676 | 1.71 (1.23, 2.38) | 3.10% | 1.04 | 1.31 |
| MICAL3 | 1 | 0.001535676 | 1.71 (1.23, 2.38) | 3.10% | 1.04 | 1.31 |
| MICAL3 | 1 | 0.001535676 | 1.71 (1.23, 2.38) | 3.10% | 1.04 | 1.31 |
| MIP | 1 | 0.000829718 | 1.39 (1.15, 1.70) | 2.20% | 1.04 | 1.28 |
| MKL2 | -1 | 0.001781182 | 0.53 (0.36, 0.79) | 3.50% | 1.04 | 1.32 |
| MPHOSPH6 | 1 | 0.001626864 | 1.51 (1.17, 1.96) | 3.30% | 1.04 | 1.3 |
| MSRA | -1 | 0.001531353 | 0.61 (0.44, 0.83) | 3.10% | 1.04 | 1.33 |
| MSRA | -1 | 0.001531353 | 0.61 (0.44, 0.83) | 3.10% | 1.04 | 1.33 |
| MSRA | -1 | 0.001531353 | 0.61 (0.44, 0.83) | 3.10% | 1.04 | 1.33 |
| MSRA | -1 | 0.001531353 | 0.61 (0.44, 0.83) | 3.10% | 1.04 | 1.33 |
| MTIF3 | -1 | 0.002132573 | 0.58 (0.41, 0.82) | 3.90% | 1.04 | 1.32 |
| MTIF3 | -1 | 0.002132573 | 0.58 (0.41, 0.82) | 3.90% | 1.04 | 1.32 |
| MTIF3 | -1 | 0.002132573 | 0.58 (0.41, 0.82) | 3.90% | 1.04 | 1.32 |
| MTIF3 | -1 | 0.002132573 | 0.58 (0.41, 0.82) | 3.90% | 1.04 | 1.32 |
| MUM1L1 | -1 | 0.001404473 | 0.61 (0.45, 0.82) | 3.00% | 1.04 | 1.33 |
| MUM1L1 | -1 | 0.001404473 | 0.61 (0.45, 0.82) | 3.00% | 1.04 | 1.33 |
| NCALD | 1 | 0.00188018 | 1.65 (1.20, 2.25) | 3.60% | 1.04 | 1.3 |
| NCALD | 1 | 0.00188018 | 1.65 (1.20, 2.25) | 3.60% | 1.04 | 1.3 |
| NCALD | 1 | 0.00188018 | 1.65 (1.20, 2.25) | 3.60% | 1.04 | 1.3 |
| NCALD | 1 | 0.00188018 | 1.65 (1.20, 2.25) | 3.60% | 1.04 | 1.3 |
| NCALD | 1 | 0.00188018 | 1.65 (1.20, 2.25) | 3.60% | 1.04 | 1.3 |
| NCALD | 1 | 0.00188018 | 1.65 (1.20, 2.25) | 3.60% | 1.04 | 1.3 |
| NCALD | 1 | 0.00188018 | 1.65 (1.20, 2.25) | 3.60% | 1.04 | 1.3 |
| NCALD | 1 | 0.00188018 | 1.65 (1.20, 2.25) | 3.60% | 1.04 | 1.3 |
| NCRNA00094 | 1 | 0.001484442 | 1.64 (1.21, 2.22) | 3.10% | 1.04 | 1.31 |
| NDUFA12 | -1 | 0.001796493 | 0.67 (0.52, 0.86) | 3.50% | 1.04 | 1.3 |
| NME1-NME2 | 1 | 0.001608921 | 1.59 (1.19, 2.12) | 3.20% | 1.04 | 1.31 |
| NME1-NME2 | 1 | 0.001608921 | 1.59 (1.19, 2.12) | 3.20% | 1.04 | 1.31 |
| NUP93 | 1 | 0.001568961 | 1.63 (1.20, 2.20) | 3.20% | 1.04 | 1.31 |
| NUTF2 | 1 | 0.002638761 | 2.03 (1.28, 3.21) | 4.40% | 1.04 | 1.28 |
| PARP11 | -1 | 0.001639941 | 0.56 (0.39, 0.80) | 3.30% | 1.04 | 1.33 |
| PCDH12 | -1 | 0.002217348 | 0.54 (0.36, 0.80) | 4.00% | 1.04 | 1.32 |
| PCOLCE | -1 | 0.001169185 | 0.69 (0.55, 0.86) | 2.70% | 1.04 | 1.3 |
| PIGA | 1 | 0.001611291 | 1.71 (1.23, 2.39) | 3.20% | 1.04 | 1.31 |
| PIGA | 1 | 0.001611291 | 1.71 (1.23, 2.39) | 3.20% | 1.04 | 1.31 |
| PIGA | 1 | 0.001611291 | 1.71 (1.23, 2.39) | 3.20% | 1.04 | 1.31 |
| PIGA | 1 | 0.001611291 | 1.71 (1.23, 2.39) | 3.20% | 1.04 | 1.31 |
| PRAMEF11 | 1 | 0.001218956 | 1.49 (1.17, 1.89) | 2.80% | 1.04 | 1.3 |
| PRDM4 | -1 | 0.002295022 | 0.57 (0.39, 0.82) | 4.10% | 1.04 | 1.32 |
| PRR11 | 1 | 0.001592327 | 1.68 (1.22, 2.31) | 3.20% | 1.04 | 1.31 |
| PRR25 | -1 | 0.00181547 | 0.61 (0.45, 0.83) | 3.50% | 1.04 | 1.32 |
| PVRL4 | 1 | 0.002362673 | 1.76 (1.22, 2.54) | 4.10% | 1.04 | 1.3 |
| RAD52 | -1 | 0.001826287 | 0.58 (0.42, 0.82) | 3.50% | 1.04 | 1.32 |
| RBM19 | -1 | 0.001710729 | 0.58 (0.41, 0.81) | 3.40% | 1.04 | 1.33 |
| RBM19 | -1 | 0.001710729 | 0.58 (0.41, 0.81) | 3.40% | 1.04 | 1.33 |
| RBM19 | -1 | 0.001710729 | 0.58 (0.41, 0.81) | 3.40% | 1.04 | 1.33 |
| RCHY1 | 1 | 0.001382457 | 1.40 (1.14, 1.71) | 3.00% | 1.04 | 1.28 |
| RCHY1 | 1 | 0.001382457 | 1.40 (1.14, 1.71) | 3.00% | 1.04 | 1.28 |
| RCHY1 | 1 | 0.001382457 | 1.40 (1.14, 1.71) | 3.00% | 1.04 | 1.28 |
| RGS12 | -1 | 0.00204249 | 0.62 (0.46, 0.84) | 3.80% | 1.04 | 1.32 |
| RGS12 | -1 | 0.00204249 | 0.62 (0.46, 0.84) | 3.80% | 1.04 | 1.32 |
| RGS12 | -1 | 0.00204249 | 0.62 (0.46, 0.84) | 3.80% | 1.04 | 1.32 |
| RHOV | 1 | 0.002052837 | 1.61 (1.19, 2.17) | 3.80% | 1.04 | 1.3 |
| RPL17-C180RF32 | 1 | 0.002254262 | 1.78 (1.23, 2.58) | 4.00% | 1.04 | 1.3 |
| RPL17-C18ORF32 | 1 | 0.002254262 | 1.78 (1.23, 2.58) | 4.00% | 1.04 | 1.3 |
| SCFD1 | 1 | 0.001110852 | 1.39 (1.14, 1.69) | 2.60% | 1.04 | 1.28 |
| SCFD1 | 1 | 0.001110852 | 1.39 (1.14, 1.69) | 2.60% | 1.04 | 1.28 |
| SENP5 | 1 | 0.001328803 | 1.44 (1.15, 1.80) | 2.90% | 1.04 | 1.29 |
| SERPINF1 | -1 | 0.001927763 | 0.58 (0.41, 0.82) | 3.60% | 1.04 | 1.32 |
| SGOL2 | 1 | 0.001766454 | 1.62 (1.20, 2.20) | 3.40% | 1.04 | 1.31 |
| SGOL2 | 1 | 0.001766454 | 1.62 (1.20, 2.20) | 3.40% | 1.04 | 1.31 |
| SGOL2 | 1 | 0.001766454 | 1.62 (1.20, 2.20) | 3.40% | 1.04 | 1.31 |
| SLC43A1 | -1 | 0.002668902 | 0.51 (0.33, 0.79) | 4.40% | 1.04 | 1.3 |
| SLC43A1 | -1 | 0.002668902 | 0.51 (0.33, 0.79) | 4.40% | 1.04 | 1.3 |
| SMPDL3A | 1 | 0.00123104 | 1.52 (1.18, 1.95) | 2.80% | 1.04 | 1.3 |
| SOX8 | 1 | 0.000931789 | 1.46 (1.17, 1.82) | 2.40% | 1.04 | 1.3 |
| SPRR1B | 1 | 0.000777744 | 1.29 (1.11, 1.50) | 2.10% | 1.04 | 1.24 |
| STX1B | -1 | 0.002150231 | 0.58 (0.41, 0.82) | 3.90% | 1.04 | 1.32 |
| SYNPO2L | -1 | 0.001652246 | 0.62 (0.45, 0.83) | 3.30% | 1.04 | 1.32 |
| SYNPO2L | -1 | 0.001652246 | 0.62 (0.45, 0.83) | 3.30% | 1.04 | 1.32 |
| TBCEL | -1 | 0.001739503 | 0.67 (0.52, 0.86) | 3.40% | 1.04 | 1.31 |
| TBCEL | -1 | 0.001739503 | 0.67 (0.52, 0.86) | 3.40% | 1.04 | 1.31 |
| TECTA | -1 | 0.00137001 | 0.68 (0.54, 0.86) | 2.90% | 1.04 | 1.31 |
| TMEM229B | -1 | 0.001467076 | 0.59 (0.43, 0.82) | 3.10% | 1.04 | 1.33 |
| TOR1A | -1 | 0.001857485 | 0.51 (0.33, 0.78) | 3.50% | 1.04 | 1.32 |
| TRIM15 | 1 | 0.001090987 | 1.36 (1.13, 1.64) | 2.60% | 1.04 | 1.27 |
| TYW3 | -1 | 0.001970278 | 0.62 (0.46, 0.84) | 3.70% | 1.04 | 1.32 |
| TYW3 | -1 | 0.001970278 | 0.62 (0.46, 0.84) | 3.70% | 1.04 | 1.32 |
| TYW3 | -1 | 0.001970278 | 0.62 (0.46, 0.84) | 3.70% | 1.04 | 1.32 |
| UBA6 | 1 | 0.001190224 | 1.54 (1.19, 2.00) | 2.70% | 1.04 | 1.31 |
| UBXN11 | -1 | 0.002331967 | 0.58 (0.41, 0.82) | 4.10% | 1.04 | 1.32 |
| UBXN11 | -1 | 0.002331967 | 0.58 (0.41, 0.82) | 4.10% | 1.04 | 1.32 |
| UBXN11 | -1 | 0.002331967 | 0.58 (0.41, 0.82) | 4.10% | 1.04 | 1.32 |
| USP28 | -1 | 0.001429182 | 0.60 (0.44, 0.82) | 3.00% | 1.04 | 1.33 |
| VSTM2B | 1 | 0.001630655 | 1.53 (1.18, 2.00) | 3.30% | 1.04 | 1.3 |
| WDR62 | 1 | 0.002082929 | 1.75 (1.22, 2.49) | 3.80% | 1.04 | 1.3 |
| WDR62 | 1 | 0.002082929 | 1.75 (1.22, 2.49) | 3.80% | 1.04 | 1.3 |
| WFDC12 | 1 | 0.001028037 | 1.38 (1.14, 1.67) | 2.50% | 1.04 | 1.27 |
| WFS1 | -1 | 0.001502849 | 0.62 (0.47, 0.84) | 3.10% | 1.04 | 1.32 |
| WFS1 | -1 | 0.001502849 | 0.62 (0.47, 0.84) | 3.10% | 1.04 | 1.32 |
| ZNF564 | -1 | 0.001983109 | 0.56 (0.39, 0.81) | 3.70% | 1.04 | 1.32 |
| ZNF627 | -1 | 0.001961641 | 0.59 (0A2, 0.82) | 3.70% | 1.04 | 1.32 |
| ZNF799 | -1 | 0.001796488 | 0.54 (0.37, 0.80) | 3.50% | 1.04 | 1.32 |
| ACBD3 | 1 | 0.003108527 | 1.67 (1.19, 2.34) | 4.80% | 1.03 | 1.29 |
| ACRBP | -1 | 0.002527232 | 0.59 (0A2, 0.83) | 4.30% | 1.03 | 1.31 |
| ADAM17 | 1 | 0.002756749 | 1.73 (1.21, 2.47) | 4.50% | 1.03 | 1.29 |
| AHCTF1 | 1 | 0.002833846 | 1.82 (1.23, 2.68) | 4.60% | 1.03 | 1.29 |
| AIDA | 1 | 0.002305105 | 1.42 (1.13, 1.77) | 4.10% | 1.03 | 1.27 |
| AK7 | -1 | 0.002397849 | 0.60 (0.43, 0.84) | 4.20% | 1.03 | 1.31 |
| AKD1 | -1 | 0.003540384 | 0.55 (0.37, 0.82) | 5.20% | 1.03 | 1.3 |
| AKD1 | -1 | 0.003540384 | 0.55 (0.37, 0.82) | 5.20% | 1.03 | 1.3 |
| ALB | 1 | 0.002271626 | 1.60 (1.18, 2.16) | 4.10% | 1.03 | 1.3 |
| ANKMY1 | -1 | 0.003302577 | 0.59 (0.42, 0.84) | 4.90% | 1.03 | 1.3 |
| ANKMY1 | -1 | 0.003302577 | 0.59 (042, 0.84) | 4.90% | 1.03 | 1.3 |
| ANKRD24 | -1 | 0.002274319 | 0.62 (0.46, 0.84) | 4.10% | 1.03 | 1.31 |
| ANKRD37 | 1 | 0.003334199 | 2.07 (1.27, 3.38) | 5.00% | 1.03 | 1.26 |
| ARHGEF6 | -1 | 0.003627718 | 0.60 (0.43, 0.85) | 5.30% | 1.03 | 1.3 |
| ASB18 | 1 | 0.002578858 | 1.74 (1.21, 2.48) | 4.40% | 1.03 | 1.29 |
| ATP2A2 | 1 | 0.003253492 | 1.60 (1.17, 2.18) | 4.90% | 1.03 | 1.29 |
| ATP2A2 | 1 | 0.003253492 | 1.60 (1.17, 2.18) | 4.90% | 1.03 | 1.29 |
| ATP2A2 | 1 | 0.003253492 | 1.60 (1.17, 2.18) | 4.90% | 1.03 | 1.29 |
| BAI2 | -1 | 0.003578065 | 0.60 (0.43, 0.85) | 5.20% | 1.03 | 1.3 |
| BANP | 1 | 0.003069281 | 1.50 (1.15, 1.96) | 4.80% | 1.03 | 1.28 |
| BANP | 1 | 0.003069281 | 1.50 (1.15, 1.96) | 4.80% | 1.03 | 1.28 |
| BANP | 1 | 0.003069281 | 1.50 (1.15, 1.96) | 4.80% | 1.03 | 1.28 |
| BANP | 1 | 0.003069281 | 1.50 (1.15, 1.96) | 4.80% | 1.03 | 1.28 |
| BANP | 1 | 0.003069281 | 1.50 (1.15, 1.96) | 4.80% | 1.03 | 1.28 |
| BANP | 1 | 0.003069281 | 1.50 (1.15, 1.96) | 4.80% | 1.03 | 1.28 |
| BANP | 1 | 0.003069281 | 1.50 (1.15, 1.96) | 4.80% | 1.03 | 1.28 |
| BLM | 1 | 0.003819008 | 1.79 (1.21, 2.65) | 5.40% | 1.03 | 1.28 |
| C10orf82 | -1 | 0.00290893 | 0.53 (0.35, 0.81) | 4.60% | 1.03 | 1.3 |
| C11orf34 | -1 | 0.003273096 | 0.60 (0.43, 0.84) | 4.90% | 1.03 | 1.3 |
| C12orf35 | -1 | 0.003559999 | 0.52 (0.33, 0.81) | 5.20% | 1.03 | 1.29 |
| C19orf76 | 1 | 0.002020895 | 1.50 (1.16, 1.94) | 3.70% | 1.03 | 1.29 |
| C1orf106 | 1 | 0.003304843 | 1.94 (1.25, 3.01) | 4.90% | 1.03 | 1.27 |
| C1orf106 | 1 | 0.003304843 | 1.94 (1.25, 3.01) | 4.90% | 1.03 | 1.27 |
| C20orf185 | 1 | 0.003007008 | 1.45 (1.13, 1.85) | 4.70% | 1.03 | 1.27 |
| C2orf65 | -1 | 0.002411185 | 0.61 (0.44, 0.84) | 4.20% | 1.03 | 1.31 |
| C4orf32 | -1 | 0.003456897 | 0.56 (0.38, 0.82) | 5.10% | 1.03 | 1.3 |
| C6orf124 | 1 | 0.002597838 | 1.72 (1.21, 2.46) | 4.40% | 1.03 | 1.29 |
| C9orf130 | -1 | 0.003706858 | 0.57 (0.39, 0.83) | 5.30% | 1.03 | 1.3 |
| C9orf130 | -1 | 0.003706858 | 0.57 (0.39, 0.83) | 5.30% | 1.03 | 1.3 |
| CADM4 | -1 | 0.002653642 | 0.63 (0.47, 0.85) | 4.40% | 1.03 | 1.31 |
| CALCOCO1 | -1 | 0.003000491 | 0.63 (0.46, 0.85) | 4.70% | 1.03 | 1.3 |
| CALCOCO1 | -1 | 0.003000491 | 0.63 (0.46, 0.85) | 4.70% | 1.03 | 1.3 |
| CALCOCO1 | -1 | 0.003000491 | 0.63 (0.46, 0.85) | 4.70% | 1.03 | 1.3 |
| CCDC144NL | 1 | 0.002348542 | 1.56 (1.17, 2.08) | 4.10% | 1.03 | 1.29 |
| CCDC76 | -1 | 0.004138708 | 0.53 (0.34, 0.82) | 5.60% | 1.03 | 1.29 |
| CCDC92 | -1 | 0.002468024 | 0.57 (0.40, 0.82) | 4.20% | 1.03 | 1.31 |
| CD300C | -1 | 0.002609931 | 0.60 (0.43, 0.84) | 4.40% | 1.03 | 1.31 |
| CD33 | -1 | 0.003435908 | 0.59 (0.42, 0.84) | 5.10% | 1.03 | 1.3 |
| CD33 | -1 | 0.003435908 | 0.59 (0.42, 0.84) | 5.10% | 1.03 | 1.3 |
| CD33 | -1 | 0.003435908 | 0.59 (0.42, 0.84) | 5.10% | 1.03 | 1.3 |
| CDC45 | 1 | 0.002832865 | 1.82 (1.23, 2.70) | 4.60% | 1.03 | 1.29 |
| CDC45 | 1 | 0.002832865 | 1.82 (1.23, 2.70) | 4.60% | 1.03 | 1.29 |
| CDC45 | 1 | 0.002832865 | 1.82 (1.23, 2.70) | 4.60% | 1.03 | 1.29 |
| CENPN | 1 | 0.003679416 | 1.73 (1.19, 2.50) | 5.30% | 1.03 | 1.28 |
| CENPN | 1 | 0.003679416 | 1.73 (1.19, 2.50) | 5.30% | 1.03 | 1.28 |
| CENPN | 1 | 0.003679416 | 1.73 (1.19, 2.50) | 5.30% | 1.03 | 1.28 |
| CENPV | -1 | 0.002354532 | 0.64 (0.49, 0.86) | 4.10% | 1.03 | 1.31 |
| CFHR1 | 1 | 0.001445939 | 1.29 (1.10, 1.51) | 3.10% | 1.03 | 1.23 |
| CHKA | 1 | 0.003263824 | 1.71 (1.20, 2.46) | 4.90% | 1.03 | 1.29 |
| CHKA | 1 | 0.003263824 | 1.71 (1.20, 2.46) | 4.90% | 1.03 | 1.29 |
| CLEC4A | -1 | 0.003825871 | 0.58 (0.40, 0.84) | 5.40% | 1.03 | 1.3 |
| CLEC4A | -1 | 0.003825871 | 0.58 (0.40, 0.84) | 5.40% | 1.03 | 1.3 |
| CLEC4A | -1 | 0.003825871 | 0.58 (0.40, 0.84) | 5.40% | 1.03 | 1.3 |
| CLEC4A | -1 | 0.003825871 | 0.58 (0.40, 0.84) | 5.40% | 1.03 | 1.3 |
| CLPTM1 | -1 | 0.00357314 | 0.60 (0.42, 0.85) | 5.20% | 1.03 | 1.3 |
| CLPTM1 | -1 | 0.00357314 | 0.60 (0.42, 0.85) | 5.20% | 1.03 | 1.3 |
| COL20A1 | 1 | 0.002174665 | 1.59 (1.18, 2.13) | 3.90% | 1.03 | 1.3 |
| COQ9 | 1 | 0.003239597 | 2.01 (1.26, 3.20) | 4.90% | 1.03 | 1.27 |
| CRYZ | -1 | 0.002899901 | 0.59 (0.42,0.84) | 4.60% | 1.03 | 1.31 |
| CRYZ | -1 | 0.002899901 | 0.59 (0.42, 0.84) | 4.60% | 1.03 | 1.31 |
| CRYZ | -1 | 0.002899901 | 0.59 (0.42, 0.84) | 4.60% | 1.03 | 1.31 |
| CRYZ | -1 | 0.002899901 | 0.59 (0.42, 0.84) | 4.60% | 1.03 | 1.31 |
| CYB5D1 | -1 | 0.003353239 | 0.67 (0.51, 0.87) | 5.00% | 1.03 | 1.29 |
| DCDC2 | -1 | 0.00250085 | 0.59 (0.42, 0.83) | 4.30% | 1.03 | 1.31 |
| DCDC2 | -1 | 0.00250085 | 0.59 (0.42, 0.83) | 4.30% | 1.03 | 1.31 |
| DEFB112 | 1 | 0.00201406 | 1.43 (1.14,1.79) | 3.70% | 1.03 | 1.28 |
| DFNA5 | 1 | 0.002721206 | 1.53 (1.16, 2.02) | 4.50% | 1.03 | 1.29 |
| DFNA5 | 1 | 0.002721206 | 1.53 (1.16, 2.02) | 4.50% | 1.03 | 1.29 |
| DFNA5 | 1 | 0.002721206 | 1.53 (1.16, 2.02) | 4.50% | 1.03 | 1.29 |
| DIO1 | -1 | 0.002787562 | 0.63 (0.46, 0.85) | 4.50% | 1.03 | 1.31 |
| DIO1 | -1 | 0.002787562 | 0.63 (0.46, 0.85) | 4.50% | 1.03 | 1.31 |
| DIO1 | -1 | 0.002787562 | 0.63 (0.46, 0.85) | 4.50% | 1.03 | 1.31 |
| DIO1 | -1 | 0.002787562 | 0.63 (0.46, 0.85) | 4.50% | 1.03 | 1.31 |
| DIS3L | -1 | 0.003001161 | 0.56 (0.38, 0.82) | 4.70% | 1.03 | 1.31 |
| DIS3L | -1 | 0.003001161 | 0.56 (0.38, 0.82) | 4.70% | 1.03 | 1.31 |
| DLGAP5 | 1 | 0.00278647 | 1.94 (1.26, 2.99) | 4.50% | 1.03 | 1.28 |
| DLGAP5 | 1 | 0.00278647 | 1.94 (1.26, 2.99) | 4.50% | 1.03 | 1.28 |
| DLX4 | 1 | 0.00416168 | 1.88 (1.22, 2.89) | 5.60% | 1.03 | 1.27 |
| DLX4 | 1 | 0.00416168 | 1.88 (1.22, 2.89) | 5.60% | 1.03 | 1.27 |
| DNMT3B | 1 | 0.002143803 | 1.59 (1.18, 2.13) | 3.90% | 1.03 | 1.3 |
| DNMT3B | 1 | 0.002143803 | 1.59 (1.18, 2.13) | 3.90% | 1.03 | 1.3 |
| DNMT3B | 1 | 0.002143803 | 1.59 (1.18, 2.13) | 3.90% | 1.03 | 1.3 |
| DNMT3B | 1 | 0.002143803 | 1.59 (1.18, 2.13) | 3.90% | 1.03 | 1.3 |
| DPM3 | 1 | 0.003717491 | 1.78 (1.21, 2.63) | 5.30% | 1.03 | 1.28 |
| DROSHA | 1 | 0.002278248 | 1.67 (1.20, 2.33) | 4.10% | 1.03 | 1.3 |
| DROSHA | 1 | 0.002278248 | 1.67 (1.20, 2.33) | 4.10% | 1.03 | 1.3 |
| DYNC1LI2 | 1 | 0.002945366 | 1.68 (1.19, 2.36) | 4.70% | 1.03 | 1.29 |
| DYX1C1 | -1 | 0.003170085 | 0.66 (0.50, 0.87) | 4.90% | 1.03 | 1.3 |
| DYX1C1 | -1 | 0.003170085 | 0.66 (0.50, 0.87) | 4.90% | 1.03 | 1.3 |
| DYX1C1 | -1 | 0.003170085 | 0.66 (0.50, 0.87) | 4.90% | 1.03 | 1.3 |
| ECEL1 | 1 | 0.001529034 | 1.25 (1.09, 1.44) | 3.10% | 1.03 | 1.21 |
| ENDOG | -1 | 0.002977538 | 0.60 (0.43, 0.84) | 4.70% | 1.03 | 1.31 |
| EPOR | -1 | 0.002953308 | 0.65 (0.49, 0.86) | 4.70% | 1.03 | 1.3 |
| EPOR | -1 | 0.002953308 | 0.65 (0.49, 0.86) | 4.70% | 1.03 | 1.3 |
| ESRP2 | 1 | 0.00358533 | 1.74 (1.20, 2.53) | 5.20% | 1.03 | 1.28 |
| FAM149A | -1 | 0.003077443 | 0.60 (0.43, 0.84) | 4.80% | 1.03 | 1.31 |
| FAM149A | -1 | 0.003077443 | 0.60 (0.43, 0.84) | 4.80% | 1.03 | 1.31 |
| FAM35A | -1 | 0.003261698 | 0.62 (0.45, 0.85) | 4.90% | 1.03 | 1.3 |
| FAM38B | -1 | 0.003890881 | 0.43 (0.24, 0.76) | 5.40% | 1.03 | 1.26 |
| FAM73A | -1 | 0.003419709 | 0.47 (0.29, 0.78) | 5.10% | 1.03 | 1.28 |
| FBX05 | 1 | 0.002655563 | 1.89 (1.25, 2.86) | 4.40% | 1.03 | 1.29 |
| FBX05 | 1 | 0.002655563 | 1.89 (1.25, 2.86) | 4.40% | 1.03 | 1.29 |
| FGG | 1 | 0.002203049 | 1.41 (1.13, 1.76) | 4.00% | 1.03 | 1.27 |
| FGG | 1 | 0.002203049 | 1.41 (1.13, 1.76) | 4.00% | 1.03 | 1.27 |
| FU14186 | 1 | 0.002707068 | 1.37 (1.12, 1.68) | 4.50% | 1.03 | 1.26 |
| FU42969 | 1 | 0.002377951 | 1.52 (1.16, 2.00) | 4.10% | 1.03 | 1.29 |
| FSTL3 | -1 | 0.003015843 | 0.60 (0.43, 0.84) | 4.70% | 1.03 | 1.31 |
| GFOD2 | 1 | 0.00388773 | 1.92 (1.23, 2.98) | 5.40% | 1.03 | 1.27 |
| GFOD2 | 1 | 0.00388773 | 1.92 (1.23, 2.98) | 5.40% | 1.03 | 1.27 |
| GFOD2 | 1 | 0.00388773 | 1.92 (1.23, 2.98) | 5.40% | 1.03 | 1.27 |
| GJC3 | -1 | 0.002130385 | 0.66 (0.51, 0.86) | 3.90% | 1.03 | 1.31 |
| GLG1 | 1 | 0.00232853 | 1.60 (1.18, 2.15) | 4.10% | 1.03 | 1.3 |
| GLG1 | 1 | 0.00232853 | 1.60 (1.18, 2.15) | 4.10% | 1.03 | 1.3 |
| GLG1 | 1 | 0.00232853 | 1.60 (1.18, 2.15) | 4.10% | 1.03 | 1.3 |
| GLG1 | 1 | 0.00232853 | 1.60 (1.18, 2.15) | 4.10% | 1.03 | 1.3 |
| GLG1 | 1 | 0.00232853 | 1.60 (1.18, 2.15) | 4.10% | 1.03 | 1.3 |
| GMPR2 | -1 | 0.002889811 | 0.61 (0.44, 0.84) | 4.60% | 1.03 | 1.31 |
| GMPR2 | -1 | 0.002889811 | 0.61 (0.44, 0.84) | 4.60% | 1.03 | 1.31 |
| GMPR2 | -1 | 0.002889811 | 0.61 (0.44, 0.84) | 4.60% | 1.03 | 1.31 |
| GMPR2 | -1 | 0.002889811 | 0.61 (0.44, 0.84) | 4.60% | 1.03 | 1.31 |
| GNPDA2 | -1 | 0.003200059 | 0.57 (0.39, 0.83) | 4.90% | 1.03 | 1.3 |
| GPR78 | 1 | 0.001899031 | 1.30 (1.10, 1.54) | 3.60% | 1.03 | 1.23 |
| HDGFRP2 | -1 | 0.002470922 | 0.58 (0.41, 0.82) | 4.20% | 1.03 | 1.31 |
| HDGFRP2 | -1 | 0.002470922 | 0.58 (0.41, 0.82) | 4.20% | 1.03 | 1.31 |
| HDGFRP3 | -1 | 0.002866543 | 0.58 (0.41, 0.83) | 4.60% | 1.03 | 1.31 |
| HLA-DQB2 | -1 | 0.002601447 | 0.61 (0.44, 0.84) | 4.40% | 1.03 | 1.31 |
| HMX2 | 1 | 0.001633074 | 1.36 (1.12, 1.66) | 3.30% | 1.03 | 1.26 |
| IVNS1ABP | 1 | 0.003215304 | 1.62 (1.17, 2.22) | 4.90% | 1.03 | 1.29 |
| JKAMP | -1 | 0.002816042 | 0.58 (0.40, 0.83) | 4.60% | 1.03 | 1.31 |
| JKAMP | -1 | 0.002816042 | 0.58 (0.40, 0.83) | 4.60% | 1.03 | 1.31 |
| KCNMB2 | 1 | 0.002365787 | 1.55 (1.17, 2.05) | 4.10% | 1.03 | 1.29 |
| KCNMB2 | 1 | 0.002365787 | 1.55 (1.17, 2.05) | 4.10% | 1.03 | 1.29 |
| KIAA0182 | 1 | 0.00400237 | 1.79 (1.21, 2.67) | 5.50% | 1.03 | 1.27 |
| KIAA0182 | 1 | 0.00400237 | 1.79 (1.21, 2.67) | 5.50% | 1.03 | 1.27 |
| KIAA1524 | 1 | 0.002658234 | 1.76 (1.22, 2.56) | 4.40% | 1.03 | 1.29 |
| KIAA1609 | 1 | 0.003411463 | 1.72 (1.20, 247) | 5.10% | 1.03 | 1.28 |
| KIF14 | 1 | 0.002967379 | 1.90 (1.24, 2.91) | 4.70% | 1.03 | 1.28 |
| KIF15 | 1 | 0.002847341 | 1.91 (1.25, 2.92) | 4.60% | 1.03 | 1.28 |
| KIR3DL1 | 1 | 0.002292839 | 1.39 (1.12, 1.72) | 4.10% | 1.03 | 1.27 |
| LILRA2 | -1 | 0.003038167 | 0.62 (0.46, 0.85) | 4.70% | 1.03 | 1.3 |
| LILRA2 | -1 | 0.003038167 | 0.62 (0.46, 0.85) | 4.70% | 1.03 | 1.3 |
| LMO2 | -1 | 0.002944661 | 0.65 (0.48, 0.86) | 4.70% | 1.03 | 1.3 |
| LMO2 | -1 | 0.002944661 | 0.65 (0.48, 0.86) | 4.70% | 1.03 | 1.3 |
| LMO2 | -1 | 0.002944661 | 0.65 (0.48, 0.86) | 4.70% | 1.03 | 1.3 |
| LOC100132707 | -1 | 0.002472978 | 0.62 (0.45, 0.84) | 4.20% | 1.03 | 1.31 |
| LOC100132707 | -1 | 0.002472978 | 0.62 (0.45, 0.84) | 4.20% | 1.03 | 1.31 |
| LOC100289187 | -1 | 0.002804418 | 0.66 (0.50, 0.87) | 4.60% | 1.03 | 1.3 |
| LOC100289187 | -1 | 0.002804418 | 0.66 (0.50, 0.87) | 4.60% | 1.03 | 1.3 |
| LOC100289187 | -1 | 0.002804418 | 0.66 (0.50, 0.87) | 4.60% | 1.03 | 1.3 |
| LOC146880 | 1 | 0.002469876 | 1.45 (1.14, 1.85) | 4.20% | 1.03 | 1.28 |
| LOC146880 | 1 | 0.002469876 | 1.45 (1.14, 1.85) | 4.20% | 1.03 | 1.28 |
| LOC283033 | -1 | 0.002659531 | 0.60 (0.43, 0.84) | 4.40% | 1.03 | 1.31 |
| LOC375190 | -1 | 0.002118296 | 0.64 (0.48, 0.85) | 3.90% | 1.03 | 1.31 |
| LOC401431 | -1 | 0.002536678 | 0.65 (0.50, 0.86) | 4.30% | 1.03 | 1.3 |
| LOC728606 | -1 | 0.003271325 | 0.60 (0.42, 0.84) | 4.90% | 1.03 | 1.3 |
| LOC730441 | 1 | 0.00284141 | 1.55 (1.16, 2.06) | 4.60% | 1.03 | 1.29 |
| LRP11 | 1 | 0.002676431 | 1.85 (1.24, 2.77) | 4.40% | 1.03 | 1.29 |
| LRRC49 | -1 | 0.004003969 | 0.52 (0.33, 0.81) | 5.50% | 1.03 | 1.29 |
| LRRC49 | -1 | 0.004003969 | 0.52 (0.33, 0.81) | 5.50% | 1.03 | 1.29 |
| LRRC49 | -1 | 0.004003969 | 0.52 (0.33, 0.81) | 5.50% | 1.03 | 1.29 |
| LRRC50 | -1 | 0.003723262 | 0.57 (0.39, 0.83) | 5.30% | 1.03 | 1.3 |
| LRRC69 | 1 | 0.00326634 | 1.64 (1.18, 2.28) | 4.90% | 1.03 | 1.29 |
| LYPD1 | -1 | 0.003495354 | 0.57 (0.39, 0.83) | 5.10% | 1.03 | 1.3 |
| LYPD1 | -1 | 0.003495354 | 0.57 (0.39, 0.83) | 5.10% | 1.03 | 1.3 |
| MDGA2 | 1 | 0.002999416 | 1.51 (1.15, 1.98) | 4.70% | 1.03 | 1.28 |
| MDGA2 | 1 | 0.002999416 | 1.51 (1.15, 1.98) | 4.70% | 1.03 | 1.28 |
| MED13 | 1 | 0.001791043 | 1.40 (1.13, 1.73) | 3.50% | 1.03 | 1.27 |
| MED23 | 1 | 0.002771728 | 1.43 (1.13, 1.80) | 4.50% | 1.03 | 1.27 |
| MED23 | 1 | 0.002771728 | 1.43 (1.13, 1.80) | 4.50% | 1.03 | 1.27 |
| MRPL33 | -1 | 0.002810203 | 0.56 (0.39, 0.82) | 4.60% | 1.03 | 1.31 |
| MRPL33 | -1 | 0.002810203 | 0.56 (0.39, 0.82) | 4.60% | 1.03 | 1.31 |
| MRPS25 | 1 | 0.00232796 | 1.57 (1.18, 2.11) | 4.10% | 1.03 | 1.29 |
| NAALADL1 | -1 | 0.003229161 | 0.59 (0.42, 0.84) | 4.90% | 1.03 | 1.3 |
| NCOA2 | 1 | 0.0028789 | 1.57 (1.17, 2.11) | 4.60% | 1.03 | 1.29 |
| NDC80 | 1 | 0.004332624 | 2.09 (1.26, 3.47) | 5.70% | 1.03 | 1.25 |
| NEGR1 | -1 | 0.003356882 | 0.60 (0.43, 0.84) | 5.00% | 1.03 | 1.3 |
| NEK2 | 1 | 0.004390513 | 2.20 (1.28, 3.77) | 5.80% | 1.03 | 1.24 |
| NEURL4 | -1 | 0.002512267 | 0.61 (0.45, 0.84) | 4.30% | 1.03 | 1.31 |
| NEURL4 | -1 | 0.002512267 | 0.61 (0.45, 0.84) | 4.30% | 1.03 | 1.31 |
| NIPBL | 1 | 0.003678237 | 1.81 (1.21, 2.69) | 5.30% | 1.03 | 1.28 |
| NIPBL | 1 | 0.003678237 | 1.81 (1.21, 2.69) | 5.30% | 1.03 | 1.28 |
| NKX2-3 | 1 | 0.00288716 | 1.51 (1.15, 1.98) | 4.60% | 1.03 | 1.28 |
| NRIP3 | -1 | 0.003165915 | 0.55 (0.37, 0.82) | 4.90% | 1.03 | 1.3 |
| NSMCE1 | -1 | 0.00397664 | 0.52 (0.33, 0.81) | 5.50% | 1.03 | 1.29 |
| OGDHL | 1 | 0.00273305 | 1.84 (1.24, 2.75) | 4.50% | 1.03 | 1.29 |
| OGDHL | 1 | 0.00273305 | 1.84 (1.24, 2.75) | 4.50% | 1.03 | 1.29 |
| OGDHL | 1 | 0.00273305 | 1.84 (1.24, 2.75) | 4.50% | 1.03 | 1.29 |
| OMA1 | -1 | 0.003329284 | 0.61 (0.44, 0.85) | 5.00% | 1.03 | 1.3 |
| ORC6 | 1 | 0.002725216 | 1.84 (1.24, 2.75) | 4.50% | 1.03 | 1.29 |
| OSCP1 | -1 | 0.002803854 | 0.65 (0.48, 0.86) | 4.60% | 1.03 | 1.3 |
| OSCP1 | -1 | 0.002803854 | 0.65 (0.48, 0.86) | 4.60% | 1.03 | 1.3 |
| P2RX4 | -1 | 0.003773817 | 0.54 (0.35, 0.82) | 5.40% | 1.03 | 1.29 |
| P4HA1 | 1 | 0.002479967 | 1.46 (1.14, 1.86) | 4.20% | 1.03 | 1.28 |
| P4HA1 | 1 | 0.002479967 | 1.46 (1.14, 1.86) | 4.20% | 1.03 | 1.28 |
| P4HA1 | 1 | 0.002479967 | 1.46 (1.14, 1.86) | 4.20% | 1.03 | 1.28 |
| P4HA1 | 1 | 0.002479967 | 1.46 (1.14, 1.86) | 4.20% | 1.03 | 1.28 |
| PCGF3 | -1 | 0.002417253 | 0.66 (0.50, 0.86) | 4.20% | 1.03 | 1.3 |
| PDE8A | 1 | 0.002954651 | 1.58 (1.17, 2.13) | 4.70% | 1.03 | 1.29 |
| PDE8A | 1 | 0.002954651 | 1.58 (1.17, 2.13) | 4.70% | 1.03 | 1.29 |
| PDIA5 | 1 | 0.003793083 | 1.64 (1.17, 2.28) | 5.40% | 1.03 | 1.28 |
| PDIA5 | 1 | 0.003793083 | 1.64 (1.17, 2.28) | 5.40% | 1.03 | 1.28 |
| PDSS2 | 1 | 0.002270205 | 1.30 (1.10, 1.53) | 4.10% | 1.03 | 1.23 |
| PHACTR2 | 1 | 0.003548548 | 1.77 (1.20, 2.59) | 5.20% | 1.03 | 1.28 |
| PHACTR2 | 1 | 0.003548548 | 1.77 (1.20, 2.59) | 5.20% | 1.03 | 1.28 |
| PHACTR2 | 1 | 0.003548548 | 1.77 (1.20, 2.59) | 5.20% | 1.03 | 1.28 |
| PHACTR2 | 1 | 0.003548548 | 1.77 (1.20, 2.59) | 5.20% | 1.03 | 1.28 |
| PHYHD1 | -1 | 0.002869757 | 0.57 (0.39, 0.82) | 4.60% | 1.03 | 1.31 |
| PHYHD1 | -1 | 0.002869757 | 0.57 (0.39, 0.82) | 4.60% | 1.03 | 1.31 |
| PHYHD1 | -1 | 0.002869757 | 0.57 (0.39, 0.82) | 4.60% | 1.03 | 1.31 |
| PNKP | -1 | 0.002981421 | 0.55 (0.37, 0.81) | 4.70% | 1.03 | 1.31 |
| PNMA2 | -1 | 0.00372139 | 0.61 (0.44, 0.85) | 5.30% | 1.03 | 1.3 |
| PODXL2 | 1 | 0.003649098 | 1.84 (1.22, 2.77) | 5.30% | 1.03 | 1.28 |
| POLQ | 1 | 0.002981188 | 1.84 (1.23, 2.76) | 4.70% | 1.03 | 1.28 |
| POLR2D | -1 | 0.002769807 | 0.69 (0.55, 0.88) | 4.50% | 1.03 | 1.29 |
| PRPF31 | -1 | 0.003398135 | 0.50 (0.31, 0.79) | 5.00% | 1.03 | 1.29 |
| PRSS23 | -1 | 0.003988776 | 0.54 (0.35, 0.82) | 5.50% | 1.03 | 1.29 |
| PSMA1 | 1 | 0.003851406 | 1.70 (1.19, 2.43) | 5.40% | 1.03 | 1.28 |
| PSMA1 | 1 | 0.003851406 | 1.70 (1.19, 2.43) | 5.40% | 1.03 | 1.28 |
| PSMA1 | 1 | 0.003851406 | 1.70 (1.19, 2.43) | 5.40% | 1.03 | 1.28 |
| PTCD3 | 1 | 0.002483485 | 1.72 (1.21, 2.43) | 4.20% | 1.03 | 1.3 |
| PTPRK | 1 | 0.003767173 | 1.70 (1.19, 2.44) | 5.40% | 1.03 | 1.28 |
| PTPRK | 1 | 0.003767173 | 1.70 (1.19, 2.44) | 5.40% | 1.03 | 1.28 |
| RAB11B | -1 | 0.003087506 | 0.59 (0.41, 0.83) | 4.80% | 1.03 | 1.31 |
| RAB14 | -1 | 0.002933672 | 0.60 (0.42, 0.84) | 4.70% | 1.03 | 1.31 |
| RAB3GAP2 | 1 | 0.002212459 | 1.62 (1.19, 2.20) | 4.00% | 1.03 | 1.3 |
| RAD51 | 1 | 0.003473055 | 1.82 (1.22, 2.71) | 5.10% | 1.03 | 1.28 |
| RAD51 | 1 | 0.003473055 | 1.82 (1.22, 2.71) | 5.10% | 1.03 | 1.28 |
| RAD51 | 1 | 0.003473055 | 1.82 (1.22, 2.71) | 5.10% | 1.03 | 1.28 |
| RAD51 | 1 | 0.003473055 | 1.82 (1.22, 2.71) | 5.10% | 1.03 | 1.28 |
| RALGPS1 | -1 | 0.003164278 | 0.62 (0.45, 0.85) | 4.90% | 1.03 | 1.3 |
| RALGPS1 | -1 | 0.003164278 | 0.62 (0.45, 0.85) | 4.90% | 1.03 | 1.3 |
| RALGPS1 | -1 | 0.003164278 | 0.62 (0.45, 0.85) | 4.90% | 1.03 | 1.3 |
| RALGPS1 | -1 | 0.003164278 | 0.62 (0.45, 0.85) | 4.90% | 1.03 | 1.3 |
| RASAL1 | 1 | 0.002891275 | 1.81 (1.22, 2.67) | 4.60% | 1.03 | 1.29 |
| RASAL1 | 1 | 0.002891275 | 1.81 (1.22, 2.67) | 4.60% | 1.03 | 1.29 |
| RASAL1 | 1 | 0.002891275 | 1.81 (1.22, 2.67) | 4.60% | 1.03 | 1.29 |
| RBKS | -1 | 0.002599049 | 0.58 (0.41, 0.83) | 4.40% | 1.03 | 1.31 |
| RETNLB | 1 | 0.00184757 | 1.34 (1.11, 1.61) | 3.50% | 1.03 | 1.25 |
| RG9MTD3 | -1 | 0.003210008 | 0.60 (0.43, 0.84) | 4.90% | 1.03 | 1.3 |
| RHCG | 1 | 0.003916067 | 1.97 (1.24, 3.13) | 5.40% | 1.03 | 1.26 |
| RIPK3 | -1 | 0.003278775 | 0.65 (0.48, 0.86) | 4.90% | 1.03 | 1.3 |
| RNF168 | 1 | 0.002917142 | 1.56 (1.16, 2.09) | 4.60% | 1.03 | 1.29 |
| RNF19A | 1 | 0.00310618 | 1.56 (1.16, 2.08) | 4.80% | 1.03 | 1.28 |
| RNF19A | 1 | 0.00310618 | 1.56 (1.16, 2.08) | 4.80% | 1.03 | 1.28 |
| ROBO1 | 1 | 0.002242507 | 1.48 (1.15, 1.90) | 4.00% | 1.03 | 1.29 |
| R0BO1 | 1 | 0.002242507 | 1.48 (1.15, 1.90) | 4.00% | 1.03 | 1.29 |
| ROBO1 | 1 | 0.002242507 | 1.48 (1.15, 1.90) | 4.00% | 1.03 | 1.29 |
| RPGR | -1 | 0.003675682 | 0.56 (0.38, 0.83) | 5.30% | 1.03 | 1.3 |
| RPGR | -1 | 0.003675682 | 0.56 (0.38, 0.83) | 5.30% | 1.03 | 1.3 |
| RPGRIP1 | -1 | 0.002745549 | 0.58 (040, 0.83) | 4.50% | 1.03 | 1.31 |
| SETBP1 | -1 | 0.003662366 | 0.54 (0.35, 0.82) | 5.30% | 1.03 | 1.3 |
| SETBP1 | -1 | 0.003662366 | 0.54 (0.35, 0.82) | 5.30% | 1.03 | 1.3 |
| SGOL1 | 1 | 0.003377474 | 1.92 (1.24, 2.96) | 5.00% | 1.03 | 1.27 |
| SGOL1 | 1 | 0.003377474 | 1.92 (1.24, 2.96) | 5.00% | 1.03 | 1.27 |
| SGOL1 | 1 | 0.003377474 | 1.92 (1.24, 2.96) | 5.00% | 1.03 | 1.27 |
| SGOL1 | 1 | 0.003377474 | 1.92 (1.24, 2.96) | 5.00% | 1.03 | 1.27 |
| SGOL1 | 1 | 0.003377474 | 1.92 (1.24, 2.96) | 5.00% | 1.03 | 1.27 |
| SGOL1 | 1 | 0.003377474 | 1.92 (1.24, 2.96) | 5.00% | 1.03 | 1.27 |
| SGOL1 | 1 | 0.003377474 | 1.92 (1.24, 2.96) | 5.00% | 1.03 | 1.27 |
| SGOL1 | 1 | 0.003377474 | 1.92 (1.24, 2.96) | 5.00% | 1.03 | 1.27 |
| SGOL1 | 1 | 0.003377474 | 1.92 (1.24, 2.96) | 5.00% | 1.03 | 1.27 |
| SGOL1 | 1 | 0.003377474 | 1.92 (1.24, 2.96) | 5.00% | 1.03 | 1.27 |
| SGOL1 | 1 | 0.003377474 | 1.92 (1.24, 2.96) | 5.00% | 1.03 | 1.27 |
| SGOL1 | 1 | 0.003377474 | 1.92 (1.24, 2.96) | 5.00% | 1.03 | 1.27 |
| SGOL1 | 1 | 0.003377474 | 1.92 (1.24, 2.96) | 5.00% | 1.03 | 1.27 |
| SIX5 | -1 | 0.002411377 | 0.60 (0.43, 0.84) | 4.20% | 1.03 | 1.31 |
| SLC25A32 | 1 | 0.002217418 | 1.60 (1.18, 2.16) | 4.00% | 1.03 | 1.3 |
| SLC26A6 | 1 | 0.002285648 | 1.55 (1.17, 2.04) | 4.10% | 1.03 | 1.29 |
| SLC26A6 | 1 | 0.002285648 | 1.55 (1.17, 2.04) | 4.10% | 1.03 | 1.29 |
| SLC26A6 | 1 | 0.002285648 | 1.55 (1.17, 2.04) | 4.10% | 1.03 | 1.29 |
| SLC26A6 | 1 | 0.002285648 | 1.55 (1.17, 2.04) | 4.10% | 1.03 | 1.29 |
| SLC34A1 | 1 | 0.002879478 | 1.64 (1.18, 2.27) | 4.60% | 1.03 | 1.29 |
| SLC34A1 | 1 | 0.002879478 | 1.64 (1.18, 2.27) | 4.60% | 1.03 | 1.29 |
| SLC3A2 | 1 | 0.003758233 | 1.76 (1.20, 2.58) | 5.40% | 1.03 | 1.28 |
| SLC3A2 | 1 | 0.003758233 | 1.76 (1.20, 2.58) | 5.40% | 1.03 | 1.28 |
| SLC3A2 | 1 | 0.003758233 | 1.76 (1.20, 2.58) | 5.40% | 1.03 | 1.28 |
| SLC3A2 | 1 | 0.003758233 | 1.76 (1.20, 2.58) | 5.40% | 1.03 | 1.28 |
| SLC3A2 | 1 | 0.003758233 | 1.76 (1.20, 2.58) | 5.40% | 1.03 | 1.28 |
| SLC3A2 | 1 | 0.003758233 | 1.76 (1.20, 2.58) | 5.40% | 1.03 | 1.28 |
| SLC3A2 | 1 | 0.003758233 | 1.76 (1.20, 2.58) | 5.40% | 1.03 | 1.28 1.29 |
| SNHG9 | 1 | 0.002692923 | 1.60 (1.18, 2.18) | 4.50% | 1.03 | 1.31 |
| SORBS3 | -1 | 0.002232728 | 0.62 (0.46, 0.84) | 4.00% | 1.03 | |
| SORBS3 | -1 | 0.002232728 | 0.62 (0.46, 0.84) | 4.00% | 1.03 | 1.31 1.29 |
| SOST | 1 | 0.002828316 | 1.57 (1.17, 2.10) | 4.60% | 1.03 | 1.3 |
| SREK1IP1 | -1 | 0.00381954 | 0.56 (0.38, 0.83) | 5.40% | 1.03 | 1.29 |
| SRP72 | 1 | 0.001984343 | 1.47 (1.15, 1.87) | 3.70% | 1.03 | 1.28 |
| STRADA | 1 | 0.001851162 | 1.44 (1.15, 1.82) | 3.50% | 1.03 | 1.28 |
| STRADA | 1 | 0.001851162 | 1.44 (1.15, 1.82) | 3.50% | 1.03 | 1.28 |
| STRADA | 1 | 0.001851162 | 1.44 (1.15, 1.82) | 3.50% | 1.03 | 1.28 |
| STRADA | 1 | 0.001851162 | 1.44 (1.15, 1.82) | 3.50% | 1.03 | 1.28 |
| STRADA | 1 | 0.001851162 | 1.44 (1.15, 1.82) | 3.50% | 1.03 | 1.28 |
| STRADA | 1 | 0.001851162 | 1.44 (1.15, 1.82) | 3.50% | 1.03 | 1.3 |
| SVIP | -1 | 0.00233855 | 0.67 (0.52, 0.87) | 4.10% | 1.03 | 1.27 |
| TAF9B | 1 | 0.003841595 | 1.82 (1.21, 2.74) | 5.40% | 1.03 | 1.29 |
| TALDO1 | 1 | 0.002598718 | 1.82 (1.23, 2.68) | 4.40% | 1.03 | 1.29 |
| TARS | 1 | 0.003276379 | 1.71 (1.20, 2.44) | 4.90% | 1.03 | 1.31 |
| TBC1D19 | -1 | 0.003039839 | 0.59 (0.41, 0.83) | 4.70% | 1.03 | 1.3 |
| TEKT3 | -1 | 0.003310535 | 0.57 (0.39, 0.83) | 4.90% | 1.03 | 1.29 |
| TIGD7 | -1 | 0.003861327 | 0.54 (0.35, 0.82) | 5.40% | 1.03 | 1.29 |
| TJP1 | 1 | 0.003251585 | 1.62 (1.17, 2.23) | 4.90% | 1.03 | 1.29 |
| TJP1 | 1 | 0.003251585 | 1.62 (1.17, 2.23) | 4.90% | 1.03 | 1.29 |
| TLE1 | 1 | 0.00347973 | 1.85 (1.22, 2.80) | 5.10% | 1.03 | 1.28 |
| TMEM221 | -1 | 0.003086562 | 0.60 (0.43, 0.84) | 4.80% | 1.03 | 1.31 |
| TNFAIP8L3 | 1 | 0.003178113 | 1.67 (1.19, 2.35) | 4.90% | 1.03 | 1.29 |
| TOMM7 | 1 | 0.002338876 | 1.53 (1.16, 2.02) | 4.10% | 1.03 | 1.29 |
| TPPP2 | 1 | 0.003263204 | 1.53 (1.15, 2.03) | 4.90% | 1.03 | 1.28 |
| TPX2 | 1 | 0.003467271 | 2.06 (1.27, 3.35) | 5.10% | 1.03 | 1.26 |
| TRIM8 | -1 | 0.002552093 | 0.58 (0.40, 0.82) | 4.30% | 1.03 | 1.31 |
| TTK | 1 | 0.003825485 | 1.94 (1.24, 3.05) | 5.40% | 1.03 | 1.27 |
| TTK | 1 | 0.003825485 | 1.94 (1.24, 3.05) | 5.40% | 1.03 | 1.27 |
| UBE2E2 | 1 | 0.003261146 | 1.77 (1.21, 2.59) | 4.90% | 1.03 | 1.28 |
| UQCRB | 1 | 0.003918973 | 2.01 (1.25, 3.24) | 5.40% | 1.03 | 1.26 |
| UQCRB | 1 | 0.003918973 | 2.01 (1.25, 3.24) | 5.40% | 1.03 | 1.26 |
| USH1G | 1 | 0.002435828 | 1.52 (1.16, 1.99) | 4.20% | 1.03 | 1.29 |
| VAMP1 | -1 | 0.003323775 | 0.55 (0.36, 0.82) | 5.00% | 1.03 | 1.3 |
| VAMP1 | -1 | 0.003323775 | 0.55 (0.36, 0.82) | 5.00% | 1.03 | 1.3 |
| VAMP1 | -1 | 0.003323775 | 0.55 (0.36, 0.82) | 5.00% | 1.03 | 1.3 |
| VNN3 | 1 | 0.002945726 | 1.57 (1.17, 2.11) | 4.70% | 1.03 | 1.29 |
| VNN3 | 1 | 0.002945726 | 1.57 (1.17, 2.11) | 4.70% | 1.03 | 1.29 |
| WBSCR17 | 1 | 0.002745076 | 1.51 (1.15, 1.97) | 4.50% | 1.03 | 1.28 |
| ZBTB34 | -1 | 0.002143612 | 0.63 (0.47, 0.85) | 3.90% | 1.03 | 1.31 |
| ZMYND10 | -1 | 0.003201542 | 0.62 (0.45, 0.85) | 4.90% | 1.03 | 1.3 |
| ZNF132 | -1 | 0.002418386 | 0.70 (0.56, 0.88) | 4.20% | 1.03 | 1.28 |
| ZNF137P | -1 | 0.002738324 | 0.66 (0.51, 0.87) | 4.50% | 1.03 | 1.3 |
| ZNF142 | -1 | 0.002752334 | 0.52 (0.34, 0.80) | 4.50% | 1.03 | 1.31 |
| ZNF143 | 1 | 0.003902798 | 1.78 (1.20, 2.62) | 5.40% | 1.03 | 1.28 |
| ZNF284 | -1 | 0.001742621 | 0.72 (0.58, 0.88) | 3.40% | 1.03 | 1.28 |
| ZNF343 | -1 | 0.002492691 | 0.67 (0.52, 0.87) | 4.30% | 1.03 | 1.3 |
| ZNF510 | -1 | 0.003447286 | 0.63 (0.46, 0.86) | 5.10% | 1.03 | 1.3 |
| ZNF85 | -1 | 0.00269138 | 0.59 (0.42, 0.83) | 4.50% | 1.03 | 1.31 |
| ZNF85 | -1 | 0.00269138 | 0.59 (0.42,0.83) | 4.50% | 1.03 | 1.31 |
| ACER2 | -1 | 0.005258002 | 0.59 (0.41, 0.86) | 6.30% | 1.02 | 1.29 |
| AFF3 | -1 | 0.006224386 | 0.61 (0.43, 0.87) | 6.90% | 1.02 | 1.28 |
| AFF3 | -1 | 0.006224386 | 0.61 (0.43, 0.87) | 6.90% | 1.02 | 1.28 |
| AGBL4 | -1 | 0.004756402 | 0.59 (0.41, 0.85) | 6.10% | 1.02 | 1.29 |
| AGTR2 | 1 | 0.004593311 | 1.31 (1.09, 1.58) | 5.90% | 1.02 | 1.23 |
| ALDH3A1 | 1 | 0.005741471 | 1.47 (1.12, 1.93) | 6.60% | 1.02 | 1.26 |
| ALDH3A1 | 1 | 0.005741471 | 1.47 (1.12, 1.93) | 6.60% | 1.02 | 1.26 |
| ALDH3A1 | 1 | 0.005741471 | 1.47 (1.12, 1.93) | 6.60% | 1.02 | 1.26 |
| ALG1L | 1 | 0.005751645 | 1.64 (1.15, 2.32) | 6.60% | 1.02 | 1.27 |
| ALG1L | 1 | 0.005751645 | 1.64 (1.15, 2.32) | 6.60% | 1.02 | 1.27 |
| ALKBH5 | -1 | 0.005864328 | 0.54 (0.35, 0.84) | 6.70% | 1.02 | 1.28 |
| ANKK1 | -1 | 0.004982445 | 0.56 (0.37, 0.84) | 6.20% | 1.02 | 1.29 |
| ANKRD11 | 1 | 0.00420042 | 1.58 (1.16, 2.16) | 5.60% | 1.02 | 1.28 |
| ANKZF1 | -1 | 0.003835427 | 0.62 (0.45, 0.86) | 5.40% | 1.02 | 1.3 |
| ANKZF1 | -1 | 0.003835427 | 0.62 (0.45, 0.86) | 5.40% | 1.02 | 1.3 |
| ANO10 | 1 | 0.005187033 | 1.81 (1.19, 2.74) | 6.30% | 1.02 | 1.26 |
| ATP6VOC | -1 | 0.004151532 | 0.59 (0.42, 0.85) | 5.60% | 1.02 | 1.3 |
| ATP6VOC | -1 | 0.004151532 | 0.59 (0.42, 0.85) | 5.60% | 1.02 | 1.3 |
| B3GALNT2 | 1 | 0.004947216 | 1.81 (1.20, 2.75) | 6.20% | 1.02 | 1.26 |
| B9D2 | -1 | 0.004413471 | 0.64 (0.47, 0.87) | 5.80% | 1.02 | 1.29 |
| BAGE | 1 | 0.003883658 | 1.52 (1.14, 2.03) | 5.40% | 1.02 | 1.28 |
| BBS12 | -1 | 0.004913742 | 0.61 (0.43, 0.86) | 6.20% | 1.02 | 1.29 |
| BBS12 | -1 | 0.004913742 | 0.61 (0.43, 0.86) | 6.20% | 1.02 | 1.29 |
| BUB1 | 1 | 0.004514942 | 1.93 (1.23, 3.03) | 5.90% | 1.02 | 1.26 |
| C12orf26 | -1 | 0.006126167 | 0.54 (0.35, 0.84) | 6.90% | 1.02 | 1.28 |
| C12orf47 | -1 | 0.005171331 | 0.67 (0.50, 0.89) | 6.30% | 1.02 | 1.28 |
| C12orf48 | 1 | 0.004544654 | 1.76 (1.19, 2.60) | 5.90% | 1.02 | 1.27 |
| C12orf5 | -1 | 0.004934346 | 0.61 (0.44, 0.86) | 6.20% | 1.02 | 1.29 |
| C12orfS7 | -1 | 0.00493224 | 0.70 (0.54, 0.90) | 6.20% | 1.02 | 1.27 |
| C16orf58 | -1 | 0.005936891 | 0.61 (0.43, 0.87) | 6.80% | 1.02 | 1.28 |
| C18orf62 | 1 | 0.004064416 | 1.63 (1.17, 2.28) | 5.60% | 1.02 | 1.28 |
| C19orf20 | -1 | 0.005286341 | 0.65 (0.48, 0.88) | 6.30% | 1.02 | 1.28 |
| C5orf30 | -1 | 0.005136301 | 0.60 (0.42, 0.86) | 6.20% | 1.02 | 1.29 |
| C6orf58 | 1 | 0.004731205 | 1.47 (1.13, 1.92) | 6.00% | 1.02 | 1.27 |
| C9orf80 | -1 | 0.004122841 | 0.61 (0.43, 0.85) | 5.60% | 1.02 | 1.3 |
| C9orf9 | -1 | 0.00449506 | 0.68 (0.52, 0.89) | 5.90% | 1.02 | 1.28 |
| CASC5 | 1 | 0.005050283 | 2.06 (1.24, 3.41) | 6.20% | 1.02 | 1.25 |
| CASC5 | 1 | 0.005050283 | 2.06 (1.24, 3.41) | 6.20% | 1.02 | 1.25 |
| CBLN4 | -1 | 0.005236558 | 0.55 (0.37, 0.84) | 6.30% | 1.02 | 1.28 |
| CDKN3 | 1 | 0.00470614 | 1.56 (1.15, 2.11) | 6.00% | 1.02 | 1.27 |
| CDKN3 | 1 | 0.00470614 | 1.56 (1.15, 2.11) | 6.00% | 1.02 | 1.27 |
| CENPE | 1 | 0.005043144 | 1.82 (1.20, 2.76) | 6.20% | 1.02 | 1.26 |
| CHP | 1 | 0.00474853 | 1.71 (1.18, 2.47) | 6.10% | 1.02 | 1.27 |
| CHRM3 | 1 | 0.004463794 | 1.55 (1.14, 2.09) | 5.80% | 1.02 | 1.27 |
| CLDN6 | 1 | 0.003130215 | 1.42 (1.13, 1.80) | 4.80% | 1.02 | 1.27 |
| CLEC10A | -1 | 0.005671891 | 0.56 (0.37, 0.85) | 6.60% | 1.02 | 1.28 |
| CLEC10A | -1 | 0.005671891 | 0.56 (0.37, 0.85) | 6.60% | 1.02 | 1.28 |
| COL4A5 | -1 | 0.005952194 | 0.63 (0.45, 0.88) | 6.80% | 1.02 | 1.28 |
| COL4A5 | -1 | 0.005952194 | 0.63 (0.45, 0.88) | 6.80% | 1.02 | 1.28 |
| COMP | -1 | 0.005307774 | 0.65 (0.48, 0.88) | 6.30% | 1.02 | 1.28 |
| CPA3 | -1 | 0.004401849 | 0.64 (0.47, 0.87) | 5.80% | 1.02 | 1.29 |
| CPLX3 | 1 | 0.003893832 | 1.47 (1.13, 1.90) | 5.40% | 1.02 | 1.27 |
| CT62 | -1 | 0.004298148 | 0.60 (0.43, 0.85) | 5.70% | 1.02 | 1.29 |
| CTDSP1 | -1 | 0.004831508 | 0.65 (0.48, 0.88) | 6.10% | 1.02 | 1.29 |
| CTDSP1 | -1 | 0.004831508 | 0.65 (0.48, 0.88) | 6.10% | 1.02 | 1.29 |
| CTXN2 | 1 | 0.004461247 | 1.53 (1.14, 2.06) | 5.80% | 1.02 | 1.27 |
| CYB5R1 | -1 | 0.005236004 | 0.67 (0.50, 0.89) | 6.30% | 1.02 | 1.28 |
| CYBRD1 | -1 | 0.00448011 | 0.61 (0.44, 0.86) | 5.80% | 1.02 | 1.29 |
| CYBRD1 | -1 | 0.00448011 | 0.61 (0.44, 0.86) | 5.80% | 1.02 | 1.29 |
| CYP4B1 | -1 | 0.006117908 | 0.62 (0.44, 0.87) | 6.90% | 1.02 | 1.28 |
| CYP4B1 | -1 | 0.006117908 | 0.62 (0.44, 0.87) | 6.90% | 1.02 | 1.28 |
| DAR52 | -1 | 0.004193228 | 0.61 (0.43, 0.86) | 5.60% | 1.02 | 1.3 |
| DCTD | -1 | 0.004336513 | 0.65 (0.48, 0.87) | 5.70% | 1.02 | 1.29 |
| DCTD | -1 | 0.004336513 | 0.65 (0.48, 0.87) | 5.70% | 1.02 | 1.29 |
| DDIT4 | 1 | 0.006657073 | 2.06 (1.22, 3.46) | 7.10% | 1.02 | 1.23 |
| DDX12 | 1 | 0.005232168 | 1.71 (1.17, 2.50) | 6.30% | 1.02 | 1.27 |
| DDX19A | 1 | 0.005506264 | 1.72 (1.17, 2.51) | 6.40% | 1.02 | 1.27 |
| DEPDC1B | 1 | 0.005895254 | 1.80 (1.18, 2.73) | 6.70% | 1.02 | 1.26 |
| DEPDC1B | 1 | 0.005895254 | 1.80 (1.18, 2.73) | 6.70% | 1.02 | 1.26 |
| DHCR7 | 1 | 0.004895545 | 1.61 (1.16, 2.25) | 6.10% | 1.02 | 1.27 |
| DHCR7 | 1 | 0.004895545 | 1.61 (1.16, 2.25) | 6.10% | 1.02 | 1.27 |
| DMWD | -1 | 0.004951827 | 0.70 (0.54, 0.90) | 6.20% | 1.02 | 1.27 |
| DNAJC16 | -1 | 0.005555333 | 0.61 (0.43, 0.87) | 6.50% | 1.02 | 1.29 |
| DNAJC5 | 1 | 0.004961512 | 1.58 (1.15, 2.17) | 6.20% | 1.02 | 1.27 |
| DNAL1 | -1 | 0.005480966 | 0.62 (0.45, 0.87) | 6.40% | 1.02 | 1.29 |
| DSG4 | 1 | 0.005276336 | 1.58 (1.15, 2.19) | 6.30% | 1.02 | 1.27 |
| DSG4 | 1 | 0.005276336 | 1.58 (1.15, 2.19) | 6.30% | 1.02 | 1.27 |
| DYNLL1 | -1 | 0.005877927 | 0.58 (0.40, 0.86) | 6.70% | 1.02 | 1.28 |
| DYNLL1 | -1 | 0.005877927 | 0.58 (0.40, 0.86) | 6.70% | 1.02 | 1.28 |
| DYNLL1 | -1 | 0.005877927 | 0.58 (0.40, 0.86) | 6.70% | 1.02 | 1.28 |
| E2F8 | 1 | 0.006071337 | 2.36 (1.28, 4.36) | 6.90% | 1.02 | 1.22 |
| EFNA2 | 1 | 0.004879866 | 1.55 (1.14, 2.11) | 6.10% | 1.02 | 1.27 |
| EGFEM1P | -1 | 0.006582566 | 0.53 (0.33, 0.84) | 7.10% | 1.02 | 1.27 |
| ELL3 | 1 | 0.005132124 | 2.00 (1.23, 3.26) | 6.20% | 1.02 | 1.25 |
| ERRFI1 | 1 | 0.004022383 | 1.57 (1.15, 2.13) | 5.50% | 1.02 | 1.28 |
| EXD2 | -1 | 0.004992909 | 0.62 (0.44, 0.87) | 6.20% | 1.02 | 1.29 |
| EXD2 | -1 | 0.004992909 | 0.62 (0.44, 0.87) | 6.20% | 1.02 | 1.29 |
| EXD2 | -1 | 0.004992909 | 0.62 (0.44, 0.87) | 6.20% | 1.02 | 1.29 |
| EXD2 | -1 | 0.004992909 | 0.62 (0.44, 0.87) | 6.20% | 1.02 | 1.29 |
| EXD2 | -1 | 0.004992909 | 0.62 (0.44, 0.87) | 6.20% | 1.02 | 1.29 |
| EXD2 | -1 | 0.004992909 | 0.62 (0.44, 0.87) | 6.20% | 1.02 | 1.29 |
| EXD2 | -1 | 0.004992909 | 0.62 (0.44, 0.87) | 6.20% | 1.02 | 1.29 |
| FADS2 | 1 | 0.00451137 | 1.85 (1.21, 2.82) | 5.90% | 1.02 | 1.27 |
| FAM19A1 | 1 | 0.004486786 | 1.72 (1.18, 2.51) | 5.80% | 1.02 | 1.27 |
| FAM24A | 1 | 0.003464128 | 1.48 (1.14, 1.93) | 5.10% | 1.02 | 1.28 |
| FAM75C1 | 1 | 0.003067161 | 1.29 (1.09, 1.52) | 4.80% | 1.02 | 1.22 |
| FAM83D | 1 | 0.005258982 | 2.10 (1.25, 3.55) | 6.30% | 1.02 | 1.24 |
| FANCA | 1 | 0.006453602 | 1.74 (1.17, 2.60) | 7.10% | 1.02 | 1.26 |
| FANCA | 1 | 0.006453602 | 1.74 (1.17, 2.60) | 7.10% | 1.02 | 1.26 |
| FARP2 | -1 | 0.00480468 | 0.61 (0.43, 0.86) | 6.10% | 1.02 | 1.29 |
| FCGBP | -1 | 0.005135347 | 0.61 (0.43, 0.86) | 6.20% | 1.02 | 1.29 |
| FGF12 | 1 | 0.00508741 | 1.49 (1.13, 1.97) | 6.20% | 1.02 | 1.27 |
| FGF12 | 1 | 0.00508741 | 1.49 (1.13, 1.97) | 6.20% | 1.02 | 1.27 |
| FU31306 | -1 | 0.005207343 | 0.56 (0.38, 0.84) | 6.30% | 1.02 | 1.29 |
| FLJ31306 | -1 | 0.005207343 | 0.56 (0.38, 0.84) | 6.30% | 1.02 | 1.29 |
| FLYWCH2 | -1 | 0.00602473 | 0.63 (0.45, 0.88) | 6.80% | 1.02 | 1.28 |
| FLYWCH2 | -1 | 0.00602473 | 0.63 (0.45, 0.88) | 6.80% | 1.02 | 1.28 |
| FLYWCH2 | -1 | 0.00602473 | 0.63 (0.45, 0.88) | 6.80% | 1.02 | 1.28 |
| FRMD8 | 1 | 0.005100953 | 1.53 (1.14, 2.07) | 6.20% | 1.02 | 1.27 |
| GDAP1L1 | -1 | 0.004064739 | 0.64 (0.48, 0.87) | 5.60% | 1.02 | 1.29 |
| GLIPR1L2 | -1 | 0.004844215 | 0.67 (0.51, 0.88) | 6.10% | 1.02 | 1.28 |
| GNAI1 | 1 | 0.004921212 | 1.52 (1.14, 2.03) | 6.20% | 1.02 | 1.27 |
| GNN | -1 | 0.00547683 | 0.55 (0.36, 0.84) | 6.40% | 1.02 | 1.28 |
| GOT1L1 | 1 | 0.005030656 | 1.50 (1.13, 2.00) | 6.20% | 1.02 | 1.27 |
| GPR56 | 1 | 0.005127944 | 1.67 (1.17, 2.39) | 6.20% | 1.02 | 1.27 |
| GPR56 | 1 | 0.005127944 | 1.67 (1.17, 2.39) | 6.20% | 1.02 | 1.27 |
| GPR56 | 1 | 0.005127944 | 1.67 (1.17, 2.39) | 6.20% | 1.02 | 1.27 |
| GPR56 | 1 | 0.005127944 | 1.67 (1.17, 2.39) | 6.20% | 1.02 | 1.27 |
| GPR56 | 1 | 0.005127944 | 1.67 (1.17, 2.39) | 6.20% | 1.02 | 1.27 |
| GPR56 | 1 | 0.005127944 | 1.67 (1.17, 2.39) | 6.20% | 1.02 | 1.27 |
| GPR56 | 1 | 0.005127944 | 1.67 (1.17, 2.39) | 6.20% | 1.02 | 1.27 |
| GPR56 | 1 | 0.005127944 | 1.67 (1.17, 2.39) | 6.20% | 1.02 | 1.27 |
| GTF2F1 | -1 | 0.004162542 | 0.65 (0.49, 0.87) | 5.60% | 1.02 | 1.29 |
| HDAC7 | -1 | 0.004641148 | 0.64 (0.46, 0.87) | 6.00% | 1.02 | 1.29 |
| HDAC7 | -1 | 0.004641148 | 0.64 (0.46, 0.87) | 6.00% | 1.02 | 1.29 |
| HERV-V2 | 1 | 0.003227481 | 1.41 (1.12, 1.76) | 4.90% | 1.02 | 1.26 |
| HHEX | -1 | 0.005145369 | 0.65 (0.48, 0.88) | 6.20% | 1.02 | 1.28 |
| HMMR | 1 | 0.005103266 | 1.91 (1.22, 3.02) | 6.20% | 1.02 | 1.26 |
| HMMR | 1 | 0.005103266 | 1.91 (1.22, 3.02) | 6.20% | 1.02 | 1.26 |
| HMMR | 1 | 0.005103266 | 1.91 (1.22, 3.02) | 6.20% | 1.02 | 1.26 |
| HMMR | 1 | 0.005103266 | 1.91 (1.22, 3.02) | 6.20% | 1.02 | 1.26 |
| HN1 | 1 | 0.005386748 | 1.69 (1.17, 2.46) | 6.40% | 1.02 | 1.27 |
| HN1 | 1 | 0.005386748 | 1.69 (1.17, 2.46) | 6.40% | 1.02 | 1.27 |
| HN1 | 1 | 0.005386748 | 1.69 (1.17, 2.46) | 6.40% | 1.02 | 1.27 |
| HRNR | -1 | 0.005968075 | 0.65 (0.48, 0.88) | 6.80% | 1.02 | 1.28 |
| HS3ST1 | 1 | 0.005320771 | 1.50 (1.13, 2.00) | 6.30% | 1.02 | 1.27 |
| HSPA9 | 1 | 0.005701331 | 1.64 (1.15, 2.32) | 6.60% | 1.02 | 1.27 |
| IBSP | 1 | 0.004575031 | 1.59 (1.15, 2.18) | 5.90% | 1.02 | 1.27 |
| ICA1L | -1 | 0.005336018 | 0.59 (0.41, 0.85) | 6.30% | 1.02 | 1.29 |
| ICA1L | -1 | 0.005336018 | 0.59 (0.41, 0.85) | 6.30% | 1.02 | 1.29 |
| IFRD1 | 1 | 0.005335466 | 1.56 (1.14, 2.14) | 6.30% | 1.02 | 1.27 |
| IFRD1 | 1 | 0.005335466 | 1.56 (1.14, 2.14) | 6.30% | 1.02 | 1.27 |
| IFRD1 | 1 | 0.005335466 | 1.56 (1.14, 2.14) | 6.30% | 1.02 | 1.27 |
| IFRD1 | 1 | 0.005335466 | 1.56 (1.14, 2.14) | 6.30% | 1.02 | 1.27 |
| IFRD2 | -1 | 0.004620541 | 0.68 (0.52, 0.89) | 6.00% | 1.02 | 1.28 |
| IL6ST | -1 | 0.004669167 | 0.56 (0.37, 0.84) | 6.00% | 1.02 | 1.29 |
| IL6ST | -1 | 0.004669167 | 0.56 (0.37, 0.84) | 6.00% | 1.02 | 1.29 |
| IL6ST | -1 | 0.004669167 | 0.56 (0.37, 0.84) | 6.00% | 1.02 | 1.29 |
| INCA1 | -1 | 0.003958143 | 0.61 (0.43, 0.85) | 5.50% | 1.02 | 1.3 |
| INCA1 | -1 | 0.003958143 | 0.61 (0.43, 0.85) | 5.50% | 1.02 | 1.3 |
| INCA1 | -1 | 0.003958143 | 0.61 (0.43, 0.85) | 5.50% | 1.02 | 1.3 |
| INCA1 | -1 | 0.003958143 | 0.61 (0.43, 0.85) | 5.50% | 1.02 | 1.3 |
| INO80 | 1 | 0.004710879 | 1.77 (1.19, 2.62) | 6.00% | 1.02 | 1.27 |
| ISOC2 | -1 | 0.006176895 | 0.61 (0.43, 0.87) | 6.90% | 1.02 | 1.28 |
| ISOC2 | -1 | 0.006176895 | 0.61 (0.43, 0.87) | 6.90% | 1.02 | 1.28 |
| ISOC2 | -1 | 0.006176895 | 0.61 (0.43, 0.87) | 6.90% | 1.02 | 1.28 |
| KCNA4 | 1 | 0.004433861 | 1.41 (1.11, 1.79) | 5.80% | 1.02 | 1.26 |
| KIAA1324 | -1 | 0.005588146 | 0.68 (0.52, 0.89) | 6.50% | 1.02 | 1.27 |
| KIF18A | 1 | 0.005735098 | 1.80 (1.19, 2.72) | 6.60% | 1.02 | 1.26 |
| KIF23 | 1 | 0.006540193 | 1.78 (1.17, 2.69) | 7.10% | 1.02 | 1.25 |
| KIF23 | 1 | 0.006540193 | 1.78 (1.17, 2.69) | 7.10% | 1.02 | 1.25 |
| KIF2C | 1 | 0.005175206 | 1.97 (1.22, 3.17) | 6.30% | 1.02 | 1.25 |
| KRCC1 | -1 | 0.004567679 | 0.63 (0.46, 0.87) | 5.90% | 1.02 | 1.29 |
| KRT16 | 1 | 0.004086786 | 1.43 (1.12, 1.83) | 5.60% | 1.02 | 1.26 |
| KRT84 | 1 | 0.004004984 | 1.49 (1.14, 1.96) | 5.50% | 1.02 | 1.27 |
| LACTB | -1 | 0.006101678 | 0.61 (0.43, 0.87) | 6.90% | 1.02 | 1.28 |
| LACTB | -1 | 0.006101678 | 0.61 (0.43, 0.87) | 6.90% | 1.02 | 1.28 |
| LAG3 | -1 | 0.005742967 | 0.62 (0.45, 0.87) | 6.60% | 1.02 | 1.28 |
| LIN9 | 1 | 0.004469851 | 1.73 (1.19, 2.53) | 5.80% | 1.02 | 1.27 |
| LOC100190938 | -1 | 0.005127469 | 0.64 (0.47, 0.88) | 6.20% | 1.02 | 1.29 |
| LOC100190938 | -1 | 0.005127469 | 0.64 (0.47, 0.88) | 6.20% | 1.02 | 1.29 |
| LOC154860 | 1 | 0.004106036 | 1.50 (1.14, 1.98) | 5.60% | 1.02 | 1.27 |
| LOC285735 | 1 | 0.003944073 | 1.43 (1.12, 1.82) | 5.50% | 1.02 | 1.26 |
| LOC400643 | 1 | 0.004781617 | 1.30 (1.08, 1.55) | 6.10% | 1.02 | 1.22 |
| LOC400657 | -1 | 0.004993934 | 0.67 (0.50, 0.88) | 6.20% | 1.02 | 1.28 |
| LY6D | 1 | 0.004888422 | 1.46 (1.12, 1.90) | 6.10% | 1.02 | 1.26 |
| LY6H | 1 | 0.004151007 | 1.39 (1.11, 1.75) | 5.60% | 1.02 | 1.25 |
| LY6H | 1 | 0.004151007 | 1.39 (1.11, 1.75) | 5.60% | 1.02 | 1.25 |
| LY6H | 1 | 0.004151007 | 1.39 (1.11, 1.75) | 5.60% | 1.02 | 1.25 |
| MAP2K2 | -1 | 0.005892515 | 0.63 (0.46, 0.88) | 6.70% | 1.02 | 1.28 |
| MAP3K4 | 1 | 0.004985634 | 1.43 (1.11, 1.84) | 6.20% | 1.02 | 1.26 |
| MAP3K4 | 1 | 0.004985634 | 1.43 (1.11, 1.84) | 6.20% | 1.02 | 1.26 |
| MAP7D2 | 1 | 0.005695576 | 1.53 (1.13, 2.06) | 6.60% | 1.02 | 1.26 |
| MAP7D2 | 1 | 0.005695576 | 1.53 (1.13, 2.06) | 6.60% | 1.02 | 1.26 |
| MAP7D2 | 1 | 0.005695576 | 1.53 (1.13, 2.06) | 6.60% | 1.02 | 1.26 |
| MAP7D2 | 1 | 0.005695576 | 1.53 (1.13, 2.06) | 6.60% | 1.02 | 1.26 |
| MCCC1 | 1 | 0.006217752 | 1.59 (1.14, 2.22) | 6.90% | 1.02 | 1.26 |
| ME3 | -1 | 0.005503433 | 0.61 (0.43, 0.86) | 6.40% | 1.02 | 1.29 |
| ME3 | -1 | 0.005503433 | 0.61 (0.43, 0.86) | 6.40% | 1.02 | 1.29 |
| ME3 | -1 | 0.005503433 | 0.61 (0.43, 0.86) | 6.40% | 1.02 | 1.29 |
| MELK | 1 | 0.006465586 | 1.77 (1.17, 2.66) | 7.10% | 1.02 | 1.26 |
| MMP15 | 1 | 0.005850262 | 1.85 (1.19, 2.86) | 6.70% | 1.02 | 1.25 |
| MOV10 | -1 | 0.005238498 | 0.58 (0.39, 0.85) | 6.30% | 1.02 | 1.29 |
| MOV10 | -1 | 0.005238498 | 0.58 (0.39, 0.85) | 6.30% | 1.02 | 1.29 |
| MRPL39 | -1 | 0.003894605 | 0.61 (0.44, 0.85) | 5.40% | 1.02 | 1.3 |
| MRPL39 | -1 | 0.003894605 | 0.61 (0.44, 0.85) | 5.40% | 1.02 | 1.3 |
| MSL3L2 | 1 | 0.005035426 | 1.60 (1.15, 2.23) | 6.20% | 1.02 | 1.27 |
| MTBP | 1 | 0.005853811 | 1.63 (1.15, 2.30) | 6.70% | 1.02 | 1.27 |
| MTFR1 | 1 | 0.005110758 | 1.55 (1.14, 2.11) | 6.20% | 1.02 | 1.27 |
| MTFR1 | 1 | 0.005110758 | 1.55 (1.14, 2.11) | 6.20% | 1.02 | 1.27 |
| MTFR1 | 1 | 0.005110758 | 1.55 (1.14, 2.11) | 6.20% | 1.02 | 1.27 |
| MTSS1L | 1 | 0.004990967 | 1.65 (1.16, 2.34) | 6.20% | 1.02 | 1.27 |
| MYOM3 | -1 | 0.00522092 | 0.63 (0.46, 0.87) | 6.30% | 1.02 | 1.29 |
| NCRNA00263 | 1 | 0.004190387 | 1.65 (1.17, 2.33) | 5.60% | 1.02 | 1.28 |
| NDUFB10 | -1 | 0.005128156 | 0.69 (0.54, 0.90) | 6.20% | 1.02 | 1.27 |
| NEIL3 | 1 | 0.006472151 | 1.95 (1.21, 3.15) | 7.10% | 1.02 | 1.24 |
| NKG7 | 1 | 0.003249298 | 1.34 (1.10, 1.62) | 4.90% | 1.02 | 1.24 |
| NMNAT3 | -1 | 0.004403783 | 0.56 (0.37, 0.83) | 5.80% | 1.02 | 1.29 |
| NOD1 | 1 | 0.003051661 | 1.40 (1.12, 1.75) | 4.70% | 1.02 | 1.26 |
| NPM1 | 1 | 0.004274784 | 1.78 (1.20, 2.66) | 5.70% | 1.02 | 1.27 |
| NPM1 | 1 | 0.004274784 | 1.78 (1.20, 2.66) | 5.70% | 1.02 | 1.27 |
| NPM1 | 1 | 0.004274784 | 1.78 (1.20, 2.66) | 5.70% | 1.02 | 1.27 |
| NPY5R | -1 | 0.005397335 | 0.56 (0.37, 0.84) | 6.40% | 1.02 | 1.28 |
| NRG2 | 1 | 0.005368867 | 1.65 (1.16, 2.34) | 6.40% | 1.02 | 1.27 |
| NRG2 | 1 | 0.005368867 | 1.65 (1.16, 2.34) | 6.40% | 1.02 | 1.27 |
| NRG2 | 1 | 0.005368867 | 1.65 (1.16, 2.34) | 6.40% | 1.02 | 1.27 |
| NRG2 | 1 | 0.005368867 | 1.65 (1.16, 2.34) | 6.40% | 1.02 | 1.27 |
| NRG2 | 1 | 0.005368867 | 1.65 (1.16, 2.34) | 6.40% | 1.02 | 1.27 |
| NUDT16P1 | -1 | 0.004513272 | 0.59 (0.41, 0.85) | 5.90% | 1.02 | 1.29 |
| NUDT16P1 | -1 | 0.004513272 | 0.59 (0.41, 0.85) | 5.90% | 1.02 | 1.29 |
| NUF2 | 1 | 0.004146016 | 1.68 (1.18, 2.41) | 5.60% | 1.02 | 1.28 |
| NUF2 | 1 | 0.004146016 | 1.68 (1.18, 2.41) | 5.60% | 1.02 | 1.28 |
| NUSAP1 | 1 | 0.004608304 | 1.85 (1.21, 2.83) | 6.00% | 1.02 | 1.27 |
| NUSAP1 | 1 | 0.004608304 | 1.85 (1.21, 2.83) | 6.00% | 1.02 | 1.27 |
| NUSAP1 | 1 | 0.004608304 | 1.85 (1.21, 2.83) | 6.00% | 1.02 | 1.27 |
| OIP5 | 1 | 0.00444449 | 1.74 (1.19, 2.54) | 5.80% | 1.02 | 1.27 |
| PARD3 | 1 | 0.00638968 | 1.81 (1.18, 2.79) | 7.10% | 1.02 | 1.25 |
| PARD3 | 1 | 0.00638968 | 1.81 (1.18, 2.79) | 7.10% | 1.02 | 1.25 |
| PARD3 | 1 | 0.00638968 | 1.81 (1.18, 2.79) | 7.10% | 1.02 | 1.25 |
| PARD3 | 1 | 0.00638968 | 1.81 (1.18, 2.79) | 7.10% | 1.02 | 1.25 |
| PARD3 | 1 | 0.00638968 | 1.81 (1.18, 2.79) | 7.10% | 1.02 | 1.25 |
| PARD3 | 1 | 0.00638968 | 1.81 (1.18, 2.79) | 7.10% | 1.02 | 1.25 |
| PARD3 | 1 | 0.00638968 | 1.81 (1.18, 2.79) | 7.10% | 1.02 | 1.25 |
| PARD3 | 1 | 0.00638968 | 1.81 (1.18, 2.79) | 7.10% | 1.02 | 1.25 |
| PARD3 | 1 | 0.00638968 | 1.81 (1.18, 2.79) | 7.10% | 1.02 | 1.25 |
| PARD3 | 1 | 0.00638968 | 1.81 (1.18, 2.79) | 7.10% | 1.02 | 1.25 |
| PARD3 | 1 | 0.00638968 | 1.81 (1.18, 2.79) | 7.10% | 1.02 | 1.25 |
| PCDH11X | 1 | 0.003459139 | 1.50 (1.14, 1.96) | 5.10% | 1.02 | 1.28 |
| PCDH11X | 1 | 0.003459139 | 1.50 (1.14, 1.96) | 5.10% | 1.02 | 1.28 |
| PCDH11X | 1 | 0.003459139 | 1.50 (1.14, 1.96) | 5.10% | 1.02 | 1.28 |
| PCDH11X | 1 | 0.003459139 | 1.50 (1.14, 1.96) | 5.10% | 1.02 | 1.28 |
| PCDH11X | 1 | 0.003459139 | 1.50 (1.14, 1.96) | 5.10% | 1.02 | 1.28 |
| PCDH11X | 1 | 0.003459139 | 1.50 (1.14, 1.96) | 5.10% | 1.02 | 1.28 |
| PCDH11X | 1 | 0.003459139 | 1.50 (1.14, 1.96) | 5.10% | 1.02 | 1.28 |
| PCDH11X | 1 | 0.003459139 | 1.50 (1.14, 1.96) | 5.10% | 1.02 | 1.28 |
| PDZD3 | -1 | 0.004829406 | 0.57 (0.39, 0.84) | 6.10% | 1.02 | 1.29 |
| PDZD3 | -1 | 0.004829406 | 0.57 (0.39, 0.84) | 6.10% | 1.02 | 1.29 |
| PDZD3 | -1 | 0.004829406 | 0.57 (0.39, 0.84) | 6.10% | 1.02 | 1.29 |
| PHIP | 1 | 0.004453412 | 1.59 (1.16, 2.20) | 5.80% | 1.02 | 1.27 |
| PHKB | 1 | 0.00471534 | 1.49 (1.13, 1.96) | 6.00% | 1.02 | 1.27 |
| PHKB | 1 | 0.00471534 | 1.49 (1.13, 1.96) | 6.00% | 1.02 | 1.27 |
| PHKG2 | -1 | 0.006596304 | 0.52 (0.33, 0.83) | 7.10% | 1.02 | 1.27 |
| PHKG2 | -1 | 0.006596304 | 0.52 (0.33, 0.83) | 7.10% | 1.02 | 1.27 |
| PIAS1 | 1 | 0.004108295 | 1.43 (1.12, 1.82) | 5.60% | 1.02 | 1.26 |
| PLA2R1 | -1 | 0.004141664 | 0.65 (0.48, 0.87) | 5.60% | 1.02 | 1.29 |
| PLA2R1 | -1 | 0.004141664 | 0.65 (0.48, 0.87) | 5.60% | 1.02 | 1.29 |
| PLA2R1 | -1 | 0.004141664 | 0.65 (0.48, 0.87) | 5.60% | 1.02 | 1.29 |
| PLCD4 | -1 | 0.005641861 | 0.60 (0.41,0.86) | 6.60% | 1.02 | 1.28 |
| POC1A | 1 | 0.005505401 | 1.66 (1.16, 2.37) | 6.40% | 1.02 | 1.27 |
| POC1A | 1 | 0.005505401 | 1.66 (1.16, 2.37) | 6.40% | 1.02 | 1.27 |
| POC1A | 1 | 0.005505401 | 1.66 (1.16, 2.37) | 6.40% | 1.02 | 1.27 |
| POMT2 | -1 | 0.005356899 | 0.61 (0.43, 0.86) | 6.40% | 1.02 | 1.29 |
| PPIL6 | -1 | 0.00393157 | 0.60 (0.43, 0.85) | 5.50% | 1.02 | 1.3 |
| PPIL6 | -1 | 0.00393157 | 0.60 (0.43, 0.85) | 5.50% | 1.02 | 1.3 |
| PPPDE1 | 1 | 0.004067992 | 1.62 (1.17, 2.26) | 5.60% | 1.02 | 1.28 |
| PRPF8 | -1 | 0.004322159 | 0.63 (0.46, 0.87) | 5.70% | 1.02 | 1.29 |
| PSMC6 | 1 | 0.004415222 | 1.51 (1.14, 2.00) | 5.80% | 1.02 | 1.27 |
| PTDSS1 | 1 | 0.005883984 | 1.64 (1.15, 2.33) | 6.70% | 1.02 | 1.26 |
| PTGR1 | -1 | 0.005430957 | 0.62 (0.44, 0.87) | 6.40% | 1.02 | 1.29 |
| PTGR1 | -1 | 0.005430957 | 0.62 (0.44, 0.87) | 6.40% | 1.02 | 1.29 |
| PTGR1 | -1 | 0.005430957 | 0.62 (0.44, 0.87) | 6.40% | 1.02 | 1.29 |
| PTK2 | 1 | 0.003632816 | 1.53 (1.15, 2.04) | 5.30% | 1.02 | 1.28 |
| PTK2 | 1 | 0.003632816 | 1.53 (1.15, 2.04) | 5.30% | 1.02 | 1.28 |
| PTK2 | 1 | 0.003632816 | 1.53 (1.15, 2.04) | 5.30% | 1.02 | 1.28 |
| PTPLB | 1 | 0.003892716 | 1.62 (1.17, 2.25) | 5.40% | 1.02 | 1.28 |
| PTRH2 | 1 | 0.004217013 | 1.50 (1.14, 1.99) | 5.60% | 1.02 | 1.27 |
| RAB1A | 1 | 0.005732236 | 1.58 (1.14, 2.19) | 6.60% | 1.02 | 1.27 |
| RAB1A | 1 | 0.005732236 | 1.58 (1.14, 2.19) | 6.60% | 1.02 | 1.27 |
| RAB32 | -1 | 0.004345803 | 0.68 (0.52, 0.89) | 5.70% | 1.02 | 1.28 |
| RAD54B | 1 | 0.004999815 | 1.60 (1.15, 2.23) | 6.20% | 1.02 | 1.27 |
| RBM34 | 1 | 0.004614592 | 1.61 (1.16, 2.24) | 6.00% | 1.02 | 1.27 |
| RBM34 | 1 | 0.004614592 | 1.61 (1.16, 2.24) | 6.00% | 1.02 | 1.27 |
| RBM34 | 1 | 0.004614592 | 1.61 (1.16, 2.24) | 6.00% | 1.02 | 1.27 |
| RERG | -1 | 0.006256771 | 0.60 (0.41, 0.86) | 7.00% | 1.02 | 1.28 |
| RERG | -1 | 0.006256771 | 0.60 (0.41, 0.86) | 7.00% | 1.02 | 1.28 |
| RG9MTD2 | -1 | 0.005451293 | 0.68 (0.52, 0.89) | 6.40% | 1.02 | 1.27 |
| RG9MTD2 | -1 | 0.005451293 | 0.68 (0.52, 0.89) | 6.40% | 1.02 | 1.27 |
| RG9MTD2 | -1 | 0.005451293 | 0.68 (0.52, 0.89) | 6.40% | 1.02 | 1.27 |
| RIBC1 | -1 | 0.005090705 | 0.63 (0.46, 0.87) | 6.20% | 1.02 | 1.29 |
| RIBC1 | -1 | 0.005090705 | 0.63 (0.46, 0.87) | 6.20% | 1.02 | 1.29 |
| RIPK2 | 1 | 0.004284645 | 1.66 (1.17, 2.34) | 5.70% | 1.02 | 1.28 |
| RIPPLY2 | 1 | 0.00362128 | 1.39 (1.11, 1.73) | 5.30% | 1.02 | 1.26 |
| RNF215 | -1 | 0.003675247 | 0.65 (0.49, 0.87) | 5.30% | 1.02 | 1.29 |
| RPL23AP82 | 1 | 0.003704396 | 1.41 (1.12, 1.78) | 5.30% | 1.02 | 1.26 |
| RPL23AP82 | 1 | 0.003704396 | 1.41 (1.12, 1.78) | 5.30% | 1.02 | 1.26 |
| RRM2 | 1 | 0.00530057 | 2.12 (1.25, 3.59) | 6.30% | 1.02 | 1.24 |
| RRM2 | 1 | 0.00530057 | 2.12 (1.25, 3.59) | 6.30% | 1.02 | 1.24 |
| RRP8 | -1 | 0.004670198 | 0.63 (0.45, 0.87) | 6.00% | 1.02 | 1.29 |
| RSPH1 | -1 | 0.005024925 | 0.63 (0.45, 0.87) | 6.20% | 1.02 | 1.29 |
| SAMD14 | -1 | 0.003593798 | 0.73 (0.59, 0.90) | 5.20% | 1.02 | 1.26 |
| SECISBP2 | -1 | 0.00565347 | 0.56 (0.37, 0.85) | 6.60% | 1.02 | 1.28 |
| SEPT1 | -1 | 0.003777972 | 0.62 (0.45, 0.86) | 5.40% | 1.02 | 1.3 |
| SEPT7 | 1 | 0.003942003 | 1.65 (1.17, 2.32) | 5.50% | 1.02 | 1.28 |
| SEPT7 | 1 | 0.003942003 | 1.65 (1.17, 2.32) | 5.50% | 1.02 | 1.28 |
| SET | -1 | 0.005433028 | 0.57 (0.39, 0.85) | 6.40% | 1.02 | 1.28 |
| SET | -1 | 0.005433028 | 0.57 (0.39, 0.85) | 6.40% | 1.02 | 1.28 |
| SH2B1 | -1 | 0.004950832 | 0.65 (0.48, 0.88) | 6.20% | 1.02 | 1.29 |
| SH2B1 | -1 | 0.004950832 | 0.65 (0.48, 0.88) | 6.20% | 1.02 | 1.29 |
| SH2B1 | -1 | 0.004950832 | 0.65 (0.48, 0.88) | 6.20% | 1.02 | 1.29 |
| SH2B1 | -1 | 0.004950832 | 0.65 (0.48, 0.88) | 6.20% | 1.02 | 1.29 |
| SH2B1 | -1 | 0.004950832 | 0.65 (0.48, 0.88) | 6.20% | 1.02 | 1.29 |
| SH2D6 | 1 | 0.004314207 | 1.42 (1.12, 1.82) | 5.70% | 1.02 | 1.26 |
| SI | 1 | 0.005422844 | 1.39 (1.10, 1.75) | 6.40% | 1.02 | 1.25 |
| SIPA1L2 | 1 | 0.005953535 | 1.62 (1.15, 2.28) | 6.80% | 1.02 | 1.26 |
| SLC28A1 | 1 | 0.005368187 | 1.71 (1.17, 2.50) | 6.40% | 1.02 | 1.27 |
| SLC28A1 | 1 | 0.005368187 | 1.71 (1.17, 2.50) | 6.40% | 1.02 | 1.27 |
| SLC6A10P | 1 | 0.004304834 | 1.34 (1.10, 1.63) | 5.70% | 1.02 | 1.24 |
| SLC7A8 | -1 | 0.006012426 | 0.59 (0.40, 0.86) | 6.80% | 1.02 | 1.28 |
| SLC7A8 | -1 | 0.006012426 | 0.59 (0.40, 0.86) | 6.80% | 1.02 | 1.28 |
| SLITRK2 | 1 | 0.004114097 | 1.45 (1.12, 1.86) | 5.60% | 1.02 | 1.27 |
| SLITRK2 | 1 | 0.004114097 | 1.45 (1.12, 1.86) | 5.60% | 1.02 | 1.27 |
| SLITRK2 | 1 | 0.004114097 | 1.45 (1.12, 1.86) | 5.60% | 1.02 | 1.27 |
| SLITRK2 | 1 | 0.004114097 | 1.45 (1.12, 1.86) | 5.60% | 1.02 | 1.27 |
| SLITRK2 | 1 | 0.004114097 | 1.45 (1.12, 1.86) | 5.60% | 1.02 | 1.27 |
| SLITRK2 | 1 | 0.004114097 | 1.45 (1.12, 1.86) | 5.60% | 1.02 | 1.27 |
| SLITRK2 | 1 | 0.004114097 | 1.45 (1.12, 1.86) | 5.60% | 1.02 | 1.27 |
| SLITRK2 | 1 | 0.004114097 | 1.45 (1.12, 1.86) | 5.60% | 1.02 | 1.27 |
| SMARCC1 | 1 | 0.004969555 | 1.61 (1.15, 2.24) | 6.20% | 1.02 | 1.27 |
| SNRPB2 | 1 | 0.003112308 | 1.40 (1.12, 1.75) | 4.80% | 1.02 | 1.26 |
| SNRPB2 | 1 | 0.003112308 | 1.40 (1.12, 1.75) | 4.80% | 1.02 | 1.26 |
| SOCS6 | -1 | 0.004297582 | 0.60 (0.42, 0.85) | 5.70% | 1.02 | 1.29 |
| SOX17 | -1 | 0.00472738 | 0.62 (0.44, 0.86) | 6.00% | 1.02 | 1.29 |
| SOX2OT | -1 | 0.006142043 | 0.55 (0.35, 0.84) | 6.90% | 1.02 | 1.28 |
| SPA17 | -1 | 0.004800037 | 0.72 (0.57, 0.90) | 6.10% | 1.02 | 1.26 |
| SPAG17 | -1 | 0.006045057 | 0.60 (0.42, 0.86) | 6.80% | 1.02 | 1.28 |
| SPINK8 | 1 | 0.005890681 | 1.59 (1.14, 2.22) | 6.70% | 1.02 | 1.27 |
| SRBD1 | 1 | 0.003804838 | 1.55 (1.15, 2.09) | 5.40% | 1.02 | 1.28 |
| SRD5A1 | 1 | 0.006103365 | 1.58 (1.14, 2.20) | 6.90% | 1.02 | 1.26 |
| SRPRB | 1 | 0.004270811 | 1.53 (1.14, 2.06) | 5.70% | 1.02 | 1.27 |
| STK3 | 1 | 0.004308969 | 1.79 (1.20, 2.67) | 5.70% | 1.02 | 1.27 |
| STON2 | -1 | 0.005917452 | 0.55 (0.36, 0.84) | 6.80% | 1.02 | 1.28 |
| TAF2 | 1 | 0.00478922 | 1.48 (1.13, 1.93) | 6.10% | 1.02 | 1.27 |
| TBX3 | -1 | 0.004788214 | 0.60 (0.43, 0.86) | 6.10% | 1.02 | 1.29 |
| TBX3 | -1 | 0.004788214 | 0.60 (0.43, 0.86) | 6.10% | 1.02 | 1.29 |
| TDO2 | 1 | 0.005420059 | 1.57 (1.14, 2.16) | 6.40% | 1.02 | 1.27 |
| TEKT2 | -1 | 0.005472115 | 0.61 (0.43, 0.86) | 6.40% | 1.02 | 1.29 |
| TEX101 | -1 | 0.004405596 | 0.57 (0.39, 0.84) | 5.80% | 1.02 | 1.29 |
| TEX101 | -1 | 0.004405596 | 0.57 (0.39, 0.84) | 5.80% | 1.02 | 1.29 |
| TFF2 | 1 | 0.002565639 | 1.30 (1.10, 1.53) | 4.30% | 1.02 | 1.23 |
| TGFBR3 | -1 | 0.005568795 | 0.59 (0.40, 0.86) | 6.50% | 1.02 | 1.28 |
| TGFBR3 | -1 | 0.005568795 | 0.59 (040, 0.86) | 6.50% | 1.02 | 1.28 |
| TGFBR3 | -1 | 0.005568795 | 0.59 (0.40, 0.86) | 6.50% | 1.02 | 1.28 |
| TGFBR3 | -1 | 0.005568795 | 0.59 (0.40, 0.86) | 6.50% | 1.02 | 1.28 |
| TLX3 | 1 | 0.005429471 | 1.44 (1.11, 1.86) | 6.40% | 1.02 | 1.26 |
| TM4SF20 | -1 | 0.005502692 | 0.61 (0.43, 0.87) | 6.40% | 1.02 | 1.29 |
| TMBIM1 | -1 | 0.005802111 | 0.62 (0.44, 0.87) | 6.70% | 1.02 | 1.28 |
| TMC6 | 1 | 0.004143969 | 1.69 (1.18, 2.42) | 5.60% | 1.02 | 1.28 |
| TMC6 | 1 | 0.004143969 | 1.69 (1.18, 2.42) | 5.60% | 1.02 | 1.28 |
| TMEM132B | 1 | 0.003802933 | 1.57 (1.16, 2.13) | 5.40% | 1.02 | 1.28 |
| TMEM165 | 1 | 0.003867527 | 1.49 (1.14, 1.96) | 5.40% | 1.02 | 1.27 |
| TMEM167B | -1 | 0.005258342 | 0.67 (0.51, 0.89) | 6.30% | 1.02 | 1.28 |
| TMEM49 | 1 | 0.005254418 | 1.54 (1.14, 2.09) | 6.30% | 1.02 | 1.27 |
| TP5313 | -1 | 0.004119014 | 0.63 (0.47, 0.87) | 5.60% | 1.02 | 1.29 |
| TP5313 | -1 | 0.004119014 | 0.63 (0.47, 0.87) | 5.60% | 1.02 | 1.29 |
| TPH1 | -1 | 0.004144358 | 0.59 (0.41, 0.85) | 5.60% | 1.02 | 1.3 |
| TPRG1 | -1 | 0.004865463 | 0.54 (0.35, 0.83) | 6.10% | 1.02 | 1.28 |
| TRIP10 | -1 | 0.004862787 | 0.66 (0.50, 0.88) | 6.10% | 1.02 | 1.28 |
| TRYX3 | 1 | 0.005334529 | 1.48 (1.12, 1.95) | 6.30% | 1.02 | 1.26 |
| TTLL9 | -1 | 0.004802396 | 0.59 (0.41, 0.85) | 6.10% | 1.02 | 1.29 |
| UBTF | -1 | 0.003797859 | 0.64 (0.48, 0.87) | 5.40% | 1.02 | 1.29 |
| UBTF | -1 | 0.003797859 | 0.64 (0.48, 0.87) | 5.40% | 1.02 | 1.29 |
| UBTF | -1 | 0.003797859 | 0.64 (0.48, 0.87) | 5.40% | 1.02 | 1.29 |
| USP10 | 1 | 0.004974453 | 1.60 (1.15, 2.22) | 6.20% | 1.02 | 1.27 |
| USP20 | -1 | 0.006090279 | 0.52 (0.33, 0.83) | 6.90% | 1.02 | 1.27 |
| USP20 | -1 | 0.006090279 | 0.52 (0.33, 0.83) | 6.90% | 1.02 | 1.27 |
| USP20 | -1 | 0.006090279 | 0.52 (0.33, 0.83) | 6.90% | 1.02 | 1.27 |
| USP6NL | 1 | 0.005838641 | 1.62 (1.15, 2.28) | 6.70% | 1.02 | 1.27 |
| USP6NL | 1 | 0.005838641 | 1.62 (1.15, 2.28) | 6.70% | 1.02 | 1.27 |
| VEGFA | 1 | 0.004912488 | 1.55 (1.14, 2.11) | 6.20% | 1.02 | 1.27 |
| VEGFA | 1 | 0.004912488 | 1.55 (1.14, 2.11) | 6.20% | 1.02 | 1.27 |
| VEGFA | 1 | 0.004912488 | 1.55 (1.14, 2.11) | 6.20% | 1.02 | 1.27 |
| VEGFA | 1 | 0.004912488 | 1.55 (1.14, 2.11) | 6.20% | 1.02 | 1.27 |
| VEGFA | 1 | 0.004912488 | 1.55 (1.14, 2.11) | 6.20% | 1.02 | 1.27 |
| VEGFA | 1 | 0.004912488 | 1.55 (1.14, 2.11) | 6.20% | 1.02 | 1.27 |
| VEGFA | 1 | 0.004912488 | 1.55 (1.14, 2.11) | 6.20% | 1.02 | 1.27 |
| VEGFA | 1 | 0.004912488 | 1.55 (1.14, 2.11) | 6.20% | 1.02 | 1.27 |
| VEGFA | 1 | 0.004912488 | 1.55 (1.14, 2.11) | 6.20% | 1.02 | 1.27 |
| VEGFA | 1 | 0.004912488 | 1.55 (1.14, 2.11) | 6.20% | 1.02 | 1.27 |
| VEGFA | 1 | 0.004912488 | 1.55 (1.14, 2.11) | 6.20% | 1.02 | 1.27 |
| VEGFA | 1 | 0.004912488 | 1.55 (1.14, 2.11) | 6.20% | 1.02 | 1.27 |
| VEGFA | 1 | 0.004912488 | 1.55 (1.14, 2.11) | 6.20% | 1.02 | 1.27 |
| VEGFA | 1 | 0.004912488 | 1.55 (1.14, 2.11) | 6.20% | 1.02 | 1.27 |
| VEGFA | 1 | 0.004912488 | 1.55 (1.14, 2.11) | 6.20% | 1.02 | 1.27 |
| VEGFA | 1 | 0.004912488 | 1.55 (1.14, 2.11) | 6.20% | 1.02 | 1.27 |
| VPS13D | 1 | 0.00462902 | 1.61 (1.16, 2.25) | 6.00% | 1.02 | 1.27 |
| VPS13D | 1 | 0.00462902 | 1.61 (1.16, 2.25) | 6.00% | 1.02 | 1.27 |
| WDR91 | 1 | 0.004652961 | 1.64 (1.16, 2.31) | 6.00% | 1.02 | 1.27 |
| XPO1 | 1 | 0.0059138 | 1.68 (1.16, 2.43) | 6.80% | 1.02 | 1.26 |
| ZNF197 | -1 | 0.004824119 | 0.64 (0.47, 0.87) | 6.10% | 1.02 | 1.29 |
| ZNF197 | -1 | 0.004824119 | 0.64 (0.47, 0.87) | 6.10% | 1.02 | 1.29 |
| ZNF239 | -1 | 0.004350897 | 0.66 (0.50, 0.88) | 5.80% | 1.02 | 1.29 |
| ZNF239 | -1 | 0.004350897 | 0.66 (0.50, 0.88) | 5.80% | 1.02 | 1.29 |
| ZNF239 | -1 | 0.004350897 | 0.66 (0.50, 0.88) | 5.80% | 1.02 | 1.29 |
| ZNF239 | -1 | 0.004350897 | 0.66 (0.50, 0.88) | 5.80% | 1.02 | 1.29 |
| ZNF346 | 1 | 0.005370981 | 1.47 (1.12, 1.94) | 6.40% | 1.02 | 1.26 |
| ZNF434 | -1 | 0.004137381 | 0.58 (0.40, 0.84) | 5.60% | 1.02 | 1.3 |
| ZNF616 | -1 | 0.004212388 | 0.57 (0.39, 0.84) | 5.60% | 1.02 | 1.29 |
| ZNF619 | -1 | 0.004847157 | 0.61 (0.43, 0.86) | 6.10% | 1.02 | 1.29 |
| ZNF619 | -1 | 0.004847157 | 0.61 (0.43, 0.86) | 6.10% | 1.02 | 1.29 |
| ZNF619 | -1 | 0.004847157 | 0.61 (0.43, 0.86) | 6.10% | 1.02 | 1.29 |
| ZNF619 | -1 | 0.004847157 | 0.61 (0.43, 0.86) | 6.10% | 1.02 | 1.29 |
| ZNF710 | 1 | 0.004198366 | 1.37 (1.11, 1.71) | 5.60% | 1.02 | 1.25 |
| ZNF738 | -1 | 0.005996712 | 0.64 (0.47, 0.88) | 6.80% | 1.02 | 1.28 |
| ZNRF2 | 1 | 0.002907072 | 1.35 (1.11, 1.64) | 4.60% | 1.02 | 1.25 |
| ZSCAN16 | -1 | 0.003648245 | 0.66 (0.50, 0.87) | 5.30% | 1.02 | 1.29 |
| ZWILCH | 1 | 0.006215321 | 1.62 (1.15, 2.28) | 6.90% | 1.02 | 1.26 |
| ZWILCH | 1 | 0.006215321 | 1.62 (1.15, 2.28) | 6.90% | 1.02 | 1.26 |
| AARS | 1 | 0.006581854 | 1.65 (1.15, 2.36) | 7.10% | 1.01 | 1.26 |
| ABAT | -1 | 0.008694362 | 0.53 (0.33, 0.85) | 8.20% | 1.01 | 1.26 |
| ABAT | -1 | 0.008694362 | 0.53 (0.33, 0.85) | 8.20% | 1.01 | 1.26 |
| ABAT | -1 | 0.008694362 | 0.53 (0.33, 0.85) | 8.20% | 1.01 | 1.26 |
| ABHD5 | 1 | 0.007921655 | 1.61 (1.13, 2.29) | 7.80% | 1.01 | 1.25 |
| ABL1 | -1 | 0.00834792 | 0.62 (0.44, 0.89) | 8.00% | 1.01 | 1.27 |
| ABL1 | -1 | 0.00834792 | 0.62 (0.44, 0.89) | 8.00% | 1.01 | 1.27 |
| ABR | -1 | 0.007662518 | 0.60 (0.41, 0.87) | 7.70% | 1.01 | 1.27 |
| ABR | -1 | 0.007662518 | 0.60 (0.41, 0.87) | 7.70% | 1.01 | 1.27 |
| ABR | -1 | 0.007662518 | 0.60 (0.41, 0.87) | 7.70% | 1.01 | 1.27 |
| ABTB2 | 1 | 0.008523209 | 1.52 (1.11, 2.07) | 8.10% | 1.01 | 1.25 |
| ADAMTS8 | -1 | 0.006436924 | 0.66 (0.48, 0.89) | 7.10% | 1.01 | 1.28 |
| AFM | 1 | 0.005145801 | 1.19 (1.05, 1.35) | 6.20% | 1.01 | 1.17 |
| ALDH7A1 | 1 | 0.008021584 | 1.72 (1.15, 2.57) | 7.90% | 1.01 | 1.25 |
| ALKBH3 | -1 | 0.009889635 | 0.61 (042, 0.89) | 8.70% | 1.01 | 1.26 |
| ALOX12 | -1 | 0.00634658 | 0.62 (0.44, 0.88) | 7.00% | 1.01 | 1.28 |
| ANTXRL | 1 | 0.00868012 | 1.56 (1.12, 2.18) | 8.20% | 1.01 | 1.25 |
| ARHGEF4 | 1 | 0.009414554 | 1.71 (1.14, 2.56) | 8.50% | 1.01 | 1.24 |
| ARHGEF4 | 1 | 0.009414554 | 1.71 (1.14, 2.56) | 8.50% | 1.01 | 1.24 |
| ASAH2B | -1 | 0.006655111 | 0.72 (0.57, 0.91) | 7.10% | 1.01 | 1.26 |
| ASB13 | -1 | 0.007573921 | 0.65 (0.47, 0.89) | 7.70% | 1.01 | 1.27 |
| ASB13 | -1 | 0.007573921 | 0.65 (0.47, 0.89) | 7.70% | 1.01 | 1.27 |
| ASB13 | -1 | 0.007573921 | 0.65 (0.47, 0.89) | 7.70% | 1.01 | 1.27 |
| ASPM | 1 | 0.006786143 | 1.91 (1.20, 3.05) | 7.20% | 1.01 | 1.24 |
| ASTN2 | -1 | 0.008824541 | 0.56 (0.36, 0.86) | 8.20% | 1.01 | 1.26 |
| ASTN2 | -1 | 0.008824541 | 0.56 (0.36, 0.86) | 8.20% | 1.01 | 1.26 |
| ASTN2 | -1 | 0.008824541 | 0.56 (0.36, 0.86) | 8.20% | 1.01 | 1.26 |
| ASTN2 | -1 | 0.008824541 | 0.56 (0.36, 0.86) | 8.20% | 1.01 | 1.26 |
| ASTN2 | -1 | 0.008824541 | 0.56 (0.36, 0.86) | 8.20% | 1.01 | 1.26 |
| ASTN2 | -1 | 0.008824541 | 0.56 (0.36, 0.86) | 8.20% | 1.01 | 1.26 |
| ATL2 | 1 | 0.008592516 | 1.47 (1.10, 1.97) | 8.10% | 1.01 | 1.25 |
| ATL2 | 1 | 0.008592516 | 1.47 (1.10, 1.97) | 8.10% | 1.01 | 1.25 |
| ATL2 | 1 | 0.008592516 | 1.47 (1.10, 1.97) | 8.10% | 1.01 | 1.25 |
| ATXN3 | -1 | 0.009014078 | 0.62 (0.43, 0.89) | 8.30% | 1.01 | 1.27 |
| ATXN3 | -1 | 0.009014078 | 0.62 (0.43, 0.89) | 8.30% | 1.01 | 1.27 |
| ATXN3 | -1 | 0.009014078 | 0.62 (0.43, 0.89) | 8.30% | 1.01 | 1.27 |
| ATXN3 | -1 | 0.009014078 | 0.62 (0.43, 0.89) | 8.30% | 1.01 | 1.27 |
| ATXN3 | -1 | 0.009014078 | 0.62 (0.43, 0.89) | 8.30% | 1.01 | 1.27 |
| ATXN3 | -1 | 0.009014078 | 0.62 (0.43, 0.89) | 8.30% | 1.01 | 1.27 |
| ATXN3 | -1 | 0.009014078 | 0.62 (0.43, 0.89) | 8.30% | 1.01 | 1.27 |
| ATXN3 | -1 | 0.009014078 | 0.62 (0.43, 0.89) | 8.30% | 1.01 | 1.27 |
| ATXN3 | -1 | 0.009014078 | 0.62 (0.43, 0.89) | 8.30% | 1.01 | 1.27 |
| ATXN3 | -1 | 0.009014078 | 0.62 (0.43, 0.89) | 8.30% | 1.01 | 1.27 |
| ATXN3 | -1 | 0.009014078 | 0.62 (0.43, 0.89) | 8.30% | 1.01 | 1.27 |
| ATXN3 | -1 | 0.009014078 | 0.62 (0.43, 0.89) | 8.30% | 1.01 | 1.27 |
| ATXN3 | -1 | 0.009014078 | 0.62 (0.43, 0.89) | 8.30% | 1.01 | 1.27 |
| ATXN3 | -1 | 0.009014078 | 0.62 (0.43, 0.89) | 8.30% | 1.01 | 1.27 |
| ATXN3 | -1 | 0.009014078 | 0.62 (0.43, 0.89) | 8.30% | 1.01 | 1.27 |
| ATXN3 | -1 | 0.009014078 | 0.62 (0.43, 0.89) | 8.30% | 1.01 | 1.27 |
| ATXN3 | -1 | 0.009014078 | 0.62 (0.43, 0.89) | 8.30% | 1.01 | 1.27 |
| ATXN3 | -1 | 0.009014078 | 0.62 (0.43, 0.89) | 8.30% | 1.01 | 1.27 |
| ATXN3 | -1 | 0.009014078 | 0.62 (0.43, 0.89) | 8.30% | 1.01 | 1.27 |
| ATXN3 | -1 | 0.009014078 | 0.62 (0.43, 0.89) | 8.30% | 1.01 | 1.27 |
| ATXN3 | -1 | 0.009014078 | 0.62 (0.43, 0.89) | 8.30% | 1.01 | 1.27 |
| ATXN3 | -1 | 0.009014078 | 0.62 (0.43, 0.89) | 8.30% | 1.01 | 1.27 |
| ATXN3 | -1 | 0.009014078 | 0.62 (0.43, 0.89) | 8.30% | 1.01 | 1.27 |
| ATXN3 | -1 | 0.009014078 | 0.62 (0.43, 0.89) | 8.30% | 1.01 | 1.27 |
| ATXN3 | -1 | 0.009014078 | 0.62 (0.43, 0.89) | 8.30% | 1.01 | 1.27 |
| ATXN3 | -1 | 0.009014078 | 0.62 (0.43, 0.89) | 8.30% | 1.01 | 1.27 |
| ATXN3 | -1 | 0.009014078 | 0.62 (0.43, 0.89) | 8.30% | 1.01 | 1.27 |
| ATXN3 | -1 | 0.009014078 | 0.62 (0.43, 0.89) | 8.30% | 1.01 | 1.27 |
| ATXN3 | -1 | 0.009014078 | 0.62 (0.43, 0.89) | 8.30% | 1.01 | 1.27 |
| ATXN3 | -1 | 0.009014078 | 0.62 (0.43, 0.89) | 8.30% | 1.01 | 1.27 |
| ATXN3 | -1 | 0.009014078 | 0.62 (0.43, 0.89) | 8.30% | 1.01 | 1.27 |
| AZIN1 | 1 | 0.009281268 | 1.47 (1.10, 1.95) | 8.50% | 1.01 | 1.25 |
| AZIN1 | 1 | 0.009281268 | 1.47 (1.10, 1.95) | 8.50% | 1.01 | 1.25 |
| BLOC1S1-RDH5 | -1 | 0.00930281 | 0.62 (0.43, 0.89) | 8.50% | 1.01 | 1.27 |
| BRD3 | -1 | 0.008625203 | 0.58 (0.39, 0.87) | 8.10% | 1.01 | 1.27 |
| BRD9 | 1 | 0.006755386 | 1.66 (1.15, 2.41) | 7.20% | 1.01 | 1.26 |
| BRD9 | 1 | 0.006755386 | 1.66 (1.15, 2.41) | 7.20% | 1.01 | 1.26 |
| BRD9 | 1 | 0.006755386 | 1.66 (1.15, 2.41) | 7.20% | 1.01 | 1.26 |
| BUB1B | 1 | 0.007054086 | 1.95 (1.20, 3.18) | 7.30% | 1.01 | 1.24 |
| C12orf52 | -1 | 0.006034719 | 0.72 (0.57, 0.91) | 6.80% | 1.01 | 1.26 |
| C12orf53 | -1 | 0.007888739 | 0.62 (0.43, 0.88) | 7.80% | 1.01 | 1.27 |
| C12orf76 | -1 | 0.006422511 | 0.60 (0.42, 0.87) | 7.10% | 1.01 | 1.28 |
| C14orf132 | -1 | 0.01017781 | 0.62 (0.43, 0.89) | 8.90% | 1.01 | 1.26 |
| C14orf145 | 1 | 0.009860907 | 1.47 (1.10, 1.97) | 8.70% | 1.01 | 1.24 |
| C14orf147 | 1 | 0.0076906 | 1.58 (1.13, 2.21) | 7.70% | 1.01 | 1.26 |
| C14orf38 | -1 | 0.008239414 | 0.63 (0.45, 0.89) | 8.00% | 1.01 | 1.27 |
| C1orf174 | -1 | 0.008033783 | 0.66 (0.48, 0.90) | 7.90% | 1.01 | 1.27 |
| C1orf38 | 1 | 0.008713276 | 1.58 (1.12, 2.22) | 8.20% | 1.01 | 1.25 |
| C1orf38 | 1 | 0.008713276 | 1.58 (1.12, 2.22) | 8.20% | 1.01 | 1.25 |
| C1orf38 | 1 | 0.008713276 | 1.58 (1.12, 2.22) | 8.20% | 1.01 | 1.25 |
| C1orf9 | 1 | 0.008402668 | 1.56 (1.12, 2.16) | 8.10% | 1.01 | 1.25 |
| C1orf9 | 1 | 0.008402668 | 1.56 (1.12, 2.16) | 8.10% | 1.01 | 1.25 |
| C1QTNF6 | -1 | 0.006525076 | 0.67 (0.51, 0.90) | 7.10% | 1.01 | 1.27 |
| C1QTNF6 | -1 | 0.006525076 | 0.67 (0.51, 0.90) | 7.10% | 1.01 | 1.27 |
| C4orf44 | -1 | 0.007314886 | 0.66 (0.49, 0.89) | 7.50% | 1.01 | 1.27 |
| C7orf63 | -1 | 0.007739854 | 0.60 (0.41, 0.87) | 7.70% | 1.01 | 1.27 |
| C7orf63 | -1 | 0.007739854 | 0.60 (0.41, 0.87) | 7.70% | 1.01 | 1.27 |
| C8orf73 | 1 | 0.00790054 | 1.92 (1.19, 3.12) | 7.80% | 1.01 | 1.24 |
| C9orf46 | -1 | 0.006592071 | 0.56 (0.37, 0.85) | 7.10% | 1.01 | 1.28 |
| C9orf47 | 1 | 0.005586283 | 1.36 (1.09, 1.68) | 6.50% | 1.01 | 1.24 |
| C9orf47 | 1 | 0.005586283 | 1.36 (1.09, 1.68) | 6.50% | 1.01 | 1.24 |
| CACNA1B | 1 | 0.008323894 | 1.47 (1.10, 1.97) | 8.00% | 1.01 | 1.25 |
| CATSPERG | -1 | 0.0079658 | 0.62 (0.43, 0.88) | 7.90% | 1.01 | 1.27 |
| CCDC101 | -1 | 0.007432279 | 0.64 (0.46, 0.89) | 7.60% | 1.01 | 1.27 |
| CCDC153 | -1 | 0.007781985 | 0.61 (0.43, 0.88) | 7.80% | 1.01 | 1.27 |
| CCDC28B | -1 | 0.009710454 | 0.62 (0.44, 0.89) | 8.70% | 1.01 | 1.26 |
| CCDC66 | -1 | 0.009413964 | 0.70 (0.54, 0.92) | 8.50% | 1.01 | 1.25 |
| CCDC66 | -1 | 0.009413964 | 0.70 (0.54, 0.92) | 8.50% | 1.01 | 1.25 |
| CCDC66 | -1 | 0.009413964 | 0.70 (0.54, 0.92) | 8.50% | 1.01 | 1.25 |
| CCDC68 | -1 | 0.006409628 | 0.65 (0.48, 0.89) | 7.10% | 1.01 | 1.28 |
| CCDC68 | -1 | 0.006409628 | 0.65 (0.48, 0.89) | 7.10% | 1.01 | 1.28 |
| CCDC85A | -1 | 0.006939346 | 0.57 (0.38, 0.86) | 7.30% | 1.01 | 1.27 |
| CCDC97 | -1 | 0.008529916 | 0.62 (0.43, 0.88) | 8.10% | 1.01 | 1.27 |
| CDC73 | 1 | 0.008041561 | 1.69 (1.15, 2.49) | 7.90% | 1.01 | 1.25 |
| CDH8 | 1 | 0.008791148 | 1.52 (1.11, 2.08) | 8.20% | 1.01 | 1.25 |
| CENPO | 1 | 0.008398103 | 1.87 (1.17, 2.98) | 8.10% | 1.01 | 1.24 |
| CENPO | 1 | 0.008398103 | 1.87 (1.17, 2.98) | 8.10% | 1.01 | 1.24 |
| CENPW | 1 | 0.010108567 | 1.63 (1.12, 2.36) | 8.90% | 1.01 | 1.24 |
| CFDP1 | 1 | 0.009181598 | 1.42 (1.09, 1.84) | 8.50% | 1.01 | 1.24 |
| CHCHD10 | 1 | 0.007522027 | 2.38 (1.26, 4.50) | 7.70% | 1.01 | 1.2 |
| CHI3L1 | 1 | 0.006668226 | 1.67 (1.15, 2.42) | 7.10% | 1.01 | 1.26 |
| CIT | 1 | 0.007377258 | 1.85 (1.18, 2.89) | 7.60% | 1.01 | 1.24 |
| CLASRP | -1 | 0.008231783 | 0.66 (0.48, 0.90) | 8.00% | 1.01 | 1.27 |
| CLCN5 | 1 | 0.009310865 | 1.55 (1.11, 2.16) | 8.50% | 1.01 | 1.25 |
| CLCN5 | 1 | 0.009310865 | 1.55 (1.11, 2.16) | 8.50% | 1.01 | 1.25 |
| CLCN5 | 1 | 0.009310865 | 1.55 (1.11, 2.16) | 8.50% | 1.01 | 1.25 |
| CLEC16A | -1 | 0.010281483 | 0.56 (0.36, 0.87) | 8.90% | 1.01 | 1.26 |
| CMA1 | -1 | 0.008693923 | 0.61 (0.42, 0.88) | 8.20% | 1.01 | 1.27 |
| CNGA1 | 1 | 0.00795389 | 1.47 (1.11, 1.95) | 7.90% | 1.01 | 1.25 |
| CNGA1 | 1 | 0.00795389 | 1.47 (1.11, 1.95) | 7.90% | 1.01 | 1.25 |
| COMMD9 | -1 | 0.007675478 | 0.65 (0.48, 0.89) | 7.70% | 1.01 | 1.27 |
| COMMD9 | -1 | 0.007675478 | 0.65 (0.48, 0.89) | 7.70% | 1.01 | 1.27 |
| CPLX2 | 1 | 0.008558358 | 1.51 (1.11, 2.05) | 8.10% | 1.01 | 1.25 |
| CPLX2 | 1 | 0.008558358 | 1.51 (1.11, 2.05) | 8.10% | 1.01 | 1.25 |
| CPLX4 | 1 | 0.00691338 | 1.42 (1.10, 1.84) | 7.30% | 1.01 | 1.25 |
| CRABP1 | 1 | 0.006626223 | 1.53 (1.13, 2.09) | 7.10% | 1.01 | 1.26 |
| CRMP1 | -1 | 0.007392616 | 0.64 (0.46, 0.89) | 7.60% | 1.01 | 1.27 |
| CRMP1 | -1 | 0.007392616 | 0.64 (0.46, 0.89) | 7.60% | 1.01 | 1.27 |
| CSPG5 | 1 | 0.009780303 | 1.84 (1.16, 2.92) | 8.70% | 1.01 | 1.23 |
| CYB5D2 | -1 | 0.006826525 | 0.64 (0.47, 0.88) | 7.20% | 1.01 | 1.28 |
| CYB5D2 | -1 | 0.006826525 | 0.64 (0.47, 0.88) | 7.20% | 1.01 | 1.28 |
| CYB5D2 | -1 | 0.006826525 | 0.64 (0.47, 0.88) | 7.20% | 1.01 | 1.28 |
| CYP4F8 | -1 | 0.006572164 | 0.56 (0.37, 0.85) | 7.10% | 1.01 | 1.28 |
| DBF4 | 1 | 0.007605751 | 1.63 (1.14, 2.33) | 7.70% | 1.01 | 1.26 |
| DCAF16 | -1 | 0.006850158 | 0.56 (0.36, 0.85) | 7.20% | 1.01 | 1.27 |
| DEFB126 | 1 | 0.009243031 | 1.35 (1.08, 1.70) | 8.50% | 1.01 | 1.23 |
| DERA | -1 | 0.005991399 | 0.67 (0.51, 0.89) | 6.80% | 1.01 | 1.27 |
| DHRS3 | 1 | 0.006553888 | 1.67 (1.15, 2.41) | 7.10% | 1.01 | 1.26 |
| DHRS4 | -1 | 0.007490633 | 0.65 (0.48, 0.89) | 7.60% | 1.01 | 1.27 |
| DHX40 | 1 | 0.006401593 | 1.44 (1.11, 1.88) | 7.10% | 1.01 | 1.25 |
| DHX40 | 1 | 0.006401593 | 1.44 (1.11, 1.88) | 7.10% | 1.01 | 1.25 |
| DLEC1 | -1 | 0.007441555 | 0.57 (0.37, 0.86) | 7.60% | 1.01 | 1.27 |
| DLEC1 | -1 | 0.007441555 | 0.57 (0.37, 0.86) | 7.60% | 1.01 | 1.27 |
| DMXL2 | 1 | 0.006523881 | 1.39 (1.10, 1.77) | 7.10% | 1.01 | 1.25 |
| DMXL2 | 1 | 0.006523881 | 1.39 (1.10, 1.77) | 7.10% | 1.01 | 1.25 |
| DMXL2 | 1 | 0.006523881 | 1.39 (1.10, 1.77) | 7.10% | 1.01 | 1.25 |
| DNAJC12 | -1 | 0.008733264 | 0.61 (0.42, 0.88) | 8.20% | 1.01 | 1.27 |
| DNAJC12 | -1 | 0.008733264 | 0.61 (0.42, 0.88) | 8.20% | 1.01 | 1.27 |
| DUS2L | 1 | 0.006904869 | 1.58 (1.13, 2.21) | 7.30% | 1.01 | 1.26 |
| ECT2 | 1 | 0.006333315 | 1.61 (1.14, 2.27) | 7.00% | 1.01 | 1.26 |
| EIF2AK1 | 1 | 0.006304207 | 1.58 (1.14, 2.19) | 7.00% | 1.01 | 1.26 |
| EIF2AK1 | 1 | 0.006304207 | 1.58 (1.14, 2.19) | 7.00% | 1.01 | 1.26 |
| ELP3 | -1 | 0.008530309 | 0.59 (0.40, 0.87) | 8.10% | 1.01 | 1.27 |
| EML4 | 1 | 0.00939952 | 1.49 (1.10, 2.00) | 8.50% | 1.01 | 1.25 |
| EML4 | 1 | 0.00939952 | 1.49 (1.10, 2.00) | 8.50% | 1.01 | 1.25 |
| EMP3 | -1 | 0.009949959 | 0.61 (0.42, 0.89) | 8.80% | 1.01 | 1.26 |
| EXT1 | 1 | 0.008271434 | 1.54 (1.12, 2.12) | 8.00% | 1.01 | 1.25 |
| EZR | 1 | 0.006750056 | 1.45 (1.11, 1.91) | 7.20% | 1.01 | 1.25 |
| EZR | 1 | 0.006750056 | 1.45 (1.11, 1.91) | 7.20% | 1.01 | 1.25 |
| FAF2 | 1 | 0.008604571 | 1.62 (1.13, 2.33) | 8.10% | 1.01 | 1.25 |
| FAM179B | -1 | 0.007462783 | 0.57 (0.38, 0.86) | 7.60% | 1.01 | 1.27 |
| FAM20A | 1 | 0.008860107 | 1.62 (1.13, 2.32) | 8.30% | 1.01 | 1.25 |
| FAM20A | 1 | 0.008860107 | 1.62 (1.13, 2.32) | 8.30% | 1.01 | 1.25 |
| FAM3C | 1 | 0.006674997 | 1.57 (1.13, 2.18) | 7.10% | 1.01 | 1.26 |
| FAM3C | 1 | 0.006674997 | 1.57 (1.13, 2.18) | 7.10% | 1.01 | 1.26 |
| FAM5C | 1 | 0.007019078 | 1.54 (1.13, 2.11) | 7.30% | 1.01 | 1.26 |
| FAM66A | -1 | 0.007271558 | 0.69 (0.53, 0.91) | 7.50% | 1.01 | 1.26 |
| FAM71E1 | -1 | 0.006859843 | 0.65 (047, 0.89) | 7.20% | 1.01 | 1.28 |
| FANCI | 1 | 0.006580511 | 1.50 (1.12, 2.01) | 7.10% | 1.01 | 1.26 |
| FANCI | 1 | 0.006580511 | 1.50 (1.12, 2.01) | 7.10% | 1.01 | 1.26 |
| FBXL13 | -1 | 0.007857688 | 0.65 (0.47, 0.89) | 7.80% | 1.01 | 1.27 |
| FBXL13 | -1 | 0.007857688 | 0.65 (0.47, 0.89) | 7.80% | 1.01 | 1.27 |
| FBXL3 | -1 | 0.006493504 | 0.58 (0.40, 0.86) | 7.10% | 1.01 | 1.28 |
| FBXW7 | 1 | 0.006175921 | 1.49 (1.12, 1.99) | 6.90% | 1.01 | 1.26 |
| FBXW7 | 1 | 0.006175921 | 1.49 (1.12, 1.99) | 6.90% | 1.01 | 1.26 |
| FBXW7 | 1 | 0.006175921 | 1.49 (1.12, 1.99) | 6.90% | 1.01 | 1.26 |
| FHAD1 | -1 | 0.008426078 | 0.71 (0.54, 0.91) | 8.10% | 1.01 | 1.26 |
| FIGF | 1 | 0.009002018 | 1.69 (1.14, 2.50) | 8.30% | 1.01 | 1.25 |
| FLII | -1 | 0.008128059 | 0.61 (0.43, 0.88) | 7.90% | 1.01 | 1.27 |
| FLI34503 | 1 | 0.006440243 | 1.52 (1.12, 2.05) | 7.10% | 1.01 | 1.26 |
| FSD1L | -1 | 0.009083561 | 0.66 (0.49, 0.90) | 8.40% | 1.01 | 1.26 |
| FSD1L | -1 | 0.009083561 | 0.66 (0.49, 0.90) | 8.40% | 1.01 | 1.26 |
| FSD1L | -1 | 0.009083561 | 0.66 (0.49, 0.90) | 8.40% | 1.01 | 1.26 |
| FST | -1 | 0.006099071 | 0.66 (0.48, 0.89) | 6.90% | 1.01 | 1.28 |
| FST | -1 | 0.006099071 | 0.66 (0.48, 0.89) | 6.90% | 1.01 | 1.28 |
| FXN | -1 | 0.00959081 | 0.63 (0.44, 0.89) | 8.60% | 1.01 | 1.26 |
| FXN | -1 | 0.00959081 | 0.63 (0.44, 0.89) | 8.60% | 1.01 | 1.26 |
| FXN | -1 | 0.00959081 | 0.63 (0.44, 0.89) | 8.60% | 1.01 | 1.26 |
| GABRG2 | 1 | 0.00801239 | 1.48 (1.11, 1.97) | 7.90% | 1.01 | 1.25 |
| GABRG2 | 1 | 0.00801239 | 1.48 (1.11, 1.97) | 7.90% | 1.01 | 1.25 |
| GABRG2 | 1 | 0.00801239 | 1.48 (1.11, 1.97) | 7.90% | 1.01 | 1.25 |
| GFPT1 | 1 | 0.006343975 | 1.56 (1.13, 2.14) | 7.00% | 1.01 | 1.26 |
| GGT8P | 1 | 0.00992679 | 1.62 (1.12, 2.33) | 8.80% | 1.01 | 1.24 |
| GKN1 | 1 | 0.007050806 | 1.47 (1.11, 1.95) | 7.30% | 1.01 | 1.25 |
| GLRX5 | -1 | 0.008313892 | 0.65 (0.47, 0.89) | 8.00% | 1.01 | 1.27 |
| GPR68 | -1 | 0.009262259 | 0.62 (0.43, 0.89) | 8.50% | 1.01 | 1.27 |
| GPR68 | -1 | 0.009262259 | 0.62 (0.43, 0.89) | 8.50% | 1.01 | 1.27 |
| GPS2 | -1 | 0.007595274 | 0.66 (0.49, 0.90) | 7.70% | 1.01 | 1.27 |
| GREB1L | -1 | 0.007839939 | 0.62 (0.43, 0.88) | 7.80% | 1.01 | 1.27 |
| GRPEL1 | -1 | 0.006700841 | 0.62 (0.44, 0.88) | 7.10% | 1.01 | 1.28 |
| GSTM4 | -1 | 0.005934056 | 0.71 (0.56, 0.91) | 6.80% | 1.01 | 1.26 |
| GSTM4 | -1 | 0.005934056 | 0.71 (0.56, 0.91) | 6.80% | 1.01 | 1.26 |
| GSTM4 | -1 | 0.005934056 | 0.71 (0.56, 0.91) | 6.80% | 1.01 | 1.26 |
| GTF21RD2 | -1 | 0.006754304 | 0.60 (0.41, 0.87) | 7.20% | 1.01 | 1.28 |
| HCN1 | -1 | 0.009201866 | 0.58 (0.38, 0.87) | 8.50% | 1.01 | 1.26 |
| HEATR6 | 1 | 0.009234691 | 1.43 (1.09, 1.87) | 8.50% | 1.01 | 1.24 |
| HEBP1 | -1 | 0.007381555 | 0.55 (0.35, 0.85) | 7.60% | 1.01 | 1.27 |
| HEBP2 | 1 | 0.009915175 | 1.57 (1.11, 2.21) | 8.80% | 1.01 | 1.25 |
| HERPUD1 | 1 | 0.008338896 | 1.48 (1.11, 1.98) | 8.00% | 1.01 | 1.25 |
| HERPUD1 | 1 | 0.008338896 | 1.48 (1.11, 1.98) | 8.00% | 1.01 | 1.25 |
| HERPUD1 | 1 | 0.008338896 | 1.48 (1.11, 1.98) | 8.00% | 1.01 | 1.25 |
| HEXA | -1 | 0.006684966 | 0.59 (0.40, 0.86) | 7.10% | 1.01 | 1.28 |
| HNRNPA2B1 | 1 | 0.006193844 | 1.35 (1.09, 1.68) | 6.90% | 1.01 | 1.24 |
| HNRNPA2B1 | 1 | 0.006193844 | 1.35 (1.09, 1.68) | 6.90% | 1.01 | 1.24 |
| HSPA1L | -1 | 0.009960713 | 0.66 (0.48, 0.91) | 8.80% | 1.01 | 1.26 |
| IFT27 | -1 | 0.008743864 | 0.65 (0.47, 0.90) | 8.20% | 1.01 | 1.27 |
| IFT27 | -1 | 0.008743864 | 0.65 (0.47, 0.90) | 8.20% | 1.01 | 1.27 |
| IFT27 | -1 | 0.008743864 | 0.65 (0.47, 0.90) | 8.20% | 1.01 | 1.27 |
| IFT27 | -1 | 0.008743864 | 0.65 (0.47, 0.90) | 8.20% | 1.01 | 1.27 |
| IGF2R | 1 | 0.009756773 | 1.63 (1.13, 2.37) | 8.70% | 1.01 | 1.24 |
| IL1A | 1 | 0.009440424 | 1.69 (1.14, 2.52) | 8.50% | 1.01 | 1.24 |
| IL20RB | 1 | 0.006998603 | 1.58 (1.13, 2.21) | 7.30% | 1.01 | 1.26 |
| IQCC | -1 | 0.006678534 | 0.66 (0.48, 0.89) | 7.10% | 1.01 | 1.28 |
| IQCC | -1 | 0.006678534 | 0.66 (0.48, 0.89) | 7.10% | 1.01 | 1.28 |
| IQCF5 | 1 | 0.007048357 | 1.29 (1.07, 1.55) | 7.30% | 1.01 | 1.21 |
| IQCG | 1 | 0.00660325 | 1.25 (1.06, 1.48) | 7.10% | 1.01 | 1.2 |
| IQCG | 1 | 0.00660325 | 1.25 (1.06, 1.48) | 7.10% | 1.01 | 1.2 |
| ITCH | 1 | 0.007636272 | 1.69 (1.15, 2.47) | 7.70% | 1.01 | 1.25 |
| ITFG1 | 1 | 0.006426544 | 1.46 (1.11, 1.91) | 7.10% | 1.01 | 1.26 |
| JHDMID | 1 | 0.006479536 | 1.57 (1.14, 2.18) | 7.10% | 1.01 | 1.26 |
| KCNK13 | -1 | 0.007077097 | 0.52 (0.32, 0.84) | 7.40% | 1.01 | 1.26 |
| KCNMB3 | -1 | 0.006827703 | 0.78 (0.65, 0.93) | 7.20% | 1.01 | 1.22 |
| KCNMB3 | -1 | 0.006827703 | 0.78 (0.65, 0.93) | 7.20% | 1.01 | 1.22 |
| KCNMB3 | -1 | 0.006827703 | 0.78 (0.65, 0.93) | 7.20% | 1.01 | 1.22 |
| KCNMB3 | -1 | 0.006827703 | 0.78 (0.65, 0.93) | 7.20% | 1.01 | 1.22 |
| KCNMB3 | -1 | 0.006827703 | 0.78 (0.65, 0.93) | 7.20% | 1.01 | 1.22 |
| KCNMB3 | -1 | 0.006827703 | 0.78 (0.65, 0.93) | 7.20% | 1.01 | 1.22 |
| KDM4D | -1 | 0.008522948 | 0.61 (0.43, 0.88) | 8.10% | 1.01 | 1.27 |
| KIAA0753 | -1 | 0.007526058 | 0.56 (0.36, 0.86) | 7.70% | 1.01 | 1.27 |
| KIAA1024 | 1 | 0.006800873 | 1.56 (1.13, 2.16) | 7.20% | 1.01 | 1.26 |
| KIF13B | -1 | 0.010103096 | 0.58 (0.39, 0.88) | 8.90% | 1.01 | 1.26 |
| KIF20B | 1 | 0.006596402 | 1.65 (1.15, 2.36) | 7.10% | 1.01 | 1.26 |
| KITLG | -1 | 0.00932099 | 0.58 (0.39, 0.87) | 8.50% | 1.01 | 1.26 |
| KITLG | -1 | 0.00932099 | 0.58 (0.39, 0.87) | 8.50% | 1.01 | 1.26 |
| KLHDC1 | -1 | 0.006587563 | 0.63 (0.45, 0.88) | 7.10% | 1.01 | 1.28 |
| KRTCAP2 | 1 | 0.009481883 | 1.58 (1.12, 2.23) | 8.60% | 1.01 | 1.25 |
| LCN2 | 1 | 0.007623799 | 1.54 (1.12, 2.13) | 7.70% | 1.01 | 1.26 |
| LGSN | 1 | 0.00714657 | 1.52 (1.12, 2.07) | 7.40% | 1.01 | 1.26 |
| LGSN | 1 | 0.00714657 | 1.52 (1.12, 2.07) | 7.40% | 1.01 | 1.26 |
| LHX1 | 1 | 0.007831619 | 1.48 (1.11, 1.98) | 7.80% | 1.01 | 1.25 |
| LIMA1 | -1 | 0.008443232 | 0.60 (0.42, 0.88) | 8.10% | 1.01 | 1.27 |
| LIMA1 | -1 | 0.008443232 | 0.60 (0.42, 0.88) | 8.10% | 1.01 | 1.27 |
| LIMA1 | -1 | 0.008443232 | 0.60 (0.42, 0.88) | 8.10% | 1.01 | 1.27 |
| LMAN2L | -1 | 0.009248927 | 0.63 (0.45, 0.89) | 8.50% | 1.01 | 1.27 |
| LMAN2L | -1 | 0.009248927 | 0.63 (0.45, 0.89) | 8.50% | 1.01 | 1.27 |
| LMAN2L | -1 | 0.009248927 | 0.63 (0.45, 0.89) | 8.50% | 1.01 | 1.27 |
| LMAN2L | -1 | 0.009248927 | 0.63 (0.45, 0.89) | 8.50% | 1.01 | 1.27 |
| LMAN2L | -1 | 0.009248927 | 0.63 (0.45, 0.89) | 8.50% | 1.01 | 1.27 |
| LMAN2L | -1 | 0.009248927 | 0.63 (0.45, 0.89) | 8.50% | 1.01 | 1.27 |
| LOC100188947 | -1 | 0.00851099 | 0.61 (0.42, 0.88) | 8.10% | 1.01 | 1.27 |
| LOC100288842 | -1 | 0.00809771 | 0.63 (0.45, 0.89) | 7.90% | 1.01 | 1.27 |
| LOC100379224 | -1 | 0.009205722 | 0.64 (0.46, 0.90) | 8.50% | 1.01 | 1.27 |
| LOC338799 | -1 | 0.007972298 | 0.55 (0.35, 0.86) | 7.90% | 1.01 | 1.27 |
| LOC440335 | -1 | 0.00981157 | 0.63 (0.44, 0.89) | 8.70% | 1.01 | 1.26 |
| LOC440335 | -1 | 0.00981157 | 0.63 (0.44, 0.89) | 8.70% | 1.01 | 1.26 |
| LOC440335 | -1 | 0.00981157 | 0.63 (0.44, 0.89) | 8.70% | 1.01 | 1.26 |
| LOC57653 | -1 | 0.009455291 | 0.66 (0.48, 0.90) | 8.60% | 1.01 | 1.26 |
| LOC57653 | -1 | 0.009455291 | 0.66 (0.48, 0.90) | 8.60% | 1.01 | 1.26 |
| LOC57653 | -1 | 0.009455291 | 0.66 (0.48, 0.90) | 8.60% | 1.01 | 1.26 |
| LOC595101 | -1 | 0.006518385 | 0.58 (0.39, 0.86) | 7.10% | 1.01 | 1.28 |
| LOC643037 | 1 | 0.009129291 | 1.46 (1.10, 1.93) | 8.40% | 1.01 | 1.24 |
| LOC729375 | -1 | 0.009550732 | 0.65 (0.47, 0.90) | 8.60% | 1.01 | 1.26 |
| LOC729668 | 1 | 0.008925516 | 1.54 (1.11, 2.12) | 8.30% | 1.01 | 1.25 |
| LRPPRC | 1 | 0.009760881 | 1.55 (1.11, 2.17) | 8.70% | 1.01 | 1.25 |
| LRRC28 | 1 | 0.009655452 | 1.58 (1.12, 2.23) | 8.60% | 1.01 | 1.25 |
| LRRC42 | -1 | 0.006569663 | 0.60 (0.42, 0.87) | 7.10% | 1.01 | 1.28 |
| LRRC4C | -1 | 0.007662013 | 0.56 (0.37, 0.86) | 7.70% | 1.01 | 1.27 |
| LSS | 1 | 0.00963785 | 1.74 (1.14, 2.65) | 8.60% | 1.01 | 1.24 |
| LSS | 1 | 0.00963785 | 1.74 (1.14, 2.65) | 8.60% | 1.01 | 1.24 |
| LSS | 1 | 0.00963785 | 1.74 (1.14, 2.65) | 8.60% | 1.01 | 1.24 |
| LSS | 1 | 0.00963785 | 1.74 (1.14, 2.65) | 8.60% | 1.01 | 1.24 |
| LYPD6 | -1 | 0.006710307 | 0.68 (0.52, 0.90) | 7.20% | 1.01 | 1.27 |
| LYPD6 | -1 | 0.006710307 | 0.68 (0.52, 0.90) | 7.20% | 1.01 | 1.27 |
| LYZL6 | 1 | 0.008275122 | 1.50 (1.11, 2.02) | 8.00% | 1.01 | 1.25 |
| LYZL6 | 1 | 0.008275122 | 1.50 (1.11, 2.02) | 8.00% | 1.01 | 1.25 |
| LZTFL1 | -1 | 0.009526935 | 0.65 (0.47, 0.90) | 8.60% | 1.01 | 1.26 |
| MAN2B2 | -1 | 0.008606344 | 0.61 (0.42, 0.88) | 8.10% | 1.01 | 1.27 |
| MAP2K7 | -1 | 0.006674321 | 0.59 (0.41, 0.86) | 7.10% | 1.01 | 1.28 |
| MAPK13 | -1 | 0.007291857 | 0.66 (0.48, 0.89) | 7.50% | 1.01 | 1.27 |
| MAT2A | 1 | 0.009104483 | 1.60 (1.12, 2.27) | 8.40% | 1.01 | 1.25 |
| MCF2 | 1 | 0.007384553 | 1.58 (1.13, 2.20) | 7.60% | 1.01 | 1.26 |
| MCF2 | 1 | 0.007384553 | 1.58 (1.13, 2.20) | 7.60% | 1.01 | 1.26 |
| MCF2 | 1 | 0.007384553 | 1.58 (1.13, 2.20) | 7.60% | 1.01 | 1.26 |
| MCF2 | 1 | 0.007384553 | 1.58 (1.13, 2.20) | 7.60% | 1.01 | 1.26 |
| MCF2 | 1 | 0.007384553 | 1.58 (1.13, 2.20) | 7.60% | 1.01 | 1.26 |
| MCF2 | 1 | 0.007384553 | 1.58 (1.13, 2.20) | 7.60% | 1.01 | 1.26 |
| MCM10 | 1 | 0.008326599 | 1.78 (1.16, 2.73) | 8.00% | 1.01 | 1.24 |
| MCM10 | 1 | 0.008326599 | 1.78 (1.16, 2.73) | 8.00% | 1.01 | 1.24 |
| ME1 | 1 | 0.00963294 | 1.57 (1.12, 2.21) | 8.60% | 1.01 | 1.25 |
| MGC87042 | 1 | 0.009587863 | 1.43 (1.09, 1.88) | 8.60% | 1.01 | 1.24 |
| MGC87042 | 1 | 0.009587863 | 1.43 (1.09, 1.88) | 8.60% | 1.01 | 1.24 |
| MGRN1 | -1 | 0.008635109 | 0.54 (0.34, 0.86) | 8.10% | 1.01 | 1.26 |
| MGRN1 | -1 | 0.008635109 | 0.54 (0.34, 0.86) | 8.10% | 1.01 | 1.26 |
| MGRN1 | -1 | 0.008635109 | 0.54 (0.34, 0.86) | 8.10% | 1.01 | 1.26 |
| MGRN1 | -1 | 0.008635109 | 0.54 (0.34, 0.86) | 8.10% | 1.01 | 1.26 |
| MINK1 | -1 | 0.0100436 | 0.66 (0.48, 0.91) | 8.80% | 1.01 | 1.26 |
| MINK1 | -1 | 0.0100436 | 0.66 (0.48, 0.91) | 8.80% | 1.01 | 1.26 |
| MINK1 | -1 | 0.0100436 | 0.66 (0.48, 0.91) | 8.80% | 1.01 | 1.26 |
| MINK1 | -1 | 0.0100436 | 0.66 (0.48, 0.91) | 8.80% | 1.01 | 1.26 |
| MLLT1 | -1 | 0.006613376 | 0.57 (0.38, 0.86) | 7.10% | 1.01 | 1.28 |
| MR1 | -1 | 0.008382286 | 0.58 (0.39, 0.87) | 8.10% | 1.01 | 1.27 |
| MR1 | -1 | 0.008382286 | 0.58 (0.39, 0.87) | 8.10% | 1.01 | 1.27 |
| MR1 | -1 | 0.008382286 | 0.58 (0.39, 0.87) | 8.10% | 1.01 | 1.27 |
| MR1 | -1 | 0.008382286 | 0.58 (0.39, 0.87) | 8.10% | 1.01 | 1.27 |
| MST4 | 1 | 0.010303281 | 1.70 (1.13, 2.54) | 8.90% | 1.01 | 1.24 |
| MST4 | 1 | 0.010303281 | 1.70 (1.13, 2.54) | 8.90% | 1.01 | 1.24 |
| MST4 | 1 | 0.010303281 | 1.70 (1.13, 2.54) | 8.90% | 1.01 | 1.24 |
| MTMR9LP | -1 | 0.007633717 | 0.60 (0.41, 0.87) | 7.70% | 1.01 | 1.27 |
| MTRF1 | -1 | 0.008816635 | 0.67 (0.50, 0.91) | 8.20% | 1.01 | 1.26 |
| MTRR | 1 | 0.007604837 | 1.45 (1.10, 1.90) | 7.70% | 1.01 | 1.25 |
| MTRR | 1 | 0.007604837 | 1.45 (1.10, 1.90) | 7.70% | 1.01 | 1.25 |
| MVD | 1 | 0.008760469 | 1.53 (1.11, 2.09) | 8.20% | 1.01 | 1.25 |
| MYBPHL | -1 | 0.006565434 | 0.69 (0.53, 0.90) | 7.10% | 1.01 | 1.27 |
| MYH3 | -1 | 0.009625687 | 0.59 (0.40, 0.88) | 8.60% | 1.01 | 1.26 |
| MYO1F | -1 | 0.007103603 | 0.64 (0.47, 0.89) | 7.40% | 1.01 | 1.27 |
| MZT2B | -1 | 0.009962642 | 0.61 (0.41, 0.89) | 8.80% | 1.01 | 1.26 |
| NAGPA | -1 | 0.009376889 | 0.65 (0.47, 0.90) | 8.50% | 1.01 | 1.26 |
| NAT15 | -1 | 0.006663053 | 0.56 (0.37, 0.85) | 7.10% | 1.01 | 1.28 |
| NAT15 | -1 | 0.006663053 | 0.56 (0.37, 0.85) | 7.10% | 1.01 | 1.28 |
| NAT15 | -1 | 0.006663053 | 0.56 (0.37, 0.85) | 7.10% | 1.01 | 1.28 |
| NCAPH | 1 | 0.008056556 | 1.81 (1.17, 2.80) | 7.90% | 1.01 | 1.24 |
| NCOA3 | 1 | 0.007667051 | 1.43 (1.10, 1.86) | 7.70% | 1.01 | 1.25 |
| NCOA3 | 1 | 0.007667051 | 1.43 (1.10, 1.86) | 7.70% | 1.01 | 1.25 |
| NCOA3 | 1 | 0.007667051 | 1.43 (1.10, 1.86) | 7.70% | 1.01 | 1.25 |
| NCOA3 | 1 | 0.007667051 | 1.43 (1.10, 1.86) | 7.70% | 1.01 | 1.25 |
| NCRNA00160 | -1 | 0.008094623 | 0.64 (0.45, 0.89) | 7.90% | 1.01 | 1.27 |
| NCRNA00200 | 1 | 0.005171237 | 1.32 (1.09, 1.61) | 6.30% | 1.01 | 1.23 |
| NDUFS6 | 1 | 0.00974941 | 1.54 (1.11, 2.15) | 8.70% | 1.01 | 1.25 |
| NECAP1 | -1 | 0.007788839 | 0.63 (0.45, 0.89) | 7.80% | 1.01 | 1.27 |
| NECAP1 | -1 | 0.007788839 | 0.63 (0.45, 0.89) | 7.80% | 1.01 | 1.27 |
| NEK10 | -1 | 0.008430987 | 0.59 (0.39, 0.87) | 8.10% | 1.01 | 1.27 |
| NFIB | 1 | 0.00819163 | 1.68 (1.14, 2.46) | 8.00% | 1.01 | 1.25 |
| NFIB | 1 | 0.00819163 | 1.68 (1.14, 2.46) | 8.00% | 1.01 | 1.25 |
| NFIB | 1 | 0.00819163 | 1.68 (1.14, 2.46) | 8.00% | 1.01 | 1.25 |
| NME2 | 1 | 0.008082262 | 1.49 (1.11, 2.01) | 7.90% | 1.01 | 1.25 |
| NME2 | 1 | 0.008082262 | 1.49 (1.11, 2.01) | 7.90% | 1.01 | 1.25 |
| NME2 | 1 | 0.008082262 | 1.49 (1.11, 2.01) | 7.90% | 1.01 | 1.25 |
| NME2 | 1 | 0.008082262 | 1.49 (1.11, 2.01) | 7.90% | 1.01 | 1.25 |
| NME2 | 1 | 0.008082262 | 1.49 (1.11, 2.01) | 7.90% | 1.01 | 1.25 |
| NOL11 | 1 | 0.00717401 | 1.48 (1.11, 1.98) | 7.40% | 1.01 | 1.25 |
| NOMO2 | -1 | 0.009847368 | 0.70 (0.54, 0.92) | 8.70% | 1.01 | 1.25 |
| NOMO2 | -1 | 0.009847368 | 0.70 (0.54, 0.92) | 8.70% | 1.01 | 1.25 |
| NOSTRIN | -1 | 0.008395695 | 0.61 (0.43, 0.88) | 8.10% | 1.01 | 1.27 |
| NOSTRIN | -1 | 0.008395695 | 0.61 (0.43, 0.88) | 8.10% | 1.01 | 1.27 |
| NOSTRIN | -1 | 0.008395695 | 0.61 (0.43, 0.88) | 8.10% | 1.01 | 1.27 |
| NOSTRIN | -1 | 0.008395695 | 0.61 (0.43, 0.88) | 8.10% | 1.01 | 1.27 |
| NXNL2 | -1 | 0.008196179 | 0.65 (0.47, 0.89) | 8.00% | 1.01 | 1.27 |
| NXNL2 | -1 | 0.008196179 | 0.65 (0.47, 0.89) | 8.00% | 1.01 | 1.27 |
| NXPH4 | 1 | 0.009631141 | 1.73 (1.14, 2.63) | 8.60% | 1.01 | 1.24 |
| OXR1 | 1 | 0.009643083 | 1.53 (1.11, 2.10) | 8.60% | 1.01 | 1.25 |
| OXR1 | 1 | 0.009643083 | 1.53 (1.11, 2.10) | 8.60% | 1.01 | 1.25 |
| OXR1 | 1 | 0.009643083 | 1.53 (1.11, 2.10) | 8.60% | 1.01 | 1.25 |
| OXR1 | 1 | 0.009643083 | 1.53 (1.11, 2.10) | 8.60% | 1.01 | 1.25 |
| OXR1 | 1 | 0.009643083 | 1.53 (1.11, 2.10) | 8.60% | 1.01 | 1.25 |
| OXR1 | 1 | 0.009643083 | 1.53 (1.11, 2.10) | 8.60% | 1.01 | 1.25 |
| PAQR7 | -1 | 0.00769964 | 0.61 (0.43, 0.88) | 7.70% | 1.01 | 1.27 |
| PCDHGA6 | 1 | 0.008575643 | 1.59 (1.12, 2.24) | 8.10% | 1.01 | 1.25 |
| PCDHGA7 | 1 | 0.010164903 | 1.80 (1.15, 2.83) | 8.90% | 1.01 | 1.23 |
| PCDHGB4 | 1 | 0.008741639 | 1.56 (1.12, 2.18) | 8.20% | 1.01 | 1.25 |
| PDIA6 | 1 | 0.006612815 | 1.66 (1.15, 2.39) | 7.10% | 1.01 | 1.26 |
| PECI | -1 | 0.008942033 | 0.71 (0.55, 0.92) | 8.30% | 1.01 | 1.25 |
| PECI | -1 | 0.008942033 | 0.71 (0.55, 0.92) | 8.30% | 1.01 | 1.25 |
| PECI | -1 | 0.008942033 | 0.71 (0.55, 0.92) | 8.30% | 1.01 | 1.25 |
| PECI | -1 | 0.008942033 | 0.71 (0.55, 0.92) | 8.30% | 1.01 | 1.25 |
| PGA3 | 1 | 0.007689689 | 1.29 (1.07, 1.55) | 7.70% | 1.01 | 1.21 |
| PHF11 | -1 | 0.008193688 | 0.62 (0.43, 0.88) | 8.00% | 1.01 | 1.27 |
| PHF11 | -1 | 0.008193688 | 0.62 (0.43, 0.88) | 8.00% | 1.01 | 1.27 |
| PHTF1 | -1 | 0.007951372 | 0.63 (0.44, 0.88) | 7.90% | 1.01 | 1.27 |
| PIAS4 | -1 | 0.008102516 | 0.62 (0.44, 0.88) | 7.90% | 1.01 | 1.27 |
| PITX3 | -1 | 0.007254858 | 0.64 (0.47, 0.89) | 7.50% | 1.01 | 1.27 |
| PLEKHG1 | 1 | 0.00933219 | 1.67 (1.13, 2.45) | 8.50% | 1.01 | 1.25 |
| POLR3K | -1 | 0.00967405 | 0.65 (0.47, 0.90) | 8.60% | 1.01 | 1.26 |
| POU6F1 | -1 | 0.007157717 | 0.65 (0.48, 0.89) | 7.40% | 1.01 | 1.27 |
| POU6F1 | -1 | 0.007157717 | 0.65 (0.48, 0.89) | 7.40% | 1.01 | 1.27 |
| PPP6R2 | 1 | 0.006788571 | 1.38 (1.09, 1.75) | 7.20% | 1.01 | 1.24 |
| PRDX4 | 1 | 0.00907468 | 1.58 (1.12, 2.23) | 8.40% | 1.01 | 1.25 |
| PRELID2 | -1 | 0.007576831 | 0.63 (0.45, 0.88) | 7.70% | 1.01 | 1.27 |
| PRELID2 | -1 | 0.007576831 | 0.63 (0.45, 0.88) | 7.70% | 1.01 | 1.27 |
| PRELID2 | -1 | 0.007576831 | 0.63 (0.45, 0.88) | 7.70% | 1.01 | 1.27 |
| PSME4 | 1 | 0.008102155 | 1.51 (1.11, 2.06) | 7.90% | 1.01 | 1.25 |
| PTGER3 | -1 | 0.008081201 | 0.58 (0.39, 0.87) | 7.90% | 1.01 | 1.27 |
| PTGER3 | -1 | 0.008081201 | 0.58 (0.39, 0.87) | 7.90% | 1.01 | 1.27 |
| PTGER3 | -1 | 0.008081201 | 0.58 (0.39, 0.87) | 7.90% | 1.01 | 1.27 |
| PTGER3 | -1 | 0.008081201 | 0.58 (0.39, 0.87) | 7.90% | 1.01 | 1.27 |
| PTGER3 | -1 | 0.008081201 | 0.58 (0.39, 0.87) | 7.90% | 1.01 | 1.27 |
| PTGER3 | -1 | 0.008081201 | 0.58 (0.39, 0.87) | 7.90% | 1.01 | 1.27 |
| PTGER3 | -1 | 0.008081201 | 0.58 (0.39, 0.87) | 7.90% | 1.01 | 1.27 |
| PTGER3 | -1 | 0.008081201 | 0.58 (0.39, 0.87) | 7.90% | 1.01 | 1.27 |
| PTGER3 | -1 | 0.008081201 | 0.58 (0.39, 0.87) | 7.90% | 1.01 | 1.27 |
| PTGER3 | -1 | 0.008081201 | 0.58 (0.39, 0.87) | 7.90% | 1.01 | 1.27 |
| PYGO2 | -1 | 0.007468171 | 0.71 (0.55, 0.91) | 7.60% | 1.01 | 1.26 |
| RABEP1 | -1 | 0.008329941 | 0.57 (0.37, 0.86) | 8.00% | 1.01 | 1.27 |
| RABEP1 | -1 | 0.008329941 | 0.57 (0.37, 0.86) | 8.00% | 1.01 | 1.27 |
| RABGAP1L | 1 | 0.006232014 | 1.54 (1.13, 2.10) | 6.90% | 1.01 | 1.26 |
| RABGAP1L | 1 | 0.006232014 | 1.54 (1.13, 2.10) | 6.90% | 1.01 | 1.26 |
| RAD9A | -1 | 0.009330391 | 0.65 (0.46, 0.90) | 8.50% | 1.01 | 1.27 |
| RAGE | -1 | 0.006700386 | 0.61 (0.42, 0.87) | 7.10% | 1.01 | 1.28 |
| RAPGEF2 | 1 | 0.008912008 | 1.80 (1.16, 2.79) | 8.30% | 1.01 | 1.24 |
| RASGEF1B | 1 | 0.006561595 | 1.50 (1.12, 2.02) | 7.10% | 1.01 | 1.26 |
| RCBTB1 | -1 | 0.007874485 | 0.60 (0.41, 0.87) | 7.80% | 1.01 | 1.27 |
| RFWD3 | 1 | 0.007383613 | 1.50 (1.12, 2.03) | 7.60% | 1.01 | 1.26 |
| RGR | 1 | 0.005959146 | 1.46 (1.12, 1.92) | 6.80% | 1.01 | 1.26 |
| RGR | 1 | 0.005959146 | 1.46 (1.12, 1.92) | 6.80% | 1.01 | 1.26 |
| RGR | 1 | 0.005959146 | 1.46 (1.12, 1.92) | 6.80% | 1.01 | 1.26 |
| RGS14 | -1 | 0.009804379 | 0.65 (0.46, 0.90) | 8.70% | 1.01 | 1.26 |
| RHBDD3 | 1 | 0.009281818 | 1.97 (1.18, 3.28) | 8.50% | 1.01 | 1.22 |
| RIPPLY1 | 1 | 0.010067371 | 1.60 (1.12, 2.30) | 8.80% | 1.01 | 1.24 |
| RIPPLY1 | 1 | 0.010067371 | 1.60 (1.12, 2.30) | 8.80% | 1.01 | 1.24 |
| RNASE11 | 1 | 0.007561084 | 1.36 (1.09, 1.71) | 7.70% | 1.01 | 1.24 |
| RNF125 | -1 | 0.006986535 | 0.65 (0.48, 0.89) | 7.30% | 1.01 | 1.27 |
| RNF180 | -1 | 0.00949659 | 0.64 (0.46, 0.90) | 8.60% | 1.01 | 1.26 |
| RNF180 | -1 | 0.00949659 | 0.64 (0.46, 0.90) | 8.60% | 1.01 | 1.26 |
| RNF214 | -1 | 0.005949891 | 0.69 (0.53, 0.90) | 6.80% | 1.01 | 1.27 |
| RNF214 | -1 | 0.005949891 | 0.69 (0.53, 0.90) | 6.80% | 1.01 | 1.27 |
| RNF216L | 1 | 0.006855698 | 1.79 (1.17, 2.73) | 7.20% | 1.01 | 1.25 |
| RNF216L | 1 | 0.006855698 | 1.79 (1.17, 2.73) | 7.20% | 1.01 | 1.25 |
| RNF216L | 1 | 0.006855698 | 1.79 (1.17, 2.73) | 7.20% | 1.01 | 1.25 |
| RPH3A | 1 | 0.007850074 | 1.42 (1.10, 1.85) | 7.80% | 1.01 | 1.25 |
| RPH3A | 1 | 0.007850074 | 1.42 (1.10, 1.85) | 7.80% | 1.01 | 1.25 |
| RPL37 | 1 | 0.008922524 | 2.00 (1.19, 3.36) | 8.30% | 1.01 | 1.22 |
| RPL3L | 1 | 0.006219667 | 1.38 (1.10, 1.74) | 6.90% | 1.01 | 1.24 |
| RPS27L | -1 | 0.009737503 | 0.63 (0.45, 0.90) | 8.70% | 1.01 | 1.26 |
| RPS8 | 1 | 0.008439438 | 1.88 (1.18, 3.01) | 8.10% | 1.01 | 1.24 |
| RPUSD3 | -1 | 0.008911008 | 0.61 (0.42, 0.88) | 8.30% | 1.01 | 1.27 |
| RPUSD3 | -1 | 0.008911008 | 0.61 (0.42, 0.88) | 8.30% | 1.01 | 1.27 |
| RUSC2 | -1 | 0.009594773 | 0.66 (0.49, 0.91) | 8.60% | 1.01 | 1.26 |
| RUSC2 | -1 | 0.009594773 | 0.66 (0.49, 0.91) | 8.60% | 1.01 | 1.26 |
| S100A8 | 1 | 0.008623961 | 1.47 (1.10, 1.96) | 8.10% | 1.01 | 1.25 |
| SCAPER | 1 | 0.009790827 | 1.64 (1.13, 2.38) | 8.70% | 1.01 | 1.24 |
| SCAPER | 1 | 0.009790827 | 1.64 (1.13, 2.38) | 8.70% | 1.01 | 1.24 |
| SCFD2 | 1 | 0.009357868 | 1.56 (1.12, 2.18) | 8.50% | 1.01 | 1.25 |
| SCUBE3 | -1 | 0.00648237 | 0.65 (0.47, 0.89) | 7.10% | 1.01 | 1.28 |
| SEC24D | 1 | 0.007226299 | 1.76 (1.17, 2.67) | 7.50% | 1.01 | 1.25 |
| SH3RF2 | -1 | 0.008949805 | 0.59 (0.40, 0.88) | 8.30% | 1.01 | 1.27 |
| SIX2 | 1 | 0.009940046 | 1.43 (1.09, 1.89) | 8.80% | 1.01 | 1.24 |
| SLC14A2 | -1 | 0.00688137 | 0.49 (0.29, 0.82) | 7.20% | 1.01 | 1.26 |
| SLC25A13 | 1 | 0.008049299 | 1.62 (1.13, 2.32) | 7.90% | 1.01 | 1.25 |
| SLC25A13 | 1 | 0.008049299 | 1.62 (1.13, 2.32) | 7.90% | 1.01 | 1.25 |
| SLC25A13 | 1 | 0.008049299 | 1.62 (1.13, 2.32) | 7.90% | 1.01 | 1.25 |
| SLC25A24 | -1 | 0.006618756 | 0.59 (0.40, 0.86) | 7.10% | 1.01 | 1.28 |
| SLC25A24 | -1 | 0.006618756 | 0.59 (0.40, 0.86) | 7.10% | 1.01 | 1.28 |
| SLC6A15 | 1 | 0.007451471 | 1.56 (1.13, 2.17) | 7.60% | 1.01 | 1.26 |
| SLC6A15 | 1 | 0.007451471 | 1.56 (1.13, 2.17) | 7.60% | 1.01 | 1.26 |
| SLC6A15 | 1 | 0.007451471 | 1.56 (1.13, 2.17) | 7.60% | 1.01 | 1.26 |
| SLC7A13 | -1 | 0.009306511 | 0.62 (0.43, 0.89) | 8.50% | 1.01 | 1.27 |
| SLURP1 | 1 | 0.008042738 | 1.34 (1.08, 1.65) | 7.90% | 1.01 | 1.23 |
| SPAM1 | 1 | 0.007221968 | 1.55 (1.13, 2.14) | 7.50% | 1.01 | 1.26 |
| SPAM1 | 1 | 0.007221968 | 1.55 (1.13, 2.14) | 7.50% | 1.01 | 1.26 |
| SPAM1 | 1 | 0.007221968 | 1.55 (1.13, 2.14) | 7.50% | 1.01 | 1.26 |
| SPAM1 | 1 | 0.007221968 | 1.55 (1.13, 2.14) | 7.50% | 1.01 | 1.26 |
| SPAM1 | 1 | 0.007221968 | 1.55 (1.13, 2.14) | 7.50% | 1.01 | 1.26 |
| SSBP1 | 1 | 0.008145394 | 1.52 (1.11, 2.07) | 7.90% | 1.01 | 1.25 |
| STAM2 | -1 | 0.007611313 | 0.54 (0.34, 0.85) | 7.70% | 1.01 | 1.27 |
| STARD13 | -1 | 0.007388965 | 0.48 (0.28, 0.82) | 7.60% | 1.01 | 1.25 |
| STARD13 | -1 | 0.007388965 | 0.48 (0.28, 0.82) | 7.60% | 1.01 | 1.25 |
| STARD13 | -1 | 0.007388965 | 0.48 (0.28, 0.82) | 7.60% | 1.01 | 1.25 |
| STC2 | -1 | 0.007653482 | 0.57 (0.38, 0.86) | 7.70% | 1.01 | 1.27 |
| STIL | 1 | 0.008856454 | 1.65 (1.13, 2.41) | 8.30% | 1.01 | 1.25 |
| STIL | 1 | 0.008856454 | 1.65 (1.13, 2.41) | 8.30% | 1.01 | 1.25 |
| STK11 | -1 | 0.00927752 | 0.59 (0.40, 0.88) | 8.50% | 1.01 | 1.26 |
| STK32B | -1 | 0.007770131 | 0.54 (0.34, 0.85) | 7.80% | 1.01 | 1.26 |
| STOX1 | -1 | 0.009373358 | 0.60 (0.41, 0.88) | 8.50% | 1.01 | 1.27 |
| STOX1 | -1 | 0.009373358 | 0.60 (0.41, 0.88) | 8.50% | 1.01 | 1.27 |
| STOX1 | -1 | 0.009373358 | 0.60 (0.41, 0.88) | 8.50% | 1.01 | 1.27 |
| STOX1 | -1 | 0.009373358 | 0.60 (0.41, 0.88) | 8.50% | 1.01 | 1.27 |
| STOX1 | -1 | 0.009373358 | 0.60 (0.41, 0.88) | 8.50% | 1.01 | 1.27 |
| SURF2 | -1 | 0.008281877 | 0.61 (0.43, 0.88) | 8.00% | 1.01 | 1.27 |
| SYCN | 1 | 0.009880133 | 1.46 (1.09, 1.94) | 8.70% | 1.01 | 1.24 |
| TBC1D10B | -1 | 0.007813488 | 0.63 (0.44, 0.88) | 7.80% | 1.01 | 1.27 |
| TCTN1 | -1 | 0.008791552 | 0.63 (0.44, 0.89) | 8.20% | 1.01 | 1.27 |
| TCTN1 | -1 | 0.008791552 | 0.63 (0.44, 0.89) | 8.20% | 1.01 | 1.27 |
| TCTN1 | -1 | 0.008791552 | 0.63 (0.44, 0.89) | 8.20% | 1.01 | 1.27 |
| TCTN1 | -1 | 0.008791552 | 0.63 (0.44, 0.89) | 8.20% | 1.01 | 1.27 |
| TCTN1 | -1 | 0.008791552 | 0.63 (0.44, 0.89) | 8.20% | 1.01 | 1.27 |
| TEX9 | -1 | 0.009763575 | 0.62 (0.43, 0.89) | 8.70% | 1.01 | 1.26 |
| TFG | 1 | 0.007007574 | 1.72 (1.16, 2.54) | 7.30% | 1.01 | 1.26 |
| TFG | 1 | 0.007007574 | 1.72 (1.16, 2.54) | 7.30% | 1.01 | 1.26 |
| TFG | 1 | 0.007007574 | 1.72 (1.16, 2.54) | 7.30% | 1.01 | 1.26 |
| TFG | 1 | 0.007007574 | 1.72 (1.16, 2.54) | 7.30% | 1.01 | 1.26 |
| TLR7 | -1 | 0.007037628 | 0.68 (0.51, 0.90) | 7.30% | 1.01 | 1.27 |
| TM6SF1 | -1 | 0.0077869 | 0.63 (0.44, 0.88) | 7.80% | 1.01 | 1.27 |
| TM6SF1 | -1 | 0.0077869 | 0.63 (0.44, 0.88) | 7.80% | 1.01 | 1.27 |
| TMEM132A | 1 | 0.007152656 | 1.73 (1.16, 2.57) | 7.40% | 1.01 | 1.25 |
| TMEM132A | 1 | 0.007152656 | 1.73 (1.16, 2.57) | 7.40% | 1.01 | 1.25 |
| TMEM140 | 1 | 0.009060643 | 1.48 (1.10, 1.99) | 8.40% | 1.01 | 1.25 |
| TMEM91 | -1 | 0.00674262 | 0.64 (0.46, 0.88) | 7.20% | 1.01 | 1.28 |
| TMEM91 | -1 | 0.00674262 | 0.64 (0.46, 0.88) | 7.20% | 1.01 | 1.28 |
| TMEM91 | -1 | 0.00674262 | 0.64 (0.46, 0.88) | 7.20% | 1.01 | 1.28 |
| TMEM91 | -1 | 0.00674262 | 0.64 (0.46, 0.88) | 7.20% | 1.01 | 1.28 |
| TMEM91 | -1 | 0.00674262 | 0.64 (0.46, 0.88) | 7.20% | 1.01 | 1.28 |
| TMEM91 | -1 | 0.00674262 | 0.64 (0.46, 0.88) | 7.20% | 1.01 | 1.28 |
| TMPRSS13 | 1 | 0.008747326 | 1.84 (1.17, 2.89) | 8.20% | 1.01 | 1.24 |
| TMUB2 | -1 | 0.008171294 | 0.63 (0.45, 0.89) | 8.00% | 1.01 | 1.27 |
| TMUB2 | -1 | 0.008171294 | 0.63 (0.45, 0.89) | 8.00% | 1.01 | 1.27 |
| TMUB2 | -1 | 0.008171294 | 0.63 (0.45, 0.89) | 8.00% | 1.01 | 1.27 |
| TNFRSF21 | 1 | 0.008234578 | 1.48 (1.11, 1.99) | 8.00% | 1.01 | 1.25 |
| TNIP2 | -1 | 0.008424446 | 0.60 (0.41, 0.88) | 8.10% | 1.01 | 1.27 |
| TNIP2 | -1 | 0.008424446 | 0.60 (0.41, 0.88) | 8.10% | 1.01 | 1.27 |
| TOPORS | -1 | 0.009571277 | 0.61 (0.42, 0.89) | 8.60% | 1.01 | 1.26 |
| TOPORS | -1 | 0.009571277 | 0.61 (0.42, 0.89) | 8.60% | 1.01 | 1.26 |
| TPPP | 1 | 0.006317198 | 1.57 (1.14, 2.18) | 7.00% | 1.01 | 1.26 |
| TRAM1 | 1 | 0.010148341 | 1.56 (1.11, 2.20) | 8.90% | 1.01 | 1.24 |
| TRIM35 | -1 | 0.006782048 | 0.59 (0.40, 0.86) | 7.20% | 1.01 | 1.28 |
| TRIM45 | -1 | 0.009288628 | 0.55 (0.36, 0.86) | 8.50% | 1.01 | 1.26 |
| TRIM45 | -1 | 0.009288628 | 0.55 (0.36, 0.86) | 8.50% | 1.01 | 1.26 |
| TRIM66 | -1 | 0.006237676 | 0.63 (0.45, 0.88) | 6.90% | 1.01 | 1.28 |
| TRPC4AP | 1 | 0.007218558 | 1.63 (1.14, 2.32) | 7.50% | 1.01 | 1.26 |
| TRPC4AP | 1 | 0.007218558 | 1.63 (1.14, 2.32) | 7.50% | 1.01 | 1.26 |
| TRPC7 | 1 | 0.010038406 | 1.63 (1.12, 2.36) | 8.80% | 1.01 | 1.24 |
| TRPC7 | 1 | 0.010038406 | 1.63 (1.12, 2.36) | 8.80% | 1.01 | 1.24 |
| TRPC7 | 1 | 0.010038406 | 1.63 (1.12, 2.36) | 8.80% | 1.01 | 1.24 |
| TSC1 | -1 | 0.008687639 | 0.58 (0.39, 0.87) | 8.20% | 1.01 | 1.27 |
| TSC1 | -1 | 0.008687639 | 0.58 (0.39, 0.87) | 8.20% | 1.01 | 1.27 |
| TSC1 | -1 | 0.008687639 | 0.58 (0.39, 0.87) | 8.20% | 1.01 | 1.27 |
| TSGA10 | -1 | 0.009624267 | 0.63 (0.44, 0.89) | 8.60% | 1.01 | 1.26 |
| TSGA10 | -1 | 0.009624267 | 0.63 (0.44, 0.89) | 8.60% | 1.01 | 1.26 |
| TSPYL2 | -1 | 0.008608205 | 0.61 (0.42, 0.88) | 8.10% | 1.01 | 1.27 |
| TTC12 | -1 | 0.00902816 | 0.53 (0.33, 0.86) | 8.30% | 1.01 | 1.26 |
| TTC31 | -1 | 0.008773497 | 0.65 (0.47, 0.90) | 8.20% | 1.01 | 1.27 |
| TTC31 | -1 | 0.008773497 | 0.65 (0.47, 0.90) | 8.20% | 1.01 | 1.27 |
| TTC8 | -1 | 0.0075677 | 0.64 (0.46, 0.89) | 7.70% | 1.01 | 1.27 |
| TTC8 | -1 | 0.0075677 | 0.64 (0.46, 0.89) | 7.70% | 1.01 | 1.27 |
| TTC8 | -1 | 0.0075677 | 0.64 (0.46, 0.89) | 7.70% | 1.01 | 1.27 |
| UBA7 | -1 | 0.008940825 | 0.61 (0.42, 0.88) | 8.30% | 1.01 | 1.27 |
| UBR1 | 1 | 0.009723514 | 1.35 (1.08, 1.70) | 8.70% | 1.01 | 1.23 |
| UCHL5 | 1 | 0.009364974 | 1.62 (1.13, 2.32) | 8.50% | 1.01 | 1.25 |
| UCHL5 | 1 | 0.009364974 | 1.62 (1.13, 2.32) | 8.50% | 1.01 | 1.25 |
| UCHL5 | 1 | 0.009364974 | 1.62 (1.13, 2.32) | 8.50% | 1.01 | 1.25 |
| UCHL5 | 1 | 0.009364974 | 1.62 (1.13, 2.32) | 8.50% | 1.01 | 1.25 |
| UFD1L | 1 | 0.010119718 | 1.59 (1.12, 2.26) | 8.90% | 1.01 | 1.24 |
| UFD1L | 1 | 0.010119718 | 1.59 (1.12, 2.26) | 8.90% | 1.01 | 1.24 |
| ULBP1 | 1 | 0.008659691 | 1.50 (1.11, 2.03) | 8.20% | 1.01 | 1.25 |
| VDAC2 | 1 | 0.007637438 | 1.47 (1.11, 1.95) | 7.70% | 1.01 | 1.25 |
| VDAC2 | 1 | 0.007637438 | 1.47 (1.11, 1.95) | 7.70% | 1.01 | 1.25 |
| VDAC2 | 1 | 0.007637438 | 1.47 (1.11, 1.95) | 7.70% | 1.01 | 1.25 |
| VDAC2 | 1 | 0.007637438 | 1.47 (1.11, 1.95) | 7.70% | 1.01 | 1.25 |
| VPS13B | 1 | 0.007972288 | 1.58 (1.13, 2.21) | 7.90% | 1.01 | 1.25 |
| VPS13B | 1 | 0.007972288 | 1.58 (1.13, 2.21) | 7.90% | 1.01 | 1.25 |
| VPS13B | 1 | 0.007972288 | 1.58 (1.13, 2.21) | 7.90% | 1.01 | 1.25 |
| VPS13B | 1 | 0.007972288 | 1.58 (1.13, 2.21) | 7.90% | 1.01 | 1.25 |
| VPS24 | -1 | 0.009864489 | 0.69 (0.52, 0.92) | 8.70% | 1.01 | 1.26 |
| VPS24 | -1 | 0.009864489 | 0.69 (0.52, 0.92) | 8.70% | 1.01 | 1.26 |
| VPS24 | -1 | 0.009864489 | 0.69 (0.52, 0.92) | 8.70% | 1.01 | 1.26 |
| VPS24 | -1 | 0.009864489 | 0.69 (0.52, 0.92) | 8.70% | 1.01 | 1.26 |
| VPS37B | 1 | 0.007784747 | 1.54 (1.12, 2.11) | 7.80% | 1.01 | 1.25 |
| WDR67 | 1 | 0.007371765 | 1.70 (1.15, 2.49) | 7.60% | 1.01 | 1.25 |
| WDR67 | 1 | 0.007371765 | 1.70 (1.15, 2.49) | 7.60% | 1.01 | 1.25 |
| WDR75 | -1 | 0.007896105 | 0.49 (0.29, 0.83) | 7.80% | 1.01 | 1.25 |
| WDTC1 | -1 | 0.008530846 | 0.63 (0.45, 0.89) | 8.10% | 1.01 | 1.27 |
| XPOT | 1 | 0.010160158 | 1.58 (1.11, 2.24) | 8.90% | 1.01 | 1.24 |
| YEATS2 | 1 | 0.007348357 | 1.58 (1.13, 2.22) | 7.60% | 1.01 | 1.26 |
| ZC3H18 | 1 | 0.007722884 | 1.66 (1.14, 2.41) | 7.70% | 1.01 | 1.25 |
| ZCWPW1 | -1 | 0.008302062 | 0.57 (0.37, 0.86) | 8.00% | 1.01 | 1.27 |
| ZGPAT | 1 | 0.008586682 | 1.47 (1.10, 1.96) | 8.10% | 1.01 | 1.25 |
| ZGPAT | 1 | 0.008586682 | 1.47 (1.10, 1.96) | 8.10% | 1.01 | 1.25 |
| ZGPAT | 1 | 0.008586682 | 1.47 (1.10, 1.96) | 8.10% | 1.01 | 1.25 |
| ZGPAT | 1 | 0.008586682 | 1.47 (1.10, 1.96) | 8.10% | 1.01 | 1.25 |
| ZGPAT | 1 | 0.008586682 | 1.47 (1.10, 1.96) | 8.10% | 1.01 | 1.25 |
| ZNF189 | -1 | 0.006959976 | 0.58 (0.39, 0.86) | 7.30% | 1.01 | 1.27 |
| ZNF189 | -1 | 0.006959976 | 0.58 (0.39, 0.86) | 7.30% | 1.01 | 1.27 |
| ZNF425 | -1 | 0.009534005 | 0.65 (0.47, 0.90) | 8.60% | 1.01 | 1.26 |
| ZNF490 | -1 | 0.006800138 | 0.57 (0.38, 0.86) | 7.20% | 1.01 | 1.27 |
| ZNF528 | -1 | 0.008402995 | 0.62 (0.43, 0.88) | 8.10% | 1.01 | 1.27 |
| ZNF578 | -1 | 0.00702429 | 0.61 (0.43, 0.88) | 7.30% | 1.01 | 1.28 |
| ZNF695 | 1 | 0.009425745 | 1.87 (1.17, 2.99) | 8.50% | 1.01 | 1.23 |
| ZNF71 | -1 | 0.006488217 | 0.67 (0.50, 0.89) | 7.10% | 1.01 | 1.27 |
| ZNF791 | -1 | 0.006897515 | 0.60 (0.42, 0.87) | 7.30% | 1.01 | 1.28 |
| ZNF813 | -1 | 0.009651252 | 0.69 (0.52, 0.91) | 8.60% | 1.01 | 1.26 |
| ZZEF1 | -1 | 0.007161028 | 0.59 (0.41, 0.87) | 7.40% | 1.01 | 1.28 |
| ABI1 | 1 | 0.011314556 | 1.53 (1.10, 2.12) | 9.40% | 1 | 1.24 |
| ABI1 | 1 | 0.011314556 | 1.53 (1.10, 2.12) | 9.40% | 1 | 1.24 |
| ABI1 | 1 | 0.011314556 | 1.53 (1.10, 2.12) | 9.40% | 1 | 1.24 |
| ABI1 | 1 | 0.011314556 | 1.53 (1.10, 2.12) | 9.40% | 1 | 1.24 |
| ABI1 | 1 | 0.011314556 | 1.53 (1.10, 2.12) | 9.40% | 1 | 1.24 |
| ABI1 | 1 | 0.011314556 | 1.53 (1.10, 2.12) | 9.40% | 1 | 1.24 |
| ABI1 | 1 | 0.011314556 | 1.53 (1.10, 2.12) | 9.40% | 1 | 1.24 |
| ABI1 | 1 | 0.011314556 | 1.53 (1.10, 2.12) | 9.40% | 1 | 1.24 |
| ABI1 | 1 | 0.011314556 | 1.53 (1.10, 2.12) | 9.40% | 1 | 1.24 |
| ABI1 | 1 | 0.011314556 | 1.53 (1.10, 2.12) | 9.40% | 1 | 1.24 |
| ABI1 | 1 | 0.011314556 | 1.53 (1.10, 2.12) | 9.40% | 1 | 1.24 |
| ABI1 | 1 | 0.011314556 | 1.53 (1.10, 2.12) | 9.40% | 1 | 1.24 |
| ADCY4 | -1 | 0.012746837 | 0.60 (0.40, 0.90) | 9.90% | 1 | 1.25 |
| ADCY4 | -1 | 0.012746837 | 0.60 (0.40, 0.90) | 9.90% | 1 | 1.25 |
| ADCY4 | -1 | 0.012746837 | 0.60 (0.40, 0.90) | 9.90% | 1 | 1.25 |
| AGBL2 | -1 | 0.012159989 | 0.69 (0.52, 0.92) | 9.70% | 1 | 1.25 |
| AKAP13 | 1 | 0.010556574 | 1.48 (1.10, 2.01) | 9.00% | 1 | 1.24 |
| AKAP13 | 1 | 0.010556574 | 1.48 (1.10, 2.01) | 9.00% | 1 | 1.24 |
| AKAP13 | 1 | 0.010556574 | 1.48 (1.10, 2.01) | 9.00% | 1 | 1.24 |
| AKR1A1 | 1 | 0.010860471 | 1.41 (1.08, 1.83) | 9.20% | 1 | 1.23 |
| AKR1A1 | 1 | 0.010860471 | 1.41 (1.08, 1.83) | 9.20% | 1 | 1.23 |
| ALDH1B1 | -1 | 0.011726926 | 0.63 (0.44, 0.90) | 9.50% | 1 | 1.26 |
| AMBN | 1 | 0.011422064 | 1.45 (1.09, 1.94) | 9.40% | 1 | 1.24 |
| AMTN | 1 | 0.010260119 | 1.39 (1.08, 1.79) | 8.90% | 1 | 1.23 |
| ANKRD30BP2 | 1 | 0.010303181 | 1.48 (1.10, 2.00) | 8.90% | 1 | 1.24 |
| ARFGAP1 | 1 | 0.011829858 | 1.47 (1.09, 1.99) | 9.60% | 1 | 1.24 |
| ARFGAP1 | 1 | 0.011829858 | 1.47 (1.09, 1.99) | 9.60% | 1 | 1.24 |
| ARMC1 | 1 | 0.01157055 | 1.46 (1.09, 1.95) | 9.50% | 1 | 1.24 |
| ARPP21 | -1 | 0.011971679 | 0.56 (0.35, 0.88) | 9.60% | 1 | 1.25 |
| ARPP21 | -1 | 0.011971679 | 0.56 (0.35, 0.88) | 9.60% | 1 | 1.25 |
| ARPP21 | -1 | 0.011971679 | 0.56 (0.35, 0.88) | 9.60% | 1 | 1.25 |
| ARPP21 | -1 | 0.011971679 | 0.56 (0.35, 0.88) | 9.60% | 1 | 1.25 |
| ARSD | -1 | 0.011023269 | 0.65 (0.47, 0.91) | 9.20% | 1 | 1.26 |
| ARSD | -1 | 0.011023269 | 0.65 (0.47, 0.91) | 9.20% | 1 | 1.26 |
| ATP11B | 1 | 0.011169252 | 1.56 (1.11, 2.20) | 9.30% | 1 | 1.24 |
| BCKDHA | -1 | 0.010291527 | 0.67 (0.49, 0.91) | 8.90% | 1 | 1.26 |
| BCKDHA | -1 | 0.010291527 | 0.67 (0.49, 0.91) | 8.90% | 1 | 1.26 |
| BRIP1 | 1 | 0.012952368 | 1.55 (1.10, 2.19) | 10.00% | 1 | 1.24 |
| C11orf74 | -1 | 0.010770379 | 0.66 (0.48, 0.91) | 9.10% | 1 | 1.26 |
| C14orf23 | 1 | 0.011832502 | 1.39 (1.08, 1.80) | 9.60% | 1 | 1.23 |
| C14orf23 | 1 | 0.011832502 | 1.39 (1.08, 1.80) | 9.60% | 1 | 1.23 |
| C16orf5 | -1 | 0.010242171 | 0.66 (0.48, 0.91) | 8.90% | 1 | 1.26 |
| C16orf5 | -1 | 0.010242171 | 0.66 (0.48, 0.91) | 8.90% | 1 | 1.26 |
| C16orf5 | -1 | 0.010242171 | 0.66 (0.48, 0.91) | 8.90% | 1 | 1.26 |
| C16orf5 | -1 | 0.010242171 | 0.66 (0.48, 0.91) | 8.90% | 1 | 1.26 |
| C17orf97 | -1 | 0.012338083 | 0.60 (0.40, 0.89) | 9.80% | 1 | 1.25 |
| C18orf55 | -1 | 0.011092298 | 0.68 (0.50, 0.91) | 9.30% | 1 | 1.26 |
| Clorf124 | 1 | 0.012304896 | 1.61 (1.11, 2.33) | 9.80% | 1 | 1.24 |
| C1orf124 | 1 | 0.012304896 | 1.61 (1.11, 2.33) | 9.80% | 1 | 1.24 |
| C20orf30 | 1 | 0.011038247 | 1.55 (1.11, 2.17) | 9.20% | 1 | 1.24 |
| C20orf30 | 1 | 0.011038247 | 1.55 (1.11, 2.17) | 9.20% | 1 | 1.24 |
| C20orf30 | 1 | 0.011038247 | 1.55 (1.11, 2.17) | 9.20% | 1 | 1.24 |
| C20orf30 | 1 | 0.011038247 | 1.55 (1.11, 2.17) | 9.20% | 1 | 1.24 |
| C20orf96 | -1 | 0.010956191 | 0.65 (0.46, 0.90) | 9.20% | 1 | 1.26 |
| C22orf26 | -1 | 0.011951339 | 0.68 (0.50, 0.92) | 9.60% | 1 | 1.25 |
| C3orf24 | 1 | 0.011566363 | 1.65 (1.12, 2.44) | 9.50% | 1 | 1.24 |
| C3orf24 | 1 | 0.011566363 | 1.65 (1.12, 2.44) | 9.50% | 1 | 1.24 |
| C4orf21 | 1 | 0.012027146 | 1.57 (1.10, 2.24) | 9.60% | 1 | 1.24 |
| C5orf38 | -1 | 0.012871615 | 0.63 (0.44, 0.91) | 10.00% | 1 | 1.25 |
| C9orf117 | -1 | 0.012155809 | 0.66 (0.48, 0.91) | 9.70% | 1 | 1.25 |
| C9orf68 | -1 | 0.011881699 | 0.64 (0.45, 0.91) | 9.60% | 1 | 1.26 |
| CAPG | -1 | 0.01139945 | 0.64 (0.45, 0.90) | 9.40% | 1 | 1.26 |
| CASP14 | 1 | 0.011468521 | 1.45 (1.09, 1.94) | 9.40% | 1 | 1.24 |
| CBX7 | -1 | 0.010411793 | 0.64 (0.46, 0.90) | 8.90% | 1 | 1.26 |
| CCDC11 | -1 | 0.01124832 | 0.62 (0.42, 0.90) | 9.30% | 1 | 1.26 |
| CD2BP2 | -1 | 0.012203297 | 0.66 (0.48, 0.91) | 9.70% | 1 | 1.25 |
| CD2BP2 | -1 | 0.012203297 | 0.66 (0.48, 0.91) | 9.70% | 1 | 1.25 |
| CDC25C | 1 | 0.012973395 | 2.13 (1.17, 3.88) | 10.00% | 1 | 1.2 |
| CDC25C | 1 | 0.012973395 | 2.13 (1.17, 3.88) | 10.00% | 1 | 1.2 |
| CDCA2 | 1 | 0.01062877 | 1.77 (1.14, 2.73) | 9.00% | 1 | 1.23 |
| CHEK2 | 1 | 0.010238312 | 1.55 (1.11, 2.17) | 8.90% | 1 | 1.24 |
| CHEK2 | 1 | 0.010238312 | 1.55 (1.11, 2.17) | 8.90% | 1 | 1.24 |
| CHEK2 | 1 | 0.010238312 | 1.55 (1.11, 2.17) | 8.90% | 1 | 1.24 |
| CHMP2A | -1 | 0.011849205 | 0.69 (0.52, 0.92) | 9.60% | 1 | 1.25 |
| CHMP2A | -1 | 0.011849205 | 0.69 (0.52, 0.92) | 9.60% | 1 | 1.25 |
| CIZ1 | -1 | 0.011264428 | 0.64 (0.45, 0.90) | 9.30% | 1 | 1.26 |
| CIZ1 | -1 | 0.011264428 | 0.64 (0.45, 0.90) | 9.30% | 1 | 1.26 |
| CIZ1 | -1 | 0.011264428 | 0.64 (0.45, 0.90) | 9.30% | 1 | 1.26 |
| CIZ1 | -1 | 0.011264428 | 0.64 (0.45, 0.90) | 9.30% | 1 | 1.26 |
| CIZ1 | -1 | 0.011264428 | 0.64 (0.45, 0.90) | 9.30% | 1 | 1.26 |
| CLEC6A | 1 | 0.011311521 | 1.54 (1.10, 2.14) | 9.40% | 1 | 1.24 |
| COL6A4P1 | 1 | 0.011152024 | 1.74 (1.13, 2.66) | 9.30% | 1 | 1.23 |
| CPT2 | -1 | 0.011138461 | 0.71 (0.55, 0.93) | 9.30% | 1 | 1.25 |
| CYHR1 | 1 | 0.010619769 | 1.71 (1.13, 2.59) | 9.00% | 1 | 1.24 |
| CYHR1 | 1 | 0.010619769 | 1.71 (1.13, 2.59) | 9.00% | 1 | 1.24 |
| CYHR1 | 1 | 0.010619769 | 1.71 (1.13, 2.59) | 9.00% | 1 | 1.24 |
| CYTH2 | -1 | 0.010385083 | 0.61 (0.41, 0.89) | 8.90% | 1 | 1.26 |
| CYTH2 | -1 | 0.010385083 | 0.61 (0.41, 0.89) | 8.90% | 1 | 1.26 |
| DDX47 | -1 | 0.010917905 | 0.59 (0.40, 0.89) | 9.20% | 1 | 1.26 |
| DDX47 | -1 | 0.010917905 | 0.59 (0.40, 0.89) | 9.20% | 1 | 1.26 |
| DEFB123 | 1 | 0.010229146 | 1.42 (1.09, 1.85) | 8.90% | 1 | 1.24 |
| DIAPH1 | 1 | 0.012626509 | 1.59 (1.10, 2.28) | 9.90% | 1 | 1.24 |
| DIAPH1 | 1 | 0.012626509 | 1.59 (1.10, 2.28) | 9.90% | 1 | 1.24 |
| DIAPH2 | 1 | 0.012938831 | 1.64 (1.11, 2.41) | 10.00% | 1 | 1.23 |
| DIAPH2 | 1 | 0.012938831 | 1.64 (1.11, 2.41) | 10.00% | 1 | 1.23 |
| DIAPH3 | 1 | 0.011764308 | 1.73 (1.13, 2.65) | 9.60% | 1 | 1.23 |
| DIAPH3 | 1 | 0.011764308 | 1.73 (1.13, 2.65) | 9.60% | 1 | 1.23 |
| DIXDC1 | -1 | 0.012908259 | 0.59 (0.39, 0.90) | 10.00% | 1 | 1.25 |
| DIXDC1 | -1 | 0.012908259 | 0.59 (0.39, 0.90) | 10.00% | 1 | 1.25 |
| DMKN | -1 | 0.011852194 | 0.69 (0.52, 0.92) | 9.60% | 1 | 1.25 |
| DMKN | -1 | 0.011852194 | 0.69 (0.52, 0.92) | 9.60% | 1 | 1.25 |
| DMKN | -1 | 0.011852194 | 0.69 (0.52, 0.92) | 9.60% | 1 | 1.25 |
| DMKN | -1 | 0.011852194 | 0.69 (0.52, 0.92) | 9.60% | 1 | 1.25 |
| DMKN | -1 | 0.011852194 | 0.69 (0.52, 0.92) | 9.60% | 1 | 1.25 |
| DMKN | -1 | 0.011852194 | 0.69 (0.52, 0.92) | 9.60% | 1 | 1.25 |
| DMKN | -1 | 0.011852194 | 0.69 (0.52, 0.92) | 9.60% | 1 | 1.25 |
| DMKN | -1 | 0.011852194 | 0.69 (0.52, 0.92) | 9.60% | 1 | 1.25 |
| DMKN | -1 | 0.011852194 | 0.69 (0.52, 0.92) | 9.60% | 1 | 1.25 |
| DMKN | -1 | 0.011852194 | 0.69 (0.52, 0.92) | 9.60% | 1 | 1.25 |
| DNALI1 | -1 | 0.011877008 | 0.64 (0.45, 0.91) | 9.60% | 1 | 1.26 |
| DPCD | -1 | 0.012550805 | 0.65 (0.47, 0.91) | 9.90% | 1 | 1.25 |
| DSCR8 | 1 | 0.010278294 | 1.47 (1.09, 1.97) | 8.90% | 1 | 1.24 |
| DSCR8 | 1 | 0.010278294 | 1.47 (1.09, 1.97) | 8.90% | 1 | 1.24 |
| DSCR8 | 1 | 0.010278294 | 1.47 (1.09, 1.97) | 8.90% | 1 | 1.24 |
| DSCR8 | 1 | 0.010278294 | 1.47 (1.09, 1.97) | 8.90% | 1 | 1.24 |
| DSCR8 | 1 | 0.010278294 | 1.47 (1.09, 1.97) | 8.90% | 1 | 1.24 |
| DYNC1I2 | -1 | 0.011091334 | 0.66 (0.48, 0.91) | 9.30% | 1 | 1.26 |
| E2F1 | 1 | 0.012893985 | 1.55 (1.10, 2.18) | 10.00% | 1 | 1.24 |
| EEF2K | -1 | 0.012968682 | 0.54 (0.33, 0.88) | 10.00% | 1 | 1.24 |
| EIF3H | 1 | 0.011777481 | 1.47 (1.09, 1.99) | 9.60% | 1 | 1.24 |
| ENO3 | -1 | 0.012599193 | 0.71 (0.55, 0.93) | 9.90% | 1 | 1.24 |
| ENO3 | -1 | 0.012599193 | 0.71 (0.55, 0.93) | 9.90% | 1 | 1.24 |
| ENO3 | -1 | 0.012599193 | 0.71 (0.55, 0.93) | 9.90% | 1 | 1.24 |
| EPX | -1 | 0.012837554 | 0.67 (0.49, 0.92) | 10.00% | 1 | 1.25 |
| EVL | -1 | 0.011561201 | 0.54 (0.34, 0.87) | 9.50% | 1 | 1.25 |
| FAM115A | -1 | 0.010185752 | 0.69 (0.52, 0.92) | 8.90% | 1 | 1.26 |
| FAM120A | -1 | 0.011120887 | 0.59 (0.39, 0.89) | 9.30% | 1 | 1.26 |
| FAM126A | 1 | 0.012480024 | 1.92 (1.15, 3.20) | 9.80% | 1 | 1.21 |
| FAM170A | 1 | 0.011370873 | 1.46 (1.09, 1.97) | 9.40% | 1 | 1.24 |
| FAM170A | 1 | 0.011370873 | 1.46 (1.09, 1.97) | 9.40% | 1 | 1.24 |
| FGB | 1 | 0.012651414 | 1.36 (1.07, 1.73) | 9.90% | 1 | 1.22 |
| FGB | 1 | 0.012651414 | 1.36 (1.07, 1.73) | 9.90% | 1 | 1.22 |
| FMNL3 | -1 | 0.010449305 | 0.68 (0.51, 0.91) | 9.00% | 1 | 1.26 |
| FMNL3 | -1 | 0.010449305 | 0.68 (0.51, 0.91) | 9.00% | 1 | 1.26 |
| GJA1 | -1 | 0.010395278 | 0.61 (0.42, 0.89) | 8.90% | 1 | 1.26 |
| GJA5 | -1 | 0.011769561 | 0.66 (0.48, 0.91) | 9.60% | 1 | 1.26 |
| GJA5 | -1 | 0.011769561 | 0.66 (0.48, 0.91) | 9.60% | 1 | 1.26 |
| GMFB | -1 | 0.012184114 | 0.64 (0.45, 0.91) | 9.70% | 1 | 1.26 |
| GPR180 | 1 | 0.011018759 | 1.78 (1.14, 2.79) | 9.20% | 1 | 1.23 |
| GUCY2E | 1 | 0.011410591 | 1.53 (1.10, 2.13) | 9.40% | 1 | 1.24 |
| HOMER1 | 1 | 0.011336137 | 1.65 (1.12, 2.42) | 9.40% | 1 | 1.24 |
| HOOK2 | -1 | 0.011600713 | 0.67 (0.49, 0.92) | 9.50% | 1 | 1.25 |
| HOOK2 | -1 | 0.011600713 | 0.67 (0.49, 0.92) | 9.50% | 1 | 1.25 |
| HOXA11 | 1 | 0.012018331 | 1.45 (1.09, 1.94) | 9.60% | 1 | 1.24 |
| HSD11B1 | 1 | 0.010395781 | 1.46 (1.09, 1.94) | 8.90% | 1 | 1.24 |
| H5D11B1 | 1 | 0.010395781 | 1.46 (1.09, 1.94) | 8.90% | 1 | 1.24 |
| HSPA12B | -1 | 0.012610444 | 0.61 (0.41, 0.90) | 9.90% | 1 | 1.25 |
| HSPA12B | -1 | 0.012610444 | 0.61 (0.41, 0.90) | 9.90% | 1 | 1.25 |
| HSPG2 | -1 | 0.010853095 | 0.61 (0.42, 0.89) | 9.20% | 1 | 1.26 |
| HUWE1 | 1 | 0.011791679 | 1.59 (1.11, 2.29) | 9.60% | 1 | 1.24 |
| HYAL2 | -1 | 0.010655266 | 0.70 (0.54, 0.92) | 9.00% | 1 | 1.25 |
| HYAL2 | -1 | 0.010655266 | 0.70 (0.54, 0.92) | 9.00% | 1 | 1.25 |
| IKBKAP | -1 | 0.011093127 | 0.59 (0.40, 0.89) | 9.30% | 1 | 1.26 |
| IKBKB | -1 | 0.011775833 | 0.61 (0.42, 0.90) | 9.60% | 1 | 1.26 |
| IKBKB | -1 | 0.011775833 | 0.61 (0.42, 0.90) | 9.60% | 1 | 1.26 |
| IKBKB | -1 | 0.011775833 | 0.61 (0.42, 0.90) | 9.60% | 1 | 1.26 |
| IKBKB | -1 | 0.011775833 | 0.61 (0.42, 0.90) | 9.60% | 1 | 1.26 |
| IKBKB | -1 | 0.011775833 | 0.61 (0.42, 0.90) | 9.60% | 1 | 1.26 |
| IKBKB | -1 | 0.011775833 | 0.61 (0.42, 0.90) | 9.60% | 1 | 1.26 |
| IKBKB | -1 | 0.011775833 | 0.61 (0.42, 0.90) | 9.60% | 1 | 1.26 |
| IL17C | 1 | 0.010547784 | 1.50 (1.10, 2.04) | 9.00% | 1 | 1.24 |
| ILF2 | 1 | 0.012045497 | 1.59 (1.11, 2.28) | 9.60% | 1 | 1.24 |
| ITPRIP | 1 | 0.010966603 | 1.63 (1.12, 2.36) | 9.20% | 1 | 1.24 |
| KIAA0528 | -1 | 0.0115925 | 0.64 (0.46, 0.91) | 9.50% | 1 | 1.26 |
| KIAA1644 | -1 | 0.010636853 | 0.65 (0.47, 0.91) | 9.00% | 1 | 1.26 |
| KIF1C | -1 | 0.0120162 | 0.67 (0.49, 0.91) | 9.60% | 1 | 1.25 |
| KRT6C | 1 | 0.012814231 | 1.38 (1.07, 1.79) | 10.00% | 1 | 1.23 |
| LGMN | 1 | 0.011416167 | 1.45 (1.09, 1.94) | 9.40% | 1 | 1.24 |
| LGMN | 1 | 0.011416167 | 1.45 (1.09, 1.94) | 9.40% | 1 | 1.24 |
| LINGO1 | 1 | 0.012057057 | 1.70 (1.12, 2.56) | 9.60% | 1 | 1.23 |
| LIX1 | 1 | 0.01111679 | 1.59 (1.11, 2.28) | 9.30% | 1 | 1.24 |
| LOC100129931 | -1 | 0.011472246 | 0.65 (0.46, 0.91) | 9.40% | 1 | 1.26 |
| LOC100233209 | 1 | 0.012607821 | 1.43 (1.08, 1.90) | 9.90% | 1 | 1.23 |
| LOC144776 | 1 | 0.012325145 | 1.38 (1.07, 1.79) | 9.80% | 1 | 1.23 |
| LOC283761 | 1 | 0.011585471 | 1.57 (1.11, 2.24) | 9.50% | 1 | 1.24 |
| LOC283761 | 1 | 0.011585471 | 1.57 (1.11, 2.24) | 9.50% | 1 | 1.24 |
| LOC283761 | 1 | 0.011585471 | 1.57 (1.11, 2.24) | 9.50% | 1 | 1.24 |
| LOC283761 | 1 | 0.011585471 | 1.57 (1.11, 2.24) | 9.50% | 1 | 1.24 |
| LOC400043 | -1 | 0.011245964 | 0.65 (0.47, 0.91) | 9.30% | 1 | 1.26 |
| LOC91316 | 1 | 0.011446822 | 1.67 (1.12, 2.48) | 9.40% | 1 | 1.24 |
| LOXL3 | -1 | 0.012709255 | 0.65 (0.46, 0.91) | 9.90% | 1 | 1.25 |
| LRGUK | -1 | 0.010254375 | 0.65 (0.47, 0.90) | 8.90% | 1 | 1.26 |
| LRPAP1 | -1 | 0.011640785 | 0.60 (0.40, 0.89) | 9.50% | 1 | 1.26 |
| LRRC23 | -1 | 0.011607799 | 0.64 (0.45, 0.90) | 9.50% | 1 | 1.26 |
| LRRC23 | -1 | 0.011607799 | 0.64 (0.45, 0.90) | 9.50% | 1 | 1.26 |
| LRRC23 | -1 | 0.011607799 | 0.64 (0.45, 0.90) | 9.50% | 1 | 1.26 |
| LRRC56 | -1 | 0.010591983 | 0.68 (0.51, 0.92) | 9.00% | 1 | 1.26 |
| LRTOMT | -1 | 0.010400431 | 0.59 (0.40, 0.88) | 8.90% | 1 | 1.26 |
| LRTOMT | -1 | 0.010400431 | 0.59 (0.40, 0.88) | 8.90% | 1 | 1.26 |
| LRTOMT | -1 | 0.010400431 | 0.59 (0.40, 0.88) | 8.90% | 1 | 1.26 |
| LRTOMT | -1 | 0.010400431 | 0.59 (0.40, 0.88) | 8.90% | 1 | 1.26 |
| LRTOMT | -1 | 0.010400431 | 0.59 (0.40, 0.88) | 8.90% | 1 | 1.26 |
| LRTOMT | -1 | 0.010400431 | 0.59 (0.40, 0.88) | 8.90% | 1 | 1.26 |
| LYRM1 | -1 | 0.012839377 | 0.59 (0.38, 0.89) | 10.00% | 1 | 1.25 |
| LYRM1 | -1 | 0.012839377 | 0.59 (0.38, 0.89) | 10.00% | 1 | 1.25 |
| LYRM1 | -1 | 0.012839377 | 0.59 (0.38, 0.89) | 10.00% | 1 | 1.25 |
| MAML1 | 1 | 0.012398823 | 1.60 (1.11, 2.31) | 9.80% | 1 | 1.24 |
| MAP3K1 | -1 | 0.012373459 | 0.60 (0.40, 0.90) | 9.80% | 1 | 1.25 |
| MAPK14 | 1 | 0.012704887 | 1.45 (1.08, 1.94) | 9.90% | 1 | 1.23 |
| MAPK14 | 1 | 0.012704887 | 1.45 (1.08, 1.94) | 9.90% | 1 | 1.23 |
| MAPK14 | 1 | 0.012704887 | 1.45 (1.08, 1.94) | 9.90% | 1 | 1.23 |
| MAPK14 | 1 | 0.012704887 | 1.45 (1.08, 1.94) | 9.90% | 1 | 1.23 |
| MDH1B | -1 | 0.012649336 | 0.68 (0.51, 0.92) | 9.90% | 1 | 1.25 |
| MEIS1 | 1 | 0.012898017 | 1.69 (1.12, 2.55) | 10.00% | 1 | 1.23 |
| MEIS3 | -1 | 0.012545351 | 0.63 (0.44, 0.91) | 9.90% | 1 | 1.25 |
| MEIS3 | -1 | 0.012545351 | 0.63 (0.44, 0.91) | 9.90% | 1 | 1.25 |
| MORN1 | -1 | 0.011397709 | 0.63 (0.45, 0.90) | 9.40% | 1 | 1.26 |
| MPHOSPH9 | 1 | 0.012594163 | 1.53 (1.10, 2.15) | 9.90% | 1 | 1.24 |
| MPZL1 | 1 | 0.012035119 | 1.49 (1.09, 2.04) | 9.60% | 1 | 1.24 |
| MPZL1 | 1 | 0.012035119 | 1.49 (1.09, 2.04) | 9.60% | 1 | 1.24 |
| MPZL1 | 1 | 0.012035119 | 1.49 (1.09, 2.04) | 9.60% | 1 | 1.24 |
| MRPL34 | 1 | 0.012406647 | 1.51 (1.09, 2.08) | 9.80% | 1 | 1.24 |
| MSTO1 | 1 | 0.012937941 | 1.59 (1.10, 2.29) | 10.00% | 1 | 1.23 |
| MYLK2 | 1 | 0.010890821 | 1.66 (1.12, 2.45) | 9.20% | 1 | 1.24 |
| MYO7A | 1 | 0.012737066 | 1.56 (1.10, 2.22) | 9.90% | 1 | 1.24 |
| MYO7A | 1 | 0.012737066 | 1.56 (1.10, 2.22) | 9.90% | 1 | 1.24 |
| MYO7A | 1 | 0.012737066 | 1.56 (1.10, 2.22) | 9.90% | 1 | 1.24 |
| MYOG | 1 | 0.011353552 | 1.36 (1.07, 1.72) | 9.40% | 1 | 1.23 |
| NAA15 | 1 | 0.012422722 | 1.54 (1.10, 2.16) | 9.80% | 1 | 1.24 |
| NBAS | 1 | 0.012672103 | 1.55 (1.10, 2.19) | 9.90% | 1 | 1.24 |
| NBPF3 | -1 | 0.012664759 | 0.70 (0.52, 0.93) | 9.90% | 1 | 1.25 |
| NDUFB6 | -1 | 0.012109776 | 0.66 (0.48, 0.91) | 9.70% | 1 | 1.25 |
| NDUFB6 | -1 | 0.012109776 | 0.66 (0.48, 0.91) | 9.70% | 1 | 1.25 |
| NDUFB6 | -1 | 0.012109776 | 0.66 (0.48, 0.91) | 9.70% | 1 | 1.25 |
| NFE2L1 | -1 | 0.010751476 | 0.65 (0.47, 0.91) | 9.10% | 1 | 1.26 |
| NOL10 | 1 | 0.011705945 | 1.59 (1.11, 2.28) | 9.50% | 1 | 1.24 |
| NPHS2 | -1 | 0.011756253 | 0.58 (0.38, 0.89) | 9.60% | 1 | 1.25 |
| NPLOC4 | 1 | 0.011913565 | 1.61 (1.11, 2.35) | 9.60% | 1 | 1.24 |
| NUDT21 | 1 | 0.011032838 | 1.60 (1.11, 2.30) | 9.20% | 1 | 1.24 |
| NUP188 | -1 | 0.011639171 | 0.55 (0.34, 0.87) | 9.50% | 1 | 1.25 |
| OTOL1 | 1 | 0.012763111 | 1.39 (1.07, 1.81) | 9.90% | 1 | 1.23 |
| PARP3 | -1 | 0.01013124 | 0.68 (0.50, 0.91) | 8.90% | 1 | 1.26 |
| PARP3 | -1 | 0.01013124 | 0.68 (0.50, 0.91) | 8.90% | 1 | 1.26 |
| PCDH11Y | 1 | 0.012243822 | 1.46 (1.09, 1.97) | 9.70% | 1 | 1.24 |
| PCDH11Y | 1 | 0.012243822 | 1.46 (1.09, 1.97) | 9.70% | 1 | 1.24 |
| PCDH11Y | 1 | 0.012243822 | 1.46 (1.09, 1.97) | 9.70% | 1 | 1.24 |
| PDE2A | 1 | 0.010557993 | 1.43 (1.09, 1.88) | 9.00% | 1 | 1.24 |
| PDE2A | 1 | 0.010557993 | 1.43 (1.09, 1.88) | 9.00% | 1 | 1.24 |
| PDE2A | 1 | 0.010557993 | 1.43 (1.09, 1.88) | 9.00% | 1 | 1.24 |
| PDE2A | 1 | 0.010557993 | 1.43 (1.09, 1.88) | 9.00% | 1 | 1.24 |
| PDE4A | -1 | 0.01282959 | 0.66 (0.48, 0.92) | 10.00% | 1 | 1.25 |
| PDE4A | -1 | 0.01282959 | 0.66 (0.48, 0.92) | 10.00% | 1 | 1.25 |
| PDE4A | -1 | 0.01282959 | 0.66 (0.48, 0.92) | 10.00% | 1 | 1.25 |
| PDE4A | -1 | 0.01282959 | 0.66 (0.48, 0.92) | 10.00% | 1 | 1.25 |
| PERP | 1 | 0.012898142 | 1.49 (1.09, 2.04) | 10.00% | 1 | 1.24 |
| PHGR1 | -1 | 0.010645275 | 0.68 (0.50, 0.91) | 9.00% | 1 | 1.26 |
| PHLDB1 | -1 | 0.010487792 | 0.62 (0.43, 0.90) | 9.00% | 1 | 1.26 |
| PHLDB1 | -1 | 0.010487792 | 0.62 (0.43, 0.90) | 9.00% | 1 | 1.26 |
| PHLDB1 | -1 | 0.010487792 | 0.62 (0.43, 0.90) | 9.00% | 1 | 1.26 |
| PIGL | -1 | 0.012014368 | 0.68 (0.51, 0.92) | 9.60% | 1 | 1.25 |
| PLA2G7 | 1 | 0.010739931 | 1.45 (1.09, 1.92) | 9.10% | 1 | 1.24 |
| PLA2G7 | 1 | 0.010739931 | 1.45 (1.09, 1.92) | 9.10% | 1 | 1.24 |
| PLEKHM2 | -1 | 0.010229479 | 0.64 (0.46, 0.90) | 8.90% | 1 | 1.26 |
| POPDC2 | -1 | 0.011856189 | 0.66 (0.47, 0.91) | 9.60% | 1 | 1.26 |
| PPM1B | 1 | 0.011847581 | 1.59 (1.11, 2.29) | 9.60% | 1 | 1.24 |
| PPM1B | 1 | 0.011847581 | 1.59 (1.11, 2.29) | 9.60% | 1 | 1.24 |
| PPM1B | 1 | 0.011847581 | 1.59 (1.11, 2.29) | 9.60% | 1 | 1.24 |
| PPM1B | 1 | 0.011847581 | 1.59 (1.11, 2.29) | 9.60% | 1 | 1.24 |
| PPM1B | 1 | 0.011847581 | 1.59 (1.11, 2.29) | 9.60% | 1 | 1.24 |
| PPP1R14C | 1 | 0.010609549 | 1.69 (1.13, 2.54) | 9.00% | 1 | 1.24 |
| PPP3R1 | 1 | 0.011797778 | 1.48 (1.09, 2.00) | 9.60% | 1 | 1.24 |
| PPP6R3 | 1 | 0.01182462 | 1.46 (1.09, 1.96) | 9.60% | 1 | 1.24 |
| PPP6R3 | 1 | 0.01182462 | 1.46 (1.09, 1.96) | 9.60% | 1 | 1.24 |
| PPP6R3 | 1 | 0.01182462 | 1.46 (1.09, 1.96) | 9.60% | 1 | 1.24 |
| PPP6R3 | 1 | 0.01182462 | 1.46 (1.09, 1.96) | 9.60% | 1 | 1.24 |
| PPP6R3 | 1 | 0.01182462 | 1.46 (1.09, 1.96) | 9.60% | 1 | 1.24 |
| PPP6R3 | 1 | 0.01182462 | 1.46 (1.09, 1.96) | 9.60% | 1 | 1.24 |
| PRAME | 1 | 0.012015796 | 1.56 (1.10, 2.20) | 9.60% | 1 | 1.24 |
| PRAME | 1 | 0.012015796 | 1.56 (1.10, 2.20) | 9.60% | 1 | 1.24 |
| PRAME | 1 | 0.012015796 | 1.56 (1.10, 2.20) | 9.60% | 1 | 1.24 |
| PRAME | 1 | 0.012015796 | 1.56 (1.10, 2.20) | 9.60% | 1 | 1.24 |
| PRAME | 1 | 0.012015796 | 1.56 (1.10, 2.20) | 9.60% | 1 | 1.24 |
| PRDM1 | 1 | 0.010864619 | 1.56 (1.11, 2.21) | 9.20% | 1 | 1.24 |
| PRDM1 | 1 | 0.010864619 | 1.56 (1.11, 2.21) | 9.20% | 1 | 1.24 |
| PRIM2 | 1 | 0.01189465 | 1.53 (1.10, 2.13) | 9.60% | 1 | 1.24 |
| PSMD11 | 1 | 0.012123119 | 1.56 (1.10, 2.21) | 9.70% | 1 | 1.24 |
| PTPRT | -1 | 0.012542322 | 0.60 (0.40, 0.90) | 9.90% | 1 | 1.25 |
| PTPRT | -1 | 0.012542322 | 0.60 (0.40, 0.90) | 9.90% | 1 | 1.25 |
| QPRT | 1 | 0.012915155 | 1.66 (1.11, 2.49) | 10.00% | 1 | 1.23 |
| RACGAP1 | 1 | 0.012817548 | 1.63 (1.11, 2.40) | 10.00% | 1 | 1.23 |
| RACGAP1 | 1 | 0.012817548 | 1.63 (1.11, 2.40) | 10.00% | 1 | 1.23 |
| RACGAP1 | 1 | 0.012817548 | 1.63 (1.11, 2.40) | 10.00% | 1 | 1.23 |
| RAMP2 | -1 | 0.011709734 | 0.68 (0.50, 0.92) | 9.50% | 1 | 1.25 |
| RARRES3 | -1 | 0.012940159 | 0.54 (0.33, 0.88) | 10.00% | 1 | 1.24 |
| RASGRF1 | 1 | 0.011225453 | 1.57 (1.11, 2.23) | 9.30% | 1 | 1.24 |
| RASGRF1 | 1 | 0.011225453 | 1.57 (1.11, 2.23) | 9.30% | 1 | 1.24 |
| RASGRF1 | 1 | 0.011225453 | 1.57 (1.11, 2.23) | 9.30% | 1 | 1.24 |
| RBM45 | -1 | 0.01057111 | 0.67 (0.49, 0.91) | 9.00% | 1 | 1.26 |
| REPS1 | 1 | 0.010521625 | 1.55 (1.11, 2.16) | 9.00% | 1 | 1.24 |
| REPS1 | 1 | 0.010521625 | 1.55 (1.11, 2.16) | 9.00% | 1 | 1.24 |
| RFC4 | 1 | 0.010308541 | 1.53 (1.11, 2.11) | 8.90% | 1 | 1.24 |
| RFC4 | 1 | 0.010308541 | 1.53 (1.11, 2.11) | 8.90% | 1 | 1.24 |
| RHOXF1 | -1 | 0.012326771 | 0.66 (0.48, 0.92) | 9.80% | 1 | 1.25 |
| RNF126 | -1 | 0.010822836 | 0.66 (0.48, 0.91) | 9.10% | 1 | 1.26 |
| RNMTL1 | -1 | 0.012150331 | 0.61 (0.41, 0.90) | 9.70% | 1 | 1.25 |
| RSPO1 | -1 | 0.010347188 | 0.61 (0.41, 0.89) | 8.90% | 1 | 1.26 |
| SARS | -1 | 0.011466736 | 0.61 (0.42, 0.90) | 9.40% | 1 | 1.26 |
| SARS | -1 | 0.011466736 | 0.61 (0.42, 0.90) | 9.40% | 1 | 1.26 |
| SARS | -1 | 0.011466736 | 0.61 (0.42, 0.90) | 9.40% | 1 | 1.26 |
| SAT1 | 1 | 0.012394064 | 1.48 (1.09, 2.02) | 9.80% | 1 | 1.24 |
| SAT1 | 1 | 0.012394064 | 1.48 (1.09, 2.02) | 9.80% | 1 | 1.24 |
| SATB2 | 1 | 0.011161894 | 1.72 (1.13, 2.61) | 9.30% | 1 | 1.23 |
| SATB2 | 1 | 0.011161894 | 1.72 (1.13, 2.61) | 9.30% | 1 | 1.23 |
| SATB2 | 1 | 0.011161894 | 1.72 (1.13, 2.61) | 9.30% | 1 | 1.23 |
| SCARF1 | -1 | 0.012672823 | 0.61 (0.41, 0.90) | 9.90% | 1 | 1.25 |
| SCARF1 | -1 | 0.012672823 | 0.61 (0.41, 0.90) | 9.90% | 1 | 1.25 |
| SCARF1 | -1 | 0.012672823 | 0.61 (0.41, 0.90) | 9.90% | 1 | 1.25 |
| SCARF1 | -1 | 0.012672823 | 0.61 (0.41, 0.90) | 9.90% | 1 | 1.25 |
| SCARF1 | -1 | 0.012672823 | 0.61 (0.41, 0.90) | 9.90% | 1 | 1.25 |
| SERPINI1 | -1 | 0.012995951 | 0.60 (0.40, 0.90) | 10.00% | 1 | 1.25 |
| SERPINI1 | -1 | 0.012995951 | 0.60 (0.40, 0.90) | 10.00% | 1 | 1.25 |
| SGSM2 | -1 | 0.012351408 | 0.61 (0.41, 0.90) | 9.80% | 1 | 1.25 |
| SGSM2 | -1 | 0.012351408 | 0.61 (0.41, 0.90) | 9.80% | 1 | 1.25 |
| SHF | -1 | 0.010407548 | 0.64 (0.46, 0.90) | 8.90% | 1 | 1.26 |
| SIRT3 | -1 | 0.010395406 | 0.66 (0.48, 0.91) | 8.90% | 1 | 1.26 |
| SIRT3 | -1 | 0.010395406 | 0.66 (0.48, 0.91) | 8.90% | 1 | 1.26 |
| SLC25A45 | -1 | 0.01047703 | 0.67 (0.49, 0.91) | 9.00% | 1 | 1.26 |
| SLC25A45 | -1 | 0.01047703 | 0.67 (0.49, 0.91) | 9.00% | 1 | 1.26 |
| SLC2A1 | 1 | 0.012060656 | 1.50 (1.09, 2.06) | 9.60% | 1 | 1.24 |
| SLC2A8 | -1 | 0.012758716 | 0.59 (0.39, 0.89) | 9.90% | 1 | 1.25 |
| SLC3OA3 | 1 | 0.012074545 | 1.47 (1.09, 1.98) | 9.60% | 1 | 1.24 |
| SMG6 | -1 | 0.011386009 | 0.56 (0.36, 0.88) | 9.40% | 1 | 1.25 |
| SMG6 | -1 | 0.011386009 | 0.56 (0.36, 0.88) | 9.40% | 1 | 1.25 |
| SOCS4 | -1 | 0.012820451 | 0.63 (0.44, 0.91) | 10.00% | 1 | 1.25 |
| SOCS4 | -1 | 0.012820451 | 0.63 (0.44, 0.91) | 10.00% | 1 | 1.25 |
| SPANXN2 | 1 | 0.010765906 | 1.37 (1.08, 1.74) | 9.10% | 1 | 1.23 |
| SPATA5L1 | 1 | 0.011141111 | 1.51 (1.10, 2.07) | 9.30% | 1 | 1.24 |
| SPATA5L1 | 1 | 0.011141111 | 1.51 (1.10, 2.07) | 9.30% | 1 | 1.24 |
| TCERG1 | 1 | 0.012536518 | 1.66 (1.12, 2.48) | 9.90% | 1 | 1.23 |
| TCERG1 | 1 | 0.012536518 | 1.66 (1.12, 2.48) | 9.90% | 1 | 1.23 |
| TMEM108 | -1 | 0.010602477 | 0.58 (0.38, 0.88) | 9.00% | 1 | 1.26 |
| TMEM108 | -1 | 0.010602477 | 0.58 (0.38, 0.88) | 9.00% | 1 | 1.26 |
| TMOD4 | -1 | 0.010546039 | 0.66 (0.48, 0.91) | 9.00% | 1 | 1.26 |
| TRAK1 | -1 | 0.012552903 | 0.57 (0.37, 0.89) | 9.90% | 1 | 1.25 |
| TRAK1 | -1 | 0.012552903 | 0.57 (0.37, 0.89) | 9.90% | 1 | 1.25 |
| TRAPPC6A | -1 | 0.012887833 | 0.72 (0.56, 0.93) | 10.00% | 1 | 1.24 |
| TRPC6 | -1 | 0.011288328 | 0.54 (0.34, 0.87) | 9.40% | 1 | 1.25 |
| TTC13 | 1 | 0.011432765 | 1.76 (1.14, 2.72) | 9.40% | 1 | 1.23 |
| TTC13 | 1 | 0.011432765 | 1.76 (1.14, 2.72) | 9.40% | 1 | 1.23 |
| TTLL2 | 1 | 0.010400094 | 1.41 (1.08, 1.84) | 8.90% | 1 | 1.24 |
| UBLCP1 | 1 | 0.010665655 | 1.72 (1.13, 2.62) | 9.00% | 1 | 1.24 |
| VAT1 | -1 | 0.011909359 | 0.69 (0.52, 0.92) | 9.60% | 1 | 1.25 |
| VWA5A | -1 | 0.012389498 | 0.63 (0.44, 0.91) | 9.80% | 1 | 1.25 |
| VWA5A | -1 | 0.012389498 | 0.63 (0.44, 0.91) | 9.80% | 1 | 1.25 |
| VWA5A | -1 | 0.012389498 | 0.63 (0.44, 0.91) | 9.80% | 1 | 1.25 |
| WASF3 | 1 | 0.011210635 | 1.68 (1.13, 2.52) | 9.30% | 1 | 1.24 |
| WDR65 | -1 | 0.010288387 | 0.67 (0.49, 0.91) | 8.90% | 1 | 1.26 |
| WDR65 | -1 | 0.010288387 | 0.67 (0.49, 0.91) | 8.90% | 1 | 1.26 |
| WDR65 | -1 | 0.010288387 | 0.67 (0.49, 0.91) | 8.90% | 1 | 1.26 |
| WDR65 | -1 | 0.010288387 | 0.67 (0.49, 0.91) | 8.90% | 1 | 1.26 |
| WDR65 | -1 | 0.010288387 | 0.67 (0.49, 0.91) | 8.90% | 1 | 1.26 |
| ZNF217 | 1 | 0.011799956 | 1.62 (1.11, 2.35) | 9.60% | 1 | 1.24 |
| ZNF223 | -1 | 0.010393203 | 0.74 (0.58, 0.93) | 8.90% | 1 | 1.24 |
| ZNF263 | -1 | 0.010124367 | 0.68 (0.51, 0.91) | 8.90% | 1 | 1.26 |
| ZNF384 | -1 | 0.012231707 | 0.52 (0.32, 0.87) | 9.70% | 1 | 1.24 |
| ZNF384 | -1 | 0.012231707 | 0.52 (0.32, 0.87) | 9.70% | 1 | 1.24 |
| ZNF384 | -1 | 0.012231707 | 0.52 (0.32, 0.87) | 9.70% | 1 | 1.24 |
| ZNF384 | -1 | 0.012231707 | 0.52 (0.32, 0.87) | 9.70% | 1 | 1.24 |
| ZNF384 | -1 | 0.012231707 | 0.52 (0.32, 0.87) | 9.70% | 1 | 1.24 |
| ZNF384 | -1 | 0.012231707 | 0.52 (0.32, 0.87) | 9.70% | 1 | 1.24 |
| ZNF384 | -1 | 0.012231707 | 0.52 (0.32, 0.87) | 9.70% | 1 | 1.24 |
| ZNF568 | -1 | 0.010974016 | 0.65 (0.47, 0.91) | 9.20% | 1 | 1.26 |
| ZNF594 | -1 | 0.010316068 | 0.64 (0.45, 0.90) | 8.90% | 1 | 1.26 |
| ZNF630 | -1 | 0.011504134 | 0.71 (0.54, 0.93) | 9.40% | 1 | 1.25 |
| ZNF630 | -1 | 0.011504134 | 0.71 (0.54, 0.93) | 9.40% | 1 | 1.25 |
| ZNF630 | -1 | 0.011504134 | 0.71 (0.54, 0.93) | 9.40% | 1 | 1.25 |
| ZNF716 | 1 | 0.010708479 | 1.34 (1.07, 1.68) | 9.10% | 1 | 1.22 |
| ZNF763 | -1 | 0.010793188 | 0.64 (0.45, 0.90) | 9.10% | 1 | 1.26 |
| ZPBP2 | 1 | 0.010005183 | 144 (1.09, 1.90) | 8.80% | 1 | 1.24 |
| ZPBP2 | 1 | 0.010005183 | 1.44 (1.09, 1.90) | 8.80% | 1 | 1.24 |

**Table 4. LincRNAs Identified as Associated with Breast Cancer Recurrence in 136 Breast Cancer Patients**

| LincRNA | Chr | start | End | Direction of Association (1 = Higher Expression Means Higher Risk) | *p*-value | Standardized Hazard Ratio Estimate (95% Confidence Interval) | *q*-value (FDR) | Maximum Lower Bound Std HR @ 10% FDR | Regression-to-the-Mean-Corrected Std HR |
|---|---|---|---|---|---|---|---|---|---|
| Khalil-787 | 15 | 49013058 | 49023258 | 1 | 6.10E-07 | 2.76 (1.85, 4.11) | 0.1% | 1.20 | 1.33 |
| Khalil-2019 | 7 | 20876450 | 21120000 | 1 | 1.48E-08 | 1.75 (1.44, 2.12) | <0.1% | 1.18 | 1.41 |
| Khalil-748 | 14 | 95988347 | 96000872 | -1 | 2.87E-06 | 0.39 (0.26, 0.58) | 0.2% | 1.18 | 1.30 |
| Khalil-1961 | 6 | 138901082 | 139020207 | 1 | 1.14E-05 | 2.01 (1.47, 2.74) | 0.6% | 1.10 | 1.31 |
| Khalil-1464 | 3 | 171491881 | 171537606 | 1 | 6.38E-05 | 1.58 (1.26, 1.98) | 2.0% | 1.04 | 1.29 |
| Khalil-769 | 14 | 102051822 | 102062397 | 1 | 6.34E-05 | 1.54 (1.25, 1.91) | 2.0% | 1.04 | 1.28 |
| Khalil-1542 | 4 | 26809677 | 26850102 | -1 | 6.86E-05 | 0.55 (0.41, 0.74) | 2.0% | 1.04 | 1.28 |
| Khalil-966 | 18 | 74178337 | 74202937 | -1 | 1.15E-04 | 0.54 (0.39, 0.74) | 2.6% | 1.04 | 1.26 |
| Khalil-1867 | 6 | 29238046 | 29248321 | 1 | 8.71E-05 | 1.53 (1.24,1.90) | 2.2% | 1.04 | 1.28 |
| Khalil-828 | 16 | 11294824 | 11305774 | -1 | 1.97E-04 | 0.54 (0.39, 0.75) | 3.7% | 1.04 | 1.25 |
| Khalil-1661 | 5 | 6829550 | 6853100 | 1 | 2.31E-04 | 1.99 (1.38, 2.88) | 4.0% | 1.04 | 1.24 |
| Khalil-1697 | 5 | 55567468 | 55766893 | -1 | 1.82E-04 | 0.49 (0.34, 0.71) | 3.7% | 1.04 | 1.24 |
| Khalil-584 | 13 | 33888375 | 33923550 | -1 | 2.72E-04 | 0.42 (0.27, 0.67) | 4.3% | 1.04 | 1.20 |
| Khalil-96 | 1 | 119551052 | 119563877 | -1 | 3.19E-04 | 0.57 (0.42, 0.77) | 4.7% | 1.03 | 1.24 |
| Khalil-1797 | 5 | 147328132 | 147409407 | 1 | 3.51E-04 | 1.58 (1.23, 2.04) | 4.8% | 1.02 | 1.25 |
| Khalil-933 | 18 | 42263052 | 42278652 | -1 | 5.25E-04 | 0.48 (0.32, 0.73) | 6.4% | 1.02 | 1.21 |
| Khalil-352 | 11 | 27594974 | 27650174 | -1 | 4.49E-04 | 0.67 (0.54, 0.84) | 5.8% | 1.02 | 1.24 |
| Khalil-207 | 1 | 239382227 | 239451677 | 1 | 6.35E-04 | 1.59 (1.22, 2.08) | 7.3% | 1.01 | 1.24 |
| Khalil-2153 | 8 | 20826745 | 20929495 | -1 | 7.42E-04 | 0.54 (0.38, 0.77) | 8.0% | 1.01 | 1.22 |
| Khalil-1788 | 5 | 139092141 | 139092141 | 1 | 8.51E-04 | 1.63 (1.22, 2.16) | 8.1% | 1.01 | 1.23 |
| Khalil-640 | 13 | 76462324 | 76475024 | 1 | 8.62E-D4 | 1.76 (1.26, 2.46) | 8.1% | 1.01 | 1.22 |
| Khalil-2253 | 8 | 111747974 | 111813899 | 1 | 8.55E-04 | 1.37 (1.14, 1.65) | 8.1% | 1.01 | 1.22 |
| Khalil-139 | 1 | 192885227 | 192945802 | 1 | 1.09E-03 | 2.00 (1.32, 3.02) | 9.7% | 1.00 | 1.20 |

**Table 5. Intergenic Region Locations Identified as Associated with Risk of Breast Cancer Recurrence in 136 Breast Cancer Patients**

| Region | Chromosome | Start | Stop | Direction of Association (1 = Higher Expression Means Higher Risk) | *p*-value | Standardized Hazard Ratio Estimate (95% Confidence Interval) | *q*-value (FDR) | Maximum Lower Bound Std HR @ 10% FDR | Regression-to-the-Mean-Corrected Std HR |
|---|---|---|---|---|---|---|---|---|---|
| 1 | chr10 | 37,544,879 | 37,547,649 | -1 | 0.00020 | 0.51 (0.36, 0.73) | 1.9% | 1.06 | 1.43 |
| 1 | chr10 | 37,619,993 | 37,623,136 | -1 | 0.00021 | 0.52 (036, 0.73) | 1.9% | 1.06 | 1.43 |
| 1 | chr10 | 37,568,808 | 37,569,830 | -1 | 0.00021 | 0.53 (0.38, 0.74) | 1.9% | 1.06 | 1.43 |
| 1 | chr10 | 37,564,164 | 37,565,625 | -1 | 0.00030 | 0.52 (0.37, 0.74) | 1.9% | 1.06 | 1.42 |
| 1 | chr10 | 37,568,381 | 37,568,752 | -1 | 0.00035 | 0.53 (0.38, 0.75) | 1.9% | 1.06 | 1.41 |
| 1 | chr10 | 37,565,983 | 37,566,523 | -1 | 0.00041 | 0.53 (0.37, 0.75) | 1.9% | 1.06 | 1.41 |
| 1 | chr10 | 37,565,659 | 37,565,893 | -1 | 0.00038 | 0.54 (039, 0.76) | 1.9% | 1.06 | 1.41 |
| 1 | chr10 | 37,566,868 | 37,567,570 | -1 | 0.00047 | 0.54 (0.38, 0.76) | 1.9% | 1.06 | 1.41 |
| 1 | chr10 | 37,536,807 | 37,542,809 | -1 | 0.00046 | 0.54 (0.38, 0.76) | 1.9% | 1.06 | 1.41 |
| 1 | chr10 | 37,543,796 | 37,544,843 | -1 | 0.00042 | 0.56 (0.40, 0.77) | 1.9% | 1.06 | 1.41 |
| 1 | chr10 | 37,561,599 | 37,562,256 | -1 | 0.00045 | 0.55 (0.39, 0.77) | 1.9% | 1.06 | 1.40 |
| 1 | chr10 | 37,683,049 | 37,685,772 | -1 | 0.00045 | 0.55 (0.39, 0.77) | 1.9% | 1.06 | 1.40 |
| 1 | chr10 | 37,556,000 | 37,560,492 | -1 | 0.00049 | 0.54 (0.38, 0.76) | 1.9% | 1.06 | 1.40 |
| 1 | chr10 | 37,575,273 | 37,575,491 | -1 | 0.00048 | 0.54 (0.38, 0.76) | 1.9% | 1.06 | 1.40 |
| 1 | chr10 | 37,524,962 | 37,527,301 | -1 | 0.00048 | 0.54 (0.39, 0.76) | 1.9% | 1.06 | 1.40 |
| 1 | chr10 | 37,576,187 | 37,605,418 | -1 | 0.00050 | 0.54 (0.38, 0.76) | 1.9% | 1.06 | 1.40 |
| 1 | chr10 | 37,571,872 | 37,573,979 | -1 | 0.00052 | 0.53 (0.37, 0.76) | 1.9% | 1.06 | 1.40 |
| 1 | chr10 | 37,698,051 | 37,701,722 | -1 | 0.00047 | 0.56 (0.41, 0.78) | 1.9% | 1.06 | 1.40 |
| 1 | chr10 | 37,672,851 | 37,673,126 | -1 | 0.00058 | 0.51 (0.35, 0.75) | 1.9% | 1.06 | 1.40 |
| 1 | chr10 | 37,562,687 | 37,562,938 | -1 | 0.00058 | 0.53 (0.37, 0.76) | 1.9% | 1.06 | 1.40 |
| 1 | chr10 | 37,527,343 | 37,532,088 | -1 | 0.00058 | 0.54 (0.38, 0.77) | 1.9% | L06 | 1.40 |
| 1 | chr10 | 37,560,679 | 37,560,951 | -1 | 0.00059 | 0.54 (0.37, 0.76) | 1.9% | 1.06 | 1.40 |
| 1 | chr10 | 37,570,039 | 37,571,493 | -1 | 0.00057 | 0.55 (0.39, 0.77) | 1.9% | 1.06 | 1.40 |
| 1 | chr10 | 37,575,935 | 37,576,119 | -1 | 0.00059 | 0.54 (0.38, 0.77) | 1.9% | 1.06 | 1.40 |
| 1 | chr10 | 37,609,308 | 37,613,953 | -1 | 0.00064 | 0.54 (0.38, 0.77) | 2.0% | 1.06 | 1.40 |
| 1 | chr10 | 37,547,787 | 37,555,941 | -1 | 0.00064 | 0.54 (0.38, 0.77) | 2.0% | 1.06 | 1.40 |
| 1 | chr10 | 37,571,633 | 37,571,823 | -1 | 0.00065 | 0.55 (0.39, 0.77) | 2.0% | 1.06 | 1.39 |
| 1 | chr10 | 37,667,401 | 37,672,780 | -1 | 0.00068 | 0.55 (0.39, 0.78) | 2.0% | 1.06 | 1.39 |
| 1 | chr10 | 37,696,906 | 37,697,533 | -1 | 0.00073 | 0.52 (0.36, 0.76) | 2.0% | 1.06 | 1.39 |
| 1 | chr10 | 37,574,016 | 37,574,684 | -1 | 0.00067 | 0.56 (0.40, 0.78) | 2.0% | 1.06 | 1.39 |
| 1 | chr10 | 37,567,627 | 37,568,328 | -1 | 0.00072 | 0.56 (0.41, 0.79) | 2.0% | 1.06 | 1.39 |
| 1 | chr10 | 37,693,467 | 37,695,809 | -1 | 0.00077 | 0.56 (0.40, 0.79) | 2.1% | 1.06 | 1.39 |
| 1 | chr10 | 37,654,871 | 37,660,982 | -1 | 0.00082 | 0.56 (0.40, 0.79) | 2.2% | 1.06 | 1.39 |
| 1 | chr10 | 37,661,152 | 37,667,368 | -1 | 0.00089 | 0.55 (0.39, 0.78) | 2.2% | 1.06 | 1.39 |
| 1 | chr10 | 37,532,337 | 37,536,539 | -1 | 0.00082 | 0.57 (0.41, 0.79) | 2.2% | 1.06 | 1.38 |
| 1 | chr10 | 37,562,300 | 37,562,581 | -1 | 0.00094 | 0.56 (0.39, 0.79) | 2.2% | 1.06 | 1.38 |
| 1 | chr10 | 37,624,793 | 37,626,867 | -1 | 0.00098 | 0.55 (0.39, 0.79) | 2.2% | 1.06 | 1.38 |
| 1 | chr10 | 37,639,567 | 37,654,034 | -1 | 0.00088 | 0.57 (0.41, 0.80) | 2.2% | 1.06 | 1.38 |
| 1 | chr10 | 37,561,214 | 37,561,565 | -1 | 0.00103 | 0.55 (0.39, 0.79) | 2.2% | 1.06 | 1.38 |
| 1 | chr10 | 37,673,162 | 37,677,786 | -1 | 0.00088 | 0.59 (0.43, 0.80) | 2.2% | 1.06 | 1.38 |
| 1 | chr10 | 37,701,753 | 37,702,125 | -1 | 0.00115 | 0.54 (0.37, 0.78) | 2.3% | 1.06 | 1.38 |
| 1 | chr10 | 37,626,921 | 37,629,035 | -1 | 0.00105 | 0.57 (0.41, 0.80) | 2.2% | 1.06 | 1.38 |
| 1 | chr10 | 37,695,882 | 37,696,129 | -1 | 0.00118 | 0.54 (0.37, 0.78) | 2.4% | 1.06 | 1.38 |
| 1 | chr10 | 37,654,201 | 37,654,768 | -1 | 0.00103 | 0.58 (0.42, 0.80) | 2.2% | 1.06 | 1.38 |
| 1 | chr10 | 37,685,838 | 37,687,256 | -1 | 0.00109 | 0.57 (0.41, 0.80) | 2.3% | 1.06 | 1.38 |
| 1 | chr10 | 37,732,968 | 37,734,110 | -1 | 0.00127 | 0.55 (0.38, 0.79) | 2.4% | 1.06 | 1.38 |
| 1 | chr10 | 37,563,652 | 37,564,121 | -1 | 0.00123 | 0.57 (0.41, 0.80) | 2.4% | 1.06 | 1.37 |
| 1 | chr10 | 37,614,131 | 37,619,802 | -1 | 0.00126 | 0.57 (0.40, 0.80) | 2.4% | 1.06 | 1.37 |
| 1 | chr10 | 37,566,588 | 37,566,765 | -1 | 0.00141 | 0.56 (0.39, 0.80) | 2.5% | 1.06 | 1.37 |
| 1 | chr10 | 37,632,316 | 37,633,017 | -1 | 0.00136 | 0.58 (0.41, 0.81) | 2.4% | 1.06 | 1.37 |
| 1 | chr10 | 37,633,094 | 37,639,262 | -1 | 0.00142 | 0.58 (0.42, 0.81) | 2.5% | 1.05 | 1.37 |
| 1 | chr10 | 37,689,902 | 37,693,366 | -1 | 0.00147 | 0.58 (0.42, 0.81) | 2.5% | 1.05 | 1.37 |
| 1 | chr10 | 37,623,556 | 37,624,754 | -1 | 0.00169 | 0.57 (0.40, 0.81) | 2.8% | 1.05 | 1.36 |
| 1 | chr10 | 37,707,161 | 37,714,709 | -1 | 0.00176 | 0.58 (0.41, 0.81) | 2.9% | 1.05 | 1.36 |
| 1 | chr10 | 37,687,488 | 37,689,827 | -1 | 0.00172 | 0.59 (0.42, 0.82) | 2.9% | 1.05 | 1.36 |
| 1 | chr10 | 37,629,082 | 37,632,280 | -1 | 0.00199 | 0.59 (0.42, 0.82) | 3.2% | 1.05 | 1.36 |
| 1 | chr10 | 37,575,553 | 37,575,759 | -1 | 0.00198 | 0.59 (0.42, 0.82) | 3.2% | 1.05 | 1.36 |
| 1 | chr10 | 37,677,954 | 37,682,763 | -1 | 0.00205 | 0.60 (0.43, 0.83) | 3.3% | 1.04 | 1.35 |
| 1 | chr10 | 37,563,315 | 37,563,610 | -1 | 0.00232 | 0.58 (0.40, 0.82) | 3.5% | 1.04 | 1.35 |
| 1 | chr10 | 37,575,794 | 37,575,896 | -1 | 0.00253 | 0.54 (0.36, 0.81) | 3.7% | 1.04 | 1.35 |
| 1 | chr10 | 37,542,848 | 37,543,623 | -1 | 0.00229 | 0.59 (0.42, 0.83) | 3.5% | 1.04 | 1.35 |
| 1 | chr10 | 37,561,067 | 37,561,175 | -1 | 0.00270 | 0.56 (0.38, 0.82) | 3.8% | 1.04 | 1.35 |
| 1 | chr10 | 37,734,169 | 37,740,911 | -1 | 0.00233 | 0.61 (0.44, 0.84) | 3.5% | 1.04 | 1.35 |
| 1 | chr10 | 37,702,517 | 37,707,119 | -1 | 0.00275 | 0.61 (0.44, 0.84) | 3.8% | 1.04 | 1.34 |
| 1 | chr10 | 37,605,587 | 37,609,120 | -1 | 0.00273 | 0.61 (0.44, 0.84) | 3.8% | 1.04 | 1.34 |
| 1 | chr10 | 37,562,986 | 37,563,198 | -1 | 0.00356 | 0.60 (0.43, 0.85) | 4.6% | 1.03 | 1.34 |
| 1 | chr10 | 37,740,954 | 37,745,137 | -1 | 0.00387 | 0.60 (0.43, 0.85) | 4.8% | 1.03 | 1.34 |
| 1 | chr10 | 37,716,056 | 37,717,321 | -1 | 0.00393 | 0.61 (0.44, 0.86) | 4.9% | 1.03 | 1.33 |
| 1 | chr10 | 37,639,364 | 37,639,473 | -1 | 0.00470 | 0.58 (0.40, 0.85) | 5.4% | 1.03 | 1.33 |
| 1 | chr10 | 37,728,632 | 37,731,719 | -1 | 0.00487 | 0.61 (0.43, 0.86) | 5.4% | 1.03 | 1.33 |
| 1 | chr10 | 37,717,419 | 37,728,598 | -1 | 0.00478 | 0.62 (0.45, 0.87) | 5.4% | 1.03 | 1.33 |
| 1 | chr10 | 37,731,776 | 37,732,927 | -1 | 0.00530 | 0.60 (0.42, 0.86) | 5.7% | 1.02 | 1.33 |
| 1 | chr10 | 37,747,085 | 37,748,228 | -1 | 0.00712 | 0.60 (0.42, 0.87) | 6.9% | 1.02 | 1.32 |
| 1 | chr10 | 37,714,745 | 37,715,803 | -1 | 0.00867 | 0.62 (0.43, 0.89) | 7.7% | 1.01 | 1.31 |
| 1 | chr10 | 37,696,547 | 37,696,778 | -1 | 0.01035 | 0.58 (0.38, 0.88) | 8.6% | 1.01 | 1.30 |
| 1 | chr10 | 37,750,435 | 37,752,430 | -1 | 0.01125 | 0.61 (0.42, 0.89) | 9.1% | 1.00 | 1.30 |
| 1 | chr10 | 37,745,221 | 37,746,267 | -1 | 0.01313 | 0.60 (0.40, 0.90) | 9.8% | 1.00 | 1.29 |
| 1 | chr10 | 37,697,835 | 37,697,938 | -1 | 0.01311 | 0.58 (0.38, 0.89) | 9.8% | 1.00 | 1.29 |
| 2 | chr13 | 33,913,029 | 33,915,362 | -1 | 0.00000 | 0.53 (0.41, 0.69) | 0.1% | 1.11 | 1.53 |
| 2 | chr13 | 33,895,486 | 33,899,980 | -1 | 0.00010 | 0.45 (0.30, 0.67) | 1.7% | 1.07 | 1.45 |
| 2 | chr13 | 33,861,025 | 33,870,309 | -1 | 0.00025 | 0.43 (0.28, 0.68) | 1.9% | 1.06 | 1.42 |
| 2 | chr13 | 33,885,431 | 33,895,429 | -1 | 0.00025 | 0.43 (0.21, 0.68) | 1.9% | 1.06 | 1.42 |
| 2 | chr13 | 33,905,649 | 33,907,334 | -1 | 0.00038 | 0.46 (0.30, 0.70) | 1.9% | 1.06 | 1.41 |
| 2 | chr13 | 33,875,986 | 33,880,221 | -1 | 0.00053 | 0.47 (0.31, 0.72) | 1.9% | 1.06 | 1.40 |
| 3 | chr14 | 100,492,181 | 100,494,055 | -1 | 0.00003 | 0.54 (0.41, 0.72) | 0.8% | 1.07 | 1.47 |
| 3 | chr14 | 100,490,070 | 100,492,136 | -1 | 0.00046 | 0.61 (0.46, 0.80) | 1.9% | 1.06 | 1.39 |
| 3 | chr14 | 100,488,826 | 100,489,839 | -1 | 0.00125 | 0.54 (0.37, 0.78) | 2.4% | 1.06 | 1.38 |
| 3 | chr14 | 100,452,553 | 100,453,714 | -1 | 0.00131 | 0.57 (0.40, 0.80) | 2.4% | 1.06 | 1.37 |
| 3 | chr14 | 100,480,756 | 100,482,681 | -1 | 0.00114 | 0.64 (0.48, 0.84) | 2.3% | 1.05 | 1.36 |
| 3 | chr14 | 100,513,361 | 100,516,723 | -1 | 0.00211 | 0.56 (0.38, 0.81) | 3.3% | 1.05 | 1.36 |
| 3 | chr14 | 100,522,353 | 100,526,500 | -1 | 0.00327 | 0.56 (0.38, 0.82) | 4.3% | 1.04 | 1.34 |
| 3 | chr14 | 100,494,916 | 100,500,335 | -1 | 0.00433 | 0.54 (0.35, 0.82) | 5.3% | 1.03 | 1.33 |
| 3 | chr14 | 100,482,810 | 100,488,781 | -1 | 0.00449 | 0.56 (0.37, 0.83) | 5.3% | 1.03 | 1.33 |
| 3 | chr14 | 100,500,384 | 100,505,674 | -1 | 0.00474 | 0.56 (037, 0.84) | 5.4% | 1.03 | 1.33 |
| 3 | chr14 | 100,505,745 | 100,509,590 | -1 | 0.00578 | 0.59 (0.41, 0.86) | 6.0% | 1.02 | 1.32 |
| 3 | chr14 | 100,510,107 | 100,513,108 | -1 | 0.00732 | 0.59 (0.40, 0.87) | 6.9% | 1.01 | 1.32 |
| 3 | chr14 | 100,494,179 | 100,494,879 | -1 | 0.00731 | 0.63 (0.45, 0.88) | 6.9% | 1.01 | 1.31 |
| 3 | chr14 | 100,466,798 | 100,467,228 | -1 | 0.00706 | 0.68 (0.51, 0.90) | 6.9% | 1.01 | 1.30 |
| 3 | chr14 | 100,509,621 | 100,510,070 | -1 | 0.01210 | 0.65 (0.47, 0.91) | 9.5% | 1.00 | 1.29 |
| 4 | chr15 | 50,998,590 | 50,999,355 | 1 | 0.00000 | 1.46 (1.27, 1.67) | 0.0% | 1.11 | 1.36 |
| 4 | chr15 | 50,995,854 | 50,996,481 | 1 | 0.00163 | 1.56 (1.18, 2.05) | 2.8% | 1.05 | 1.25 |
| 5 | chr15 | 71,427,690 | 71,430,497 | -1 | 0.00459 | 0.62 (0.44, 0.86) | 5.4% | 1.03 | 1.33 |
| 5 | chr15 | 71,423,356 | 71,427,046 | -1 | 0.00474 | 0.64 (0.47, 0.87) | 5.4% | 1.02 | 1.32 |
| 5 | chr15 | 71,420,670 | 71,423,120 | -1 | 0.00938 | 0.63 (0.45, 0.89) | 8.1% | 1.01 | 1.30 |
| 6 | chr17 | 42,098,064 | 42,100,474 | -1 | 0.00003 | 0.46 (0.32, 0.66) | 0.8% | 1.07 | 1.49 |
| 6 | chr17 | 42,097,068 | 42,097,990 | -1 | 0.00003 | 0.50 (0.36, 0.70) | 0.8% | 1.07 | 1.48 |
| 6 | chr17 | 42,095,337 | 42,097,009 | -1 | 0.00007 | 0.51 (0.36, 0.71) | 1.4% | 1.07 | 1.46 |
| 6 | chr17 | 42,093,032 | 42,094,287 | -1 | 0.00105 | 0.62 (0.47, 0.82) | 2.2% | 1.06 | 1.37 |
| 7 | chr19 | 15,685,213 | 15,685,925 | -1 | 0.00126 | 0.63 (0.47, 0.83) | 2.4% | 1.05 | 1.36 |
| 7 | chr19 | 15,686,036 | 15,688,801 | -1 | 0.00136 | 0.65 (0.49, 0.84) | 2.4% | 1.05 | 1.35 |
| 7 | chr19 | 15,681,763 | 15,685,139 | -1 | 0.00292 | 0.66 (0.51, 0.87) | 3.9% | 1.03 | 1.33 |
| 8 | chr2 | 162,136,055 | 162,136,356 | 1 | 0.00049 | 1.67 (1.25, 2.24) | 1.9% | 1.06 | 1.28 |
| 8 | chr2 | 162,135,070 | 162,135,249 | 1 | 0.00086 | 1.61 (1.22, 2.12) | 2.2% | 1.06 | 1.26 |
| 9 | chr5 | 34,201,715 | 34,201,852 | 1 | 0.00323 | 1.91 (1.24, 2.94) | 4.3% | 1.04 | 1.19 |
| 9 | chr5 | 34,170,829 | 34,170,949 | 1 | 0.00430 | 1.91 (1.22, 2.97) | 5.3% | 1.03 | 1.18 |
| 10 | chr6 | 72,099,739 | 72,107,969 | -1 | 0.00250 | 0.52 (0.34, 0.80) | 3.7% | 1.04 | 1.35 |
| 10 | chr6 | 72,092,048 | 72,099,685 | -1 | 0.00447 | 0.57 (0.39, 0.84) | 5.3% | 1.03 | 1.33 |
| 10 | chr6 | 72,082,646 | 72,084,661 | -1 | 0.00514 | 0.64 (0.47, 0.87) | 5.6% | 1.02 | 1.32 |
| 10 | chr6 | 72,090,253 | 72,091,283 | -1 | 0.01000 | 0.57 (0.37, 0.87) | 8.5% | 1.01 | 1.30 |
| 11 | chr6 | 151,715,844 | 151,717,137 | 1 | 0.00002 | 1.51 (1.25, 1.83) | 0.8% | 1.07 | 1.33 |
| 11 | chr6 | 151,717,215 | 151,718,164 | 1 | 0.00782 | 1.59 (1.13, 2.23) | 7.2% | 1.01 | 1.19 |
| 12 | chr7 | 149,668,770 | 149,669,327 | -1 | 0.00090 | 0.60 (0.44, 0.81) | 2.2% | 1.06 | 1.38 |
| 12 | chr7 | 149,925,596 | 149,925,704 | -1 | 0.00327 | 0.60 (0.43, 0.84) | 4.3% | 1.03 | 1.34 |
| 12 | chr7 | 149,699,811 | 149,700,117 | -1 | 0.00689 | 0.64 (0.47, 0.89) | 6.8% | 1.02 | 1.31 |
| 13 | chr8 | 12,401,386 | 12,401,654 | -1 | 0.00022 | 0.64 (0.51, 0.81) | 1.9% | 1.06 | 1.38 |
| 13 | chr8 | 12,432,626 | 12,433,347 | -1 | 0.00238 | 0.60 (0.43, 0.83) | 3.5% | 1.04 | 1.35 |
| 13 | chr8 | 12,403,417 | 12,403,612 | -1 | 0.00522 | 0.62 (0.45, 0.87) | 5.6% | 1.02 | 1.32 |
| 14 | chr9 | 134,279,738 | 134,284,311 | -1 | 0.00095 | 0.54 (0.38, 0.78) | 2.2% | 1.06 | 1.38 |
| 14 | chr9 | 134,271,924 | 134,274,441 | -1 | 0.00137 | 0.56 (0.40, 0.80) | 2.4% | 1.06 | 1.37 |
| 14 | chr9 | 134,291,733 | 134,295,852 | -1 | 0.00826 | 0.62 (0.43, 0.88) | 7.5% | 1.01 | 1.31 |
| 15 | chrX | 93,652,357 | 93,666,290 | -1 | 0.00619 | 0.62 (0.44, 0.87) | 6.3% | 1.02 | 1.32 |
| 15 | chrX | 93,688,784 | 93,693,228 | -1 | 0.00613 | 0.63 (0.45, 0.88) | 6.3% | 1.02 | 1.32 |
| 15 | chrX | 93,669,788 | 93,672,149 | -1 | 0.00674 | 0.61 (0.43, 0.87) | 6.8% | 1.02 | 1.32 |
| 15 | chrX | 93,666,349 | 93,667,298 | -1 | 0.01336 | 0.59 (0.39, 0.90) | 10.0% | 1.00 | 1.29 |
| **16** | chrX | 93,717,247 | 93,720,845 | -1 | 0.00466 | 0.61 (0.44, 0.86) | 5.4% | 1.03 | 1.33 |
| **16** | chrX | 93,725,918 | 93,737,489 | -1 | 0.00522 | 0.62 (0.44, 0.87) | 5.6% | 1.02 | 1.32 |
| **16** | chrX | 93,751,305 | 93,757,302 | -1 | 0.00855 | 0.64 (0.45, 0.89) | 7.7% | 1.01 | 1.31 |
| **16** | chrX | 93,748,573 | 93,749,757 | -1 | 0.01008 | 0.63 (0.44, 0.89) | 8.5% | 1.01 | 1.30 |
| **16** | chrX | 93,746,612 | 93,748,536 | -1 | 0.01154 | 0.63 (0.45, 0.90) | 9.1% | 1.00 | 1.30 |
| 17 | chrX | 93,772,799 | 93,777,937 | -1 | 0.00940 | 0.63 (0.44, 0.89) | 8.1% | 1.01 | 1.30 |
| 17 | chrX | 93,778,121 | 93,784,508 | -1 | 0.01142 | 0.65 (0.47, 0.91) | 9.1% | 1.00 | 1.29 |
| 18 | chrX | 93,824,387 | 93,825,877 | -1 | 0.00564 | 0.61 (0.43, 0.86) | 5.9% | 1.02 | 1.32 |
| 18 | chrX | 93,830,179 | 93,833,764 | -1 | 0.00623 | 0.61 (0.43, 0.87) | 6.3% | 1.02 | 1.32 |
| 19 | chr4 | 14,787,521 | 14,788,354 | -1 | 0.00032 | 0.57 (0.41, 0.77) | 1.9% | 1.06 | 1.41 |
| 20 | chr3 | 27,150,797 | 27,152,817 | -1 | 0.00057 | 0.51 (0.35, 0.75) | 1.9% | 1.06 | 1.40 |
| 21 | chr16 | 30,875,605 | 30,875,752 | -1 | 0.00037 | 0.60 (0.45, 0.79) | 1.9% | 1.06 | 1.40 |
| 22 | chr4 | 39,536,187 | 39,536,524 | -1 | 0.00018 | 0.63 (0.49, 0.80) | 1.9% | 1.06 | 1.40 |
| 23 | chr14 | 81,726,879 | 81,729,708 | -1 | 0.00094 | 0.52 (0.36, 0.77) | 2.2% | 1.06 | 1.39 |
| 24 | chrll | 781,048 | 781,371 | -1 | 0.00062 | 0.61 (0.46, 0.81) | 2.0% | 1.06 | 1.38 |
| 25 | chr12 | 57,793,185 | 57,793,823 | 1 | 0.00042 | 1.49 (1.19, 1.86) | 1.9% | 1.06 | 1.28 |
| 26 | chr8 | 81,525,870 | 81,526,021 | 1 | 0.00237 | 1.70 (1.21, 2.38) | 3.5% | 1.04 | 1.23 |
| 27 | chr4 | 74,877,348 | 74,877,549 | 1 | 0.00276 | 1.69 (1.20, 2.37) | 3.8% | 1.04 | 1.22 |
| 28 | chrl | 36,798,999 | 36,802,115 | -1 | 0.00305 | 0.60 (0.43, 0.84) | 4.2% | 1.04 | 1.34 |
| 29 | chr19 | 12,378,905 | 12,380,075 | -1 | 0.00340 | 0.58 (0.41, 0.84) | 4.4% | 1.03 | 1.34 |
| 30 | chr21 | 9,837,324 | 9,837,442 | 1 | 0.00270 | 1.50 (1.15, 1.95) | 3.8% | 1.03 | 1.23 |
| 31 | chr3 | 123,210,205 | 123,211,948 | 1 | 0.00339 | 1.72 (1.20, 2.48) | 4.4% | 1.03 | 1.21 |
| 32 | chr8 | 12,054,779 | 12,054,926 | -1 | 0.00359 | 0.59 (0.41, 0.84) | 4.6% | 1.03 | 1.34 |
| 33 | chrll | 63,336,814 | 63,339,112 | -1 | 0.00382 | 0.62 (0.45, 0.86) | 4.8% | 1.03 | 1.33 |
| 34 | chr6 | 36,575,163 | 36,576,155 | -1 | 0.00332 | 0.72 (0.58, 0.90) | 4.4% | 1.03 | 1.29 |
| 35 | chr14 | 38,291,258 | 38,299,693 | -1 | 0.00440 | 0.65 (0.49, 0.88) | 5.3% | 1.03 | 1.32 |
| 36 | chr1 | 168,218,748 | 168,220,325 | 1 | 0.00439 | 1.55 (1.15, 2.09) | 5.3% | 1.03 | 1.21 |
| 37 | chr3 | 112,414,798 | 112,415,017 | 1 | 0.00494 | 1.80 (1.20, 2.71) | 5.4% | 1.03 | 1.19 |
| 38 | chr10 | 133,732,921 | 133,733,113 | -1 | 0.00553 | 0.64 (0.46, 0.88) | 5.8% | 1.02 | 1.32 |
| 39 | chr3 | 195,671,271 | 195,671,479 | 1 | 0.00550 | 1.55 (1.14, 2.11) | 5.8% | 1.02 | 1.21 |
| 40 | chr5 | 180,678,078 | 180,680,053 | 1 | 0.00682 | 1.65 (1.15, 2.38) | 6.8% | 1.02 | 1.19 |
| 41 | chr10 | 29,738,073 | 29,738,359 | -1 | 0.00621 | 0.71 (0.55, 0.91) | 6.3% | 1.02 | 1.29 |
| 42 | chr2 | 91,816,094 | 91,819,627 | -1 | 0.00735 | 0.63 (0.44, 0.88) | 6.9% | 1.01 | 1.31 |
| 43 | chr15 | 39,777,520 | 39,813,177 | -1 | 0.00738 | 0.63 (0.45, 0.88) | 6.9% | 1.01 | 1.31 |
| 44 | chr16 | 21,527,242 | 21,527,480 | -1 | 0.00715 | 0.68 (0.51, 0.90) | 6.9% | 1.01 | 1.30 |
| 45 | chrl | 222,906,688 | 222,910,172 | 1 | 0.00738 | 1.55 (1.13, 2.15) | 6.9% | 1.01 | 1.19 |
| 46 | chr20 | 3,074,943 | 3,075,062 | 1 | 0.00770 | 1.67 (1.15, 2.43) | 7.2% | 1.01 | 1.18 |
| 47 | chr15 | 50,653,452 | 50,656,894 | 1 | 0.00787 | 1.49 (1.11, 1.99) | 7.2% | 1.01 | 1.20 |
| 48 | chr11 | 8,681,526 | 8,687,251 | -1 | 0.00851 | 0.61 (0.42, 0.88) | 7.7% | 1.01 | 1.31 |
| 49 | chr18 | 14,274,671 | 14,274,823 | -1 | 0.00912 | 0.59 (0.39, 0.88) | 7.9% | 1.01 | 1.31 |
| 50 | chr7 | 94,189,030 | 94,190,366 | -1 | 0.00943 | 0.60 (0.41, 0.88) | 8.1% | 1.01 | 1.31 |
| 51 | chr19 | 56,149,766 | 56,151,179 | -1 | 0.00958 | 0.59 (0.39, 0.88) | 8.1% | 1.01 | 1.31 |
| 52 | chr19 | 22,021,429 | 22,024,672 | -1 | 0.00909 | 0.65 (0.47, 0.90) | 7.9% | 1.01 | 1.30 |
| 53 | chr10 | 42,398,467 | 42,398,655 | 1 | 0.00822 | 1.37 (1.08, 1.73) | 7.5% | 1.01 | 1.19 |
| 54 | chr1 | 15,936,663 | 15,936,819 | 1 | 0.00905 | 1.53 (1.11, 2.10) | 7.9% | 1.01 | 1.19 |
| 55 | chr19 | 57,811,563 | 57,811,715 | 1 | 0.00906 | 1.61 (1.13, 2.29) | 7.9% | 1.01 | 1.18 |
| 56 | chrX | 115,108,909 | 115,109,012 | 1 | 0.01070 | 1.63 (1.12, 2.38) | 8.9% | 1.01 | 1.17 |
| 57 | chr10 | 38,637,965 | 38,638,108 | -1 | 0.01097 | 0.63 (0.44, 0.90) | 8.9% | 1.00 | 1.30 |
| 58 | chr7 | 76,741,733 | 76,741,898 | -1 | 0.01084 | 0.65 (0.47, 0.91) | 8.9% | 1.00 | 1.30 |
| 59 | chr2 | 96,492,560 | 96,493,149 | -1 | 0.01096 | 0.68 (0.50, 0.91) | 8.9% | 1.00 | 1.29 |
| 60 | chr6 | 26,130,873 | 26,132,970 | 1 | 0.01081 | 1.58 (1.11, 2.25) | 8.9% | 1.00 | 1.18 |
| 61 | chr16 | 25,271,035 | 25,272,071 | -1 | 0.01152 | 0.61 (0.42, 0.90) | 9.1% | 1.00 | 1.30 |
| 62 | chr5 | 10,435,961 | 10,438,476 | 1 | 0.01152 | 1.81 (1.14, 2.86) | 9.1% | 1.00 | 1.15 |
| 63 | chr16 | 31,465,709 | 31,467,707 | -1 | 0.01273 | 0.59 (0.39, 0.89) | 9.8% | 1.00 | 1.30 |
| 64 | chr9 | 33,018,805 | 33,020,959 | -1 | 0.01273 | 0.55 (0.35, 0.88) | 9.8% | 1.00 | 1.29 |
| 65 | chr17 | 3,710,044 | 3,712,797 | -1 | 0.01240 | 0.67 (0.48, 0.92) | 9.7% | 1.00 | 1.29 |
| 66 | chr1 | 121,484,124 | 121,485,310 | 1 | 0.01262 | 1.56 (1.10, 2.21) | 9.8% | 1.00 | 1.17 |
| 67 | chr17 | 41,379,297 | 41,379,450 | -1 | 0.01286 | 0.68 (0.51, 0.92) | 9.8% | 1.00 | 1.28 |
| 68 | chr6 | 138,982,087 | 138,982,527 | 1 | 0.01300 | 1.73 (1.12, 2.67) | 9.8% | 1.00 | 1.15 |
| 69 | chr1 | 11,968,187 | 11,968,792 | 1 | 0.01310 | 1.78 (1.13, 2.81) | 9.8% | 1.00 | 1.14 |

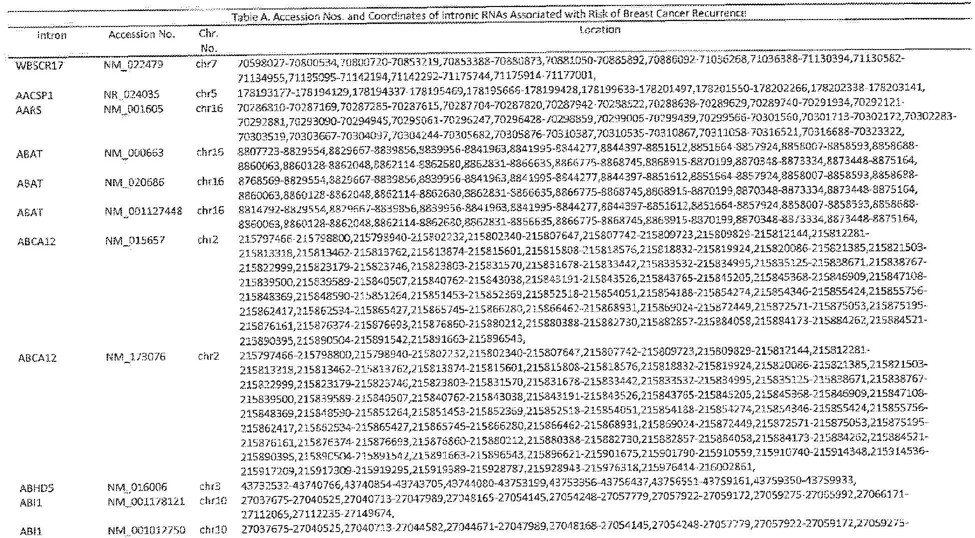

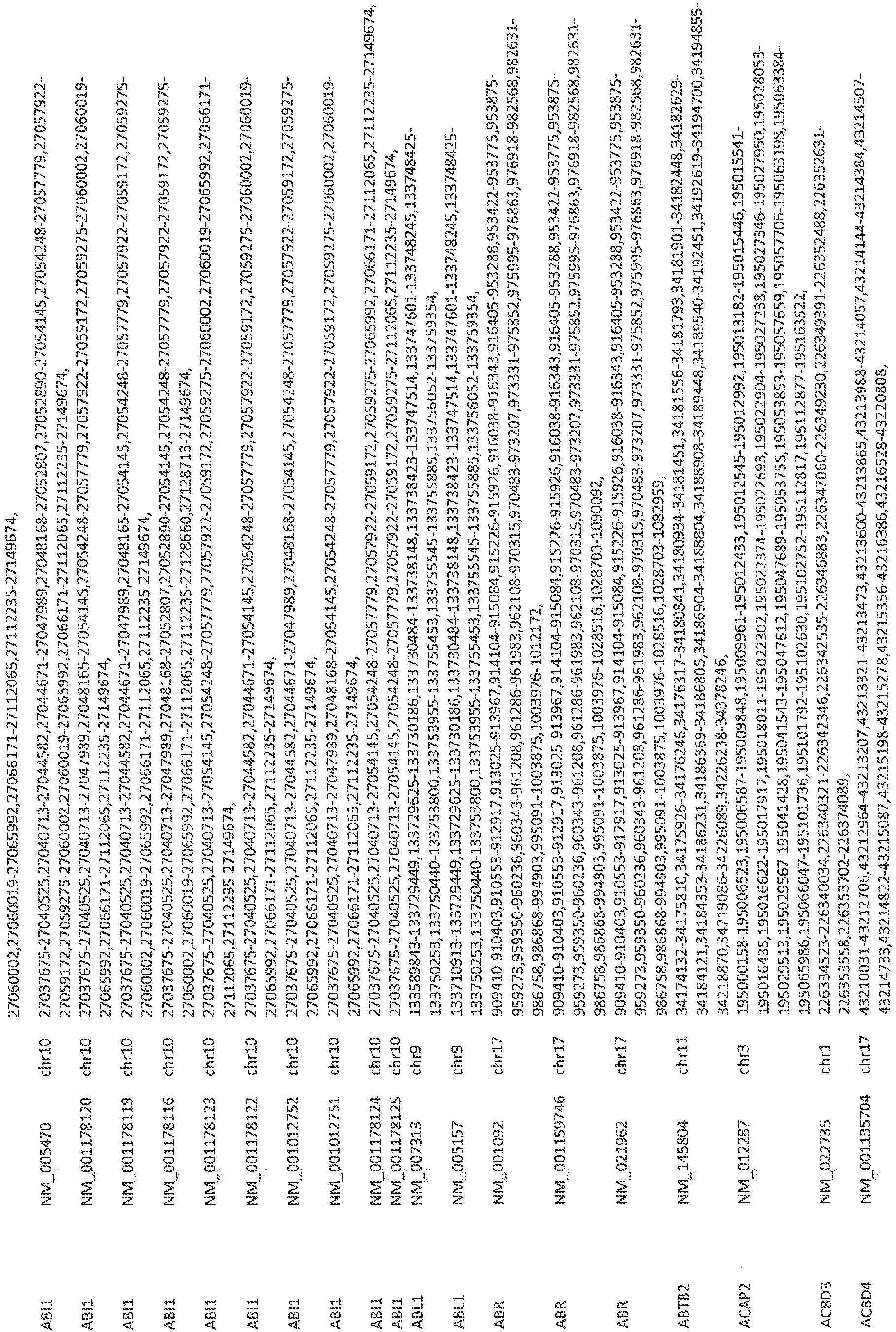

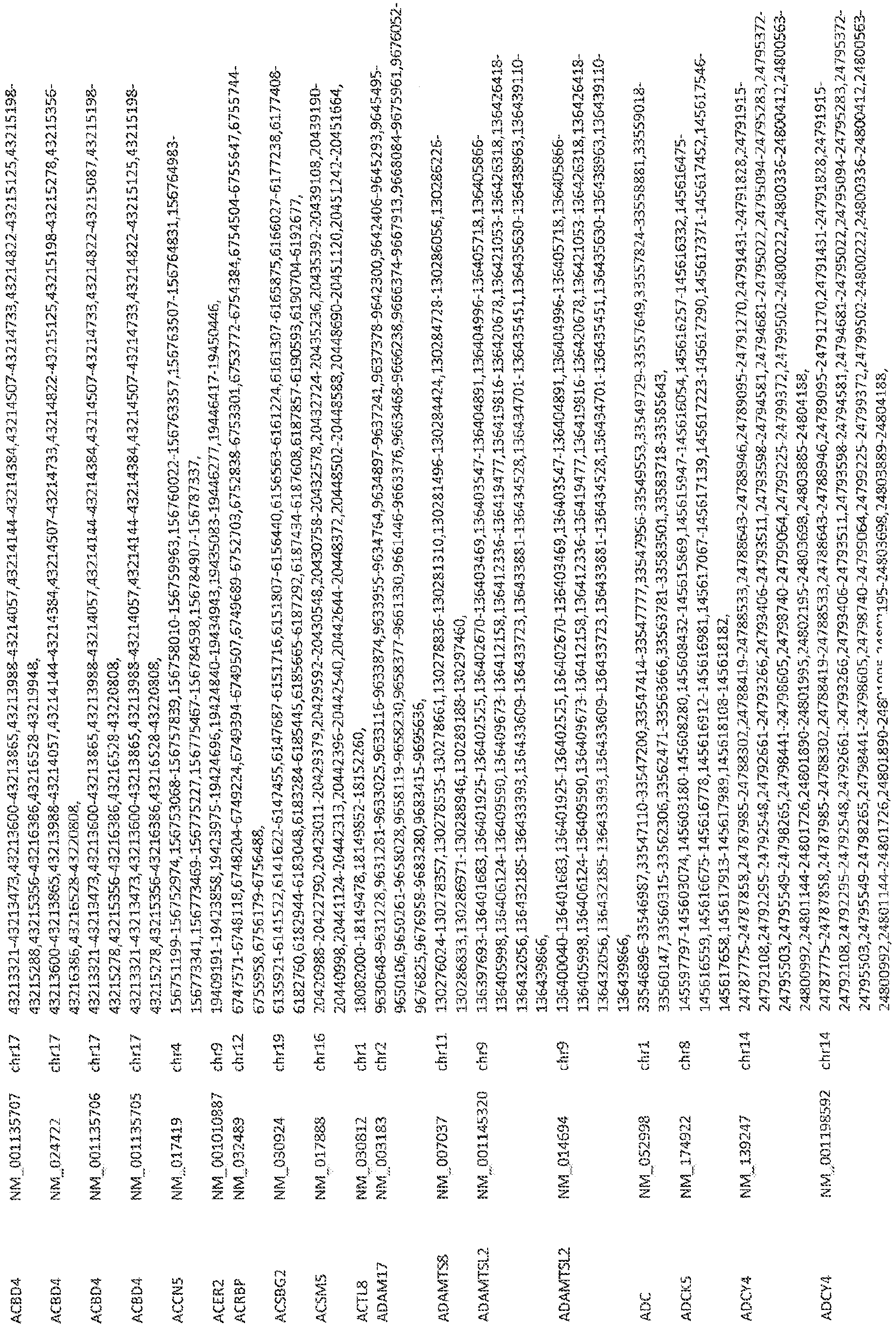

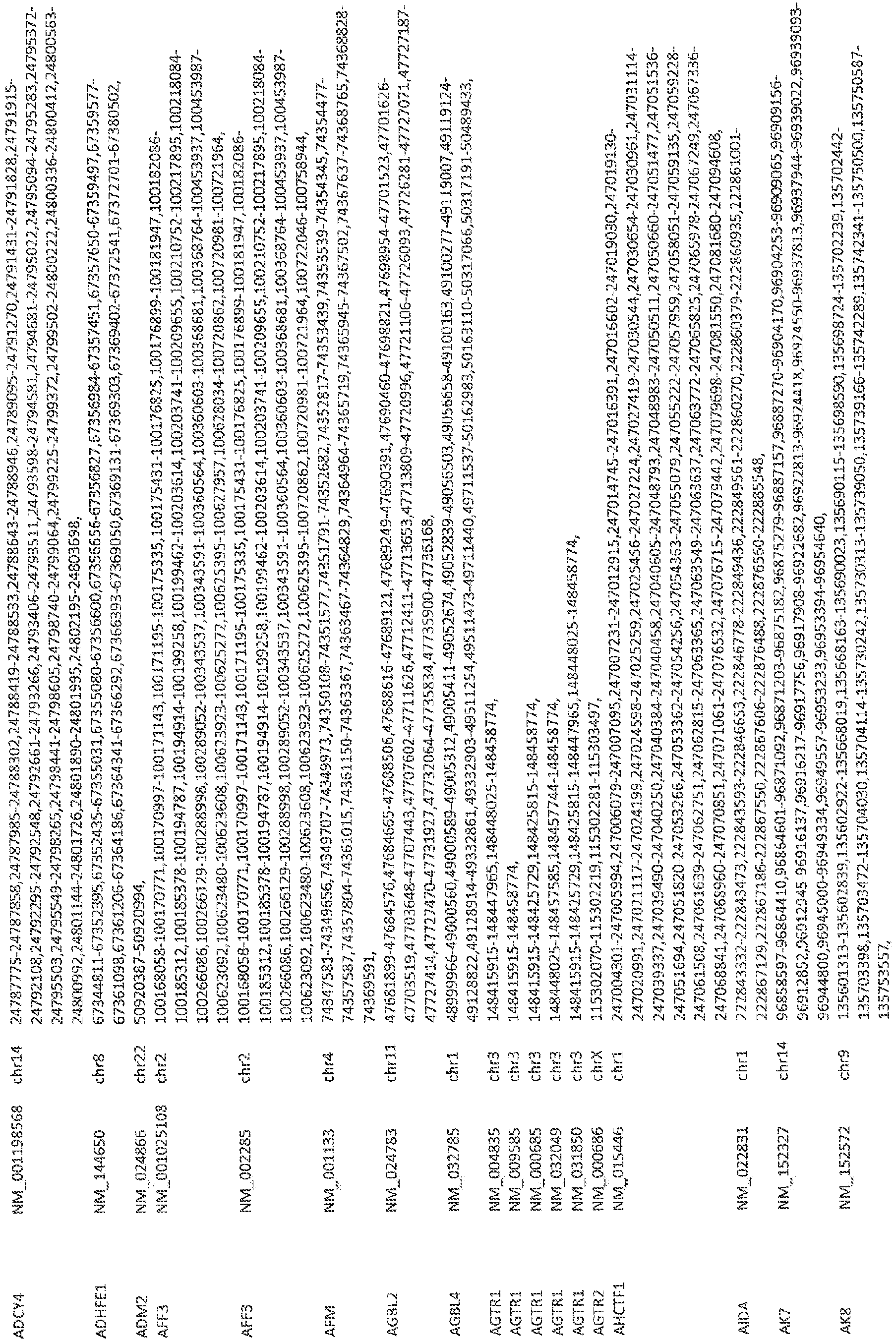

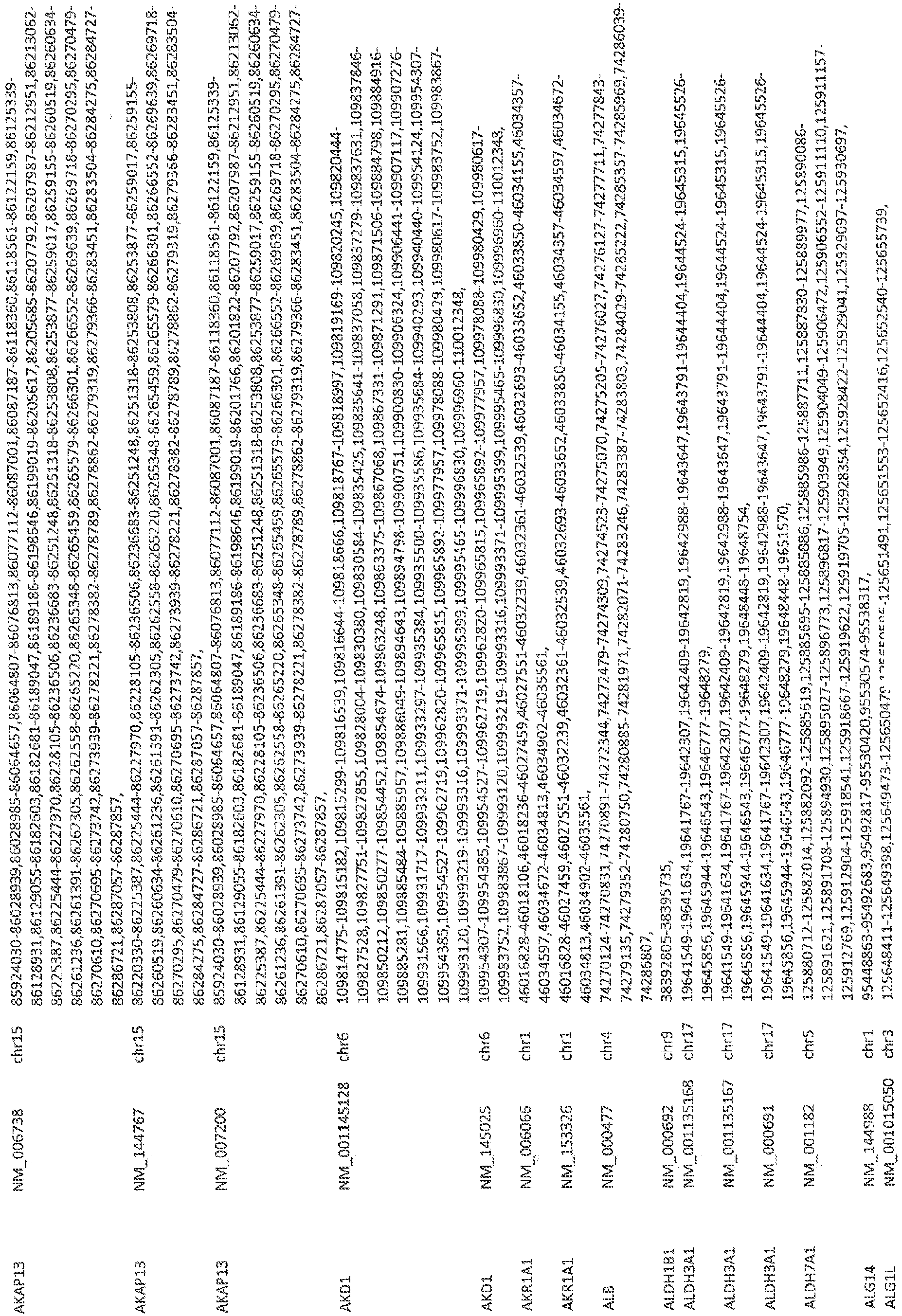

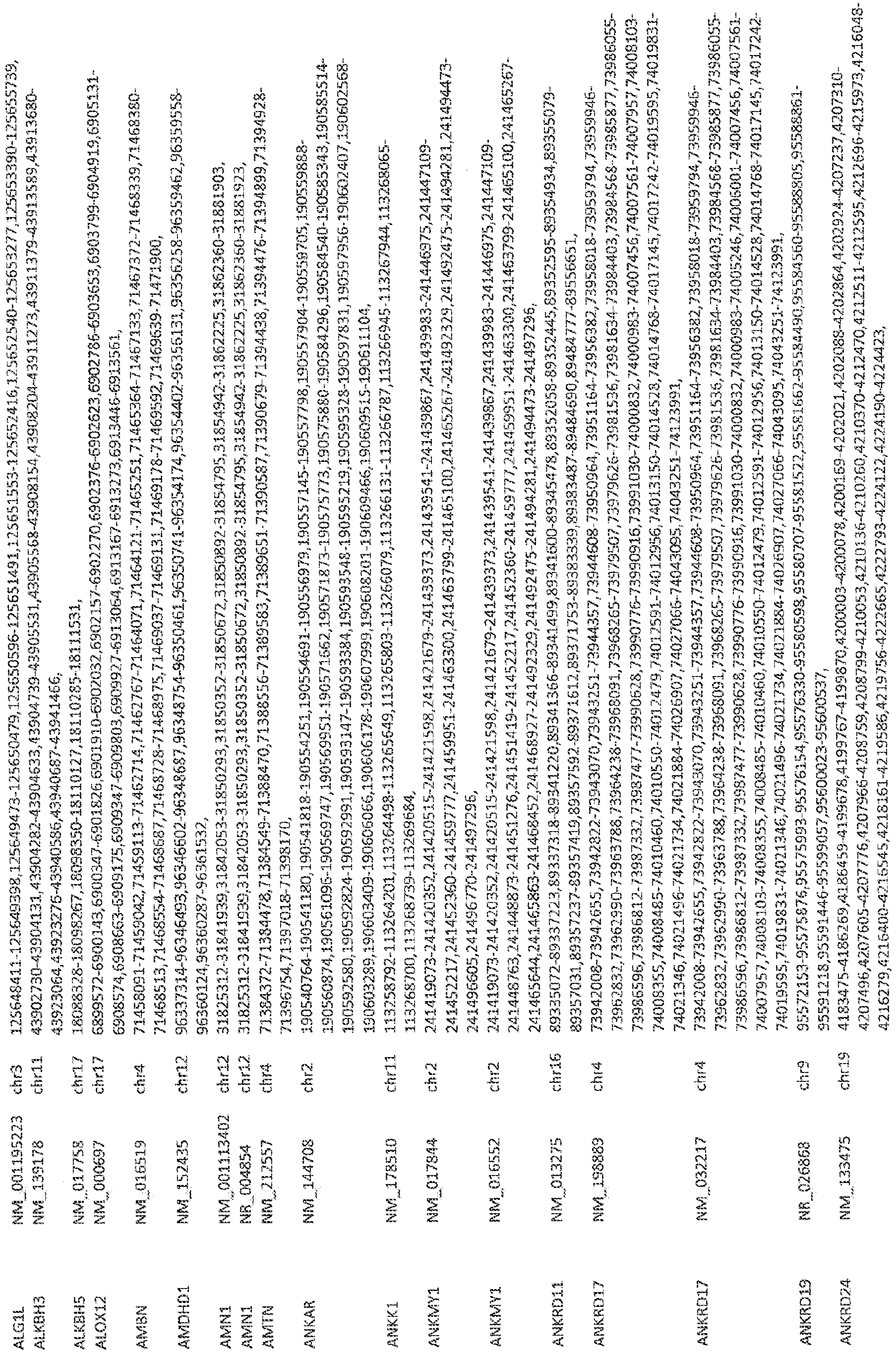

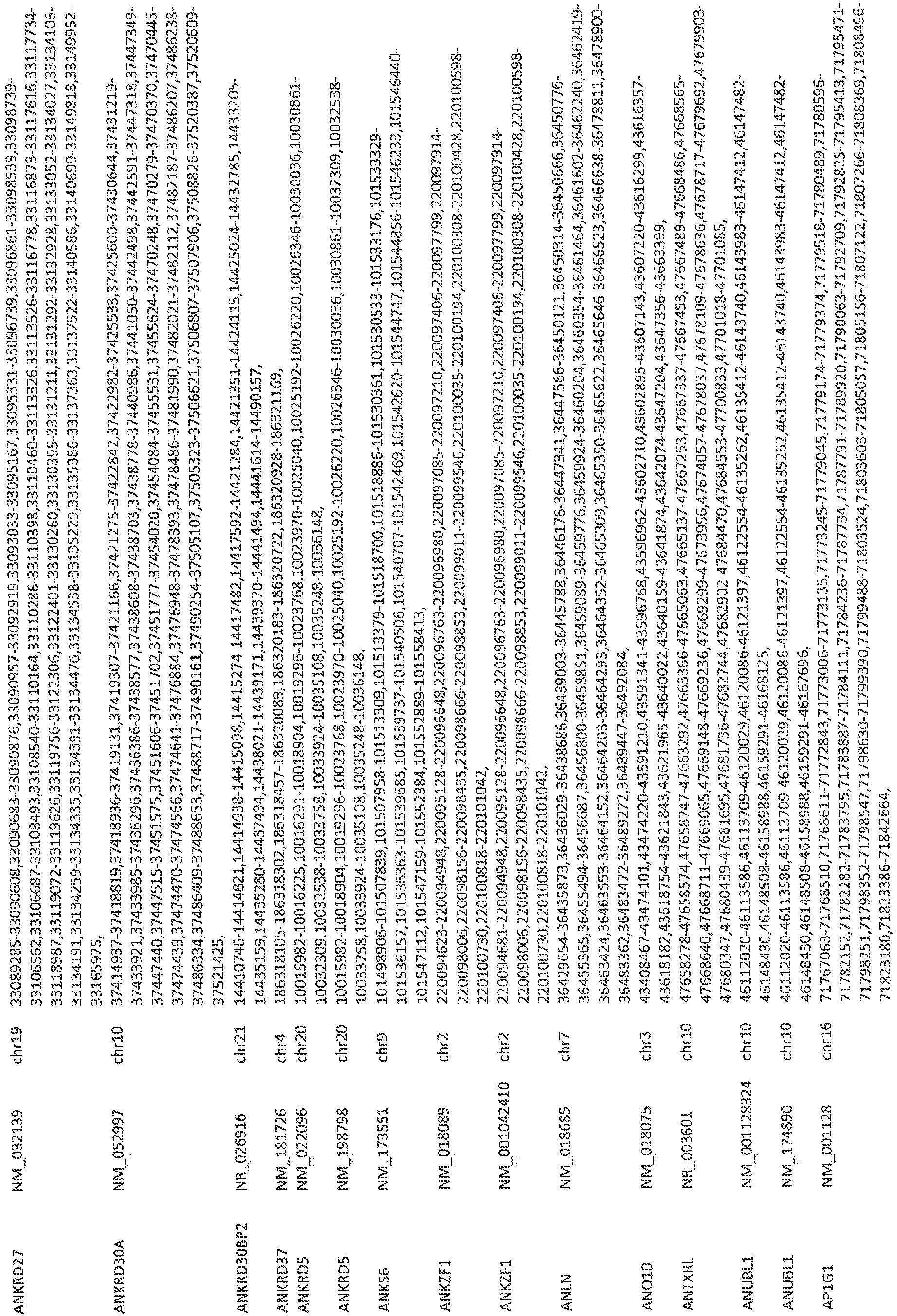

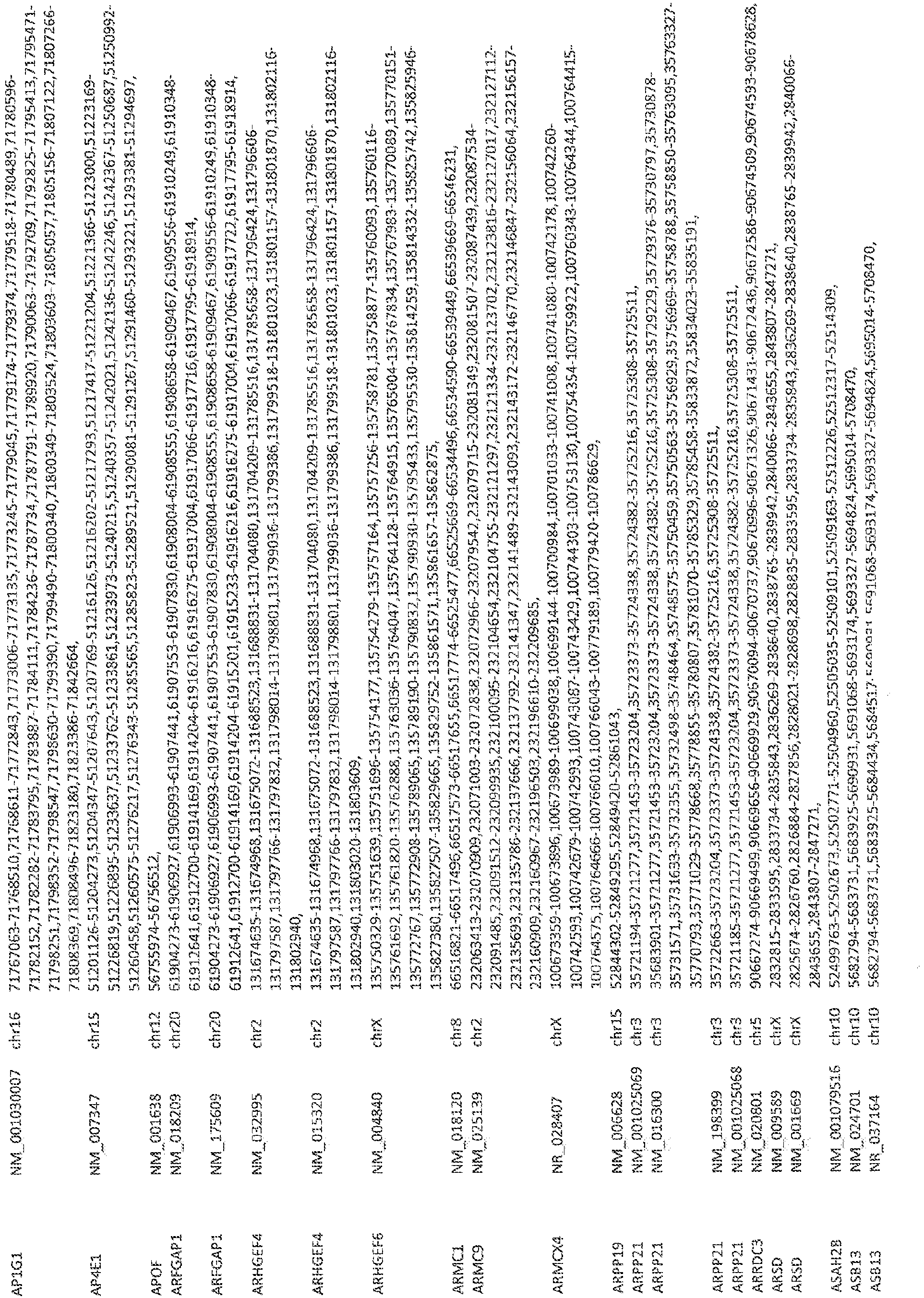

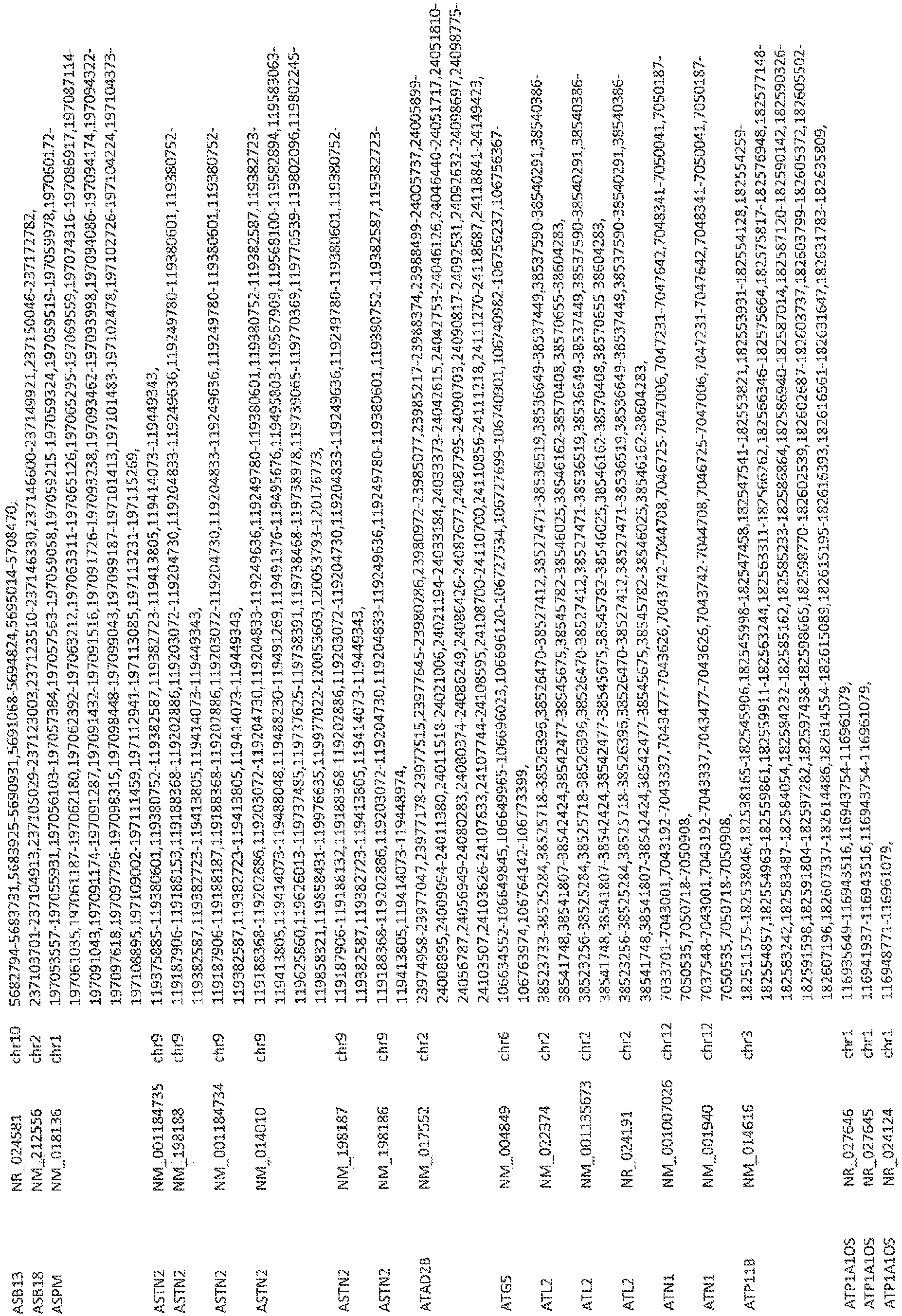

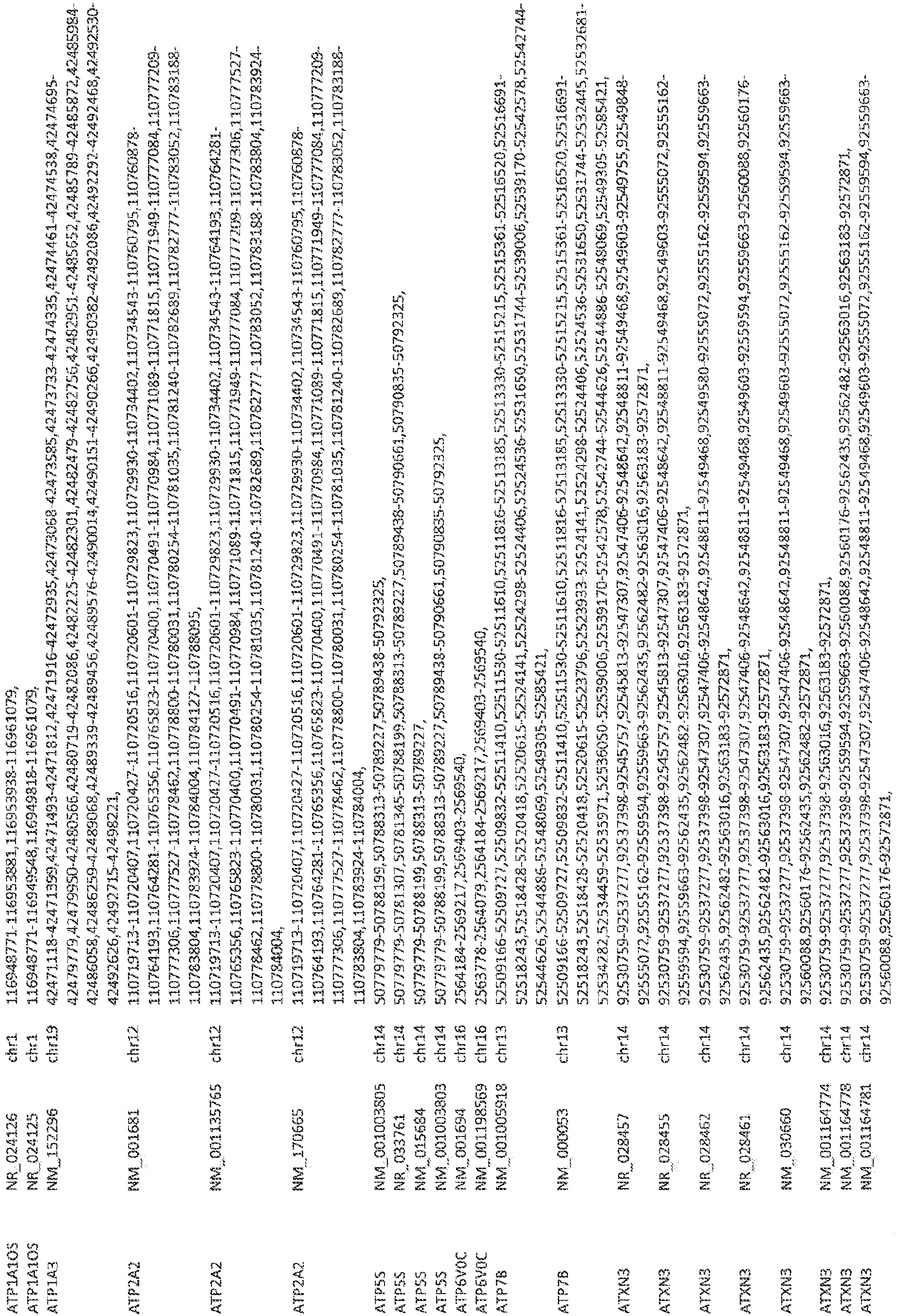

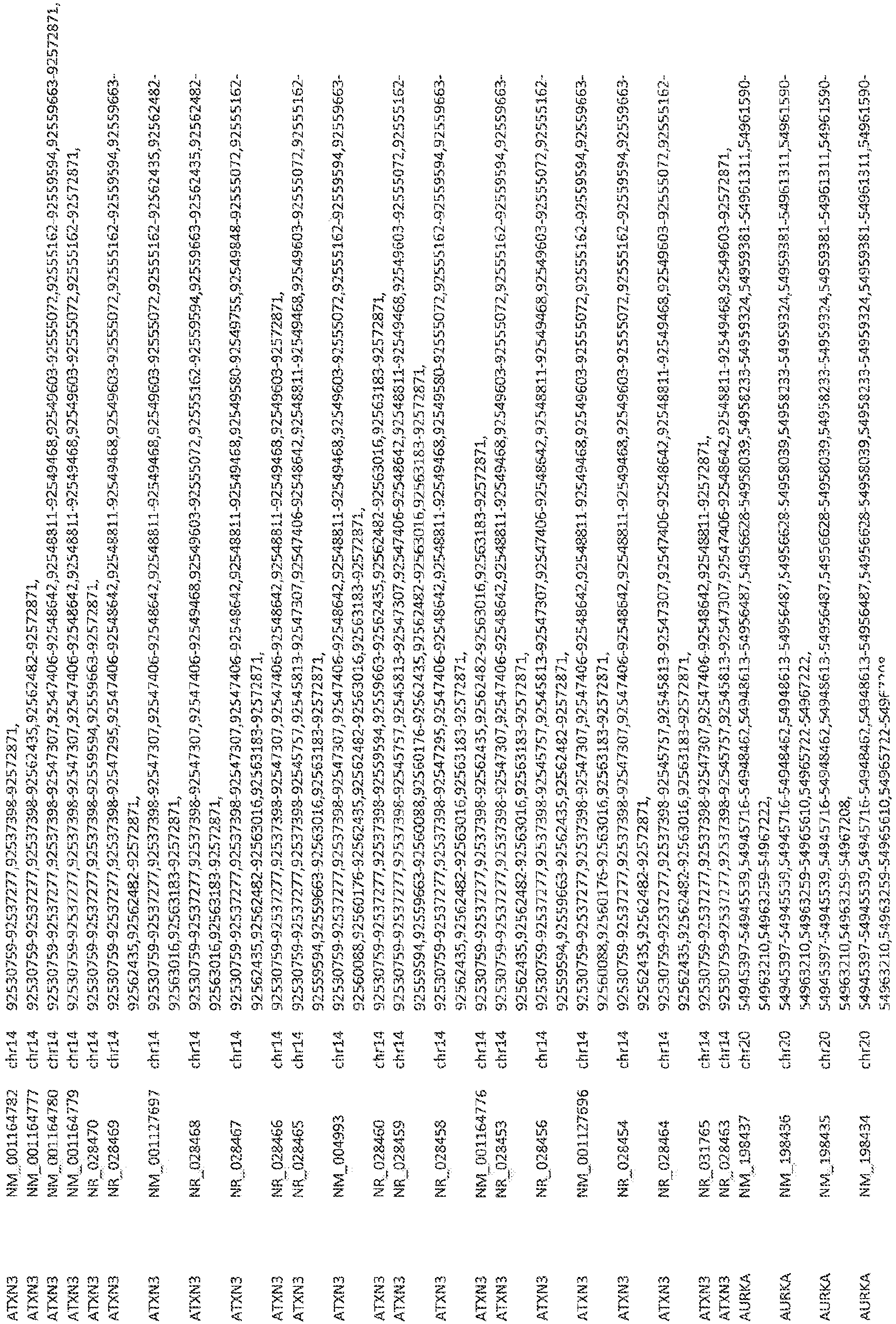

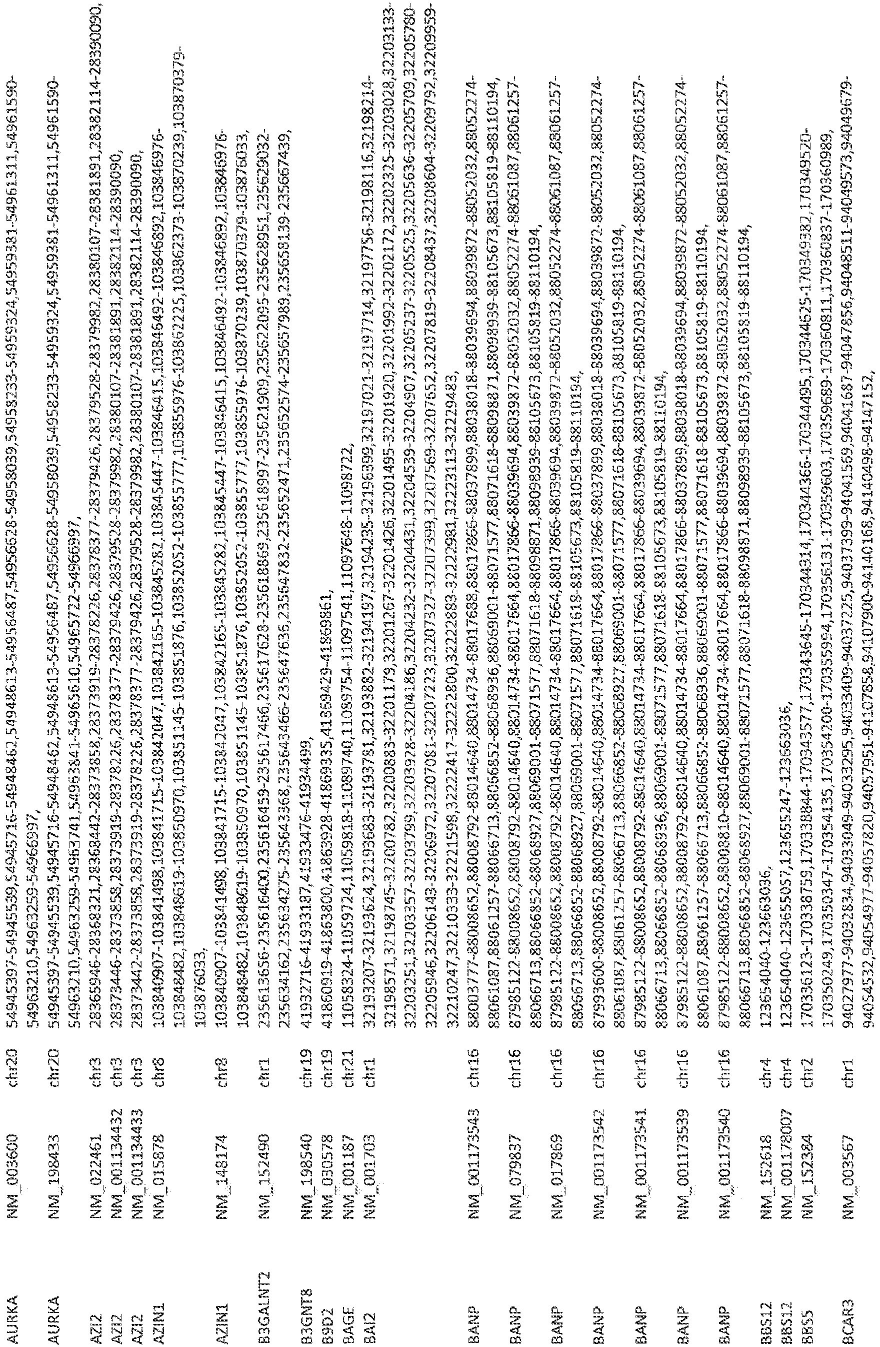

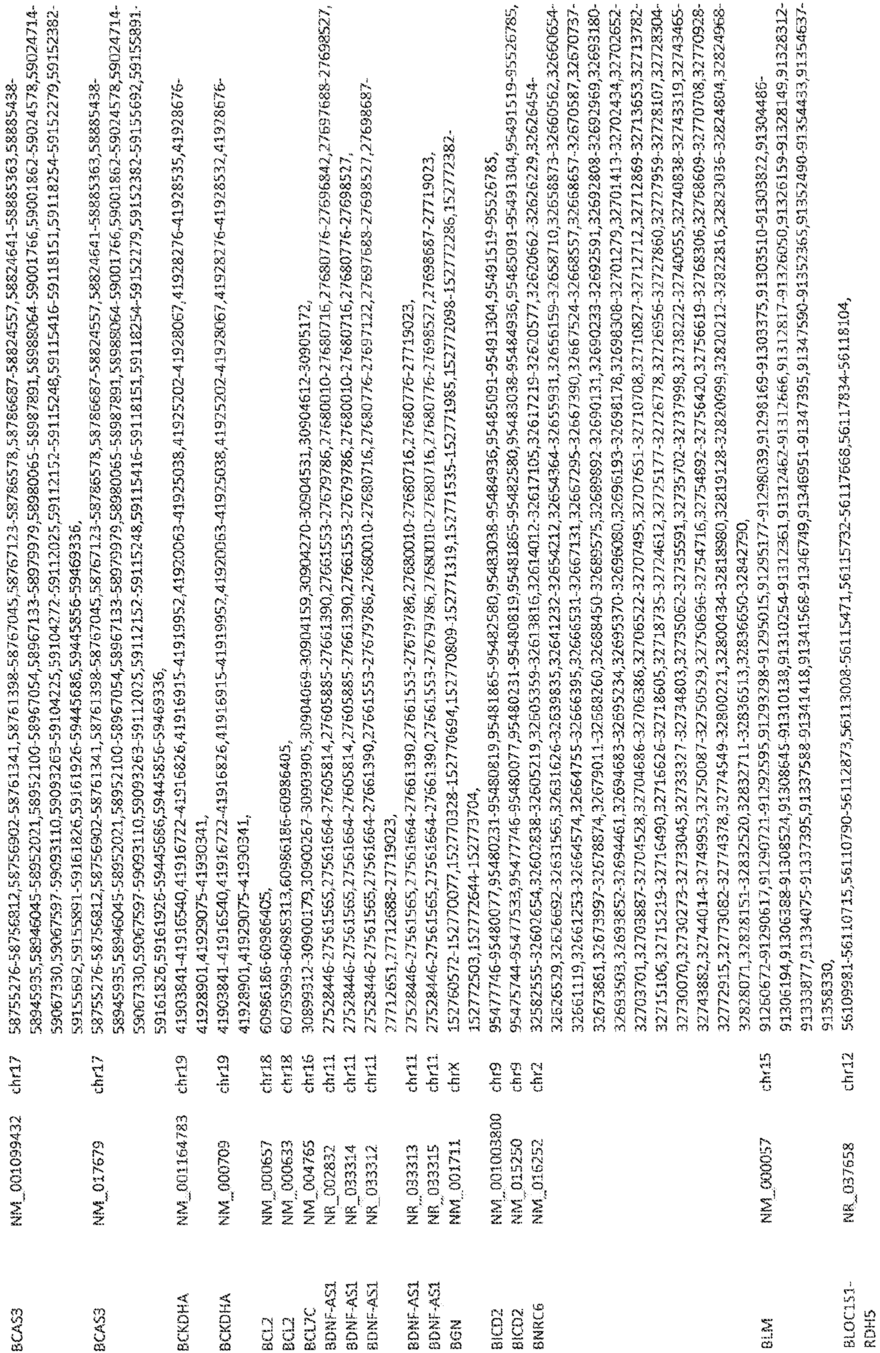

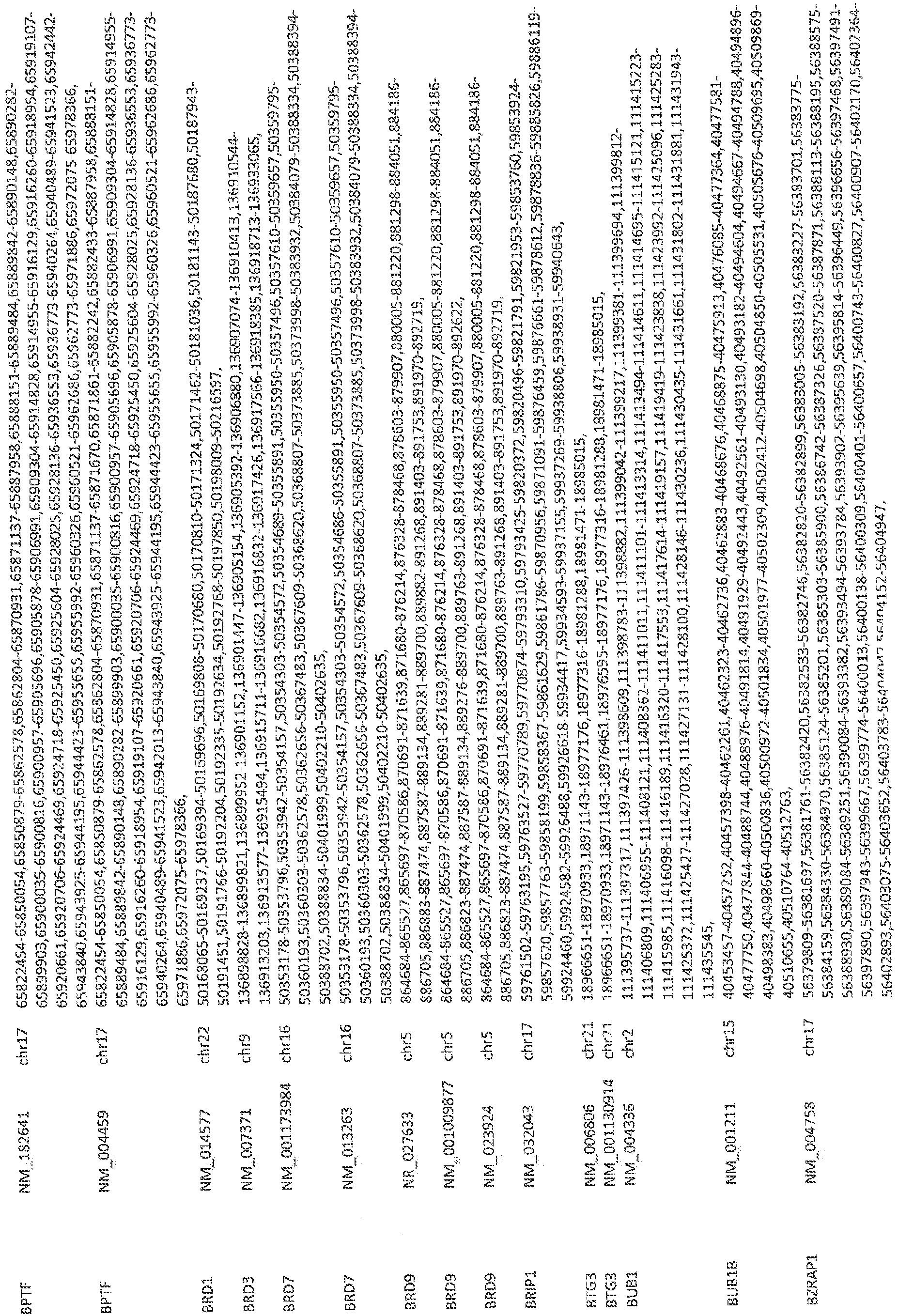

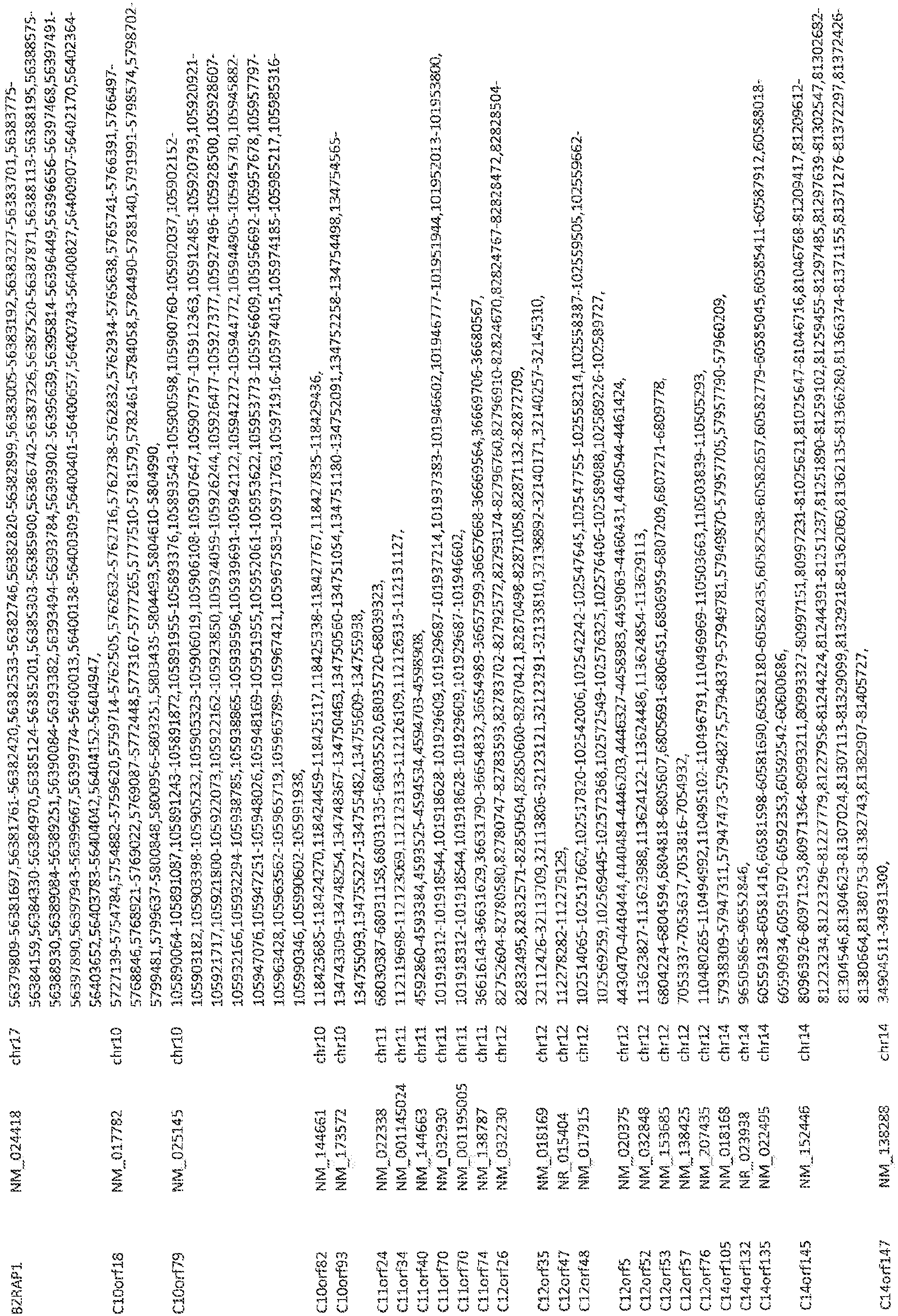

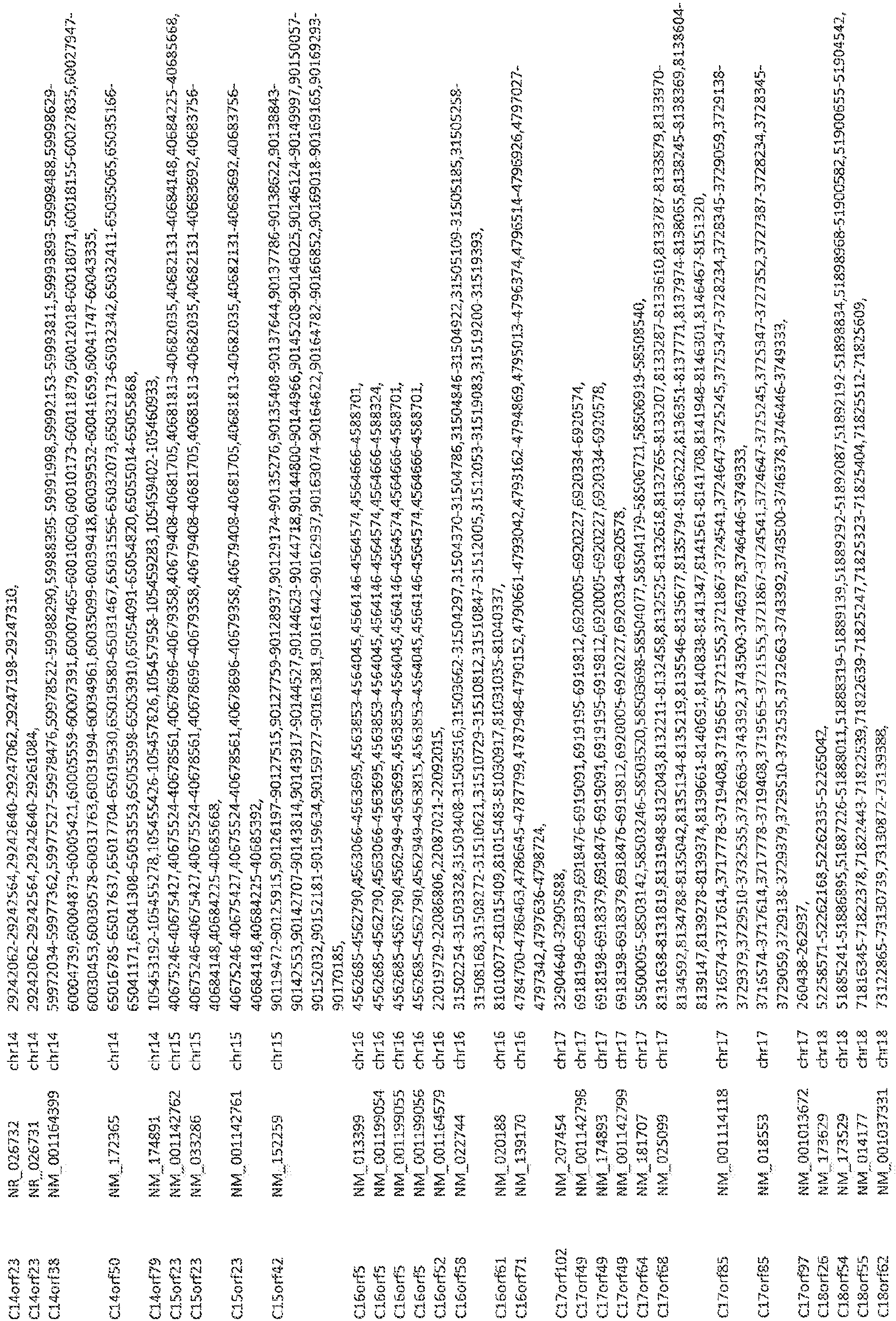

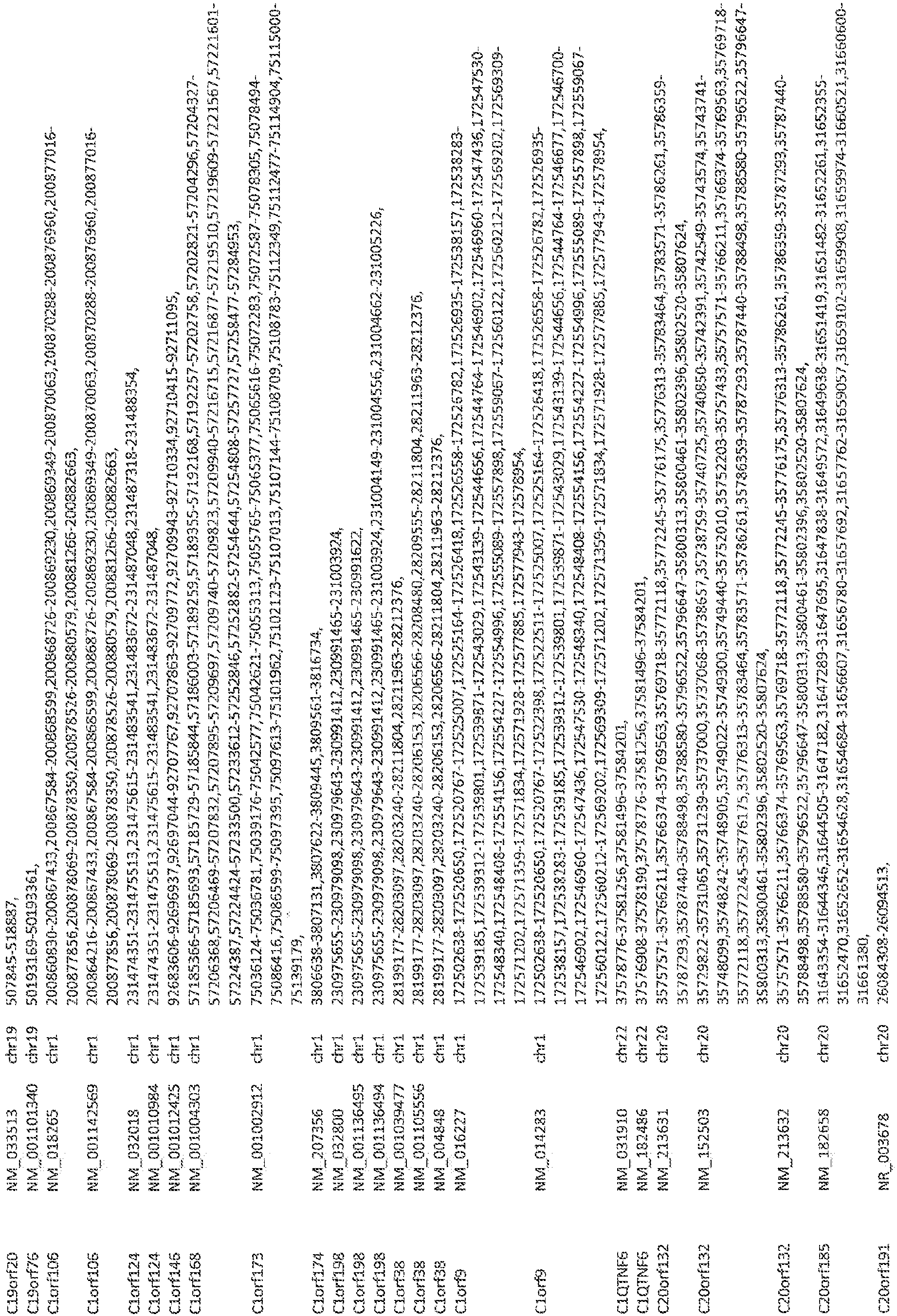

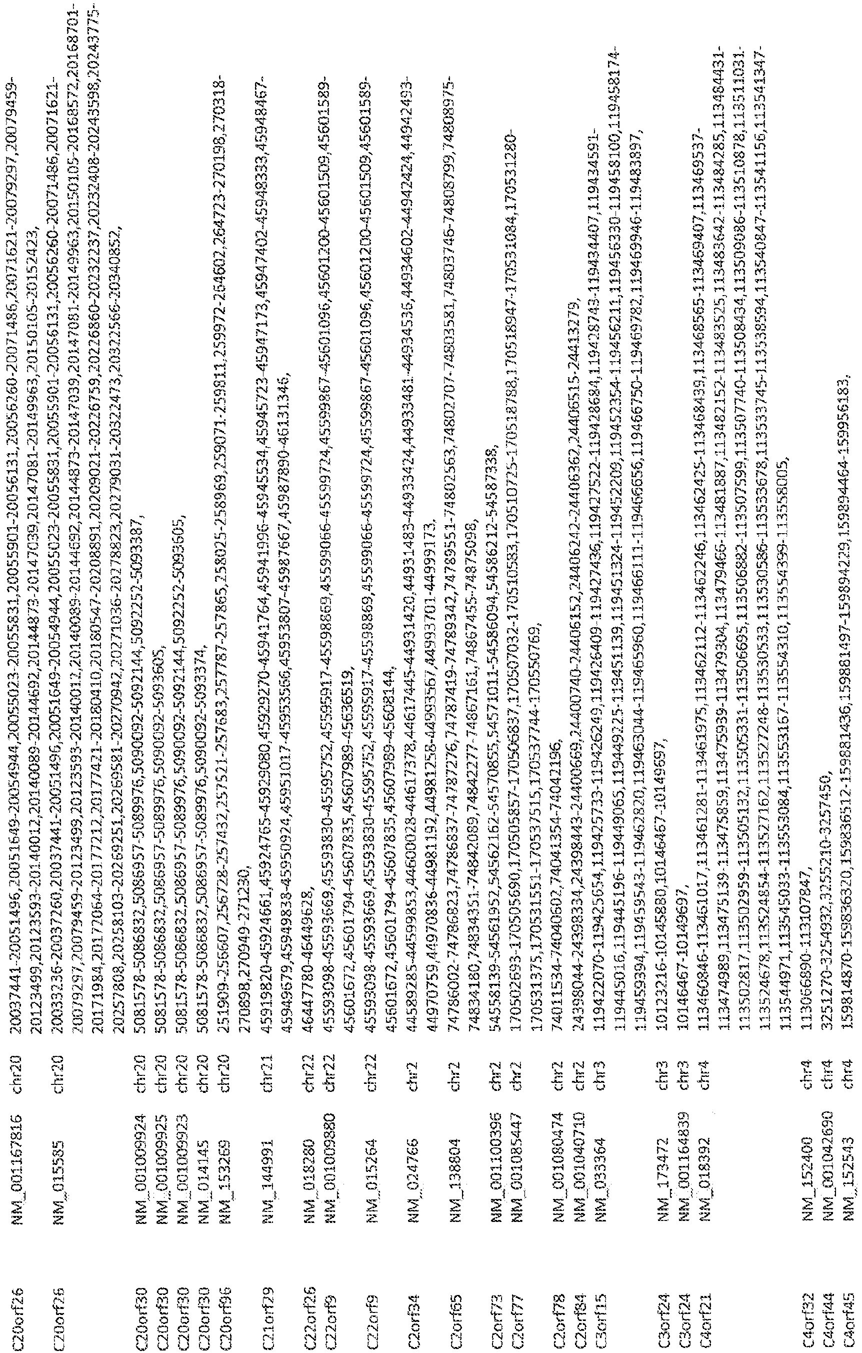

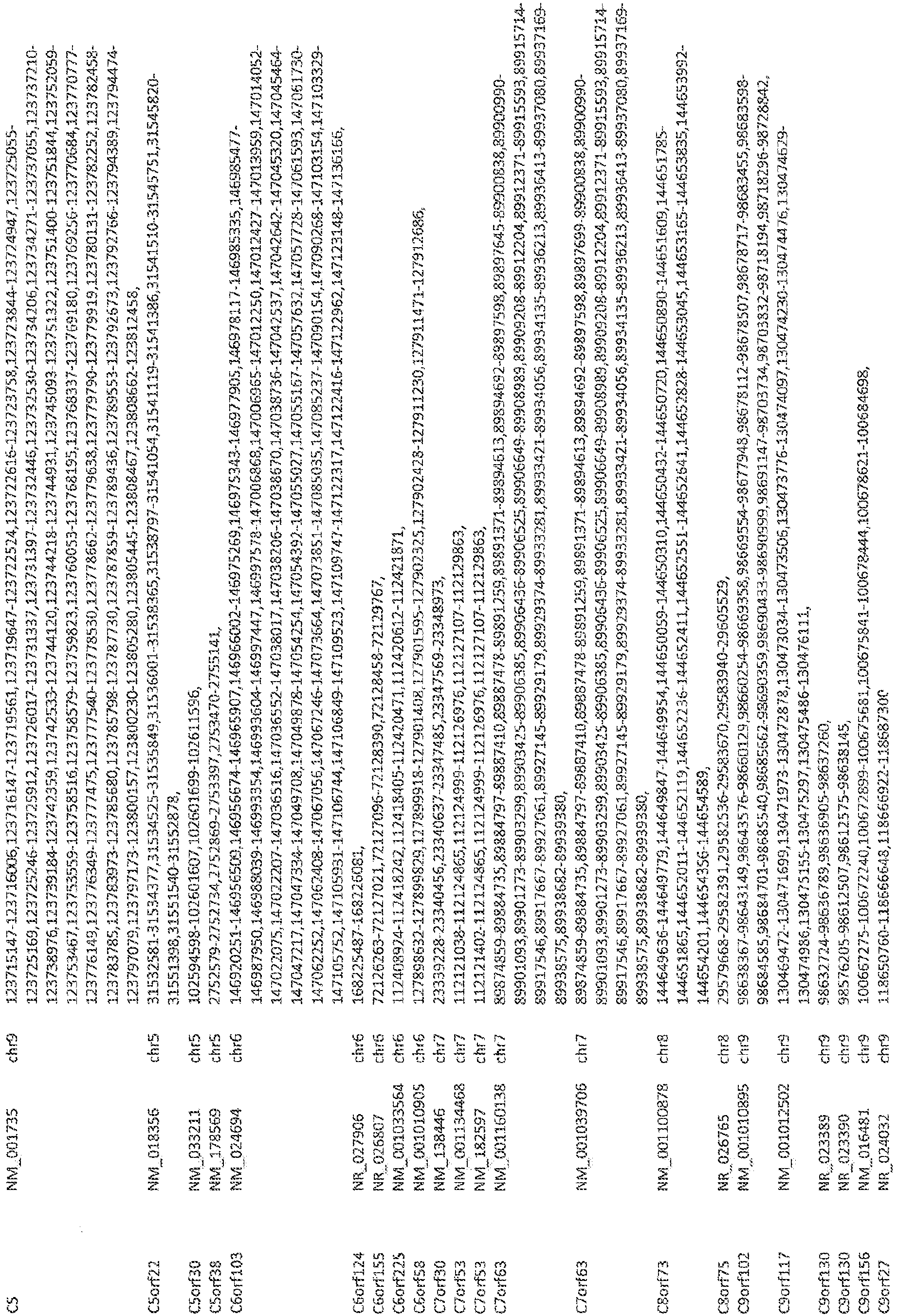

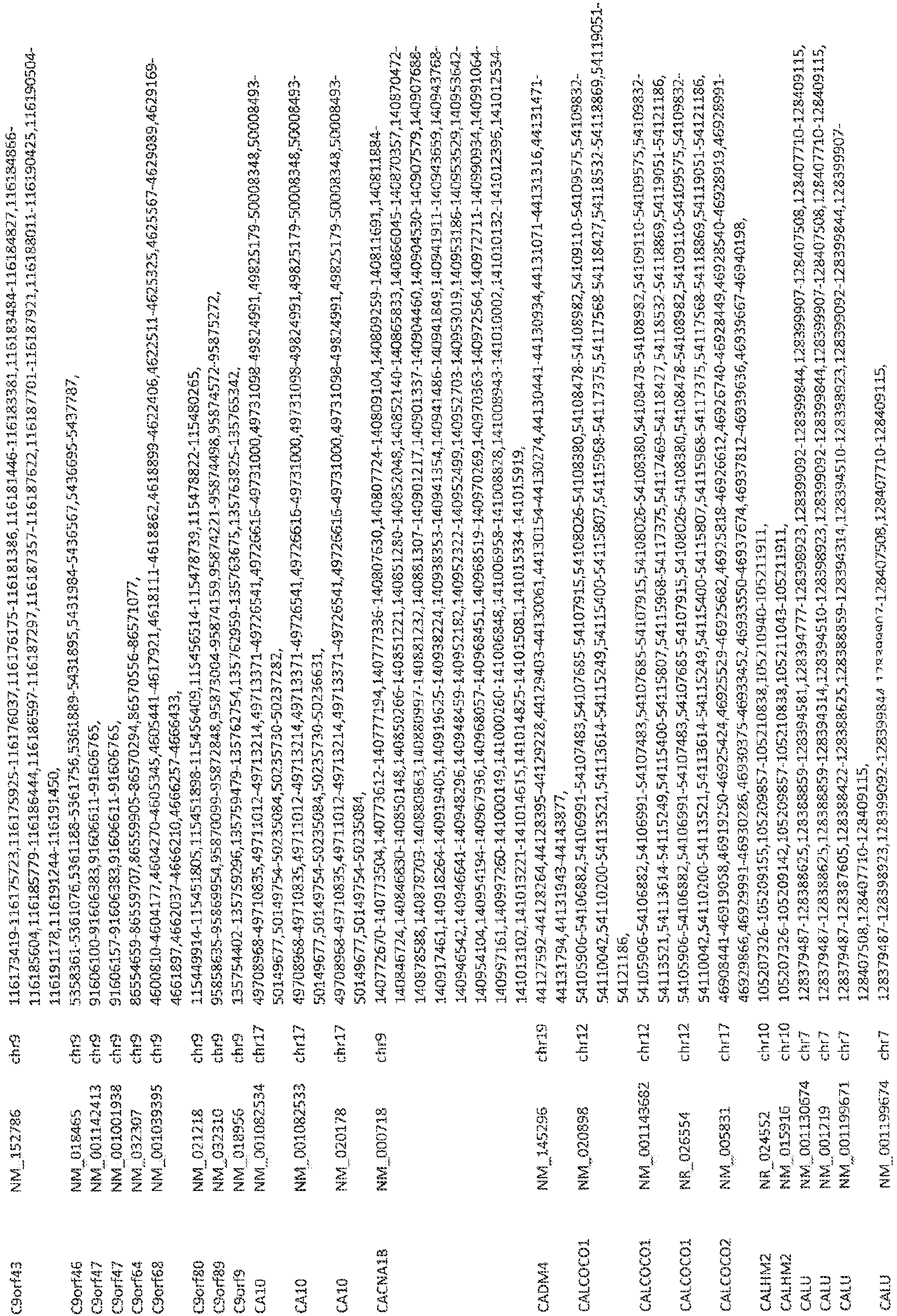

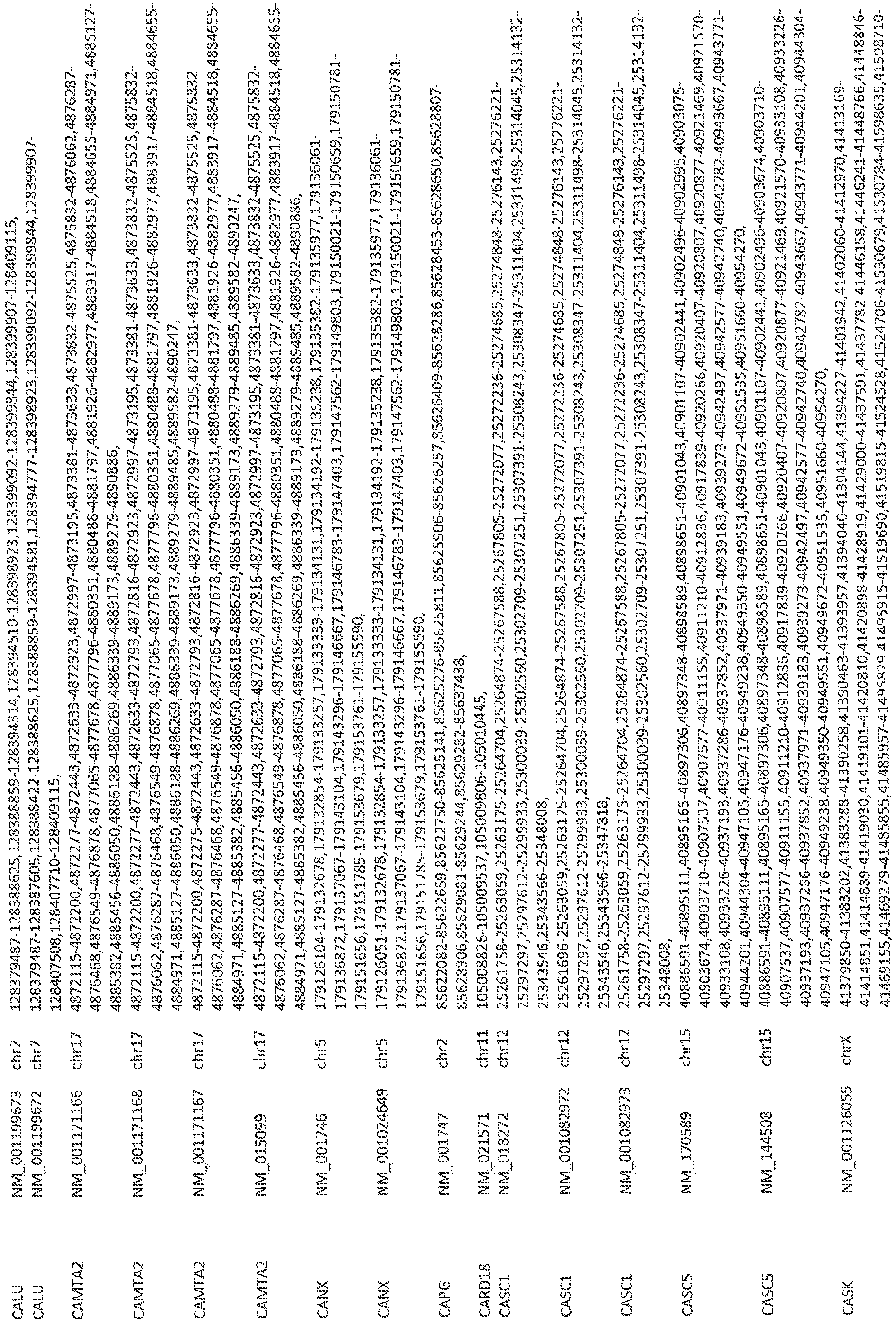

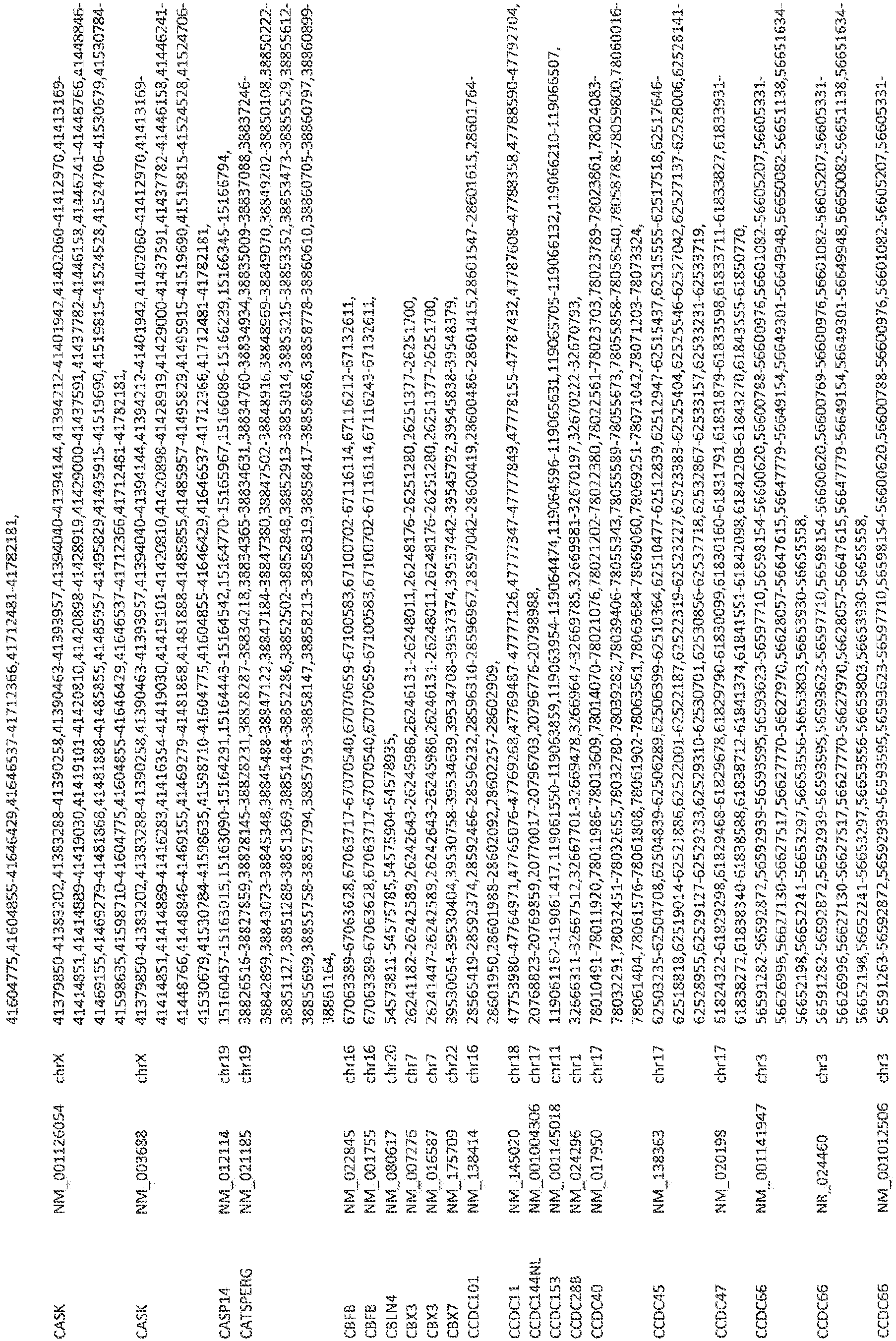

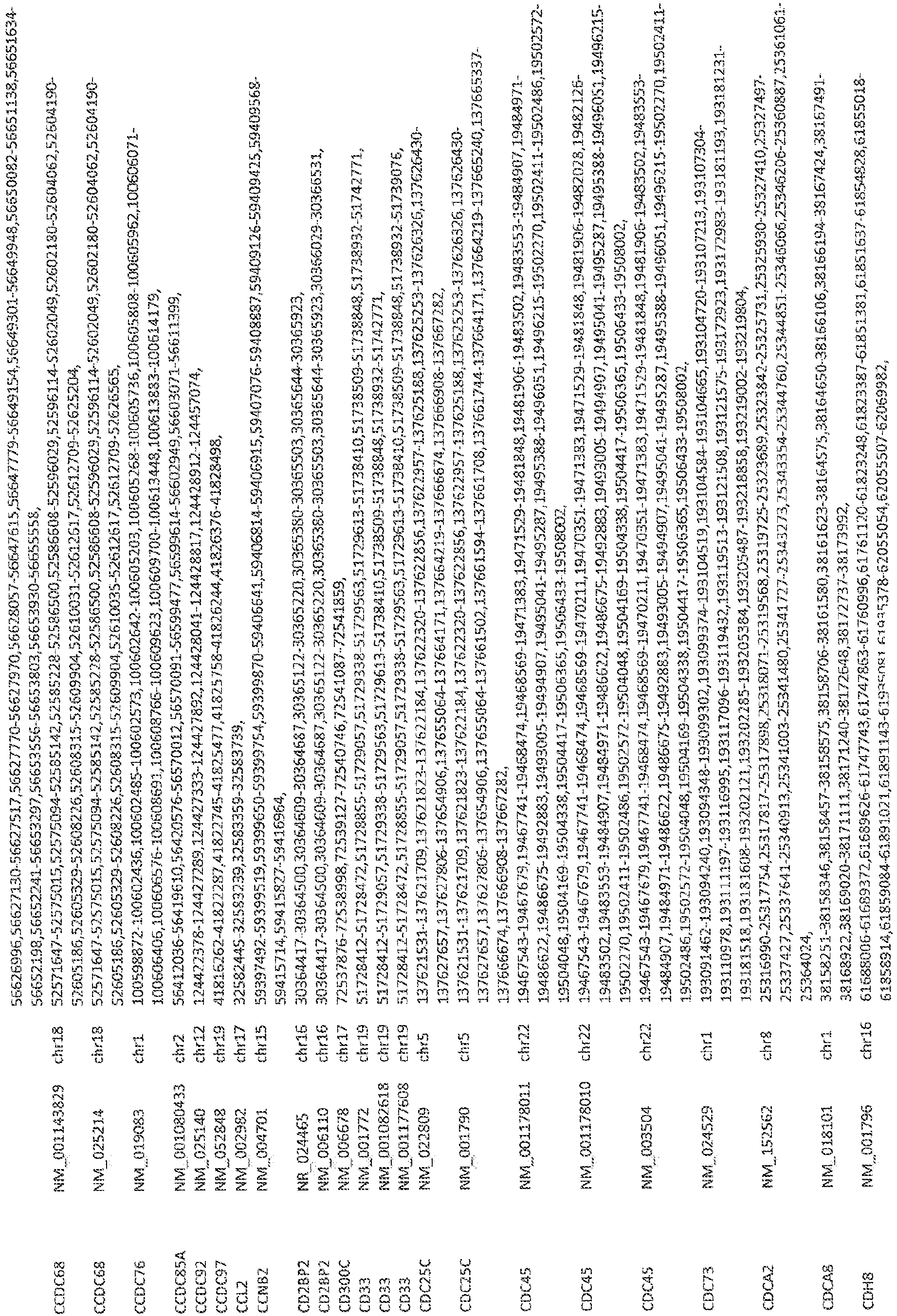

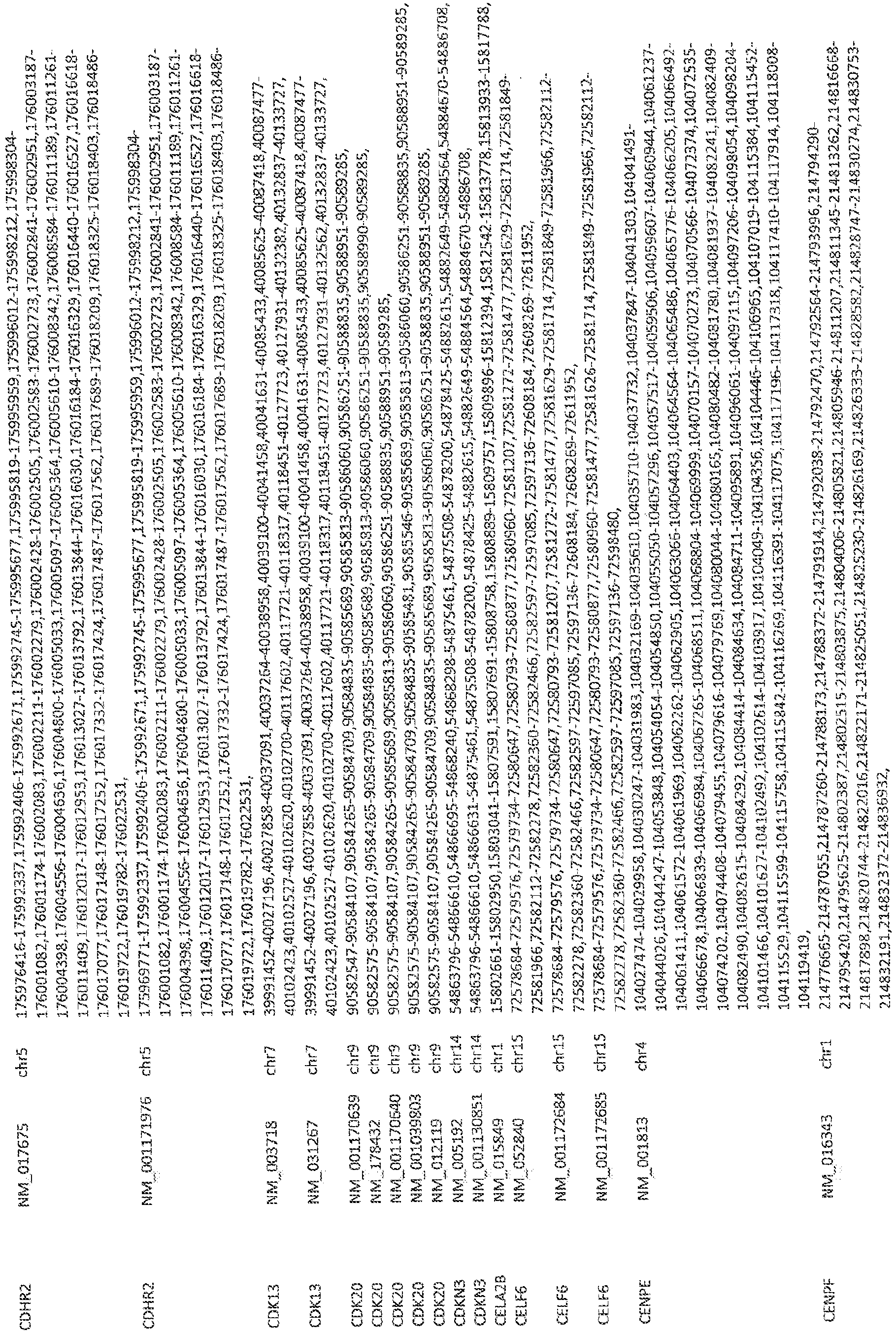

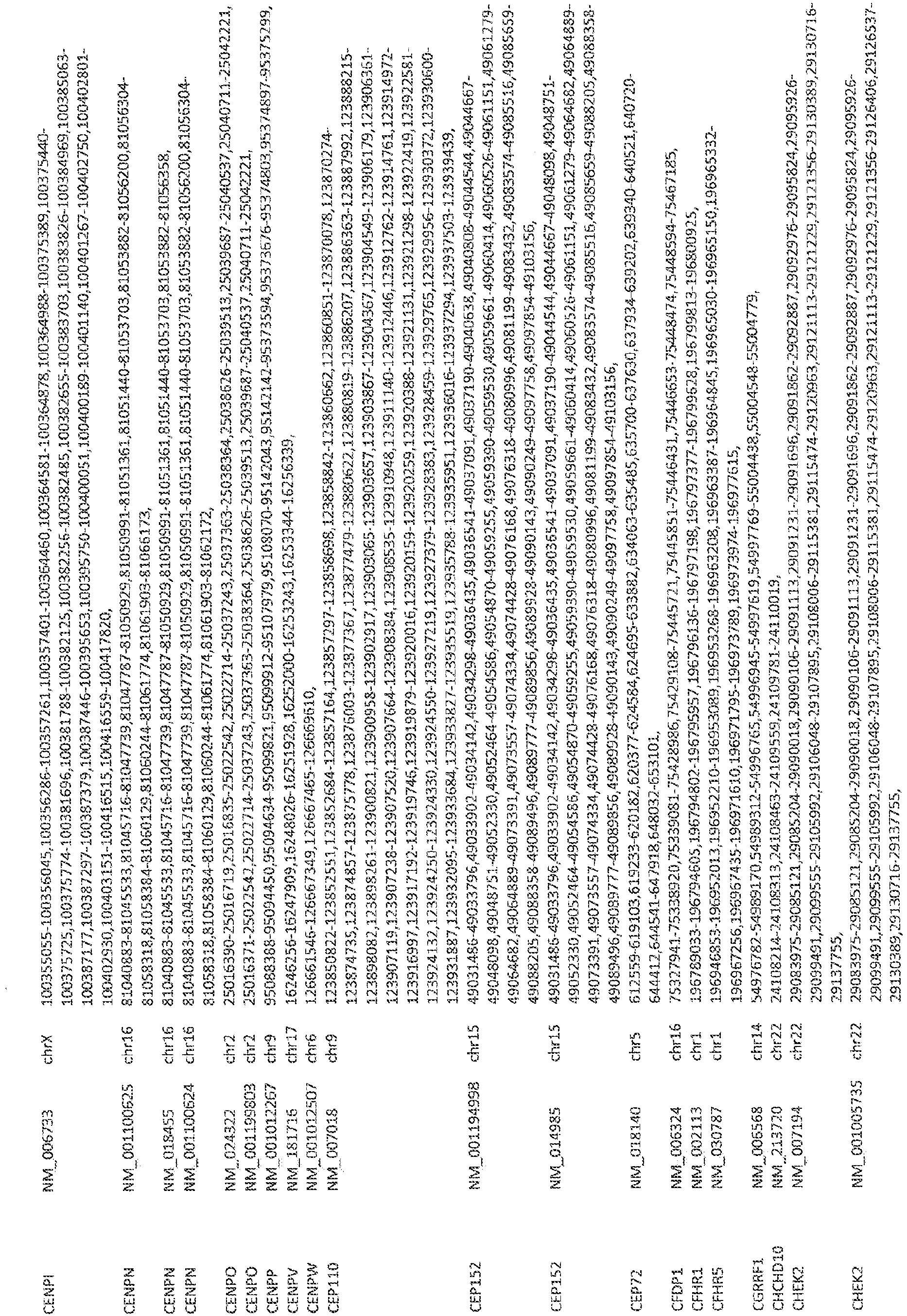

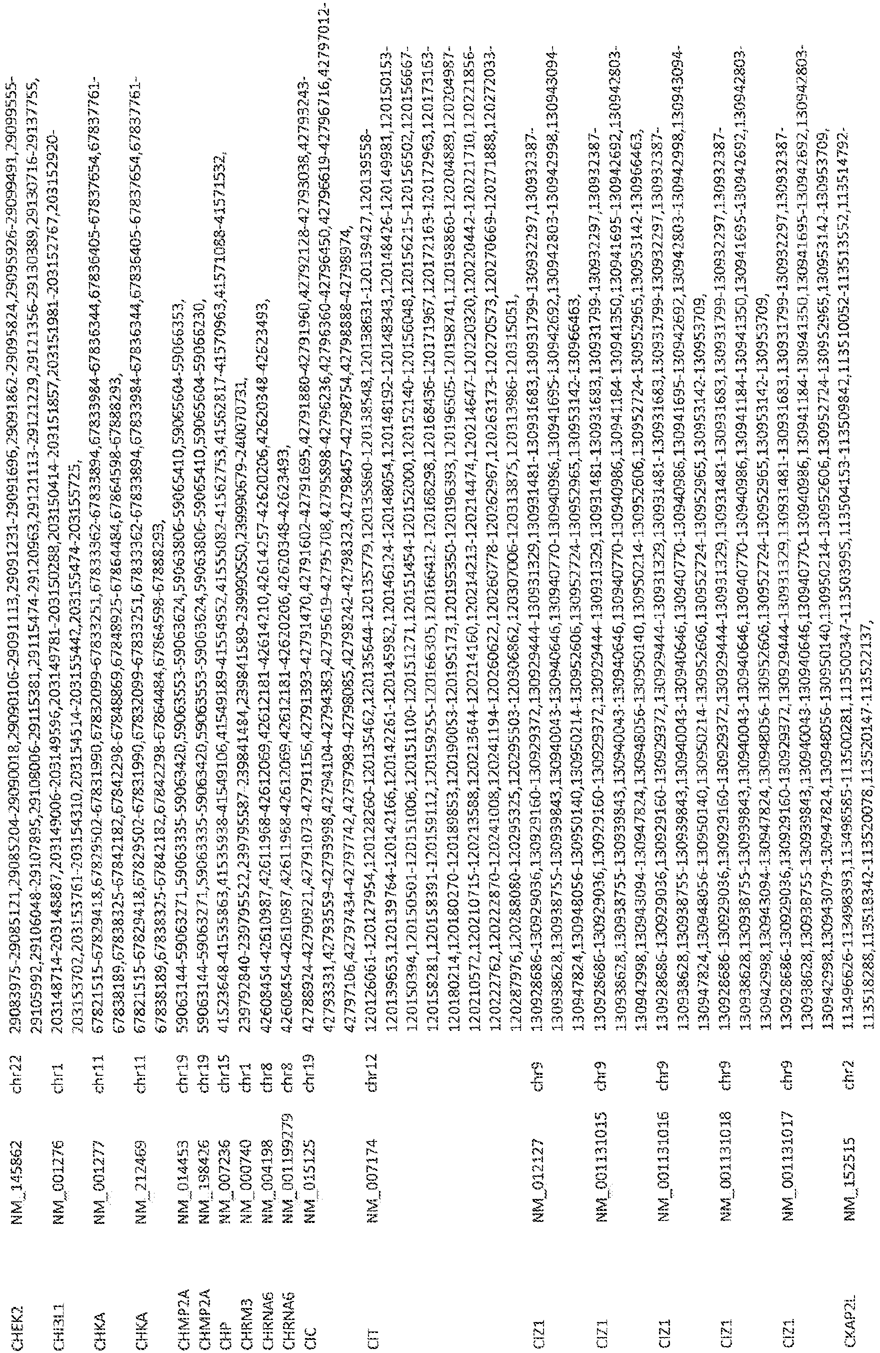

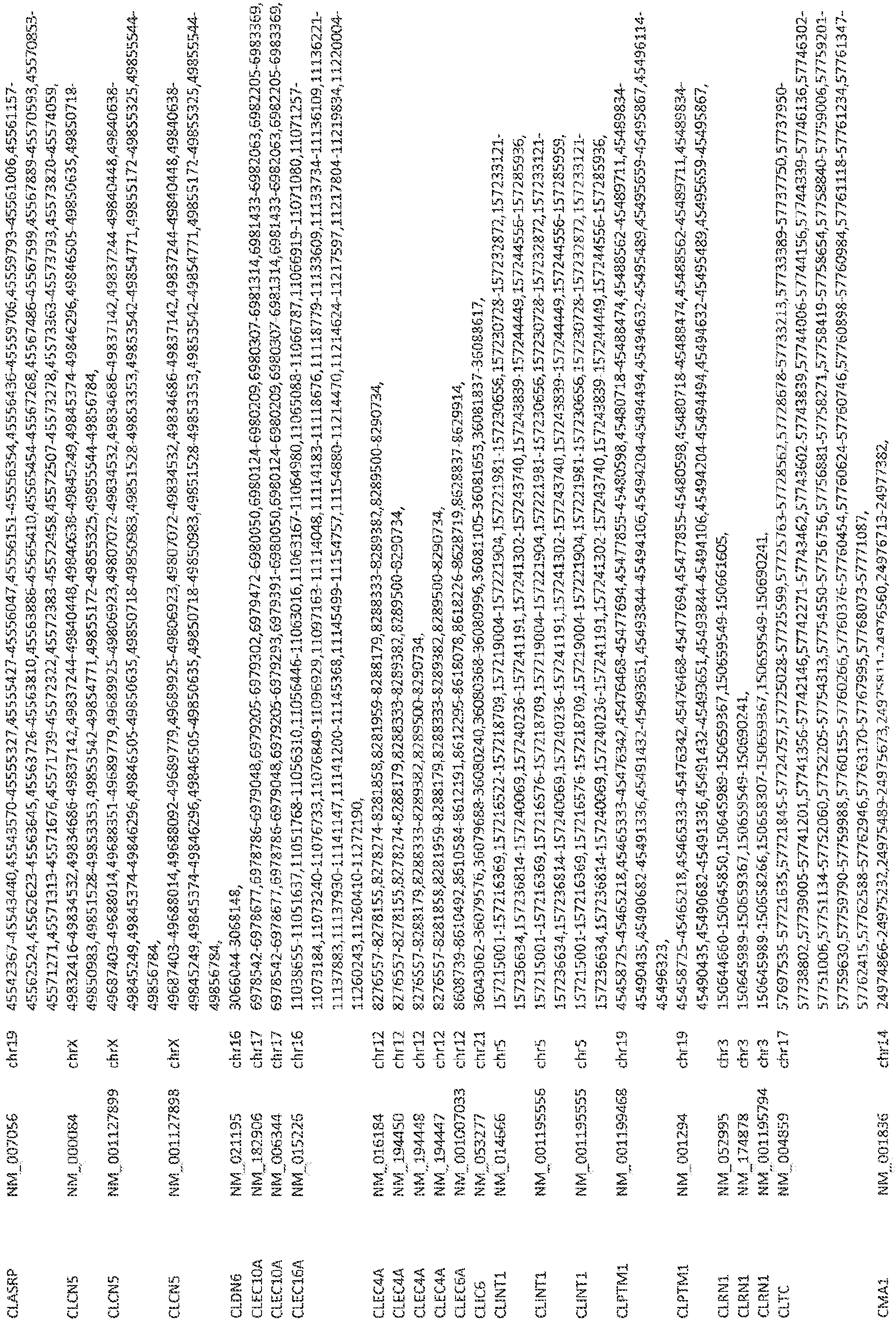

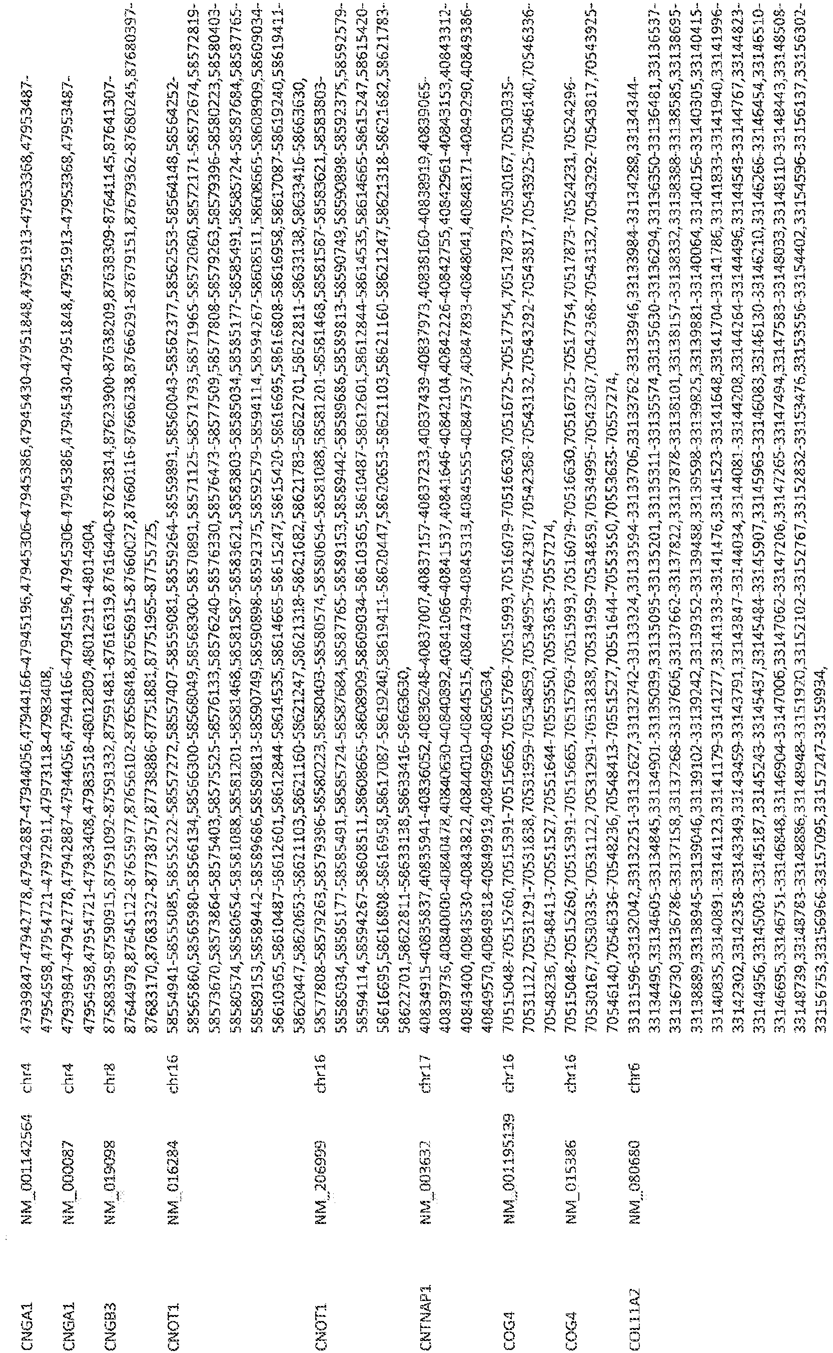

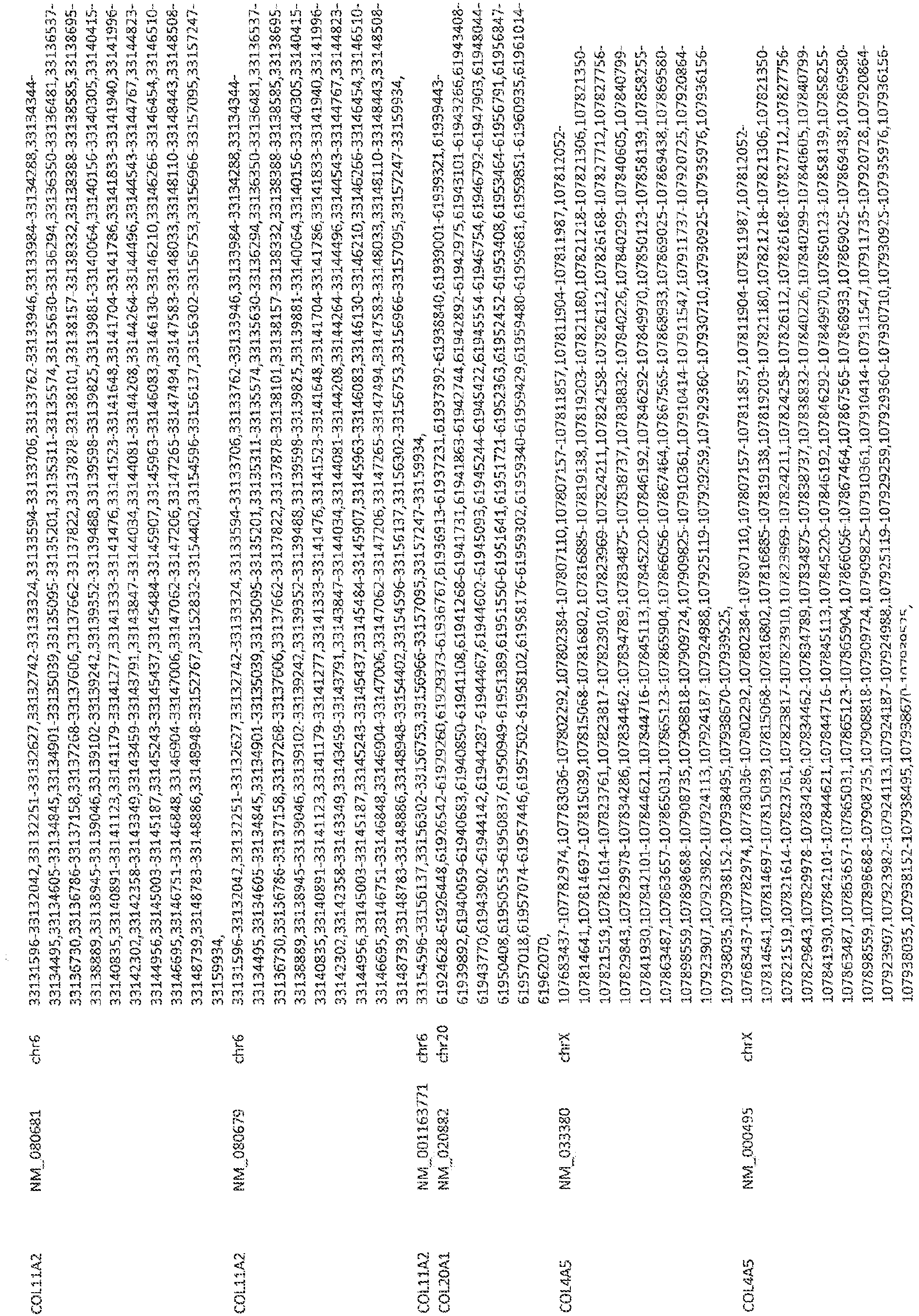

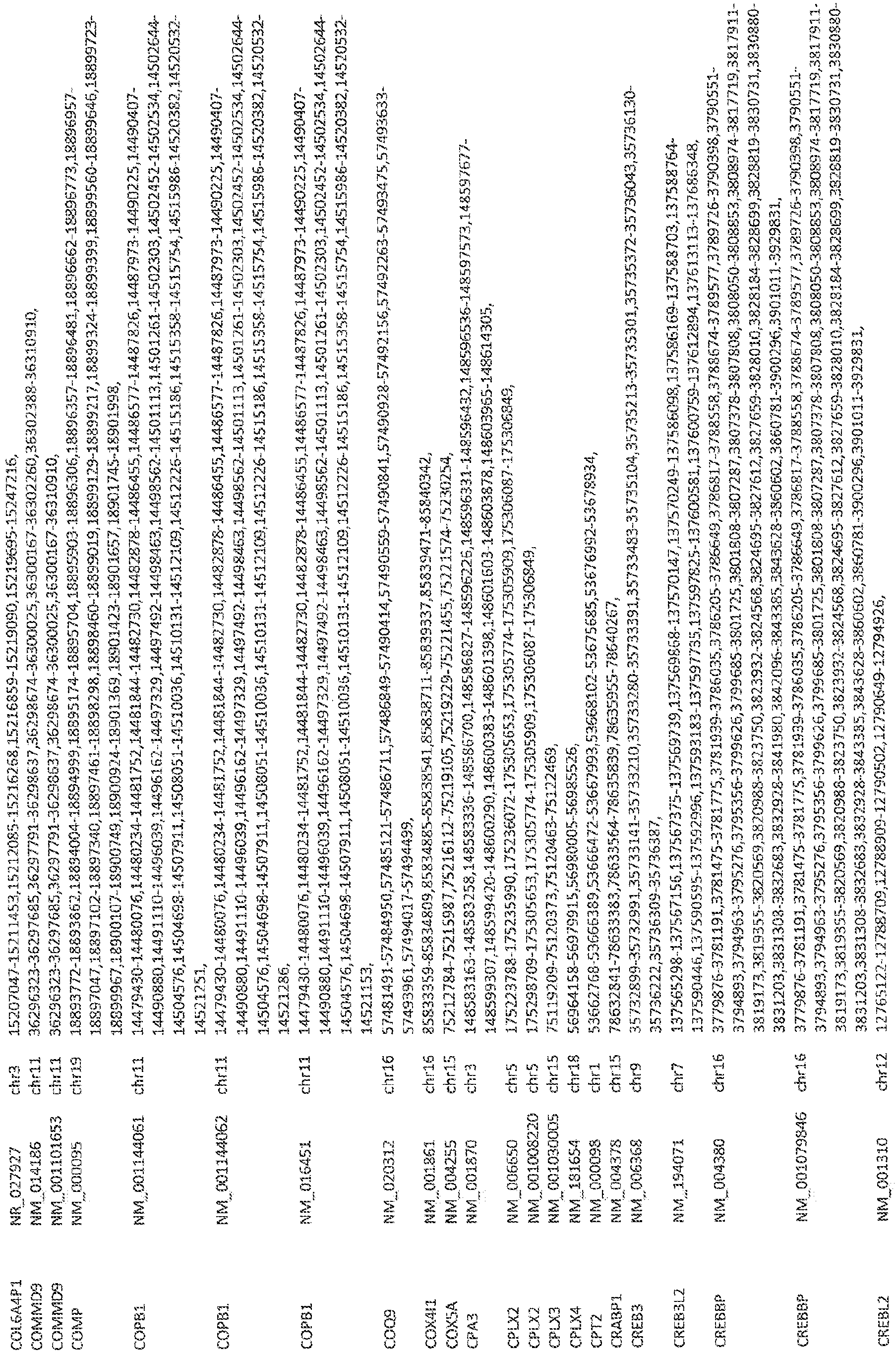

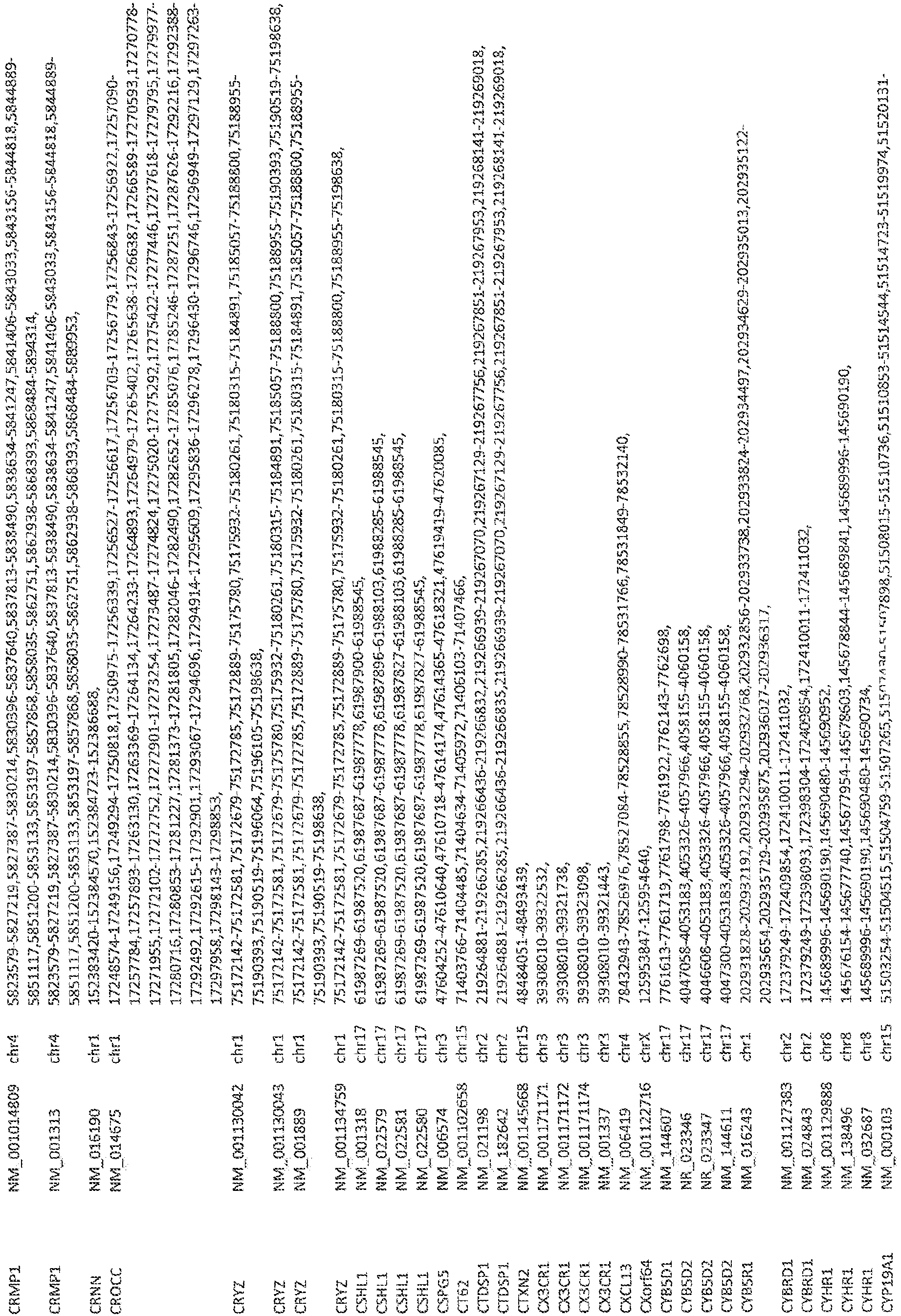

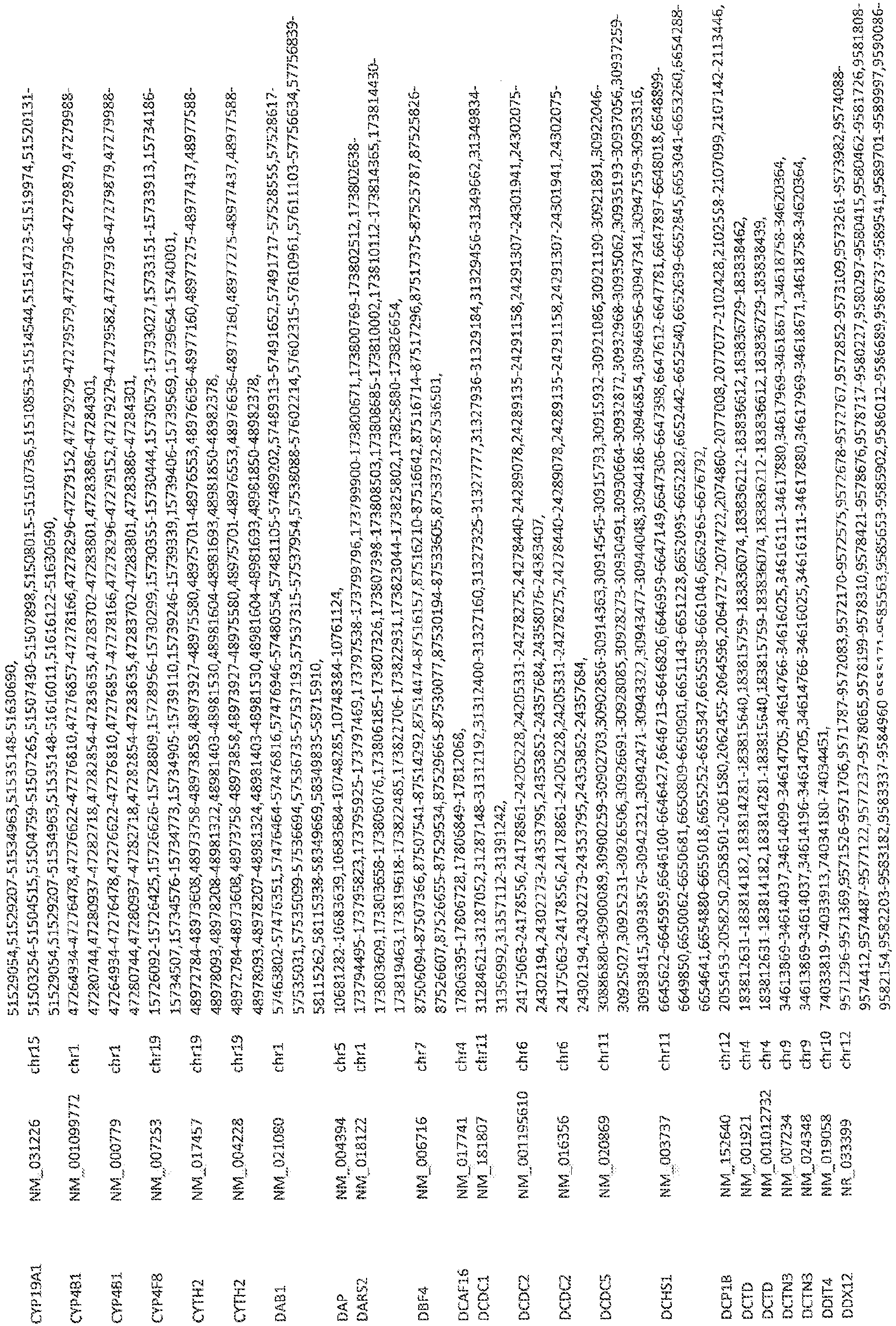

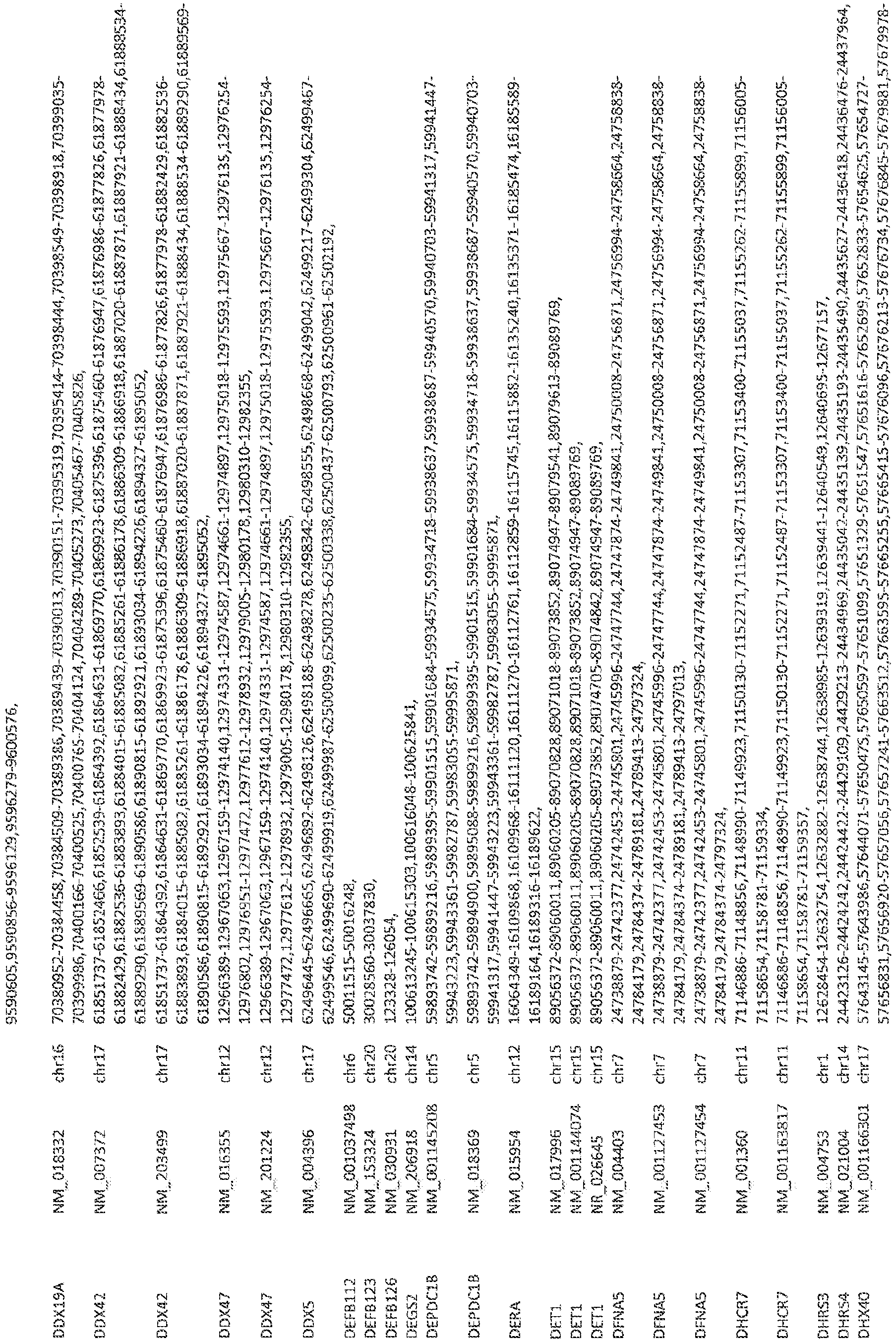

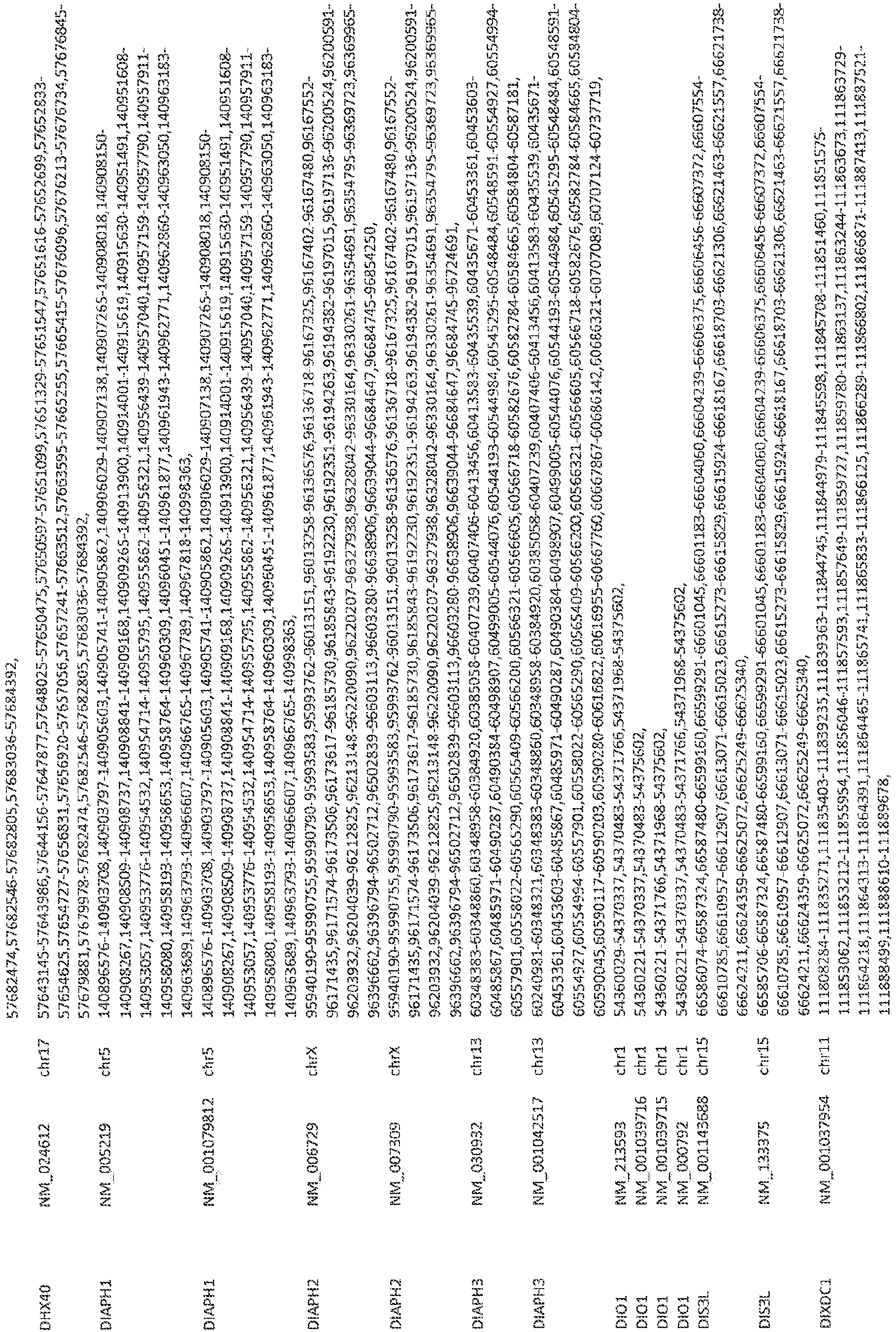

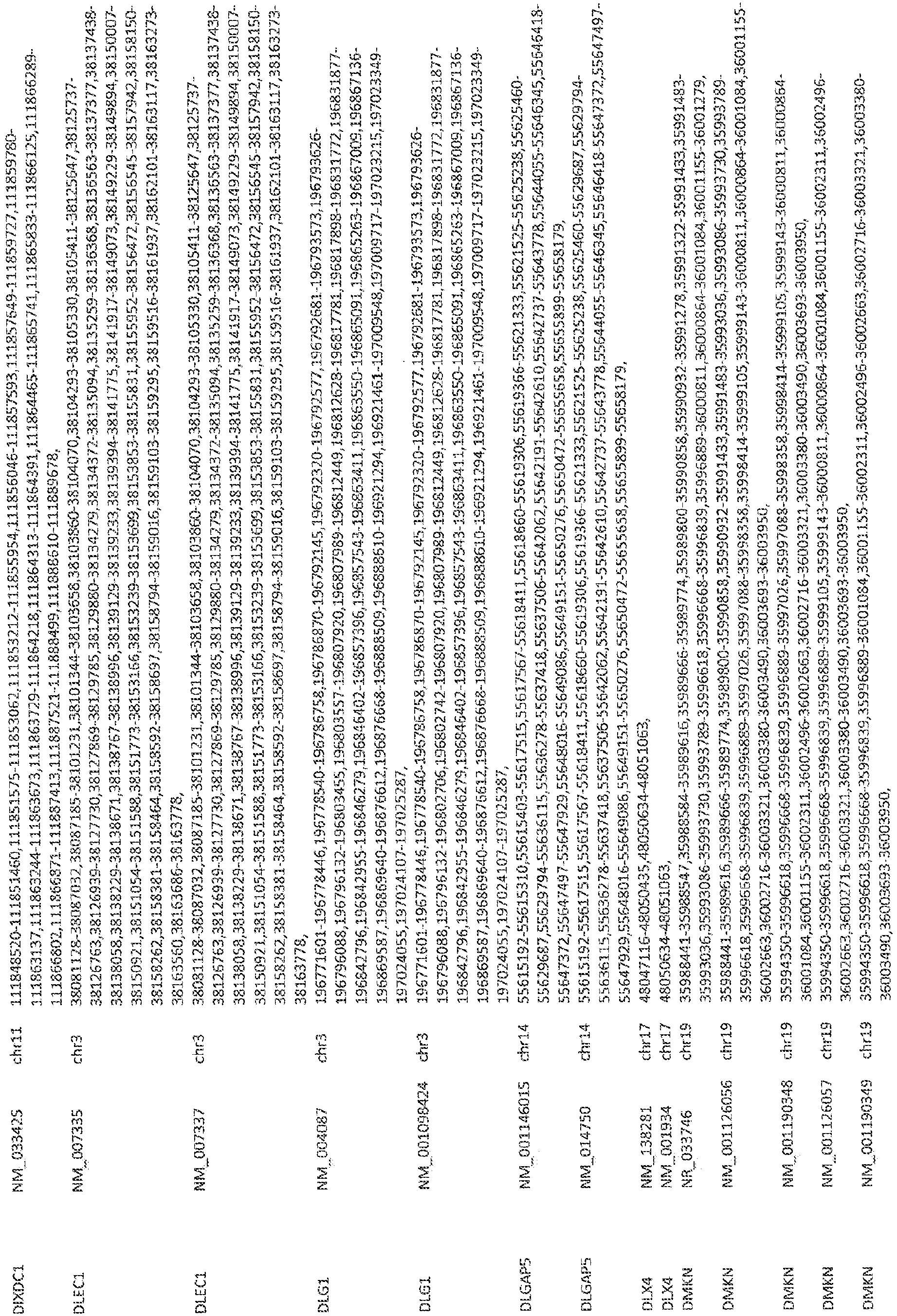

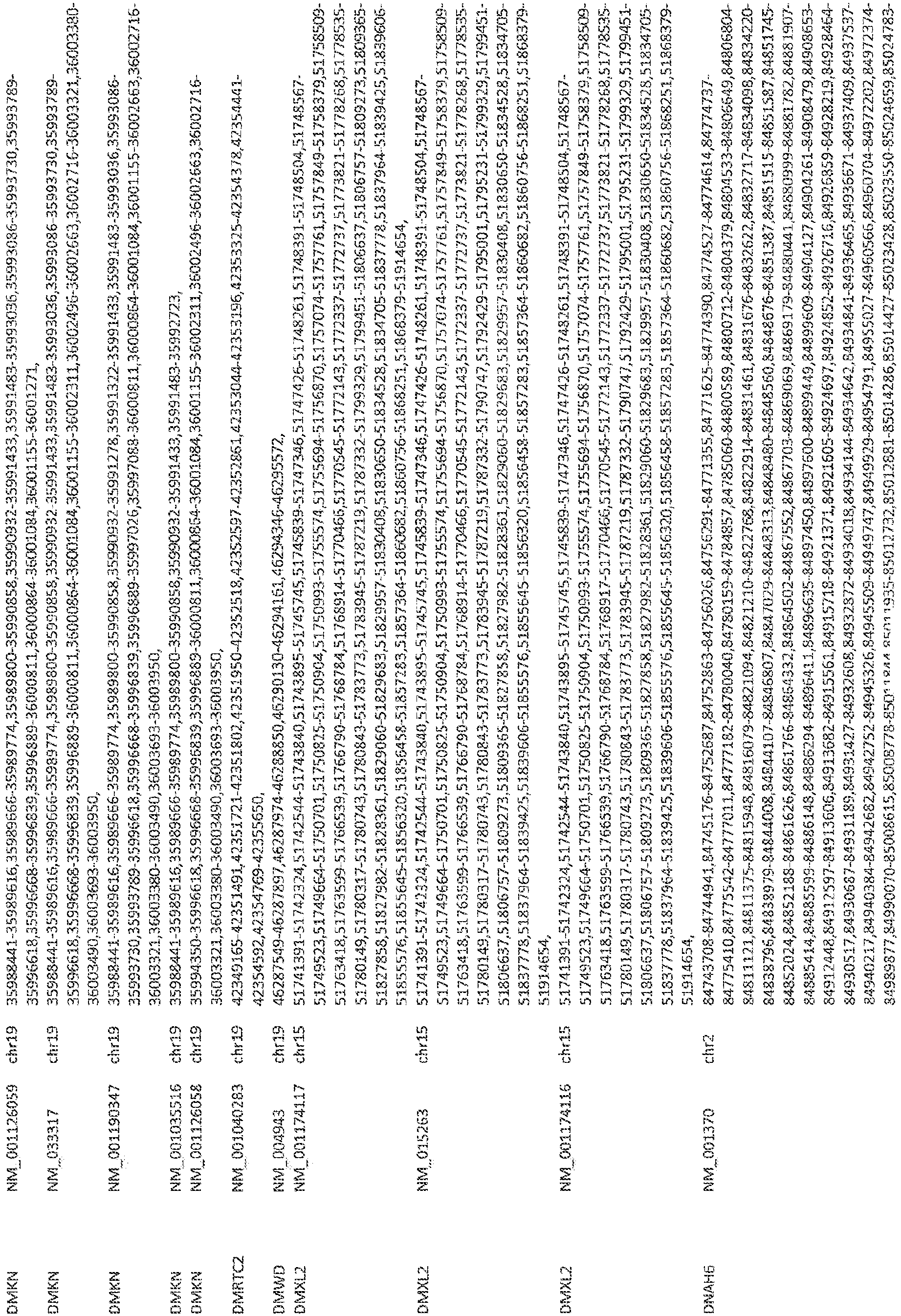

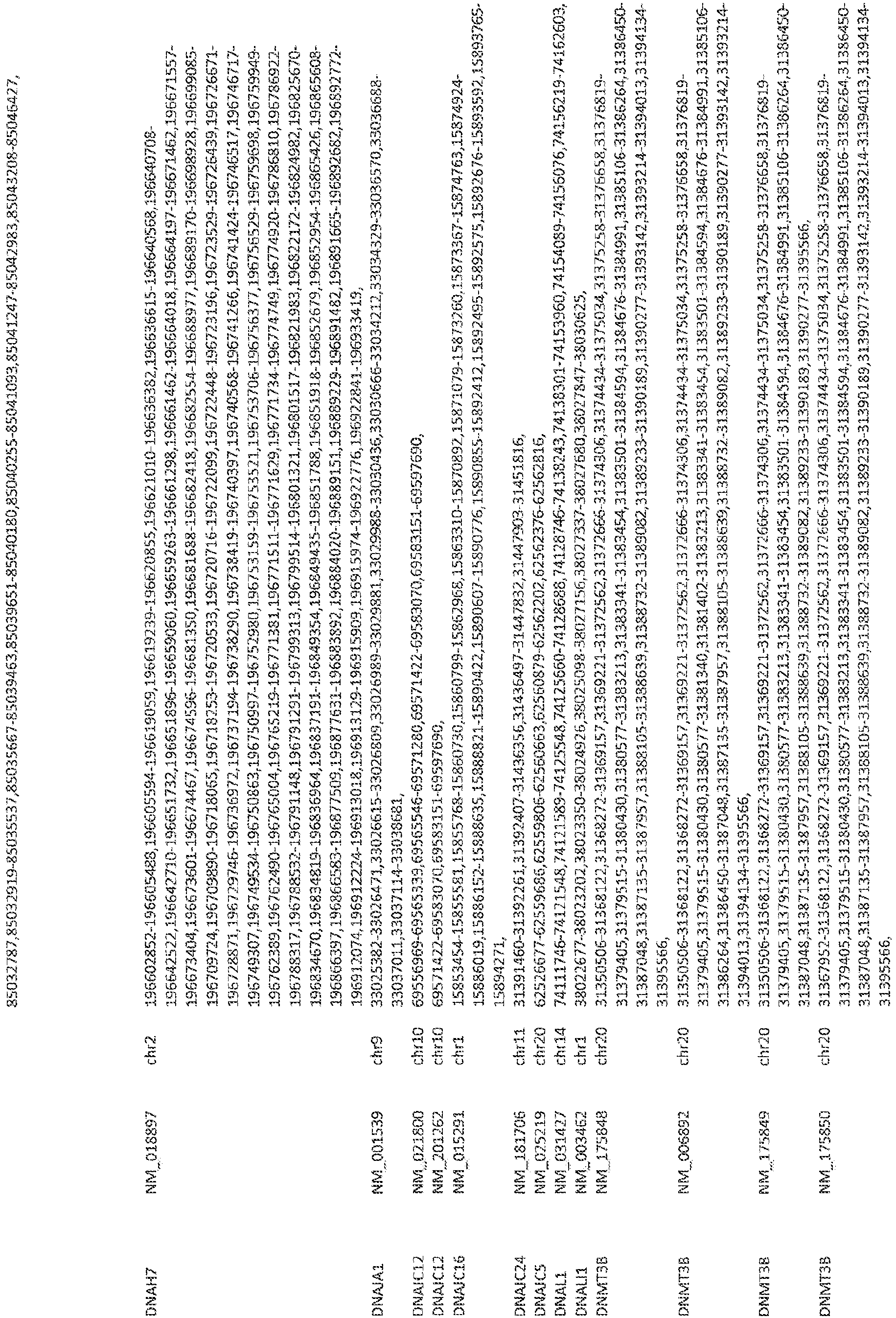

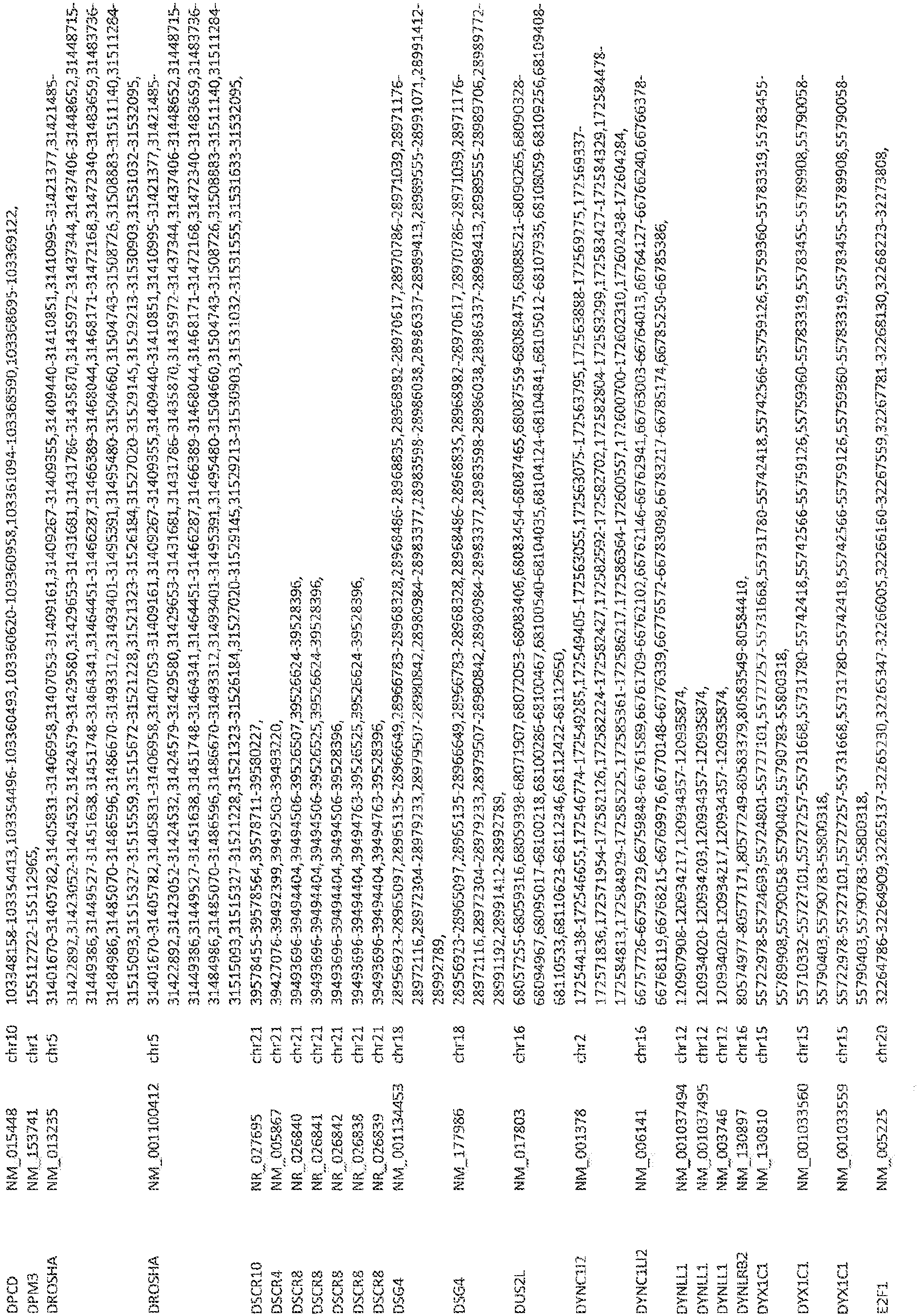

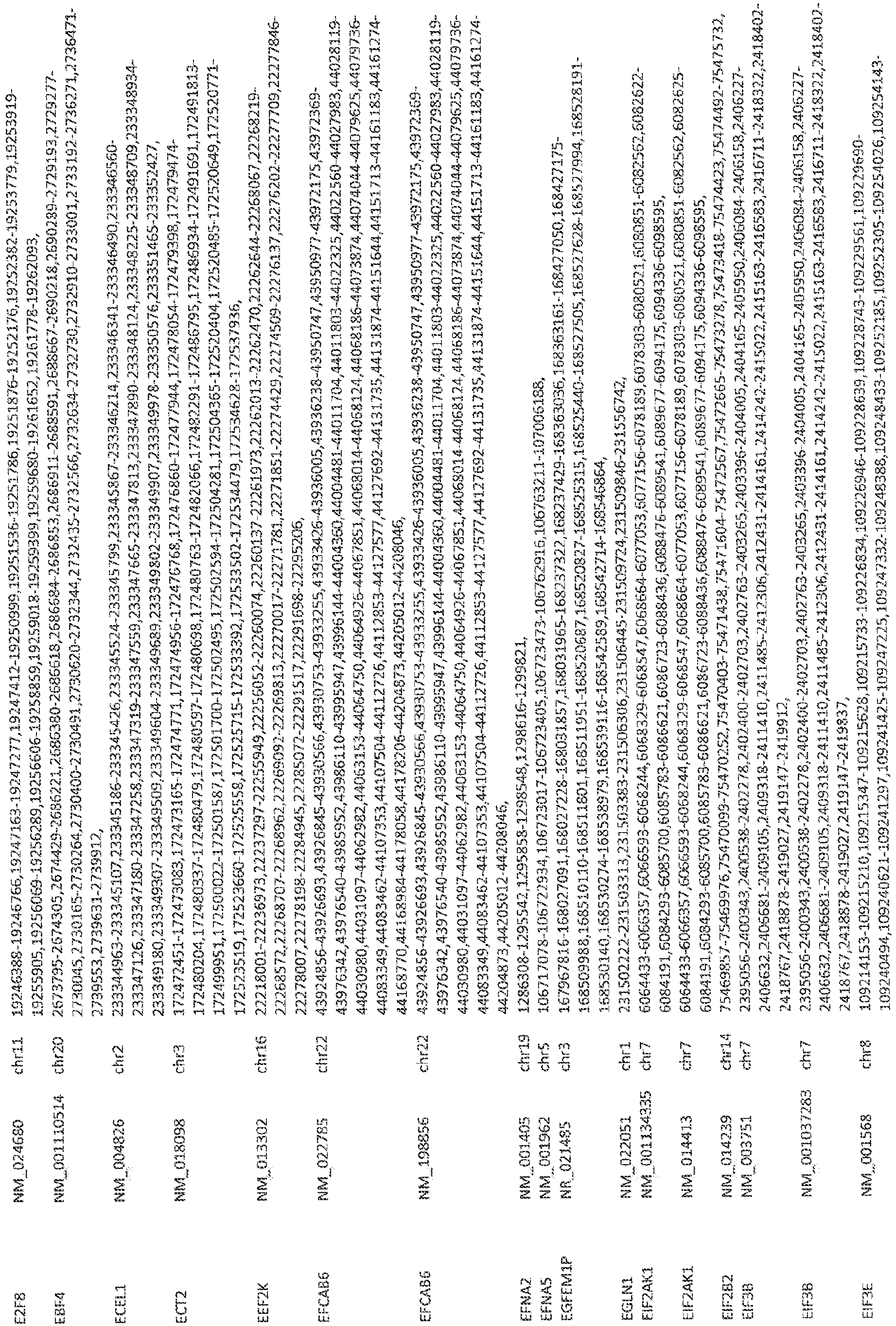

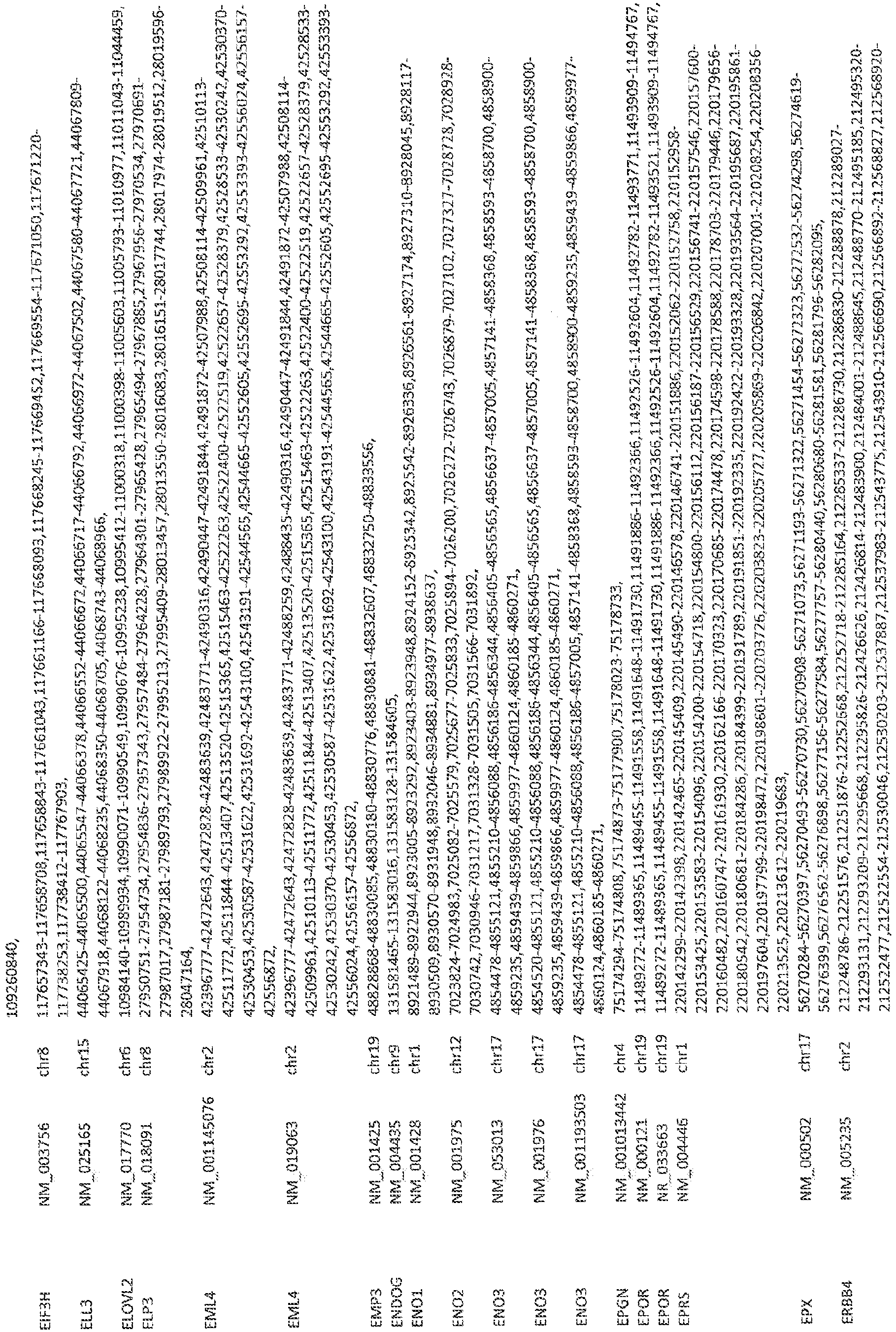

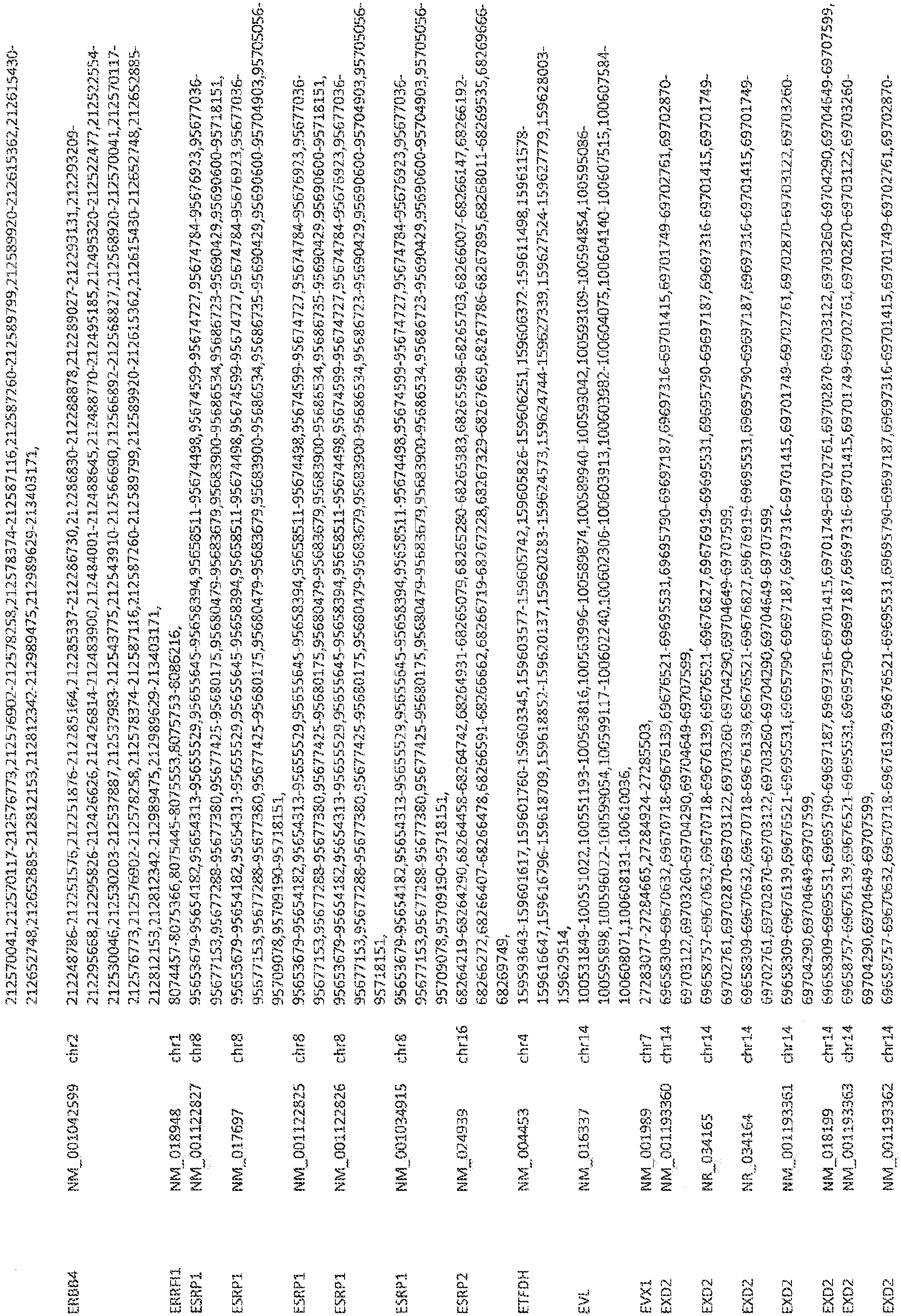

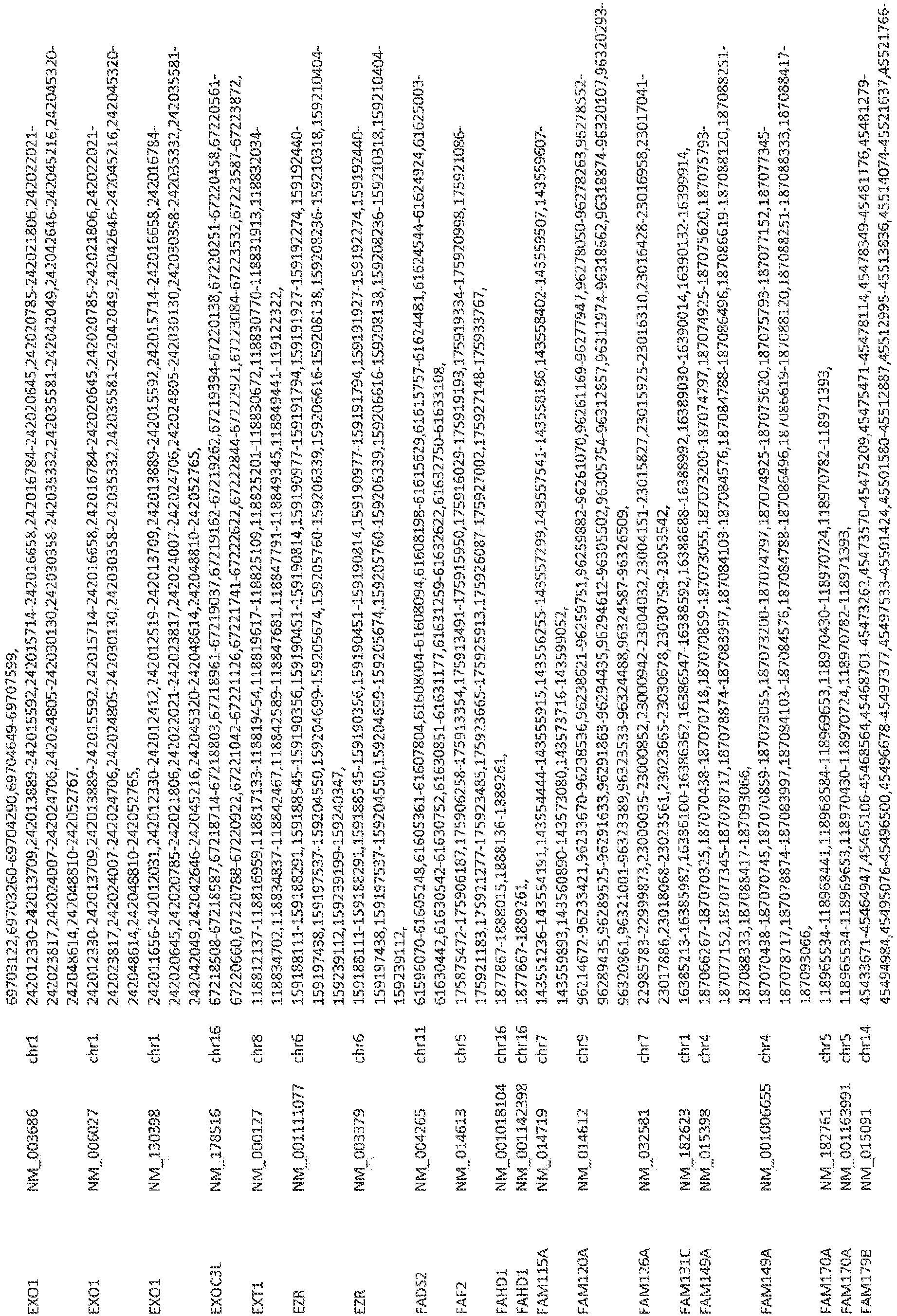

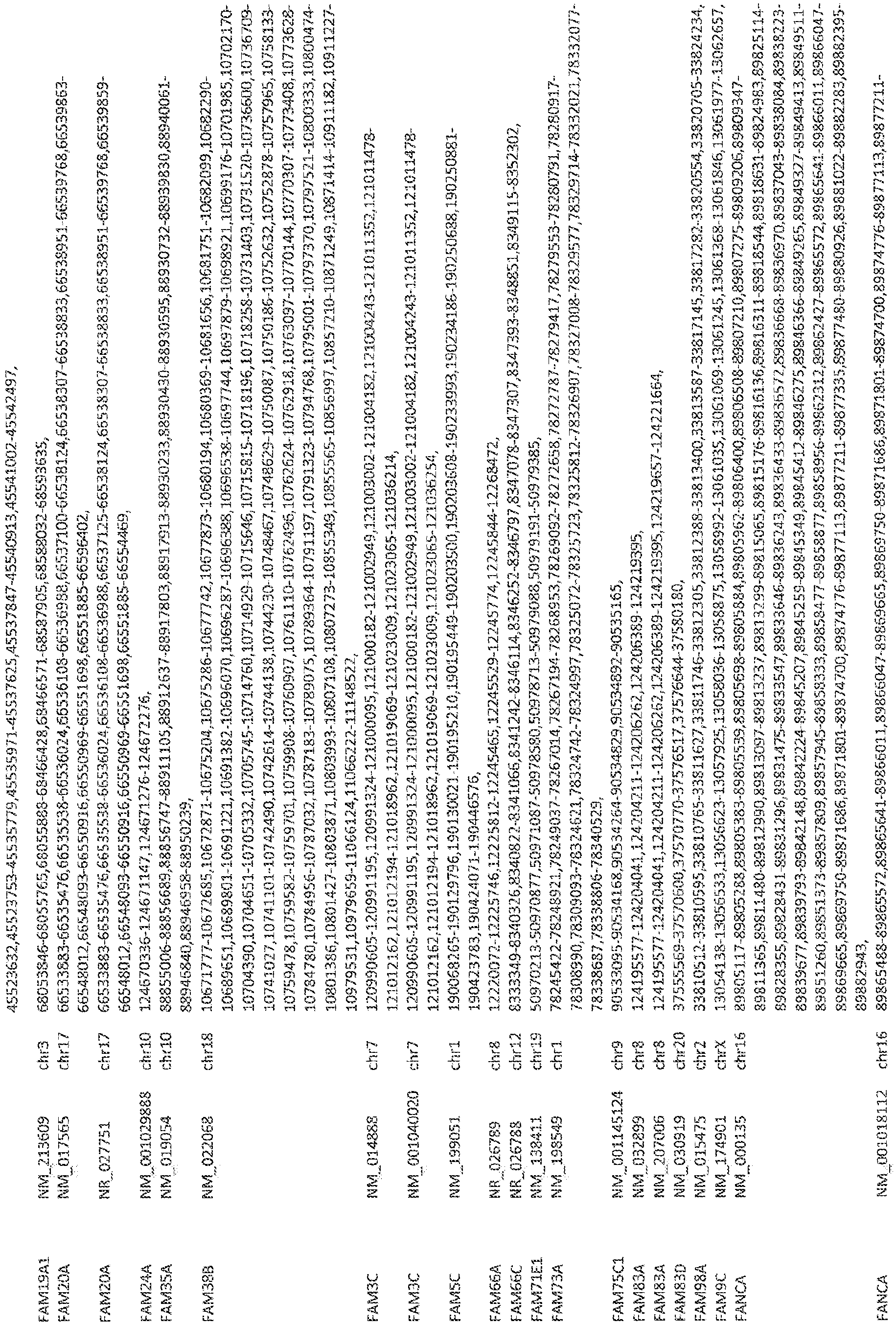

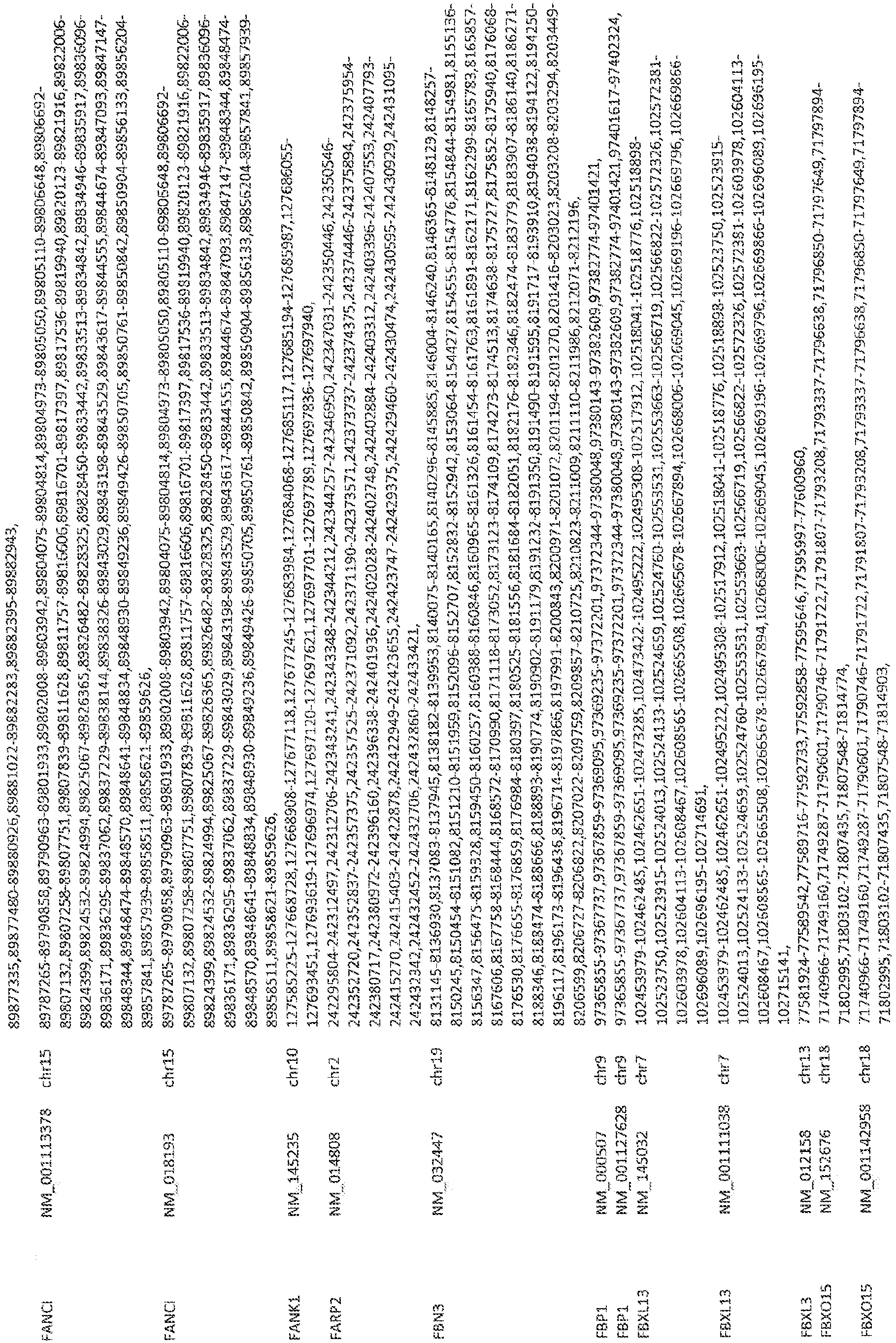

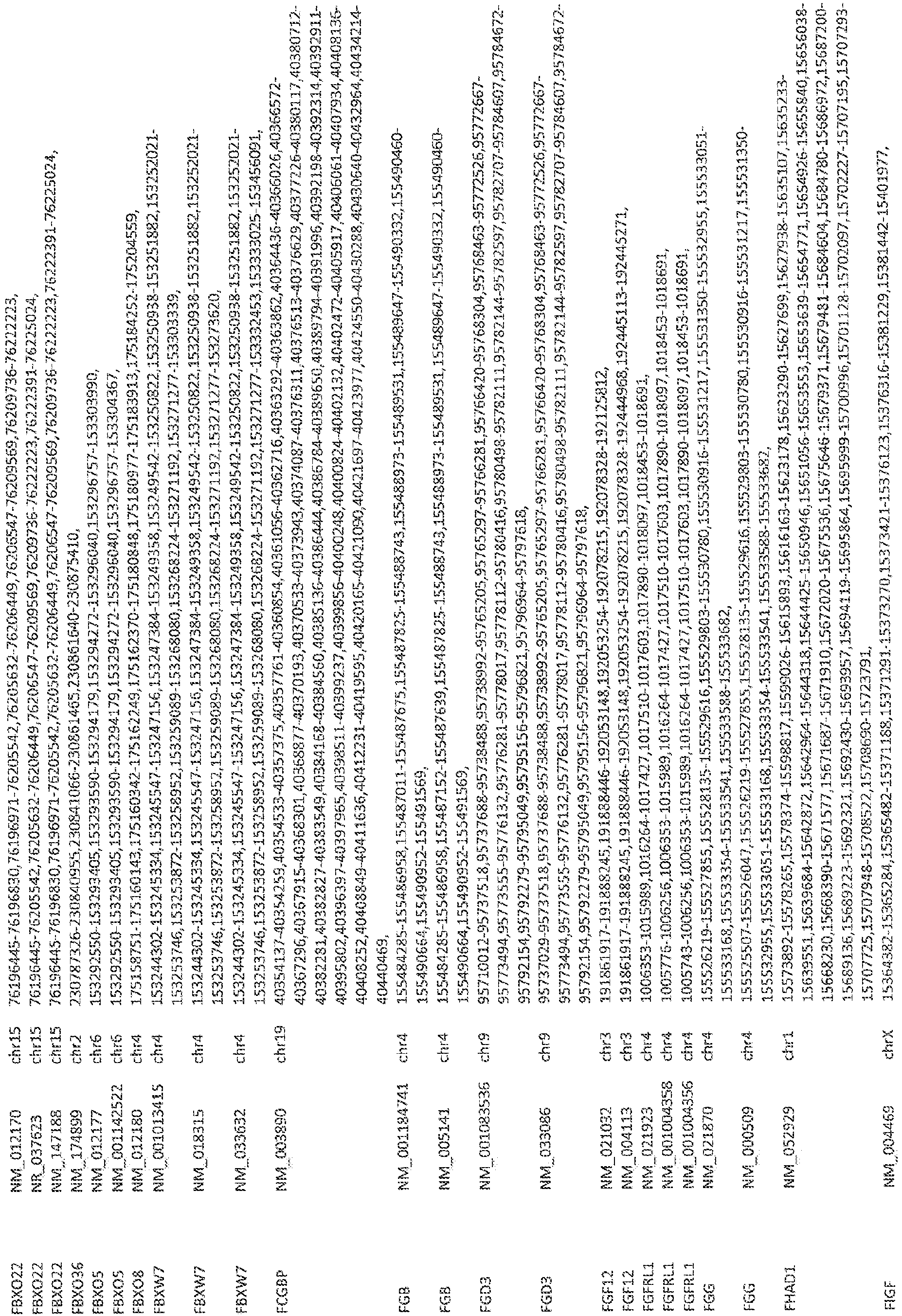

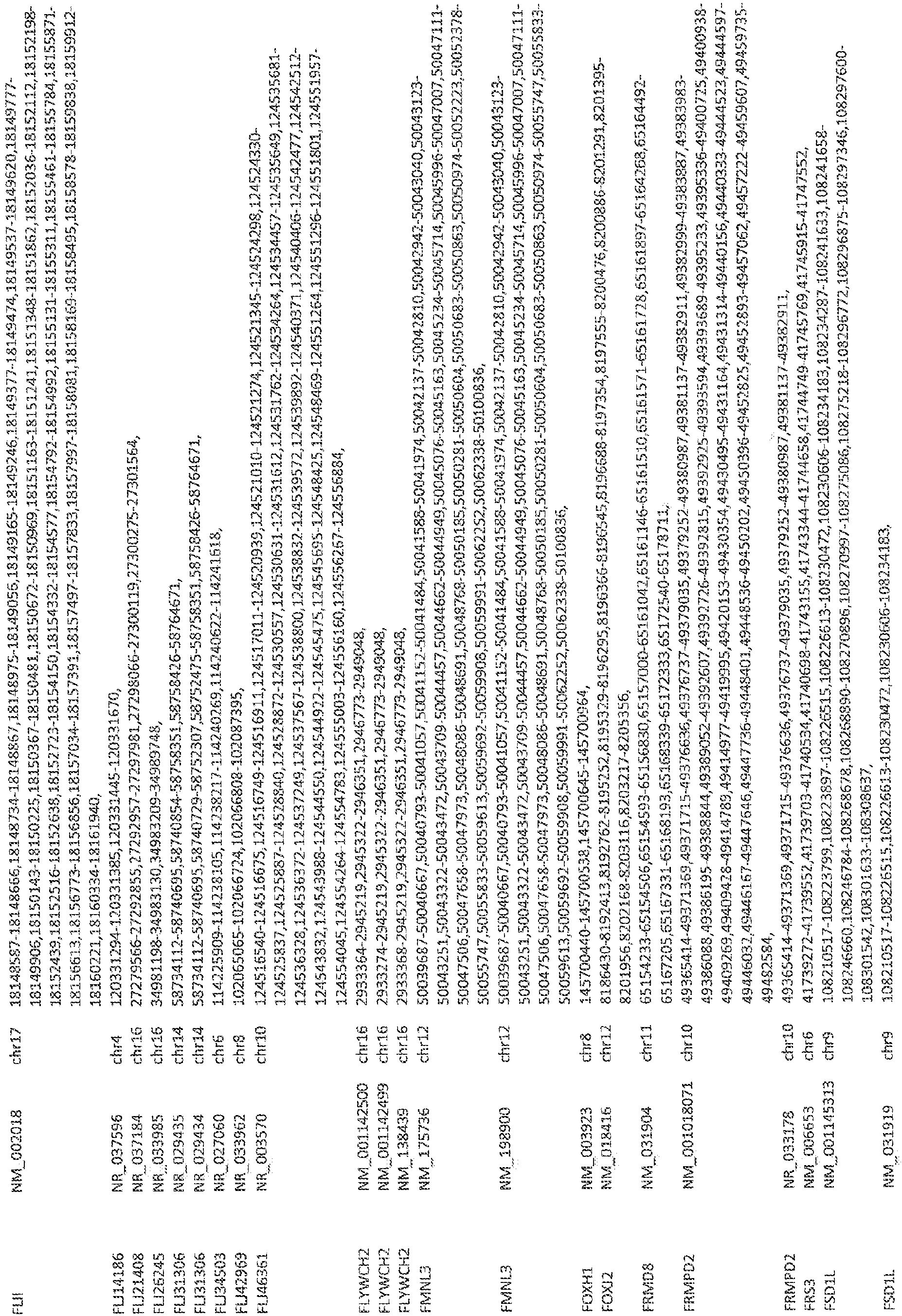

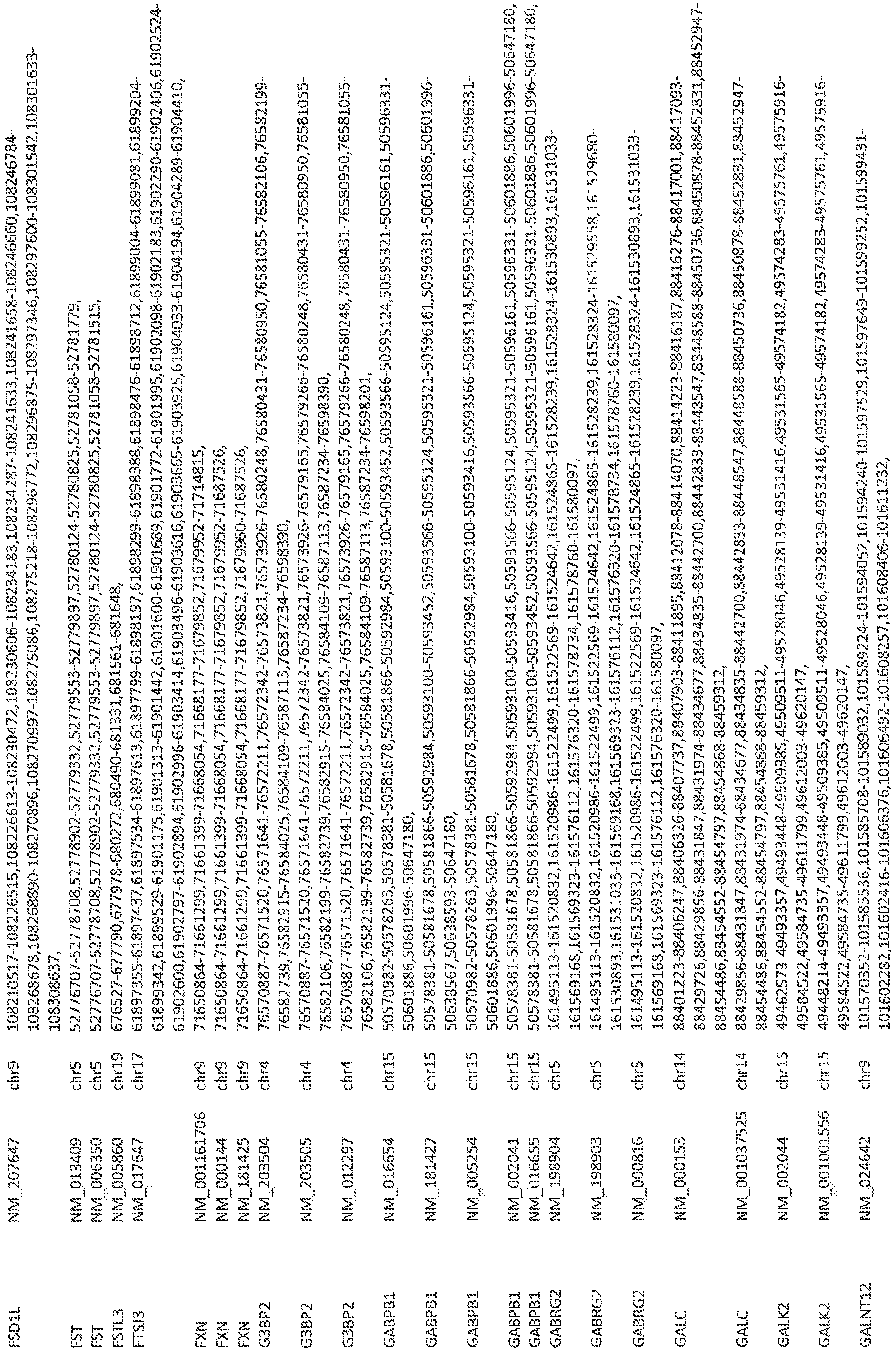

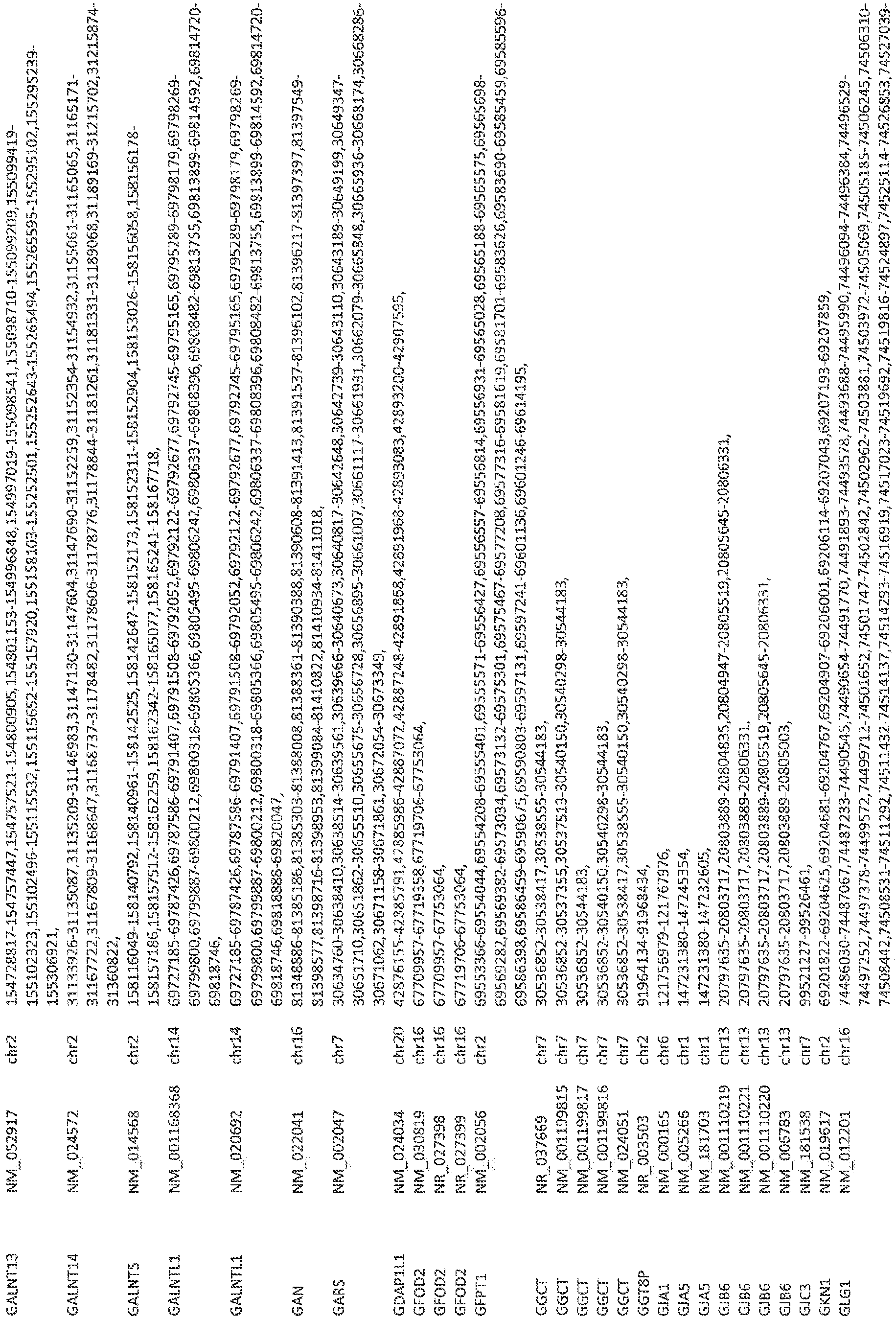

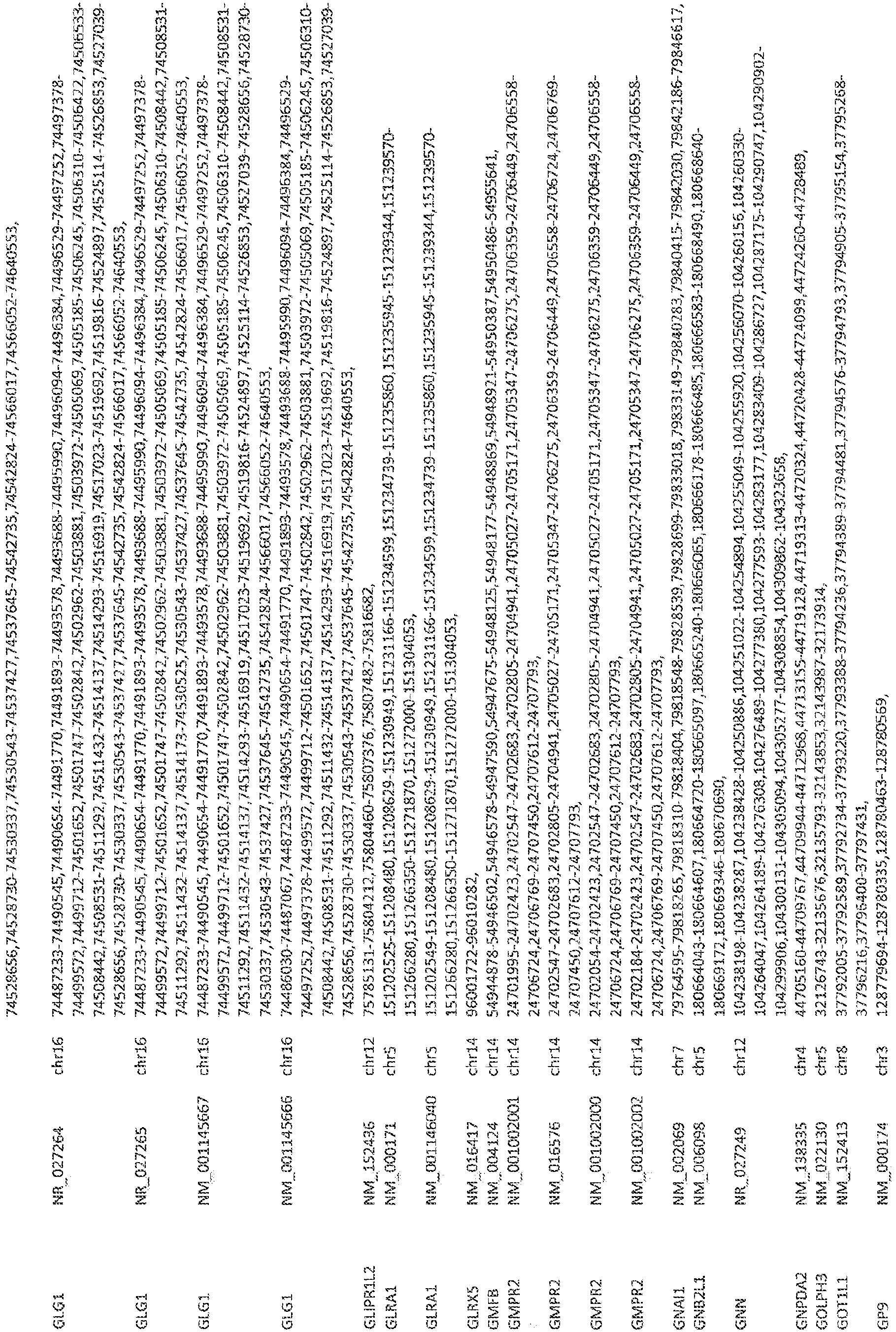

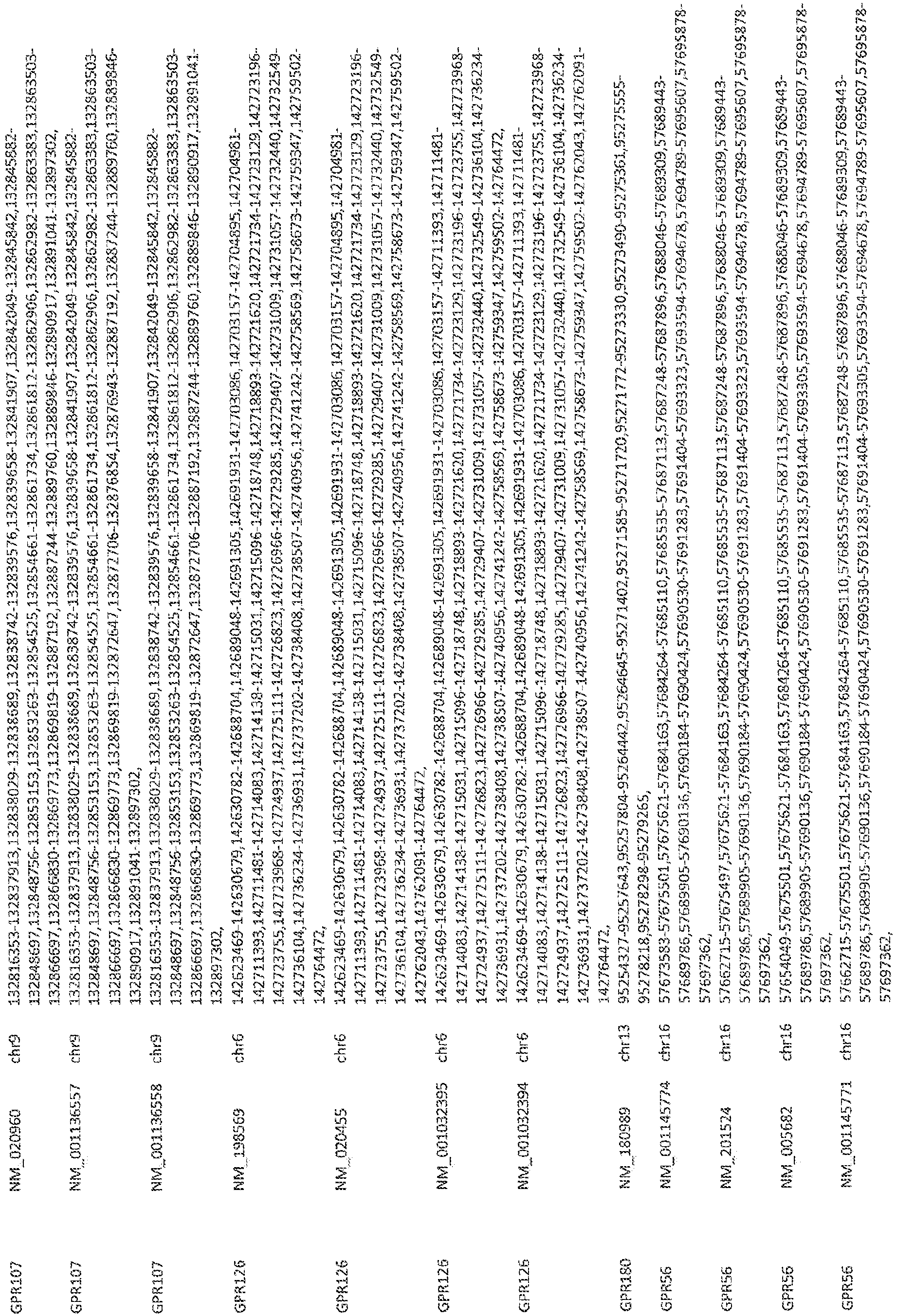

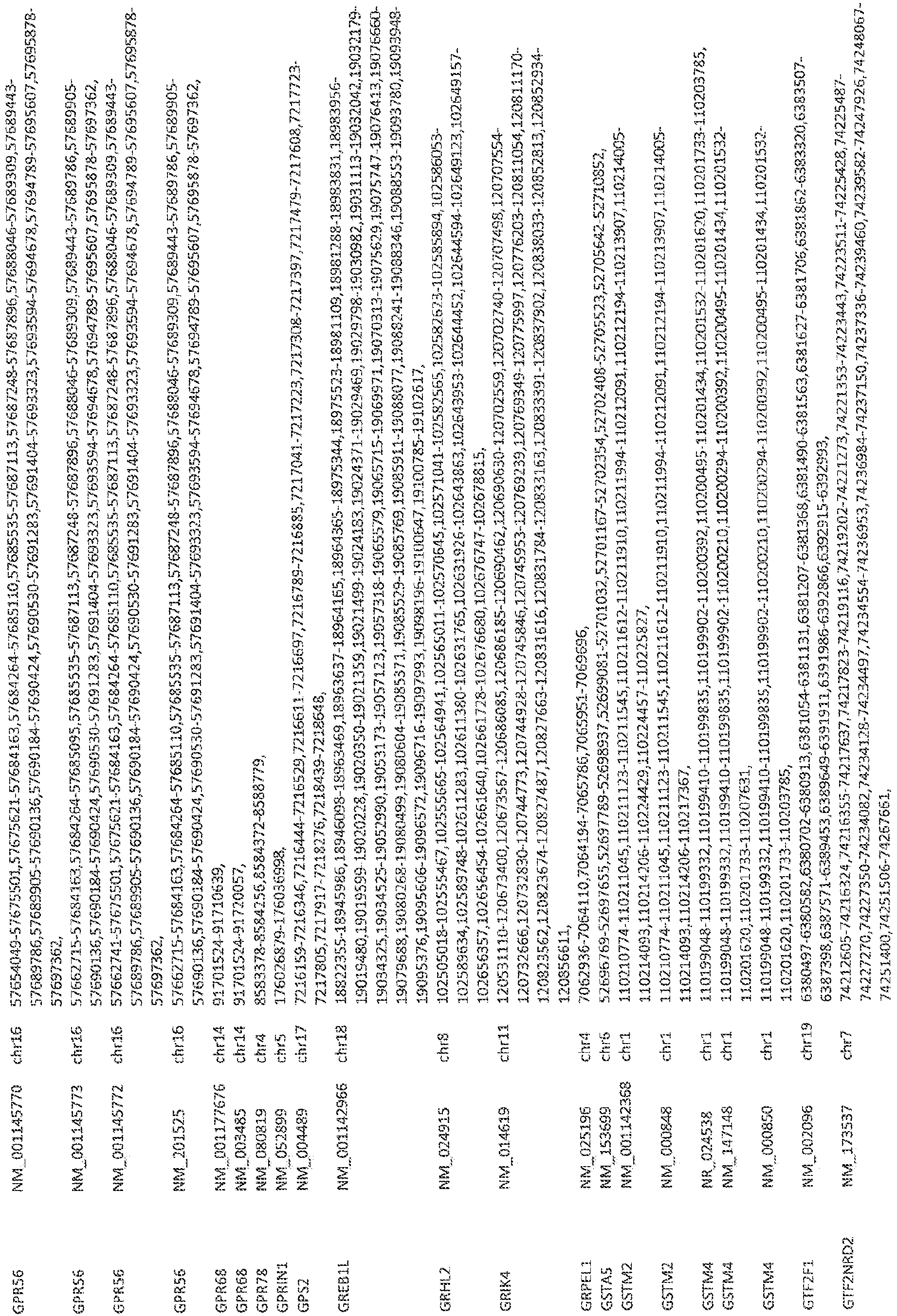

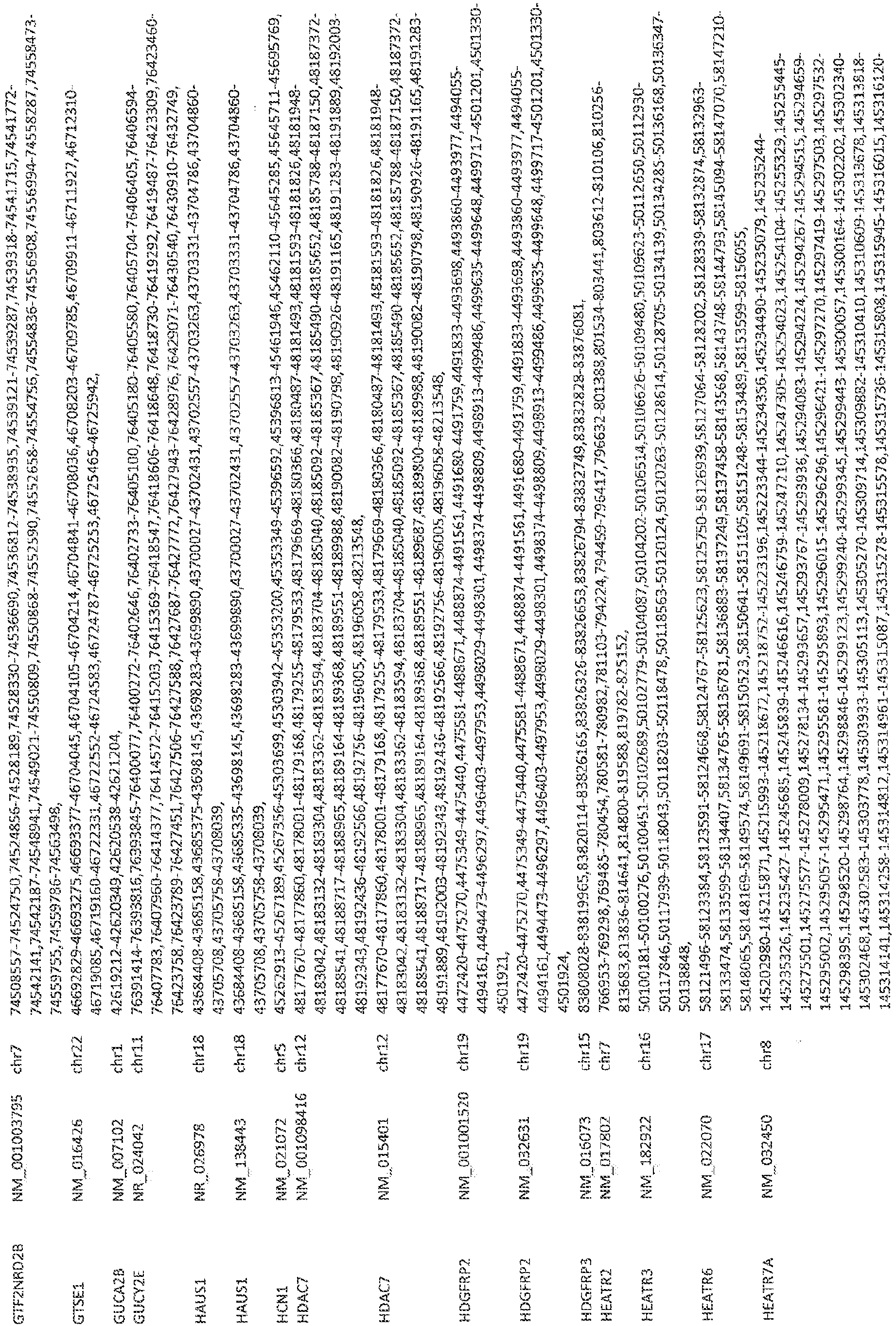

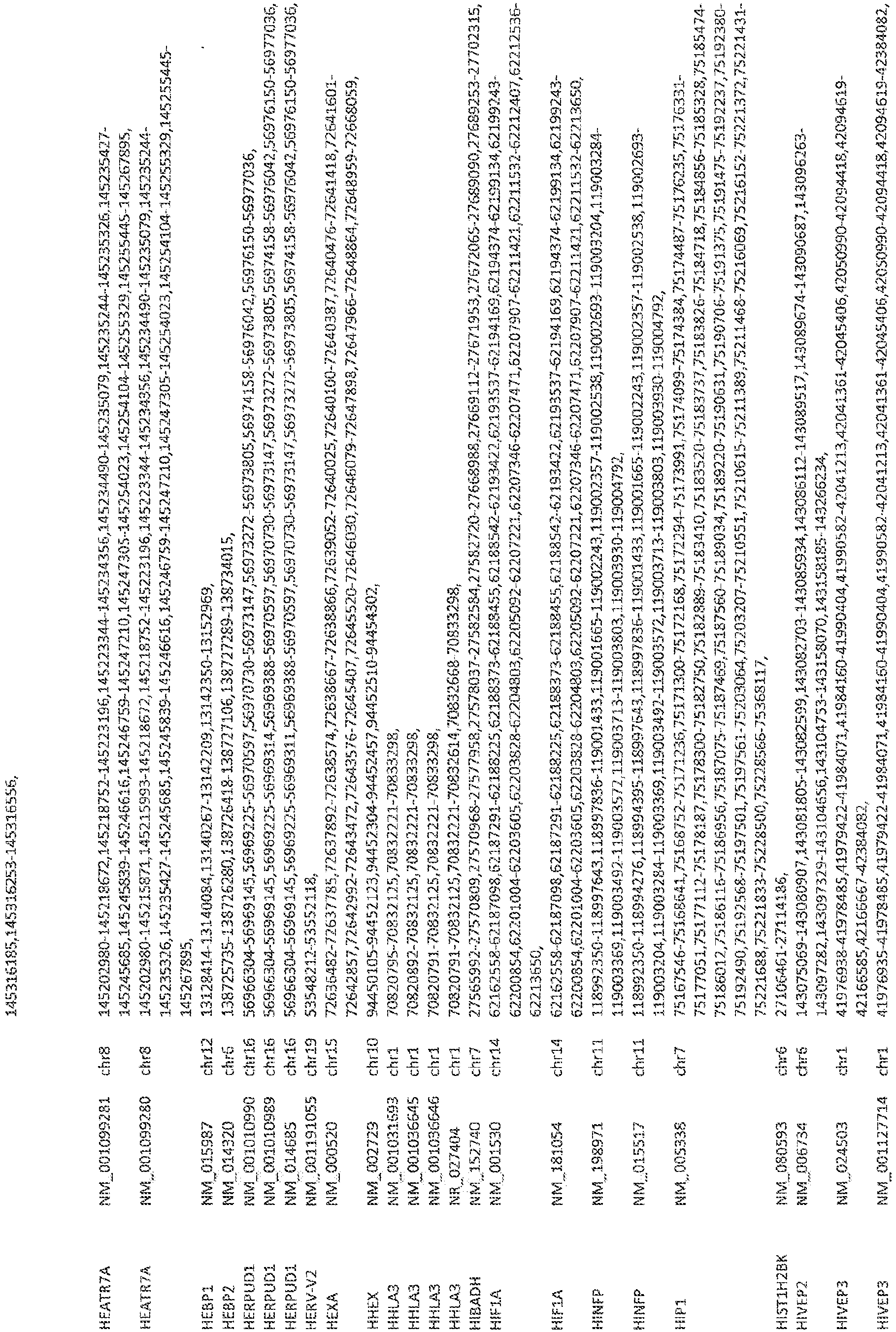

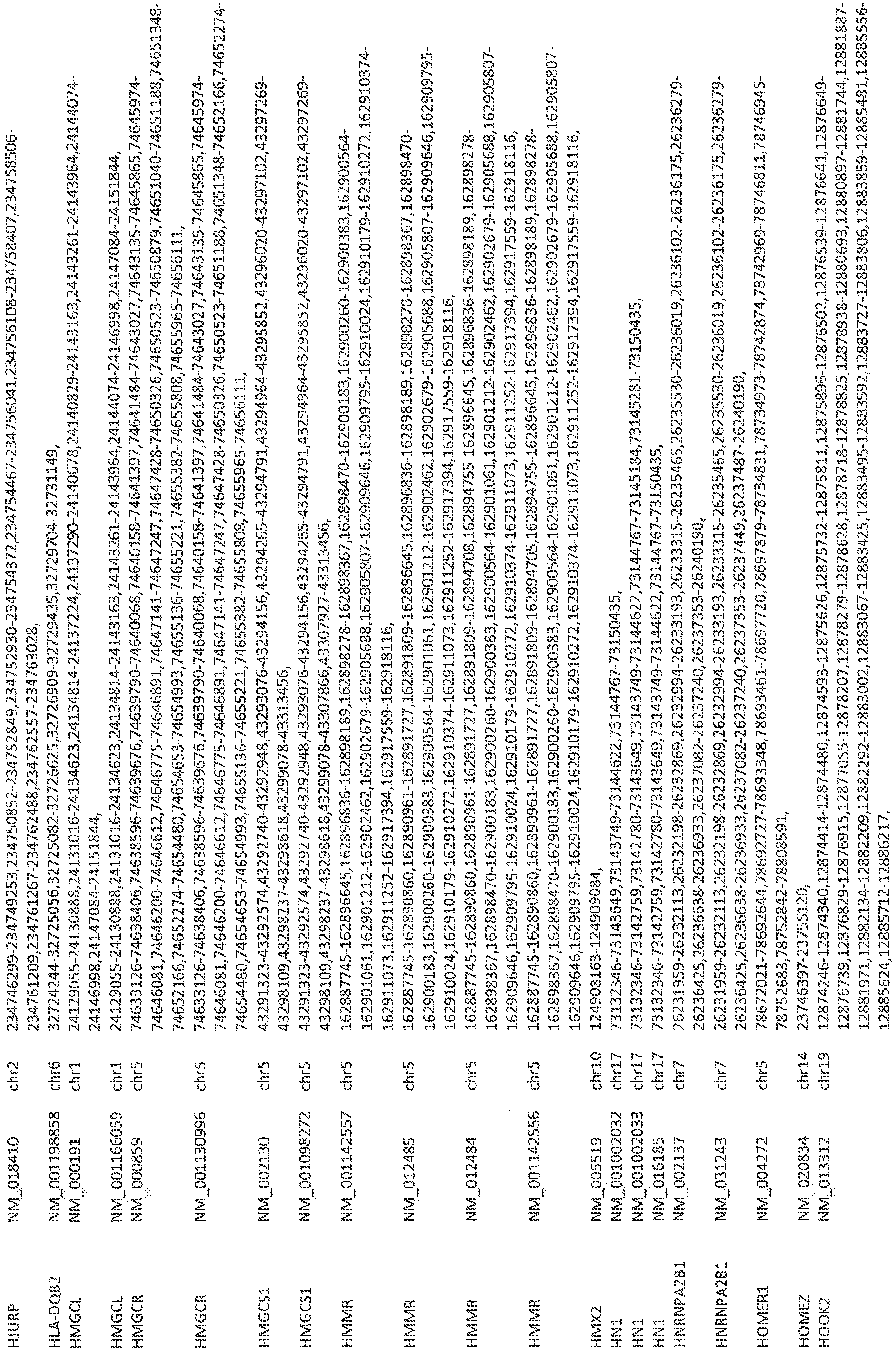

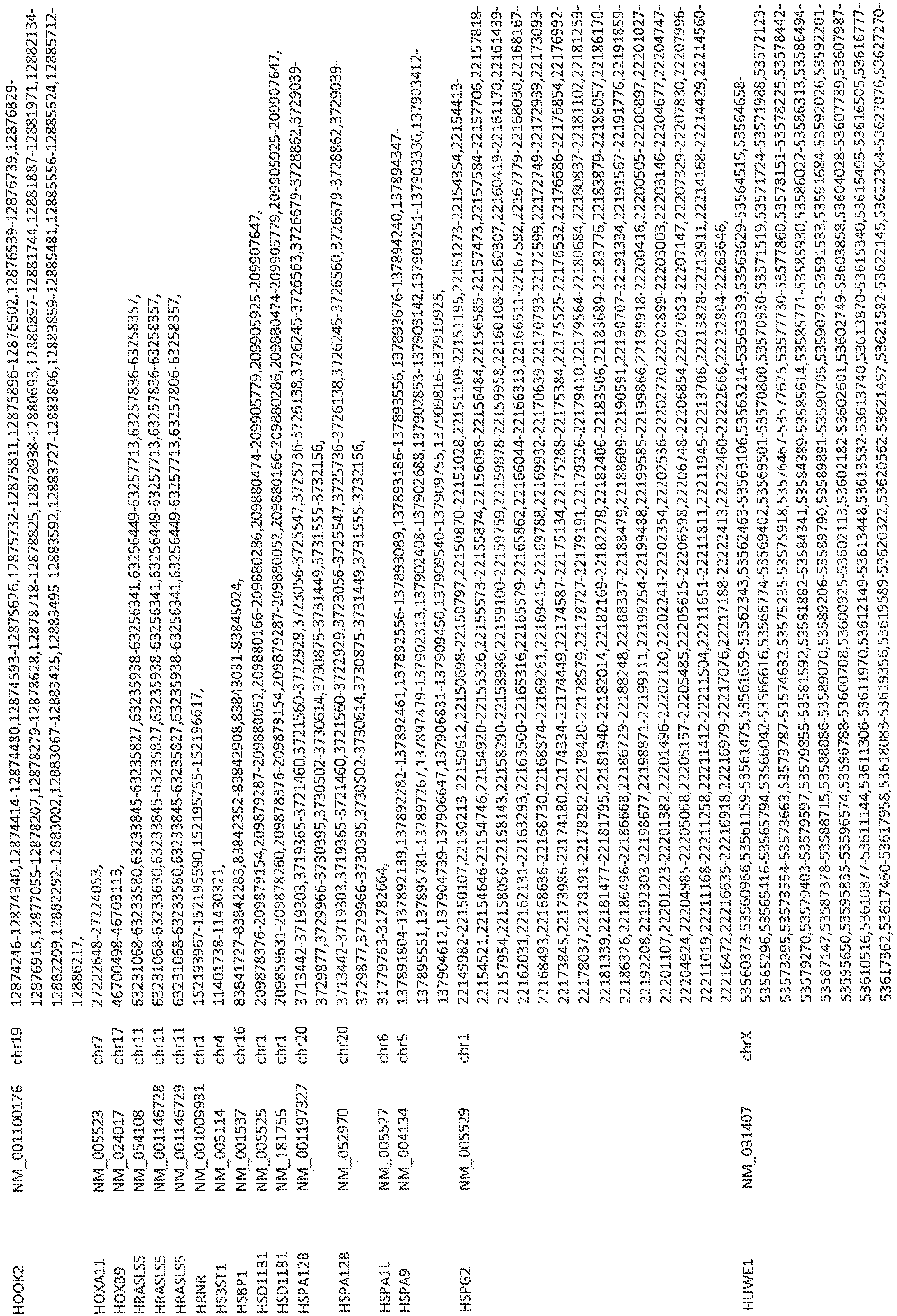

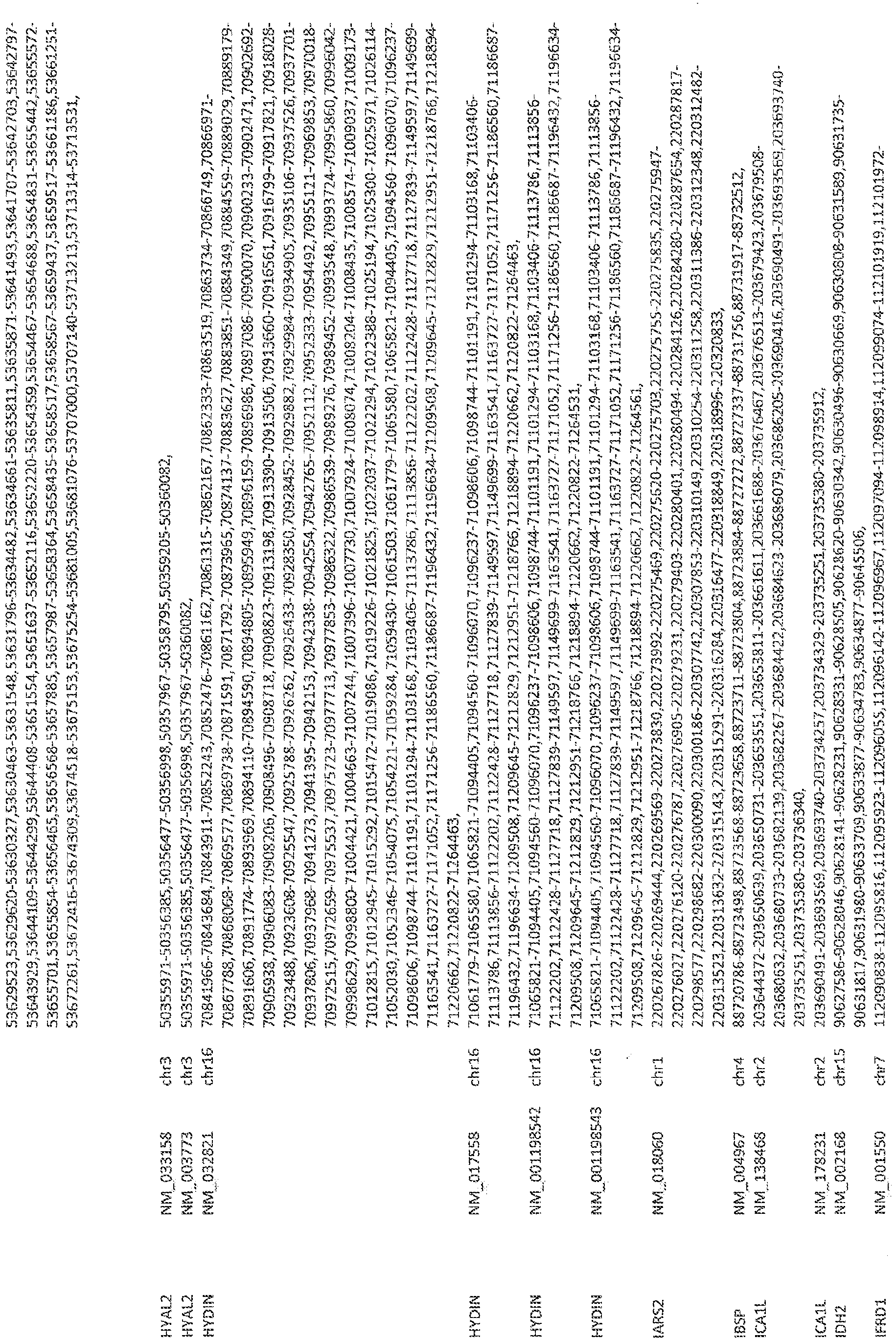

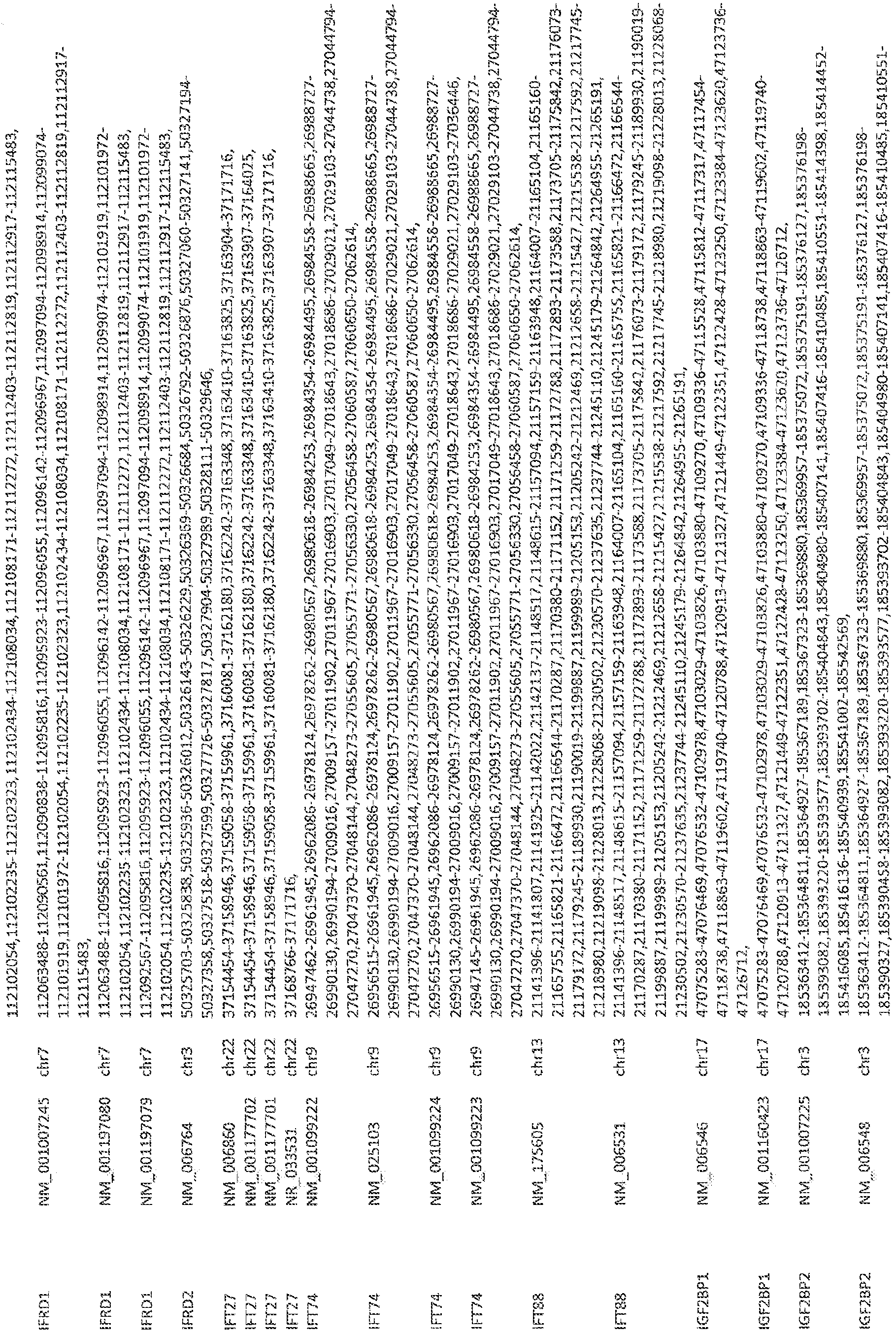

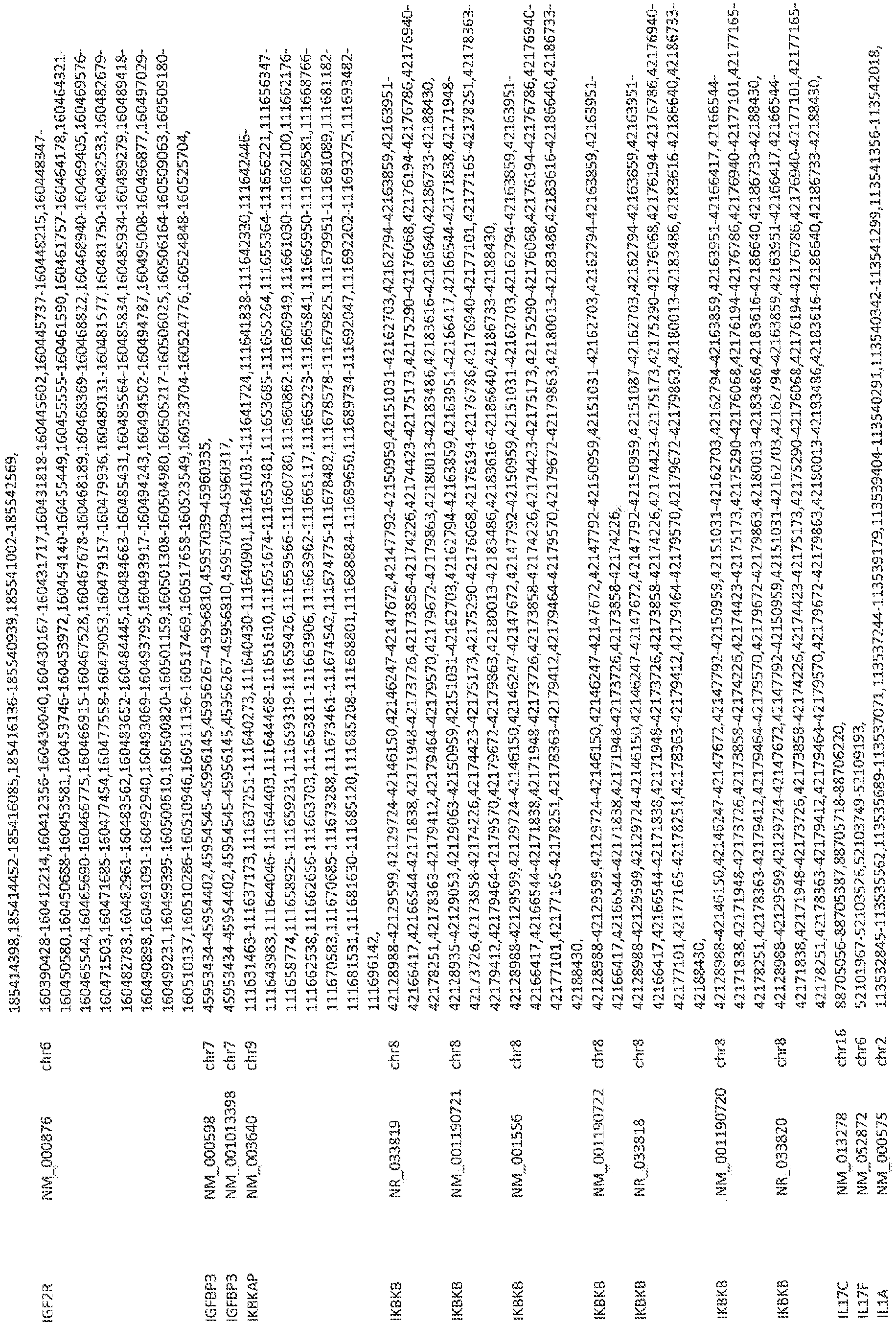

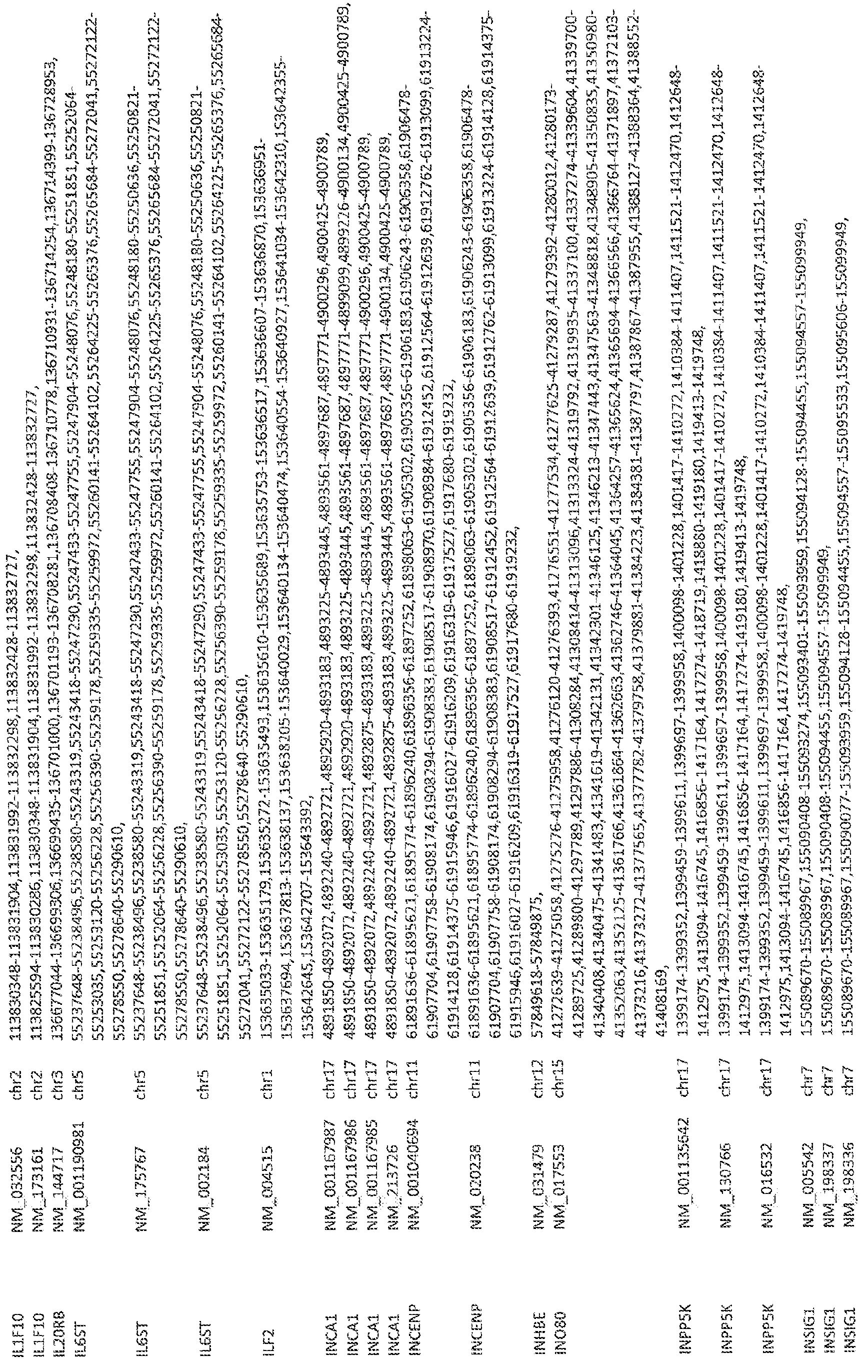

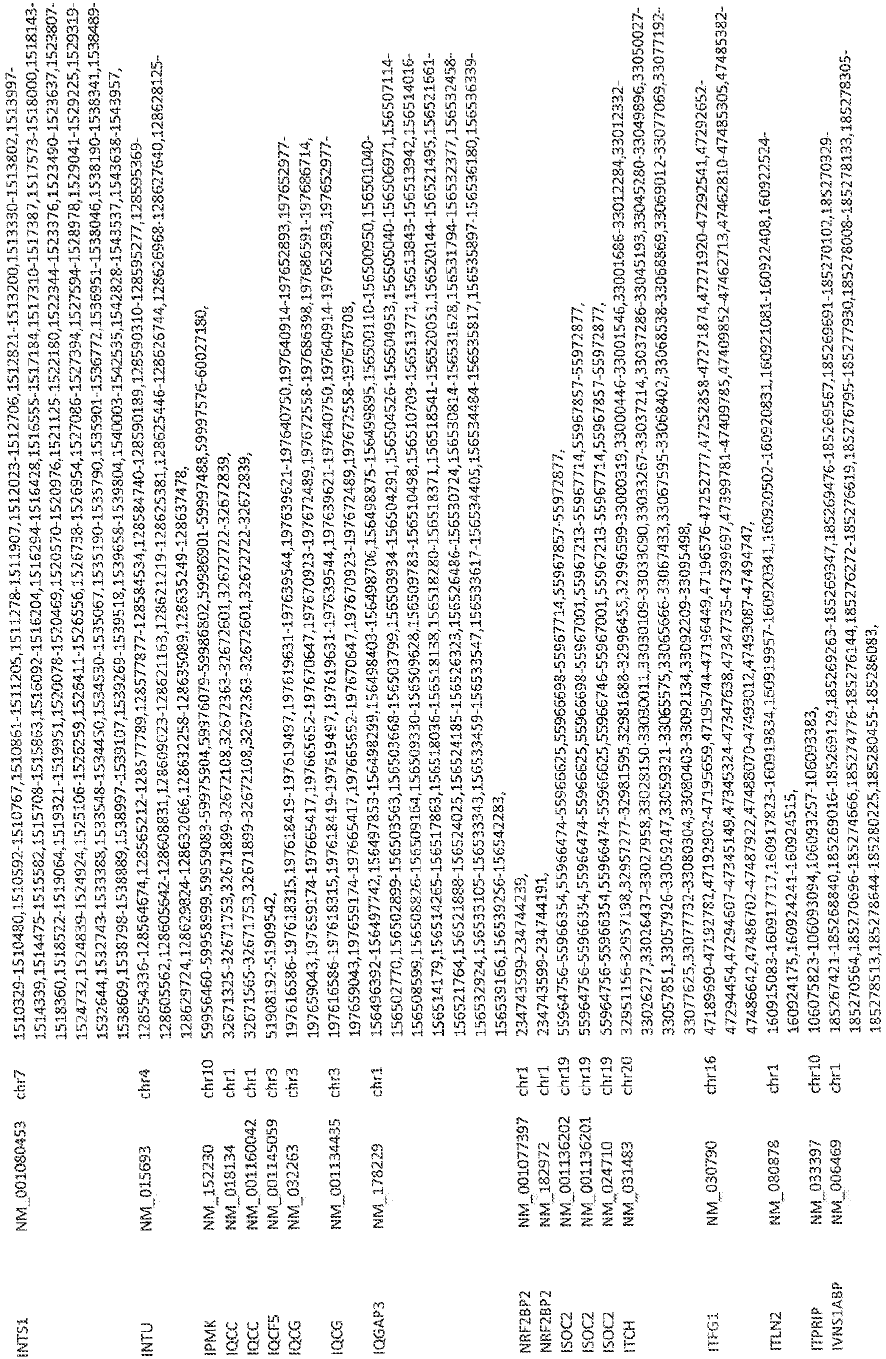

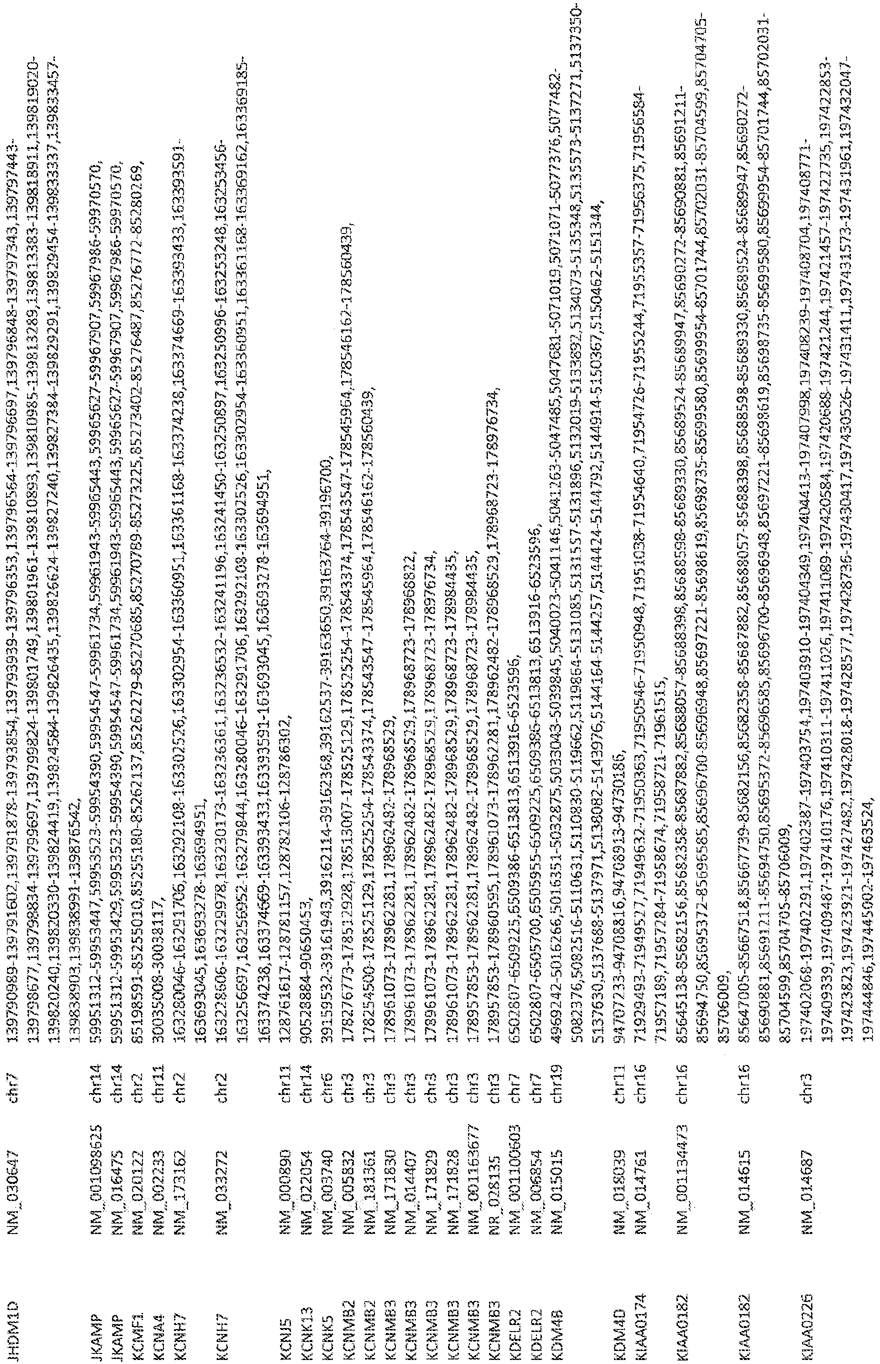

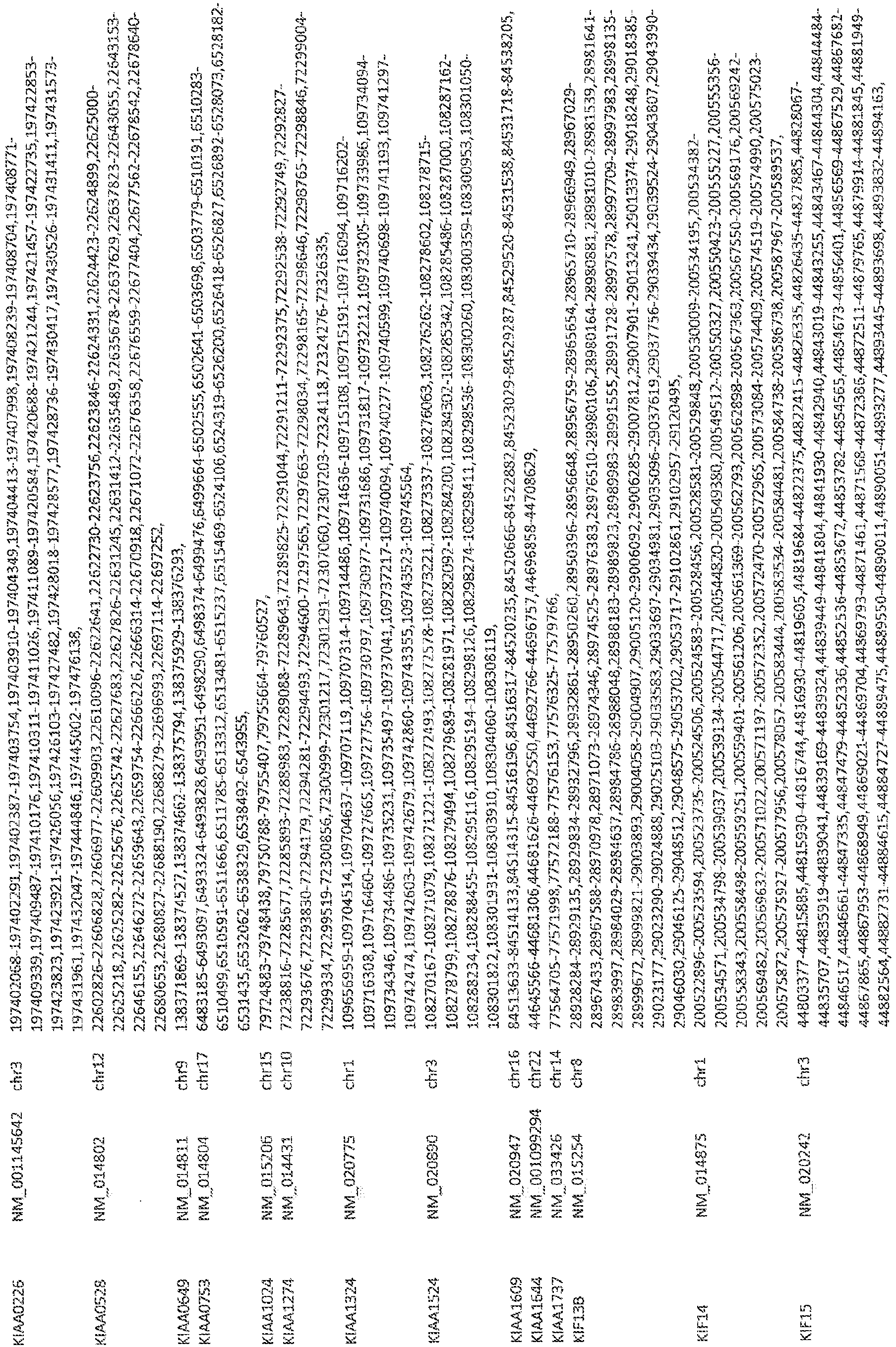

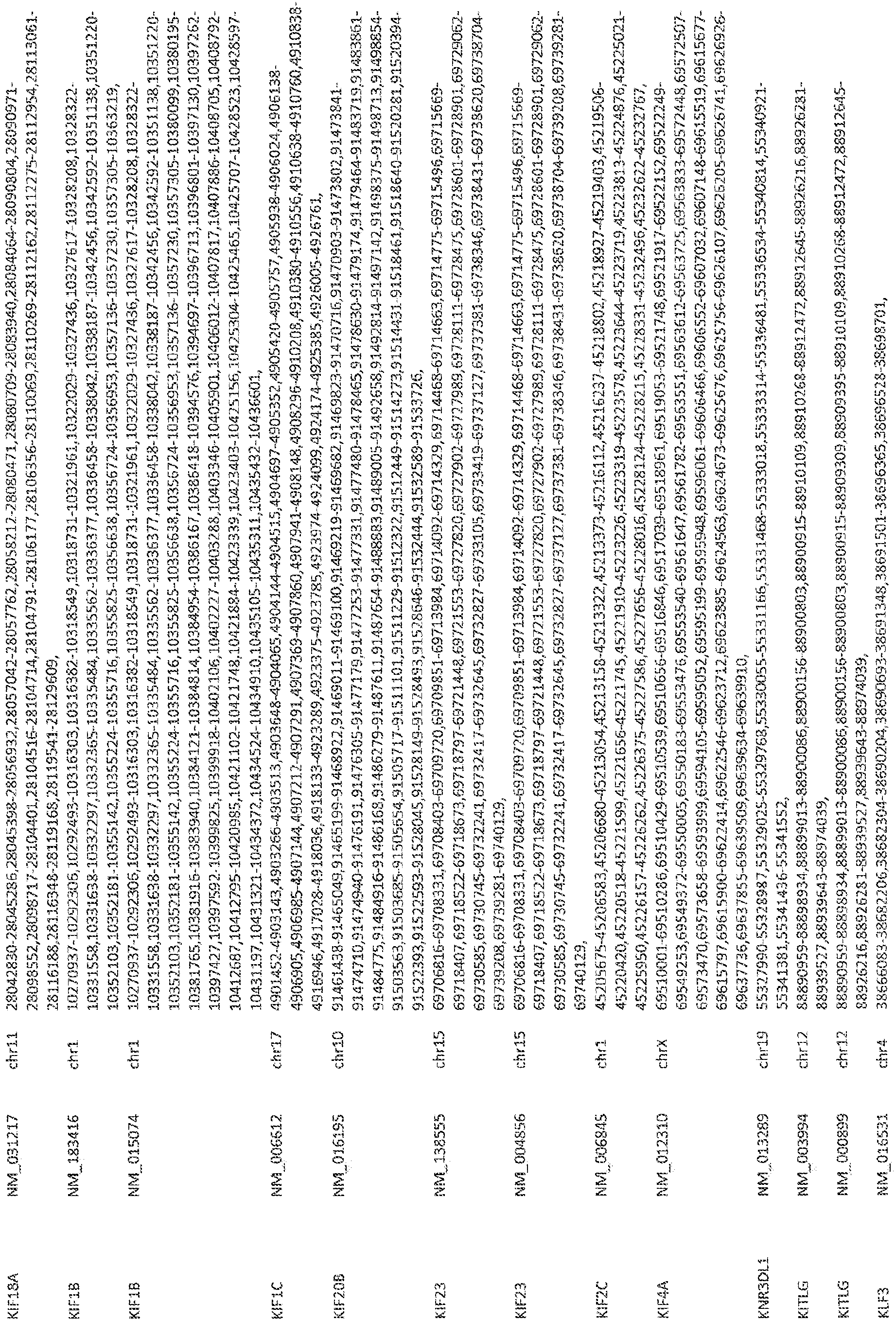

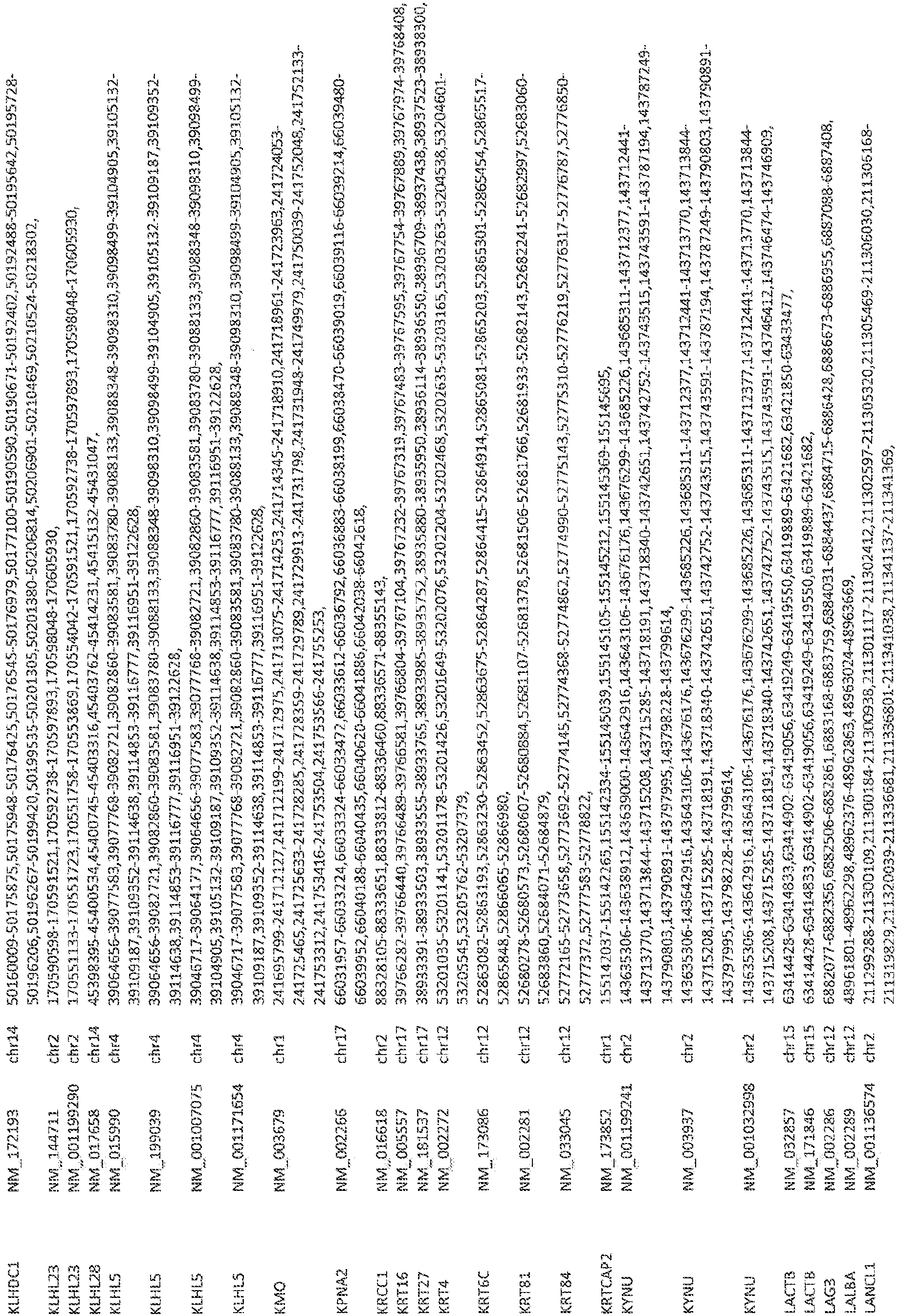

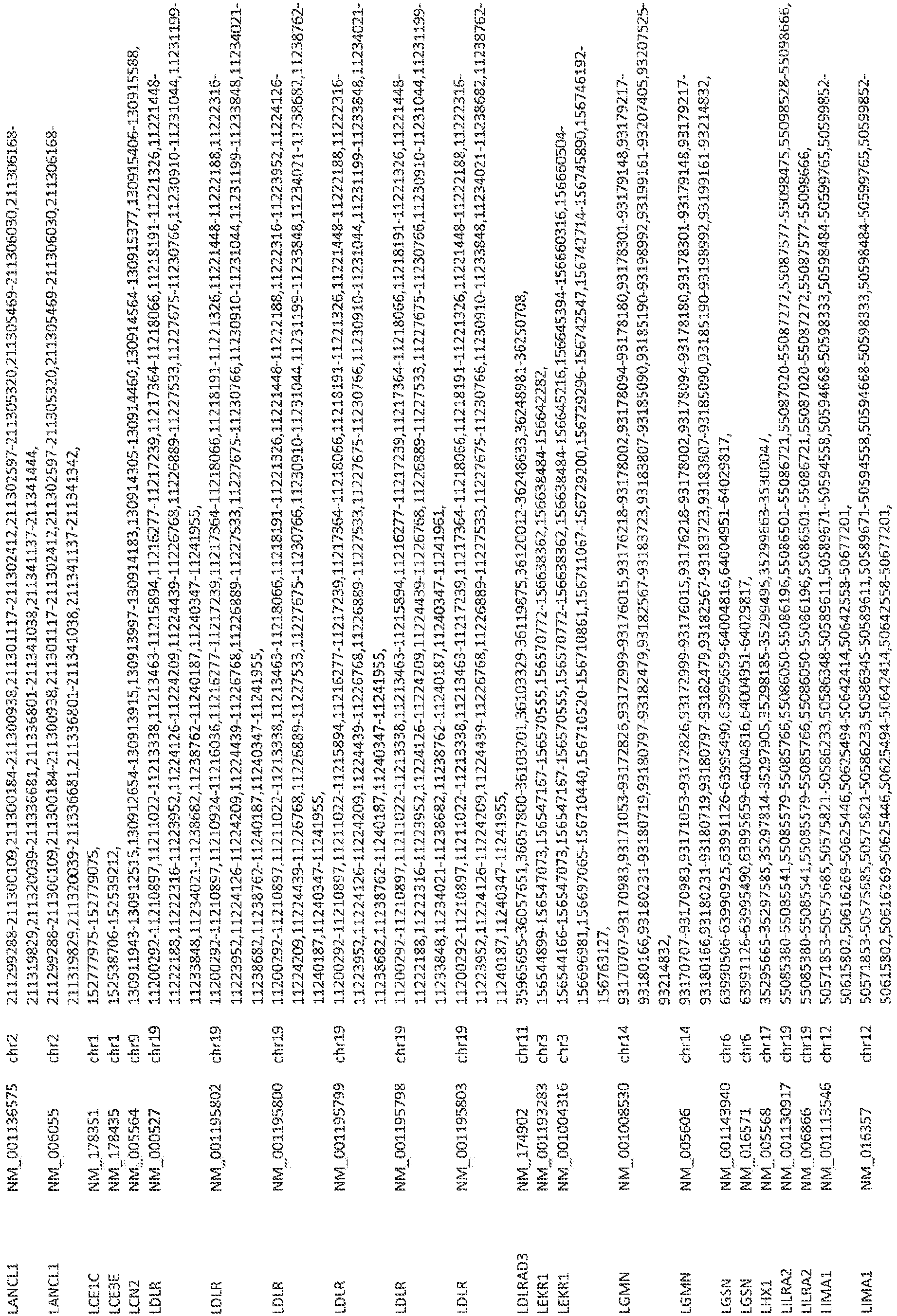

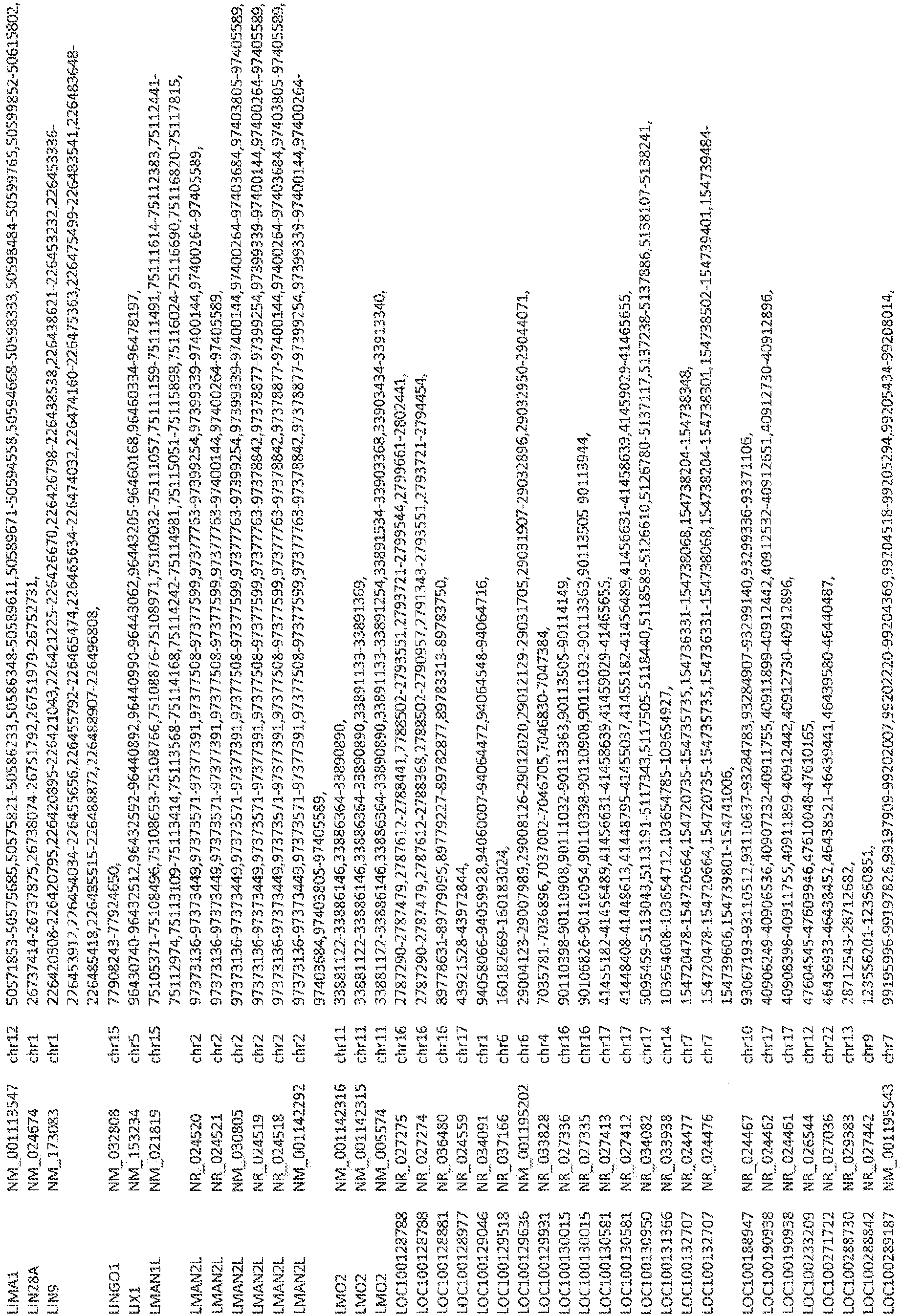

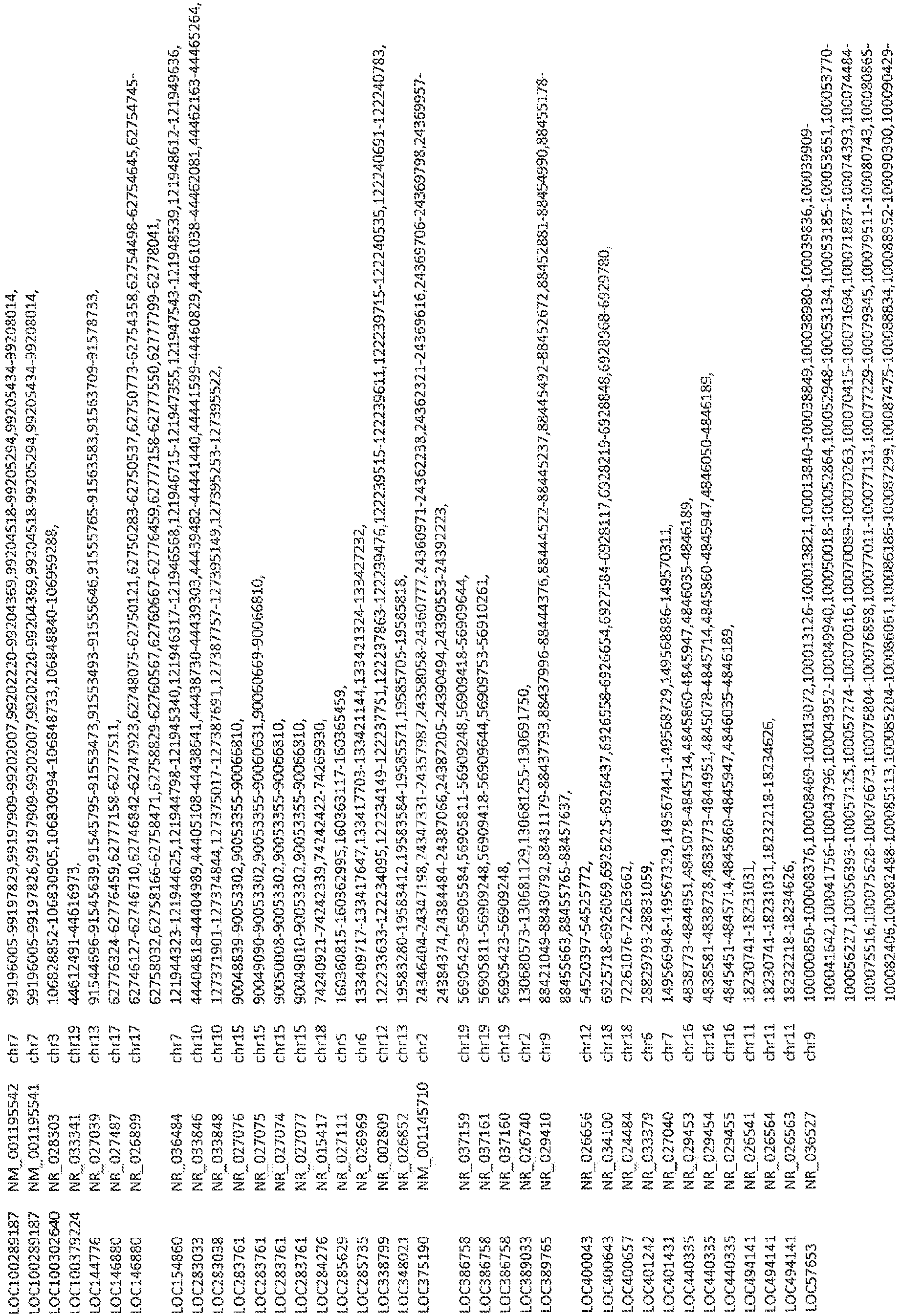

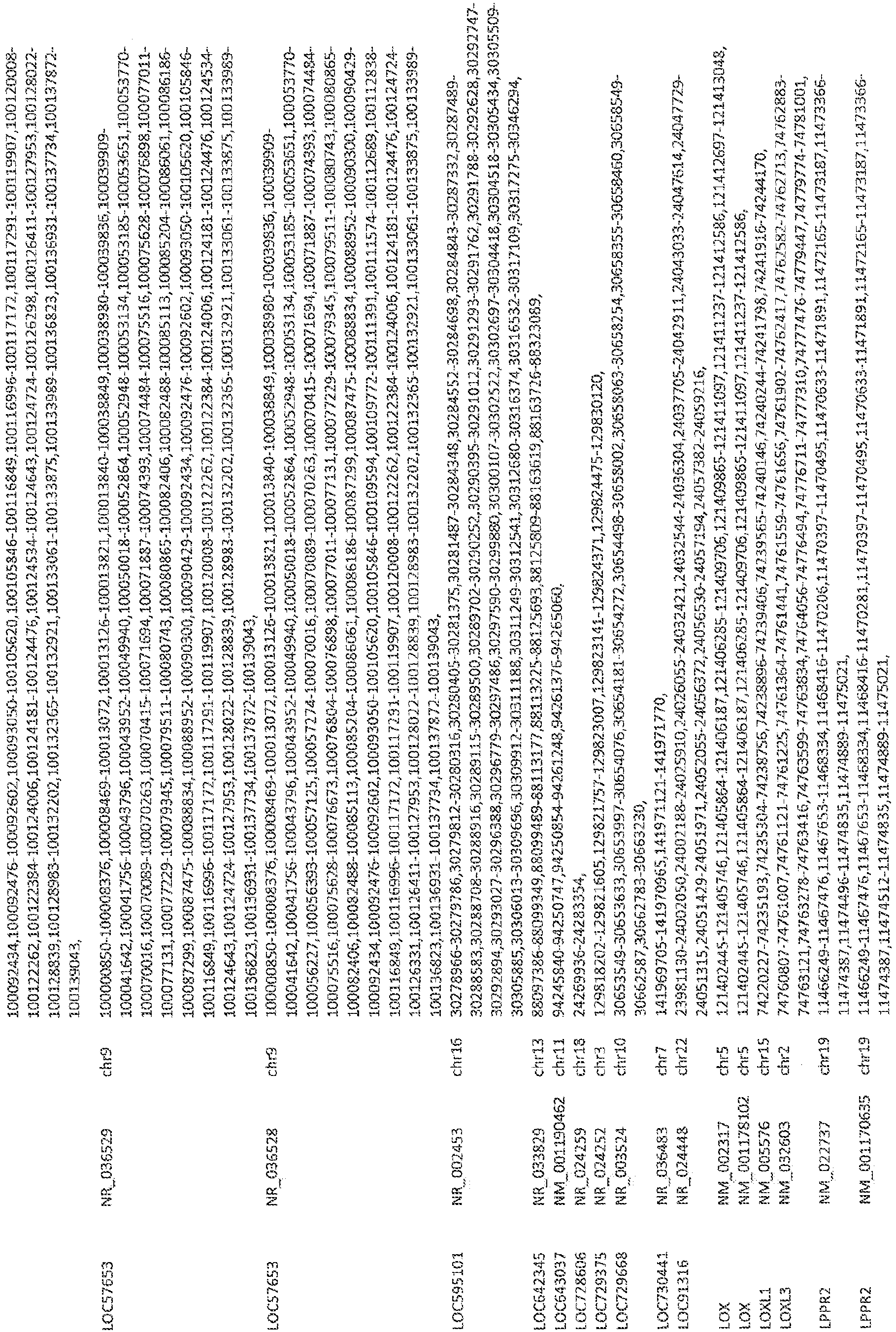

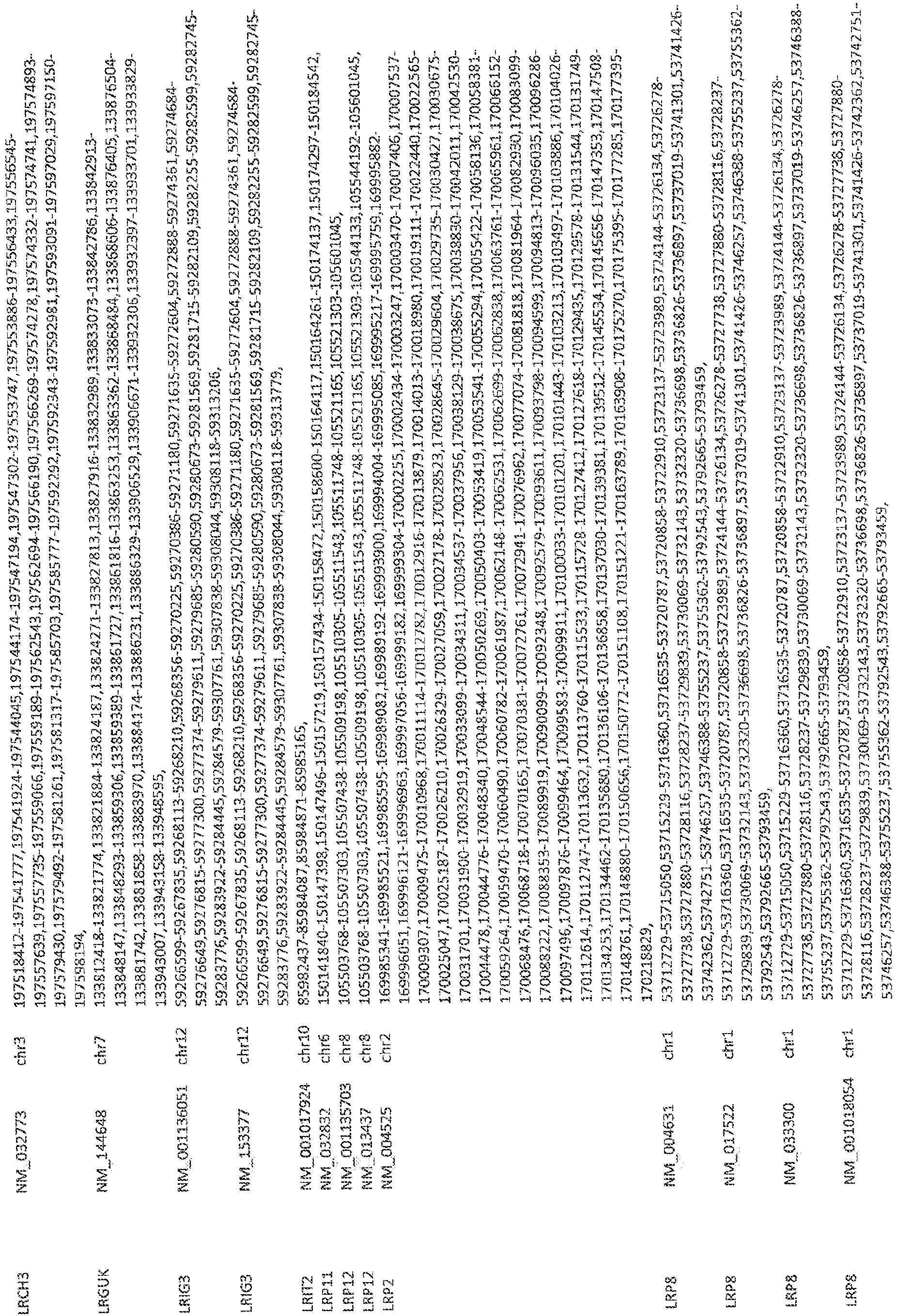

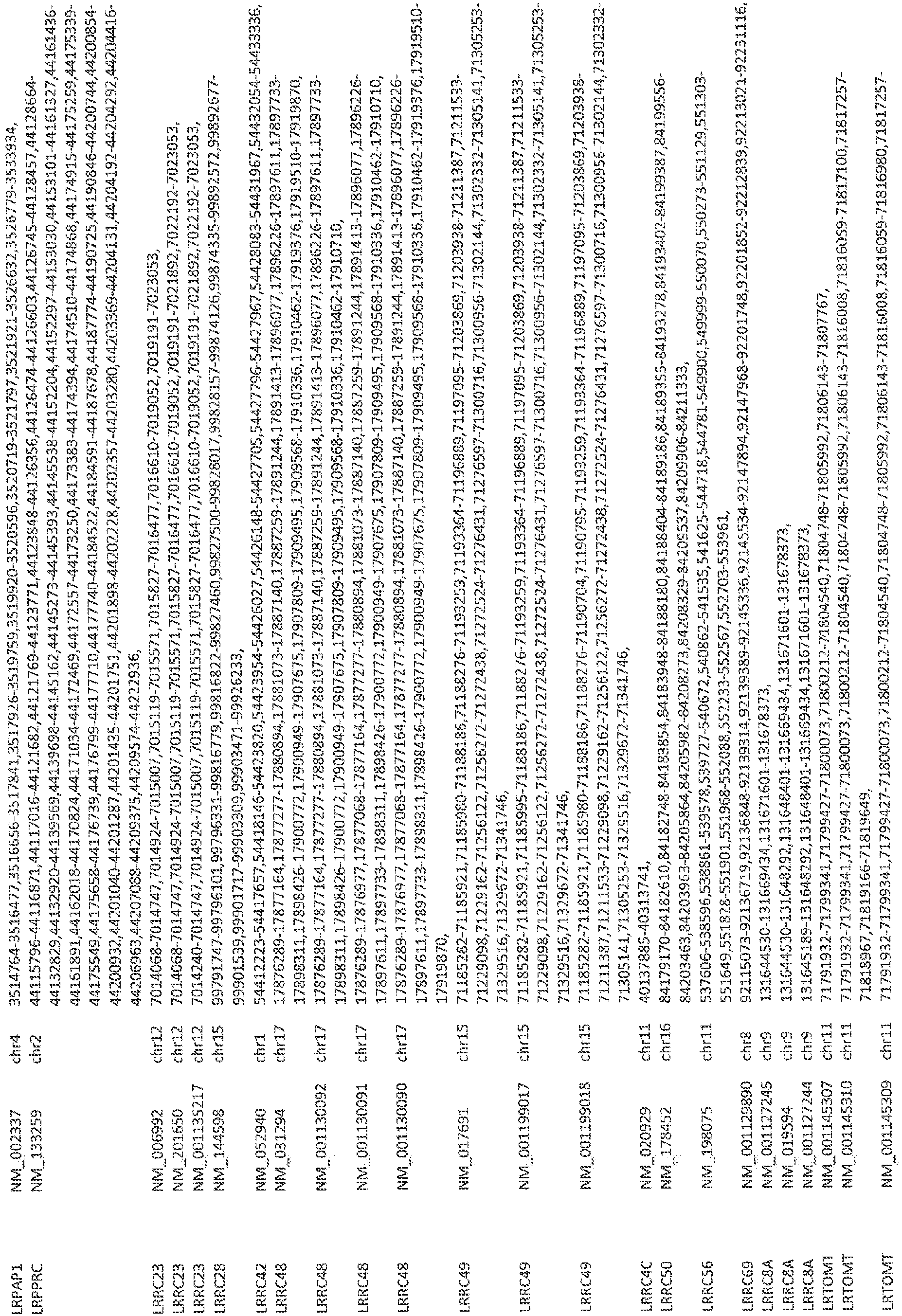

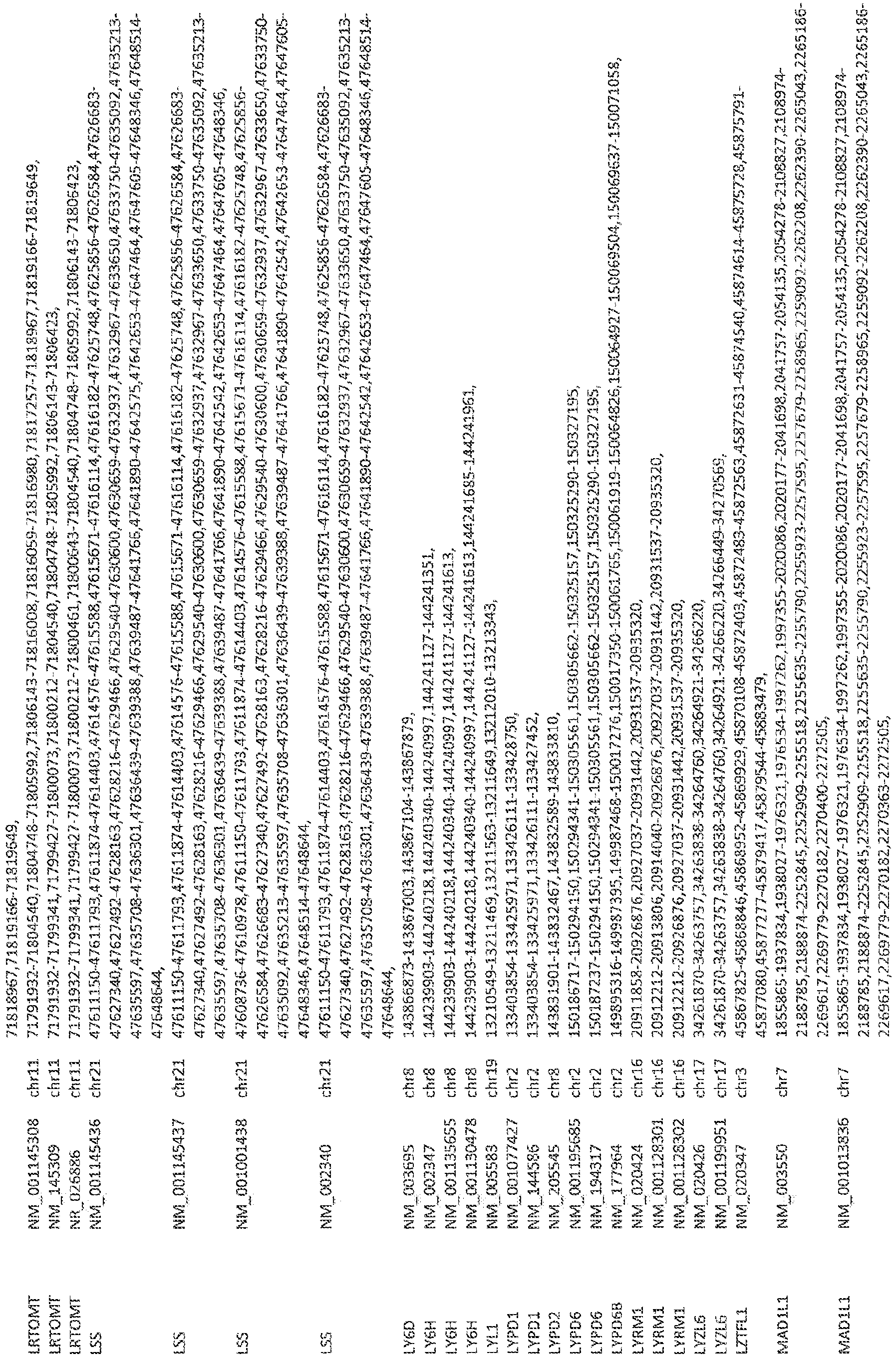

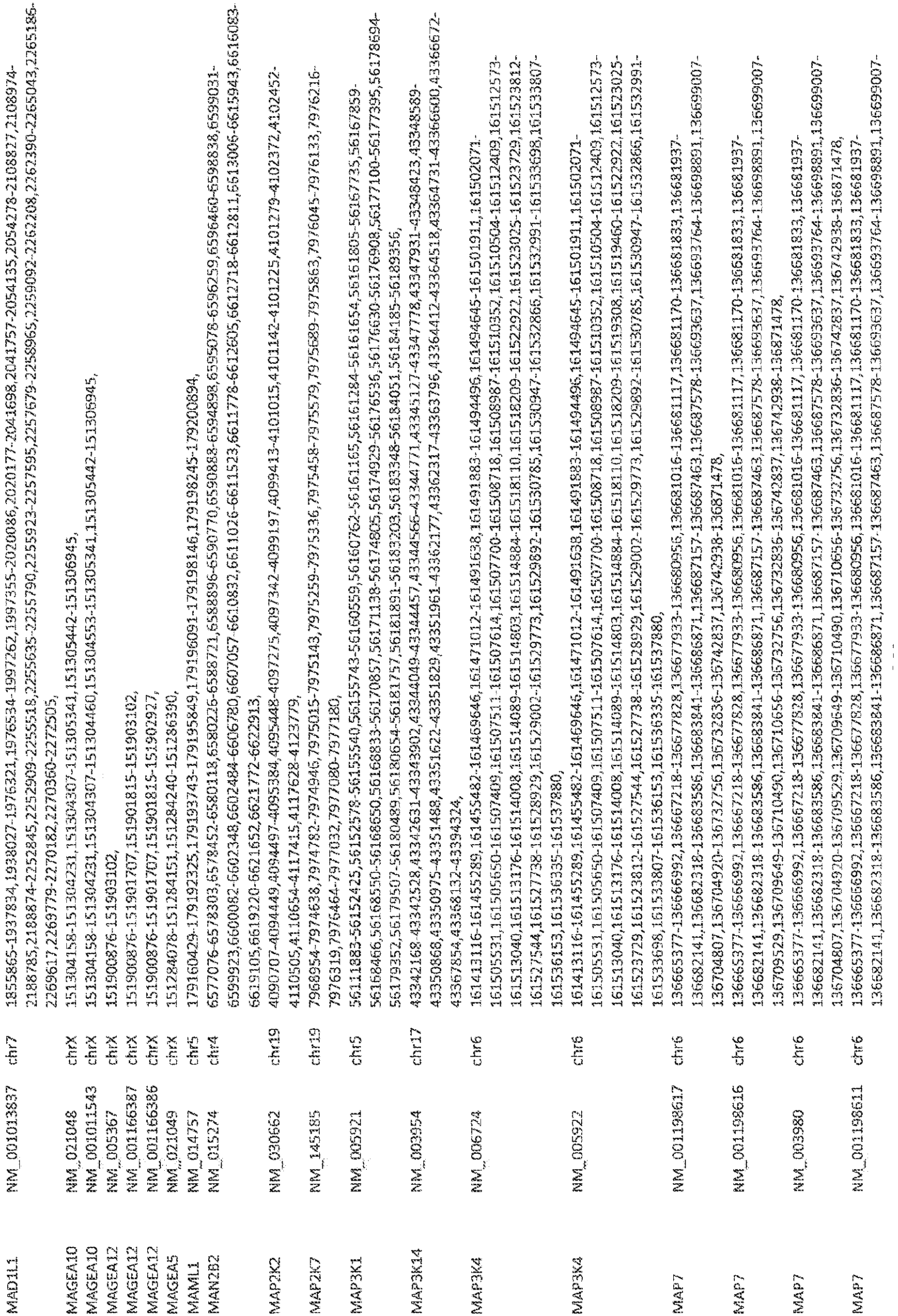

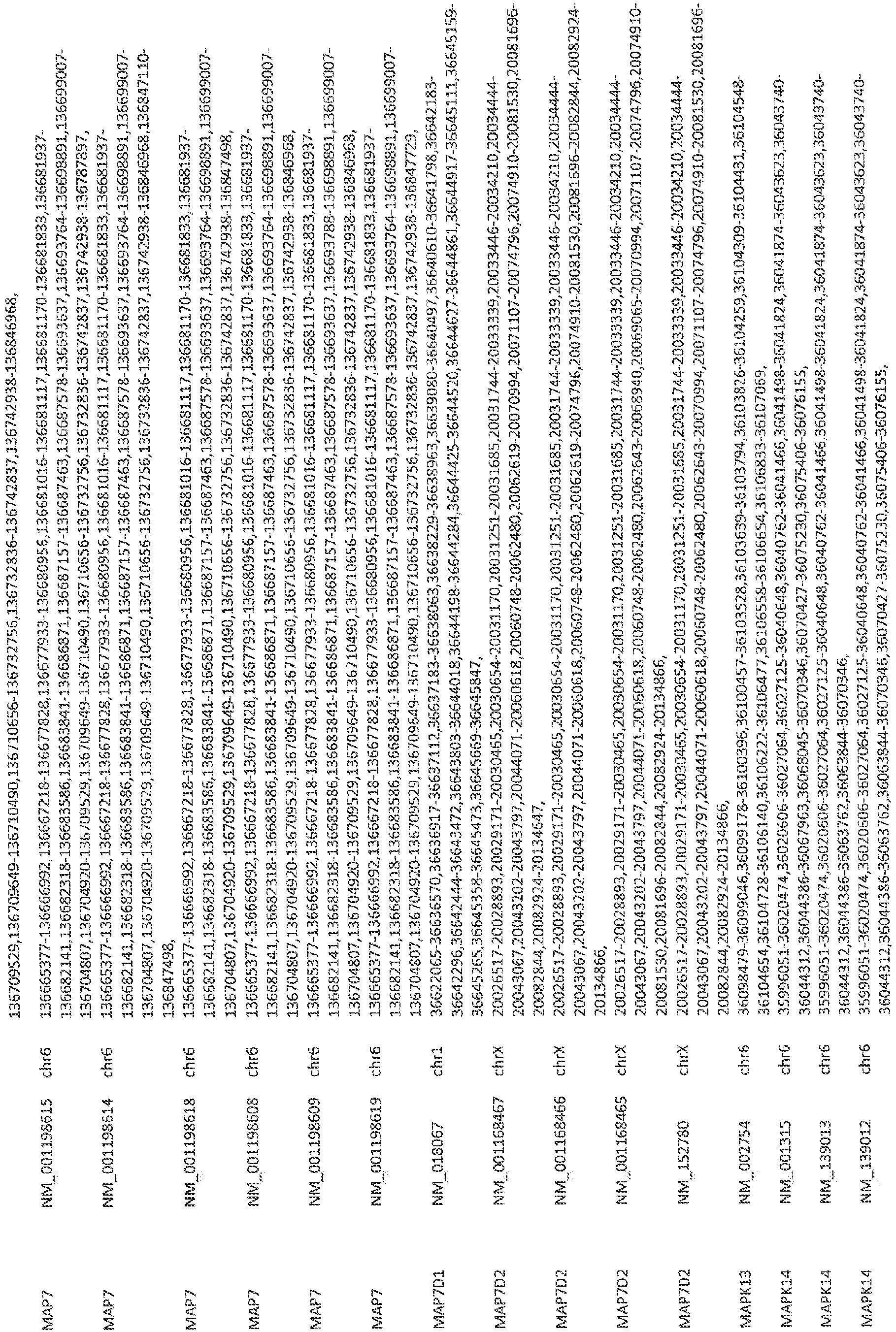

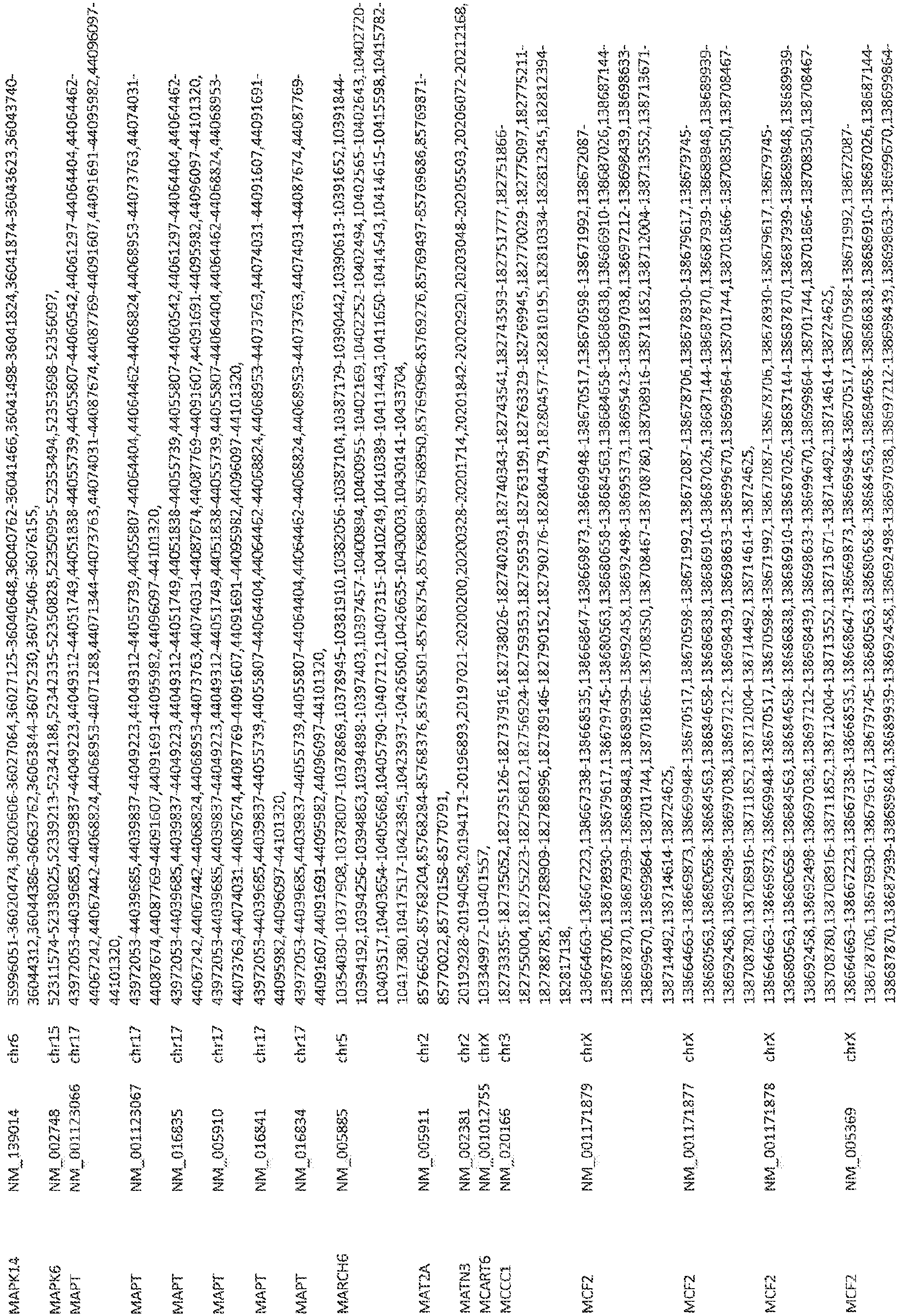

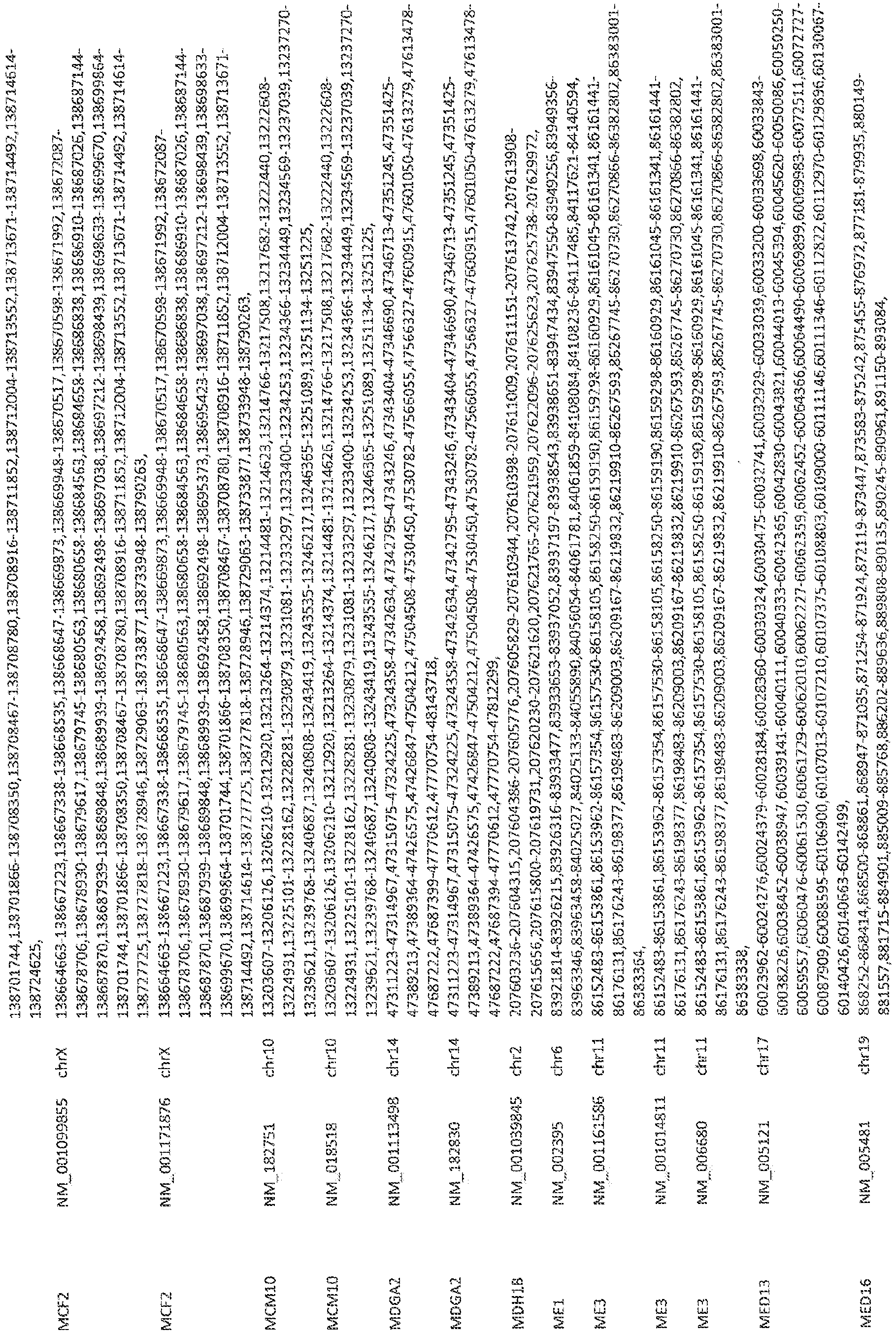

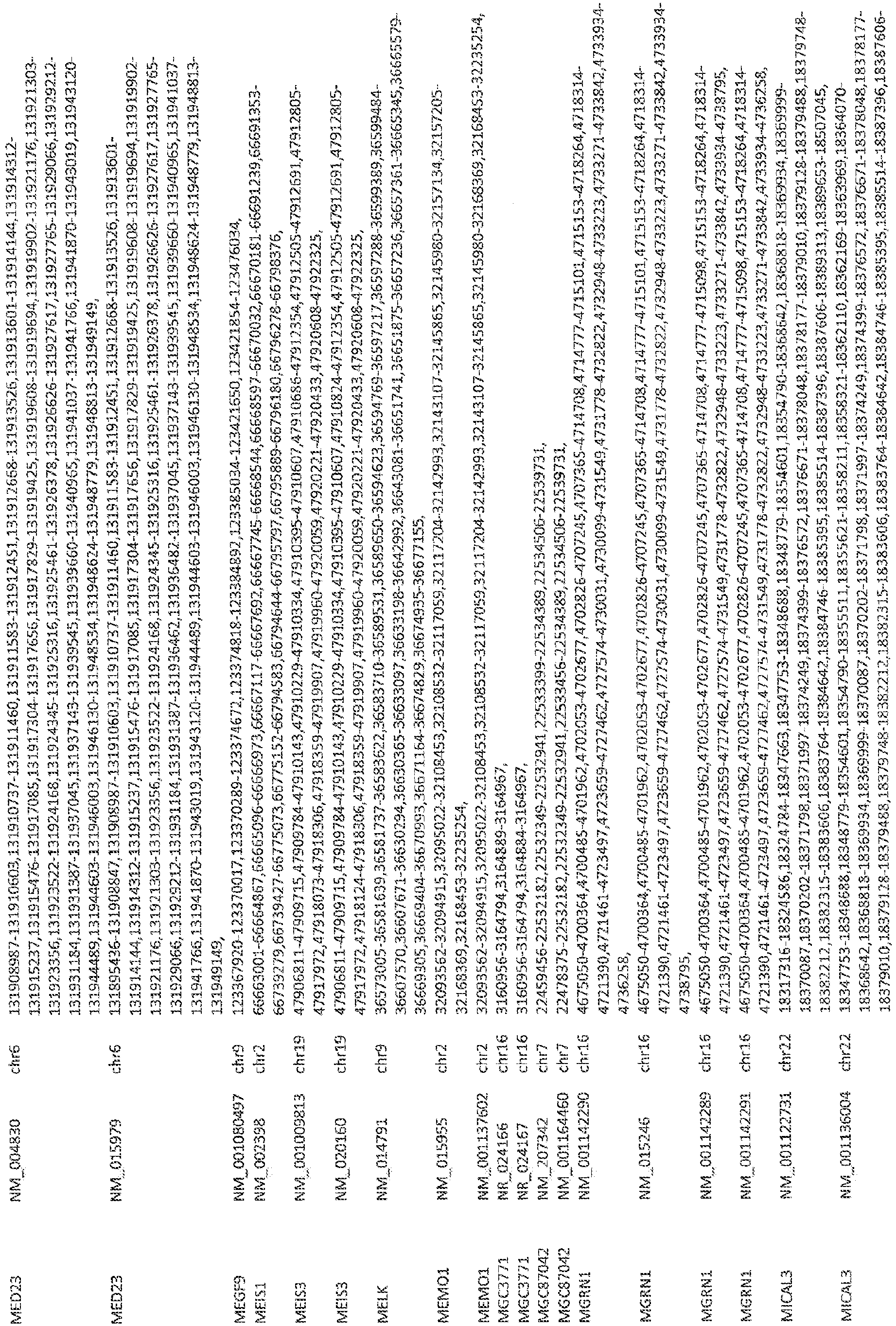

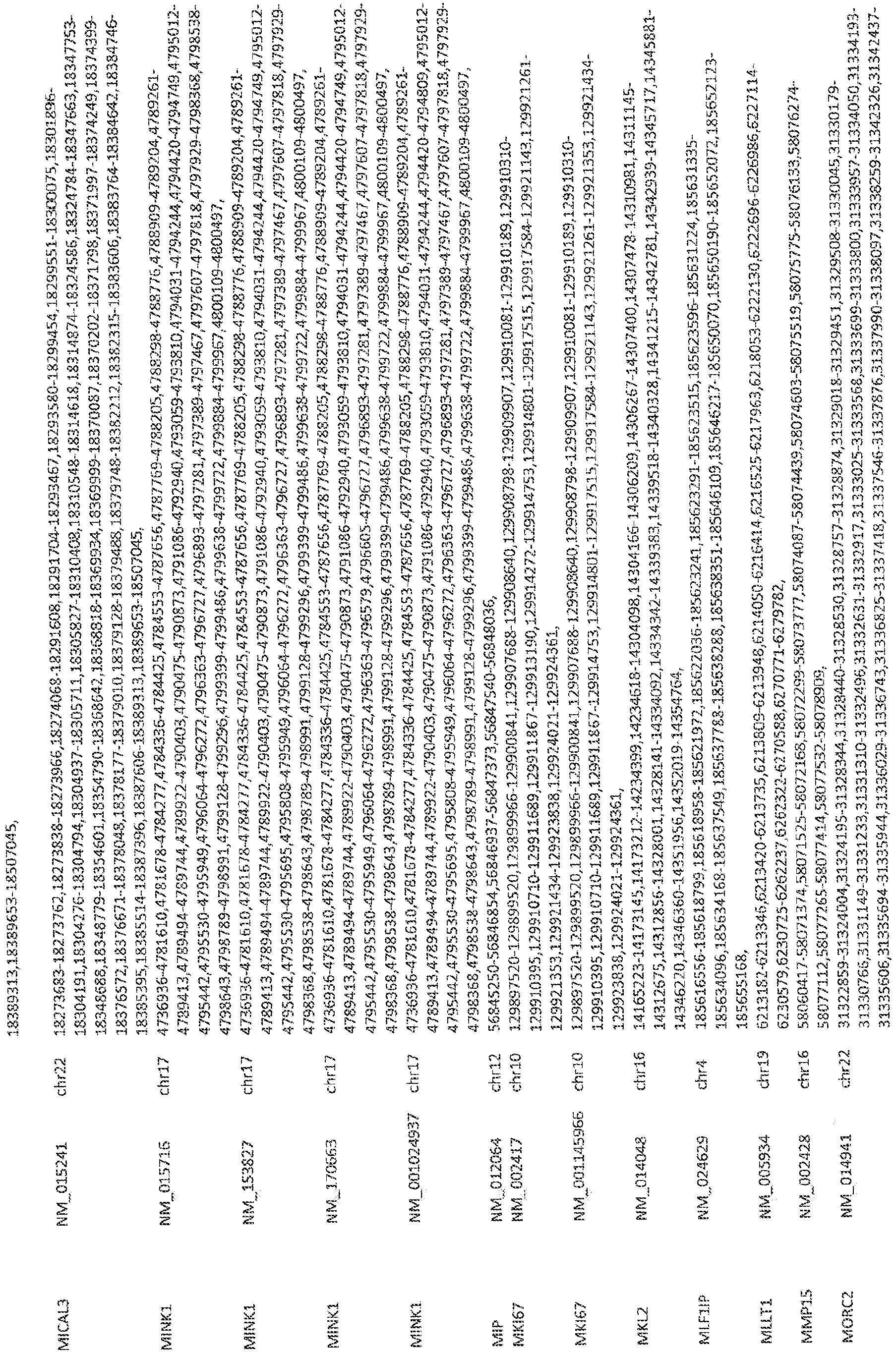

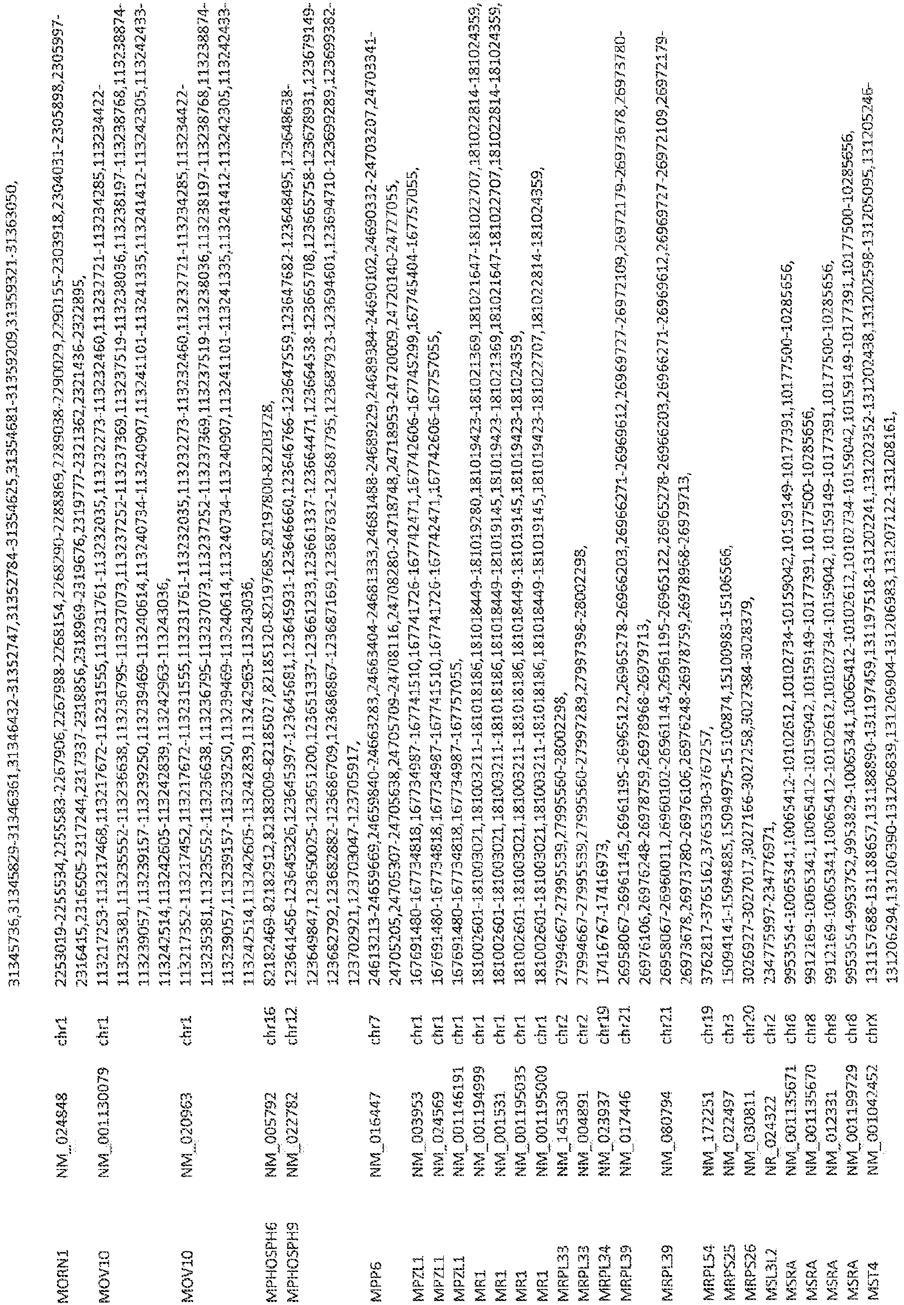

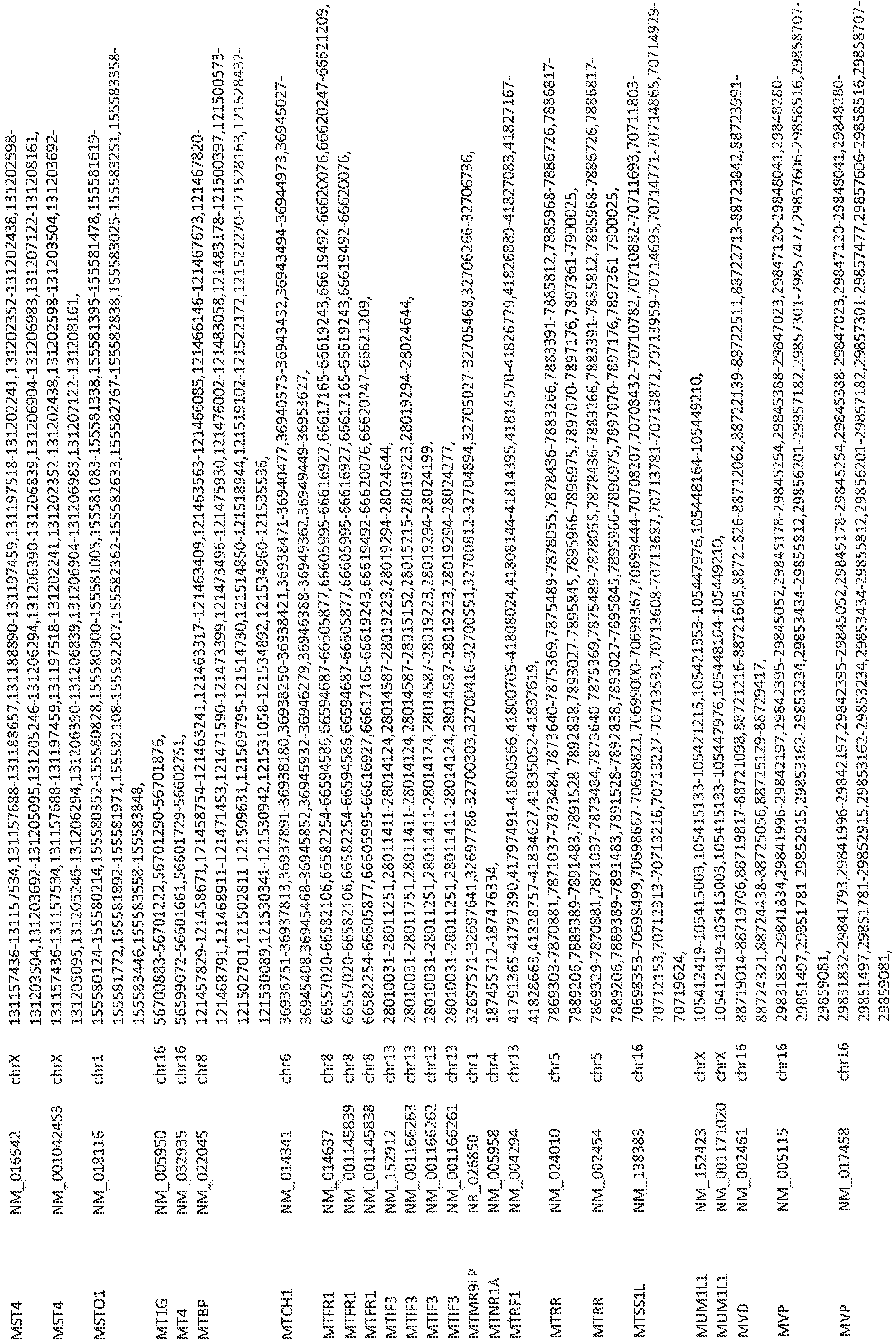

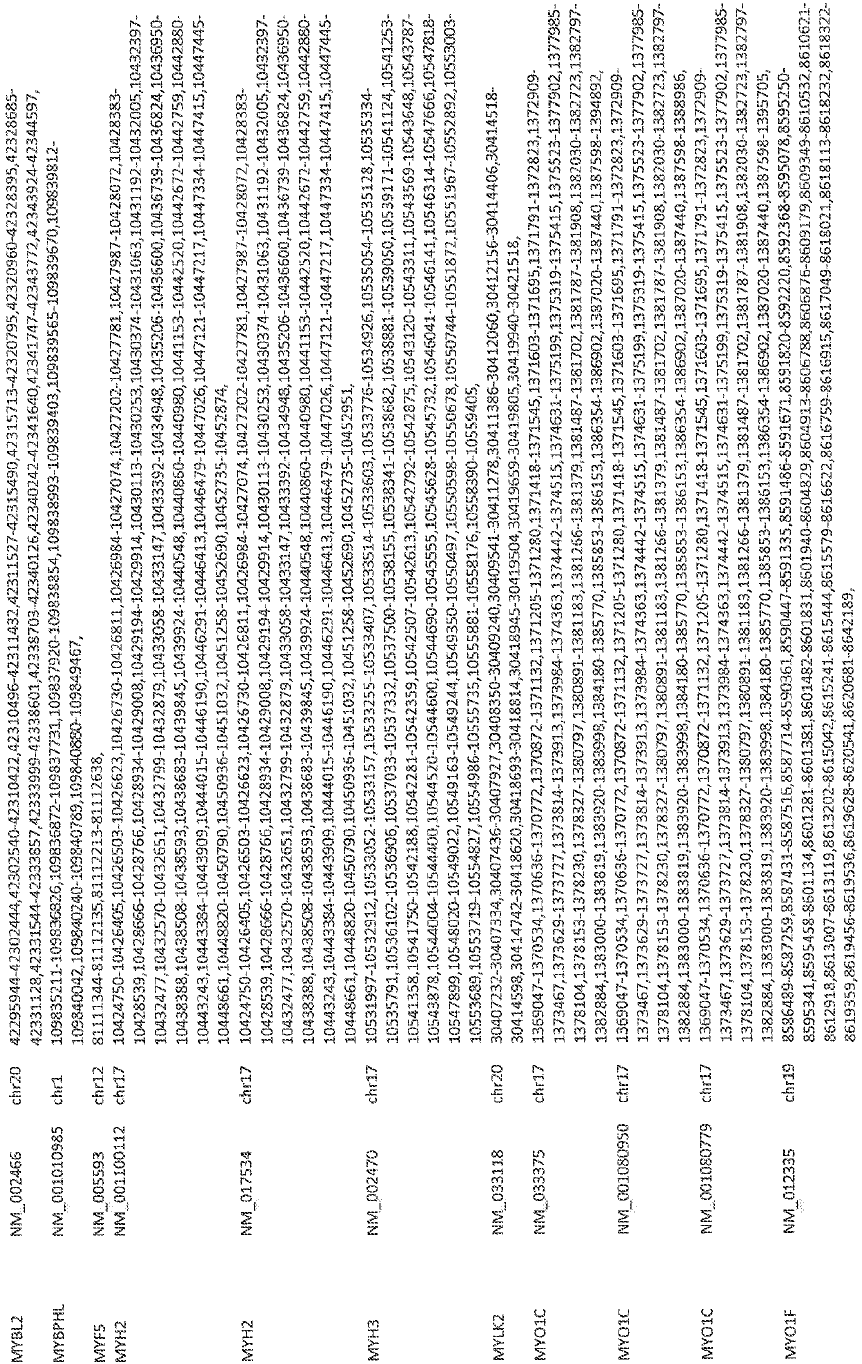

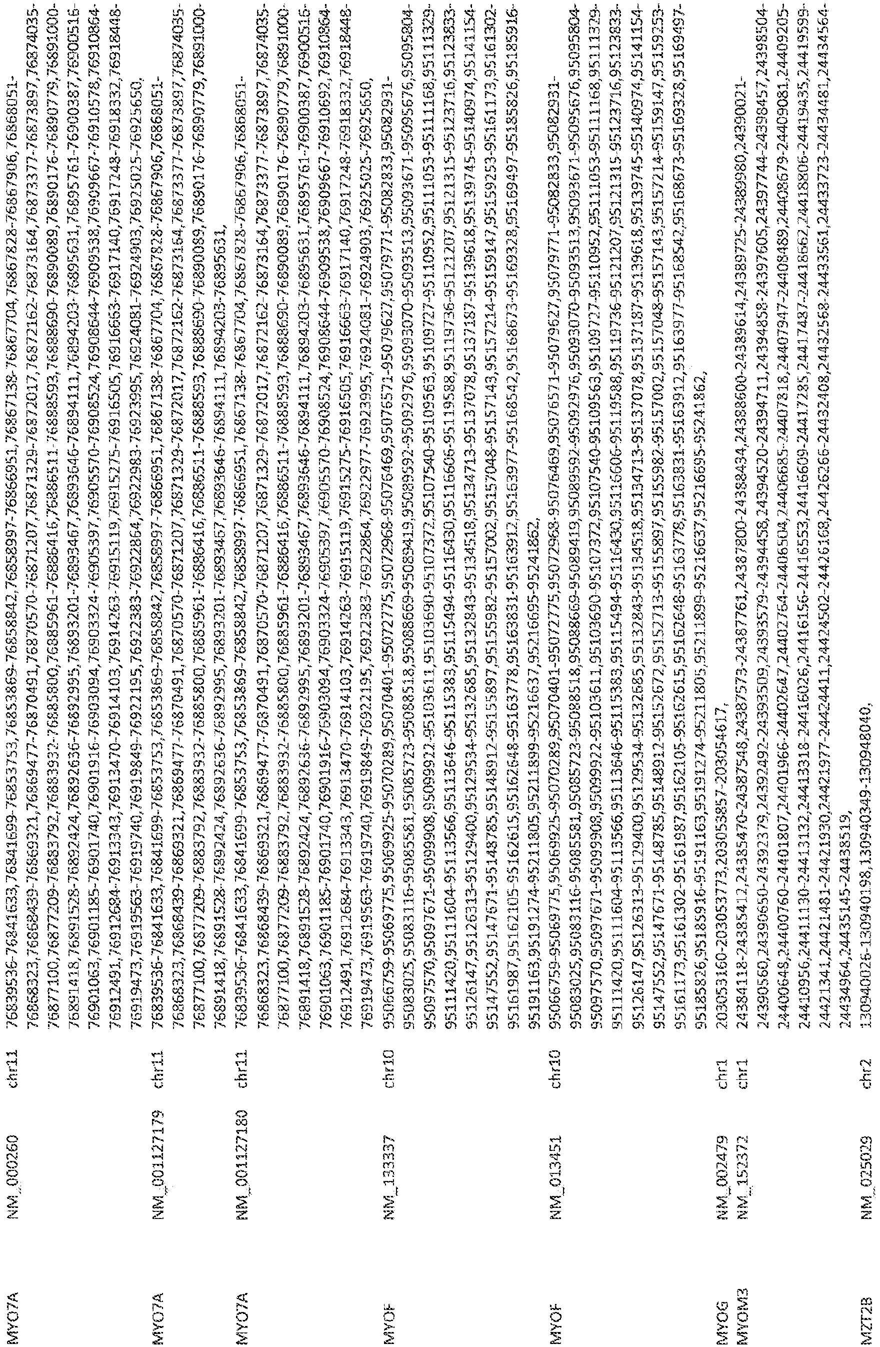

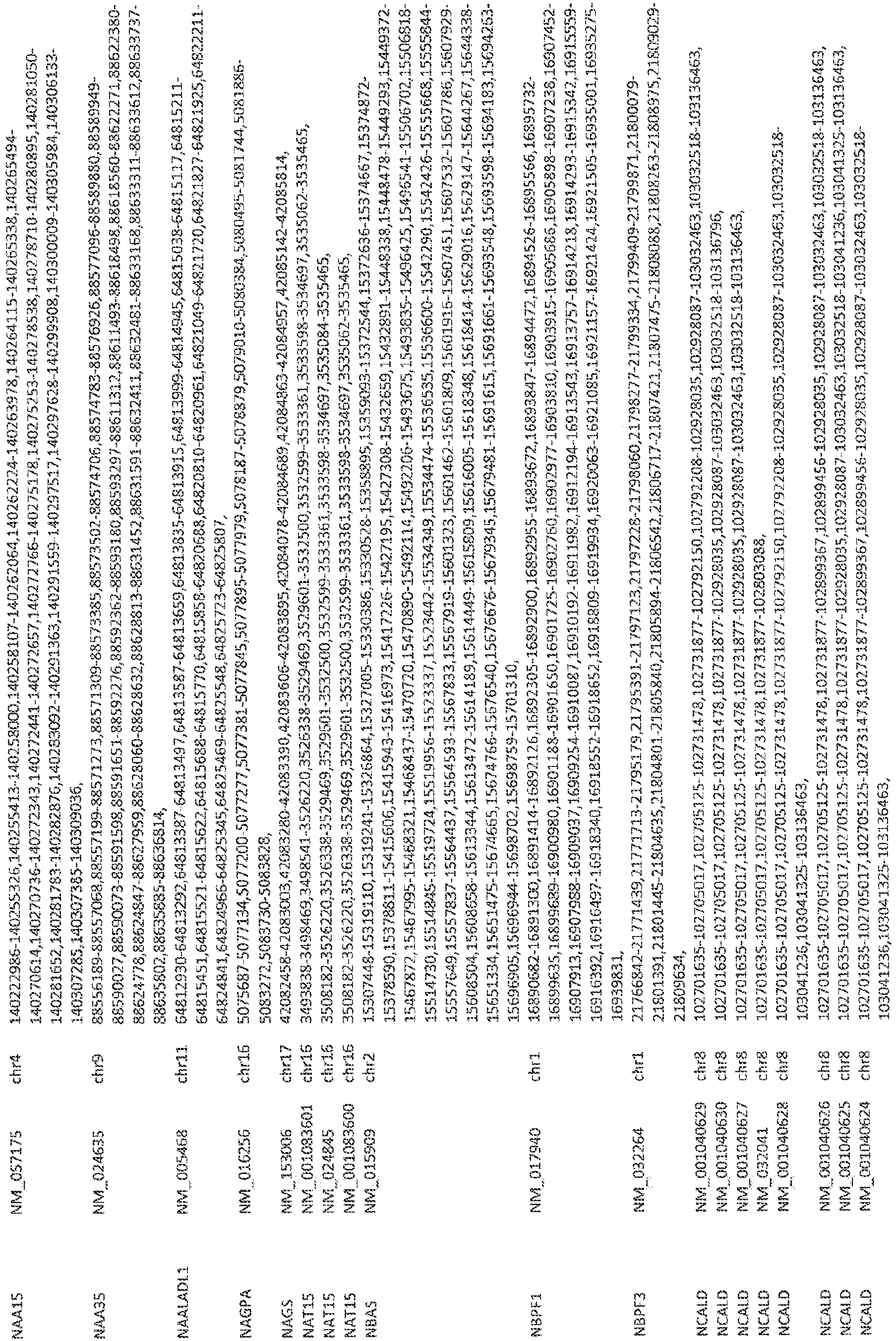

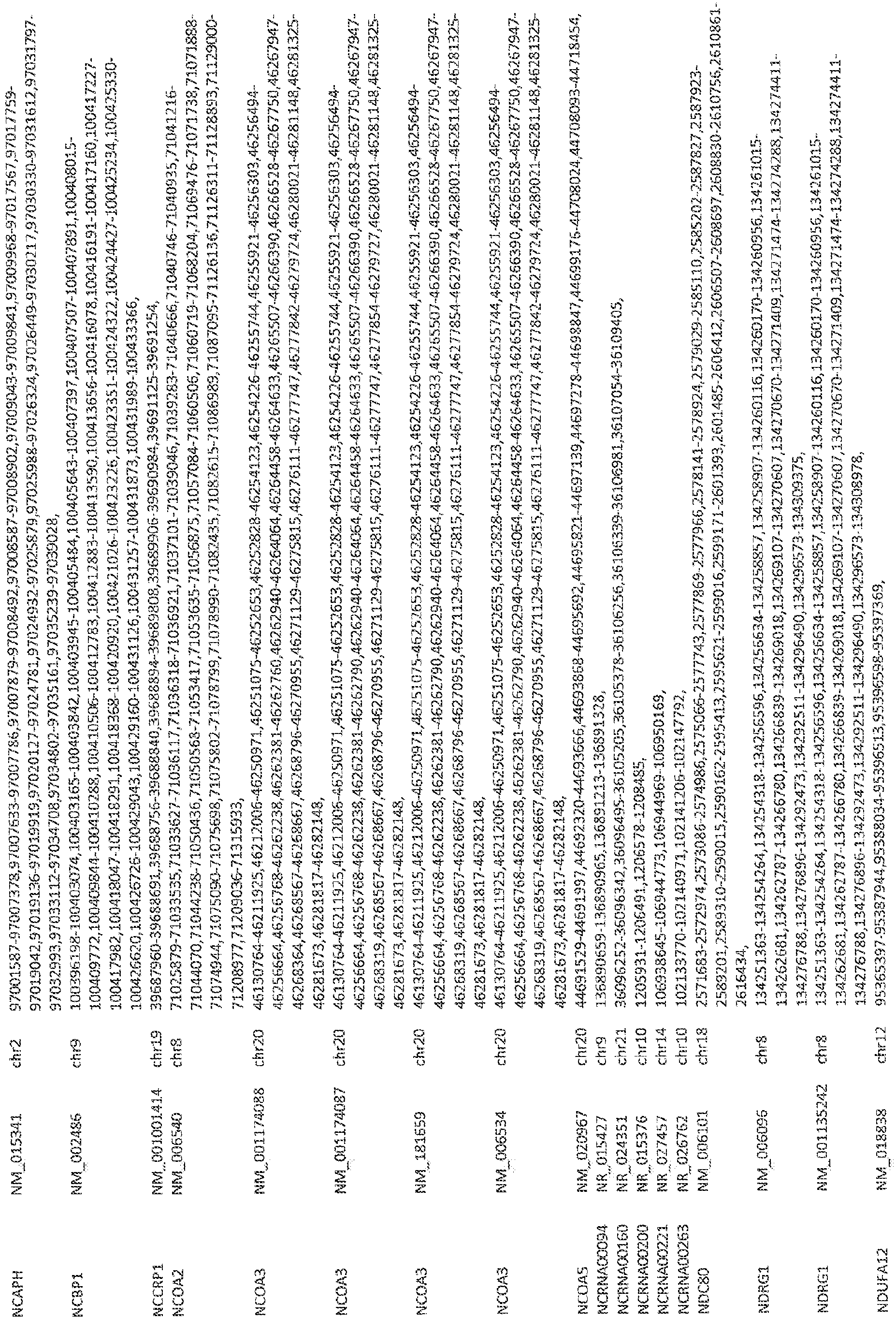

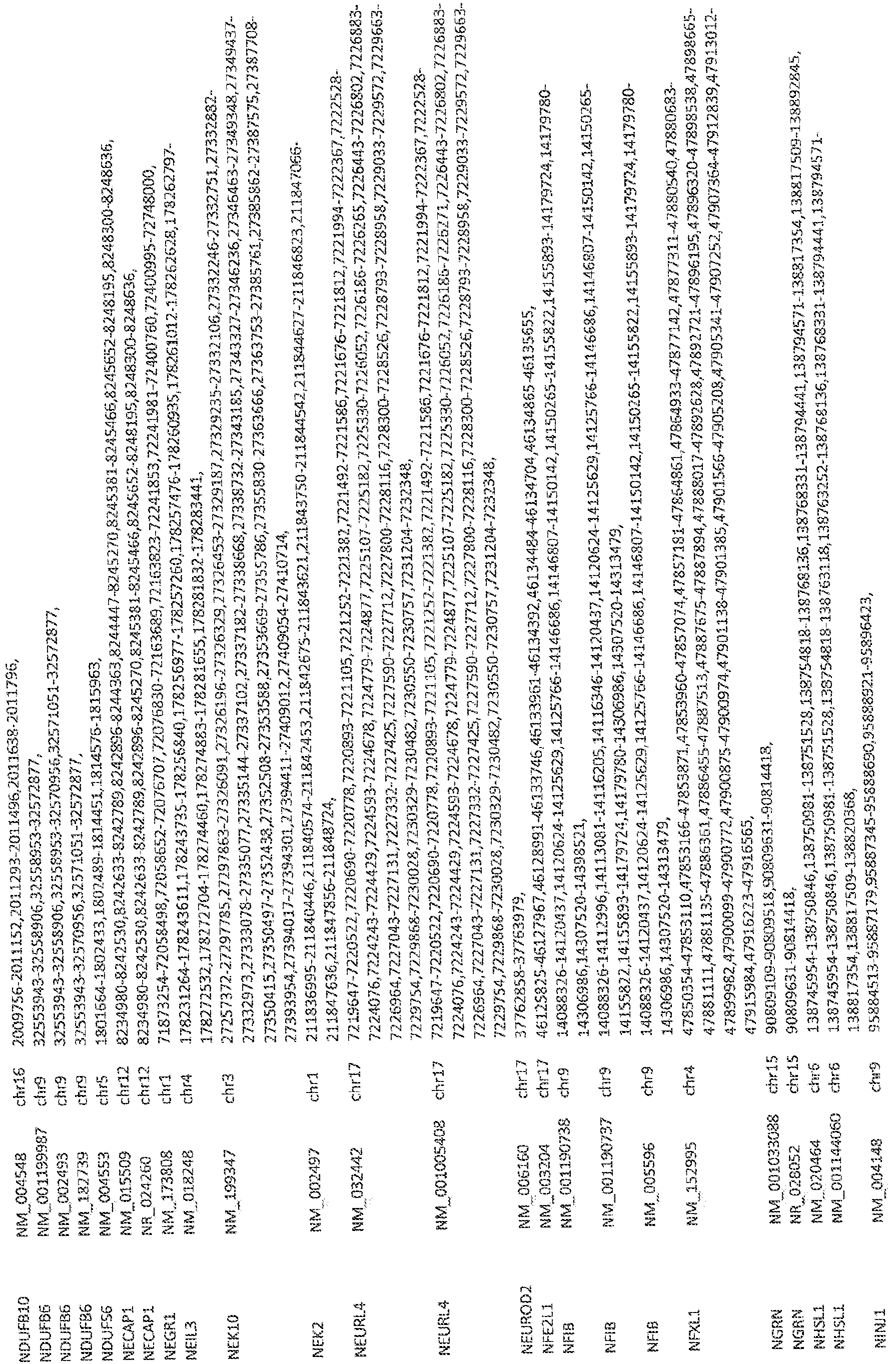

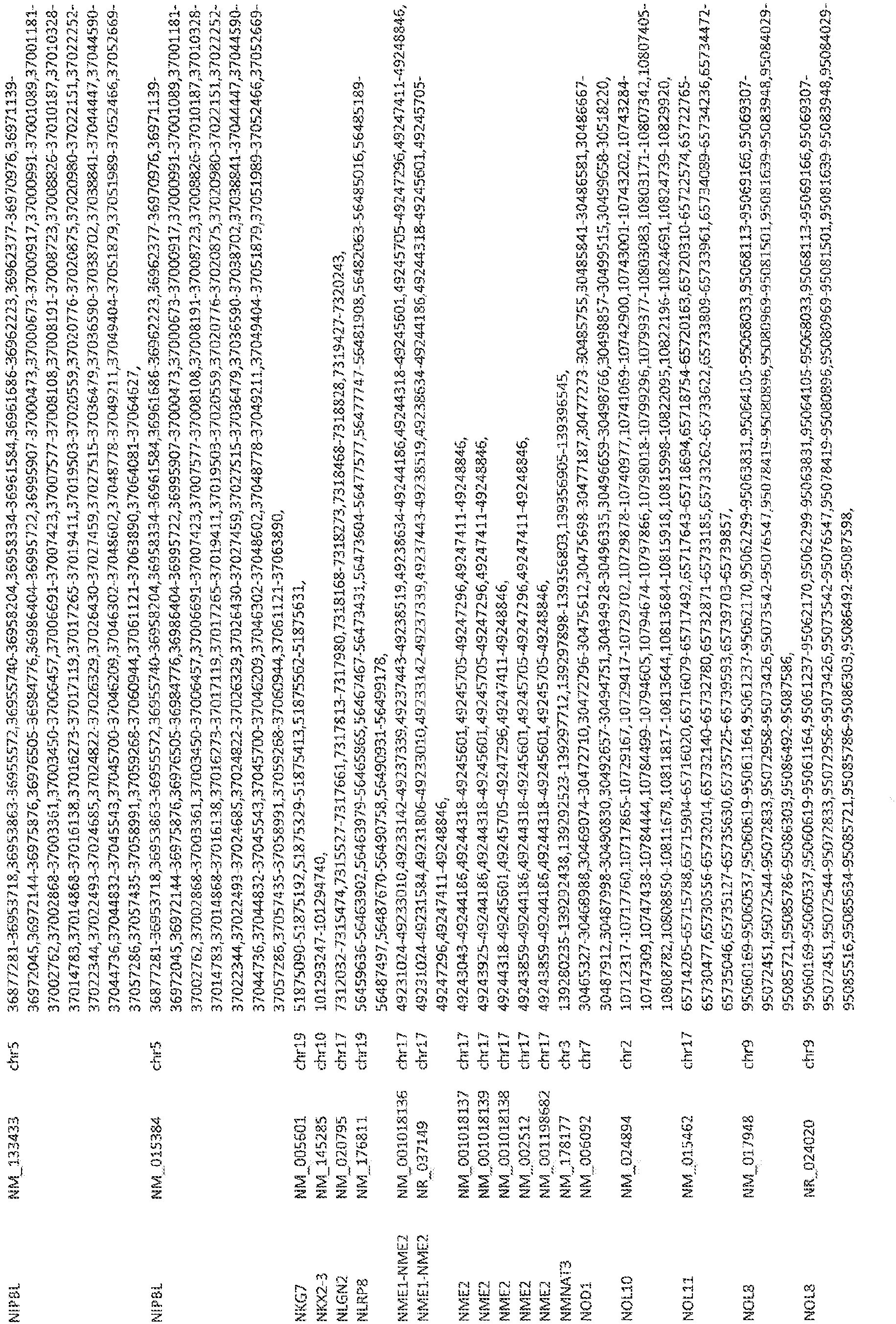

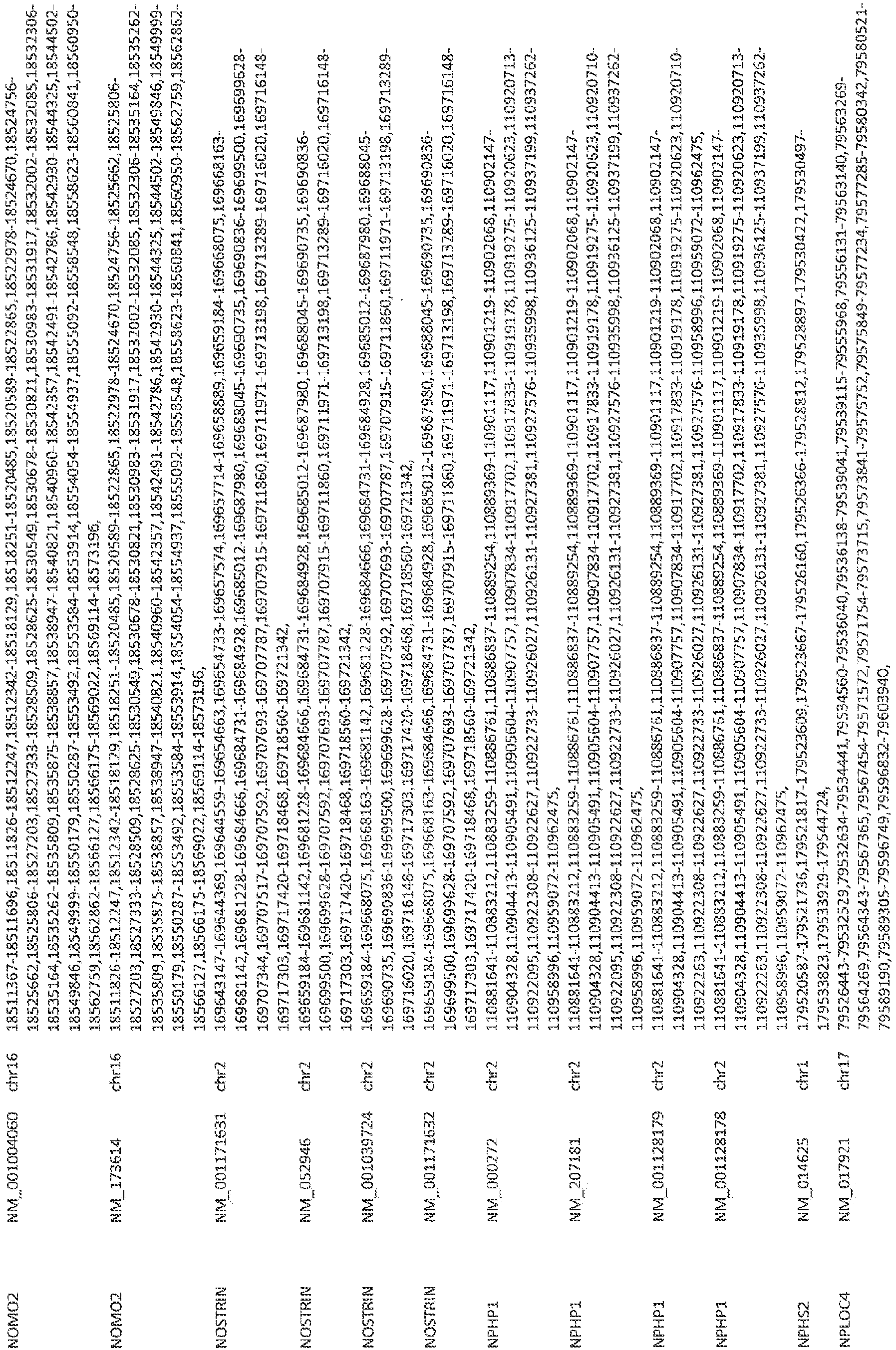

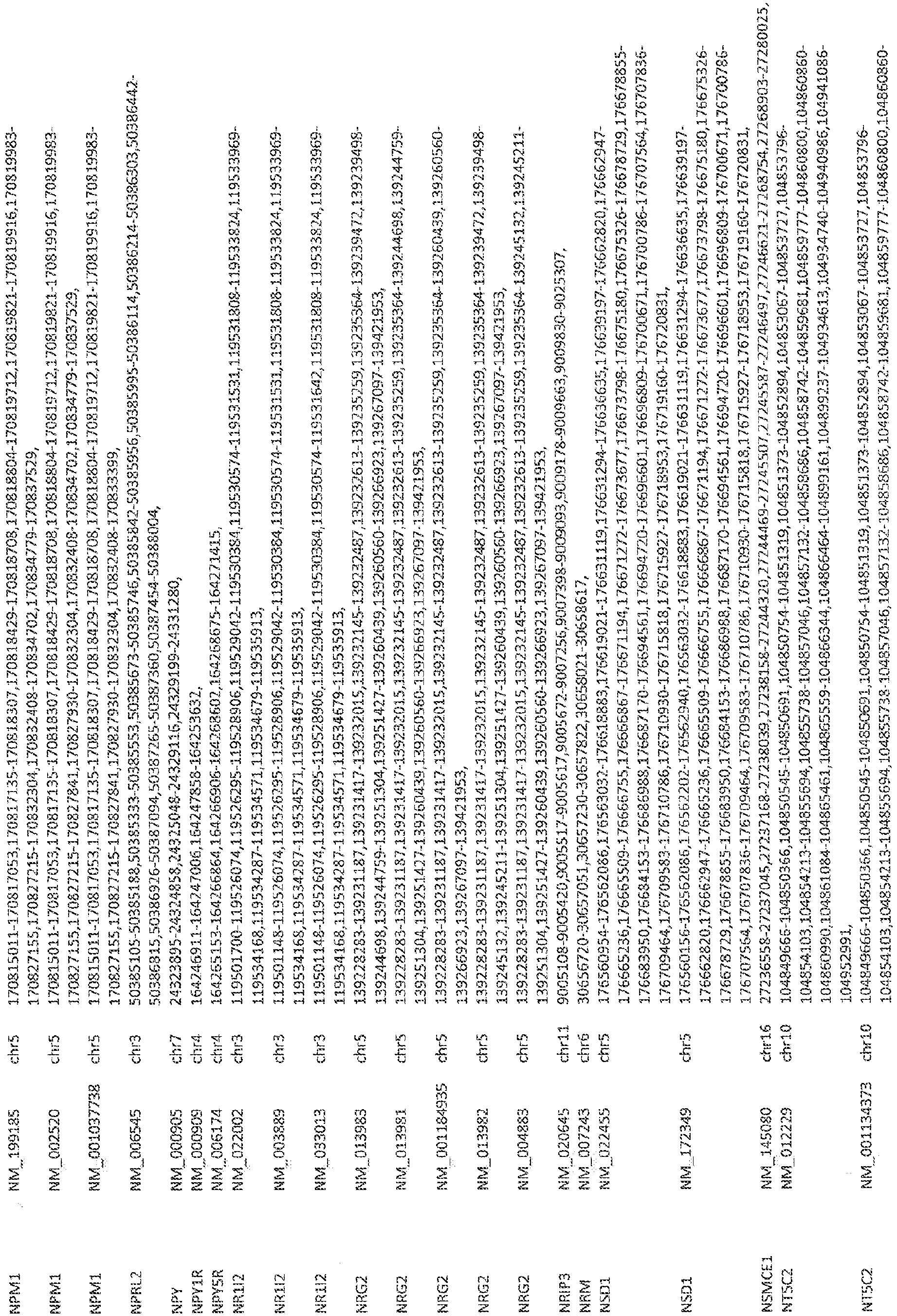

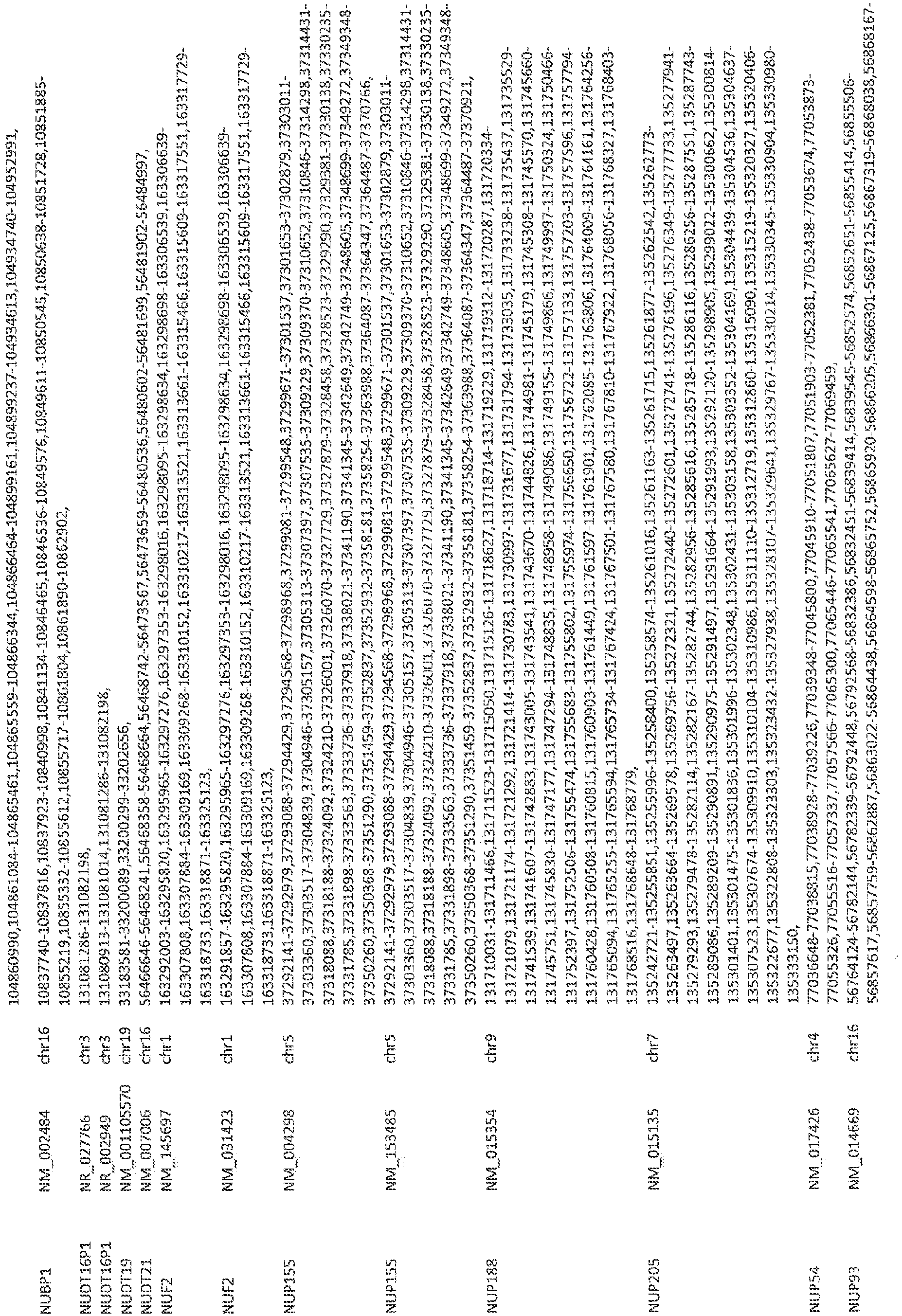

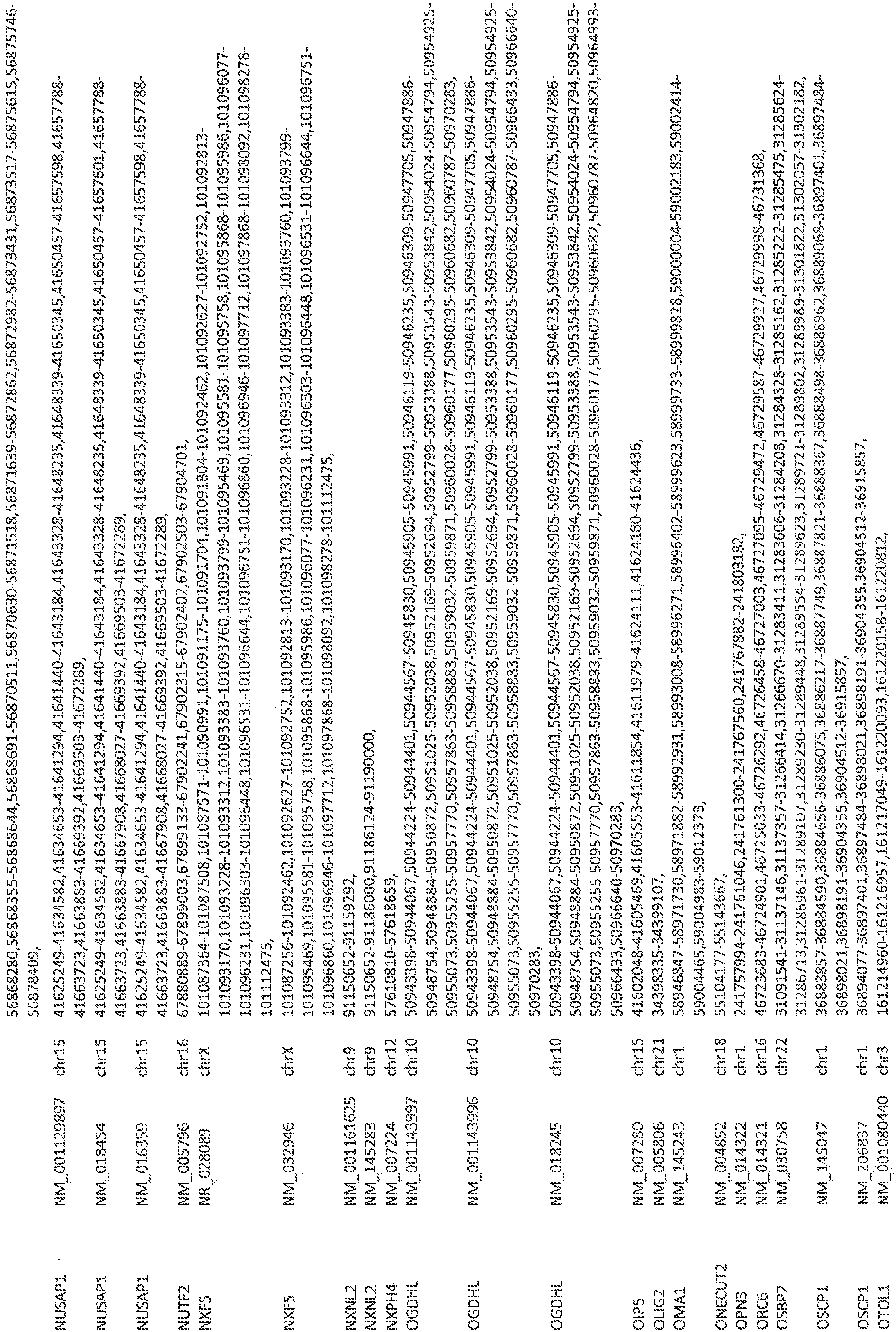

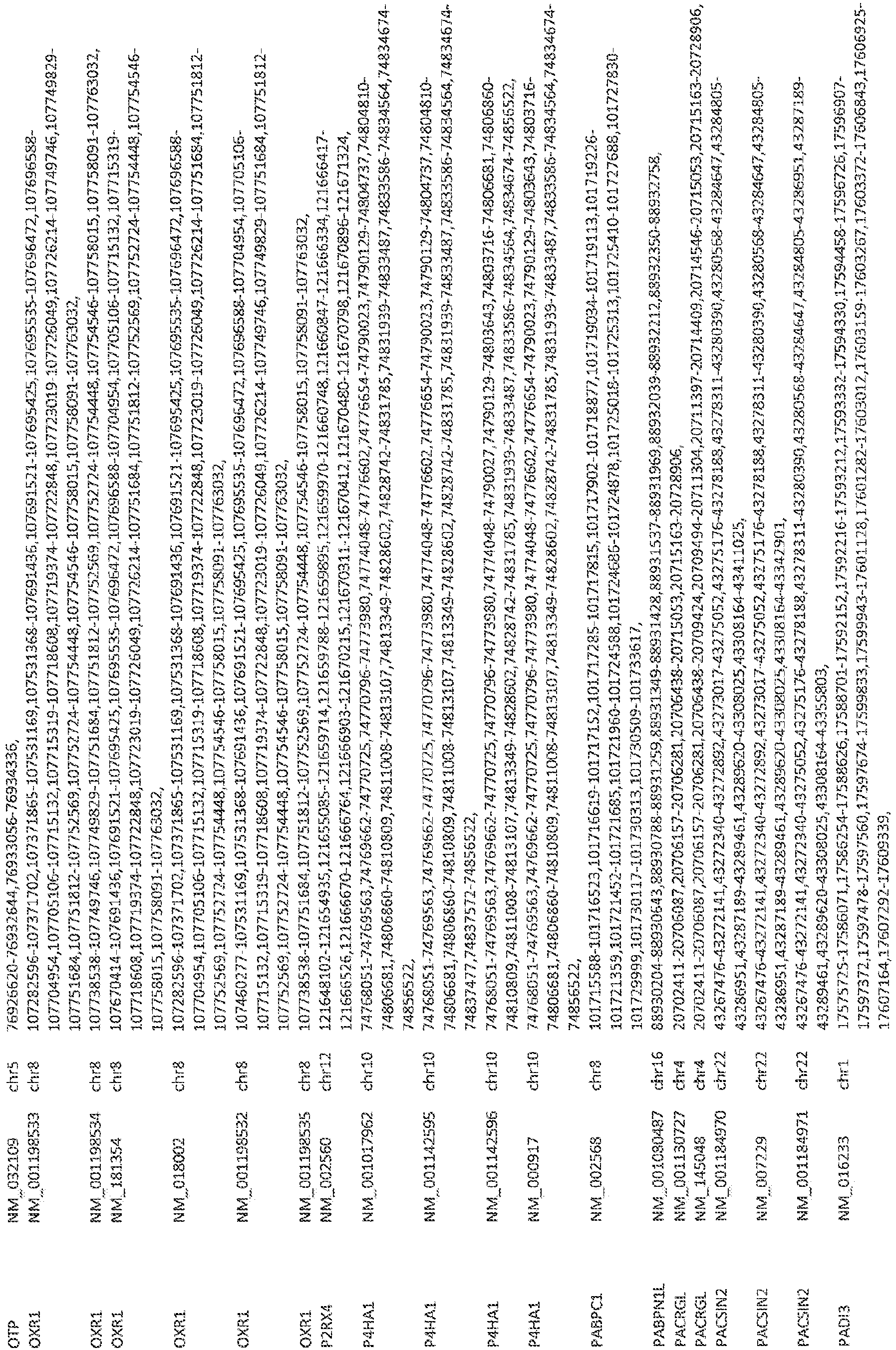

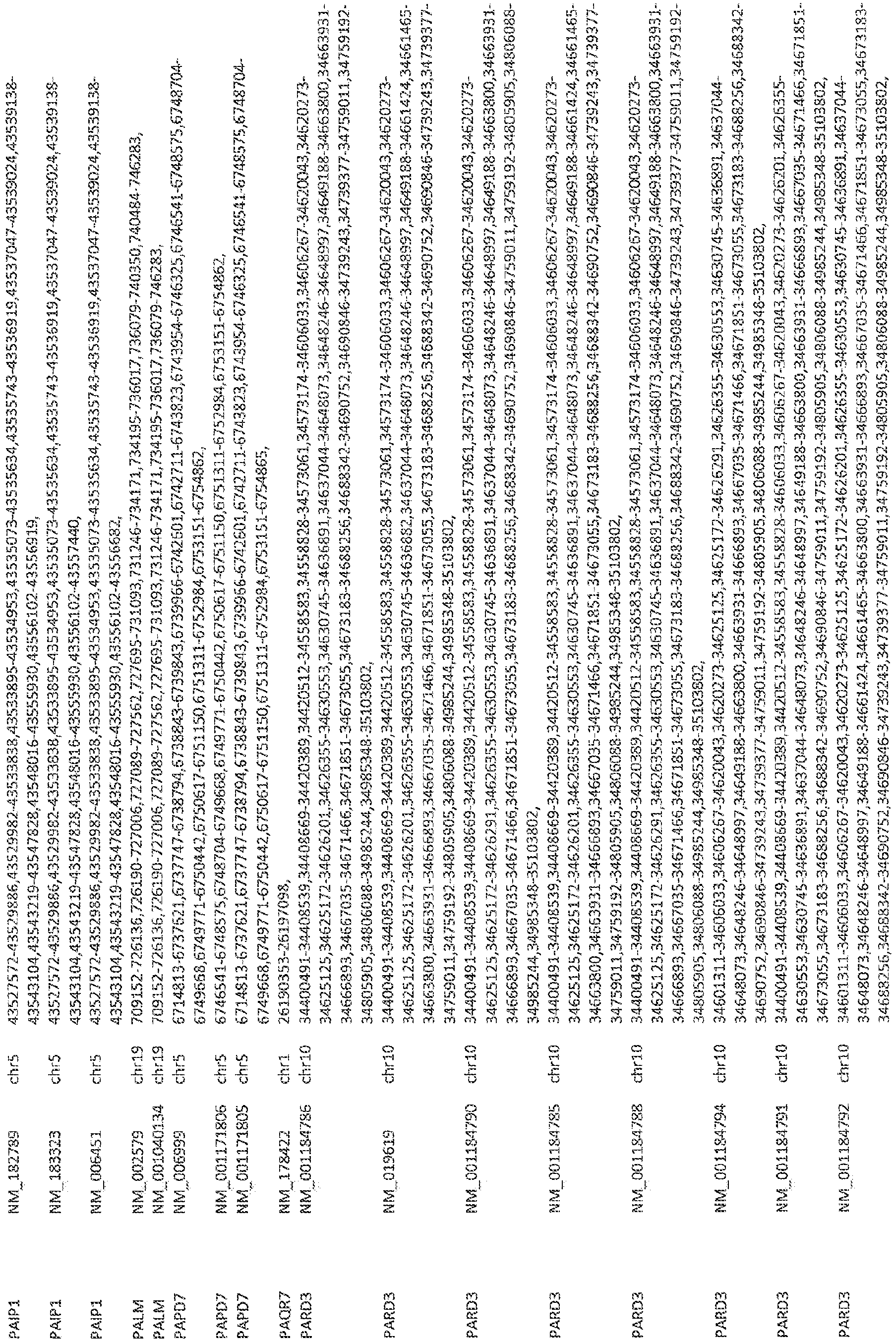

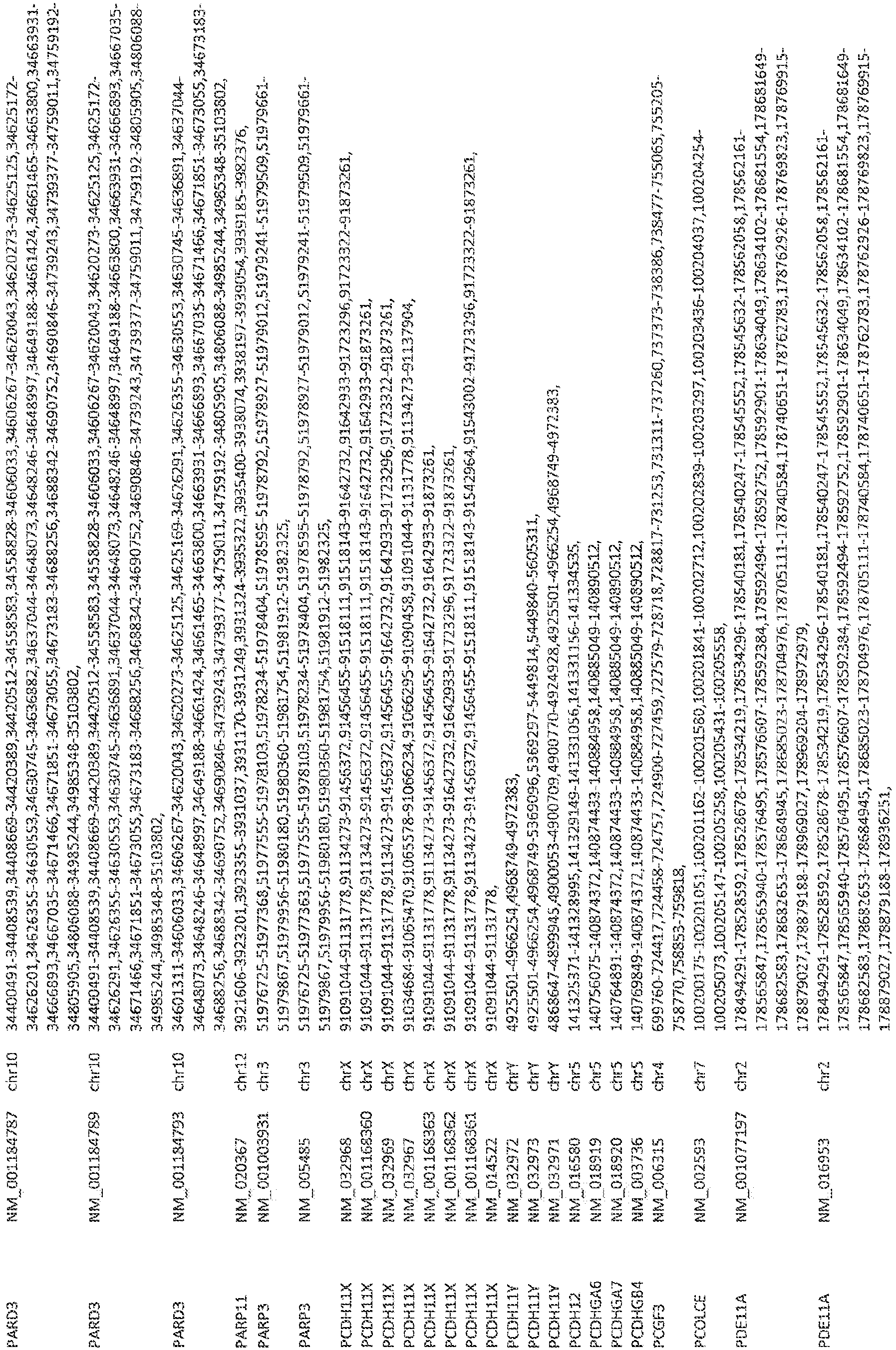

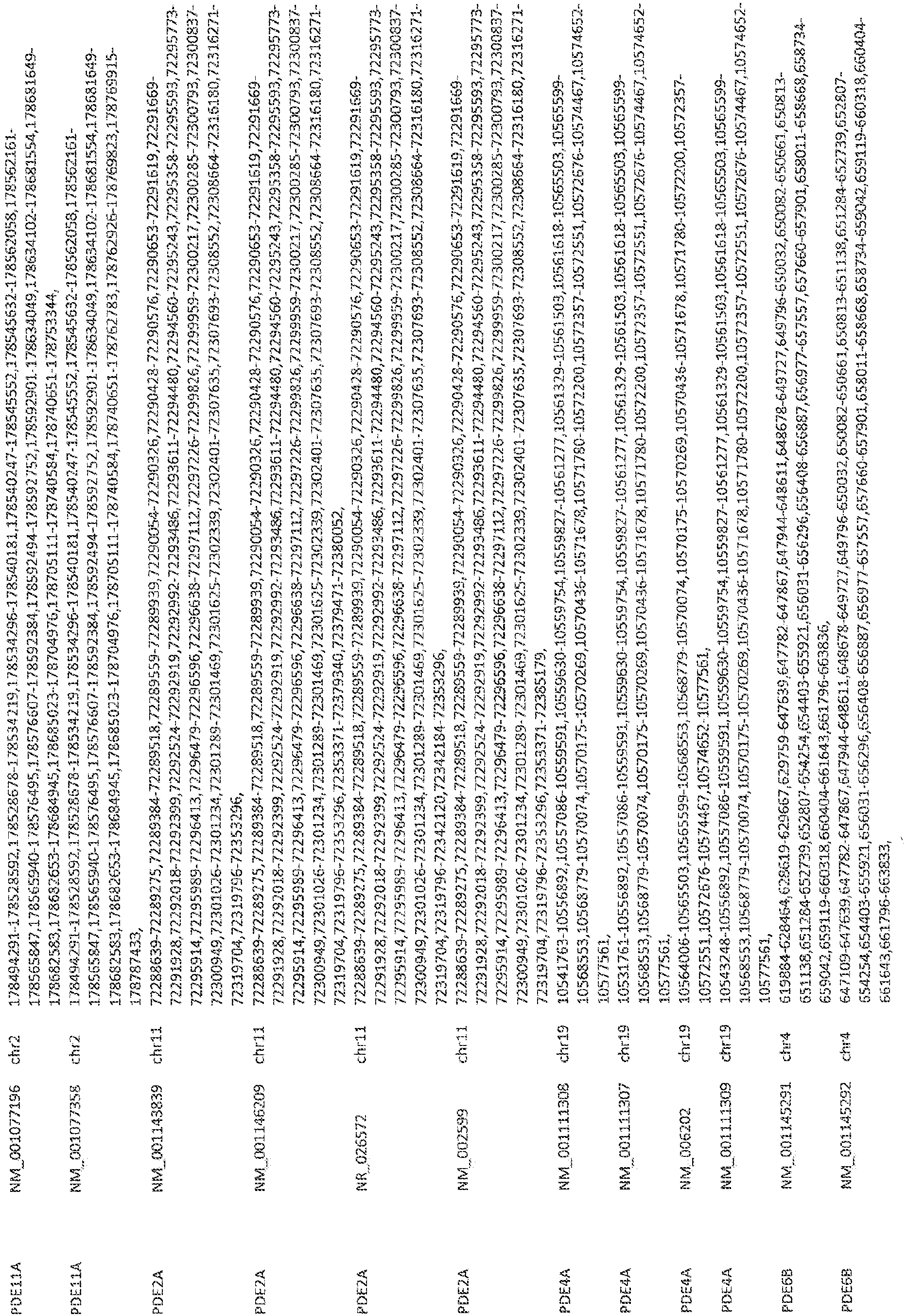

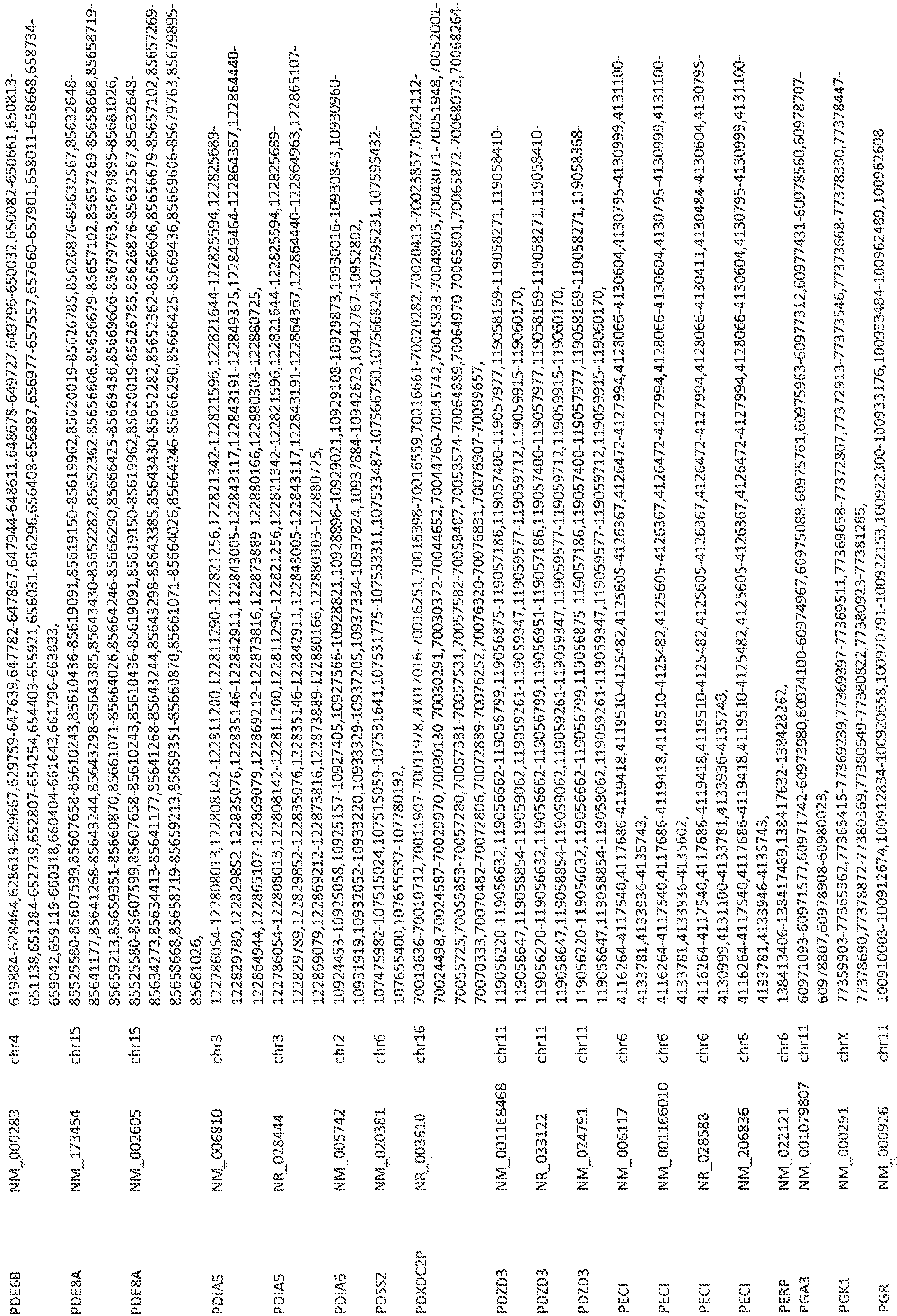

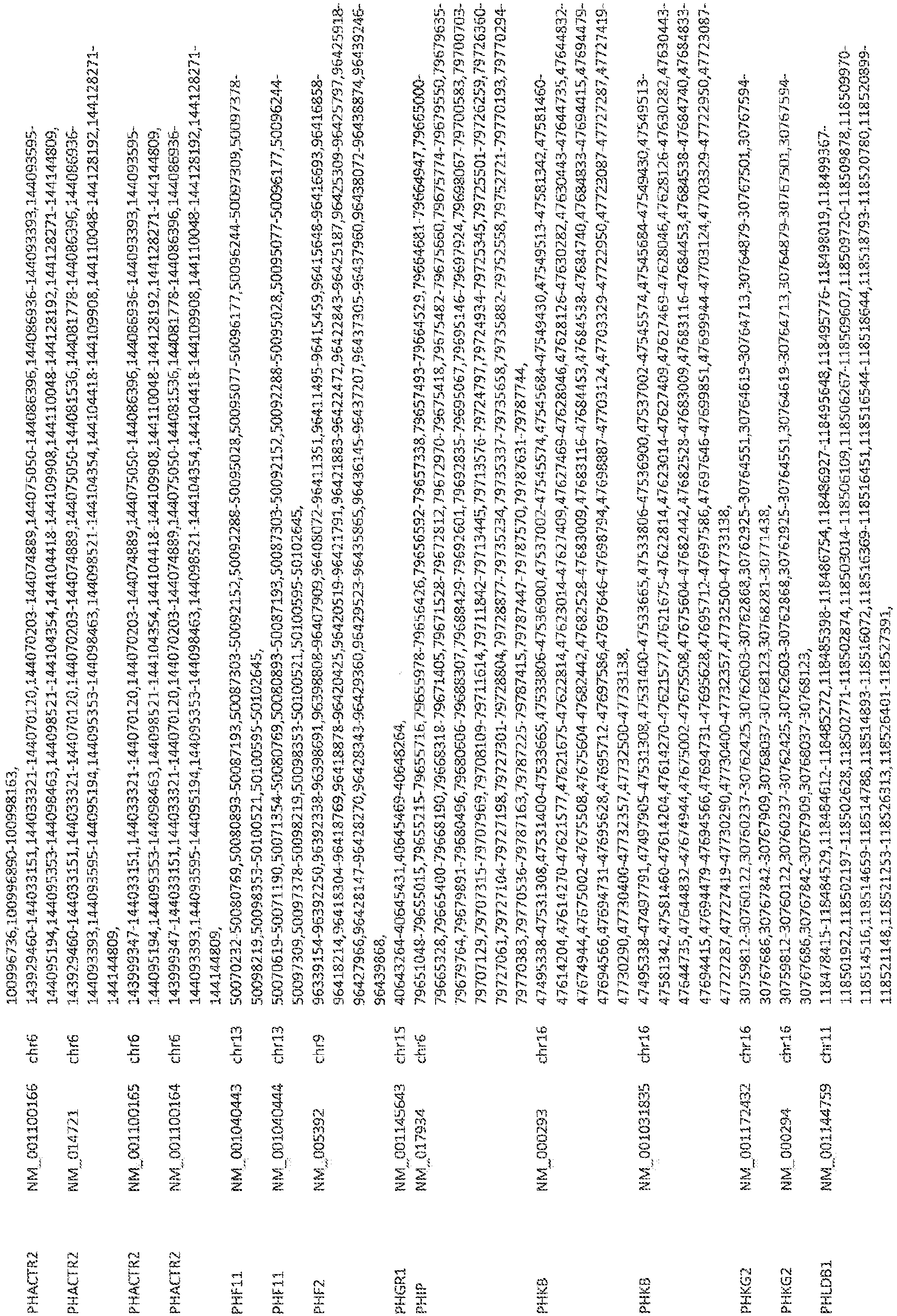

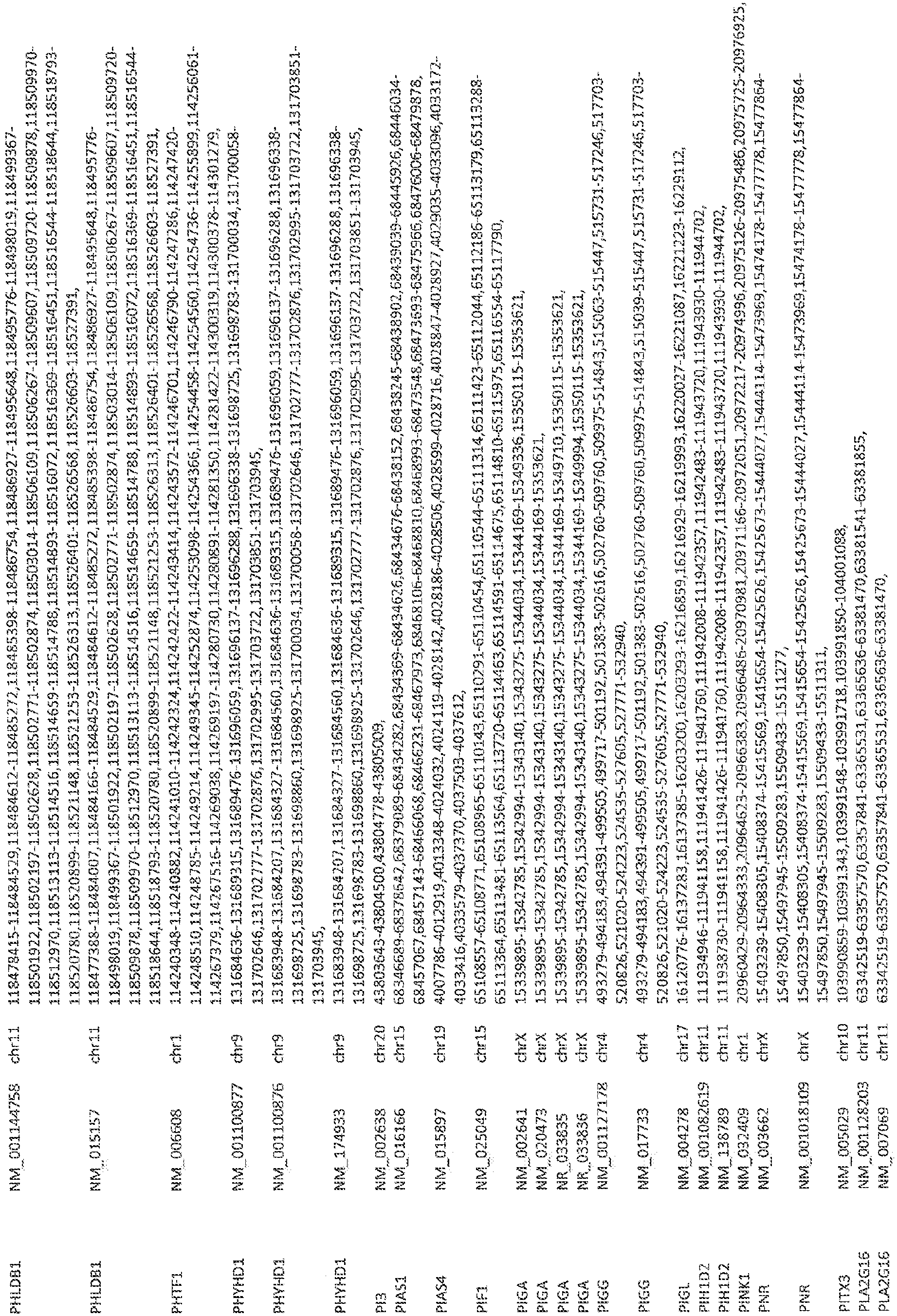

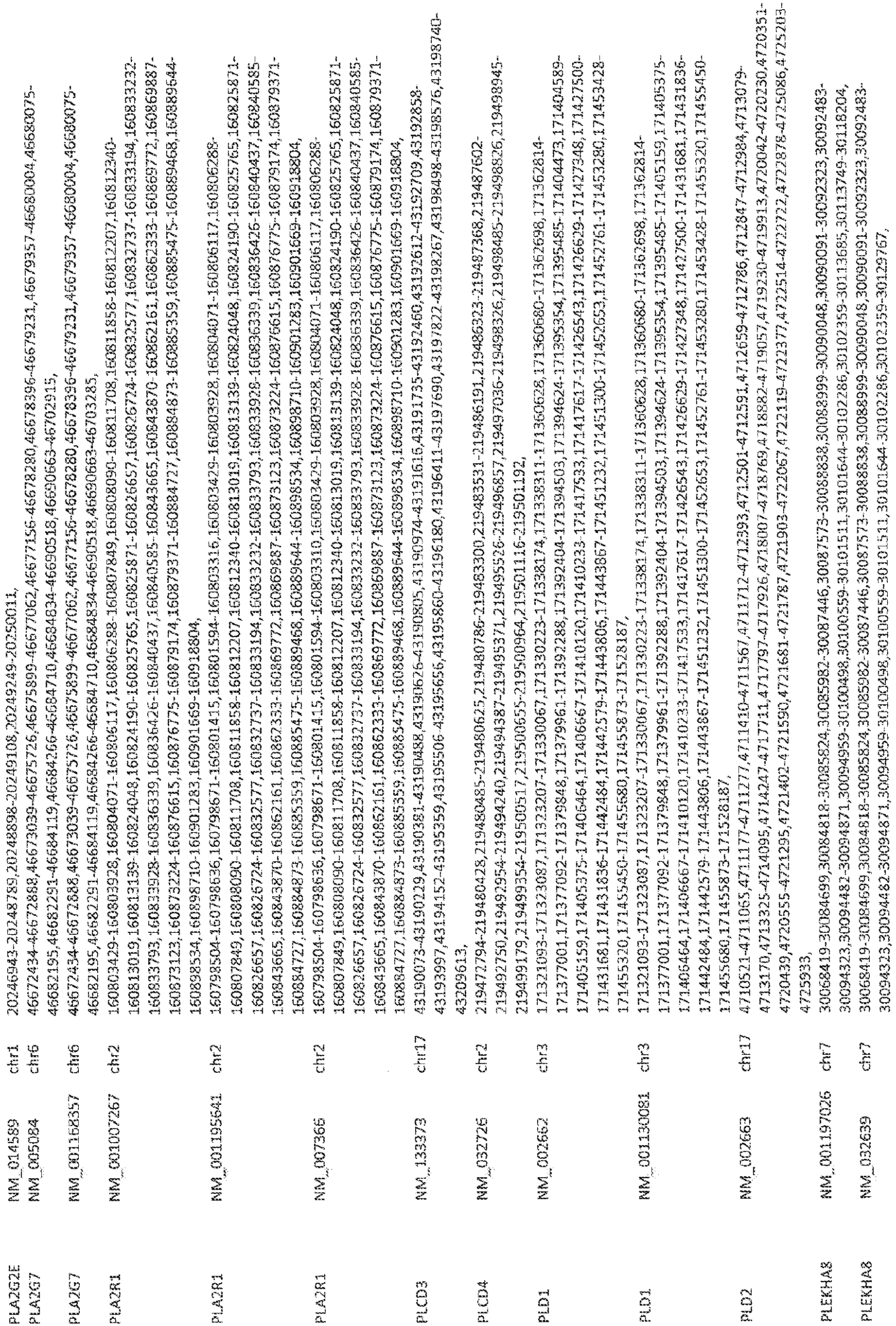

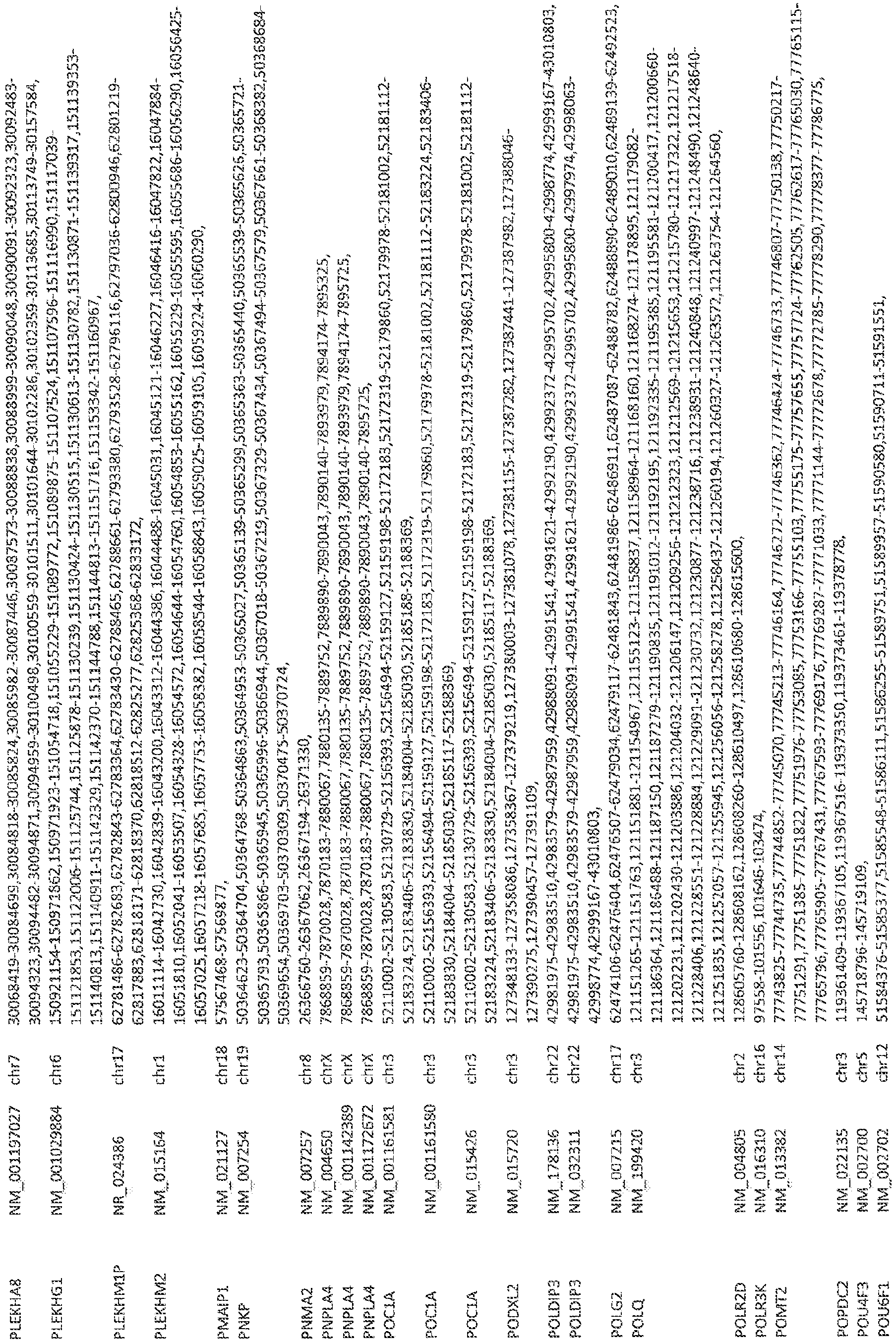

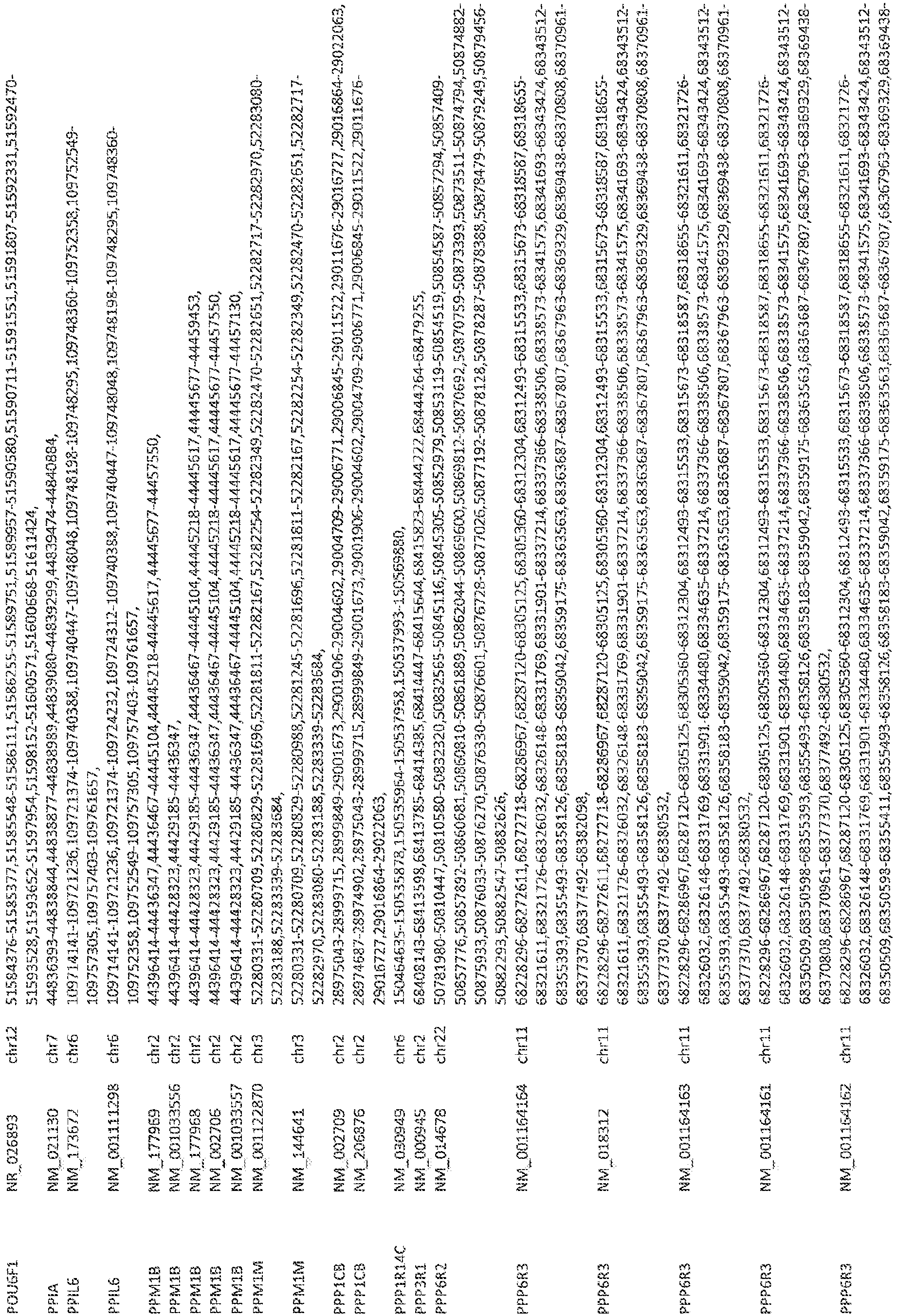

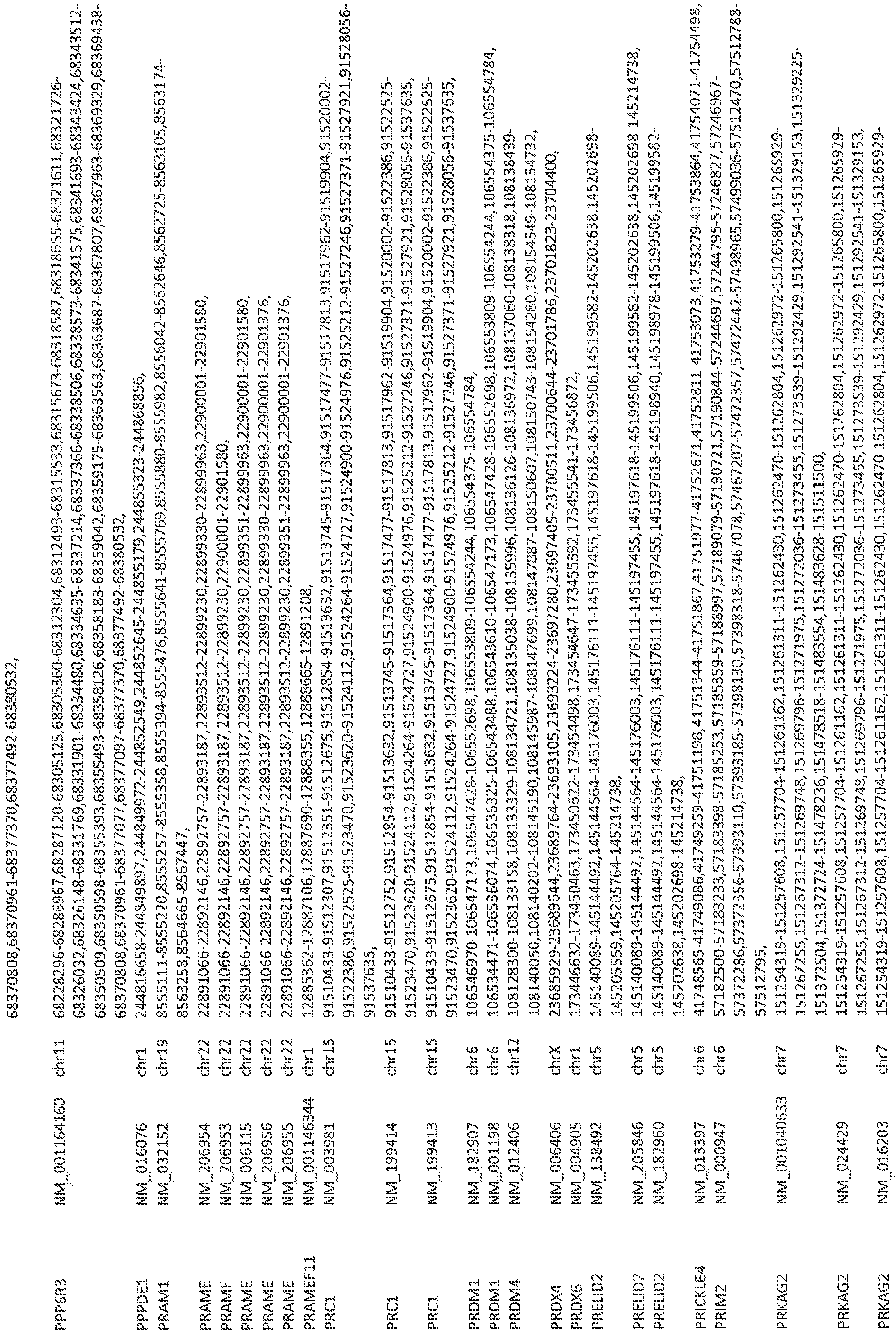

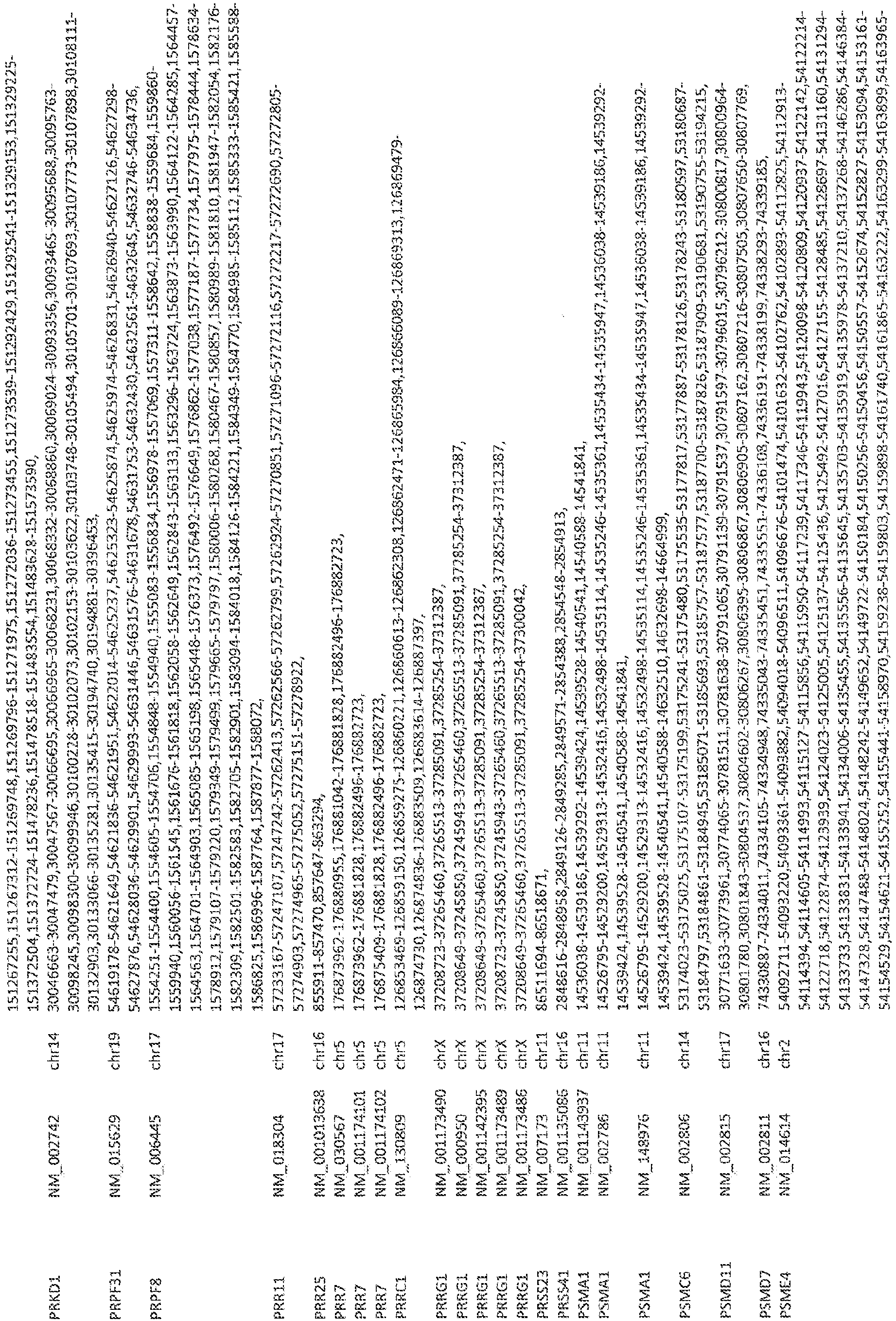

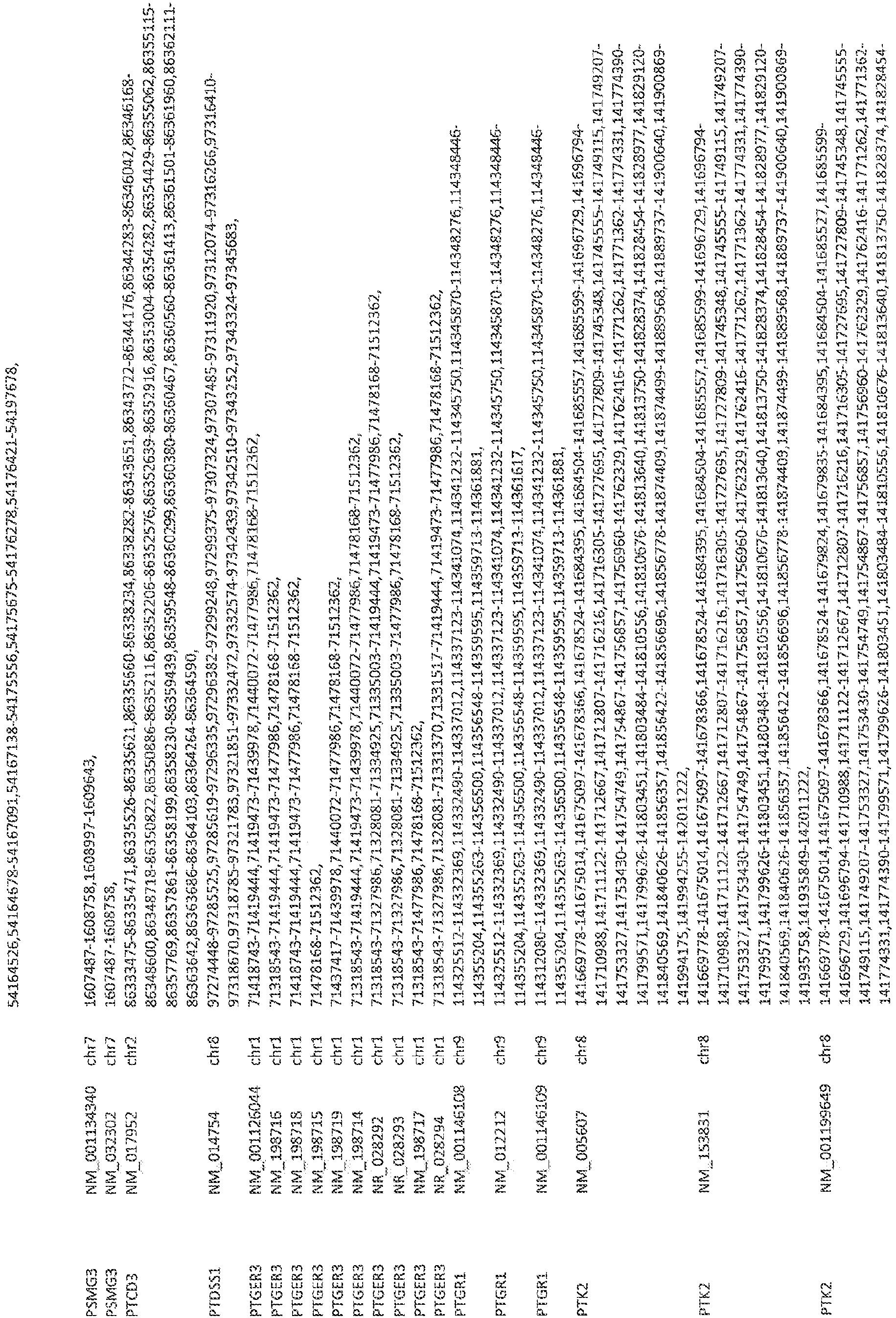

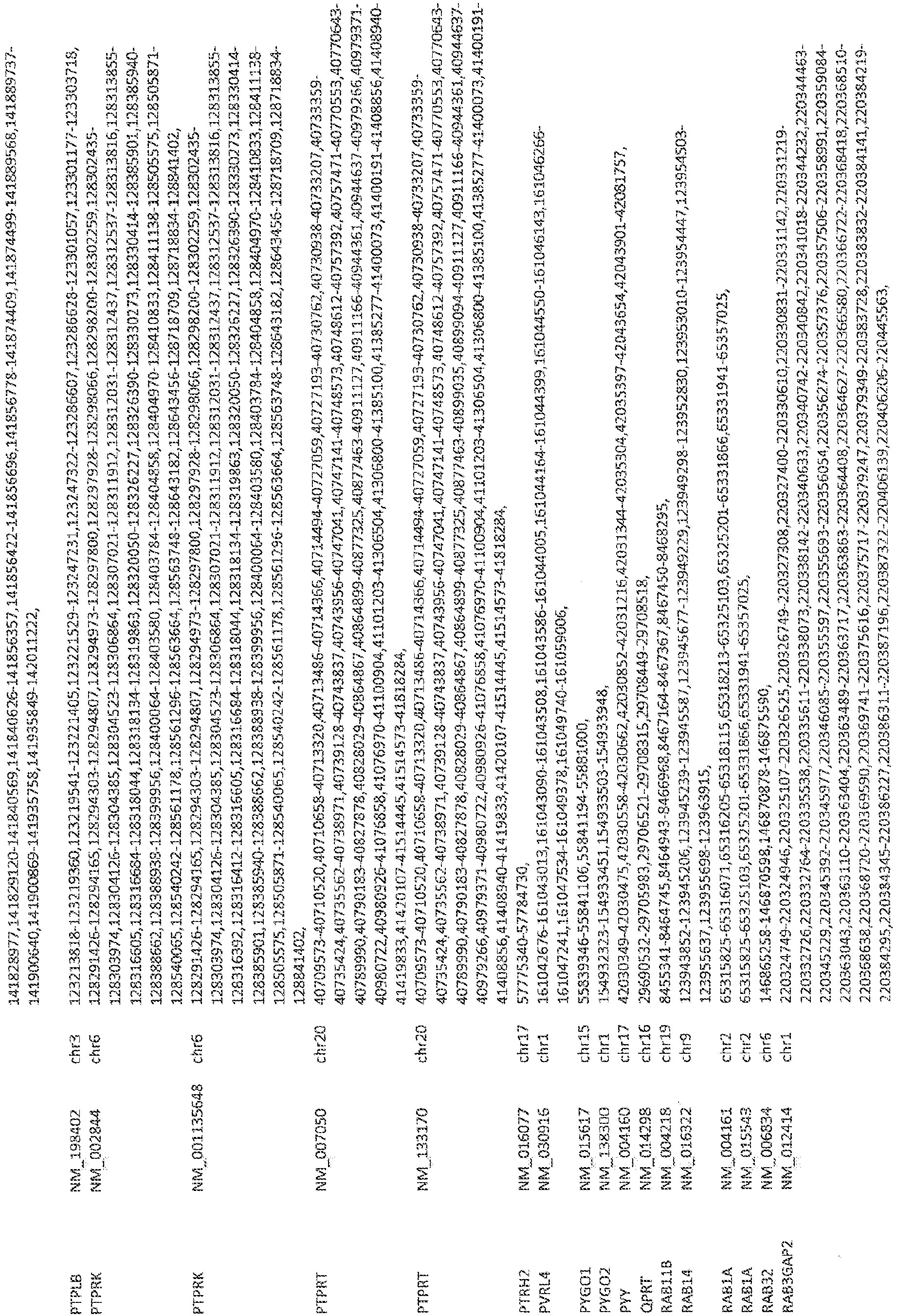

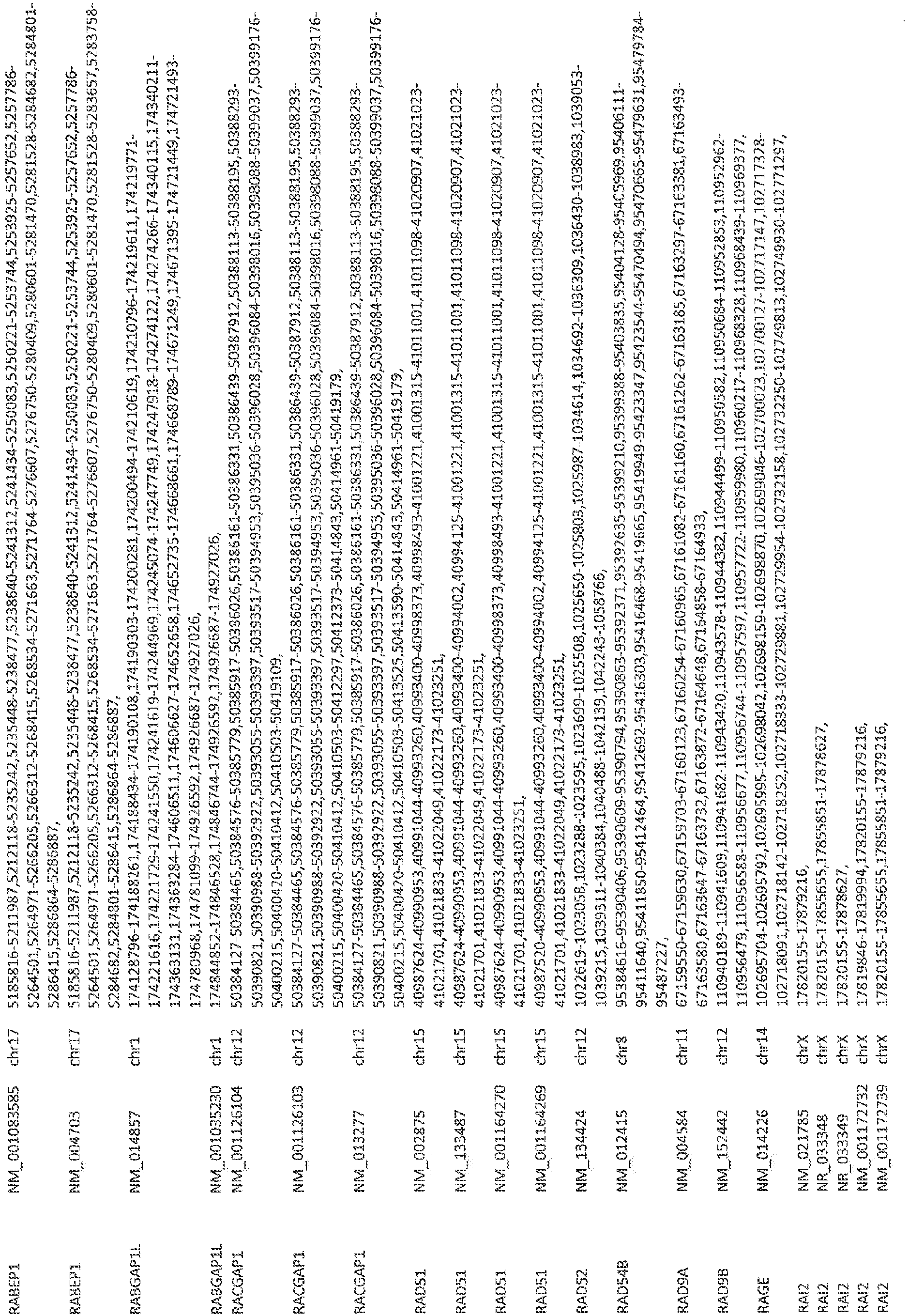

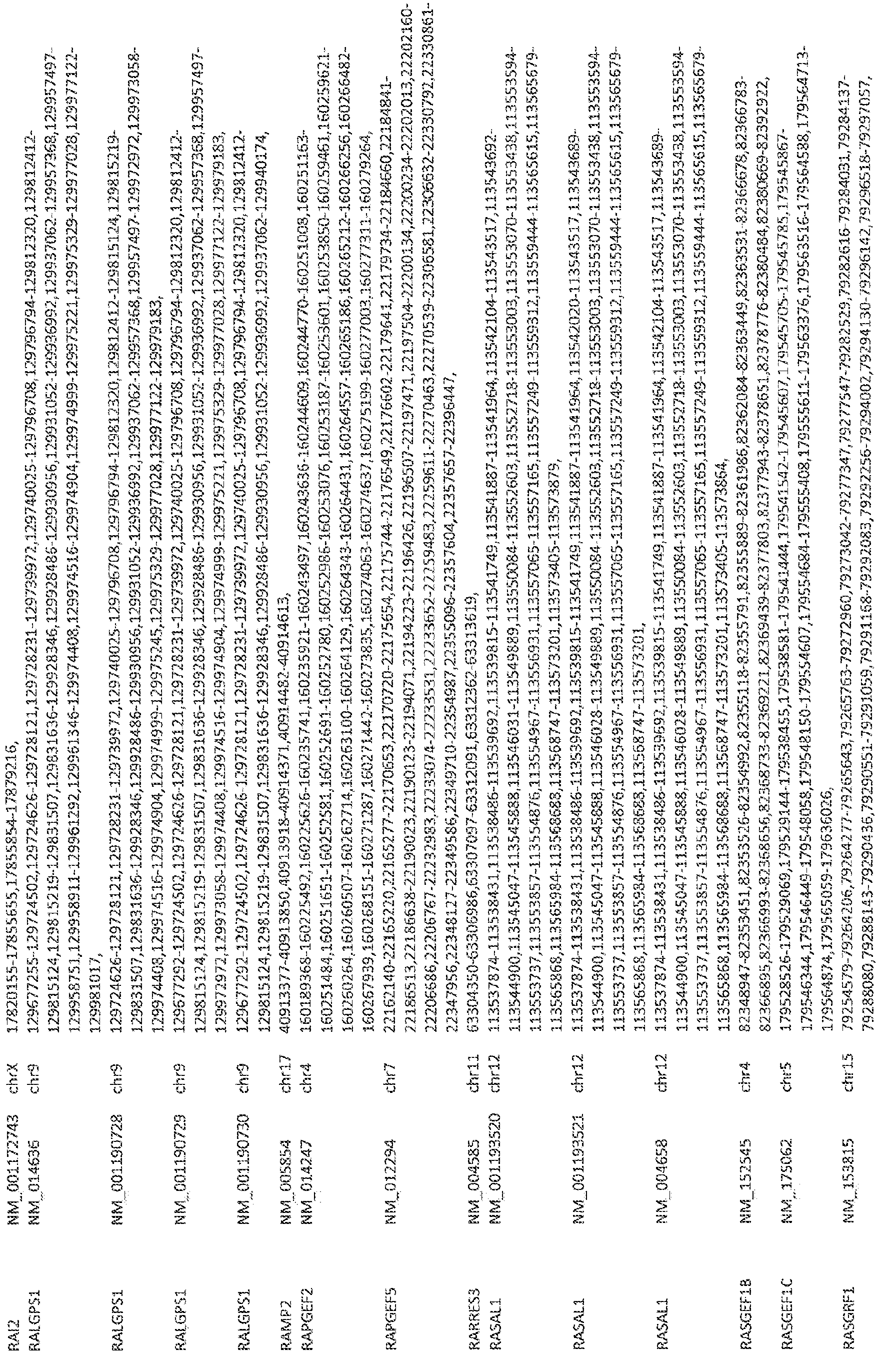

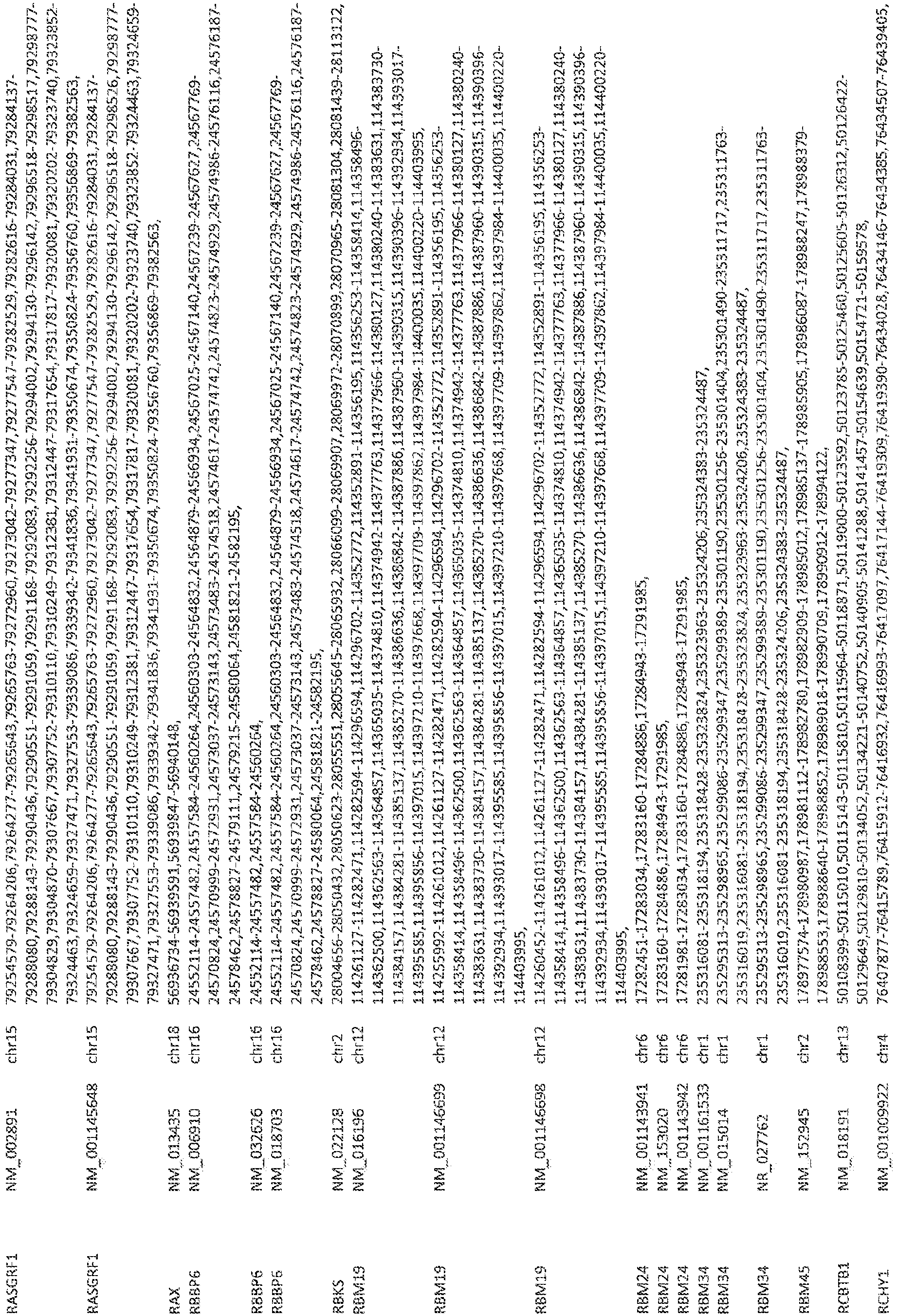

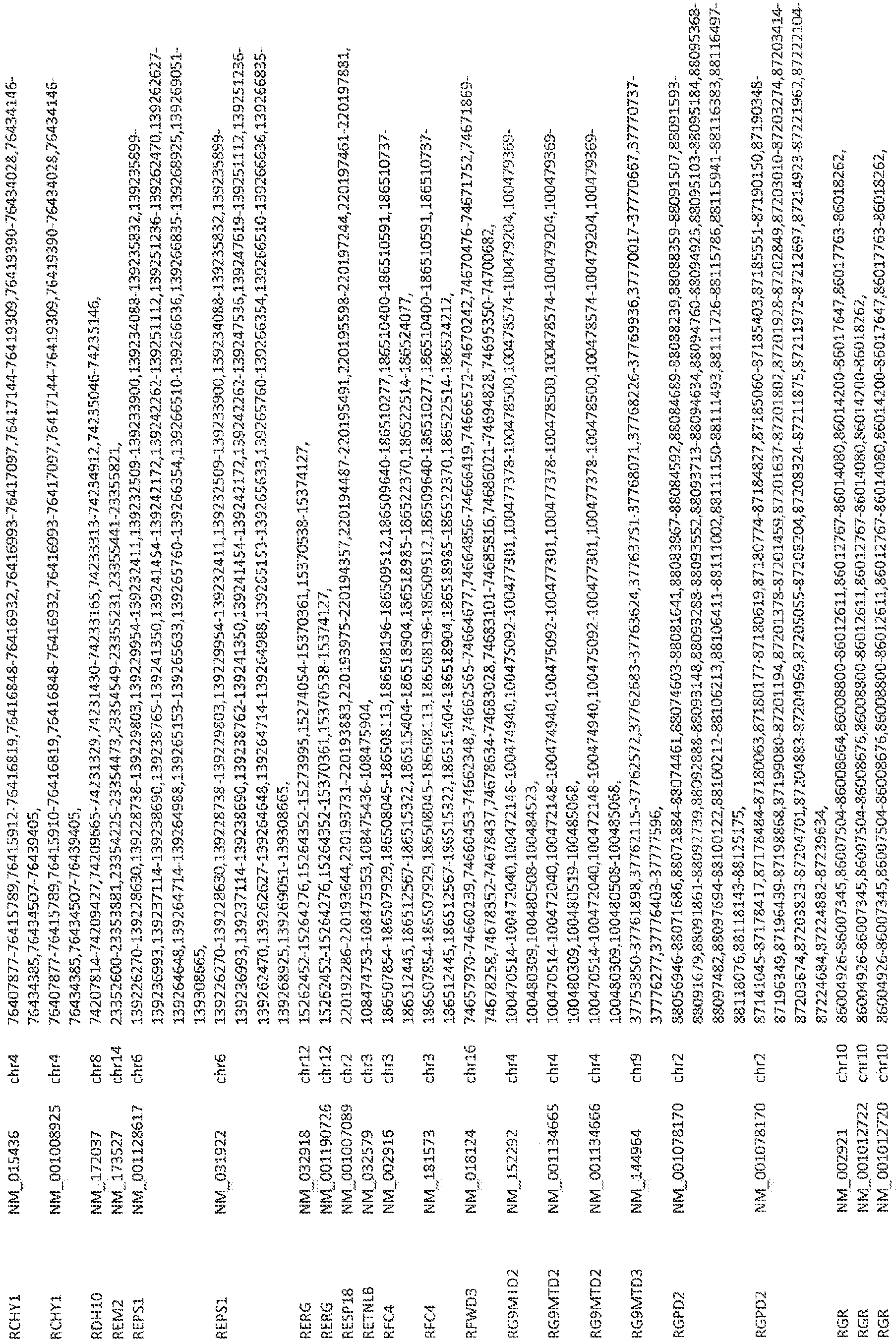

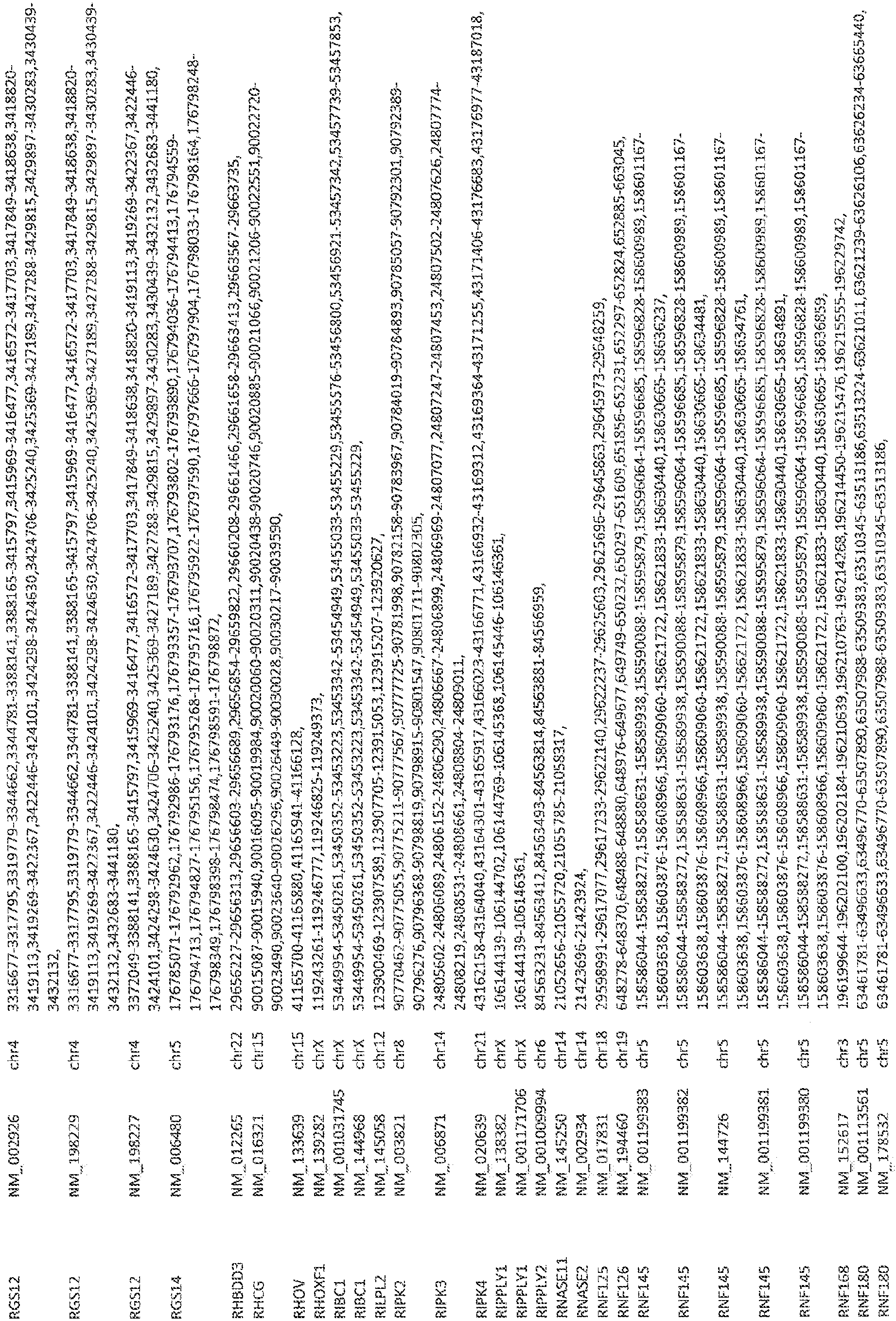

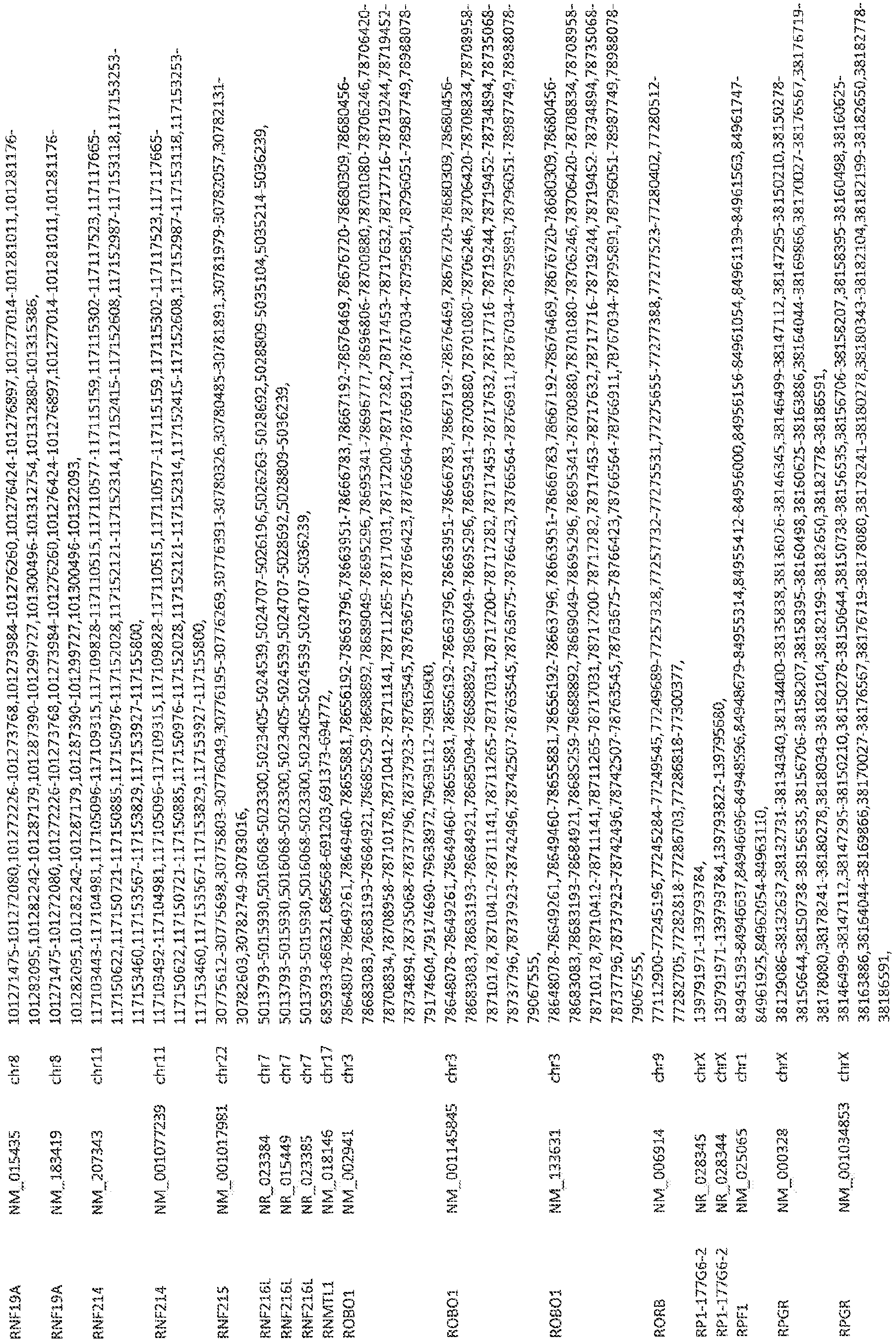

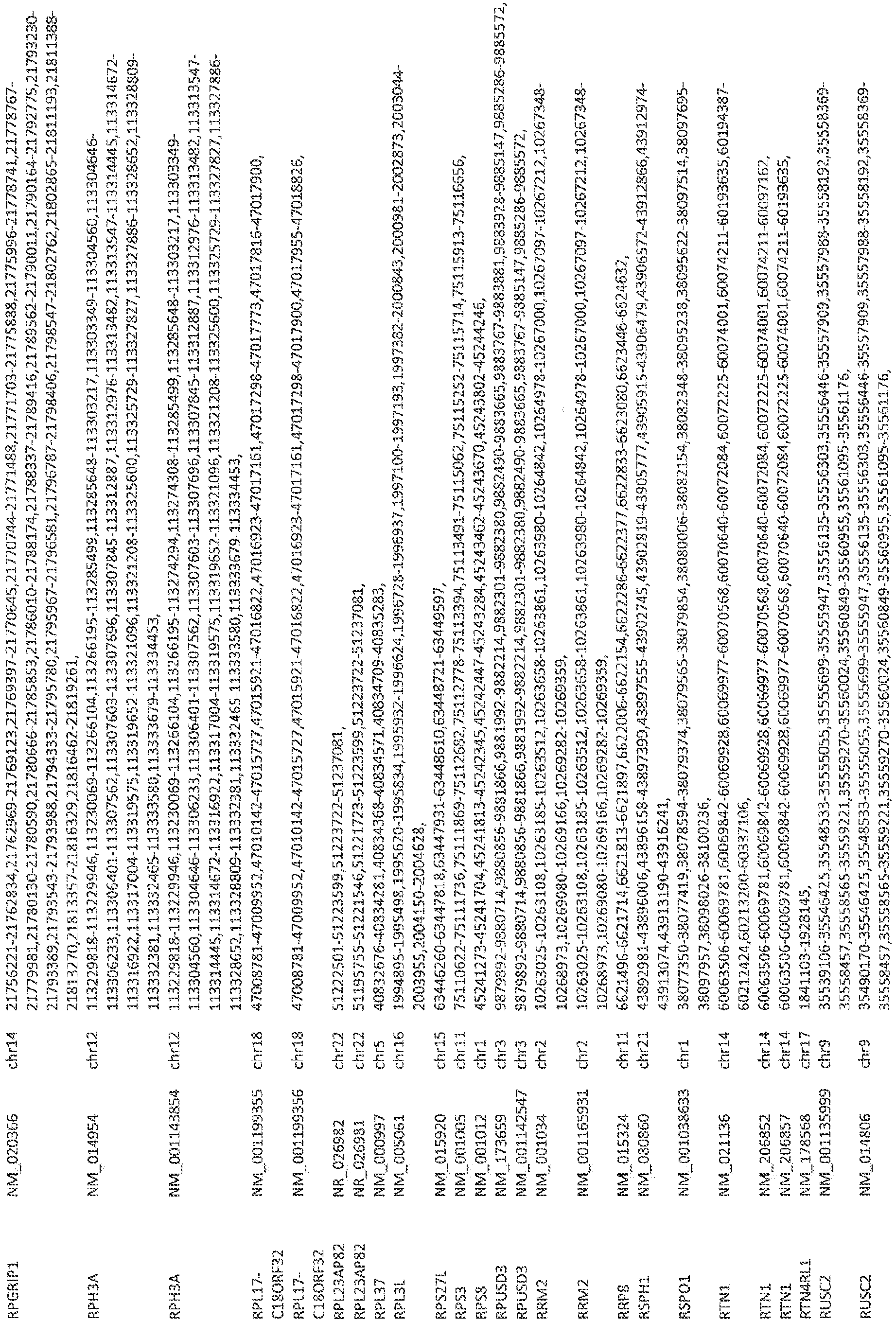

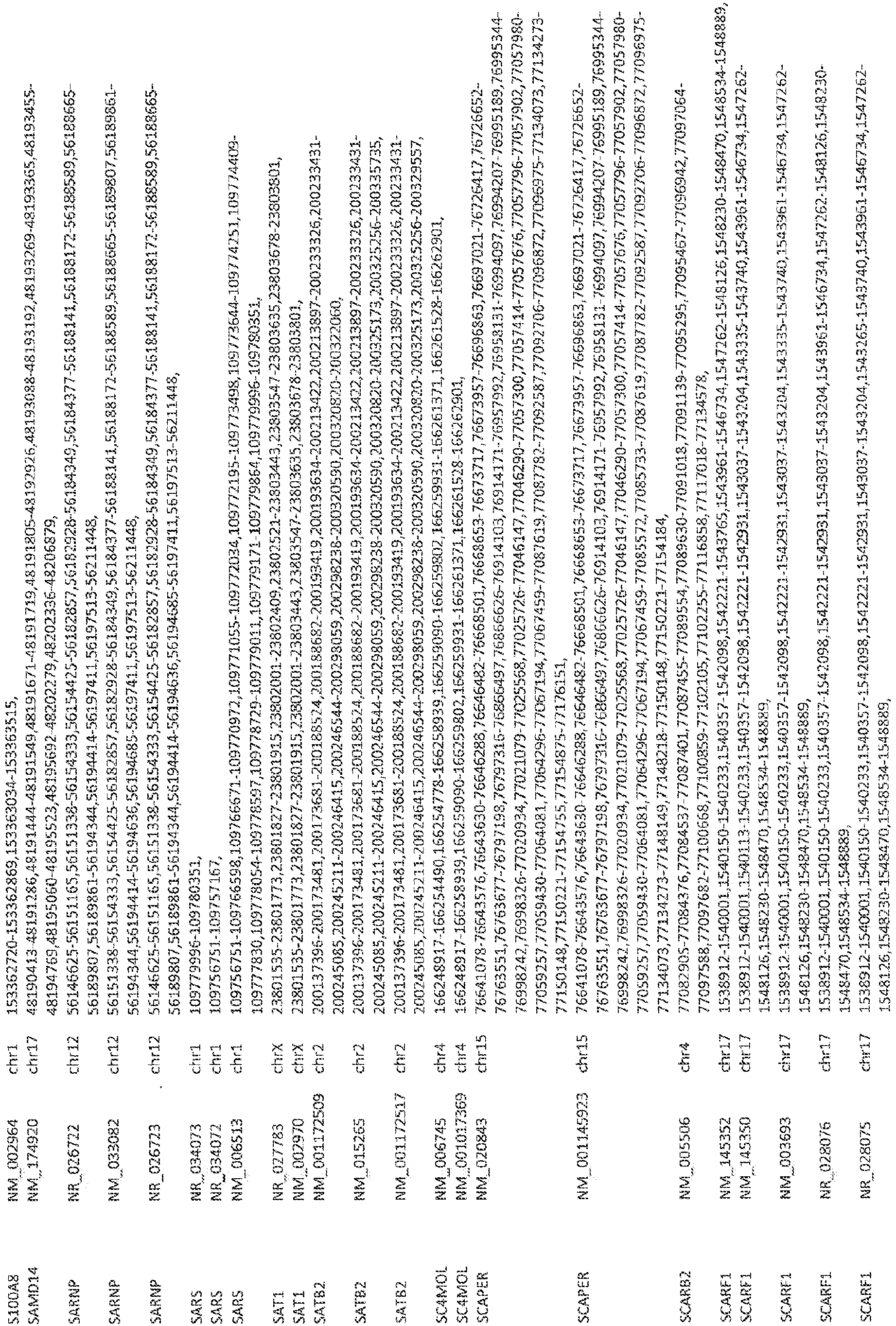

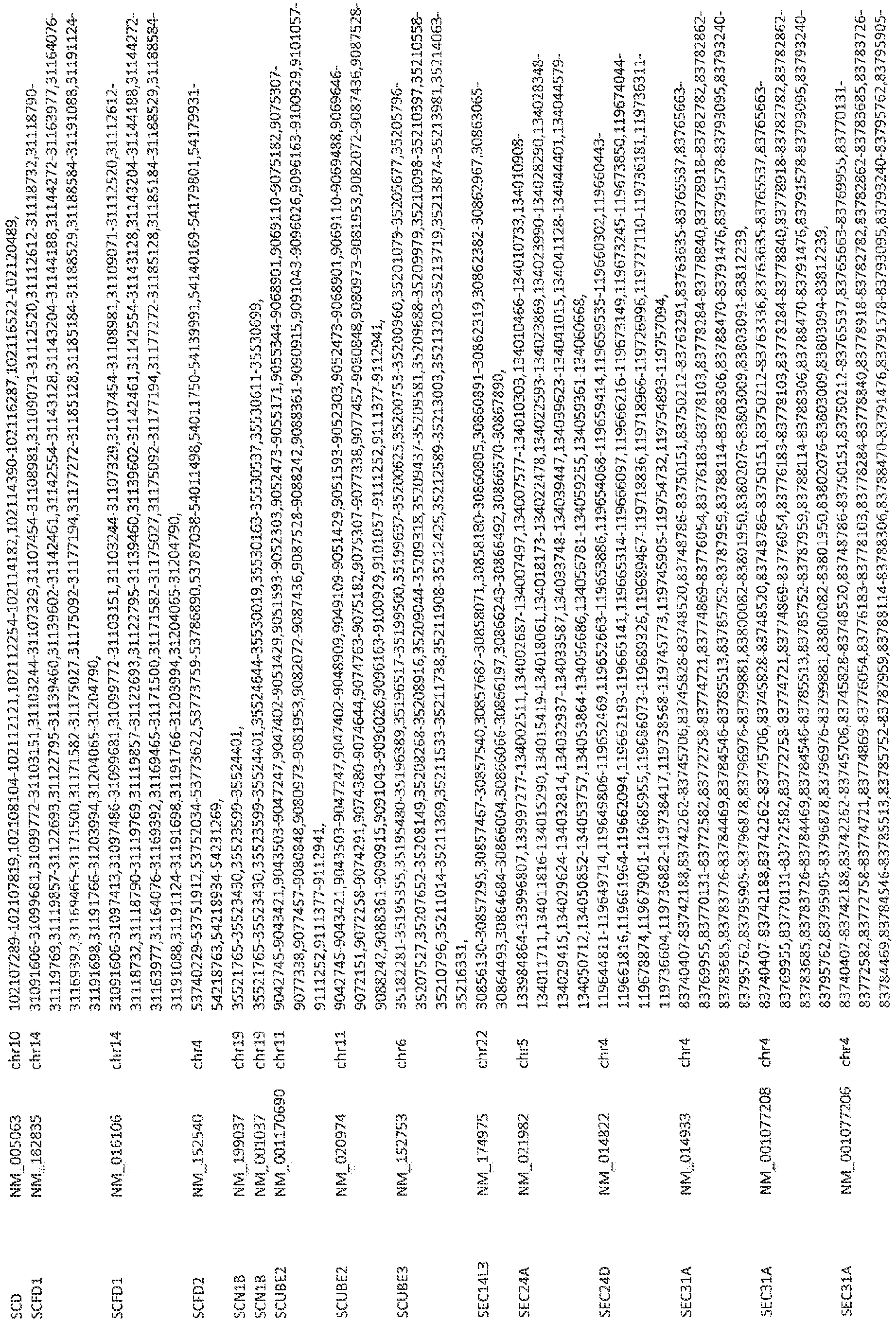

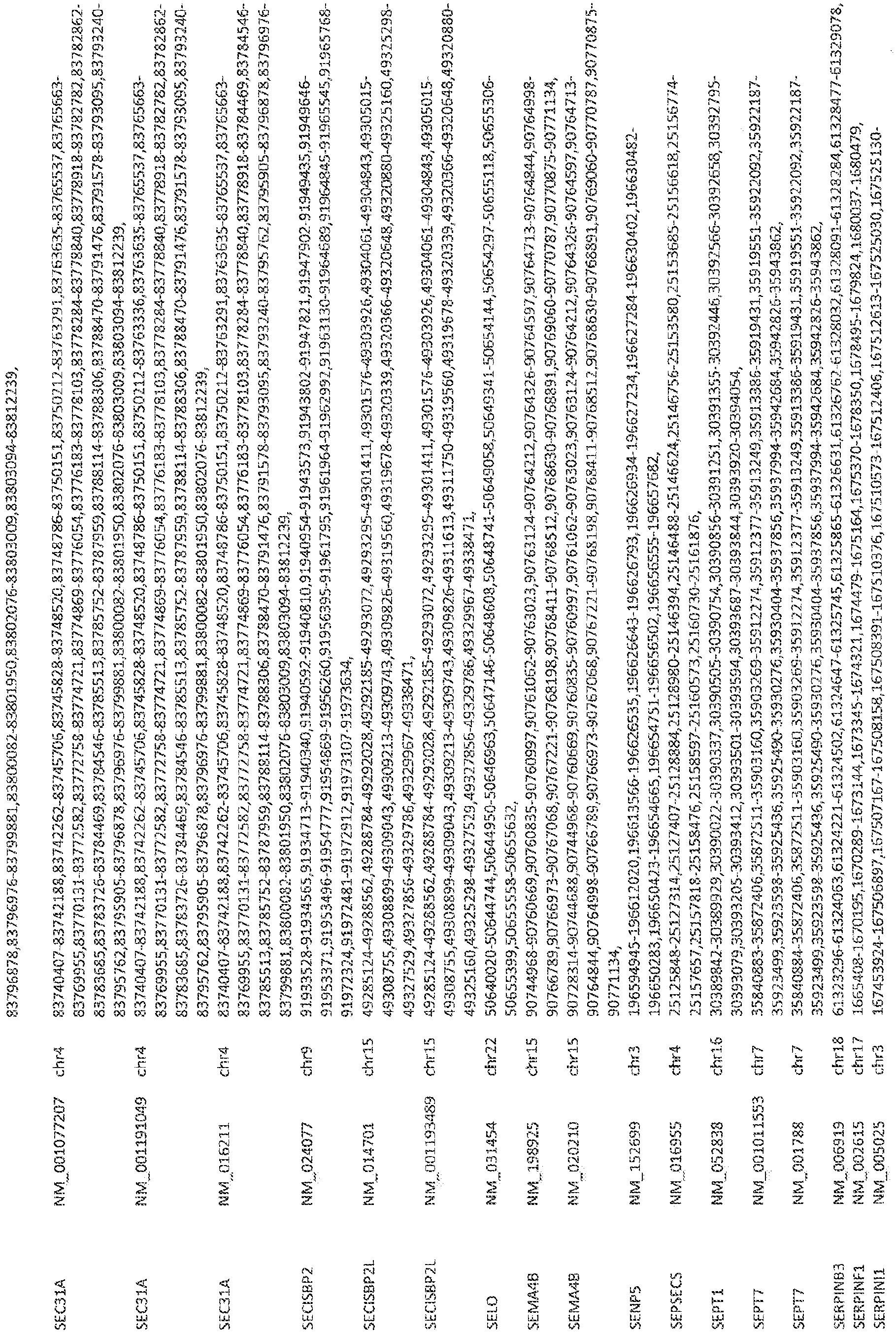

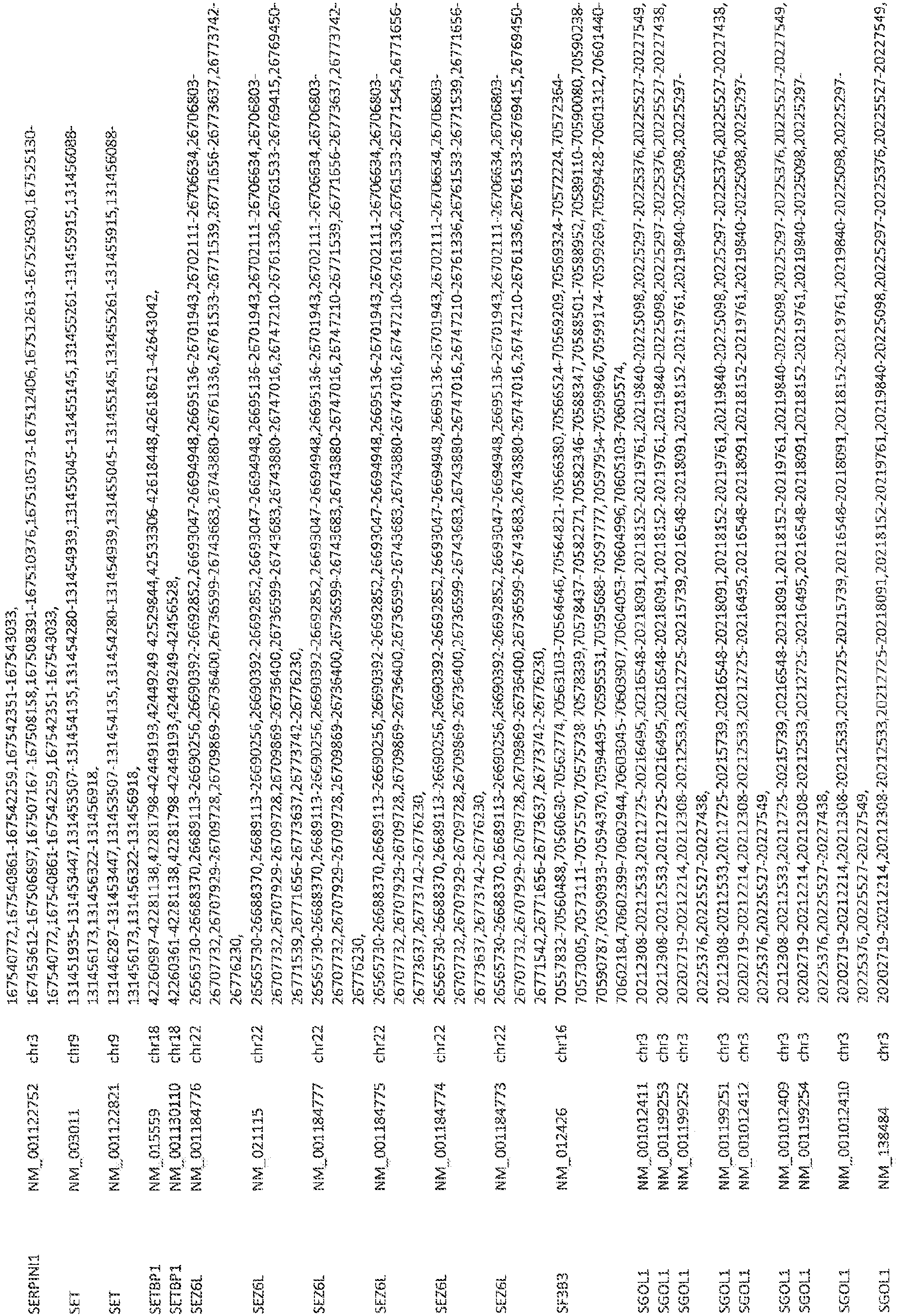

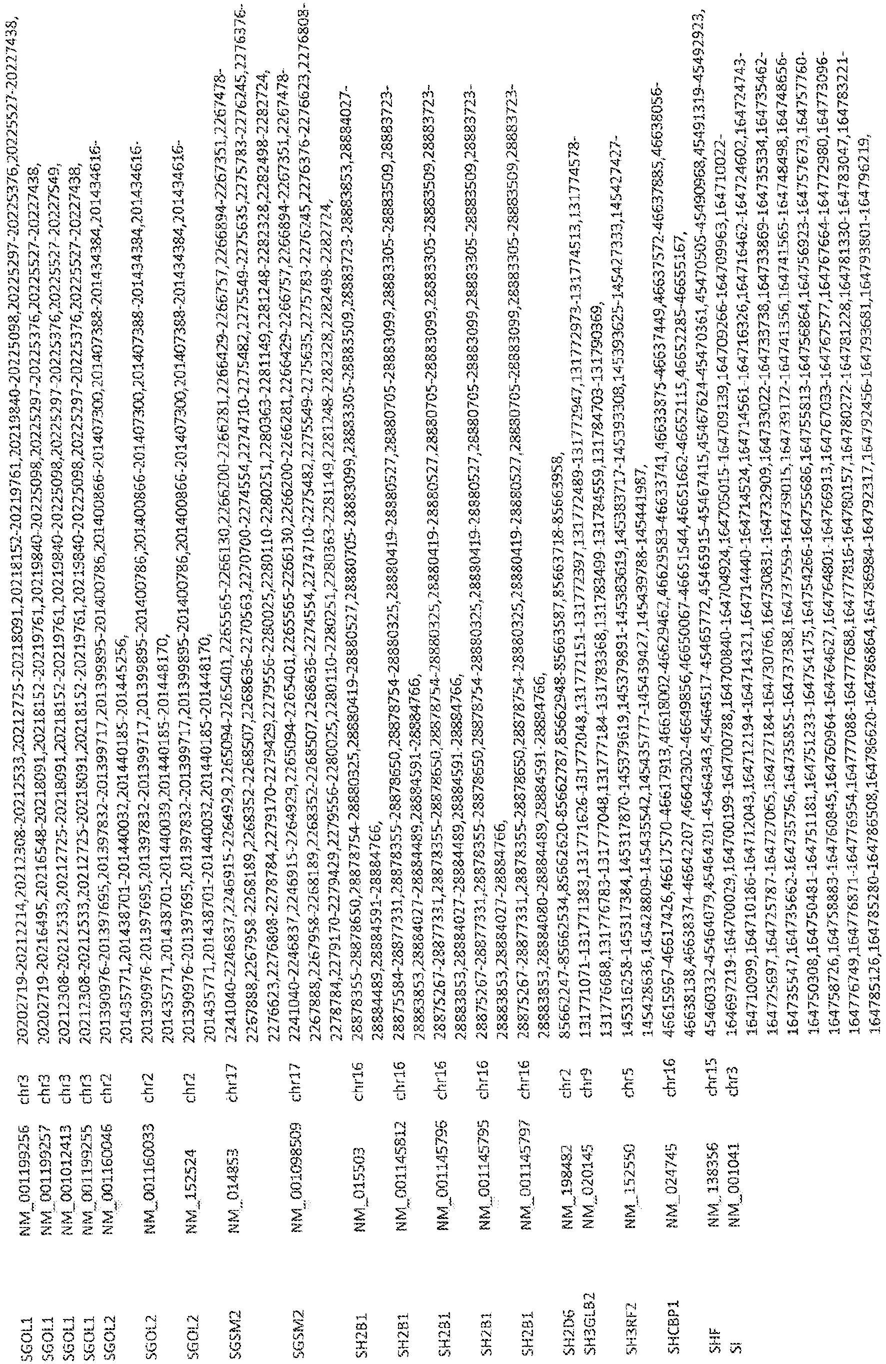

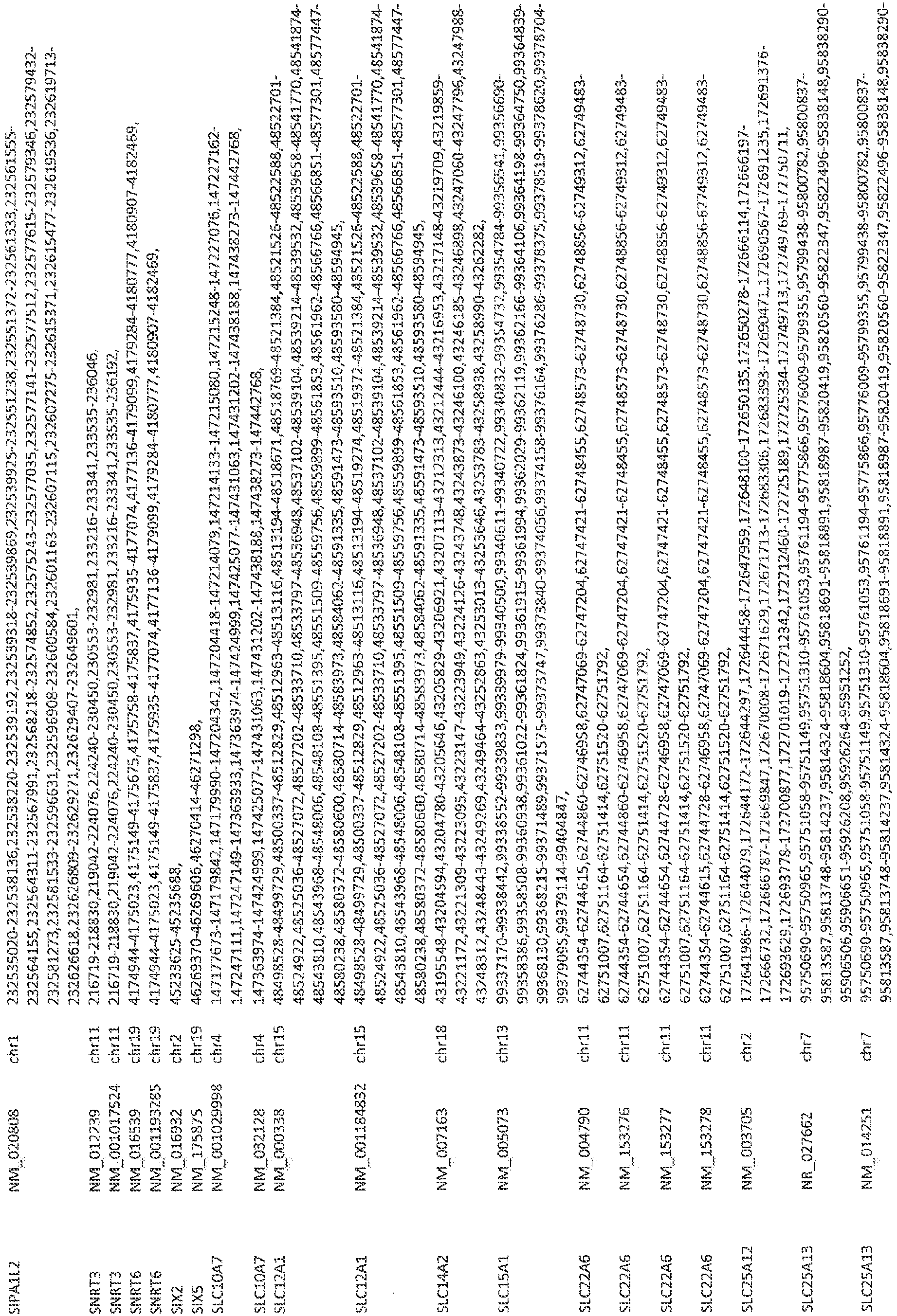

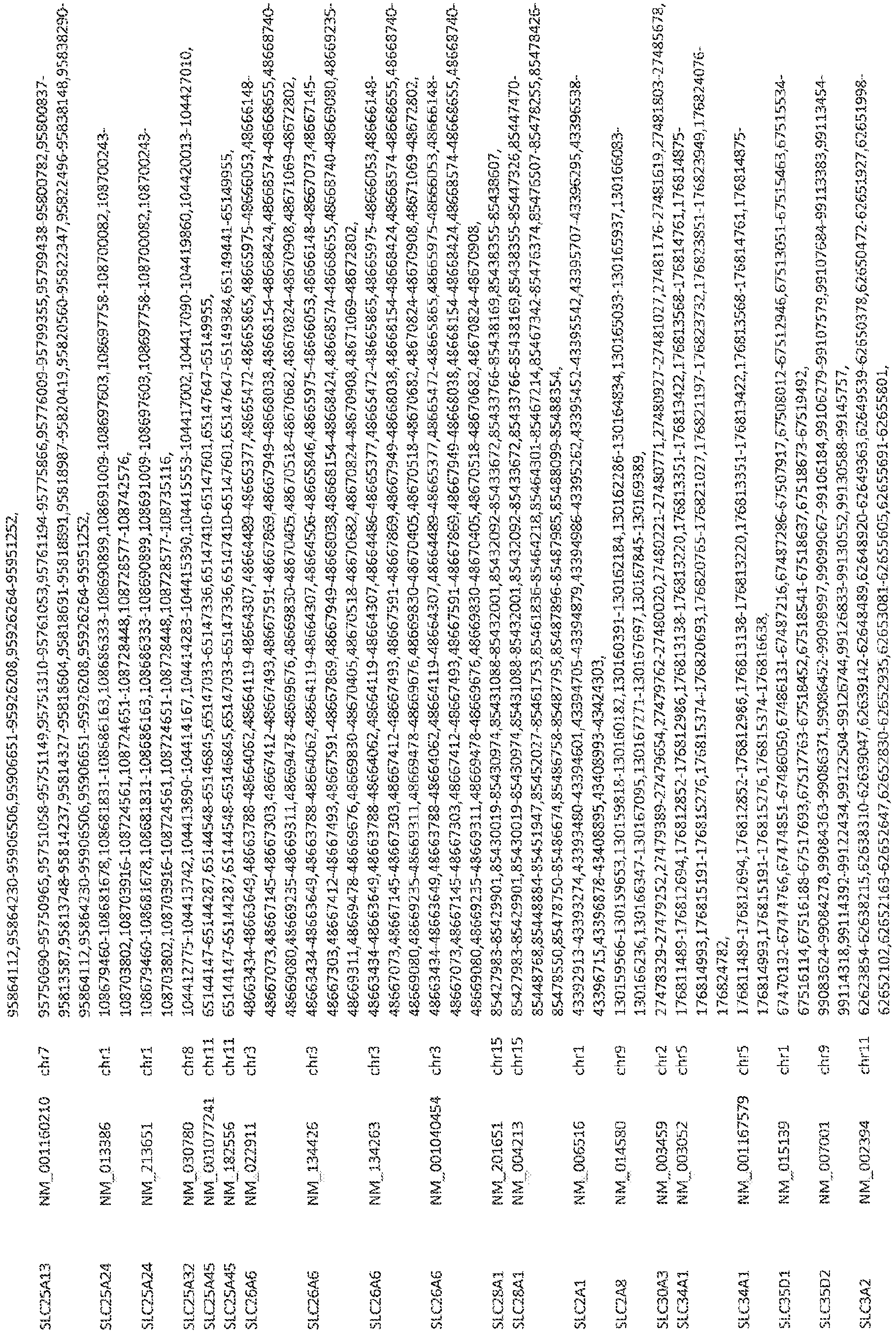

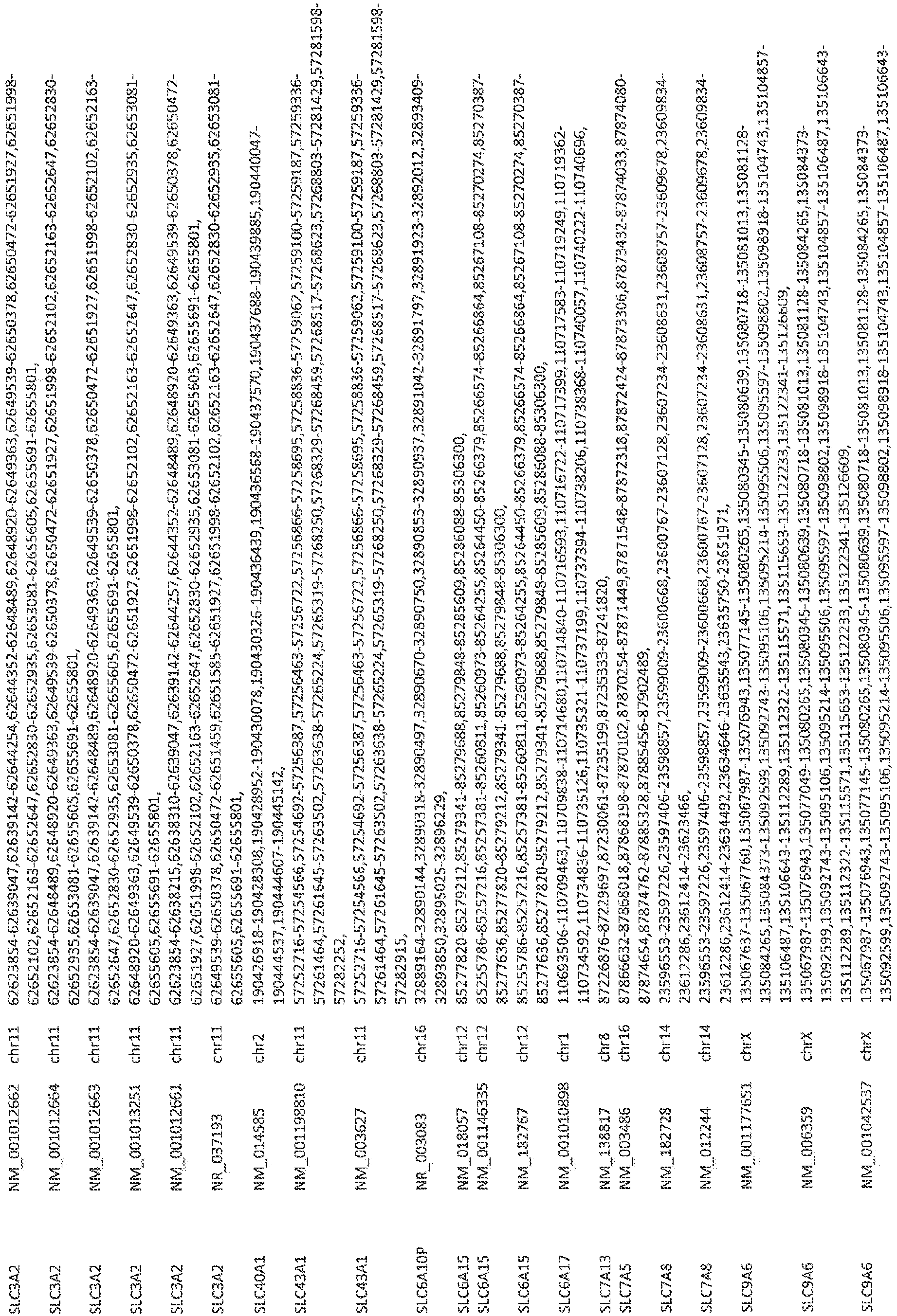

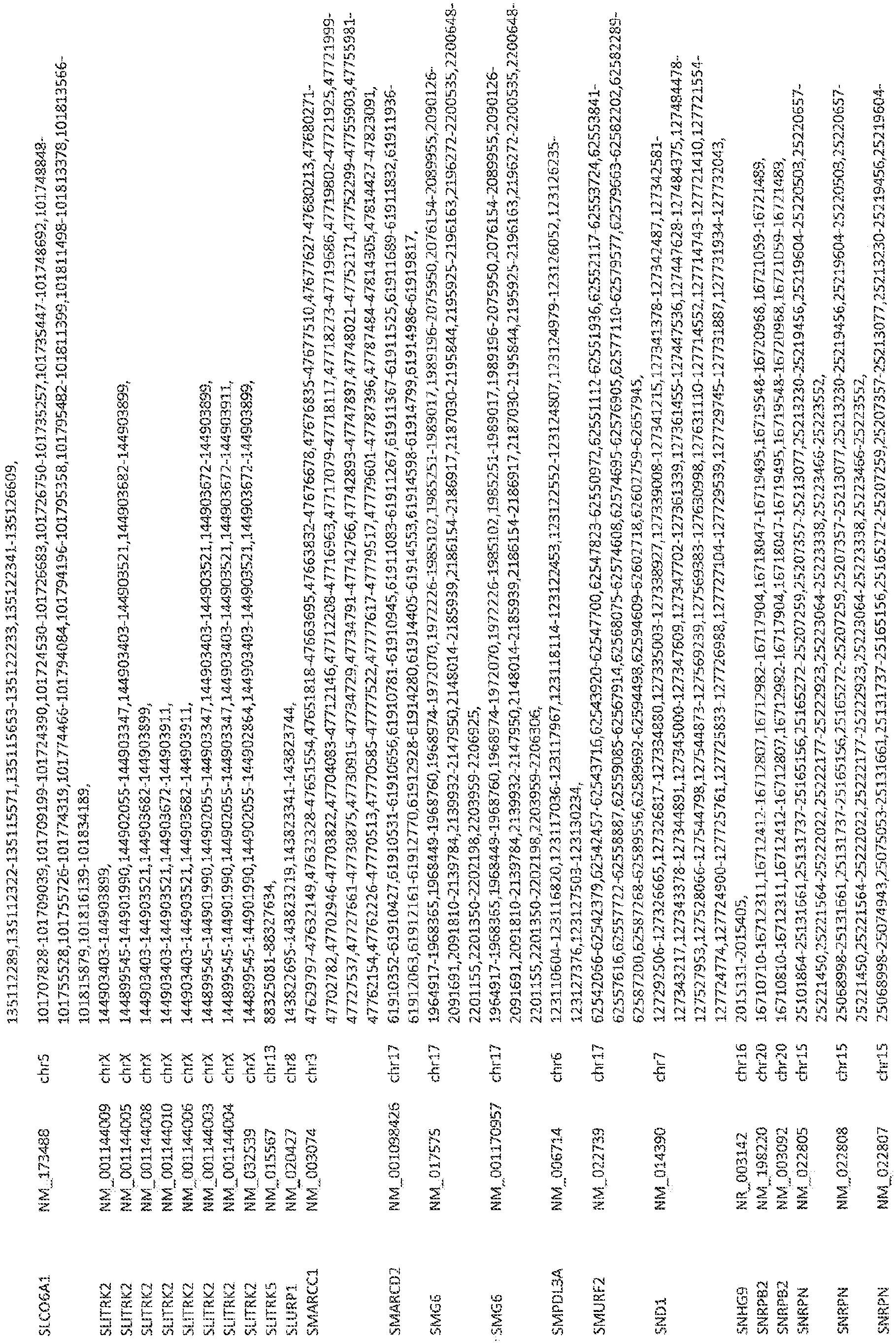

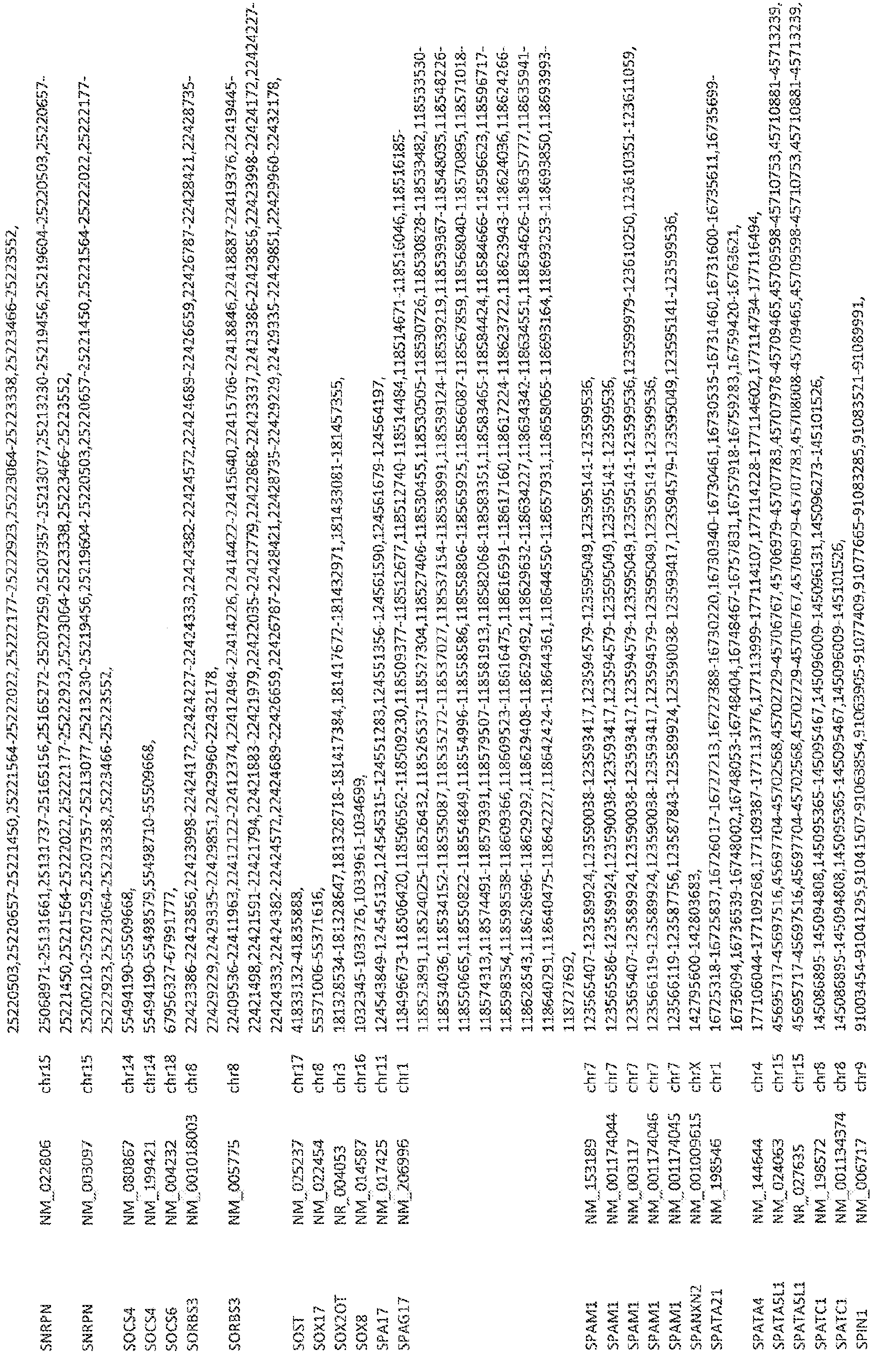

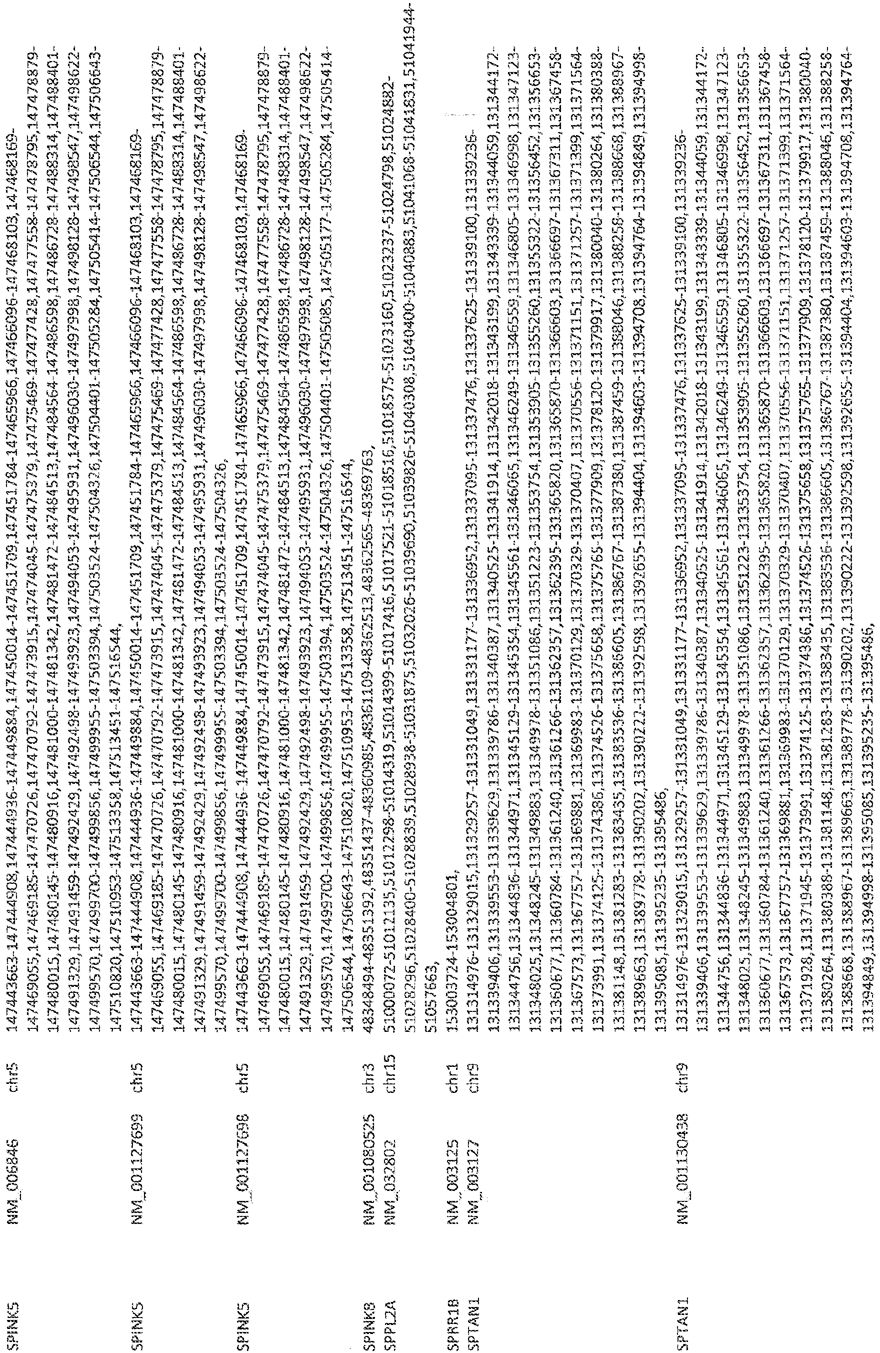

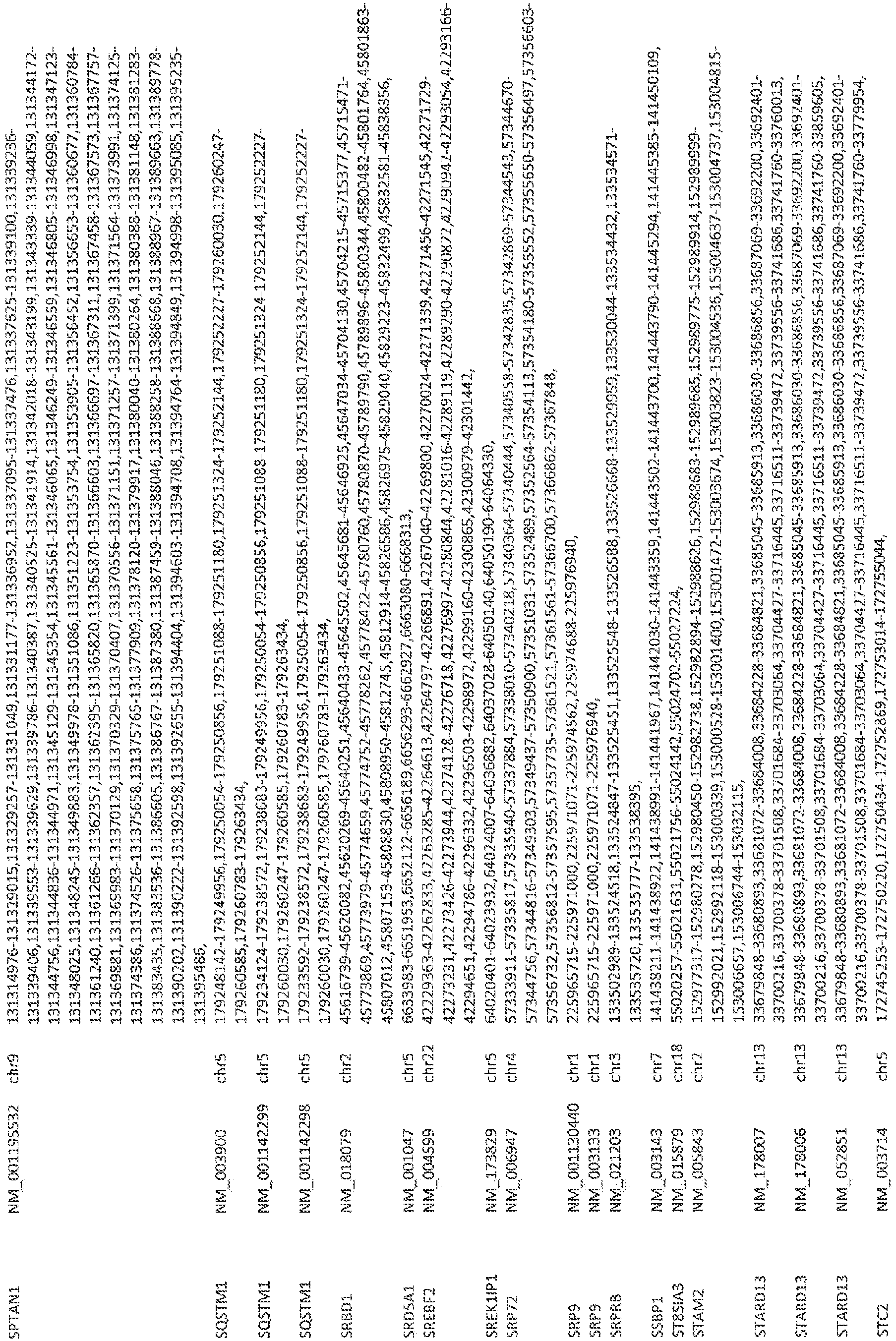

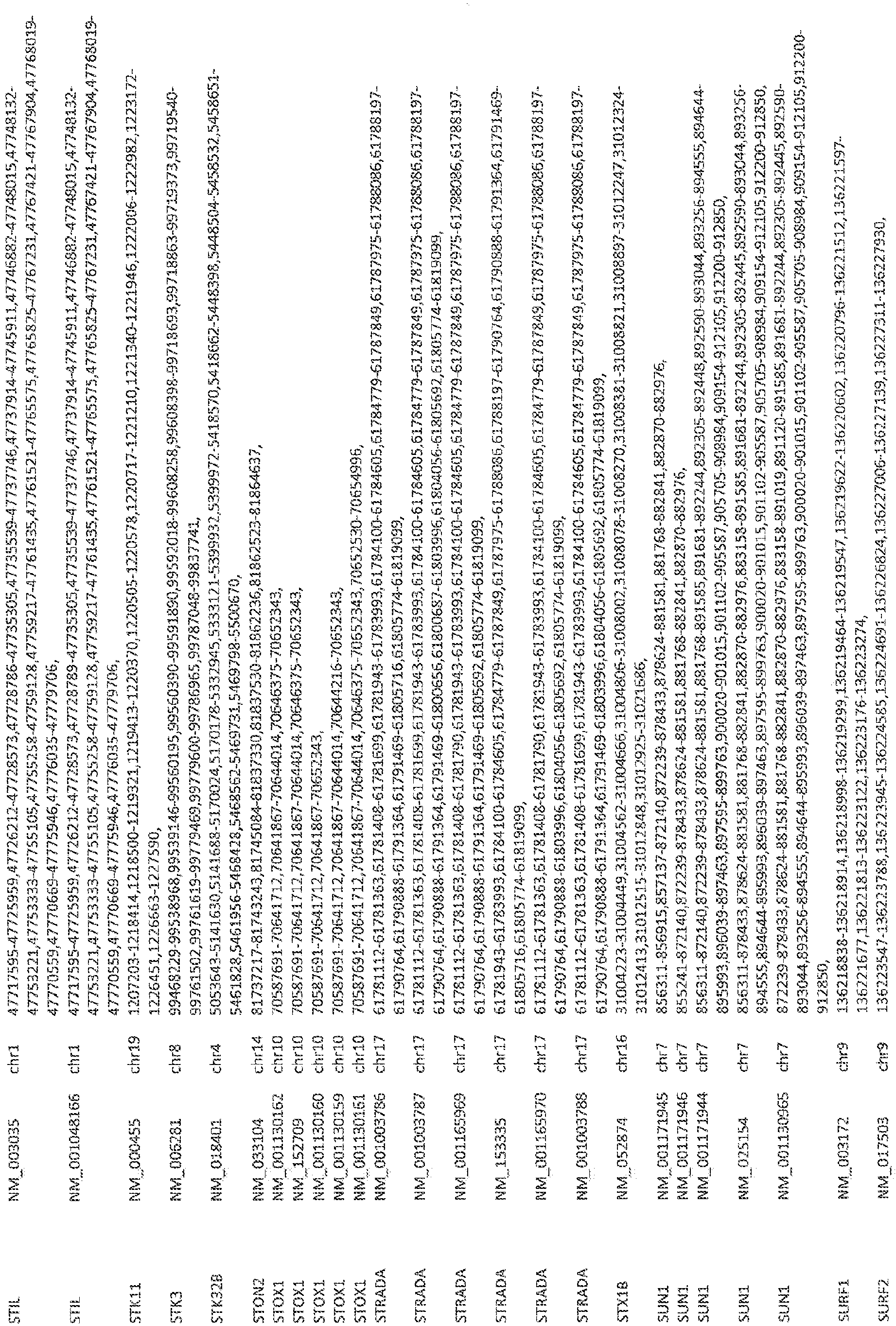

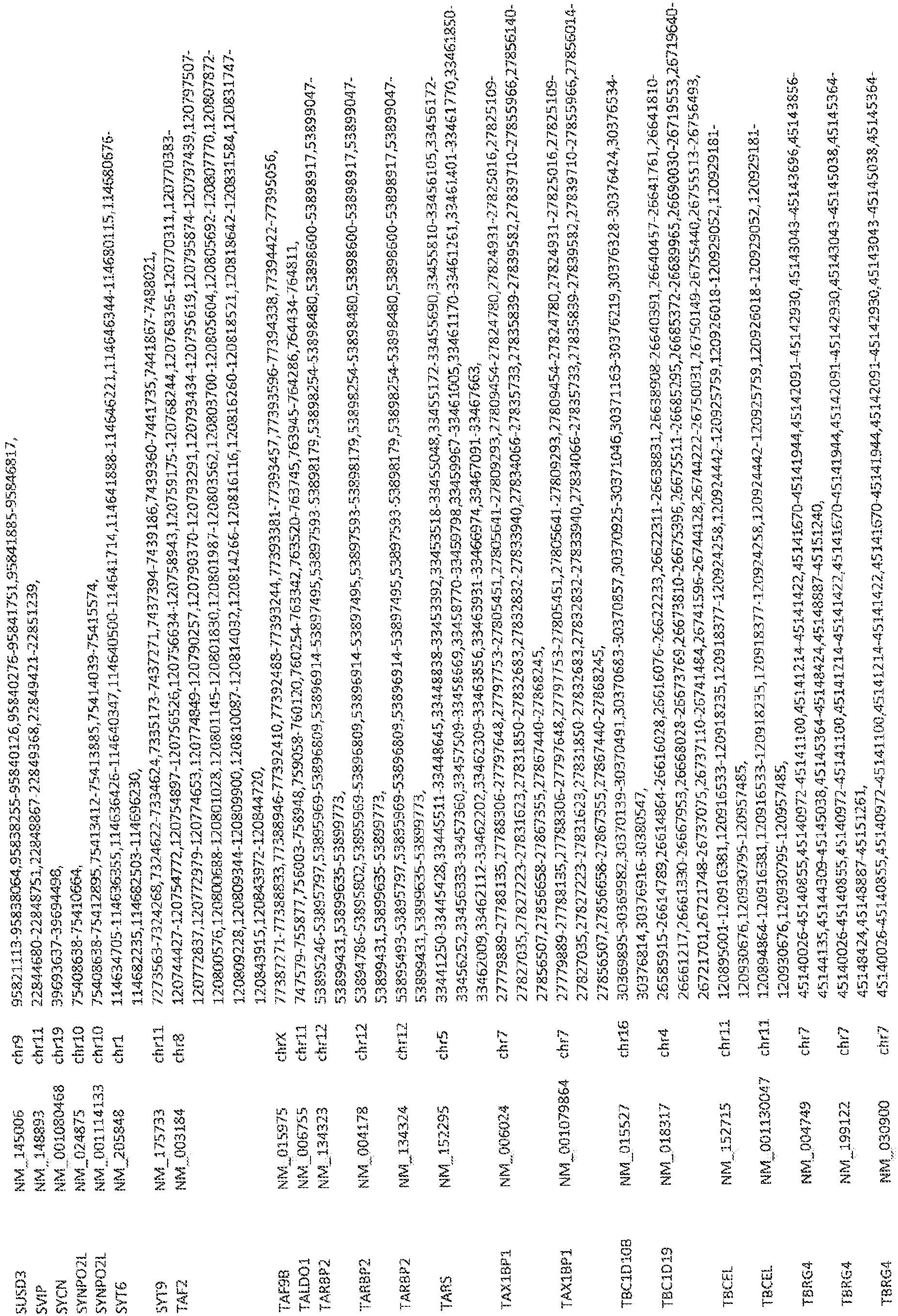

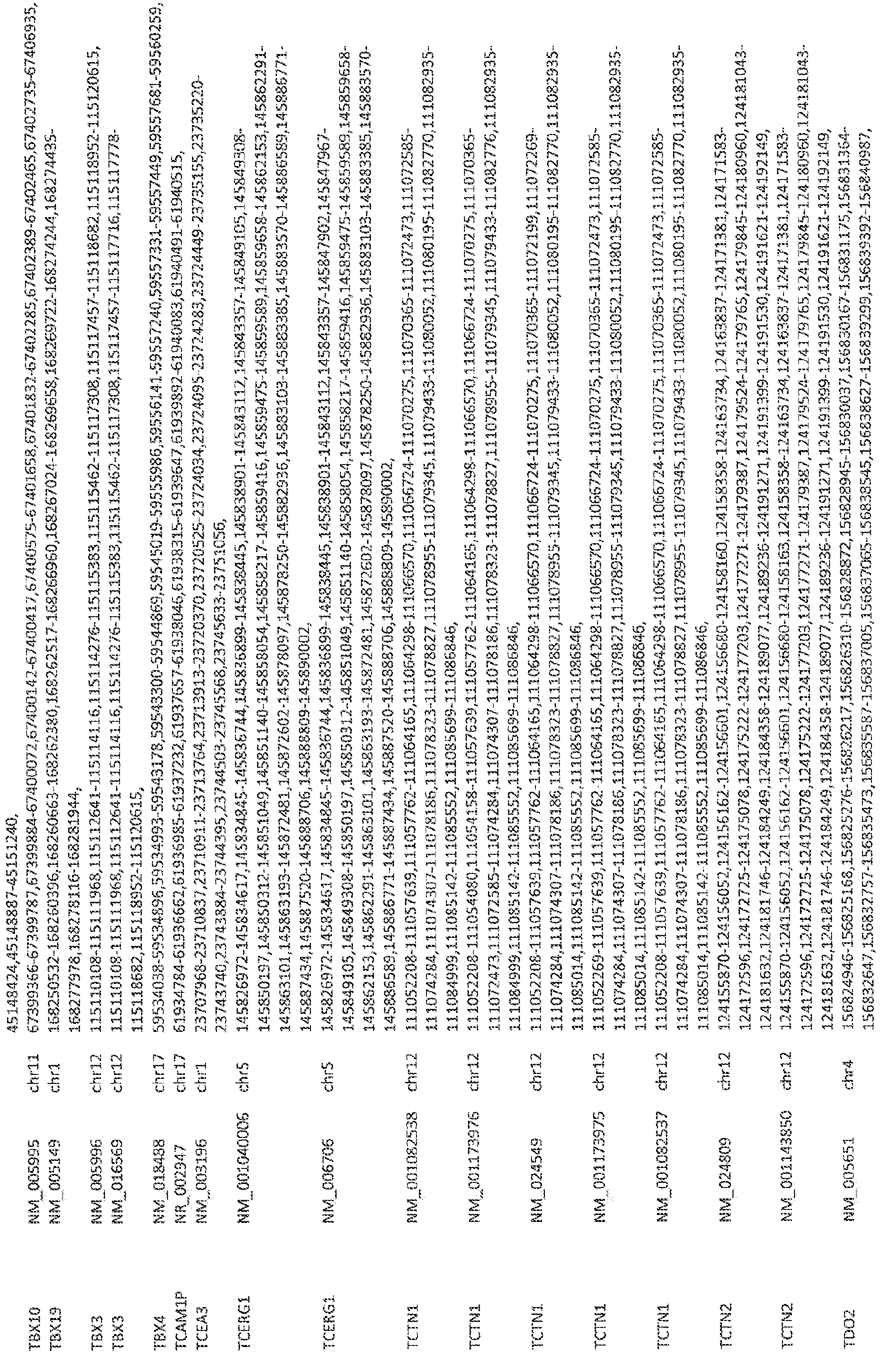

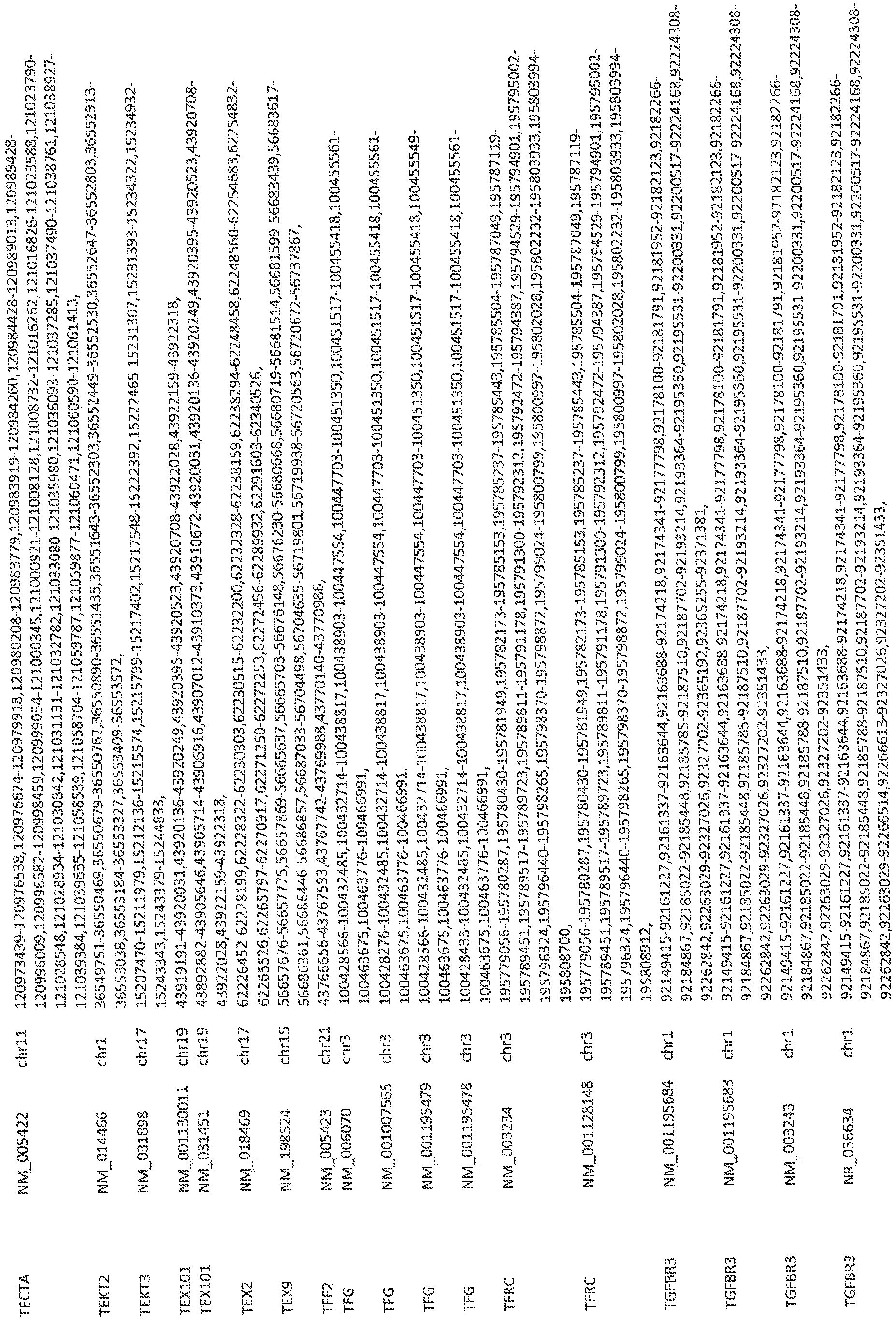

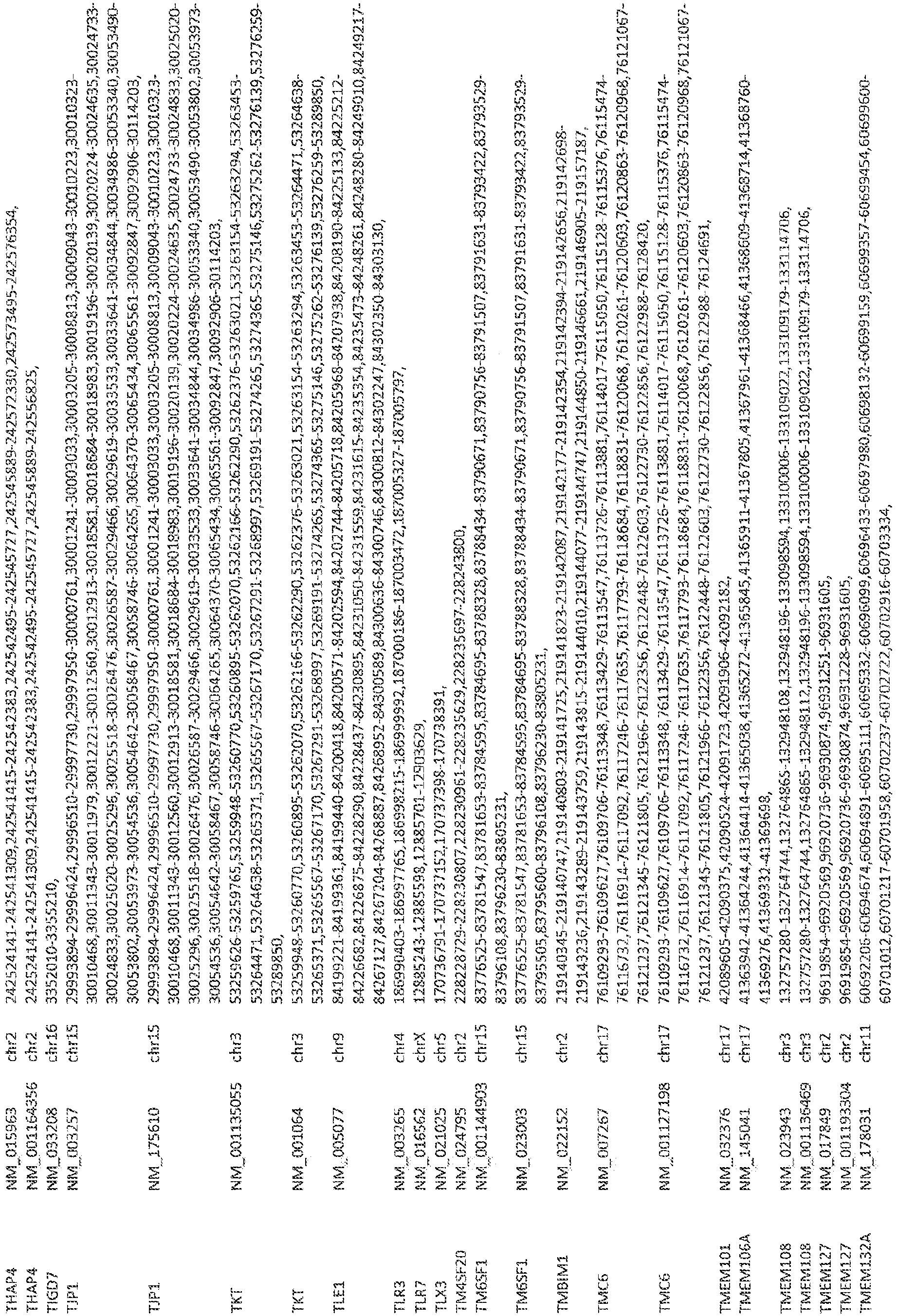

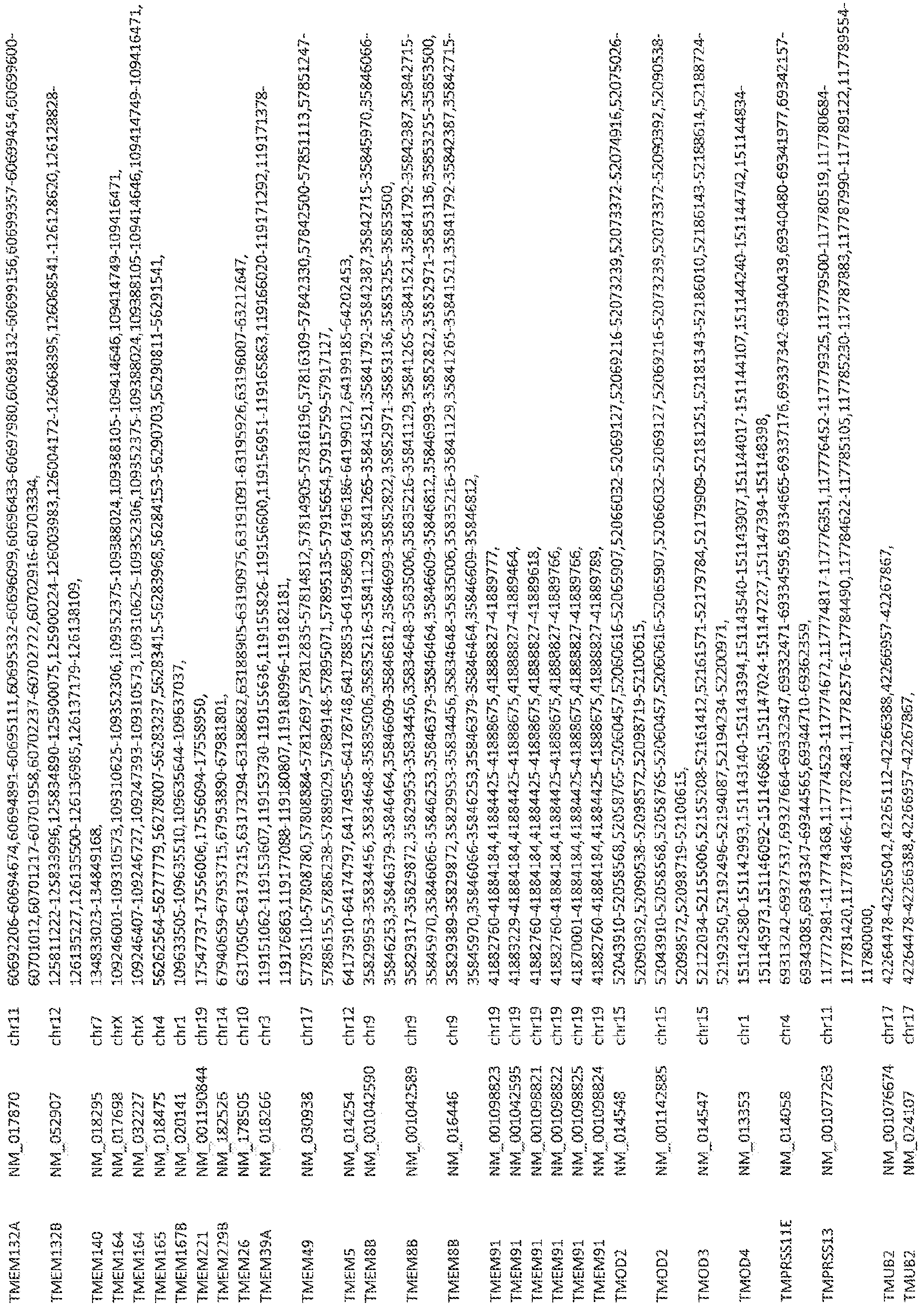

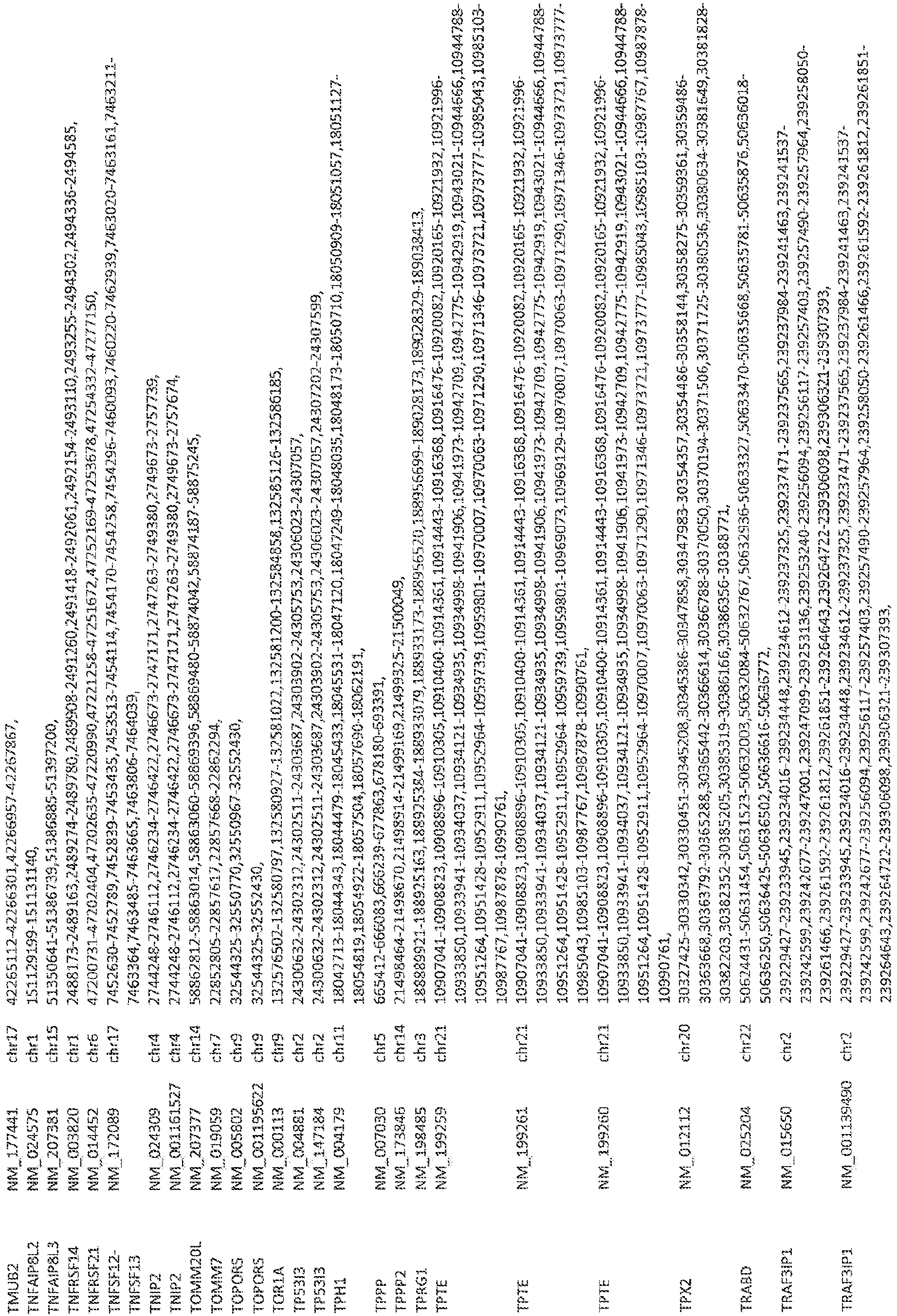

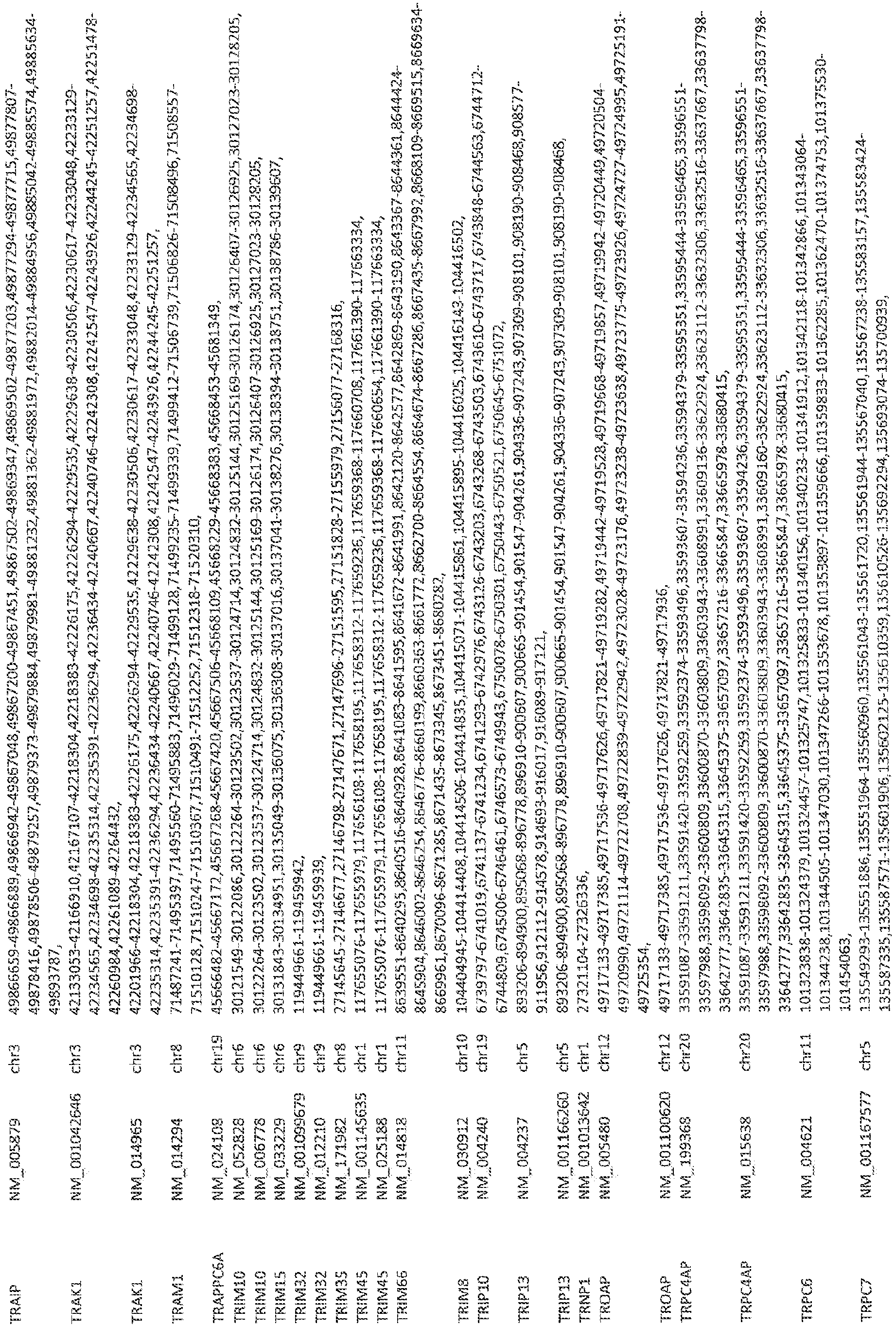

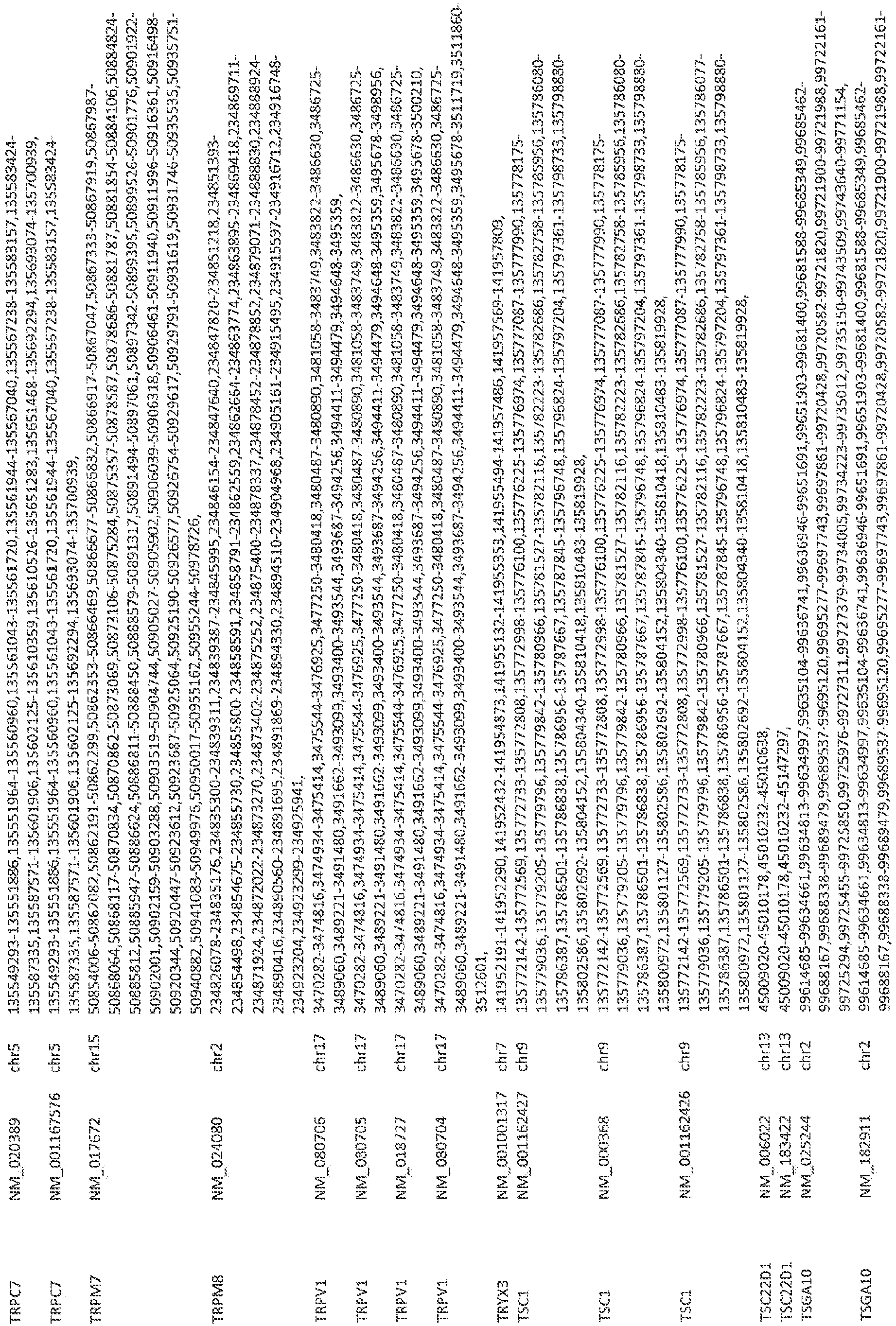

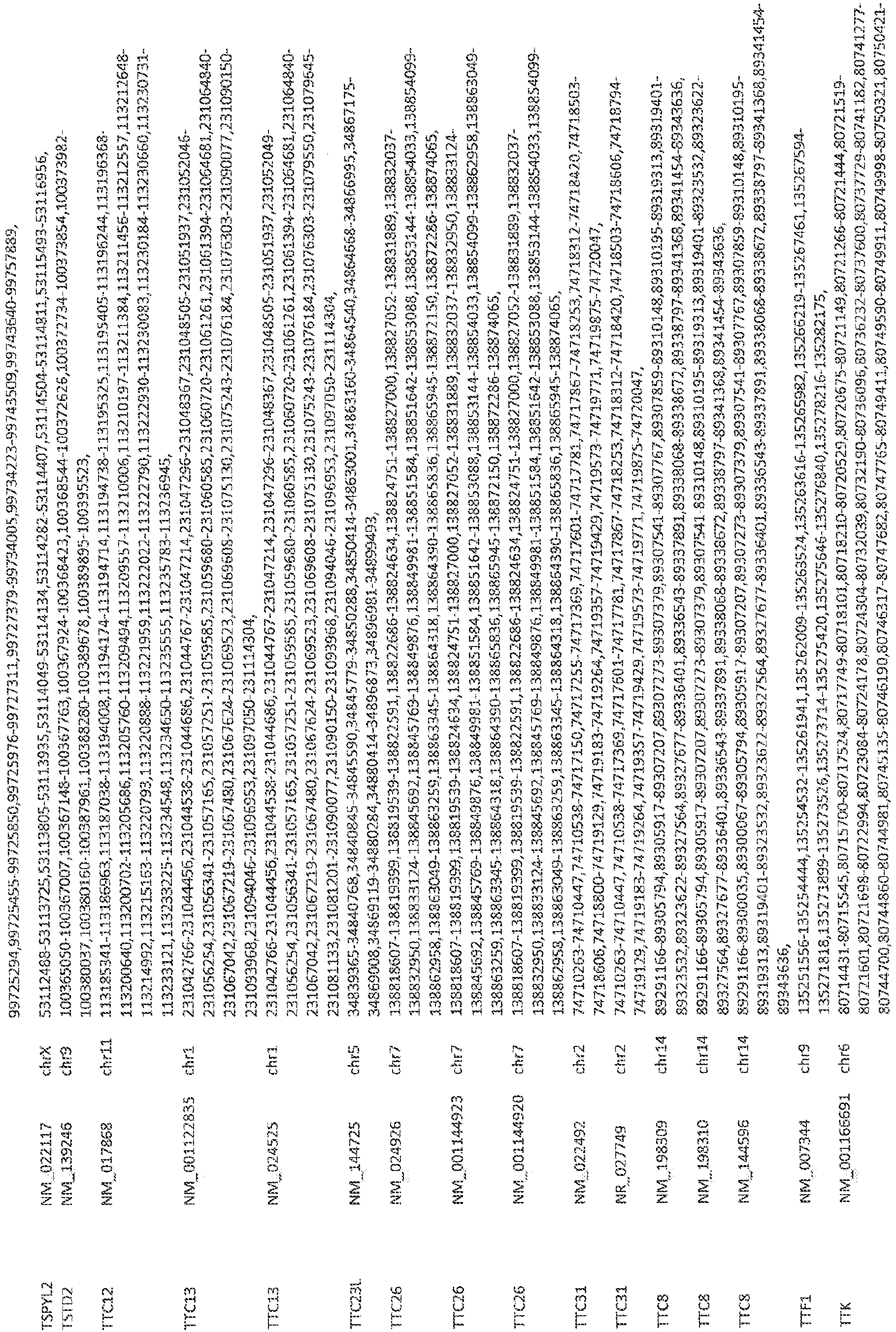

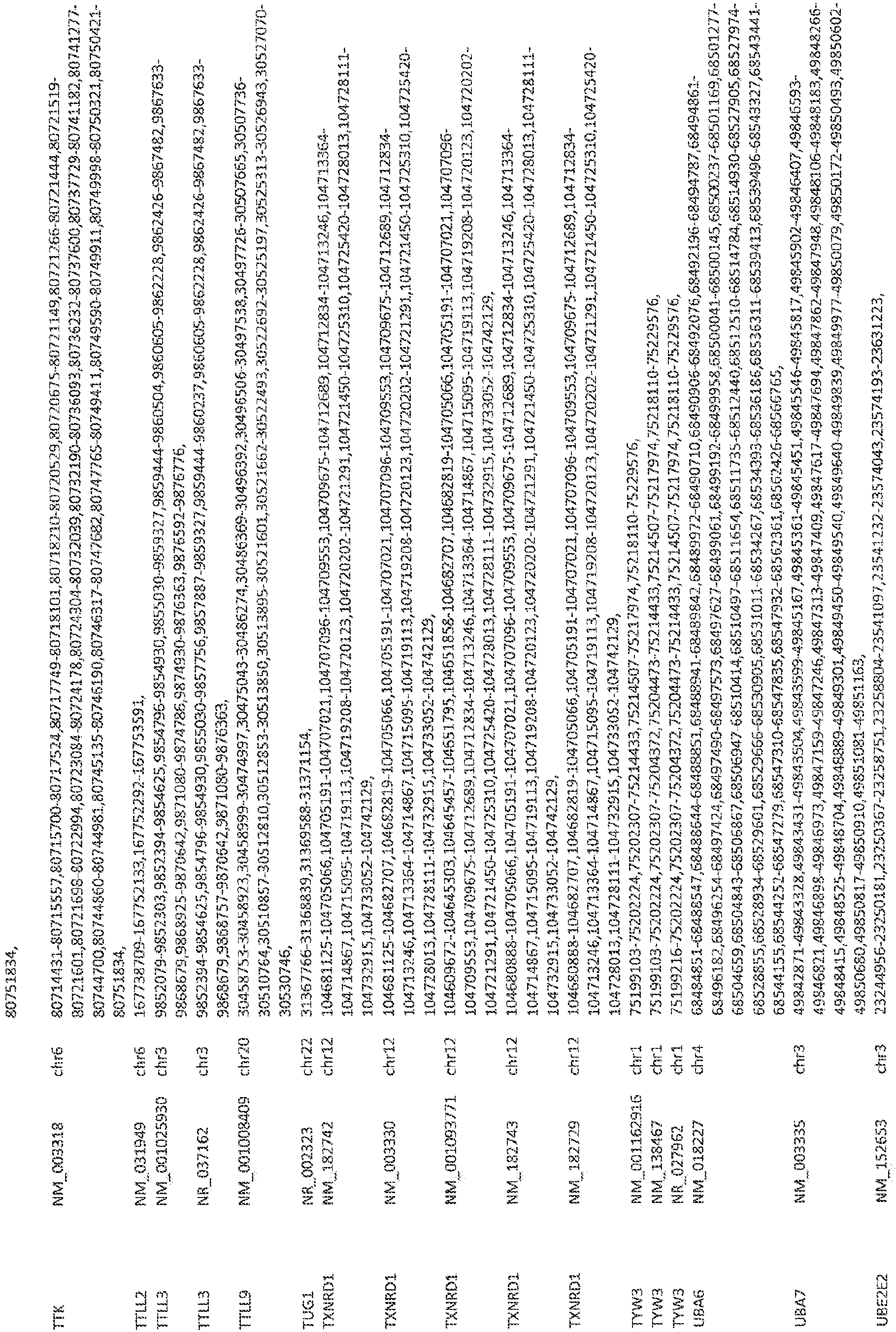

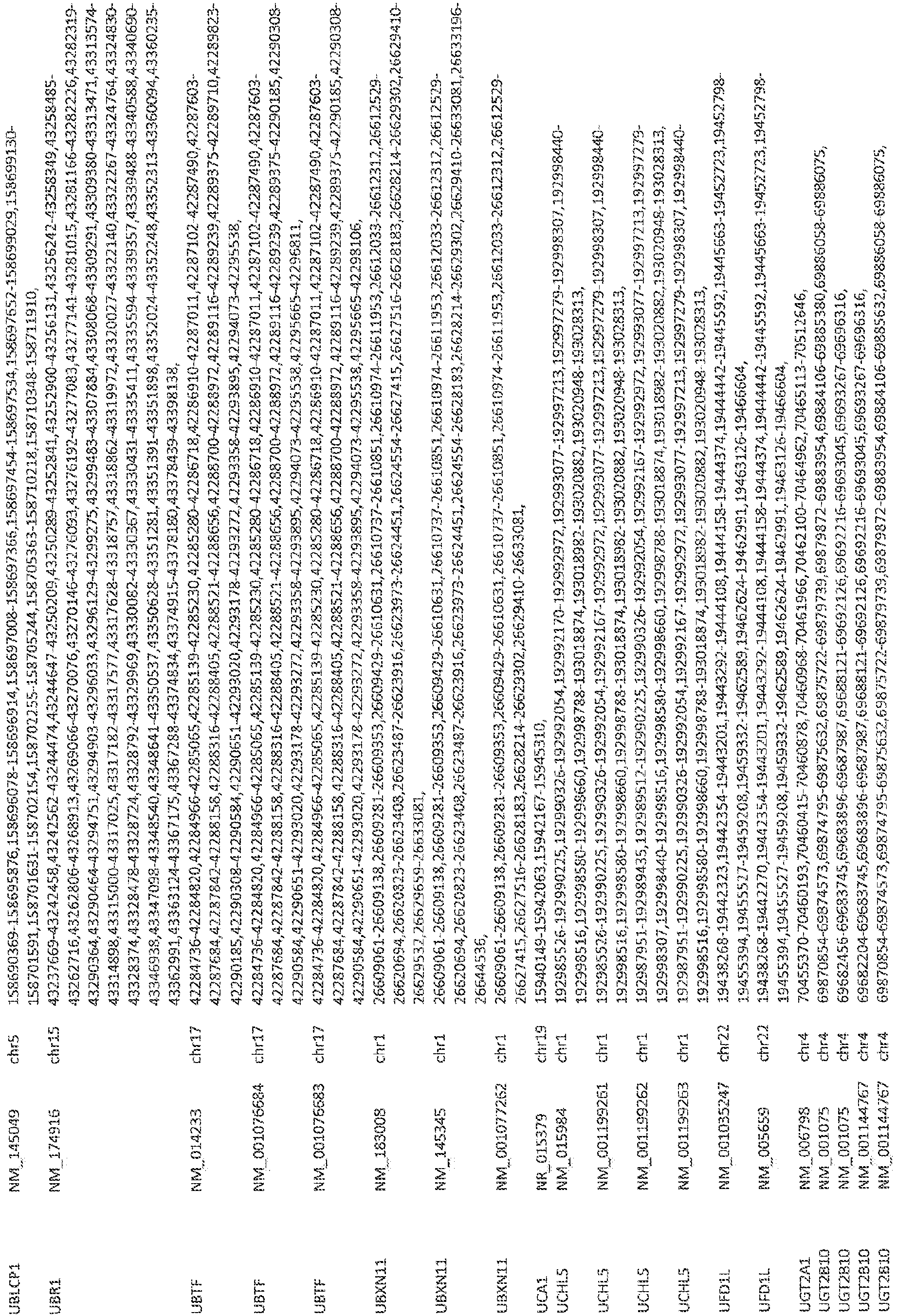

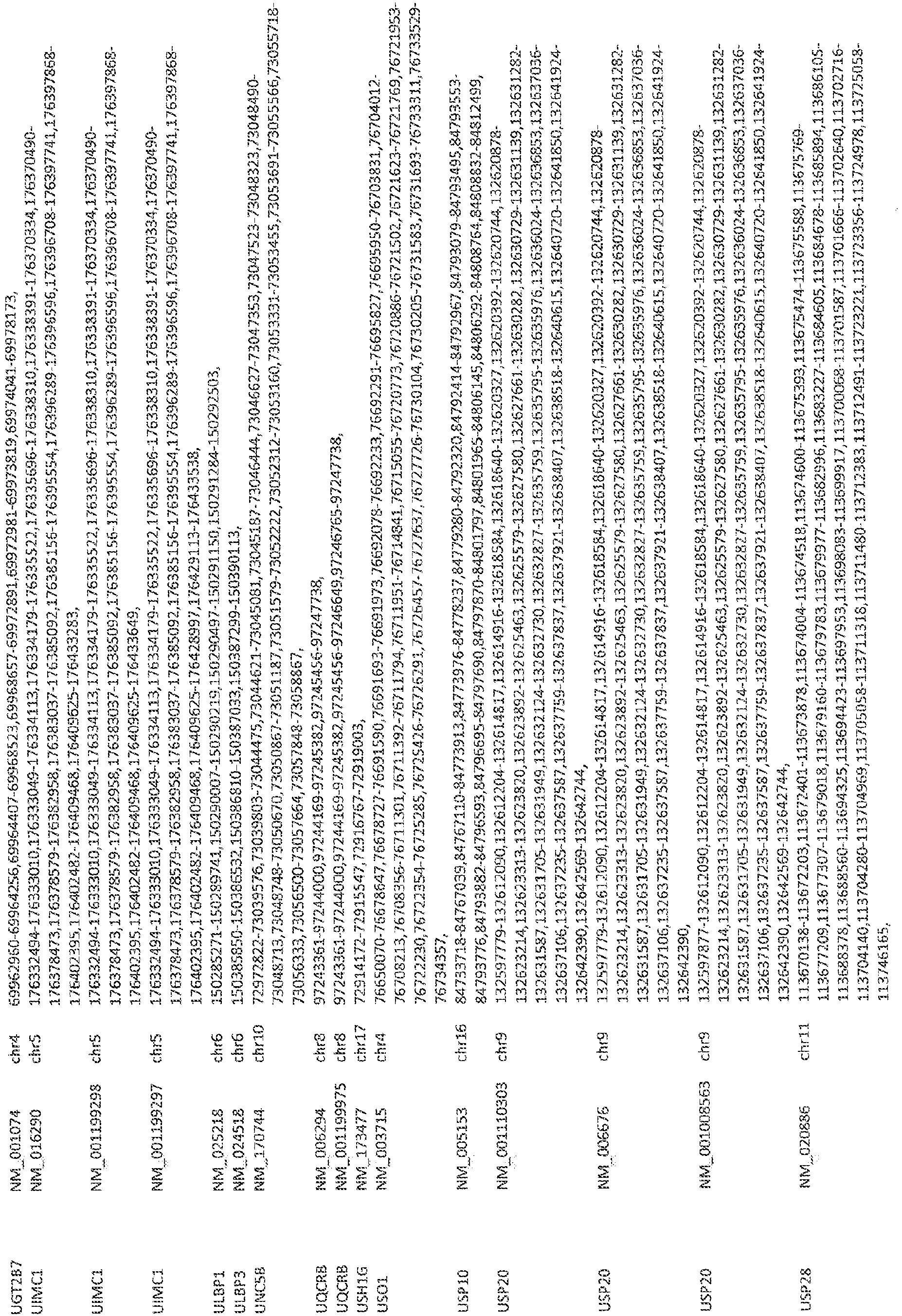

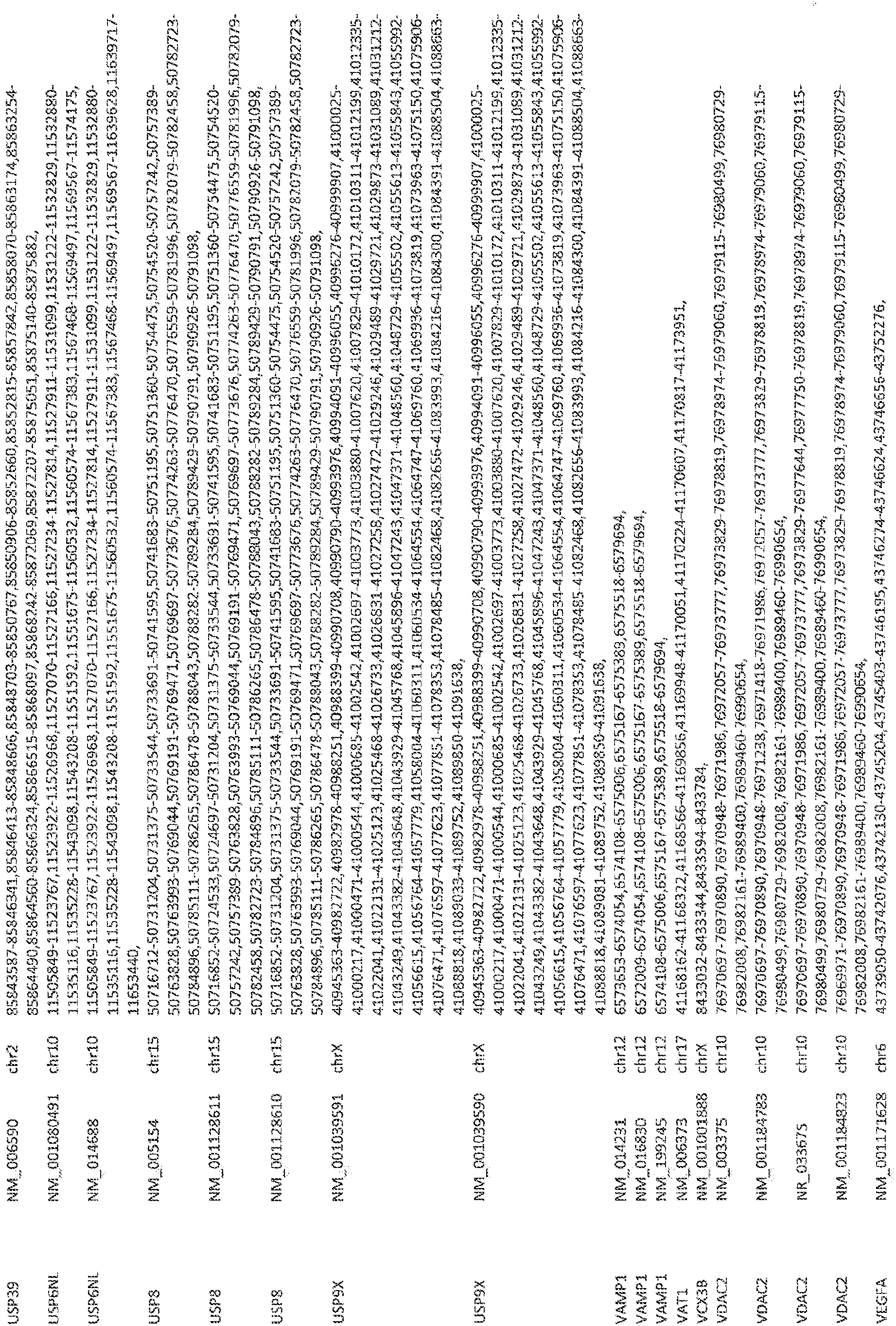

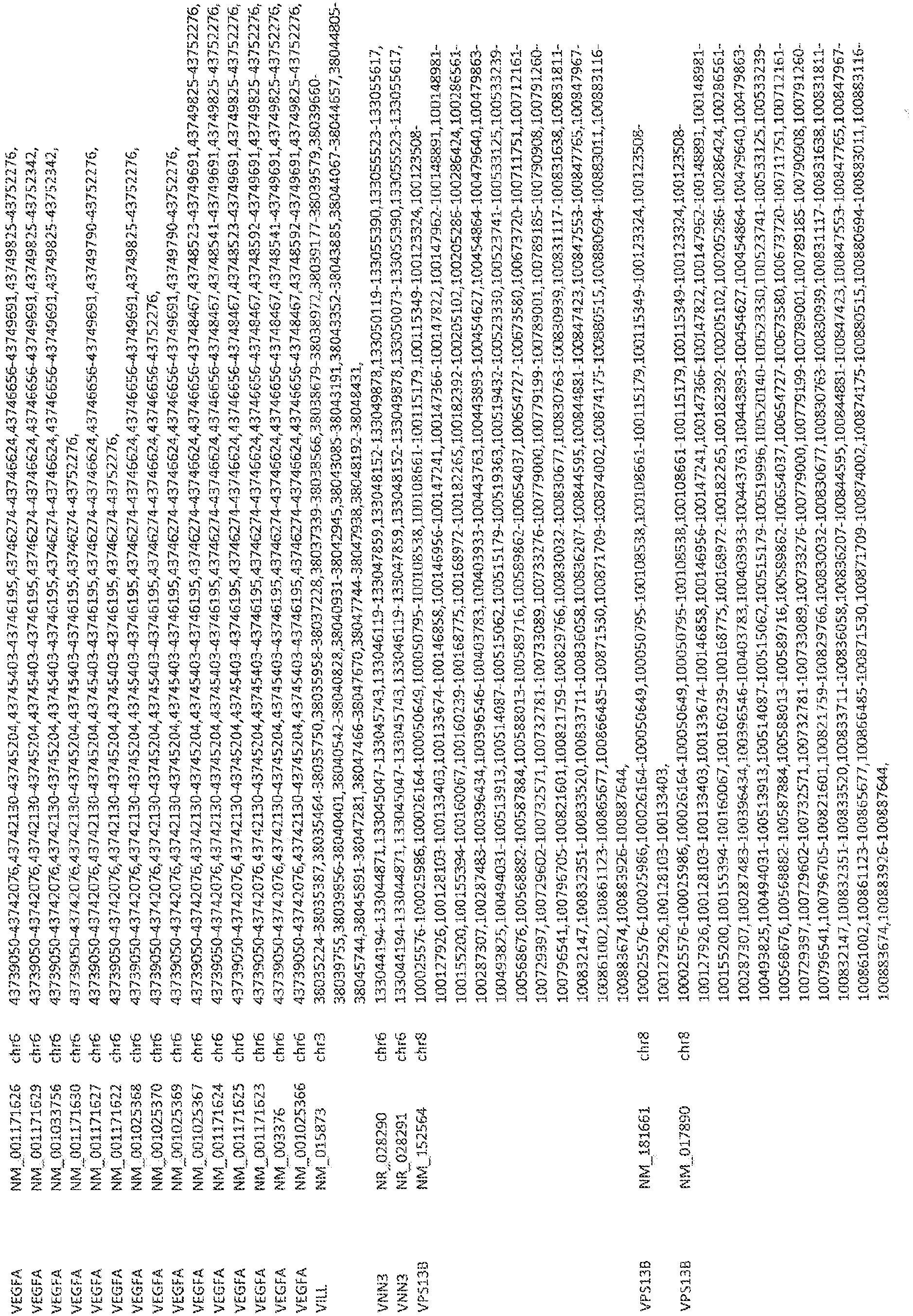

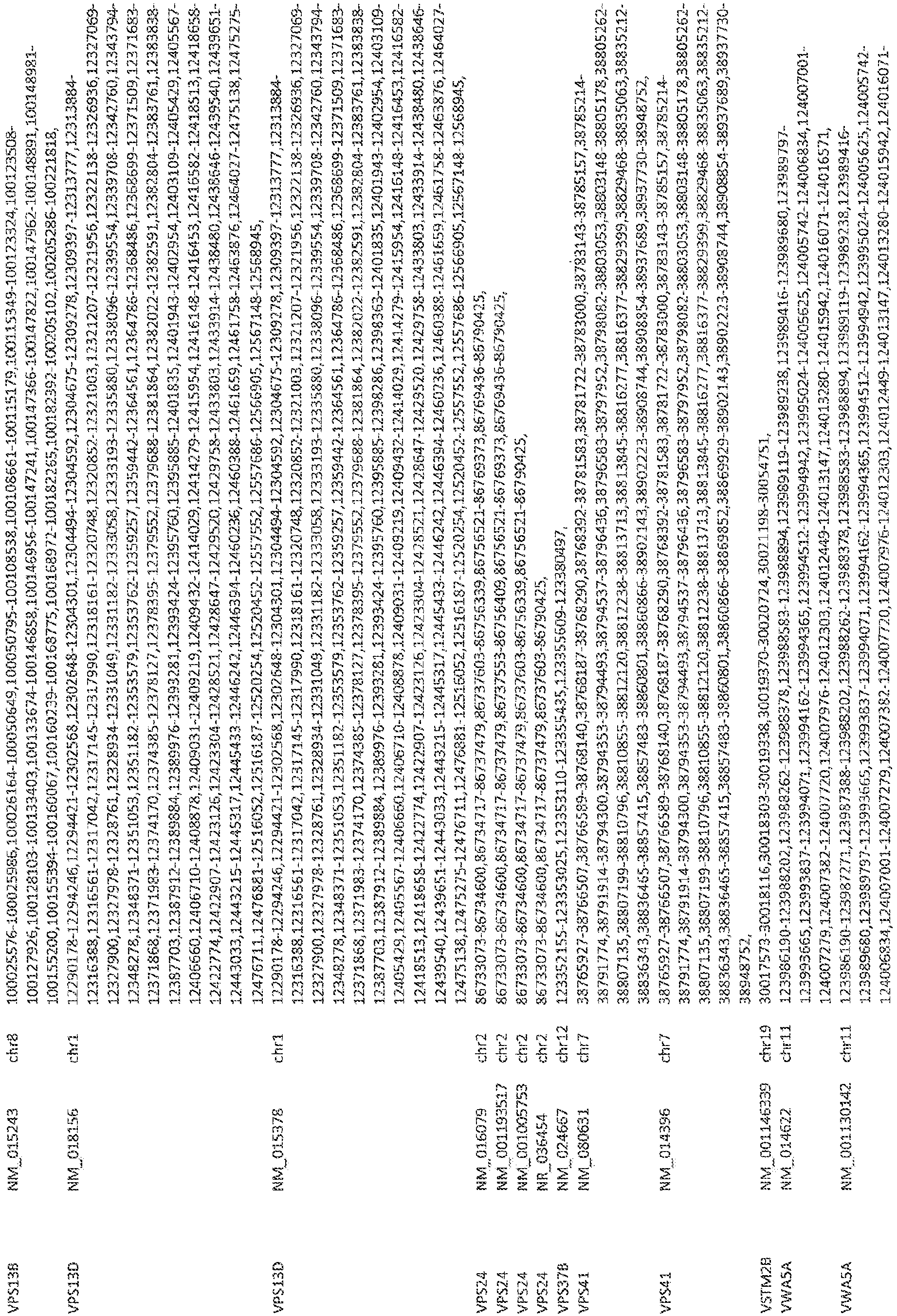

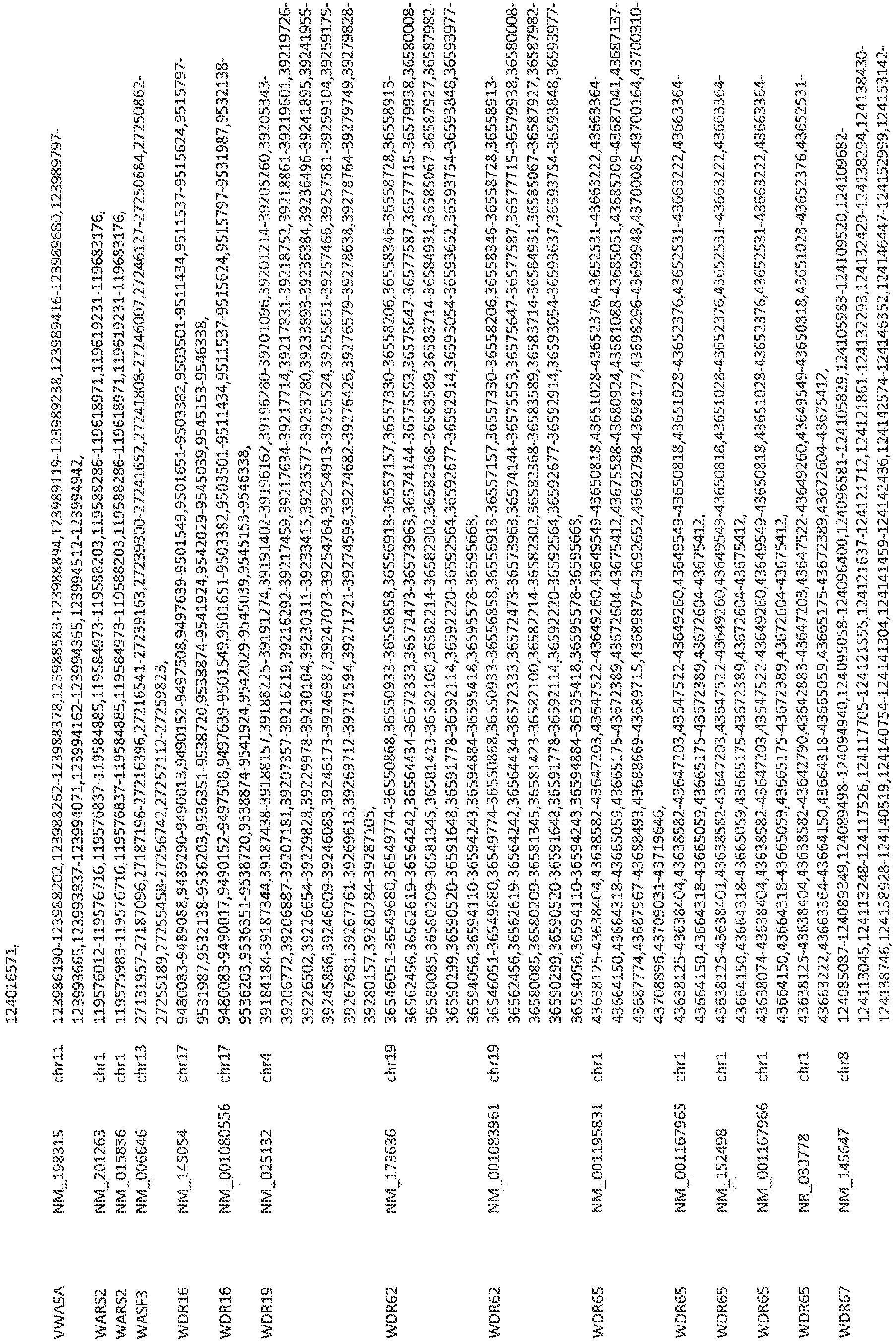

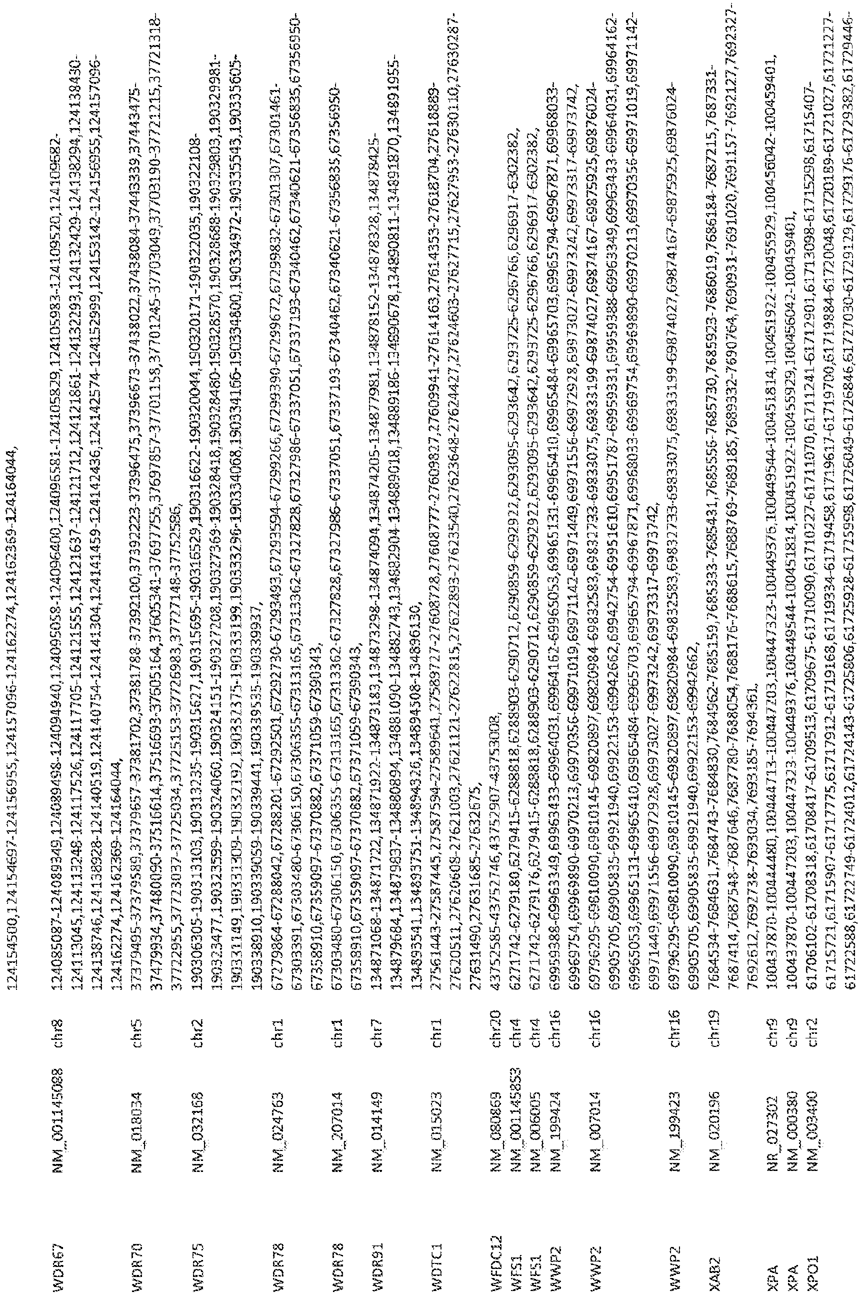

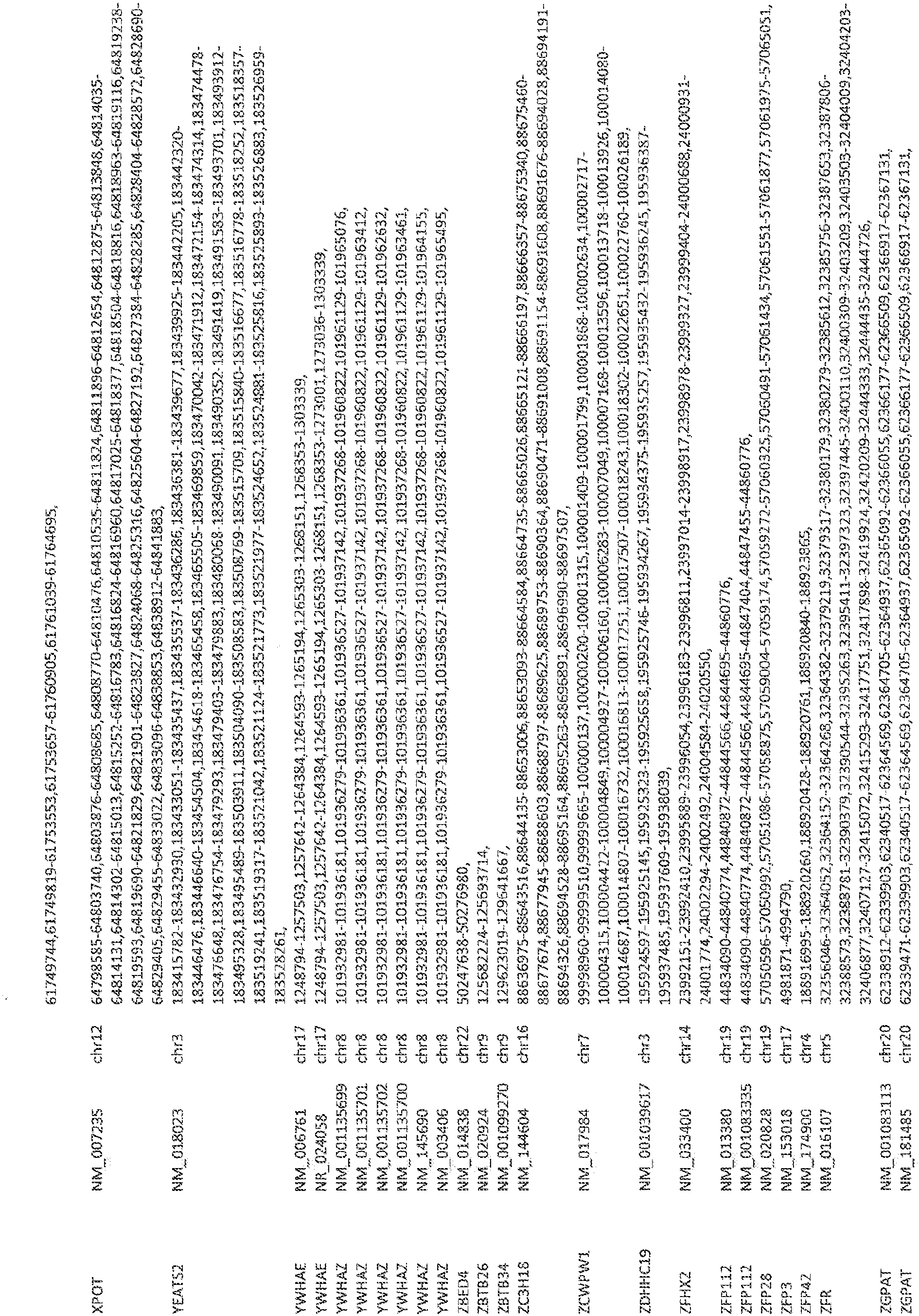

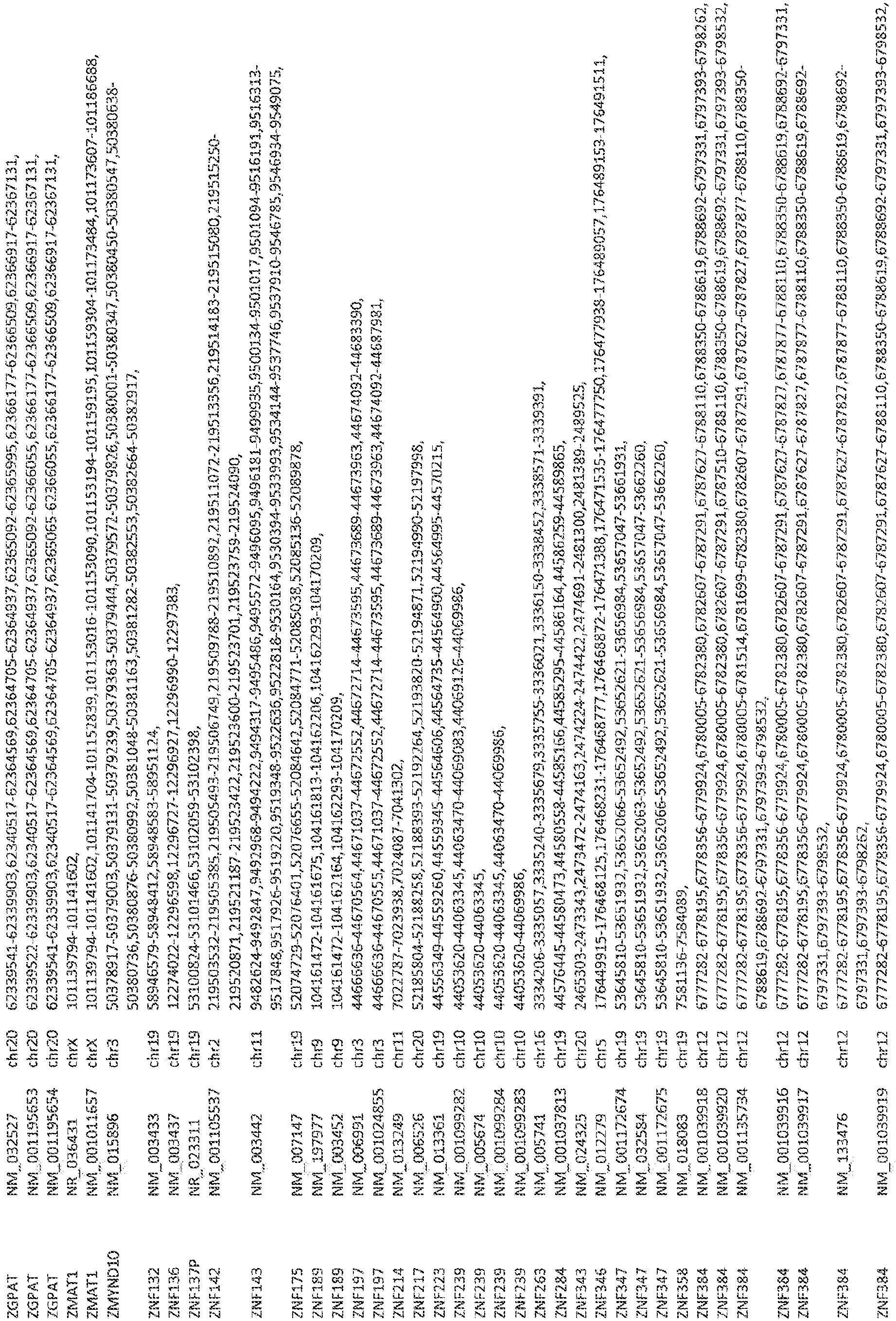

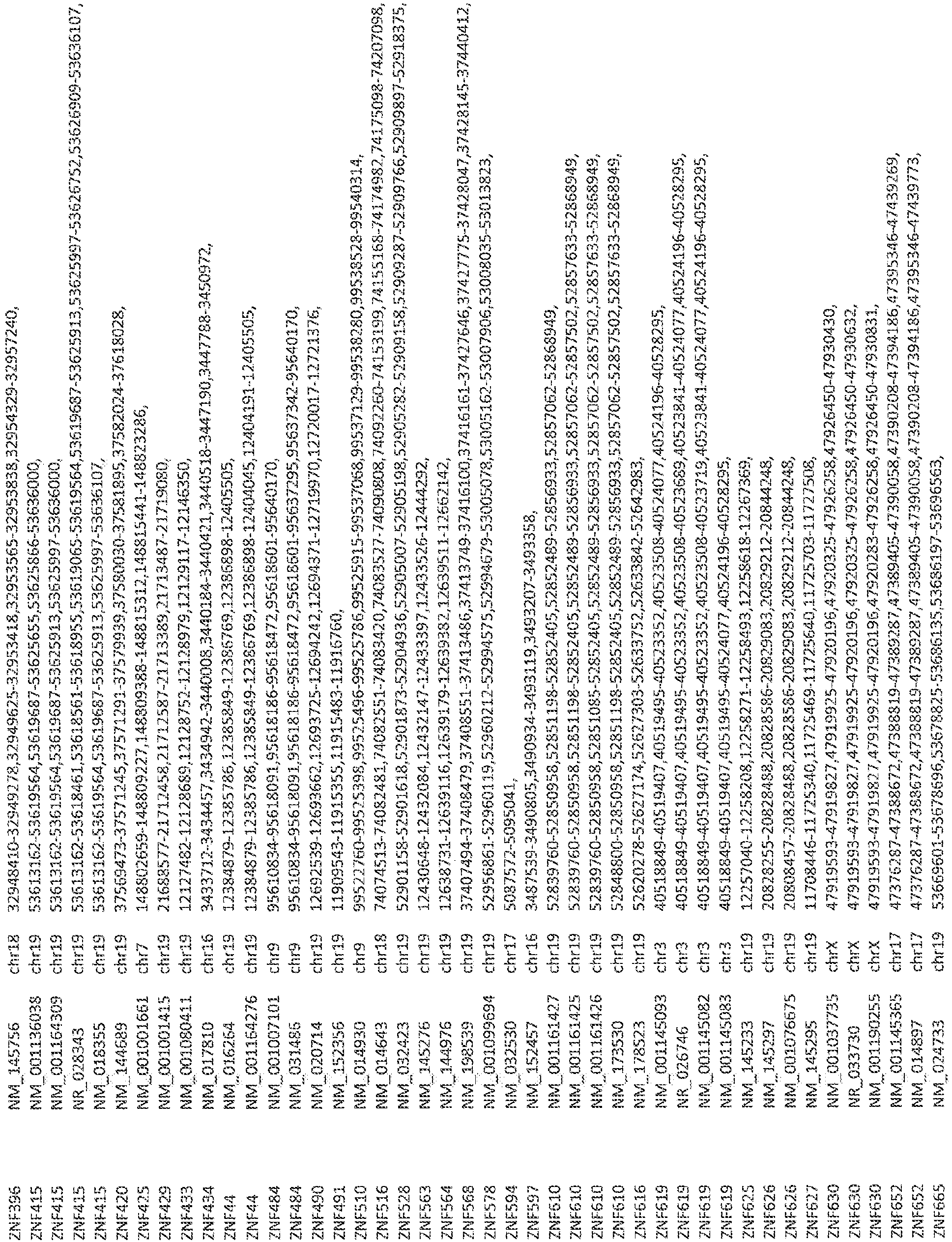

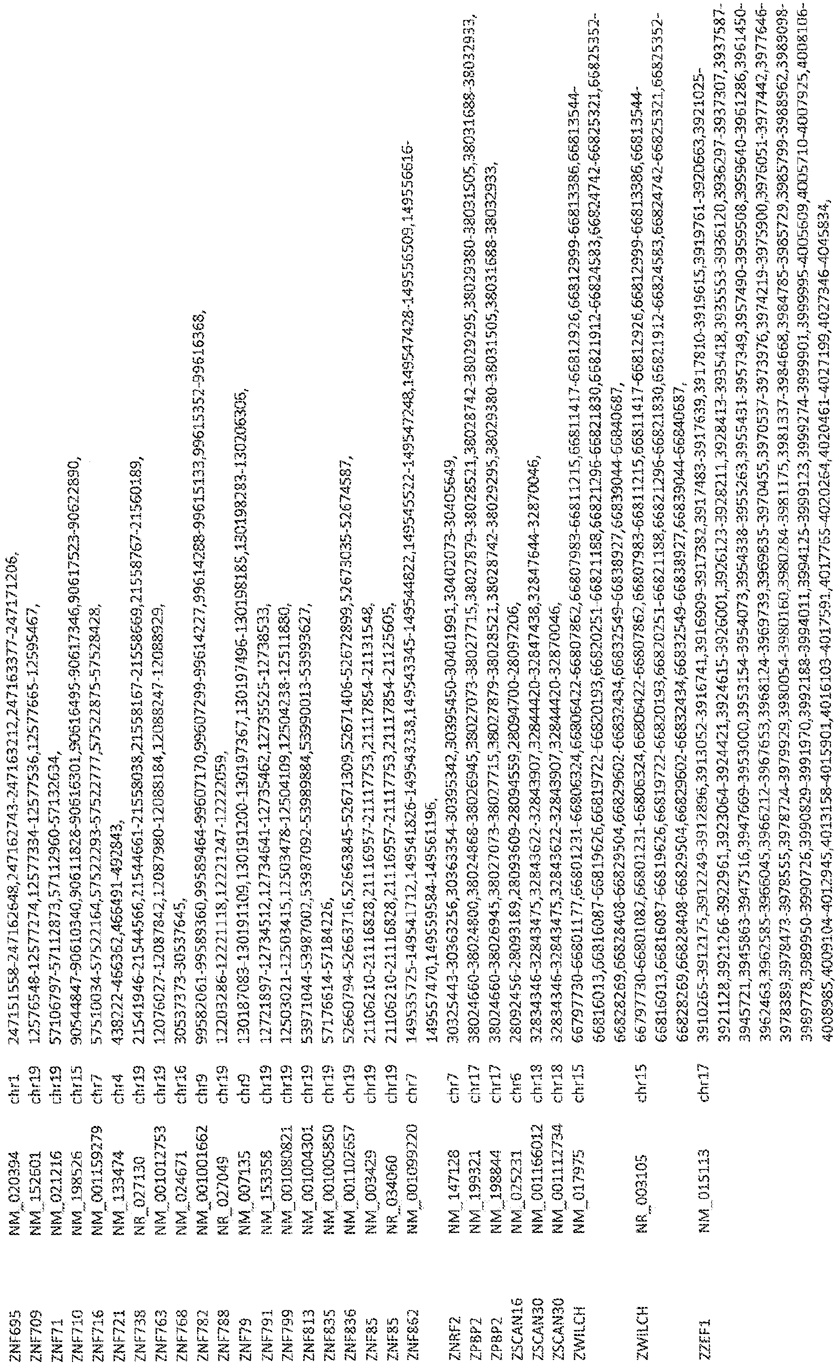

| **TABLE B** | | | | | |
|---|---|---|---|---|---|
| **GENE** | **Accession No.** | **GENE** | **Accession No.** | **GENE** | **Accession** No. |
| TRPS1 | NM_014112 | C3orf18 | NM_001171743 | LRRC49 | NM_001199018 |
| SHROOM3 | NM_020859 | C3orf18 | NM_001171740 | CYBASC3 | NM_001161452 |
| GREB1L | NM_001142966 | C3orf18 | NM_001171741 | CYBASC3 | NM_153611 |
| MICA | NR_036523 | C3orf15 | NM_033364 | CYBASC3 | NM_001161454 |
| MICA | NM_182924 | DIS3L | NM_001143688 | CLINT1 | NM_014666 |
| MICA | NR_036523 | DIS3L | NM_152383 | CLINT1 | NM_001195556 |
| MICA | NR_036523 | DIS3L | NM_133375 | CLINT1 | NM_001195555 |
| MICA | NR_036523 | PSMD8 | NM_002812 | CLCA2 | NM_006536 |
| MICA | NM_000247 | B4GALT5 | NM_004776 | RPRD1A | NM_018170 |
| MICA | NM_000247 | ARPC1B | NM_905720 | TSPAN10 | NM_031945 |
| MICA | NM_014632 | SLC26A5 | NM_001167962 | BCL2 | NM_000657 |
| MICA | NR_036523 | SLC26A5 | NM_206884 | BCL2 | NM_001040668 |
| MICA | NM_000247 | SLC26A5 | NM_206883 | BCL2 | NM_004049 |
| MICA | NR_036524 | SLC26A5 | NM_198999 | BCL2 | NM_001199864 |
| MICA | NR_036524 | SLC26A5 | NM_206885 | BCL2 | NM_138621 |
| MICA | NM_033386 | ZNF837 | NM_138466 | BCL2 | NM_020396 |
| MICA | NM_001122731 | ZNF837 | NM_001129730 | BCL2 | NM_138639 |
| MICA | NM_153206 | BEND5 | NM_024603 | BCL2 | NM_138723 |
| MICA | NM_022765 | LOC100128675 | NR_024561 | BCL2 | NM_001191 |
| MICA | NM_001136004 | LOC100128675 | NR_024562 | BCL2 | NM_138722 |
| MICA | NM0_01098526 | GRTP1 | NM_024719 | BCL2 | NM_ 006538 |
| MICA | NM_032867 | AP1M1 | NM_032493 | BCL2 | NM_001010922 |
| MICA | NM_001159291 | AP1M1 | NM_001130524 | BCL2 | NM_030766 |
| MICA | NM_000247 | RTN4RL2 | NM_178570 | BCL2 | NM_138578 |
| MICA | NR_036524 | MAGI1 | NM_001033057 | BCL2 | NM_001114735 |
| MICA | NR_036524 | MAGI1 | NM_015520 | BCL2 | NM_ 207002 |
| MICA | NM_015241 | MAGI1 | NM_004742 | BCL2 | NM_015367 |
| MICA | NM_001177519 | ENPP5 | NM_021572 | BCL2 | NM_004050 |
| MICA | NM_001177519 | BZRAP1 | NM_0004758 | BCL2 | NM_001199839 |
| MICA | NM_001177519 | BZRAP1 | NM_024418 | BCL2 | NM_000633 |
| MICA | NM_000247 | DDB1 | NM_001923 | BBS5 | NM_152384 |
| MICA | NM_001177519 | NEDD4L | NM_001144966 | LOC100128640 | NR_028389 |
| MICA | NM_001177519 | NEDD4L | NM_001144969 | CAMK2G | NM_172171 |
| MICA | NR_036524 | NEDD4L | NM_001144965 | CAMK2G | NM_172169 |
| PIP4K2B | NM_003559 | NEDD4L | NM_001144967 | CAMK2G | NM_172173 |
| CWC25 | NM_017748 | NEDD4L | NM_001144971 | CAMK2G | NM_172170 |
| SLITRK2 | NM_001144009 | NEDD4L | NM_001144964 | CAMK2G | NM_001222 |
| SLITRK2 | NM_001144005 | NEDD4L | NM_015277 | TSPAN14 | NM_030927 |
| SLITRK2 | NM_001144008 | NEDD4L | NM_001144968 | TSPAN14 | NM_001128309 |
| SLITRK2 | NM_001144010 | NEDD4L | NM_001144970 | RWDD3 | NM_015485 |
| SLITRK2 | NM_001144006 | TMEM56 | NM_152487 | RWDD3 | NM_001128142 |
| SLITRK2 | NM_001144003 | TMEM56 | NM_001199691 | RWDD3 | NM_001199691 |
| SLITRK2 | NM_001144004 | TMEM56 | NM_001199679 | RWDD3 | NR_037643 |
| SLITRK2 | NM_032539 | LRRC49 | NM_017691 | RWDD3 | NM_001199682 |
| C3orf18 | NM_016210 | LRRC49 | NM_001199017 | TRAK1 | NM_001042646 |
| TRAK1 | NM_014965 | EML2 | NR_034098 | MTR | NM_000254 |
| LOC730668 | NR_027240 | EML2 | NM_012155 | MTR | NM_002454 |
| ZNF621 | NM_198484 | EML2 | NR_002794 | MTR | NM_001190470 |
| ZNF621 | NM_001098414 | LAPTM4B | NM_018407 | MTR | NM_001190472 |
| PELI3 | NM_001098510 | SLC38A2 | NM_018976 | MTR | NM_001190452 |
| PELI3 | NM_145065 | SCARNA15 | NR_003011 | MTR | NM_001190489 |
| NCOA3 | NM_001174088 | MID2 | NM_133457 | MTR | NM_001190478 |
| NCOA3 | NM_001174087 | MID2 | NM_052817 | MTR | NM_019041 |
| NCOA3 | NM_181659 | MID2 | NM_012216 | MTR | NM_001190487 |
| NCOA3 | NM_006534 | SLC26A8 | NM_052961 | MTR | NM_001190706 |
| PSMB3 | NM_002795 | SLC26A8 | NM_ 138718 | MTR | NM_001190708 |
| ZNF763 | NM_001012753 | SLC26A8 | NM_001193476 | MTR | NM_001190702 |
| CHDH | NM_018397 | NKAPP1 | NR_027131 | WBSCR27 | NM_152559 |
| SUSD4 | NM_001037175 | CLN5 | NM_006493 | LOC100130093 | NR_024485 |
| SUSD4 | NM_017992 | TLR5 | NM_003268 | LOC100130093 | NR_024486 |
| DNAH14 | NM_144989 | NPY5R | NM_006174 | FGFRL1 | NM_021923 |
| DNAH14 | NM_001373 | LOC402778 | NM_001170820 | FGFRL1 | NM_001004358 |
| DNAH14 | NM_001145154 | RPL14 | NM_032111 | FGFRL1 | NM_001004356 |
| KRT4 | NM_182497 | RPL14 | NM_003973 | TSNAXIP1 | NM_018430 |
| KRT4 | NR_033415 | RPL14 | NM_001034996 | BAIAP3 | NM_003933 |
| KRT4 | NM_002272 | C6orf155 | NR_026807 | BAIAP3 | NM_001199099 |
| STK3 | NM_015690 | ABCA3 | NM_001089 | BAIAP3 | NM_001199097 |
| STK3 | NM_001112724 | LOC723809 | NR_027374 | BAIAP3 | NM_001199096 |
| STK3 | NM_018401 | EPB49 | NM_001114139 | BAIAP3 | NM_001199098 |
| STK3 | NM_006281 | EPB49 | NM_001114138 | TSC1 | NM_001162427 |
| STK3 | NM_173575 | EPB49 | NM_001114137 | TSC1 | NM_000368 |
| STK3 | NM_015000 | EPB49 | NM_001978 | TSC1 | NM_001162426 |
| STK3 | NM_007271 | EPB49 | NM_001114136 | LDLRAD3 | NM_174902 |
| STK3 | NM_031414 | EPB49 | NM_001114135 | NPY1R | NM_000909 |
| STK3 | NM_013233 | PLEKHA7 | NM_175058 | IKBIP | NM_153687 |
| STK3 | NM_080836 | RNU6ATAC | NR_023344 | IKBIP | NM_201613 |
| STK3 | NM_030906 | LAMC2 | NM_005562 | IKBIP | NM_201612 |
| STK3 | NM_145001 | LAMC2 | NM_018891 | WDR67 | NM_145647 |
| STK3 | NM_001122833 | MST1 | NR_927504 | WDR67 | NM_001145088 |
| STK3 | NM_032944 | MST1 | NM_002447 | HMGCR | NM_000859 |
| CCDC24 | NM_152499 | MST1 | NM_020998 | HMGCR | NM_001130996 |
| IQCH | NM_001031715 | MST1 | NR_002729 | ZNF415 | NM_001136038 |
| IQCH | NM_022784 | GSTM2 | NM_001142368 | ZNF415 | NM_001164309 |
| NCRNA00173 | NR_027346 | GSTM2 | NR_002932 | ZNF415 | NR_028343 |
| NCRNA00173 | NR_027345 | GSTM2 | NM_000848 | ZNF415 | NM_018355 |
| GTPBP10 | NM_001042717 | ZNF337 | NM_015655 | LMX1B | NM_001174147 |
| GTPBP10 | NM_033107 | MTR | NM_024010 | LMX1B | NM_001174146 |
| EML2 | NM_001193268 | MTR | NM_001190476 | LMX1B | NM_002316 |
| EML2 | NM_001193269 | MTR | NM_001114184 | RBBP8 | NM_203292 |
| EML2 | NM_024807 | MTR | NM_004294 | RBBP8 | NM_203291 |
| RBBP8 | NM_002894 | PSD | NM_002779 | PTPN18 | NM_001142370 |
| SORBS2 | NM_001145673 | PSD | NM_184231 | PTPN18 | NM_014369 |
| SORBS2 | NM_021069 | PSD | NM_015310 | ENTPD8 | NM_198585 |
| SORBS2 | NM_003603 | PSD | NM_032289 | ENTPD8 | NM_001033113 |
| SORBS2 | NM_001145674 | PSD | NM_012217 | MTFR1 | NM_014637 |
| SORBS2 | NM_001145675 | PSD | NM_012455 | MTFR1 | NM_001145839 |
| SORBS2 | NM_001145672 | FAM201A | NR_027294 | MTFR1 | NM_001145838 |
| SORBS2 | NM_001145670 | C9orf156 | NM_016481 | APC2 | NM_013366 |
| SORBS2 | NM_001145671 | LOC644759 | NR_033737 | APC2 | NR_030717 |
| LASP1 | NM_001142273 | PPP1R9A | NM_001166161 | APC2 | NM_005883 |
| LASP1 | NM_001142274 | PPP1R9A | NM_001166163 | PSD | NM_016453 |
| LASP1 | NM_006148 | PPP1R9A | NM_001166162 | PSD | NM_206909 |
| LASP1 | NM_015282 | PPP1R9A | NM_017650 | ARMCX6 | NM_019007 |
| PLCB3 | NM_000932 | PPP1R9A | NM_001166160 | ARMCX6 | NR_033669 |
| PLCB3 | NM_001184883 | LRRN2 | NM_006338 | ARMCX6 | NR_033670 |
| CKAP4 | NM_006825 | LRRN2 | NM_201630 | ARMCX6 | NM_001184768 |
| RPPH1 | NR_002312 | OGFRL1 | NM_024576 | ARMCX6 | NM_001009584 |
| SCARNA16 | NR_003013 | OGDHL | NM_001143997 | | |
| MFSD2A | NM_032793 | OGDHL | NM_001143996 | | |
| MFSD2A | NM_001136493 | OGDHL | NM_018245 | | |
| ICA1 | NM_138468 | G6PD | NM_000402 | | |
| ICA1 | NM_001136020 | G6PD | NM_001042351 | | |
| ICA1 | NM_153206 | TMSB10 | NM_021103 | | |
| ICA1 | NM_001098526 | VASP | NM_003370 | | |
| ICA1 | NM_004968 | SEC23A | NM_006364 | | |
| ICA1 | NM_178231 | TMEM86A | NM_153347 | | |
| ICA1 | NM_022307 | ZNF774 | NM_001004309 | | |
| OPLAH | NM_017570 | CLIC6 | NM_053277 | | |
| C2orf55 | NM_207362 | ATL1 | NM_080661 | | |
| ZNF48 | NM_052852 | ATL1 | NM_181598 | | |
| ZNF48 | NM_901007101 | ATL1 | NM_001127713 | | |
| ZNF48 | NM_153034 | ATL1 | NM_015915 | | |
| ZNF48 | NM_144684 | ATL1 | NM_152716 | | |
| ZNF48 | NM_001007169 | ATL1 | NM_001012980 | | |
| ZNF48 | NM_133464 | ATL1 | NM_032558 | | |
| ZNF48 | NM_031486 | C3orf23 | NM_173826 | | |
| ZNF48 | NR_026693 | C3orf23 | NM_001029840 | | |
| ZNF48 | NM_145312 | C3orf23 | NM_001029839 | | |
| ZNF48 | NM_152652 | CHKB | NM_005198 | | |
| APC2 | NM_003083 | CHKB | NR_027928 | | |

## Claims

1. A method of predicting a likelihood of survival for at least 3 years without recurrence of breast cancer in a breast cancer patient, comprising:
determining a level of the breast cancer prognostic biomarker kinesin family member 1B (KIF1B) in a breast cancer tumor sample obtained from the patient,
normalizing the level of the breast cancer prognostic biomarker KIF1B to obtain a normalized level of KIF1B; and
predicting a likelihood of survival for at least 3 years without recurrence of breast cancer of said patient,
wherein an increased normalized level of the breast cancer prognostic biomarker KIF 1 B compared with the amount found in a cancer tissue reference set is negatively correlated with an increased likelihood of survival for at least 3 years without recurrence of breast cancer.

2. A method of predicting a likelihood of survival for at least 3 years without recurrence of breast cancer in a breast cancer patient, comprising:
determining levels of at least three RNA transcripts, or expression products thereof, in a breast cancer tumor sample obtained from said patient, wherein the at least three RNA transcripts, or expression products thereof, are selected from ENO1, IDH2, TMSB10, PGK1, and G6PD,
normalizing the levels of the at least three RNA transcripts, or expression products thereof, to obtain normalized expression levels of the at least three RNA transcripts or expression products thereof, and
predicting a likelihood of survival for at least 3 years without recurrence of breast
cancer of said patient using the normalized expression levels, wherein increased normalized expression levels compared with the amount found in a cancer tissue reference set are negatively correlated with an increased likelihood of survival for at least 3 years without recurrence of breast cancer.

3. The method of claim 2, wherein the levels of at least IDH2, PGK1, and G6PD are determined.

4. The method of claim 2, wherein the levels of ENO1, IDH2, TMSB10, PGK1, and G6PD are determined.

5. A method of predicting a likelihood of survival for at least 3 years without recurrence of breast cancer in a breast cancer patient, comprising:
determining levels of at least five RNA transcripts, or expression product thereof,
in a breast cancer tumor sample obtained from said patient, wherein the at least five RNA transcripts, or expression products thereof, are selected from PGD,
TKT, TALDO1, G6PD, GP1, SLC1A5, SLC7A5, OGDH, SUCLG1, ENOI, PGK1, IDH2, AC02, and FBP1,
normalizing the levels of the at least five RNA transcripts, or expression products thereof, to obtain normalized expression levels of the at least five RNA transcripts or expression products thereof, and
predicting a likelihood of survival for at least 3 years without recurrence of breast
cancer of said patient using the normalized expression levels, wherein increased normalized expression levels of PGD, TKT, TALDO 1, G6PD, GP 1, SLC1A5, SLC7A5, OGDH, SUCLG1, ENO1, PGK1, IDH2, and AC02 compared with the amount found in a cancer tissue reference set are negatively correlated with an increased likelihood of survival for at least 3 years without recurrence of breast cancer, and increased normalized expression level of FBP 1 is positively correlated with an increased likelihood of survival for at least 3 years without recurrence of breast cancer.

6. The method of claim 5, wherein the levels of PGD, TKT, TALDO1, G6PD, GP1, SLC1A5, SLC7A5, OGDH, SUCLG1, ENO1, PGK1, IDH2, ACO2, and FBP1 are determined.

7. The method of any one of claims 1-6, wherein the level is determined by whole
transcriptome sequencing, reverse transcription polymerase chain reaction (RT - PCR), or array.

8. The method of any one of claims 1-7, wherein the breast cancer tumor sample is a fixed, wax-embedded tissue sample.

9. The method of any one of claims 1-8, wherein the breast cancer tumor sample is a fine needle biopsy sample.

10. The method of any one of claims 1-9, further comprising creating a report based on the level of the one or more RNA transcripts, or an expression product thereof.

## Patentansprüche

1. Verfahren zur Vorhersage einer Wahrscheinlichkeit eines Überlebens für mindestens 3 Jahre ohne Wiederauftreten von Brustkrebs bei einem Brustkrebspatienten, umfassend:
Bestimmen eines Spiegels des Brustkrebsprognose-Biomarkers Kinesin-Familienmitglied 1B (KIF1B) in einer Brustkrebstumorprobe, die von dem Patienten erhalten wurde,
Normalisieren des Spiegels des Brustkrebsprognose-Biomarkers KIF1B, um einen normalisierten Spiegel von KIF1B zu erhalten, und
Vorhersagen einer Wahrscheinlichkeit eines Überlebens des Patienten für mindestens 3 Jahre ohne Wiederauftreten von Brustkrebs,
wobei ein erhöhter normalisierter Spiegel des Brustkrebsprognose-Biomarkers KIF1B im Vergleich zu der Menge, die in einem Krebsgewebereferenzsatz gefunden wird, mit einer erhöhten Wahrscheinlichkeit eines Überlebens für mindestens 3 Jahre ohne Wiederauftreten von Brustkrebs negativ korreliert ist.

2. Verfahren zur Vorhersage einer Wahrscheinlichkeit eines Überlebens für mindestens 3 Jahre ohne Wiederauftreten von Brustkrebs bei einem Brustkrebspatienten, umfassend:
Bestimmen von Spiegeln von mindestens drei RNA-Transkripten oder
Expressionsprodukten davon in einer Brustkrebstumorprobe, die von dem Patienten erhalten wurde, wobei die mindestens drei RNA-Transkripte oder Expressionsprodukte davon aus ENO1, IDH2, TMSB10, PGK1 und G6PD ausgewählt sind,
Normalisieren der Spiegel der mindestens drei RNA-Transkripte oder Expressionsprodukte davon, um normalisierte Expressionsspiegel der mindestens drei RNA-Transkripte oder Expressionsprodukte davon zu erhalten, und
Vorhersagen einer Wahrscheinlichkeit eines Überlebens des Patienten für mindestens 3 Jahre ohne Wiederauftreten von Brustkrebs unter Verwendung der normalisierten Expressionsspiegel, wobei erhöhte normalisierte Expressionsspiegel im Vergleich zu der Menge, die in einem Krebsgewebereferenzsatz gefunden wird, mit einer erhöhten Wahrscheinlichkeit eines Überlebens für mindestens 3 Jahre ohne Wiederauftreten von Brustkrebs negativ korreliert sind.

3. Verfahren nach Anspruch 2, wobei die Spiegel von mindestens IDH2, PGK1 und G6PD bestimmt werden.

4. Verfahren nach Anspruch 2, wobei die Spiegel von ENO1, IDH2, TMSB10, PGK1 und G6PD bestimmt werden.

5. Verfahren zur Vorhersage einer Wahrscheinlichkeit eines Überlebens für mindestens 3 Jahre ohne Wiederauftreten von Brustkrebs bei einem Brustkrebspatienten, umfassend:
Bestimmen von Spiegeln von mindestens fünf RNA-Transkripten oder einem Expressionsprodukt davon in einer Brustkrebstumorprobe, die von dem Patienten erhalten wurde, wobei die mindestens fünf RNA-Transkripte oder
Expressionsprodukte davon aus PGD, TKT, TALDO1, G6PD, GP1, SLC1A5, SLC7A5, OGDH, SUCLG1, ENO1, PGK1, IDH2, ACO2 und FBP1 ausgewählt sind, Normalisieren der Spiegel der mindestens fünf RNA-Transkripte oder Expressionsprodukte davon, um normalisierte Expressionsspiegel der mindestens fünf RNA-Transkripte oder Expressionsprodukte davon zu erhalten, und
Vorhersagen einer Wahrscheinlichkeit eines Überlebens des Patienten für mindestens 3 Jahre ohne Wiederauftreten von Brustkrebs unter Verwendung der normalisierten Expressionsspiegel, wobei erhöhte normalisierte Expressionsspiegel von PGD, TKT, TALDO1, G6PD, GP1, SLC1A5, SLC7A5, OGDH, SUCLG1, ENO1, PGK1, IDH2 und ACO2 im Vergleich zu der Menge, die in einem Krebsgewebereferenzsatz gefunden wird, mit einer erhöhten Wahrscheinlichkeit eines Überlebens für mindestens 3 Jahre ohne Wiederauftreten von Brustkrebs negativ korreliert sind und ein erhöhter normalisierter Expressionsspiegel von FBP1 mit einer erhöhten Wahrscheinlichkeit eines Überlebens für mindestens 3 Jahre ohne Wiederauftreten von Brustkrebs positiv korreliert ist.

6. Verfahren nach Anspruch 5, wobei die Spiegel von PGD, TKT, TALDO1, G6PD, GP1, SLC1A5, SLC7A5, OGDH, SUCLG1, ENO1, PGK1, IDH2, ACO2 und FBP1 bestimmt werden.

7. Verfahren nach einem der Ansprüche 1-6, wobei der Spiegel durch Gesamt-Transkriptom-Sequenzierung, Umkehrtranskriptionspolymerase-Kettenreaktion (RT-PCR) oder ein Array bestimmt wird.

8. Verfahren nach einem der Ansprüche 1-7, wobei die Brustkrebstumorprobe eine fixierte, in Wachs eingebettete Gewebeprobe ist.

9. Verfahren nach einem der Ansprüche 1-8, wobei die Brustkrebstumorprobe eine Feinnadelbiopsieprobe ist.

10. Verfahren nach einem der Ansprüche 1-9, das weiterhin das Erstellen eines Berichts auf der Basis des Spiegels des einen oder der mehreren RNA-Transkripte oder eines Expressionsprodukts davon umfasst.

## Revendications

1. Un procédé de prédiction de la probabilité de survie pendant au moins 3 ans sans récurrence du cancer du sein chez une patiente atteinte de cancer du sein, comprenant les étapes consistant à :
déterminer un niveau du membre de la famille kinésine biomarqueur de pronostic du cancer du sein 1B (KIF1B) dans un échantillon tumoral du cancer du sein prélevé sur la patiente,
normaliser le niveau du biomarqueur de pronostic du cancer du sein KIF1B pour obtenir un niveau normalisé du KIF1B ; et
prédire une probabilité de survie pendant au moins 3 ans sans récurrence du cancer du sein de ladite patiente,
dans lequel un niveau normalisé accru du biomarqueur de pronostic du cancer du sein KIF1B comparé à la quantité trouvée dans un ensemble de référence de tissu cancéreux est négativement corrélé avec une probabilité accrue de survie pendant au moins 3 ans sans récurrence du cancer du sein.

2. Un procédé de prédiction de la probabilité de survie pendant au moins 3 ans sans récurrence du cancer du sein chez une patiente atteinte de cancer du sein, comprenant les étapes consistant à :
déterminer les niveaux d'au moins trois transcrits d'ARN, ou leurs produits d'expression, dans un échantillon tumoral du cancer du sein prélevé sur ladite patiente,
dans lequel les au moins trois transcrits d'ARN, ou leurs produits d'expression, sont sélectionnés parmi ENO1, IDH2, TMSB10, PGK1 et G6PD,
normaliser les niveaux des au moins trois transcrits d'ARN, ou leurs produits d'expression, pour obtenir des niveaux d'expression normalisés des au moins trois transcrits d'ARN, ou leurs produits d'expression, et
prédire une probabilité de survie pendant au moins 3 ans sans récurrence du cancer du sein de ladite patiente à l'aide des niveaux d'expression normalisés, dans lequel des niveaux d'expression normalisés accrus comparés à la quantité trouvée dans un ensemble de référence de tissu cancéreux sont négativement corrélés avec une probabilité accrue de survie pendant au moins 3 ans sans récurrence du cancer du sein.

3. Le procédé de la revendication 2, dans lequel les niveaux d'au moins IDH2, PGK1 et G6PD sont déterminés.

4. Le procédé de la revendication 2, dans lequel les niveaux d'ENO1, IDH2, TMSB10, PGK1 et G6PD sont déterminés.

5. Un procédé de prédiction de la probabilité de survie pendant au moins 3 ans sans récurrence du cancer du sein chez une patiente atteinte de cancer du sein, comprenant les étapes consistant à :
déterminer les niveaux d'au moins cinq transcrits d'ARN, ou leurs produits d'expression, dans un échantillon tumoral du cancer du sein prélevé sur ladite patiente, dans lequel les au moins cinq transcrits d'ARN, ou leurs produits d'expression, sont sélectionnés parmi PGD, TKT, TALDO1, G6PD, GP1, SLC1A5, SLC7A5, OGDH, SUCLG1, ENOI, PGK1, IDH2, AC02 et FBP1,
normaliser les niveaux des au moins cinq transcrits d'ARN, ou leurs produits d'expression, pour obtenir des niveaux d'expression normalisés des au moins cinq transcrits d'ARN, ou leurs produits d'expression, et
prédire une probabilité de survie pendant au moins 3 ans sans récurrence du cancer du sein de ladite patiente à l'aide des niveaux d'expression normalisés, dans lequel des niveaux d'expression normalisés accrus de PGD, TKT, T ALDO 1, G6PD, GP 1, SLC1A5, SLC7A5, OGDH, SUCLG1, ENO1, PGK1, IDH2 et AC02 comparés à la quantité trouvée dans un ensemble de référence de tissu cancéreux sont négativement corrélés avec une probabilité accrue de survie pendant au moins 3 ans sans récurrence du cancer du sein, et un niveau d'expression normalisé accru de FBP 1 est positivement corrélé avec une probabilité accrue de survie pendant au moins 3 ans sans récurrence du cancer du sein.

6. Le procédé de la revendication 5, dans lequel les niveaux de PGD, TKT, TALDO1, G6PD, GP1, SLC1A5, SLC7A5, OGDH, SUCLG1, ENO1, PGK1, IDH2, ACO2 et FBP1 sont déterminés.

7. Le procédé d'une quelconque des revendications 1-6, dans lequel le niveau est déterminé par séquençage de transcriptome entier, réaction en chaîne de polymérase par transcription inverse (RT - PCR), ou réseau.

8. Le procédé d'une quelconque des revendications 1-7, dans lequel l'échantillon tumorale de cancer du sein est un échantillon de tissu fixe, inclus dans la cire.

9. Le procédé d'une quelconque des revendications 1-8, dans lequel l'échantillon tumoral de cancer du sein est un échantillon de biopsie à l'aiguille fine.

10. Le procédé d'une quelconque des revendications 1-9, comprenant en sus la création d'un rapport basé sur le niveau du ou des transcrits d'ARN, ou leurs produits d'expression.
